# EUROPEAN PATENT SPECIFICATION

(11) **EP 2 260 110 B1**
(45) Date of publication and mention of the grant of the patent: **12.11.2014**
(21) Application number: 09709348.8
(22) Date of filing: 09.02.2009
(51) Int. Cl.: C12Q 1/68

(54) **miRNAs DIFFERENTIALLY EXPRESSED IN LYMPH NODES FROM CANCER PATIENTS**
IN LYMPHKNOTEN VON KREBSPATIENTEN DIFFERENTIELL EXPRIMIERTE MIRNAS
MICRO ARN (MIRNA) EXPRIMÉS DIFFÉRENTIELLEMENT DANS DES NOEUDS LYMPHOÏDES PRÉLEVÉS CHEZ DES PATIENTS ATTEINTS D'UN CANCER

(30) Priority: 08.02.2008 US 27198 P
(43) Date of publication of application: 15.12.2010
(73) Proprietor: Asuragen, INC., Austin, TX 78744 (US)
(72) Inventor: BEAUDENON, Sylvie, Austin, TX 78744 (US); ELIZONDO, Laura, Austin, TX 78744 (US); DOLESHAL, Martina, Austin, TX 78744 (US); BROWN, David, Austin, TX 78704 (US); LABOURIER, Emmanuel, Austin, TX 78749 (US)
(74) Representative: Sonn & Partner Patentanwälte
(86) International application number: PCT/US2009/033556
(87) International publication number: WO 2009/100430

(56) References cited:
- WO-A-2007/073737
- US-A1- 2007 054 287
- US-A1- 2007 161 004
- "POSTER ABSTRACTS" ANNALS OF SURGICAL ONCOLOGY, SPRINGER-VERLAG, NE, vol. 15, no. 1, 29 January 2008 (2008-01-29), pages 33-64, XP019568707 ISSN: 1534-4681
- LU JUN ET AL: "MicroRNA expression profiles classify human cancers" NATURE, NATURE PUBLISHING GROUP, GB, vol. 435, no. 7043, 9 June 2005 (2005-06-09), pages 834-838, XP002485555
- LIANG YU ET AL: "Characterization of microRNA expression profiles in normal human tissues" BMC GENOMICS, BIOMED CENTRAL, LONDON, GB, vol. 8, no. 1, 12 June 2007 (2007-06-12), page 166, XP021027980 ISSN: 1471-2164

## Description

### BACKGROUND OF THE INVENTION

This application claims priority to U.S. Provisional Patent Application serial number 61/027,198 filed February 8, 2008.

### I. FIELD OF THE INVENTION

The present invention relates generally to the fields of molecular biology and oncology. More particularly, it concerns methods and compositions involving microRNA (miRNAs) molecules and cancer prognosis and staging. Certain aspects of the invention include applications for miRNAs in diagnosis and staging of various cancers and in patient monitoring.

### II. BACKGROUND

Cancer remains a serious public health problem in the United States and other developed countries. Currently, one in four deaths in the United States is due to cancer (Jemal *et al*., 2007). Cancers of the lung/bronchus, prostate, and colon/rectum in men, and cancers of the lung/bronchus, breast, and colon/rectum in women are the most common fatal cancers in the US, accounting for half of all cancer deaths (Jemal *et al*., 2007). Only a handful of treatments are available for specific types of cancer, and these provide no guarantee of success. To be most effective, cancer treatment requires not only an early detection of the malignancy, but a reliable assessment of the severity of the malignancy.

Cancer staging describes the extent or severity of an individual's cancer based on characteristics of the original (primary) tumor and the extent, if any, of cancer spread in the body. Clinical trials have compared patients of similar stages to predict the probability of cancer recurrence after surgical removal and to choose appropriate clinical management and surveillance.

In the United States, the American Joint Committee on Cancer (AJCC) provides criteria for staging based on the TNM staging system (Greene, 2002). The TNM system was developed by the International Union Against Cancer (UICC) and the American Joint Committee on Cancer (AJCC) and attempts to provide a uniform stratification of patients that allows for the comparison of patients in clinical studies and for determining optimal treatment and survival rates. Common elements considered in the TNM and in most other staging systems include location of the primary tumor, tumor size and number of tumors (T), lymph node involvement (spread of cancer into lymph nodes) (N), cell type and tumor grade (how closely the cancer cells resemble normal tissue), and presence or absence of cancer metastasis (M). TNM criteria are different for each anatomic cancer site. Cancer staging criteria are modified and updated over time, as scientists learn more about individual cancer types and identify which aspects of the system represent accurate predictors for disease recurrence and patient survival.

For many cancers, lymph node involvement, or metastasis to lymph nodes, has become recognized as a strong predictor of disease recurrence and patient survival. Metastatic deposits visible to the naked eye (>2 mm) are used to denote whether a lymph node is positive (Greene, 2002). Currently, metastatic cancer deposits <2 mm in size that are found in a lymph node are referred to as micrometastatic lesions or micrometastatic disease (MMD). However, under current staging systems, lymph nodes with MMD remain classified as negative (NO) for cancer metastasis. MMD is currently only recognized in the staging of breast cancer, due to extenisve evaluations of the prognostic significance of sentinel lymph nodes from breast cancer patients.

In general, current methods for assessing lymph node involvement, especially those for evaluating the presence of MMD, have significant limitations. Classifying a tumor as metastatic is a coordinated effort among a pathologist, a radiologist, a surgeon, and an oncologist. Microscopic examination of lymph nodes, typically performed by a pathologist, is essential for proper cancer staging. Only a very small fraction of a lymph node (approximately 0.1-0.2%), is evaluated microscopically for metastatic cancer cells by a pathologist. Immunohistochemical (IHC) staining can increase the sensitivity by subjecting an additional small fraction of the lymph node to a more specific assay. Likewise, tissue sectioning at additional levels of the lymph node can increase the percentage of the lymph node that is examined, but this procedure is used infrequently and most commonly for sentinel lymph node evaluation. Furthermore, protocols for lymph node evaluation are not standardized among pathologists, and no current protocol allows for microscopic examination of more than 10% of a lymph node (Coello *et al*., 2004; Hughes *et al*., 2006; Ferris *et al.,* 2005; Xi *et al*., 2005; Xi *et al*., 2006; Takeuchi *et al.,* 2004; Scoggins *et al*., 2006; D'Cunha *et al*., 2005; Gillanders *et al*., 2004; Schurr *et al.,* 2006; Houvenaeghel *et al.,* 2006; Kammula *et al*., 2004). In addition, at least one group has reported alarming discordance in histologic interpretations by different pathologists (Roberts et al., 2003). As a result of these limitations, 25% to 35% of cancer patients, who are lymph node-negative by the present state of the art examination, will experience a recurrence of their cancer. It is believed that a major contributing factor to missed metastases is the presence in the lymph node of occult metastasis (i.e., not readily apparent or hidden) and/or MMD that is not detected by the present state of the art examination. The current invention overcomes these problems in the art by describing quantitative molecular methods and novel molecular markers (microRNAs) (Lagos-Quintana *et al*., 2001; Lau *et al*., 2001; Lee and Ambros, 2001) for detection of metastatic cancer in lymph nodes..

Beaudenon et al. describe in abstract P01 on page 55 of "POSTER ABSTRACTS", ANNALS OF SURGICAL ONCOLOGY( SPRINGER-VERLAG, NE, vol. 15, no. 1, 29 January 2008, pages 33-64) microRNAs as novel biomarkers for detecting cervical cancer lymph node metastasis.

microRNAs (miRNAs) are short RNA molecules (17-24 nucleotides in length) that arise from longer precursors, which are transcribed from non-protein-encoding genes. See review of Carrington *et al.* (2003). The precursors are processed by cellular proteins to generate the short double-stranded miRNA. One of the miRNA strands is incorporated into a complex of proteins and miRNA called the RNA-induced silencing complex (RISC). The miRNA guides the RISC complex to a target mRNA, which is then cleaved or translationally silenced, depending on the degree of sequence complementarity of the miRNA to its target mRNA (Bagga *et al*., 2005; Lim *et al*., 2005). Recent studies have shown that expression levels of numerous miRNAs are associated specifically with various cancers (reviewed in Esquela-Kerscher and Slack, 2006; Calin and Croce, 2006). miRNAs have also been implicated in regulating cell growth, cell proliferation, and cell and tissue differentiation - cellular processes that are associated with the development of cancer. The present invention advances the current art for cancer staging by describing the use of novel miRNA markers and combinations of miRNA markers for the detection of specific cancer metastases to lymph nodes.

### SUMMARY OF THE INVENTION

The invention is defined by the claims. Aspects of the present disclosure provide additional prognostic and/or diagnostics methods by identifying miRNAs that are differentially expressed or mis-regulated in various states of diseased, normal, cancerous, and/or abnormal tissues, including but not limited to lymph node (LN), cervix, skin, colon, breast, bladder, esophagus, liver, ovary, prostate, lung, pancreas, thyroid, kidney, stomach, testicle, uterus, spleen, tongue, brain, thymus, trachea and/or small intestine. As refered to herein, a tissue is a biological tissue. Biological tissue is typically defined as a collection of interconnected cells that perform a similar function within an organism or collectively form an organ. One of skill in the art can readily determine what part of the body or organ a particular tissue designation refers to. In certain apsects, differential miRNA expression is initially determined by comparing miRNA expression between a normal tissue and cancer negative lymph node (LNneg), a normal tissue and a cancer positive lymph node (LNpos), a cancerous tissue and LNneg and/or LNpos, LNneg and LNpos. In further aspects, the methods of the invention is used to identify the presence or absence of ectopic cells from other organs or tissues, and/or metastatic cancer cells present in lymph nodes (LN), which are detectable by the described methods. Lymph nodes are components of the lymphatic system. They are nodes that typically contain white blood cells and are found throughout the body functioning as filters or traps for foreign particles. Further, the disclosure describes a method for diagnosing diseased, normal, cancerous, abnormal tissues and/or dissemination of cancer cells based on determining levels (increased or decreased) of selected miRNAs in patient-derived samples, particularly lymph nodes (e.g., sentinel, proximal, and/or distal LN). Samples obtained and/or analyzed from patients, including but not limited to patients that have had, have or are suspected of having a cancer of the cervix, skin, colon, breast, bladder, esophagus, liver, ovary, prostate, lung, pancreas, thyroid, kidney, stomach, testicle, uterus, spleen, tongue, brain, thymus, trachea and/or small intestine or other hyperproliferative or cancerous condition.

In some aspects, it may be useful to know whether a cell expresses a particular miRNA or set of miRNAs endogenously or whether such expression is affected under particular conditions or when it is in a particular disease state. Thus, in some aspects of the invention, methods include assaying one or more cell or sample containing a cell for the presence of one or more miRNA. Consequently, in some aspects, methods include a step of generating a miRNA profile for one or more samples. The term "miRNA profile" refers to a set of data regarding the expression pattern for a plurality of miRNAs in a sample(s); it is contemplated that the miRNA profile can be obtained, using for example, nucleic acid amplification or hybridization techniques well known to one of ordinary skill in the art.

In some aspects of the invention, a miRNA profile is generated by steps that include: (a) labeling miRNA in the sample; (b) hybridizing miRNA to a number of probes, or amplifying a number of miRNA, and (c) determining miRNA hybridization to the probes or detecting miRNA amplification products, wherein a miRNA profile is generated. See U.S. Patent No. 20080026951 and U.S. Patent No. 20070161004.

Methods of the invention involve prognosing and/or diagnosing a patient based on a miRNA expression profile in lymph node(s). In certain embodiments, the elevation or reduction in the level of expression of a particular miRNA or set of miRNA in a sample is correlated with a disease or normal state compared to the expression level of that miRNA or set of miRNA in a normal tissue reference or a diseased tissue reference. This correlation allows for the identification of non-lymphoid cells in lymph nodes and enhances or provides prognostic and/or diagnostic methods. It is specifically contemplated that miRNA profiles for patients having or suspected of having a particular disease or condition such as cervix, skin, colon, breast, bladder, esophagus, liver, ovary, prostate, lung, pancreas, thyroid, kidney, stomach, testicle, uterus, spleen, tongue, brain, thymus, trachea and/or small intestine cancer can be generated by evaluating any of the sets or subsets of the miRNAs discussed in this application. The miRNA profile that is generated from the patient will be one that provides prognostic, diagnostic or staging information regarding the particular disease or condition. In many embodiments, the miRNA profile is generated using miRNA hybridization or amplification, (e.g., array hybridization or RT-PCR). In certain aspects, a miRNA profile can be used in conjunction with other diagnostic tests, such as protein levels or mRNA expression levels.

A miRNA or probe set comprising or identifying a segment of a corresponding miRNA can include all or part of 1, 2, 3, 4, 5, 6, 7, 8, 9, 10, 11, 12 ,13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 125, 150, 175, 200, 250, 300, 350, 400, 500 600, 700, 800, 900, 1,000 miRNAs, including any integer or range derivable there between, of Table 4, 5, 6, 10, 11, 12, 13, 14, 16, 17, 18, 19, 20, 21, 22, 23, 24, 25, 25, 27, 28, 29, 30, 31, 32, 33, 34, 35, 36, 37, 39, 40, 41, 43, and/or 44.

A sample may be taken from a patient having or suspected of having a disease or pathological condition. In certain aspects, the sample can be, but is not limited to tissue (e.g., biopsy, particularly fine needle biopsy), blood, serum, plasma, fluids (sputum, secretions, etc.) or lymph nodes (such as sentinel, proximal to a disease site, or distal from a disease site). The sample can be fresh, frozen, fixed (*e.g.,* formalin fixed), or embedded *(e.g.,* paraffin embedded). In a particular aspect, the sample can be a biopsy sample, or a lymph node sample.

In certain aspects of the invention a plurality of miRNAs have been identified that can be used to evaluate LN for the presence of a number of different non-lymphoid tissue or cell types. This pandiagnostic or panprognostic set of miRNAs include miRNA that are typically differentially expressed between normal tissues and normal lymph node. These miRNA include one or more miRNA selected from Table 37.

It is contemplated that various subsets of the miRNA listed herein can be used to the exclusion of other subsets of miRNA described herein.

miRNAs differentially expressed between cervix and LNneg tissue include one or more of miRNA selected from Table 4.

miRNAs differentially expressed between LNpos and LNneg tissue in cervical cancer patients include one or more miRNA selected from Table 5.

miRNAs differentially expressed in both cervix versus LNneg and LNpos versus LNneg include one or more miRNA selected from Table 6.

miRNAs differentially expressed between melanoma and LNneg tissue include one or more miRNA selected from Table 10.

miRNAs differentially expressed between LNpos and LNneg tissue from melanoma patients include one or more miRNA selected from Table 11 and/or 12.

miRNAs differentially expressed between normal colon and LNneg include one or more miRNA selected from Table 16 and/or 17.

miRNAs differentially expressed between normal breast and LNneg include one or more miRNA selected from Table 18.

miRNAs differentially expressed between normal bladder and LNneg include one or more miRNA selected from Table 19.

miRNAs differentially expressed between normal esophagus and LNneg include one or more miRNA selected from Table 20.

miRNAs differentially expressed between normal liver and LNneg include one or more miRNA selected from Table 21.

miRNAs differentially expressed between normal ovary and LNneg include one or more miRNA selected from Table 22.

miRNAs differentially expressed between normal prostate and LNneg include one or more miRNA selected from Table 23.

miRNAs differentially expressed between normal lung and LNneg include one or more miRNA selected from Table 24.

miRNAs differentially expressed between normal pancreas and LNneg include one or more miRNA selected from Table 25.

miRNAs differentially expressed between normal thyroid and LNneg include one or more miRNA selected from Table 26.

miRNAs differentially expressed between normal kidney and LNneg include one or more miRNA selected from Table 27.

miRNAs differentially expressed between normal stomach and LNneg include one or more miRNA selected from Table 28.

miRNAs differentially expressed between normal testicle and LNneg include one or more miRNA selected from Table 29.

miRNAs differentially expressed between normal uterus and LNneg include one or more miRNA selected from Table 30.

miRNAs differentially expressed between normal spleen and LNneg include one or more miRNA selected from Table 31.

miRNAs differentially expressed between normal tongue and LNneg include one or more miRNA selected from Table 32.

miRNAs differentially expressed between normal brain and LNneg include one or more miRNA selected from Table 33.

miRNAs differentially expressed between normal thymus and LNneg include one or more miRNA selected from Table 34.

miRNAs differentially expressed between normal trachea and LNneg include one or more miRNA selected from Table 35.

miRNAs differentially expressed between normal small intestine and LNneg include one or more miRNA selected from Table 36.

miRNAs differentially expressed between cancer-positive LN and/or cancer-negative LN from breast cancer patients include one or more miRNA selected from Table 39.

miRNAs differentially expressed between normal breast and LNneg include one or more miRNA selected from Table 40.

miRNAs differentially expressed between normal breast tissue and cancer-negative axillary LN, and between LNneg and LNpos axillary LN include one or more miRNA selected from Table 41.

miRNAs differentially expressed between LNneg and LNpos from breast cancer patients include one or more miRNA selected from Table 43.

miRNAs differentially expressed between LNpos and LNneg from cervical cancer patients include one or more miRNA selected from Table 44.

miRNA sequences can be found in the miRBase database (release 12.0 (Sept. 2008)). miRBase is accessible via the world wide web at microma.sanger.ac.uk, the content of release 12.0, as it relates to the miRNA identified in the tables.

The methods can further comprise one or more of steps including: (a) obtaining a sample from the patient, (b) isolating nucleic acids from the sample, (c) labeling the nucleic acids isolated from the sample, (d) hybridizing the labeled nucleic acids to one or more probes, and/or evaluating or assessing the level of expression of miRNA of interest. Once evaluation or assessment has been completed then a prognosis, diagnosis, stage of disease or other report can be generated and transmitted to a person of interest, *e.g.,* a physician.

Certain aspects of the invention include determining expression of one or more miRNA by using an amplification assay or a hybridization assay, a variety of which are well known to one of ordinary skill in the art. In certain aspects, an amplification assay can be a quantitative amplification assay, such as quantitative RT-PCR or the like. In still further aspects, a hybridization assay can include array hybridization assays or solution hybridization assays.

Aspects of the invention can be used to diagnose or assess a patient's condition. For example, the methods can be used to screen for a pathological condition, assess prognosis of a pathological condition, stage a pathological condition, or assess response of a pathological condition to therapy.

The present disclosure also concerns kits containing compositions of the invention or compositions to implement methods of the invention. In some embodiments, kits can be used to evaluate one or more miRNA molecules. In certain aspects, a kit contains, contains at least or contains at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 100, 500, 1,000 or more miRNA probes, synthetic miRNA molecules or miRNA inhibitors, or any value or range and combination derivable therein. In some aspects, there are kits for evaluating miRNA activity in a cell.

Kits may comprise components, which may be individually packaged or placed in a container, such as a tube, bottle, vial, syringe, or other suitable container means.

Individual components may also be provided in a kit in concentrated amounts; in some aspects, a component is provided individually in the same concentration as it would be in a solution with other components. Concentrations of components may be provided as 1x, 2x, 5x, 10x, or 20x or more.

Kits for using miRNA probes, synthetic miRNAs, nonsynthetic miRNAs, and/or miRNA inhibitors of the invention for prognostic or diagnostic applications are included as part of the disclosure. Specifically contemplated are any such molecules corresponding to any miRNA identified herein.

In certain aspects, negative and/or positive control synthetic miRNAs and/or miRNA inhibitors are included in some kit aspects. The control molecules can be used to verify transfection efficiency and/or control for transfection-induced changes in cells.

It is contemplated that any method or composition described herein can be implemented with respect to any other method or composition described herein and that different aspects may be combined. It is specifically contemplated that any methods and compositions discussed herein with respect to miRNA molecules or miRNA may be implemented with respect to synthetic miRNAs to the extent the synthetic miRNA is exposed to the proper conditions to allow it to become a mature miRNA under physiological circumstances.

Any aspect of the disclosure involving specific miRNAs by name is contemplated also to cover embodiments involving miRNAs whose sequences are at least 80, 81, 82, 83, 84, 85, 86, 87, 88, 89, 90, 91, 92, 93, 94, 95, 96, 97, 98, 99% identical to the mature sequence of the specified miRNA.

Aspects of the invention include kits for analysis of a pathological sample by assessing miRNA profile for a sample comprising, in suitable container means, two or more miRNA probes, wherein the miRNA probes detect one or more of the miRNA identified herein. The kit can further comprise reagents for labeling miRNA in the sample. The kit may also include labeling reagents, including at least one of amine-modified nucleotide, poly(A) polymerase, and poly(A) polymerase buffer. Labeling reagents can include an amine-reactive dye.

Other embodiments of the invention are discussed throughout this application. Any embodiment discussed with respect to one aspect of the invention applies to other aspects of the invention as well and vice versa. The embodiments in the Example section are understood to be embodiments of the invention that are applicable to all aspects of the invention.

The terms "inhibiting," "reducing," or "prevention," or any variation of these terms, when used in the claims and/or the specification includes any measurable decrease or complete inhibition to achieve a desired result.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed herein can be implemented with respect to any method or composition of the invention, and vice versa. Furthermore, compositions and kits of the invention can be used to achieve methods of the invention.

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

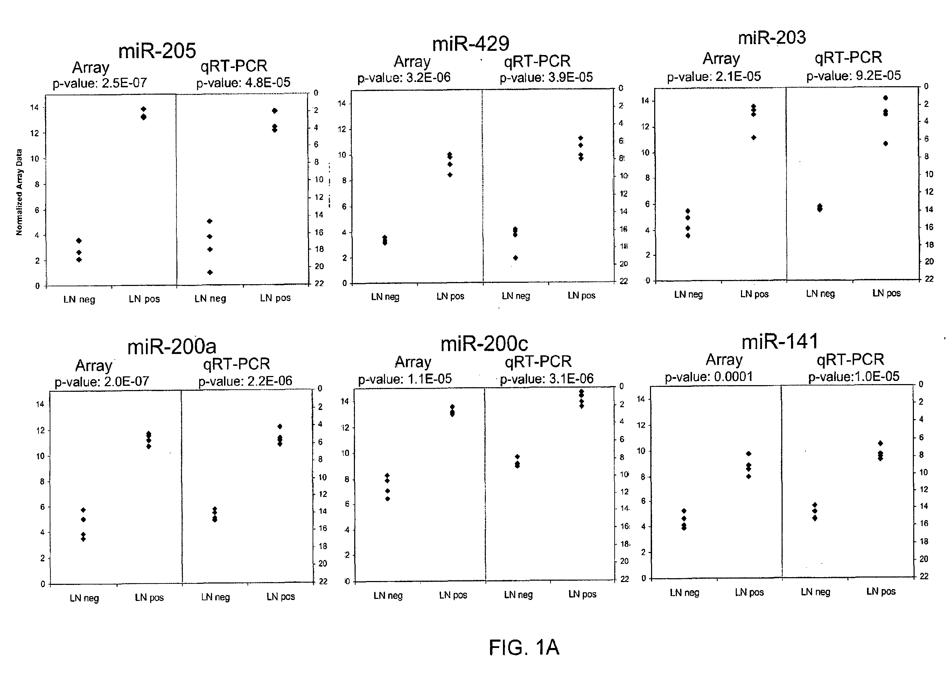

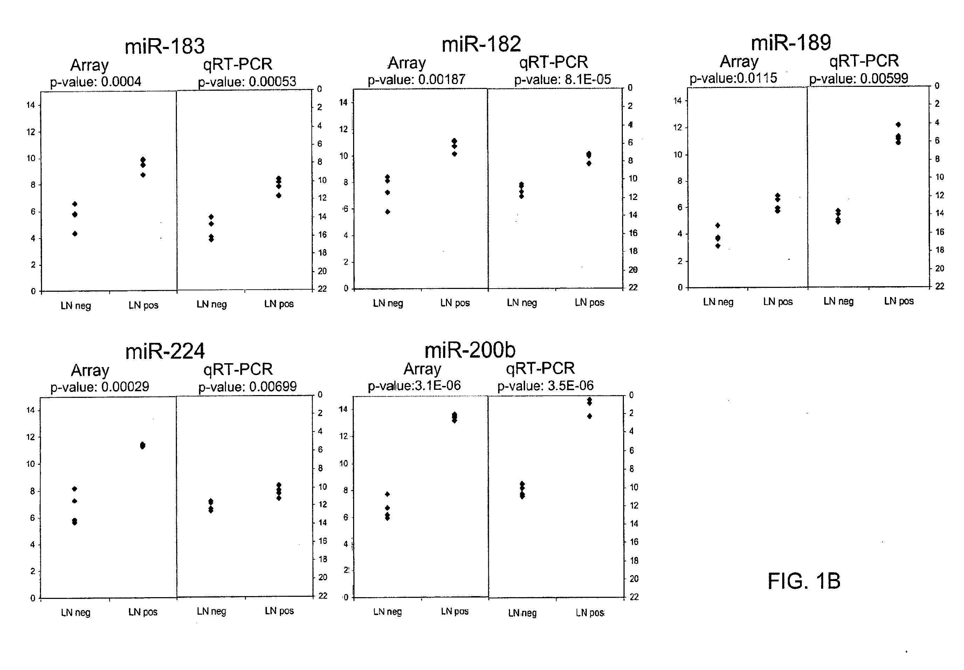

**FIGs. 1A-1B** Differences in expression levels of eleven miRNAs, between LNpos1-4 and LNneg1-4 samples from cervical cancer patients, measured by qRT-PCR and microarray quantification. The graphs show the individual normalized expression levels determined by array or qRT-PCR and associated *p*-values (pair-wise *t*-test).

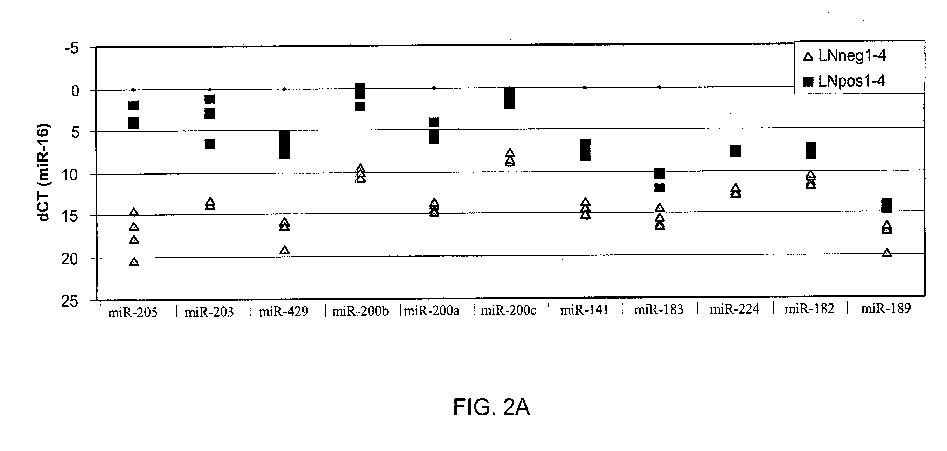

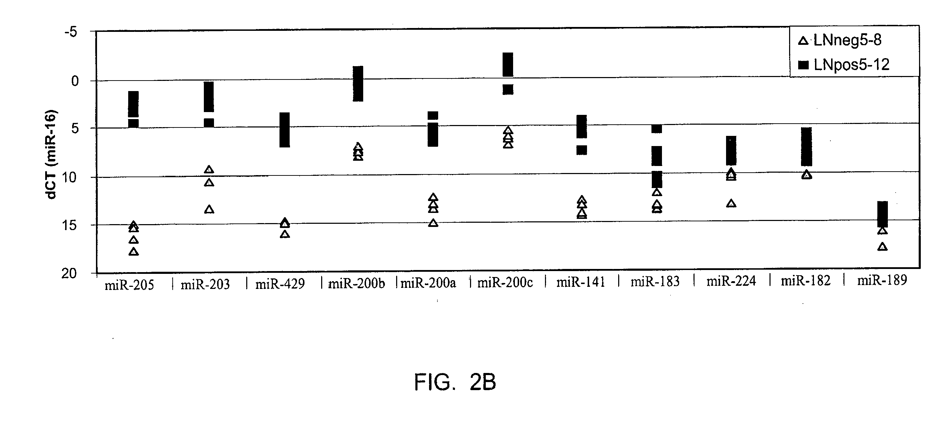

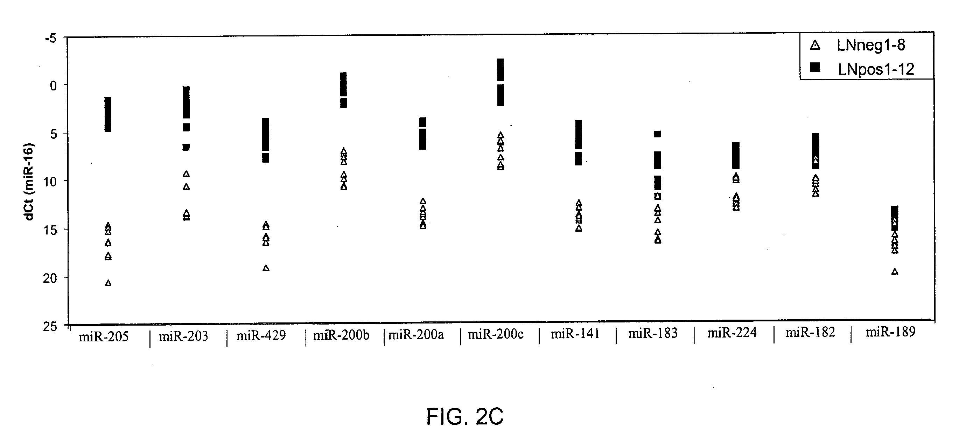

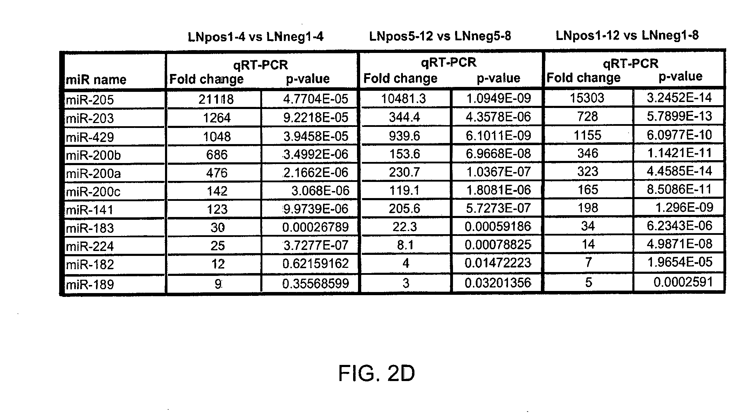

**FIGs**. **2A-2D** qRT-PCR quantification of eleven microRNAs in two independent sets of cancer-positive (LNpos) and cancer-negative (LNneg) lymph nodes obtained from cervical cancer patients. (FIG. 2A) Samples LNpos1-4 and LNneg1-4. FIG. 2B. Samples LNpos5-12 and LNneg5-8. (FIG. 2C) Combined qRT-PCR data for samples LNpos1-12 and LNneg1-8. (FIG. 2D) Summary table of the qRT-PCR data with fold changes and *p*-values for LNpos1-4/LNneg1-4, LNpos5-12/LNneg5-8, and LNpos1-12 and LNneg1-8.

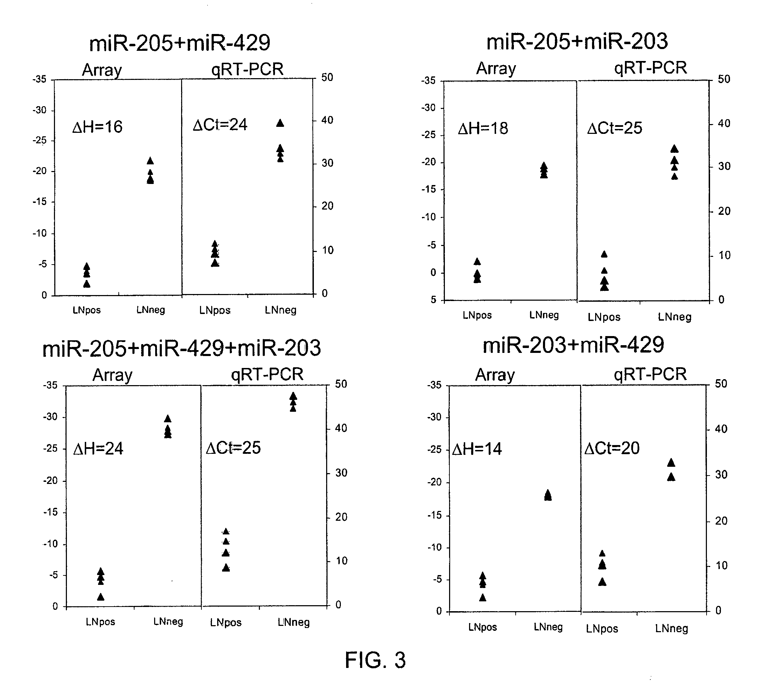

**FIG. 3** Differences in expression levels of various miRNA combinations, between LNpos1-4 and LNneg1-4 samples from cervical cancer patients, measured by qRT-PCR and microarray quantification. For qRT-PCR assays, the difference in Ct between the combined normalized Ct values in each sample group is indicated on each graph. For array quantification assays, the difference in the combined ΔH values for each sample group is indicated on each graph.

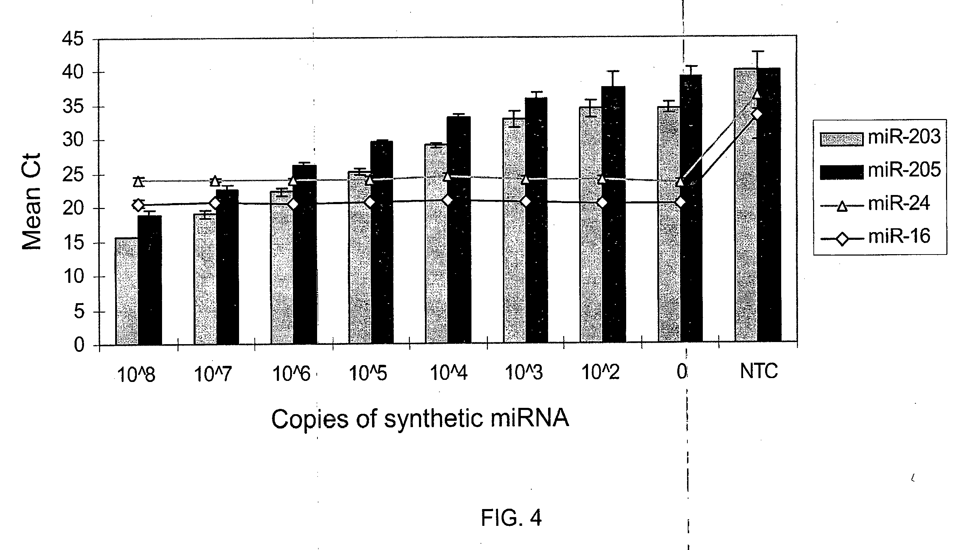

**FIG. 4** Sensitivity of detection of synthetic miR-205 and miR-203 in a background of total RNA purified from a cervical cancer-negative inguinal lymph node (LNneg). The number of copies of each individual synthetic miRNA added to a background of LNneg total RNA are indicated on the X axis. Mean Ct values indicated on the Y axis represent the mean of duplicated qRT-PCR reactions. Error bars in the figures represent the values for each of the two reactions. Both miR-16 and miR-24 were used as internal controls.

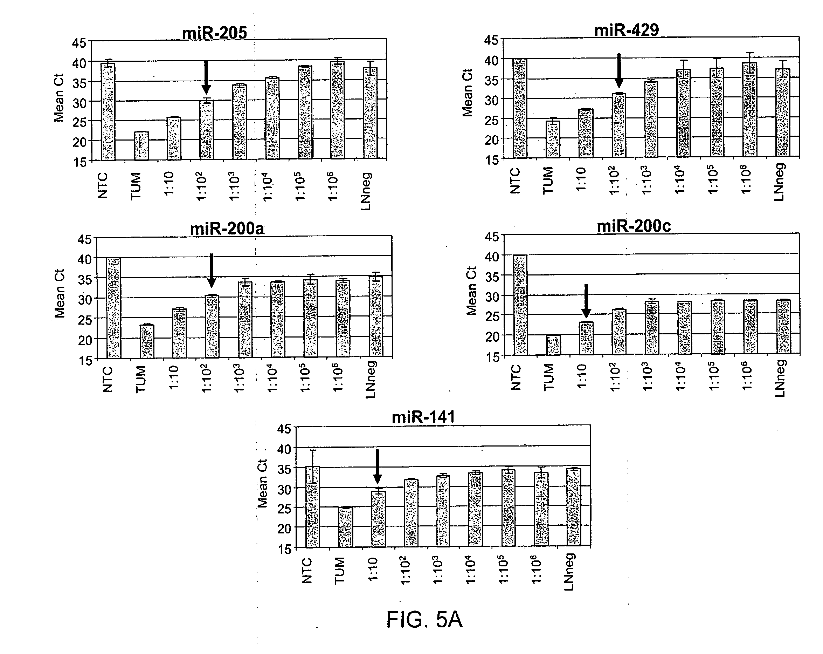

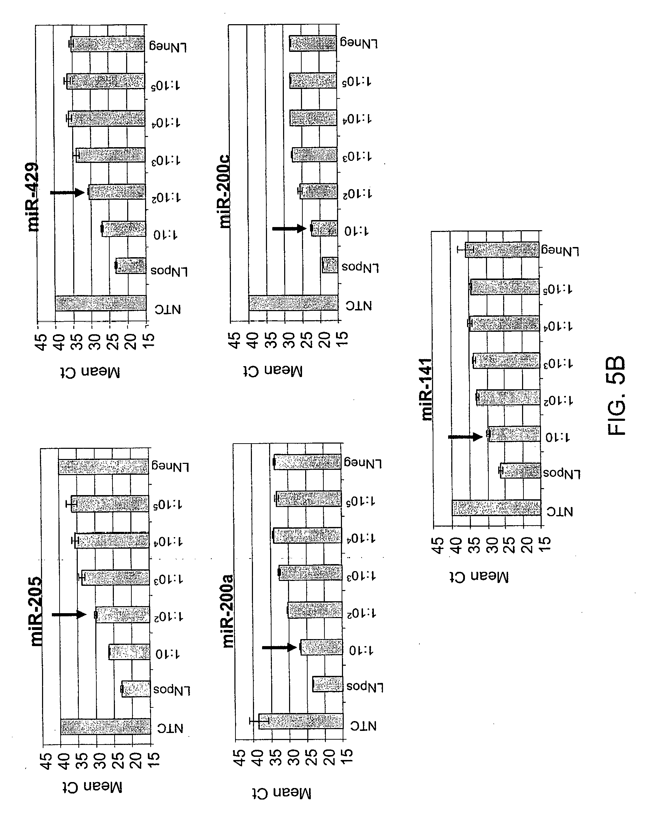

**FIGs. 5A-5B** (FIG. 5A) Sensitivity of detection of five miRNAs (miR-205, -429, -200a, -200c, and -141) determined by qRT-PCR, following dilution of total RNA from a cervical tumor (TUM) into total RNA from a cancer-negative inguinal lymph node (LNneg). Arrows indicate limits of detection. Mean Ct, mean of duplicate qRT-PCR reactions. NTC, no template control (i.e., no cervical tumor RNA added). (FIG. 5B) Sensitivity of detection of five miRNAs (miR-205, -429, -200a, -200c, and -141) determined by qRT-PCR, following dilution of total RNA from a cancer-positive lymph node (LNpos) into total RNA from a cancer-negative lymph node (LNneg). Arrows indicate limits of detection. Mean Ct, mean of duplicate qRT-PCR reactions. NTC, no template control (i.e., no LNpos RNA added).

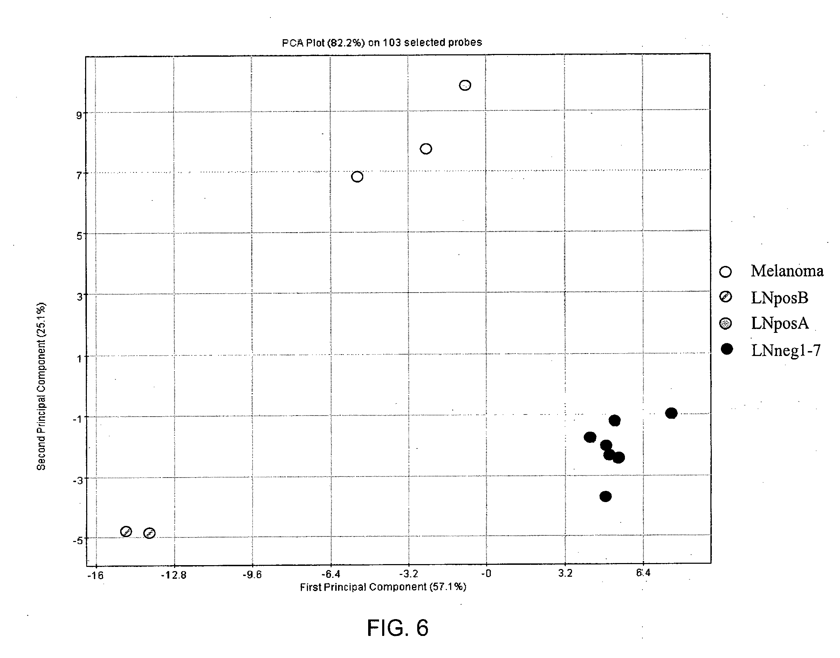

**FIG. 6** Principal component analysis (PCA) of miRNAs differentially expressed in melanoma, cancer-positive lymph node group A (LNposA - LNpos1A), cancer-positive lymph node group B (LNposB - LNpos2B, LNpos3B), and cancer-negative lymph node (LNneg1-7) samples from melanoma patients, as determined by ANOVA.

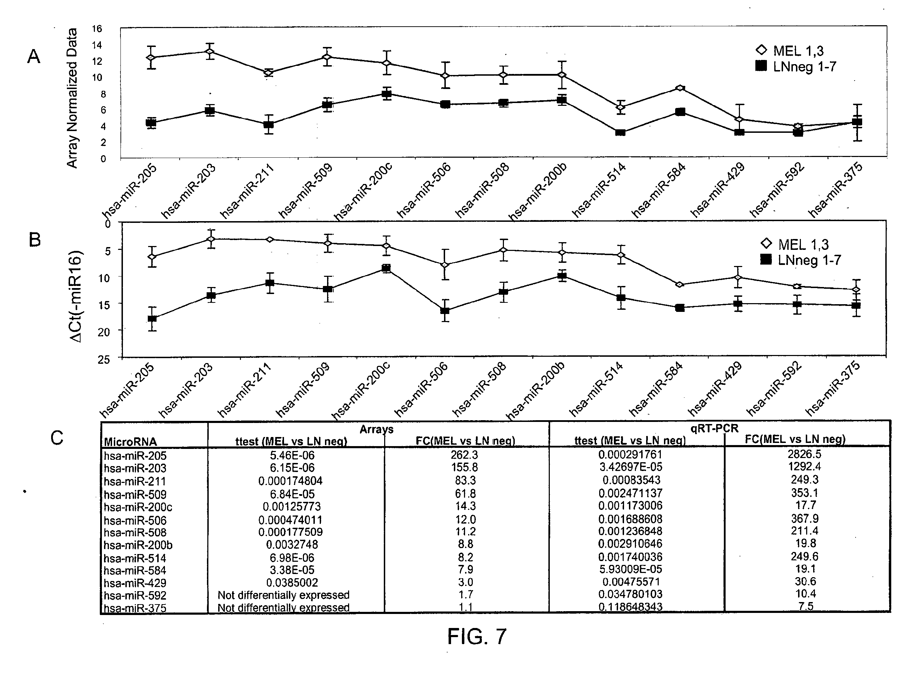

**FIGs. 7A-7C** Comparison of array and qRT-PCR data for selected miRNAs differentially expressed between melanoma (MEL1, MEL3) and cancer-negative lymph nodes (LNneg1-7). (FIG. 7A) Normalized array data for indicated miRNAs. (FIG. 7B) qRT-PCR data obtained with primer sets for the indicated miRNAs, normalized to miR-16 expression level for each samples (miRNACt - miR-16Ct). (FIG. 7C) Associated p-values and fold change for each miRNA as determined by microarray expression analysis or qRT-PCR.

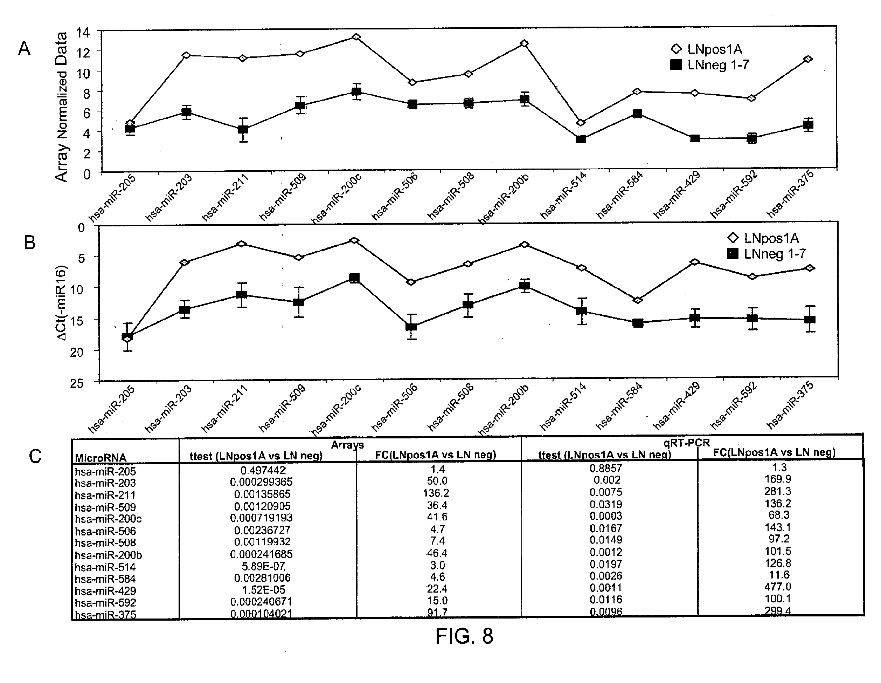

**FIGs. 8A-8C** Comparison of array and qRT-PCR data for selected miRNAs differentially expressed between the cancer-positive group A (LNpos1A) lymph node and cancer-negative lymph nodes (LNneg) from melanoma patients. (FIG 8A) Normalized array data for indicated miRNAs. (FIG. 8B) qRT-PCR data obtained with primer sets for the indicated miRNAs, normalized to miR-16 expression level for each samples (miRNACt-miR-16Ct). (FIG. 8C) Associated p-values and fold change for each miRNA as determined by microarray expression analysis or qRT-PCR.

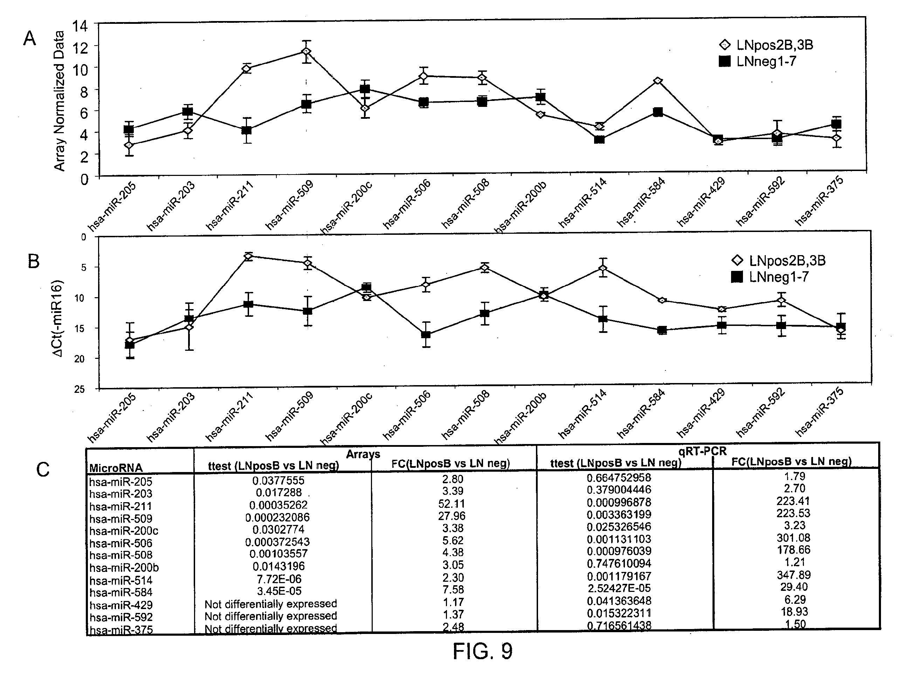

**FIGs. 9A-9C** Comparison of array and qRT-PCR data for selected miRNAs differentially expressed between the cancer-positive group B (LNpos2B, 3B) lymph nodes and cancer-negative lymph nodes (LNneg1-7) from melanoma patients. (FIG. 9A) Normalized array data for indicated miRNAs. (FIG. 9B) qRT-PCR data obtained with primer sets for the indicated miRNAs, normalized to miR-16 expression level for each sample (miRNACt - miR-16Ct). (FIG. 9C) Associated p-values and fold change for each miRNA as determined by microarray expression analysis or qRT-PCR.

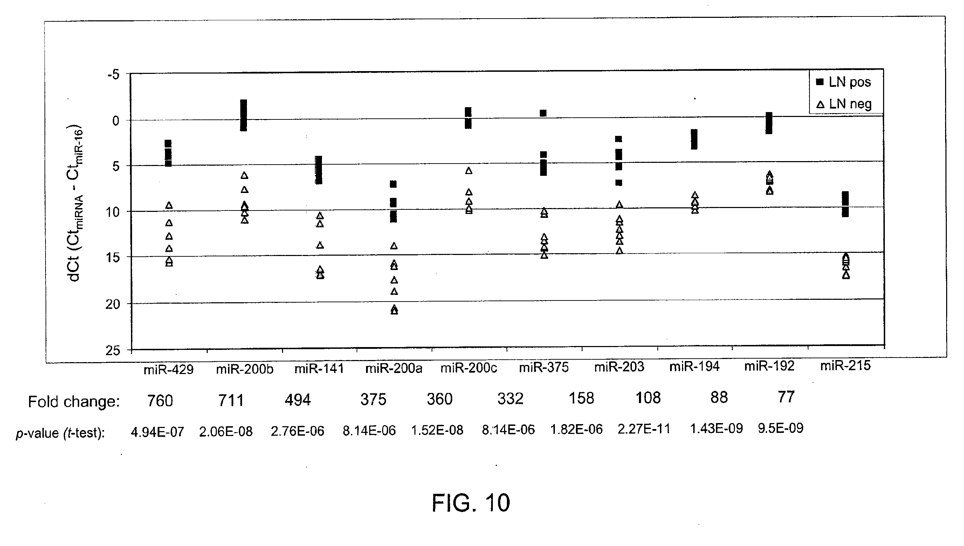

**FIG. 10** Differences in expression levels of ten miRNAs, between cancer-positive lymph nodes from colon cancer patients (LNpos) and cancer-negative lymph nodes (LNneg), measured by qRT-PCR The graph shows the individual normalized expression levels (dCt=miRNACt - miR-16Ct). Differences in the normalized mean expression values between the two sample sets were transformed to a general fold change (FC) by exponentiation base 2, as indicated below the graph with the associated *p*-values (pair-wise *t-*test).

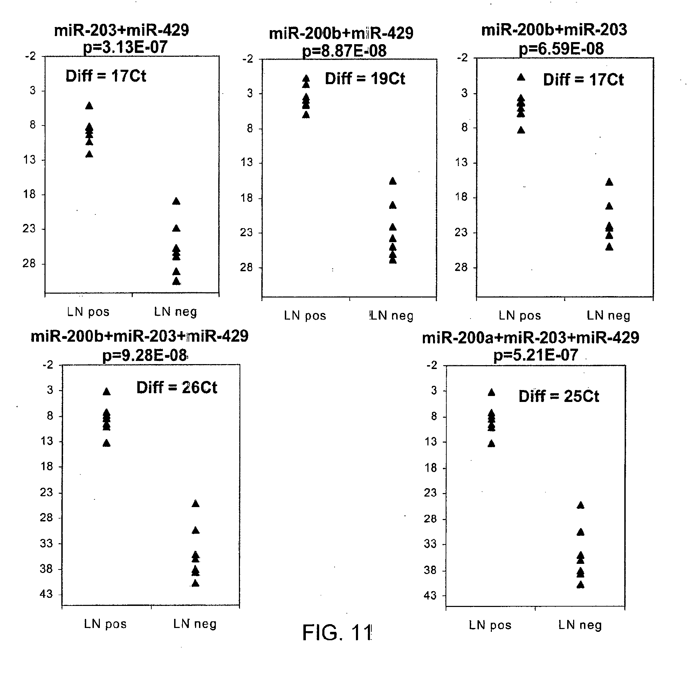

**FIG. 11** Differences in expression levels of various miRNA combinations, between cancer-positive (LNpos) lymph nodes from colon cancer patients and cancer-negative lymph nodes (LNneg), measured by qRT-PCR. The difference in Ct between the combined mean normalized Ct values in each sample group and the associated p-value are indicated on each graph. miR-16 was used as the normalizer.

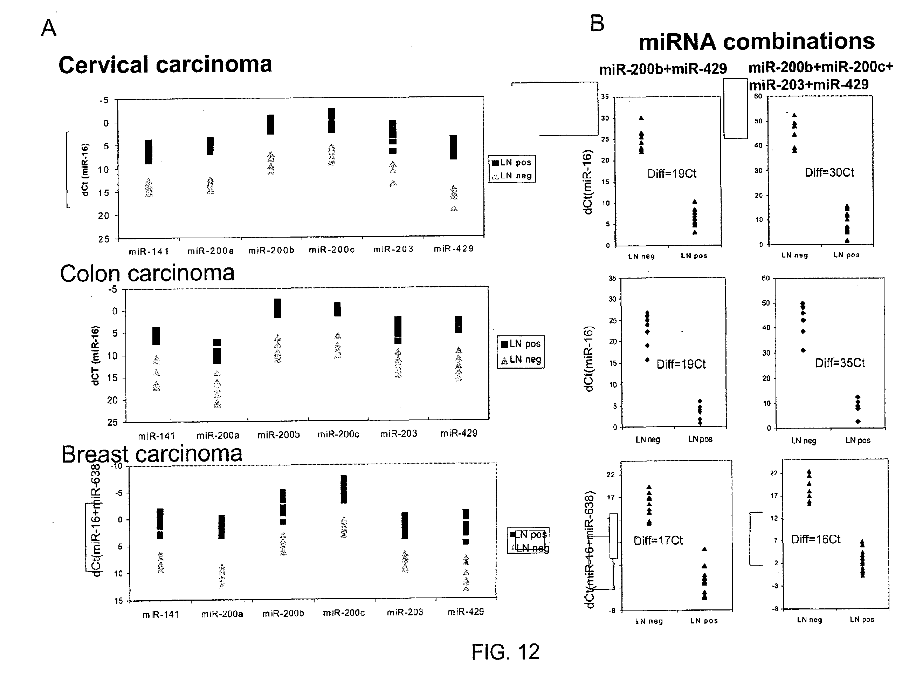

**FIGs. 12A-12B** Six miRNAs (miR-429, -141, -203, -200a, -200b, and -200c) can distinguish positive and negative lymph nodes from patients with either cervical colon or breast cancer. The graphs show (FIG. 12A) the individual normalized expression levels determined by qRT-PCR for the indicated miRNAs for each positive and negative lymph node analyzed in each cancer group. (FIG. 12B) Differences in expression levels of two combination sets of miRNAs between positive and negative lymph nodes from patients with cervical cancer, colon cancer and breast cancer, measured by qRT-PCR quantification. The miRNAs used in combinations are indicated above the graphs. The difference in Ct between the combined normalized Ct values in each sample group is indicated on each graph.

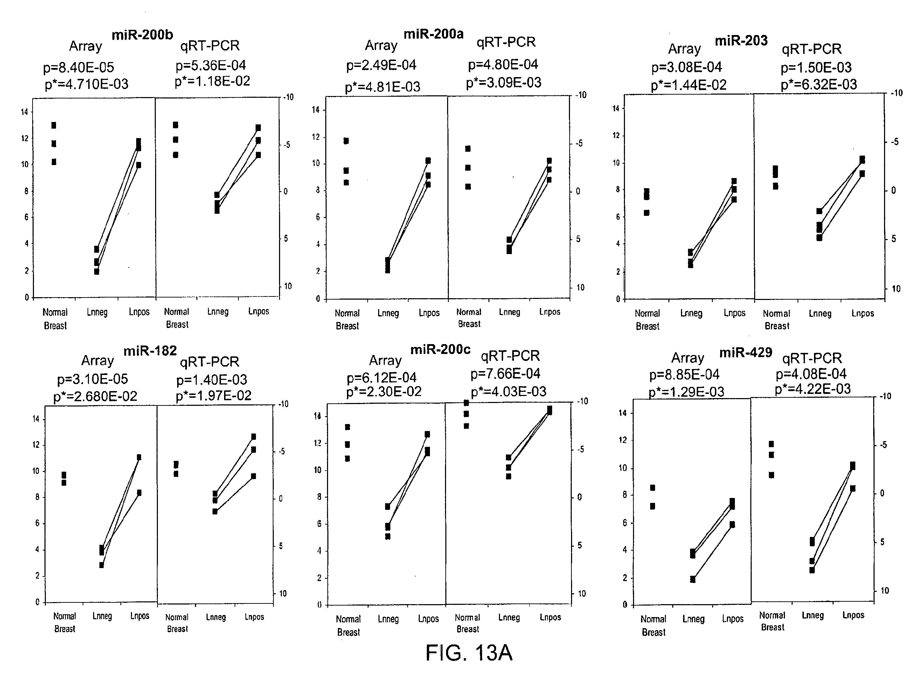

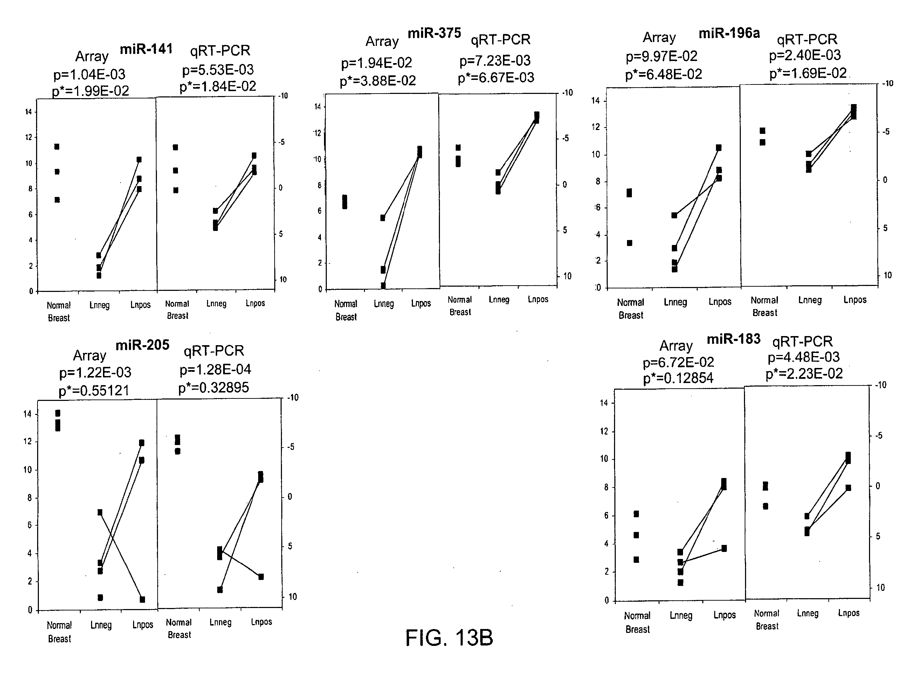

**FIGs. 13A-13B** Comparison of array and qRT-PCR data for selected miRNAs differentially expressed between normal breast (NBr) tissue and cancer-positive (LNpos) and cancer-negative (LNneg) axillary lymph nodes from ductal breast cancer patients. (FIG. 13A) Normalized array data for indicated miRNAs. (FIG. 13B) qRT-PCR data obtained using specific primer sets for each indicated miRNA, normalized to miR-16 expression level (miRNACt - miR-16Ct). Associated p-values (pair-wise-t-test) indicated on top of each graph represents the p-values for NBr and LNneg comparison (p) and the p-values for the comparison of the paired LNpos and LNneg samples (p*).

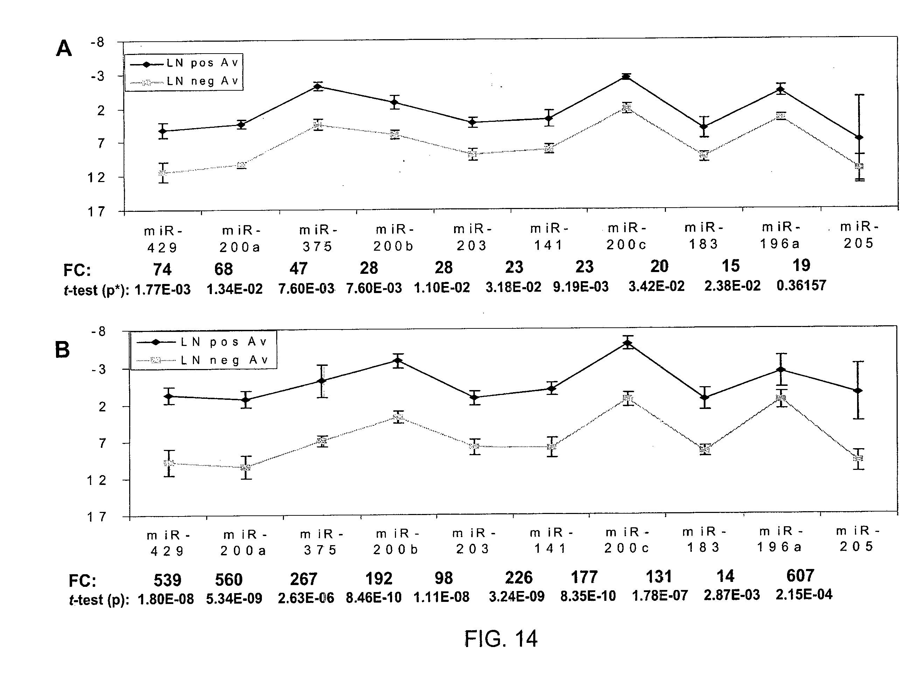

**FIGs. 14A-14B** Differences in mean expression levels of ten miRNAs, between cancer-positive (LNpos) and cancer-negative (LNneg) lymph nodes from breast cancer patients. Data for two independent sets of lymph nodes, represented by FIG. 14A (LNpos2-4 and LNneg1-4) and FIG. 14B (LNpos5-14 and LNneg5-9). Graphs show the mean expression levels of each miRNA after normalization to both miR-16 and miR-638 (dCt= CtmiRNA - 1/2(CtmiR16 + CtmiR-638)). Ct values indicated on the y-axis represent the mean of duplicate qRT-PCR reactions. Error bars represent the values for each of the two reactions. Differences in the normalized mean expression values between the LNpos and LNneg of each sample set were transformed to a general fold change (FC) by exponentiation base 2 as indicated below each graph. Associated *p*-values (pair-wise *t*-test) of the comparison between the paired LNpos and LNneg samples (FIG. 14A - p*) and between the independent and unmatched set of LNpos and LNneg samples (FIG. 14B - p) are indicated below each graph.

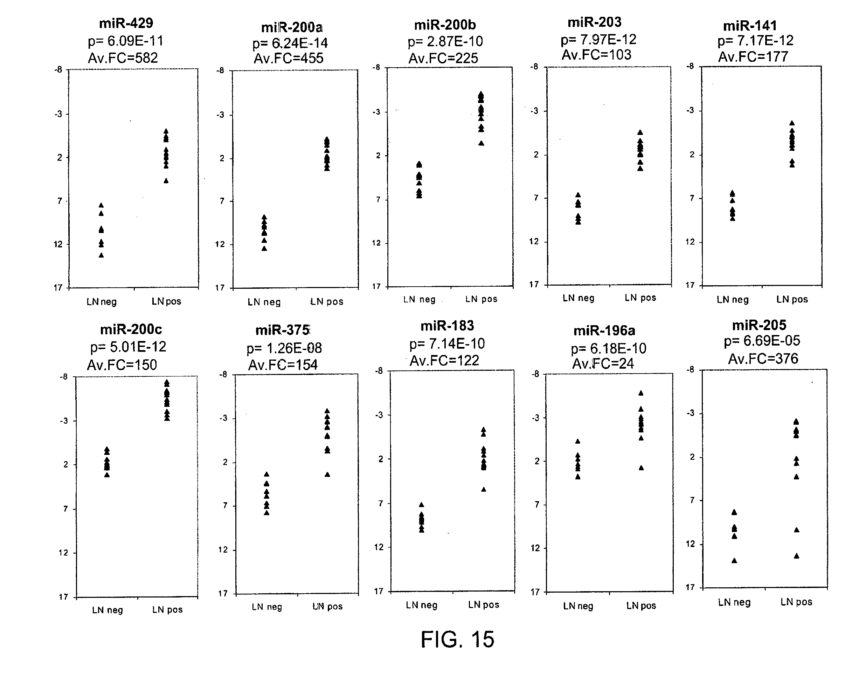

**FIG. 15** Combined qRT-PCR data of ten miRNAs for two independent sets of cancer-positive (LNpos2-14) and cancer-negative (LNneg1-9) lymph nodes from breast cancer patients. For each qRT-PCR assay, the difference in Ct between the combined normalized Ct values in each sample group was transformed to a general average fold change (Av.FC) by exponentiation base 2, as indicated on each graph, with associated p-values.

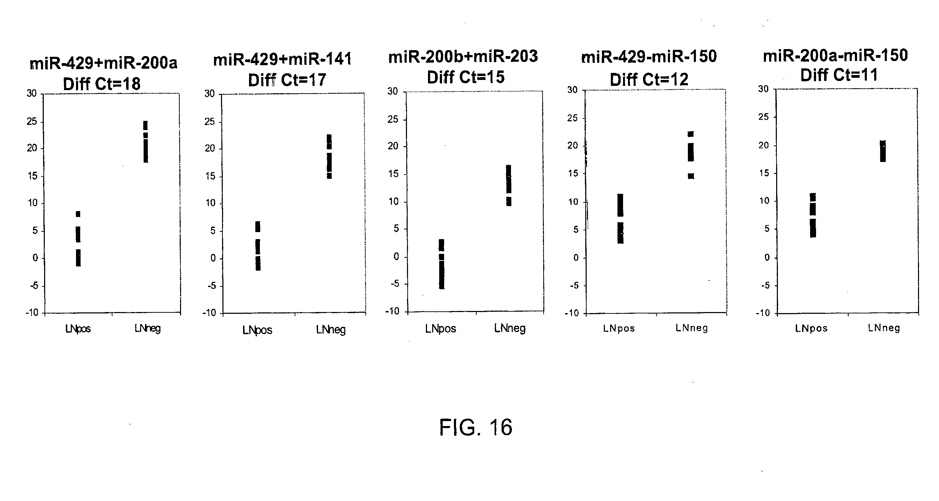

**FIG. 16** Differences in expression levels of various miRNA combinations, between LNpos2-14 and LNneg1-9) samples from breast cancer patients, as measured by qRT-PCR. The difference in Ct between the combined normalized Ct values in each sample group is indicated on the three first graphs. For the two last combinations, miR-429-miR-150 and miR-200a-miR-150, the difference in Ct between the two sample groups is expressed by the subtraction of the raw Ct values of each miRNA.

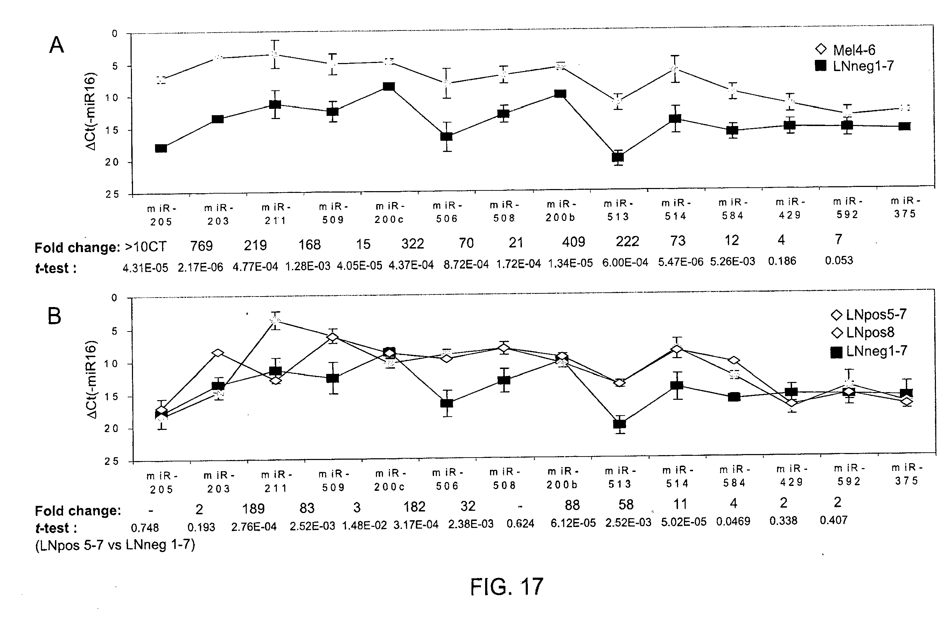

**FIGs**. **17A-17B** qRT-PCR quantification of 14 selected miRNAs in FFPE melanoma samples (MEL4-6) and FFPE cancer-positive lymph nodes (LNpos5-8) from melanoma patients compared to cancer-negative lymph nodes (LNneg1-7). (FIG. 17A) Comparison between MEL4-6 and LNneg1-7, as indicated on the graph. (FIG. 17B) Comparison between LNpos5-8 and LNneg1-7 samples, as indicated on the graph. Values represent the mean Ct of duplicated qRT-PCR reactions. miR-16 was used as a normalizer. Error bars in the figures represent the values for each of the two qRT-PCR reactions. For each miRNA, the associated fold change and *p*-value (student *t*-test) are indicated below each graph.

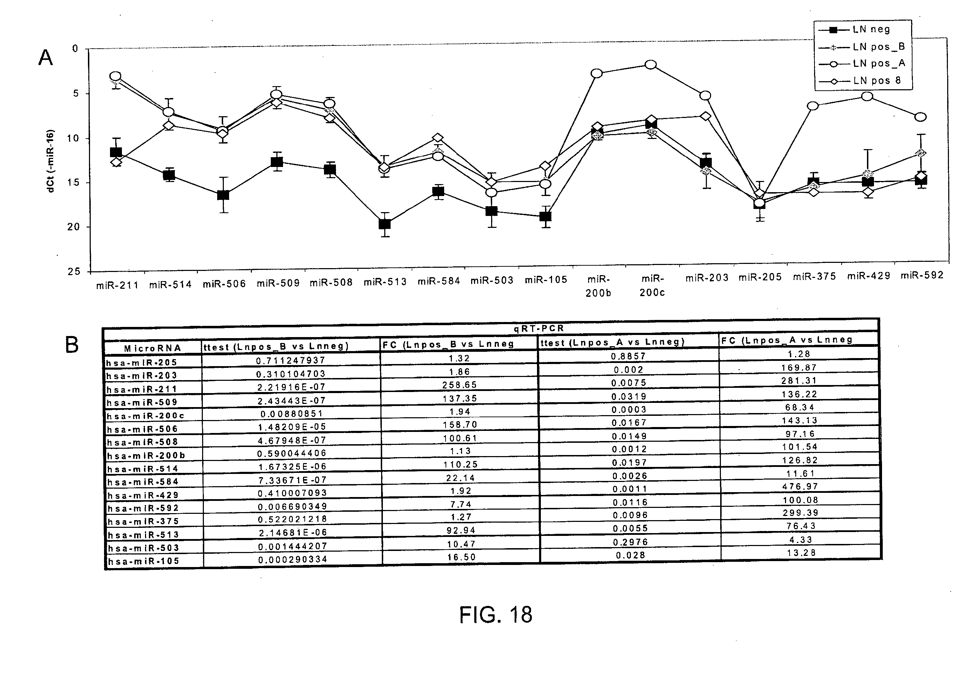

**FIGs**. **18A-18B** Differences in expression levels of fourteen miRNAs between cancer-positive (LNpos) and cancer-negative (LNneg) lymph nodes from melanoma patients. (FIG. 18A) The graph shows the qRT-PCR data obtained with primer sets specific for the indicated miRNAs, normalized to miR-16 expression level (miRNACt-miR-16Ct) in LNneg1-7, LNposA (LNpos1A), LNposB (LNpos2-7), and LNpos8, as indicated on the graph. (FIG. 18B) Summary table of the data with fold-changes and associated p-values for LNposA vs LNneg and LNposB vs LNneg.

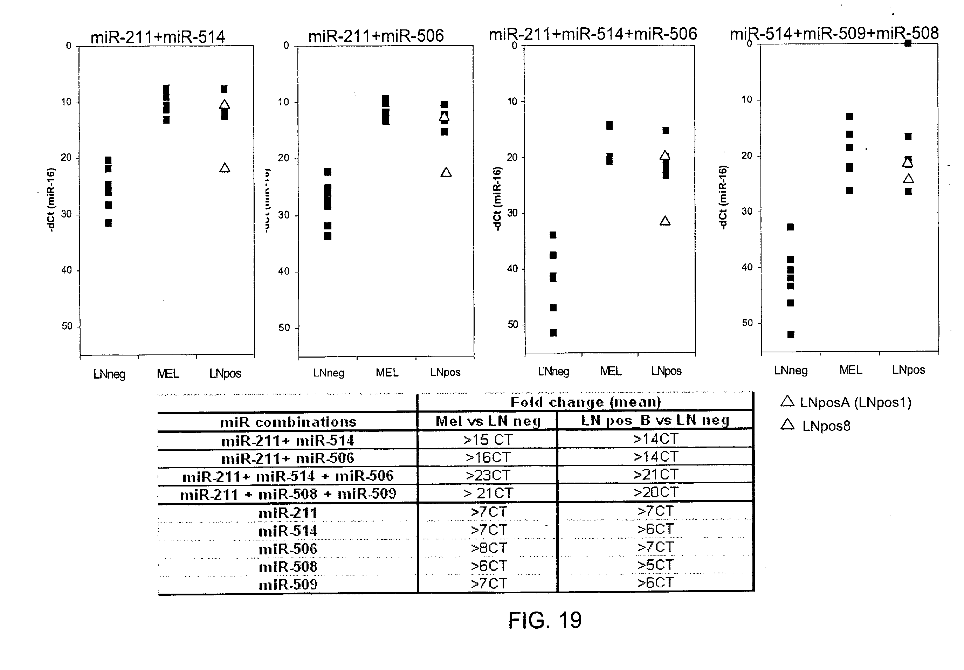

**FIGs. 19A-19B** Differences in expression levels of various miRNA combinations, between melanomas (MEL), cancer-negative (LNneg) lymph nodes, and cancer-positive (LNpos) lymph nodes. The miRNAs used in combinations are indicated above the graphs. (FIG. 19A) The graphs show the combined mean Ct values in each of LNneg, LNpos, and MEL samples, normalized to miR-16. (FIG. 19B) The table displays the fold changes observed between the mean Ct values of each sample group.

### DETAILED DESCRIPTION OF THE INVENTION

Aspects of the present disclosure are directed to compositions and methods relating to preparation and characterization of miRNAs, as well as to the use of miRNAs for therapeutic, prognostic, diagnostic, and staging applications, particularly those methods and compositions related to assessing and/or identifying a non-lymphoid cell in a lymph node tissue.

### 1. MIRNA MOLECULES

The term "miRNA" is used according to its ordinary and plain meaning and refers to a microRNA molecule found in eukaryotes that is involved in RNA-based gene regulation. See, *e.g.,* Carrington *et al*., 2003. The term will be used to refer to the single-stranded RNA molecule processed from a precursor. Individual miRNAs have been identified and sequenced in different organisms, and they have been given names. Names of miRNAs related to the present invention are provided herein. The sequences related to these miRNA are know and available through public resources such as scientfic publications, miRBase 12.0 and other nucleic acid databases. The methods and compositions should not be limited to miRNAs identified in the application, as they are provided as examples, not necessarily as limitations of the disclosure.

MicroRNA molecules ("miRNAs") are generally 21 to 22 nucleotides in length, though lengths of 16 and up to 35 nucleotides have been reported. The miRNAs are each processed from a longer precursor RNA molecule ("precursor miRNA"). Precursor miRNAs are transcribed from non-protein-encoding genes. The precursor miRNAs have two regions of complementarity that enables them to form a stem-loop- or fold-back-like structure, which is cleaved in animals by a ribonuclease III-like nuclease enzyme called Dicer. The processed miRNA is typically a portion of the stem.

The processed miRNA (also referred to as "mature miRNA") become part of a large complex to down-regulate a particular target gene. Examples of animal miRNAs include those that imperfectly basepair with the target, which halts translation (Olsen *et al*., 1999; Seggerson *et al*., 2002). siRNA molecules also are processed by Dicer, but from a long, double-stranded RNA molecule. siRNAs are not naturally found in animal cells, but they can direct the sequence-specific cleavage of an mRNA target through a RNA-induced silencing complex (RISC) (Denli *et al.,* 2003).

### A. Nucleic Acids

The present disclosure concerns miRNAs that can be labeled, used in array analysis, or employed in diagnostic, therapeutic, prognostic, and/or staging applications, particularly those related to pathological and/or cancerous conditions. The RNA may have been endogenously produced by a cell, or been synthesized or produced chemically or recombinantly. They may be isolated and/or purified. The term "miRNA," unless otherwise indicated, refers to the processed RNA, after it has been cleaved from its precursor. Mature and precursor sequences of miRNA can typically be located in Sanger miRBase™ accessible on the internet at microrna.sanger.ac.uk/ or in various scientific publications. The name of the miRNA is often abbreviated and referred to without a hsa-, mmu-, or rno- prefix and will be understood as such, depending on the context. Unless otherwise indicated, miRNAs referred to in the application are human sequences identified as miR-X or let-X, where X is a number and/or letter.

It is understood that a "synthetic nucleic acid" of the invention means that the nucleic acid does not have a chemical structure or sequence of a naturally occurring nucleic acid. Consequently, it will be understood that the term "synthetic miRNA" refers to a "synthetic nucleic acid" that functions in a cell or under physiological conditions as a naturally occurring miRNA.

While many of the aspects of the disclosure involve synthetic miRNAs or synthetic nucleic acids, in some aspects of the disclosure, the nucleic acid molecule(s) need not be "synthetic." The term miRNA will generically refer to both non-synthetic (e.g., endogenous) miRNA and synthetic miRNA. In certain aspects, a non-synthetic miRNA employed in methods and compositions of the invention may have the entire sequence and structure of a naturally occurring miRNA precursor or the mature miRNA. For example, non-synthetic miRNAs used in methods and compositions of the disclosure may not have one or more modified nucleotides or nucleotide analogs. In these aspects, the non-synthetic miRNA may or may not be recombinantly produced. Any aspects discussed with respect to the use of synthetic miRNAs can be applied with respect to non-synthetic miRNAs, and vice versa.

It will be understood that the term "naturally occurring" refers to something found in an organism without any intervention by a person; it could refer to a naturally-occurring wildtype or mutant molecule. In some aspects a synthetic miRNA molecule does not have the sequence of a naturally occurring miRNA molecule. In other aspects, a synthetic miRNA molecule may have the sequence of a naturally occurring miRNA molecule, but the chemical structure of the molecule, particularly in the part unrelated specifically to the precise sequence (non-sequence chemical structure) differs from chemical structure of the naturally occurring miRNA molecule with that sequence. In some cases, the synthetic miRNA has both a sequence and non-sequence chemical structure that are not found in a naturally-occurring miRNA. Moreover, the sequence of the synthetic molecules will identify which miRNA is effectively being provided or inhibited; the endogenous miRNA will be referred to as the "corresponding miRNA." Corresponding miRNA sequences that can be used in the context of the invention include, but are not limited to, all or a portion of those sequences referred to herein, as well as any other miRNA sequence, miRNA precursor sequence, or any sequence complementary thereof. In some embodiments, the sequence is or is derived from or contains all or part of a sequence identified herein to target or identify or measure a particular miRNA (or set of miRNAs).

In certain experiments, a miRNA probe designated by a suffix "5P" or "3P" can be used. "5P" indicates that the mature miRNA derives from the 5' end of the precursor and a corresponding "3P" indicates that it derives from the 3' end of the precursor, as described on the miRBase™ website. Moreover, in some aspects, a miRNA probe is used that does not correspond to a known human miRNA. It is contemplated that these non-human miRNA probes may be used in aspects of the invention or that there may exist a human miRNA that is homologous to the non-human miRNA. While the disclosure is not limited to human miRNA, in certain aspects, miRNA from human cells or a human biological sample is evaluated. In other aspects, any mammalian cell, biological sample, or preparation thereof may be employed.

In some aspects of the invention, methods and compositions involving miRNA may concern miRNA and/or other nucleic acids. Nucleic acids may be, be at least, or be at most 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, or 10,000 nucleotides or more in length, including any range derivable therein, in length. Such lengths cover the lengths of processed miRNA, miRNA probes, precursor miRNA, miRNA containing vectors, control nucleic acids, and other probes and primers. In many embodiments, miRNA are 19-24 nucleotides in length, while miRNA probes are 19-35 nucleotides in length, depending on the length of the processed miRNA and any flanking regions added. miRNA precursors are generally between 62 and 110 nucleotides in humans.

Nucleic acids of the disclosure may have regions of identity or complementarity to another nucleic acid. It is contemplated that the region of complementarity or identity can be at least 5 contiguous residues, though it is specifically contemplated that the region is, is at least, or is at most 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or 110 contiguous nucleotides including ranges there between. It is further understood that the length of complementarity within a precursor miRNA or between a miRNA probe and a miRNA or a miRNA gene are such lengths. Moreover, the complementarity may be expressed as a percentage, meaning that the complementarity or identity between a probe and its target is 90% or greater over the length of the probe. In some aspects, complementarity is or is at least 90%, 95% or 100%. In particular, such lengths may be applied to any nucleic acid comprising a nucleic acid sequence identified or disclosed herein. The commonly used name of the miRNA is given (with its identifying source in the prefix, for example, "hsa" for human sequences) and the processed miRNA sequence. Unless otherwise indicated, a miRNA without a prefix will be understood to refer to a human miRNA. A miRNA designated, for example, as miR-1-2 in the application will be understood to refer to hsa-miR-1-2. Moreover, a lowercase letter in one reference may or may not be lowercase in another; for example, hsa-mir-130b can also be referred to as miR-130B. Other prefix can be found using the miRBase website or similar databases. The term "miRNA probe" refers to a nucleic acid probe that can identify a particular miRNA or structurally related miRNAs.

The term "recombinant" may be used and this generally refers to a molecule that has been manipulated *in vitro* or that is a replicated or expressed product of such a molecule.

The term "nucleic acid" is well known in the art. A "nucleic acid" as used herein will generally refer to a molecule (one or more strands) of DNA, RNA or a derivative or analog thereof, comprising a nucleobase. A nucleobase includes, for example, a naturally occurring purine or pyrimidine base found in DNA *(e.g.,* an adenine "A," a guanine "G," a thymine "T" or a cytosine "C") or RNA (e.g., an A, a G, an uracil "U" or a C). The term "nucleic acid" encompasses the terms "oligonucleotide" and "polynucleotide," each as a subgenus of the term "nucleic acid."

The term "miRNA" generally refers to a single-stranded molecule, but in specific embodiments, molecules implemented in the invention will also encompass a region or an additional strand that is partially (between 10 and 50% complementary across length of strand), substantially (greater than 50% but less than 100% complementary across length of strand) or fully complementary to another region of the same single-stranded molecule or to another nucleic acid. Thus, nucleic acids may encompass a molecule that comprises one or more complementary or self-complementary strand(s) or "complement(s)" of a particular sequence comprising a molecule. For example, precursor miRNA may have a self-complementary region, which is up to 100% complementary miRNA probes or nucleic acids of the invention can include, can be or can be at least 60, 65, 70, 75, 80, 85, 90, 95, 96, 97, 98, 99 or 100% complementary to their target.

As used herein, "hybridization", "hybridizes" or "capable of hybridizing" is understood to mean the forming of a double or triple stranded molecule or a molecule with partial double or triple stranded nature. The term "anneal" as used herein is synonymous with "hybridize." The term "hybridization", "hybridize(s)" or "capable of hybridizing" encompasses the terms "stringent condition(s)" or "high stringency" and the terms "low stringency" or "low stringency condition(s)."

As used herein "stringent condition(s)" or "high stringency" are those conditions that allow hybridization between or within one or more nucleic acid strand(s) containing complementary sequence(s), but preclude hybridization of random sequences. Stringent conditions tolerate little, if any, mismatch between a nucleic acid and a target strand. Such conditions are well known to those of ordinary skill in the art, and are preferred for applications requiring high selectivity. Non-limiting applications include isolating a nucleic acid, such as a gene or a nucleic acid segment thereof, or detecting at least one specific mRNA transcript or a nucleic acid segment thereof, and the like.

Stringent conditions may comprise low salt and/or high temperature conditions, such as provided by about 0.02 M to about 0.5 M NaCl at temperatures of about 42°C to about 70°C. It is understood that the temperature and ionic strength of a desired stringency are determined in part by the length of the particular nucleic acid(s), the length and nucleobase content of the target sequence(s), the charge composition of the nucleic acid(s), and the presence or concentration of formamide, tetramethylammonium chloride or other solvent(s) in a hybridization mixture.

It is also understood that these ranges, compositions and conditions for hybridization are mentioned by way of non-limiting examples only, and that the desired stringency for a particular hybridization reaction is often determined empirically by comparison to one or more positive or negative controls. Depending on the application envisioned it is preferred to employ varying conditions of hybridization to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions," and non-limiting examples of low stringency include hybridization performed at about 0.15 M to about 0.9 M NaCl at a temperature range of about 20°C to about 50°C. Of course, it is within the skill of one in the art to further modify the low or high stringency conditions to suite a particular application.

In some aspects, there is a synthetic miRNA having a length of between 17 and 130 residues. The present disclosure concerns synthetic miRNA molecules that are, are at least, or are at most 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 140, 145, 150, 160, 170, 180, 190, 200 or more residues in length, including any integer or any range derivable therein.

In certain aspects, synthetic miRNA have (a) a "miRNA region" whose sequence from 5' to 3' is identical to all or a segment of a mature miRNA sequence, and (b) a "complementary region" whose sequence from 5' to 3' is between 60% and 100% complementary to the miRNA sequence. In certain aspects, these synthetic miRNA are also isolated, i.e., . The term "miRNA region" refers to a region on the synthetic miRNA that is at least 75, 80, 85, 90, 95, or 100% identical, including all integers there between, to the entire sequence of a mature, naturally occurring miRNA sequence. In certain aspects, the miRNA region is or is at least 90, 91, 92, 93, 94, 95, 96, 97, 98, 99, 99.1, 99.2, 99.3, 99.4, 99.5, 99.6, 99.7, 99.8, 99.9 or 100% identical to the sequence of a naturally-occurring miRNA.

The term "complementary region" refers to a region of a synthetic miRNA that is or is at least 60% complementary to the mature, naturally occurring miRNA sequence that the miRNA region is identical to. The complementary region is or is at least 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein. With single polynucleotide sequences, there is a hairpin loop structure as a result of chemical bonding between the miRNA region and the complementary region. In other aspects, the complementary region is on a different nucleic acid molecule than the miRNA region, in which case the complementary region is on the complementary strand and the miRNA region is on the active strand.

In other aspects of the disclosure, there are synthetic nucleic acids that are miRNA inhibitors. A miRNA inhibitor is between about 17 to 25 nucleotides in length and comprises a 5' to 3' sequence that is at least 90% complementary to the 5' to 3' sequence of a mature miRNA. In certain aspects, a miRNA inhibitor molecule is 17, 18, 19, 20, 21, 22, 23, 24, or 25 nucleotides in length, or any range derivable therein. Moreover, a miRNA inhibitor has a sequence (from 5' to 3') that is or is at least 90, 91, 92, 93, 94, 95, 96, 97, 98, 99, 99.1, 99.2, 99.3, 99.4, 99.5, 99.6, 99.7, 99.8, 99.9 or 100% complementary, or any range derivable therein, to the 5' to 3' sequence of a mature miRNA, particularly a mature, naturally occurring miRNA. One of skill in the art could use a portion of the probe sequence that is complementary to the sequence of a mature mRNA as the sequence for a miRNA inhibitor. Moreover, that portion of the probe sequence can be altered so that it is still 90% complementary to the sequence of a mature miRNA.

In some aspects of the disclosure, a synthetic miRNA contains one or more design elements. These design elements include, but are not limited to: (i) a replacement group for the phosphate or hydroxyl of the nucleotide at the 5' terminus of the complementary region; (ii) one or more sugar modifications in the first or last 1 to 6 residues of the complementary region; or, (iii) noncomplementarity between one or more nucleotides in the last 1 to 5 residues at the 3' end of the complementary region and the corresponding nucleotides of the miRNA region.

In certain aspects, a synthetic miRNA has a nucleotide at its 5' end of the complementary region in which the phosphate and/or hydroxyl group has been replaced with another chemical group (referred to as the "replacement design"). In some cases, the phosphate group is replaced, while in others, the hydroxyl group has been replaced. In particular embodiments, the replacement group is biotin, an amine group, a lower alkylamine group, an aminohexyl phosphate group, an acetyl group, 2'O-Me (2'oxygen-methyl), DMTO (4,4'-dimethoxytrityl with oxygen), fluoroscein, a thiol, or acridine, though other replacement groups are well known to those of skill in the art and can be used as well. This design element can also be used with a miRNA inhibitor.

Additional aspects concern a synthetic miRNA having one or more sugar modifications in the first or last 1 to 6 residues of the complementary region (referred to as the "sugar replacement design"). In certain cases, there is one or more sugar modifications in the first 1, 2, 3, 4, 5, 6 or more residues of the complementary region, or any range derivable therein. In additional cases, there is one or more sugar modifications in the last 1, 2, 3, 4, 5, 6 or more residues of the complementary region, or any range derivable therein, have a sugar modification. It will be understood that the terms "first" and "last" are with respect to the order of residues from the 5' end to the 3' end of the region. In particular embodiments, the sugar modification is a 2'O-Me modification, a 2'F modification , a 2'H modification, a 2'amino modification, a 4'ribose modification, or a phosphorothioate modification on the carboxy group linked to the carbon at position 6. In further aspects, there is one or more sugar modifications in the first or last 2 to 4 residues of the complementary region or the first or last 4 to 6 residues of the complementary region. This design element can also be used with a miRNA inhibitor. Thus, a miRNA inhibitor can have this design element and/or a replacement group on the nucleotide at the 5' terminus, as discussed above.

In other aspects of the disclosure, there is a synthetic miRNA in which one or more nucleotides in the last 1 to 5 residues at the 3' end of the complementary region are not complementary to the corresponding nucleotides of the miRNA region ("noncomplementarity") (referred to as the "noncomplementarity design"). The noncomplementarity may be in the last 1, 2, 3, 4, and/or 5 residues of the complementary miRNA. In certain aspects, there is noncomplementarity with at least 2 nucleotides in the complementary region.

It is contemplated that synthetic miRNA of the invention have one or more of the replacement, sugar modification, or noncomplementarity designs. In certain cases, synthetic RNA molecules have two of them, while in others these molecules have all three designs in place.

The miRNA region and the complementary region may be on the same or separate polynucleotides. In cases in which they are contained on or in the same polynucleotide, the mRNA molecule will be considered a single polynucleotide. In aspects in which the different regions are on separate polynucleotides, the synthetic miRNA will be considered to be comprised of two polynucleotides.

When the RNA molecule is a single polynucleotide, there is a linker region between the miRNA region and the complementary region. In some aspects, the single polynucleotide is capable of forming a hairpin loop structure as a result of bonding between the miRNA region and the complementary region. The linker constitutes the hairpin loop. It is contemplated that in some embodiments, the linker region is, is at least, or is at most 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 residues in length, or any range derivable therein. In certain aspects, the linker is between 3 and 30 residues (inclusive) in length.

In addition to having a miRNA region and a complementary region, there may be flanking sequences as well at either the 5' or 3' end of the region. In some aspects, there is or is at least 1, 2, 3, 4, 5, 6, 7, 8, 9, 10 nucleotides or more, or any range derivable therein, flanking one or both sides of these regions.

Methods of the disclosure include reducing or eliminating activity of one or more miRNAs in a cell comprising introducing into a cell a miRNA inhibitor; or supplying or enhancing the activity of one or more miRNAs in a cell. The present disclosure also concerns inducing certain cellular characteristics by providing to a cell a particular nucleic acid, such as a specific synthetic miRNA molecule or a synthetic miRNA inhibitor molecule. However, in methods of the disclosure, the miRNA molecule or miRNA inhibitor need not be synthetic. They may have a sequence that is identical to a naturally occurring miRNA or they may not have any design modifications. In certain aspects, the miRNA molecule and/or a miRNA inhibitor are synthetic, as discussed above.

The particular nucleic acid molecule provided to the cell is understood to correspond to a particular miRNA in the cell, and thus, the miRNA in the cell is referred to as the "corresponding miRNA." In situations in which a named miRNA molecule is introduced into a cell, the corresponding miRNA will be understood to be the induced miRNA. It is contemplated, however, that the miRNA molecule introduced into a cell is not a mature miRNA but is capable of becoming a mature miRNA under the appropriate physiological conditions. In cases in which a particular corresponding miRNA is being inhibited by a miRNA inhibitor, the particular miRNA will be referred to as the targeted miRNA. It is contemplated that multiple corresponding miRNAs may be involved. In particular aspects, more than one miRNA molecule is introduced into a cell. Moreover, in other aspects, more than one miRNA inhibitor is introduced into a cell. Furthermore, a combination of miRNA molecule(s) and miRNA inhibitor(s) may be introduced into a cell.

Methods include identifying a cell or patient in need of inducing those cellular characteristics. Also, it will be understood that an amount of a synthetic nucleic acid that is provided to a cell or organism is an "effective amount," which refers to an amount needed to achieve a desired goal, such as inducing a particular cellular characteristic(s).

In certain aspects of the methods include providing or introducing to a cell a nucleic acid molecule corresponding to a mature miRNA in the cell in an amount effective to achieve a desired physiological result.

Moreover, methods can involve providing synthetic or nonsynthetic miRNA molecules. It is contemplated that in these aspects, methods may or may not be limited to providing only one or more synthetic miRNA molecules or only on or more nonsynthetic miRNA molecules. Thus, in certain aspects, methods may involve providing both synthetic and nonsynthetic miRNA molecules. In this situation, a cell or cells are most likely provided a synthetic miRNA molecule corresponding to a particular miRNA and a nonsynthetic miRNA molecule corresponding to a different miRNA. Furthermore, any method articulated a list of miRNAs using Markush group language may be articulated without the Markush group language and a disjunctive article *(i.e.,* or) instead, and vice versa.

In some aspects, there is a method for reducing or inhibiting cell proliferation in a cell comprising introducing into or providing to the cell an effective amount of (i) a miRNA inhibitor molecule or (ii) a synthetic or nonsynthetic miRNA molecule that corresponds to a miRNA sequence. In certain aspects the methods involves introducing into the cell an effective amount of (i) a miRNA inhibitor molecule having a 5' to 3' sequence that is at least 90% complementary to the 5' to 3' sequence of one or more mature miRNA described herein.

Certain aspects of the disclosure include methods of treating a pancreatic condition. In one aspect, the method comprises contacting a pancreatic cell with one or more nucleic acid, synthetic miRNA, or miRNA comprising at least one nucleic acid segment having all or a portion of a miRNA sequence. The segment may be 5, 6, 7, 8, 9, 10, 11, 12, 13, 14, 15, 16, 17, 18, 19, 20, 21, 22, 23, 24, 25, 30 or more nucleotides or nucleotide analog, including all integers there between. An aspect of the disclosure includes the modulation of a miRNA or a mRNA within a target cell.

Typically, an endogenous gene, miRNA or mRNA is modulated in the cell. In particular aspects, the nucleic acid sequence comprises at least one segment that is at least 70, 75, 80, 85, 90, 95, or 100% identical in nucleic acid sequence to one or more miRNA sequence described herein. Modulation of the expression or processing of an endogenous gene, miRNA, or mRNA can be through modulation of the processing of a mRNA, such processing including transcription, transportation and/or translation with in a cell. Modulation may also be effected by the inhibition or enhancement of miRNA activity with a cell, tissue, or organ. Such processing may effect the expression of an encoded product or the stability of the mRNA. In still other embodiments, a nucleic acid sequence can comprise a modified nucleic acid sequence.

Methods of the disclosure can further comprise administering a second therapy, such as chemotherapy, radiotherapy, surgery, or immunotherapy. The nucleic acid can be transcribed from a nucleic acid vector, such as a plasmid vector or a viral vector.

Method of treating a cancerous or hyperproliferative condition include contacting or administering to a target cell with one or more nucleic acid comprise a miRNA sequence, wherein expression of an endogenous miRNA is modulated in cancerous cell; where the miRNA sequence is at least 70, 75, 80, 85% or more identical to one or more of the identified miRNAs.

The methods may further comprise administering a second therapy. The second therapy can be, but is not limited to chemotherapy, radiotherapy, surgery, or immunotherapy.

In still further aspects, one or more miRNA are transcribed from a nucleic acid vector, such as a plasmid or viral vector.

Synthetic nucleic acids can be administered to the subject or patient using modes of administration that are well known to those of skill in the art, particularly for therapeutic applications. It is particularly contemplated that a patient is human or any other mammal or animal having miRNA.

It will be understood in methods of the disclosure that a cell or other biological matter such as an organism (including patients) can be provided a miRNA or miRNA molecule corresponding to a particular miRNA by administering to the cell or organism a nucleic acid molecule that functions as the corresponding miRNA once inside the cell. The form of the molecule provided to the cell may not be the form that acts as a miRNA once inside the cell. Thus, it is contemplated that in some aspects, biological matter is provided a synthetic miRNA or a nonsynthetic miRNA, such as one that becomes processed into a mature and active miRNA once it has access to the cell's miRNA processing machinery. In certain aspects, it is specifically contemplated that the miRNA molecule provided to the biological matter is not a mature miRNA molecule but a nucleic acid molecule that can be processed into the mature miRNA once it is accessible to miRNA processing machinery. The term "nonsynthetic" in the context of miRNA means that the miRNA is not "synthetic," as defined herein. Furthermore, it is contemplated that in aspects of the disclosure that concern the use of synthetic miRNAs, the use of corresponding nonsynthetic miRNAs is also considered an aspect of the disclosure, and vice versa.

In other aspects, the methods involve reducing cell viability comprising introducing into or providing to the cell an effective amount of (i) a miRNA inhibitor molecule or (ii) a synthetic or nonsynthetic miRNA molecule that corresponds to a miRNA sequence. Methods for inducing apoptosis have a number of therapeutic applications including, but not limited to, the treatment of cancer.

The present disclosure also concerns using miRNA compositions to treat diseases or conditions or to prepare therapeutics for the treatment of diseases or conditions. It is contemplated that 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 more miRNA (or any range derivable therein) may be used for these aspects. In certain aspects, methods involve one or more miRNA inhibitors and/or a miRNA molecules corresponding to any of these miRNAs, particularly for the treatment or prevention of cancer. Cancer includes, but is not limited to, malignant cancers, tumors, metastatic cancers, unresectable cancers, chemo- and/or radiation-resistant cancers, and terminal cancers.

It will be understood that shorthand notations are employed such that a generic description of a miRNA refers to any of its gene family members (distinguished by a number), unless otherwise indicated. It is understood by those of skill in the art that a "gene family" refers to a group of genes having the same miRNA coding sequence. Typically, members of a gene family are identified by a number following the initial designation. For example, miR-16-1 and miR-16-2 are members of the miR-16 gene family and "miR-7" refers to miR-7-1, miR-7-2 and miR-7-3. Moreover, unless otherwise indicated, a shorthand notation refers to related miRNAs (distinguished by a letter). Thus, "let-7," for example, refers to let-7a-1, let7-a-2, let-7b, let-7c, let-7d, let-7e, let-7f-1, and let-7f-2." Exceptions to these shorthand notations will be otherwise identified.

It will be understood that the term "providing" an agent is used to include "administering" the agent to a patient.

In certain aspects, methods also include targeting a miRNA to modulate in a cell or organism. The term "targeting a miRNA to modulate" means a nucleic acid of the invention will be employed so as to modulate the selected miRNA. In some embodiments the modulation is achieved with a synthetic or non-synthetic miRNA that corresponds to the targeted miRNA, which effectively provides the targeted miRNA to the cell or organism (positive modulation). In other aspects, the modulation is achieved with a miRNA inhibitor, which effectively inhibits the targeted miRNA in the cell or organism (negative modulation).

In some aspects, the miRNA targeted to be modulated is a miRNA that affects a disease, condition, or pathway. In certain aspects, the miRNA is targeted because a treatment can be provided by negative modulation of the targeted miRNA. In other aspects, the miRNA is targeted because a treatment can be provided by positive modulation of the targeted miRNA.

In certain methods of the disclosure, there is a further step of administering the selected miRNA modulator to a cell, tissue, organ, or organism (collectively "biological matter") in need of treatment related to modulation of the targeted miRNA or in need of the physiological or biological results discussed herein (such as with respect to a particular cellular pathway or result like decrease in cell viability). Consequently, in some methods of the disclosure there is a step of identifying a patient in need of treatment that can be provided by the miRNA modulator(s). It is contemplated that an effective amount of a miRNA modulator can be administered in some aspects. In particular aspects, there is a therapeutic benefit conferred on the biological matter, where a "therapeutic benefit" refers to an improvement in the one or more conditions or symptoms associated with a disease or condition or an improvement in the prognosis, duration, or status with respect to the disease. It is contemplated that a therapeutic benefit includes, but is not limited to, a decrease in pain, a decrease in morbidity, a decrease in a symptom. For example, with respect to cancer, it is contemplated that a therapeutic benefit can be inhibition of tumor growth, prevention of metastasis, reduction in number of metastases, inhibition of cancer cell proliferation, inhibition of cancer cell proliferation, induction of cell death in cancer cells, inhibition of angiogenesis near cancer cells, induction of apoptosis of cancer cells, reduction in pain, reduction in risk of recurrence, induction of chemo- or radiosensitivity in cancer cells, prolongation of life, and/or delay of death directly or indirectly related to cancer.

Furthermore, it is contemplated that the miRNA compositions may be provided as part of a therapy to a patient, in conjunction with traditional therapies or preventative agents. Moreover, it is contemplated that any method discussed in the context of therapy may be applied as preventatively, particularly in a patient identified to be potentially in need of the therapy or at risk of the condition or disease for which a therapy is needed.

In addition, methods of the disclosure concern employing one or more nucleic acids corresponding to a miRNA and a therapeutic drug. The nucleic acid can enhance the effect or efficacy of the drug, reduce any side effects or toxicity, modify its bioavailability, and/or decrease the dosage or frequency needed. In certain aspects, the therapeutic drug is a cancer therapeutic. Consequently, in some aspects, there is a method of treating cancer in a patient comprising administering to the patient the cancer therapeutic and an effective amount of at least one miRNA molecule that improves the efficacy of the cancer therapeutic or protects non-cancer cells. Cancer therapies also include a variety of combination therapies with both chemical and radiation based treatments. Combination chemotherapies include but are not limited to, for example, bevacizumab, cisplatin (CDDP), carboplatin, EGFR inhibitors (gefitinib and cetuximab), procarbazine, mechlorethamine, cyclophosphamide, camptothecin, COX-2 inhibitors (e.g., celecoxib) ifosfamide, melphalan, chlorambucil, busulfan, nitrosurea, dactinomycin, daunorubicin, doxorubicin (adriamycin), bleomycin, plicomycin, mitomycin, etoposide (VP16), tamoxifen, raloxifene, estrogen receptor binding agents, taxol, taxotere, gemcitabien, navelbine, farnesyl-protein transferase inhibitors, transplatinum, 5-fluorouracil, vincristin, vinblastin and methotrexate, or any analog or derivative variant of the foregoing.

Generally, inhibitors of miRNAs can be given to achieve the opposite effect as compared to when nucleic acid molecules corresponding to the mature miRNA are given. Similarly, nucleic acid molecules corresponding to the mature miRNA can be given to achieve the opposite effect as compared to when inhibitors of the miRNA are given. For example, miRNA molecules that increase cell proliferation can be provided to cells to increase proliferation or inhibitors of such molecules can be provided to cells to decrease cell proliferation. The present disclosure contemplates these embodiments in the context of the different physiological effects observed with the different miRNA molecules and miRNA inhibitors disclosed herein. These include, but are not limited to, the following physiological effects: increase and decreasing cell proliferation, increasing or decreasing apoptosis, increasing transformation, increasing or decreasing cell viability, activating ERK, activating/inducing or inhibiting hTert, inhibit stimulation of Stat3, reduce or increase viable cell number, and increase or decrease number of cells at a particular phase of the cell cycle. Methods of the disclosure are generally contemplated to include providing or introducing one or more different nucleic acid molecules corresponding to one or more different miRNA molecules. It is contemplated that the following, at least the following, or at most the following number of different nucleic acid molecules may be provided or introduced: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or any range derivable therein. This also applies to the number of different miRNA molecules that can be provided or introduced into a cell.

### 1. Nucleobases

As used herein a "nucleobase" refers to a heterocyclic base, such as for example a naturally occurring nucleobase (*i.e.,* an A, T, G, C or U) found in at least one naturally occurring nucleic acid *(i.e.,* DNA and RNA), and naturally or non-naturally occurring derivative(s) and analogs of such a nucleobase. A nucleobase generally can form one or more hydrogen bonds ("anneal" or "hybridize") with at least one naturally occurring nucleobase in manner that may substitute for naturally occurring nucleobase pairing (e.g., the hydrogen bonding between A and T, G and C, and A and U).

"Purine" and/or "pyrimidine" nucleobase(s) encompass naturally occurring purine and/or pyrimidine nucleobases and also derivative(s) and analog(s) thereof, including but not limited to, those a purine or pyrimidine substituted by one or more of an alkyl, carboxyalkyl, amino, hydroxyl, halogen *(i.e.,* fluoro, chloro, bromo, or iodo), thiol or alkylthiol moiety. Preferred alkyl *(e.g.,* alkyl, carboxyalkyl, *etc.)* moieties comprise of from about 1, about 2, about 3, about 4, about 5, to about 6 carbon atoms. Other non-limiting examples of a purine or pyrimidine include a deazapurine, a 2,6-diaminopurine, a 5-fluorouracil, a xanthine, a hypoxanthine, a 8-bromoguanine, a 8-chloroguanine, a bromothymine, a 8-aminoguanine, a 8-hydroxyguanine, a 8-methylguanine, a 8-thioguanine, an azaguanine, a 2-aminopurine, a 5-ethylcytosine, a 5-methylcyosine, a 5-bromouracil, a 5-ethyluracil, a 5-iodouracil, a 5-chlorouracil, a 5-propyluracil, a thiouracil, a 2-methyladenine, a methylthioadenine, a N,N-diemethyladenine, an azaadenines, a 8-bromoadenine, a 8-hydroxyadenine, a 6-hydroxyaminopurine, a 6-thiopurine, a 4-(6-aminohexyl/cytosine), and the like. Other examples are well known to those of skill in the art.

A nucleobase may be comprised in a nucleoside or nucleotide, using any chemical or natural synthesis method described herein or known to one of ordinary skill in the art. Such nucleobase may be labeled or it may be part of a molecule that is labeled and contains the nucleobase.

As used herein, a "nucleoside" refers to an individual chemical unit comprising a nucleobase covalently attached to a nucleobase linker moiety. A non-limiting example of a "nucleobase linker moiety" is a sugar comprising 5-carbon atoms *(i.e.,* a "5-carbon sugar"), including but not limited to a deoxyribose, a ribose, an arabinose, or a derivative or an analog of a 5-carbon sugar. Non-limiting examples of a derivative or an analog of a 5-carbon sugar include a 2'-fluoro-2'-deoxyribose or a carbocyclic sugar where a carbon is substituted for an oxygen atom in the sugar ring. Different types of covalent attachment(s) of a nucleobase to a nucleobase linker moiety are known in the art (Kornberg and Baker, 1992).

As used herein, a "nucleotide" refers to a nucleoside further comprising a "backbone moiety". A backbone moiety generally covalently attaches a nucleotide to another molecule comprising a nucleotide, or to another nucleotide to form a nucleic acid. The "backbone moiety" in naturally occurring nucleotides typically comprises a phosphorus moiety, which is covalently attached to a 5-carbon sugar. The attachment of the backbone moiety typically occurs at either the 3'- or 5'-position of the 5-carbon sugar. However, other types of attachments are known in the art, particularly when a nucleotide comprises derivatives or analogs of a naturally occurring 5-carbon sugar or phosphorus moiety.

### 2. Nucleic Acid Analogs

A nucleic acid may comprise, or be composed entirely of, a derivative or analog of a nucleobase, a nucleobase linker moiety and/or backbone moiety that may be present in a naturally occurring nucleic acid. RNA with nucleic acid analogs may also be labeled according to methods of the disclosure. As used herein a "derivative" refers to a chemically modified or altered form of a naturally occurring molecule, while the terms "mimic" or "analog" refer to a molecule that may or may not structurally resemble a naturally occurring molecule or moiety, but possesses similar functions. As used herein, a "moiety" generally refers to a smaller chemical or molecular component of a larger chemical or molecular structure. Nucleobase, nucleoside and nucleotide analogs or derivatives are well known in the art, and have been described (see for example, Scheit, 1980 ).

Additional non-limiting examples of nucleosides, nucleotides or nucleic acids comprising 5-carbon sugar and/or backbone moiety derivatives or analogs, include those in: U.S. Patent 5,681,947, which describes oligonucleotides comprising purine derivatives that form triple helixes with and/or prevent expression of dsDNA; U.S. Patents 5,652,099 and 5,763,167, which describe nucleic acids incorporating fluorescent analogs of nucleosides found in DNA or RNA, particularly for use as fluorescent nucleic acids probes; U.S. Patent 5,614,617, which describes oligonucleotide analogs with substitutions on pyrimidine rings that possess enhanced nuclease stability; U.S. Patents 5,670,663, 5,872,232 and 5,859,221, which describe oligonucleotide analogs with modified 5-carbon sugars *(i.e.,* modified 2'-deoxyfuranosyl moieties) used in nucleic acid detection; U.S. Patent 5,446,137, which describes oligonucleotides comprising at least one 5-carbon sugar moiety substituted at the 4' position with a substituent other than hydrogen that can be used in hybridization assays; U.S. Patent 5,886,165, which describes oligonucleotides with both deoxyribonucleotides with 3'-5' internucleotide linkages and ribonucleotides with 2'-5' internucleotide linkages; U.S. Patent 5,714,606, which describes a modified internucleotide linkage wherein a 3'-position oxygen of the internucleotide linkage is replaced by a carbon to enhance the nuclease resistance of nucleic acids; U.S. Patent 5,672,697, which describes oligonucleotides containing one or more 5' methylene phosphonate internucleotide linkages that enhance nuclease resistance; U.S. Patents 5,466,786 and 5,792,847, which describe the linkage of a substituent moiety which may comprise a drug or label to the 2' carbon of an oligonucleotide to provide enhanced nuclease stability and ability to deliver drugs or detection moieties; U.S. Patent 5,223,618, which describes oligonucleotide analogs with a 2 or 3 carbon backbone linkage attaching the 4' position and 3' position of adjacent 5-carbon sugar moiety to enhanced cellular uptake, resistance to nucleases and hybridization to target RNA; U.S. Patent 5,470,967, which describes oligonucleotides comprising at least one sulfamate or sulfamide internucleotide linkage that are useful as nucleic acid hybridization probe; U.S. Patents 5,378,825, 5,777,092, 5,623,070, 5,610,289 and 5,602,240, which describe oligonucleotides with three or four atom linker moiety replacing phosphodiester backbone moiety used for improved nuclease resistance, cellular uptake, and regulating RNA expression; U.S. Patent 5,858,988, which describes hydrophobic carrier agent attached to the 2'-O position of oligonucleotides to enhanced their membrane permeability and stability; U.S. Patent 5,214,136, which describes oligonucleotides conjugated to anthraquinone at the 5' terminus that possess enhanced hybridization to DNA or RNA; enhanced stability to nucleases; U.S. Patent 5,700,922, which describes PNA-DNA-PNA chimeras wherein the DNA comprises 2'-deoxy-erythro-pentofuranosyl nucleotides for enhanced nuclease resistance, binding affinity, and ability to activate RNase H; and U.S. Patent 5,708,154, which describes RNA linked to a DNA to form a DNA-RNA hybrid; U.S. Patent 5,728,525, which describes the labeling of nucleoside analogs with a universal fluorescent label.

Additional teachings for nucleoside analogs and nucleic acid analogs are U.S. Patent 5,728,525, which describes nucleoside analogs that are end-labeled; U.S. Patent 5,637,683, 6,251,666 (L-nucleotide substitutions), and 5,480,980 (7-deaza-2'deoxyguanosine nucleotides and nucleic acid analogs thereof).

Additional non-limiting examples of nucleosides, nucleotides or nucleic acids include those in: U.S. Patents 5,681,947, 5,652,099 and 5,763,167, 5,614,617, 5,670,663, 5,872,232, 5,859,221, 5,446,137, 5,886,165, 5,714,606, 5,672,697, 5,466,786, 5,792,847, 5,223,618, 5,470,967, 5,378,825, 5,777,092, 5,623,070, 5,610,289, 5,602,240, 5,858,988, 5,214,136, 5,700,922, 5,708,154, 5,728,525, 5,637,683, 6,251,666, 5,480,980, and 5,728,525.

### 3. Modified Nucleotides

Labeling methods and kits of the disclosure specifically contemplate the use of nucleotides that are both modified for attachment of a label and can be incorporated into a miRNA molecule. Such nucleotides include those that can be labeled with a dye, including a fluorescent dye, or with a molecule such as biotin. Labeled nucleotides are readily available; they can be acquired commercially or they can be synthesized by reactions known to those of skill in the art.

Modified nucleotides for use in the disclosure may not be naturally occurring nucleotides, but instead, refer to prepared nucleotides that have a reactive moiety on them. Specific reactive functionalities of interest include: amino, sulfhydryl, sulfoxyl, aminosulfhydryl, azido, epoxide, isothiocyanate, isocyanate, anhydride, monochlorotriazine, dichlorotriazine, mono-or dihalogen substituted pyridine, mono- or disubstituted diazine, maleimide, epoxide, aziridine, sulfonyl halide, acid halide, alkyl halide, aryl halide, alkylsulfonate, N-hydroxysuccinimide ester, imido ester, hydrazine, azidonitrophenyl, azide, 3-(2-pyridyl dithio)-propionamide, glyoxal, aldehyde, iodoacetyl, cyanomethyl ester, p-nitrophenyl ester, o-nitrophenyl ester, hydroxypyridine ester, carbonyl imidazole, and the other such chemical groups. In some aspects, the reactive functionality may be bonded directly to a nucleotide, or it may be bonded to the nucleotide through a linking group. The functional moiety and any linker cannot substantially impair the ability of the nucleotide to be added to the miRNA or to be labeled. Representative linking groups include carbon containing linking groups, typically ranging from about 2 to 18, usually from about 2 to 8 carbon atoms, where the carbon containing linking groups may or may not include one or more heteroatoms, e.g. S, O, N etc., and may or may not include one or more sites of unsaturation. Of particular interest in many aspects are alkyl linking groups, typically lower alkyl linking groups of 1 to 16, usually 1 to 4 carbon atoms, where the linking groups may include one or more sites of unsaturation. The functionalized nucleotides (or primers) used in the above methods of functionalized target generation may be fabricated using known protocols or purchased from commercial vendors, *e.g*., Sigma, Roche, Ambion, Biosearch Technologies and NEN. Functional groups may be prepared according to ways known to those of skill in the art, including the representative information found in U.S. Patents 4,404,289; 4,405,711; 4,337,063 and 5,268,486, and U.K. Patent 1,529,202.

Amine-modified nucleotides are used in several aspects of the disclosure. The amine-modified nucleotide is a nucleotide that has a reactive amine group for attachment of the label. It is contemplated that any ribonucleotide (G, A, U, or C) or deoxyribonucleotide (G, A, T, or C) can be modified for labeling. Examples include, but are not limited to, the following modified ribo- and deoxyribo-nucleotides: 5-(3-aminoallyl)-UTP; 8-[(4-amino)butyl]-amino-ATP and 8-[(6-amino)butyl]-amino-ATP; N6-(4-amino)butyl-ATP, N6-(6-amino)butyl-ATP, N4-[2,2-oxy-bis-(ethylamine)]-CTP; N6-(6-Amino)hexyl-ATP; 8-[(6-Amino)hexyl]-amino-ATP; 5-propargylamino-CTP, 5-propargylamino-UTP; 5-(3-aminoallyl)-dUTP; 8-[(4-amino)butyl]-amino-dATP and 8-[(6-amino)butyl]-amino-dATP; N6-(4-amino)butyl-dATP, N6-(6-amino)butyl-dATP, N4-[2,2-oxy-bis-(ethylamine)]-dCTP; N6-(6-Amino)hexyl-dATP; 8-[(6-Amino)hexyl]-amino-dATP; 5-propargylamino-dCTP, and 5-propargylamino-dUTP. Such nucleotides can be prepared according to methods known to those of skill in the art. Moreover, a person of ordinary skill in the art could prepare other nucleotide entities with the same amine-modification, such as a 5-(3-aminoallyl)-CTP, GTP, ATP, dCTP, dGTP, dTTP, or dUTP in place of a 5-(3-aminoallyl)-UTP.

### B. Preparation of Nucleic Acids

A nucleic acid may be made by any technique known to one of ordinary skill in the art, such as for example, chemical synthesis, enzymatic production or biological production. It is specifically contemplated that miRNA probes of the disclosure are chemically synthesized.

In some aspects of the disclosure, miRNAs are recovered or isolated from a biological sample. The miRNA may be recombinant or it may be natural or endogenous to the cell (produced from the cell's genome). It is contemplated that a biological sample may be treated in a way so as to enhance the recovery of small RNA molecules such as miRNA. U.S. Patent US 20050059024 describes such methods. Generally, methods involve lysing cells with a solution having guanidinium and a detergent.

Alternatively, nucleic acid synthesis is performed according to standard methods. See, for example, Itakura and Riggs (1980). Additionally, U.S. Patents 4,704,362, 5,221,619, and 5,583,013 each describe various methods of preparing synthetic nucleic acids. Non-limiting examples of a synthetic nucleic acid *(e.g.,* a synthetic oligonucleotide), include a nucleic acid made by *in vitro* chemically synthesis using phosphotriester, phosphite, or phosphoramidite chemistry and solid phase techniques such as described in EP 266,032, or via deoxynucleoside H-phosphonate intermediates as described by Froehler *et al*., 1986 and U.S. Patent 5,705,629. In the methods of the present disclosure, one or more oligonucleotide may be used. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

A non-limiting example of an enzymatically produced nucleic acid include one produced by enzymes in amplification reactions such as PCR™ (see for example, U.S. Patents 4,683,202 and 4,682,195), or the synthesis of an oligonucleotide described in U.S. Patent 5,645,897. A non-limiting example of a biologically produced nucleic acid includes a recombinant nucleic acid produced (*i.e*., replicated) in a living cell, such as a recombinant DNA vector replicated in bacteria (see for example, Sambrook *et al*., 2001).

Oligonucleotide synthesis is well known to those of skill in the art. Various different mechanisms of oligonucleotide synthesis have been disclosed in for example, U.S. Patents 4,659,774, 4,816,571, 5,141,813, 5,264,566, 4,959,463, 5,428,148, 5,554,744, 5,574,146, 5,602,244.

Basically, chemical synthesis can be achieved by the diester method, the triester method polynucleotides phosphorylase method and by solid-phase chemistry. The diester method was the first to be developed to a usable state, primarily by Khorana and co-workers. (Khorana, 1979). The basic step is the joining of two suitably protected deoxynucleotides to form a dideoxynucleotide containing a phosphodiester bond.

The main difference between the diester and triester methods is the presence in the latter of an extra protecting group on the phosphate atoms of the reactants and products (Itakura *et al*., 1975). Purification's are typically done in chloroform solutions. Other improvements in the method include (i) the block coupling of trimers and larger oligomers, (ii) the extensive use of high-performance liquid chromatography for the purification of both intermediate and final products, and (iii) solid-phase synthesis.

Recombinant methods for producing nucleic acids in a cell are well known to those of skill in the art. These include the use of vectors (viral and non-viral), plasmids, cosmids, and other vehicles for delivering a nucleic acid to a cell, which may be the target cell *(e.g.,* a cancer cell) or simply a host cell (to produce large quantities of the desired RNA molecule). Alternatively, such vehicles can be used in the context of a cell free system so long as the reagents for generating the RNA molecule are present. Such methods include those described in Sambrook, 2003, Sambrook, 2001 and Sambrook, 1989.

In certain aspects, the present disclosure concerns nucleic acid molecules that are not synthetic. In some aspects, the nucleic acid molecule has a chemical structure of a naturally occurring nucleic acid and a sequence of a naturally occurring nucleic acid, such as the exact and entire sequence of a single stranded primary miRNA (see Lee 2002), a single-stranded precursor miRNA, or a single-stranded mature miRNA. In addition to the use of recombinant technology, such non-synthetic nucleic acids may be generated chemically, such as by employing technology used for creating oligonucleotides.

### C. Isolation of Nucleic Acids

Nucleic acids may be isolated using techniques well known to those of skill in the art, though in particular embodiments, methods for isolating small nucleic acid molecules, and/or isolating RNA molecules can be employed. Chromatography is a process often used to separate or isolate nucleic acids from protein or from other nucleic acids. Such methods can involve electrophoresis with a gel matrix, filter columns, alcohol precipitation, and/or other chromatography. If miRNA from cells is to be used or evaluated, methods generally involve lysing the cells with a chaotropic (*e*.*g*., guanidinium isothiocyanate) and/or detergent (e.g., N-lauroyl sarcosine) prior to implementing processes for isolating particular populations of RNA.

In particular methods for separating miRNA from other nucleic acids, a gel matrix is prepared using polyacrylamide, though agarose can also be used. The gels may be graded by concentration or they may be uniform. Plates or tubing can be used to hold the gel matrix for electrophoresis. Usually one-dimensional electrophoresis is employed for the separation of nucleic acids. Plates are used to prepare a slab gel, while the tubing (glass or rubber, typically) can be used to prepare a tube gel. The phrase "tube electrophoresis" refers to the use of a tube or tubing, instead of plates, to form the gel. Materials for implementing tube electrophoresis can be readily prepared by a person of skill in the art or purchased, such as from C.B.S. Scientific Co., Inc. or Scie-Plas.

Methods may involve the use of organic solvents and/or alcohol to isolate nucleic acids, particularly miRNA used in methods and compositions of the invention. Some embodiments are described in U.S. Patent Application Serial No. 10/667,126. Generally, this disclosure provides methods for efficiently isolating small RNA molecules from cells comprising: adding an alcohol solution to a cell lysate and applying the alcohol/lysate mixture to a solid support before eluting the RNA molecules from the solid support. In some aspects, the amount of alcohol added to a cell lysate achieves an alcohol concentration of about 55% to 60%. While different alcohols can be employed, ethanol works well. A solid support may be any structure, and it includes beads, filters, and columns, which may include a mineral or polymer support with electronegative groups. A glass fiber filter or column has worked particularly well for such isolation procedures.

In specific aspects, miRNA isolation processes include: (a) lysing cells in the sample with a lysing solution comprising guanidinium, wherein a lysate with a concentration of at least about 1 M guanidinium is produced; (b) extracting miRNA molecules from the lysate with an extraction solution comprising phenol; (c) adding to the lysate an alcohol solution for form a lysate/alcohol mixture, wherein the concentration of alcohol in the mixture is between about 35% to about 70%; (d) applying the lysate/alcohol mixture to a solid support; (e) eluting the miRNA molecules from the solid support with an ionic solution; and, (f) capturing the miRNA molecules. Typically the sample is dried down and resuspended in a liquid and volume appropriate for subsequent manipulation.

### II. LABELS AND LABELING TECHNIQUES

In some aspects, the present disclosure concerns miRNA or mRNA that are labeled. It is contemplated that miRNA or mRNA may first be isolated and/or purified prior to labeling. This may achieve a reaction that more efficiently labels the miRNA, as opposed to other RNA in a sample in which the miRNA is not isolated or purified prior to labeling. In many embodiments of the invention, the label is non-radioactive. Generally, nucleic acids may be labeled by adding labeled nucleotides (one-step process) or adding nucleotides and labeling the added nucleotides (two-step process).

### A. Labeling Techniques

In some aspects, nucleic acids are labeled by catalytically adding to the nucleic acid an already labeled nucleotide or nucleotides. One or more labeled nucleotides can be added to miRNA molecules. See U.S Patent 6,723,509.

In other aspects, an unlabeled nucleotide or nucleotides is catalytically added to a miRNA, and the unlabeled nucleotide is modified with a chemical moiety that enables it to be subsequently labeled. In aspects of the disclosure, the chemical moiety is a reactive amine such that the nucleotide is an amine-modified nucleotide. Examples of amine-modified nucleotides are well known to those of skill in the art, many being commercially available such as from Ambion, Sigma, Jena Bioscience, and TriLink.

In contrast to labeling of cDNA during its synthesis, the issue for labeling miRNA is how to label the already existing molecule. An enzyme using a di- or tri-phosphate ribonucleotide or deoxyribonucleotide as a substrate for its addition to a miRNA can be used. Moreover, in specific aspects, it involves using a modified di- or tri-phosphate ribonucleotide, which is added to the 3' end of a miRNA. Examples of sources for the enzymes include yeast, gram-negative bacteria such as *E. coli, lactococcus lactis*, and sheep pox virus. Enzymes capable of adding such nucleotides include, but are not limited to, poly(A) polymerase, terminal transferase, and polynucleotide phosphorylase. In specific aspects of the invention, a ligase is contemplated as not being the enzyme used to add the label, and instead, a non-ligase enzyme is employed.

### B. Labels

Labels on miRNA, miRNA probes, or RNA probes may be colorimetric (includes visible and UV spectrum, including fluorescent), luminescent, enzymatic, or positron emitting (including radioactive). The label may be detected directly or indirectly. Radioactive labels include ¹²⁵I, ³²P, ³³P, and ³⁵S. Examples of enzymatic labels include alkaline phosphatase, luciferase, horseradish peroxidase, and β-galactosidase. Labels can also be proteins with luminescent properties, *e.g*., green fluorescent protein and phycoerythrin.

The colorimetric and fluorescent labels contemplated for use as conjugates include, but are not limited to, Alexa Fluor dyes, BODIPY dyes, such as BODIPY FL; Cascade Blue; Cascade Yellow; coumarin and its derivatives, such as 7-amino-4-methylcoumarin, aminocoumarin and hydroxycoumarin; cyanine dyes, such as Cy3 and Cy5; eosins and erythrosins; fluorescein and its derivatives, such as fluorescein isothiocyanate; macrocyclic chelates of lanthanide ions, such as Quantum Dye™; Marina Blue; Oregon Green; rhodamine dyes, such as rhodamine red, tetramethylrhodamine and rhodamine 6G; Texas Red; , fluorescent energy transfer dyes, such as thiazole orange-ethidium heterodimer; and, TOTAB.

Specific examples of dyes include, but are not limited to, those identified above and the following: Alexa Fluor 350, Alexa Fluor 405, Alexa Fluor 430, Alexa Fluor 488, Alexa Fluor 500. Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, and, Alexa Fluor 750; amine-reactive BODIPY dyes, such as BODIPY 493/503, BODIPY 530/550, BODIPY 558/568, BODIPY 564/570, BODIPY 576/589, BODIPY 581/591, BODIPY 630/650, BODIPY 650/655, BODIPY FL, BODIPY R6G, BODIPY TMR, and, BODIPY-TR; Cy3, Cy5, 6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, SYPRO, TAMRA, 2',4',5',7'-Tetrabromosulfonefluorescein, and TET.

Specific examples of fluorescently labeled ribonucleotides are available from Molecular Probes, and these include, Alexa Fluor 488-5-UTP, Fluorescein-12-UTP, BODIPY FL-14-UTP, BODIPY TMR-14-UTP, Tetramethylrhodamine-6-UTP, Alexa Fluor 546-14-UTP, Texas Red-5-UTP, and BODIPY TR-14-UTP. Other fluorescent ribonucleotides are available from Amersham Biosciences, such as Cy3-UTP and Cy5-UTP.

Examples of fluorescently labeled deoxyribonucleotides include Dinitrophenyl (DNP)-11-dUTP, Cascade Blue-7-dUTP, Alexa Fluor 488-5-dUTP, Fluorescein-12-dUTP, Oregon Green 488-5-dUTP, BODIPY FL-14-dUTP, Rhodamine Green-5-dUTP, Alexa Fluor 532-5-dUTP, BODIPY TMR-14-dUTP, Tetramethylrhodamine-6-dUTP, Alexa Fluor 546-14-dUTP, Alexa Fluor 568-5-dUTP, Texas Red-12-dUTP, Texas Red-5-dUTP, BODIPY TR-14-dUTP, Alexa Fluor 594-5-dUTP, BODIPY 630/650-14-dUTP, BODIPY 650/665-14-dUTP; Alexa Fluor 488-7-OBEA-dCTP, Alexa Fluor 546-16-OBEA-dCTP, Alexa Fluor 594-7-OBEA-dCTP, Alexa Fluor 647-12-OBEA-dCTP.

It is contemplated that nucleic acids may be labeled with two different labels. Furthermore, fluorescence resonance energy transfer (FRET) may be employed in methods of the disclosure (*e.g.,* Klostermeier *et al.,* 2002; Emptage, 2001; Didenko, 2001).

Alternatively, the label may not be detectable *per se*, but indirectly detectable or allowing for the isolation or separation of the targeted nucleic acid. For example, the label could be biotin, digoxigenin, polyvalent cations, chelator groups and the other ligands, include ligands for an antibody.

### C. Visualization Techniques

A number of techniques for visualizing or detecting labeled nucleic acids are readily available. Such techniques include, microscopy, arrays, Fluorometry, Light cyclers or other real time PCR machines, FACS analysis, scintillation counters, Phosphoimagers, Geiger counters, MRI, CAT, antibody-based detection methods (Westerns, immunofluorescence, immunohistochemistry), histochemical techniques, HPLC (Griffey *et al.,* 1997), spectroscopy, capillary gel electrophoresis (Cummins *et al.,* 1996), spectroscopy; mass spectroscopy; radiological techniques; and mass balance techniques.

When two or more differentially colored labels are employed, fluorescent resonance energy transfer (FRET) techniques may be employed to characterize association of one or more nucleic acid. Furthermore, a person of ordinary skill in the art is well aware of ways of visualizing, identifying, and characterizing labeled nucleic acids, and accordingly, such protocols may be used as part of the invention. Examples of tools that may be used also include fluorescent microscopy, a BioAnalyzer, a plate reader, Storm (Molecular Dynamics), Array Scanter, FACS (fluorescent activated cell sorter), or any instrument that has the ability to excite and detect a fluorescent molecule.

### III. ARRAY PREPARATION AND SCREENING

Nucleic acids of the disclosure include one or more nucleic acid comprising at least one segment having a sequence or complementary sequence of one or more of the miRNA sequences described herein. Nucleic acid probes of the disclosure are typically coupled to a support. Such supports are well known to those of ordinary skill in the art and include, but are not limited to glass, plastic, metal, or latex. In particular aspects of the disclosure, the support can be planar or in the form of a bead or other geometric shapes or configurations.

### A. Array Preparation

Certain embodiments of the present invention concerns the preparation and use of mRNA or nucleic acid arrays, miRNA or nucleic acid arrays, and/or miRNA or nucleic acid probe arrays, which are macroarrays or microarrays of nucleic acid molecules (probes) that are fully or nearly complementary (over the length of the prove) or identical (over the length of the prove) to a plurality of nucleic acid, mRNA or miRNA molecules, precursor miRNA molecules, or nucleic acids derived from the various genes and gene pathways modulated by miR-10 miRNAs and that are positioned on a support or support material in a spatially separated organization. Macroarrays are typically sheets of nitrocellulose or nylon upon which probes have been spotted. Microarrays position the nucleic acid probes more densely such that up to 10,000 nucleic acid molecules can be fit into a region typically 1 to 4 square centimeters. Microarrays can be fabricated by spotting nucleic acid molecules, e.g., genes, oligonucleotides, *etc.,* onto substrates or fabricating oligonucleotide sequences *in situ* on a substrate. Spotted or fabricated nucleic acid molecules can be applied in a high density matrix pattern of up to about 30 non-identical nucleic acid molecules per square centimeter or higher, e.g. up to about 100 or even 1000 per square centimeter. Microarrays typically use coated glass as the solid support, in contrast to the nitrocellulose-based material of filter arrays. By having an ordered array of marker RNA and/or miRNA-complementing nucleic acid samples, the position of each sample can be tracked and linked to the original sample.

A variety of different array devices in which a plurality of distinct nucleic acid probes are stably associated with the surface of a solid support are known to those of skill in the art. Useful substrates for arrays include nylon, glass, metal, plastic, latex, and silicon. Such arrays may vary in a number of different ways, including average probe length, sequence or types of probes, nature of bond between the probe and the array surface, e.g. covalent or non-covalent, and the like. The labeling and screening methods of the present disclosure and the arrays are not limited in its utility with respect to any parameter except that the probes detect miRNA, or genes or nucleic acid representative of genes; consequently, methods and compositions may be used with a variety of different types of nucleic acid arrays.

miRNA arrays or miRNA probe arrays are ordered macroarrays or microarrays of nucleic acid molecules (probes) that are fully or nearly complementary or identical to a plurality of miRNA molecules, precursor miRNA molecules, or segments thereof and that are positioned on a support or support material in a spatially separated organization. Representative methods and apparatus for preparing a microarray have been described, for example, in U.S. Patents 5,143,854; 5,202,231; 5,242,974; 5,288,644; 5,324,633; 5,384,261; 5,405,783; 5,412,087; 5,424,186; 5,429,807; 5,432,049; 5,436,327; 5,445,934; 5,468,613; 5,470,710; 5,472,672; 5,492,806; 5,525,464; 5,503,980; 5,510,270; 5,525,464; 5,527,681; 5,529,756; 5,532,128; 5,545,531; 5,547,839; 5,554,501; 5,556,752; 5,561,071; 5,571,639; 5,580,726; 5,580,732; 5,593,839; 5,599,695; 5,599,672; 5,610;287; 5,624,711; 5,631,134; 5,639,603; 5,654,413; 5,658,734; 5,661,028; 5,665,547; 5,667,972; 5,695,940; 5,700,637; 5,744,305; 5,800,992; 5,807,522; 5,830,645; 5,837,196; 5,871,928; 5,847,219; 5,876,932; 5,919,626; 6,004,755; 6,087,102; 6,368,799; 6,383,749; 6,617,112; 6,638,717; 6,720,138, as well as WO 93/17126; WO 95/11995; WO 95/21265; WO 95/21944; WO 95/35505; WO 96/31622; WO 97/10365; WO 97/27317; WO 99/35505; WO 09923256; WO 09936760; WO0138580; WO 0168255; WO 03020898; WO 03040410; WO 03053586; WO 03087297; WO 03091426; WO03100012; WO 04020085; WO 04027093; EP 373 203; EP 785 280; EP 799 897 and UK 8 803 000.

It is contemplated that the arrays contain 2, 20, 25, 50, 80, 100 or more different probes. It is contemplated that they may contain 1000, 16,000, 65,000, 250,000 or 1,000,000 or more different probes. The probes can be directed to targets in one or more different organisms or cell types. The oligonucleotide probes range from 5 to 50, 5 to 45, 10 to 40, 9 to 34, or 15 to 40 nucleotides in length in some embodiments. In certain aspects, the oligonucleotide probes are 5, 10, 15, 20 to 20, 25, 30, 35, 40 nucleotides in length including all integers and ranges there between.

The location and sequence of each different probe sequence in the array are generally known. Moreover, the large number of different probes can occupy a relatively small area providing a high density array having a probe density of generally greater than about 60, 100, 600, 1000, 5,000, 10,000, 40,000, 100,000, or 400,000 different oligonucleotide probes per cm². The surface area of the array can be about or less than about 1, 1.6, 2, 3, 4, 5, 6, 7, 8, 9, or 10 cm².

Moreover, a person of ordinary skill in the art could readily analyze data generated using an array. Such protocols are disclosed above, and include information found in WO 9743450; WO 03023058; WO 03022421; WO 03029485; WO 03067217; WO 03066906; WO 03076928; WO 03093810; WO 03100448A1.

### B. Sample Preparation

It is contemplated that the miRNA of a wide variety of samples can be analyzed using the arrays, index of miRNA probes, or array technology of the disclosure. Samples may be biological samples, in which case, they can be from biopsy, fine needle aspirates, exfoliates, blood, tissue, organs, semen, saliva, tears, other bodily fluid, hair follicles, skin, lymph nodes (e.g., sentinel, proximal to a disease site, or distal to a disease site) or any sample containing or constituting biological cells. In certain aspects samples may be, but are nt limted to biopsy or cells purified or enriched to some extent from a biopsy or other bodily fluids or tissues. In certain embodiments, samples may be, but are not limited to, fresh, frozen, fixed, formalin fixed, paraffin embedded, or formalin fixed and paraffin embedded. Alternatively, the sample may not be a biological sample, but be a chemical mixture, such as a cell-free reaction mixture (which may contain one or more biological enzymes). While assessment of endogenous miRNA is contemplated in the methods described herein, recombinant or synthetic miRNA may also be used in various aspects of the disclosure inlcuding therapy or as standards or references.

### C. Hybridization

After an array or a set of miRNA probes is prepared and the miRNA in the sample is labeled, the population of target nucleic acids is contacted with the array or probes under hybridization conditions, where such conditions can be adjusted, as desired, to provide for an optimum level of specificity in view of the particular assay being performed. Suitable hybridization conditions are well known to those of skill in the art and reviewed in Sambrook *et al.* (2001) and WO 95/21944. Of particular interest in many aspects is the use of stringent conditions during hybridization. Stringent conditions are known to those of skill in the art.

It is specifically contemplated that a single array or set of probes may be contacted with multiple samples. The samples may be labeled with different labels to distinguish the samples. For example, a single array can be contacted with a tumor tissue sample labeled with Cy3, and normal tissue sample labeled with Cy5. Differences between the samples for particular miRNAs corresponding to probes on the array can be readily ascertained and quantified.

The small surface area of the array permits uniform hybridization conditions, such as temperature regulation and salt content. Moreover, because of the small area occupied by the high density arrays, hybridization may be carried out in extremely small fluid volumes (*e.g.*, about 250 µl or less, including volumes of about or less than about 5, 10, 25, 50, 60, 70, 80 ,90, 100 µl, or any range derivable therein). In small volumes, hybridization may proceed very rapidly.

### D. Differential Expression Analyses

Arrays of the diclosure can be used to detect differences between two samples. Specifically contemplated applications include identifying and/or quantifying differences between miRNA from a sample that is normal and from a sample that is not normal, between a cancerous condition and a non-cancerous condition, or between two differently treated samples. Also, miRNA may be compared between a sample believed to be susceptible to a particular disease or condition and one believed to be not susceptible or resistant to that disease or condition. A sample that is not normal is one exhibiting phenotypic trait(s) of a disease or condition or one believed to be not normal with respect to that disease or condition. It may be compared to a cell that is normal with respect to that disease or condition. Phenotypic traits include symptoms of, or susceptibility to, a disease or condition of which a component is or may or may not be genetic or caused by a hyperproliferative or neoplastic cell or cells.

An array comprises a solid support with nucleic acid probes attached to the support. Arrays typically comprise a plurality of different nucleic acid probes that are coupled to a surface of a substrate in different, known locations. These arrays, also described as "microarrays" or colloquially "chips" have been generally described in the art, for example, U.S. Pat. Nos. 5,143,854, 5,445,934, 5,744,305, 5,677,195, 6,040,193, 5,424,186 and Fodor *et al.,* 1991). Techniques for the synthesis of these arrays using mechanical synthesis methods are described in, e.g., U.S. Pat. No. 5,384,261. Although a planar array surface is used in certain aspects, the array may be fabricated on a surface of virtually any shape or even a multiplicity of surfaces. Arrays may be nucleic acids on beads, gels, polymeric surfaces, fibers such as fiber optics, glass or any other appropriate substrate, see U.S. Pat. Nos. 5,770,358, 5,789,162, 5,708,153, 6,040,193 and 5,800,992.

Tissues and/or corresponding cancer cells that may be evaluated by methods and compositions of the invention include bladder, blood, bone, bone marrow, brain, breast, colon, esophagus, gastrointestine, gum, head, kidney, liver, lung, lymph node, nasopharynx, neck, ovary, pancreas, prostate, skin, small intesting, spleen, stomach, testis, thymus, thyroid, tongue, trachea, or uterus. Moreover, miRNA can be evaluated in precancers, such as metaplasia, dysplasia, and hyperplasia. The methods of the disclosure can detect metastasis and can be used in detecting, prognosing, and/or staging a patient's disease. It is specifically contemplated that the invention can be used to evaluate differences between stages of disease, such as between hyperplasia, neoplasia, pre-cancer and cancer, or between a primary tumor and a metastasized tumor.

In certain aspects, miRNA profiles may be generated to evaluate and correlate those profiles with pharmacokinetics. For example, miRNA profiles may be created and evaluated for patient tumor and blood samples prior to the patient's being treated or during treatment to determine if there are miRNAs whose expression correlates with the outcome of treatment. Identification of differential miRNAs can lead to a prognostic and/or diagnostic assay that can be used to evaluate tumor and/or blood samples to determine what drug regimen the patient should be provided. In addition, it can be used to identify or select patients suitable for a particular clinical trial. If a miRNA profile is determined to be correlated with drug efficacy or drug toxicity that may be relevant to whether that patient is an appropriate patient for receiving the drug or for a particular dosage of the drug.

In addition to the above prognostic assay, blood samples from patients with a variety of diseases can be evaluated to determine if different diseases can be identified based on blood miRNA levels. A diagnostic assay can be created based on the profiles that doctors can use to identify individuals with a disease or who are at risk to develop a disease or experience a recurrence of disease.

### E. Other Assays

In addition to the use of arrays and microarrays, it is contemplated that a number of difference assays could be employed to analyze miRNAs, their activities, and their effects. Such assays include, but are not limited to, nucleic amplification, polymerase chain reaction, quantitative PCR, RT-PCR, *in situ* hybridization, Northern hybridization, hybridization protection assay (HPA)(GenProbe), branched DNA (bDNA) assay (Chiron), rolling circle amplification (RCA), single molecule hybridization detection (US Genomics), Invader assay (ThirdWave Technologies), and/or Bridge Litigation Assay (Genaco).

### IV. KITS

Any of the compositions described herein may be comprised in a kit. In a non-limiting example, reagents for isolating miRNA, labeling miRNA, and/or evaluating a miRNA population using an array, nucleic acid amplification, and/or hybridization can be included in a kit, as well reagents for preparation of samples from patient samples. The kit may further include miRNA, miRNA probes, or reagents for creating or synthesizing miRNA probes. The kits will thus comprise, in suitable container means, an enzyme for labeling the miRNA by incorporating labeled nucleotide or unlabeled nucleotides that are subsequently labeled. In certain aspects, the kit can include amplification reagents. In other aspects, the kit may include various supports, such as glass, nylon, polymeric beads, and the like, having probes coupled there to and/or reagents for coupling any probes and/or target nucleic acids. It may also include one or more buffers, such as reaction buffer, labeling buffer, washing buffer, or a hybridization buffer, compounds for preparing the miRNA probes, and components for isolating miRNA. Other kits of the disclosure may include components for making a nucleic acid array comprising miRNA.

Kits for implementing methods of the invention described herein are specifically contemplated. In some aspects, there are kits for preparing miRNA for multi-labeling and kits for preparing miRNA probes and/or miRNA arrays. In these aspects, kit comprise, in suitable container means, 1, 2, 3, 4, 5, 6, 7, 8, 9, 10, 11, 12 or more of the following: (1) poly(A) polymerase; (2) unmodified nucleotides (G, A, T, C, and/or U); (3) a modified nucleotide (labeled or unlabeled); (4) poly(A) polymerase buffer; and, (5) at least one microfilter; (6) label that can be attached to a nucleotide; (7) at least one miRNA probe; (8) reaction buffer; (9) a miRNA array or components for making such an array; (10) acetic acid; (11) alcohol; and/or (12) solutions for preparing, isolating, enriching, and purifying miRNAs or miRNA probes or arrays. Other reagents include those generally used for manipulating RNA, such as formamide, loading dye, ribonuclease inhibitors, and DNase.

In specific aspects, kits of the disclosure include an array containing miRNA probes, as described in the application. An array may have probes corresponding to all known miRNAs of an organism or a particular tissue or organ in particular conditions, or to a subset of such probes. The subset of probes on arrays of the disclosure may be or include those identified as relevant to a particular diagnostic, therapeutic, prognostic or staging application. For example, the array may contain one or more probes that is indicative or suggestive of (1) a disease or condition, (2) susceptibility or resistance to a particular drug or treatment; (3) susceptibility to toxicity from a drug or substance; (4) the stage of development or severity of a disease or condition (prognosis); and (5) genetic predisposition to a disease or condition, or combinations thereof.

For any kit, including an array, there can be nucleic acid molecules that contain or can be used to amplify a sequence that is a variant of, identical to or complementary to all or part of any of miRNA described or named herein. In certain aspects, a kit or array of the disclosure can contain one or more probes for the miRNAs identified. Any nucleic acid discussed above may be implemented as part of a kit.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one component in the kit (labeling reagent and label may be packaged together), the kit also will generally contain a second, third or other additional container into which the additional components may be separately placed. However, various combinations of components may be comprised in a vial.

When the components of the kit are provided in one and/or more liquid solutions, the liquid solution is an aqueous solution, with a sterile aqueous solution being particularly preferred.

However, the components of the kit may be provided as dried or lyophilized powder(s). When reagents and/or components are provided as a dry powder, the powder can be reconstituted by the addition of a suitable solvent. It is envisioned that the solvent may also be provided in another container means. In some aspects, labeling dyes are provided as a dried power. It is contemplated that 10, 20, 30, 40, 50, 60, 70, 80, 90, 100, 120, 120, 130, 140, 150, 160, 170, 180, 190, 200, 300, 400, 500, 600, 700, 800, 900, 1000 µg or at least or at most those amounts of dried dye are provided in kits of the invention. The dye may then be resuspended in any suitable solvent, such as DMSO.

The container means will generally include at least one vial, test tube, flask, bottle, syringe and/or other container means, into which the nucleic acid formulations are placed, preferably, suitably allocated. The kits may also comprise a second container means for containing a sterile, pharmaceutically acceptable buffer and/or other diluent.

The kits of the present disclosure will also typically include a means for containing the vials in close confinement for commercial sale, such as, e.g., injection and/or blow-molded plastic containers into which the desired vials are retained.

Such kits may also include components that facilitate isolation of the labeled miRNA. It may also include components that preserve or maintain the miRNA or that protect against its degradation. Such components may be RNAse-free or protect against RNAses. Such kits generally will comprise, in suitable means, distinct containers for each individual reagent or solution.

A kit will also include instructions for employing the kit components as well the use of any other reagent not included in the kit. Instructions may include variations that can be implemented.

Kits of the disclosure may also include one or more of the following: Control RNA; nuclease-free water; RNase-free containers, such as 1.5 ml tubes; RNase-free elution tubes; PEG or dextran; ethanol; acetic acid; sodium acetate; ammonium acetate; guanidinium; detergent; nucleic acid size marker; RNase-free tube tips; and RNase or DNase inhibitors.

It is contemplated that such reagents are embodiments of kits of the disclosure. Such kits, however, are not limited to the particular items identified above and may include any reagent used for the manipulation or characterization of miRNA.

### V. EXAMPLES

The following examples are given for the purpose of illustrating various embodiments of the invention and are not meant to limit the present invention in any fashion. One skilled in the art will appreciate readily that the present invention is well adapted to carry out the objects and obtain the ends and advantages mentioned, as well as those objects, ends and advantages inherent herein. The present examples, along with the methods described herein are presently representative of preferred embodiments, are exemplary, and are not intended as limitations on the scope of the invention.

### EXAMPLE 1:

### CERVIX TISSUE SAMPLES AND RNA FROM CERVICAL SPECIMENS

Total RNA, purified from four separate normal human cervixes (NCX), was purchased from Ambion (FirstChoice^{®} Human Cervix Total RNA, cat. no. AM6992; Ambion, Austin, TX, USA;) (Table 1). Nine, separate squamous cell carcinoma, frozen tissue samples (TUM) collected from human uterine cervixes were purchased from ProteoGenex (ProteoGenex, Inc.; Culver City, CA, USA) (Table 2). Total RNA isolation from the TUM tissue samples was performed using the *mir*Vana™ miRNA Isolation Kit (Ambion; cat. no.-AM1560), following the manufacturer's protocol.

**Table 1. Patient information for normal cervix (NCX) tissue samples. HPV, human papillomavirus**

| **Sample** | **Patient Age** | **Patient Ethnicity** | **HPV16/18 Status** |
|---|---|---|---|
| NCX3 | 72 | Caucasian | Negative |
| NCX10 | 56 | Caucasian | Negative |
| NCX13 | 57 | Caucasian | Negative |
| NCX17 | 53 | Caucasian | N/A |

**Table 2. Patient and tissue sample pathology information for nine squamous cell carcinoma tissue samples (TUM) from human uterine cervixes. Histological Diagnosis refers to diagnosis of the cervix tissue sample. SCC, squamous cell carcinoma; HPV, human papillomavirus**

| **Sample** | **Age (yr)** | **Patient Ethnicity** | **Histological Diagnosis** | **TNM Stage** (Sobin and Wittekind, 2002) | **HPV16/18 Status** |
|---|---|---|---|---|---|
| TUM1 | 52 | Caucasian | SCC | T1N1M0 | 16 |
| TUM2 | 32 | Caucasian | SCC, poorly differentiated | T1bN0M0 | 18 |
| TUM3 | 47 | Caucasian | Non-keratinizing SCC | T1aN1M0 | 16 |
| TUM4 | 45 | Caucasian | SCC, moderately differentiated | TlaNlMO | 16 |
| TUM5 | 29 | Caucasian | SCC, poorly differentiated | Tla2N0M0 | 18 |
| TUM6 | 55 | Caucasian | SCC, moderately differentiated | T3aNlM0 | Negative |
| TUM7 | 40 | Caucasian | SCC, moderately differentiated | T2aNlM0 | 16 + 18 |
| TUM8 | 44 | Caucasian | SCC, well to poorly differentiated | Tla2N0M0 | 16 |
| TUM9 | 39 | Caucasian | SCC, poorly differentiated | T3aN0M0 | 18 |

In addition, formalin-fixed, paraffin-embedded (FFPE) tissue samples, prepared from four cancer-positive and four cancer-negative inguinal lymph nodes from eight different cervical cancer patients, were purchased from ProteoGenex (Table 3). All tissue samples were less than 2 years old. A hematoxilin-eosin (H&E)-stained slide from each sample was reviewed by a pathologist at Asuragen to confirm lymph node statuses as cancer cell-positive (LNpos) or cancer cell-negative (LNneg).

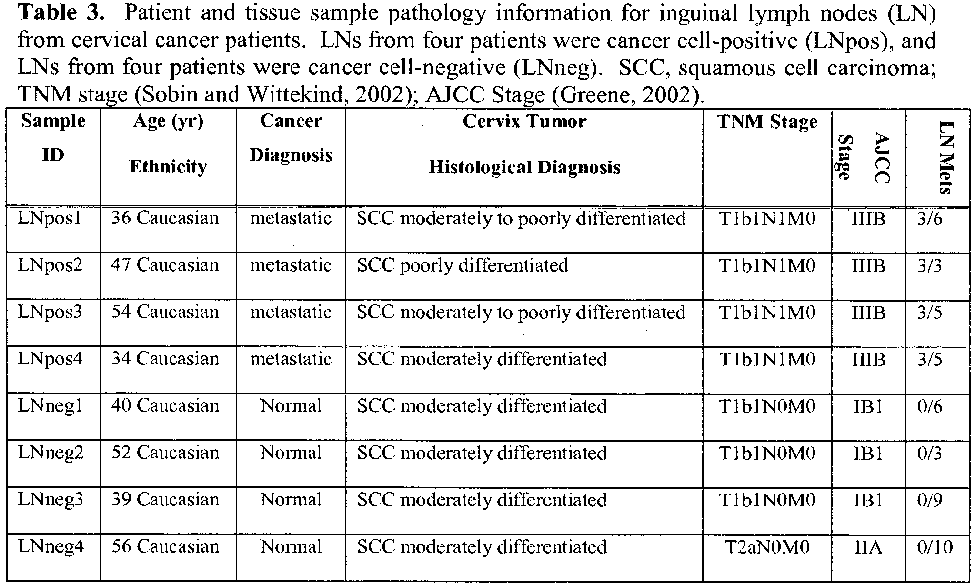

Total RNA was extracted from FFPE tissue samples using a RecoverAll™ kit (Ambion; RecoverAll™ Total Nucleic Acid Isolation Kit for FFPE, cat no.AM1975), using the manufacturer's protocol as modified in Doleshal *et al*.). Purified total RNA was quantified using a Nanodrop® ND-1000 (NanoDrop Technologies, Wilmington, DE, USA).

### EXAMPLE 2:

### MIRNAS DIFFERENTALLY EXPRESSED BETWEEN NORMAL CERVIX TISSUE AND CANCER-NEGATIVE INGUINAL LYMPH NODES

To identify miRNAs that may be useful markers for metastasis of cervical cancer to inguinal lymph nodes, miRNAs were identified that are expressed at significantly higher levels in normal cervix tissue than in cancer-negative inguinal lymph nodes.

miRNA expression was determined for four normal cervix tissue samples (NCX3, NCX10, NCX13, NCX17 - described in Example 1, Table 1 above) and four cancer-negative lymph nodes (LNneg1-4 - described in Example 1, Table 3 above). For each tissue sample, microRNA-containing fractions were purified from 10 µg of total RNA using a flashPAGE™ Fractionation Apparatus (Ambion). Purified small RNAs were enzymatically biotinylated at their 3'-termini and hybridized to a custom-made microRNA array (miRChip V1.0, manufactured by Affymetrix, Santa Clara, CA, USA for Ambion) by DiscovArray™ miRNA Expression Service (Asuragen). The miRChip V1.0 contains probes for more than 13,000 verified and candidate miRNAs from a number of sources including Sanger miRBase v9.2 database (Griffiths-Jones *et al.,* 2006) and published reports (Bentwich *et al.,* 2005; *Berezikov et al.,* 2005; Xie *et al.,* 2005; Cummins *et al.,* 2006). Hybridization, washing, staining, imaging, and signal extraction were performed as recommended by the miRChip manufacturer, except that the 20X GeneChip® Eukaryotic Hybridization Control cocktail was omitted.

Signal processing with the miRChip is a multi-step process involving probe-specific signal detection calls, background estimate and correction, constant variance stabilization and either array scaling or global normalization. For an overview of miRNA processing and analysis see Davison *et al.,*(2006).

***Probe specific Signal detection calls***. Each probe on an array is assayed for detection based on a Wilcoxon rank-sum test of the miRNA probe signal compared to the distribution of signals from GC-content-matched, anti-genomic probes. If the resulting p-value for the probes is ≤ 0.06 then it is considered "detected above background." Probes with p-values > 0.06 have insufficient signal to discriminate from the background and are thus considered not detected.

***Background estimate and correction.*** The same set of anti-genomic probes used to determine detection calls are used to estimate GC-content-matched background signals. Each miRNA probe signal has a GC-content-matched background estimate subtracted from its value. This GC-specific background contribution is estimated by the median signal from the distribution of GC-matched, anti-genomic probes.

***Constant variance stabilization.*** Probes with low signal often exhibit a high degree of variability once transformed into logarithmic scales. To stabilize this low signal variability, the inventors add a constant value of 16 to the background corrected signal, a technique commonly performed on microarray data and the recommended data preprocessing method by Affymetrix and Illumina in the MicroArray Quality Control (MAQC) project (MAQC Consortium, 2006).

***Normalization.*** The data were normalized with the VSN method (Huber *et al.,* 2002). VSN is a global normalization process that stabilizes the variance evenly across the entire range of expression. Differences in VSN-transformed expression between samples are denoted by "log2Diff" and can be transformed to a generalized fold change via exponentiation base 2. These values will exhibit a compression for small differences in expression.

A total of 269 miRNAs (130 verified, 139 predicted) exhibited significant differential expression between normal cervix (NCX) samples and cancer-negative inguinal lymph nodes (LNneg), (*t*-test (LNneg vs NCX) <0.05 and log2Diff (LNneg vs NCX) ≤ -1 (Table 4). Among the verified miRNAs, the average expression levels of six miRNAs (hsa-miR-205, -368, -203, -495, -375, and -487b) were at least 100-fold less in LNneg samples than in NCX samples; the average expression levels of ten miRNAs (hsa-miR-200a, -299-5p, -493-5p, -200b, -133b, -187, -133a, -200c, -411, and -127) were between 50- and 100-fold lower in LNneg samples; the average expression levels of 32 miRNAs were between 10- and 50-fold lower in LNneg samples; and the average expression levels of 23 miRNAs were between 5- and 10 -fold lower in LNneg samples than in NCX samples. Among the predicted miRNAs, the average expression levels of three miRNAs (hsa-asg-5021_st1,-13254_st1, and -9696_st1) were at least 50-fold lower in LNneg samples than in NCX samples; the average expression levels of fourteen miRNAs were between 10- and 50-fold lower in LNneg samples; and the average expression levels of 31 miRNAs were between 5-and 10-fold lower in LNneg samples than in NCX samples.

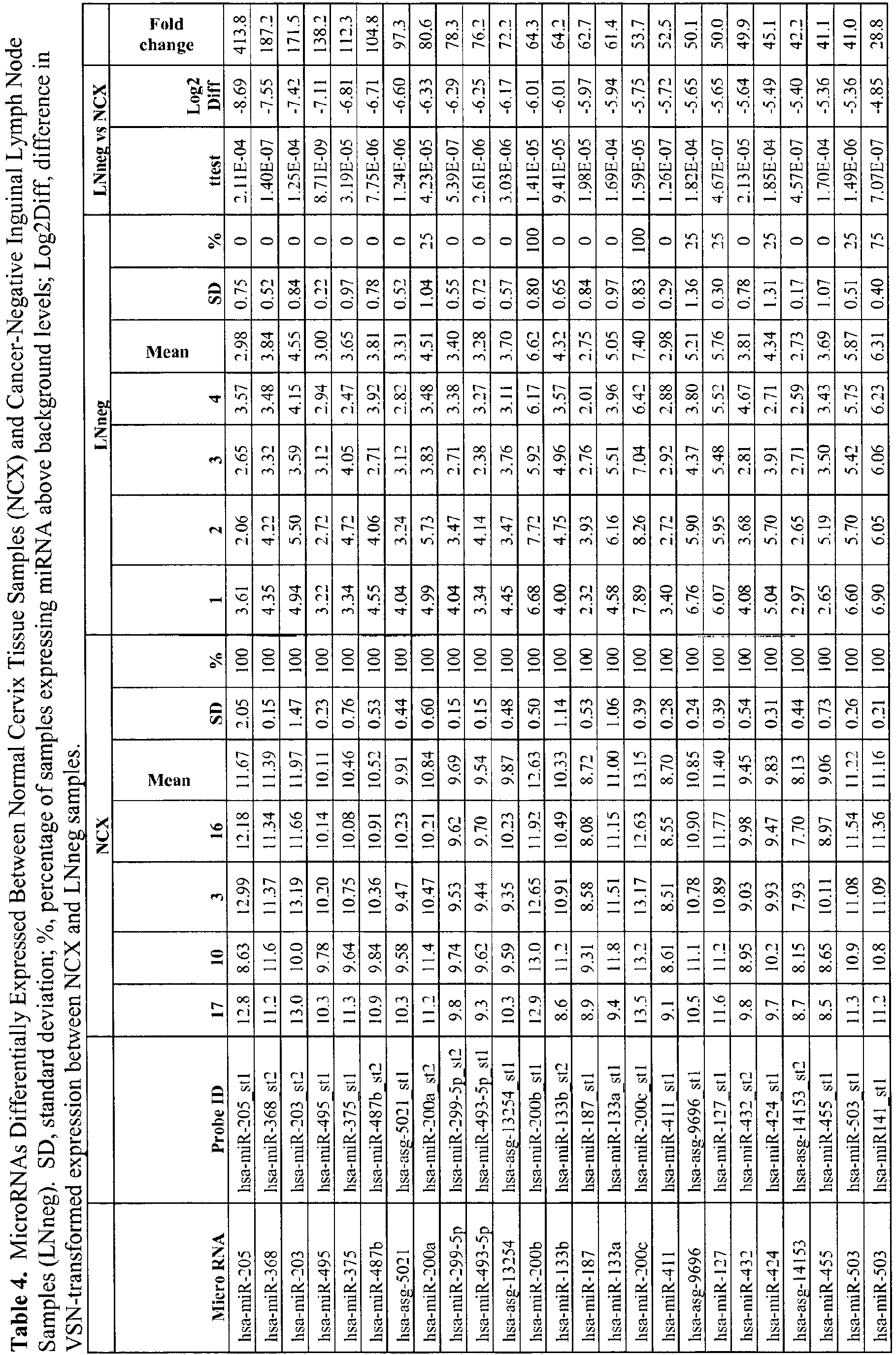

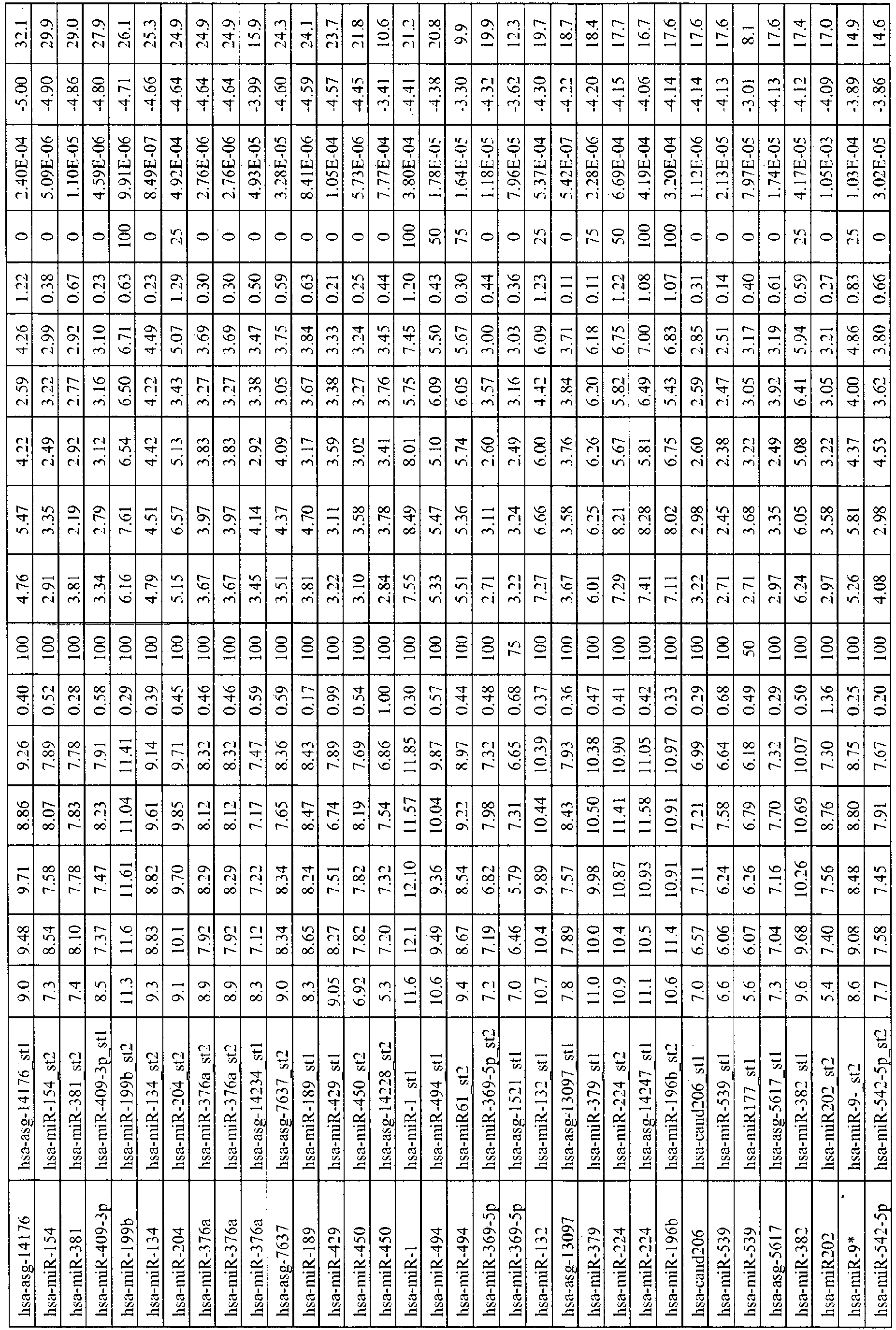

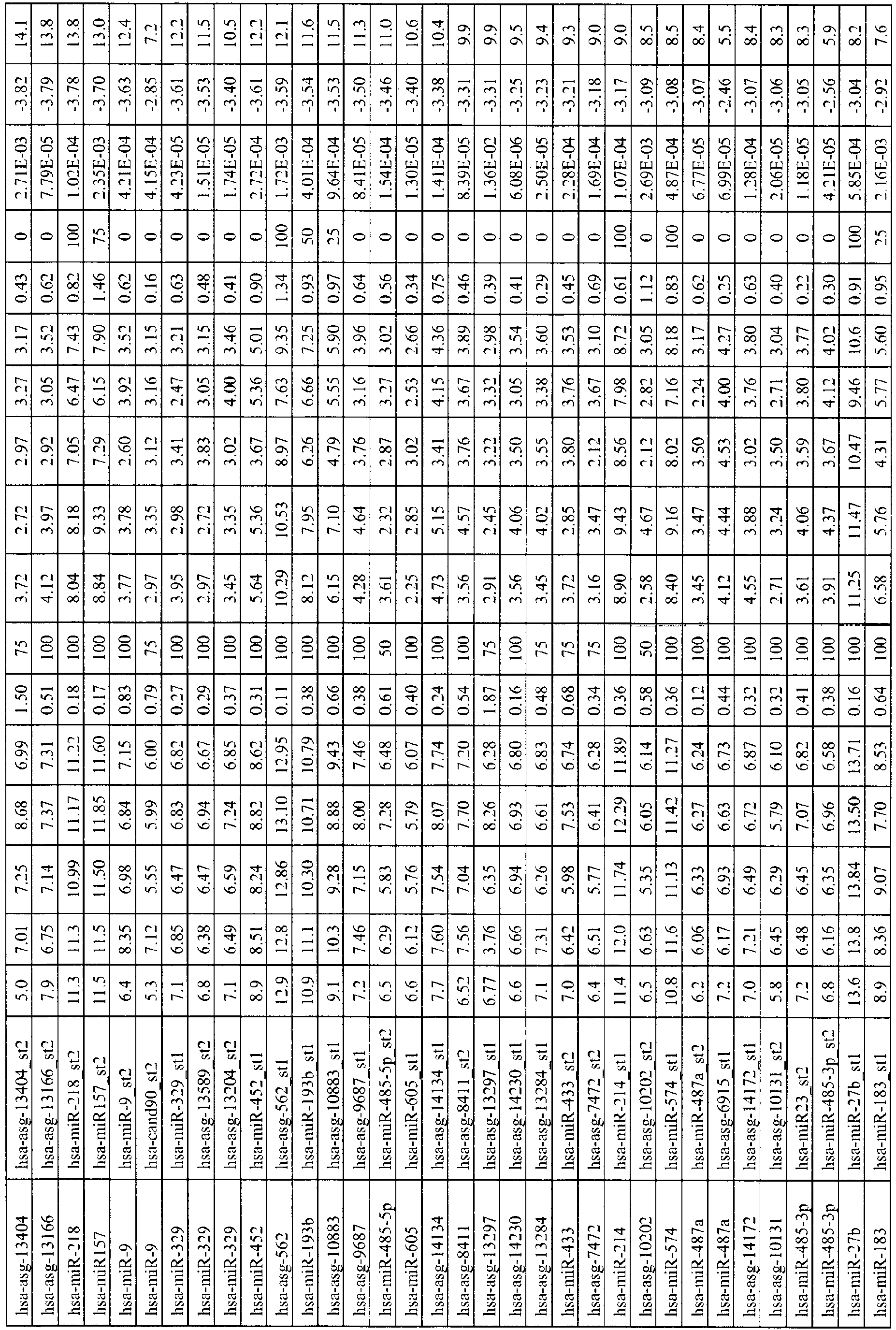

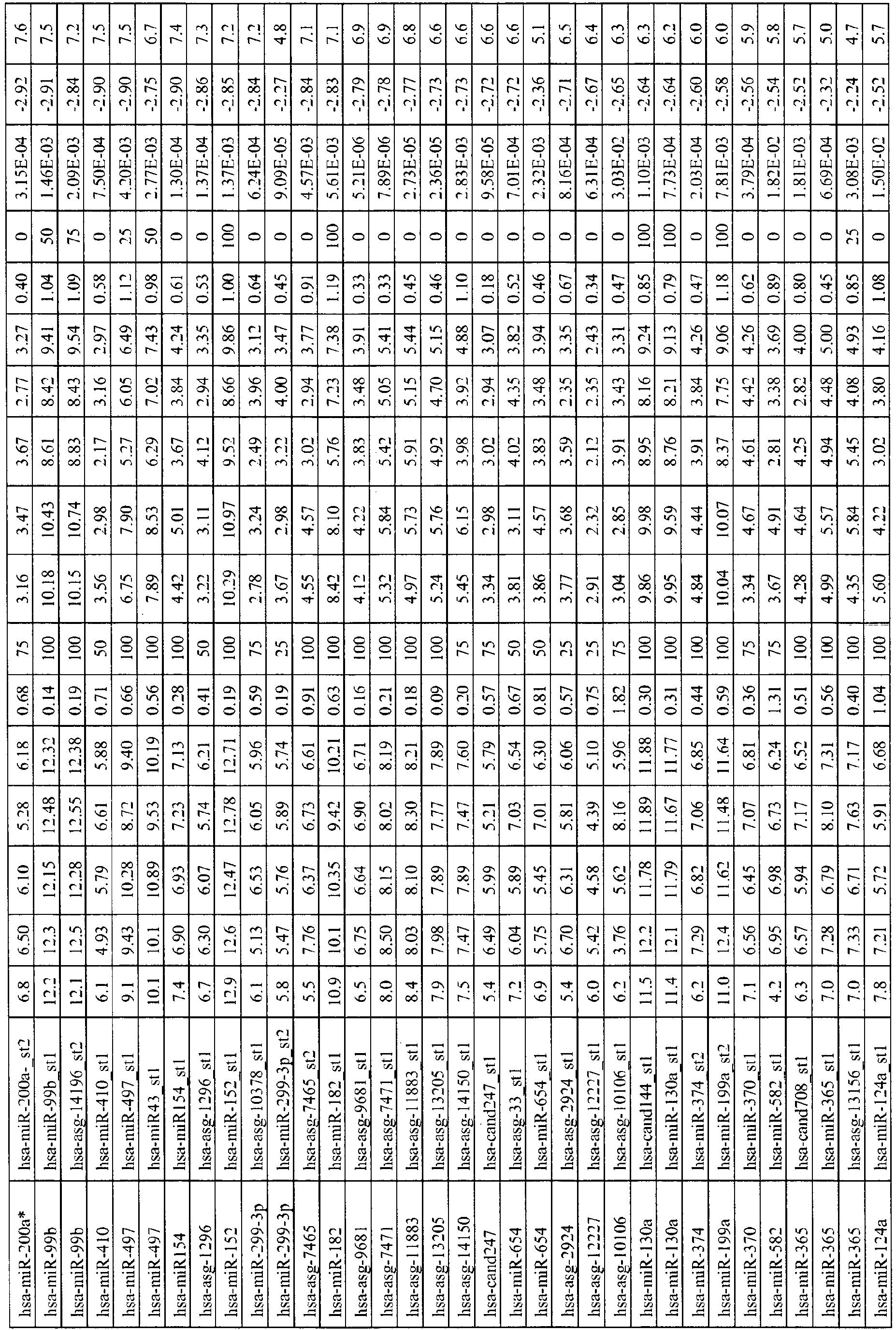

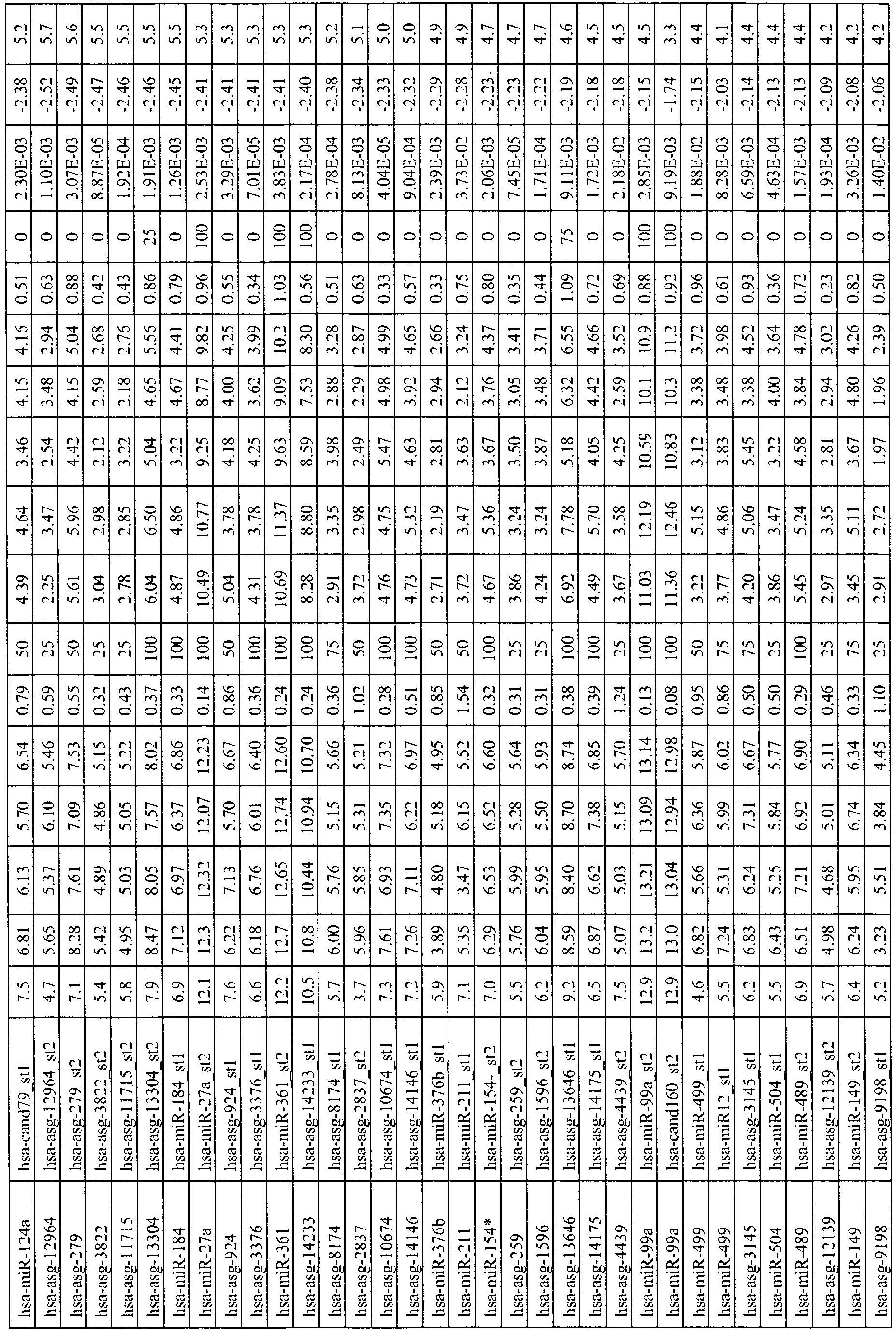

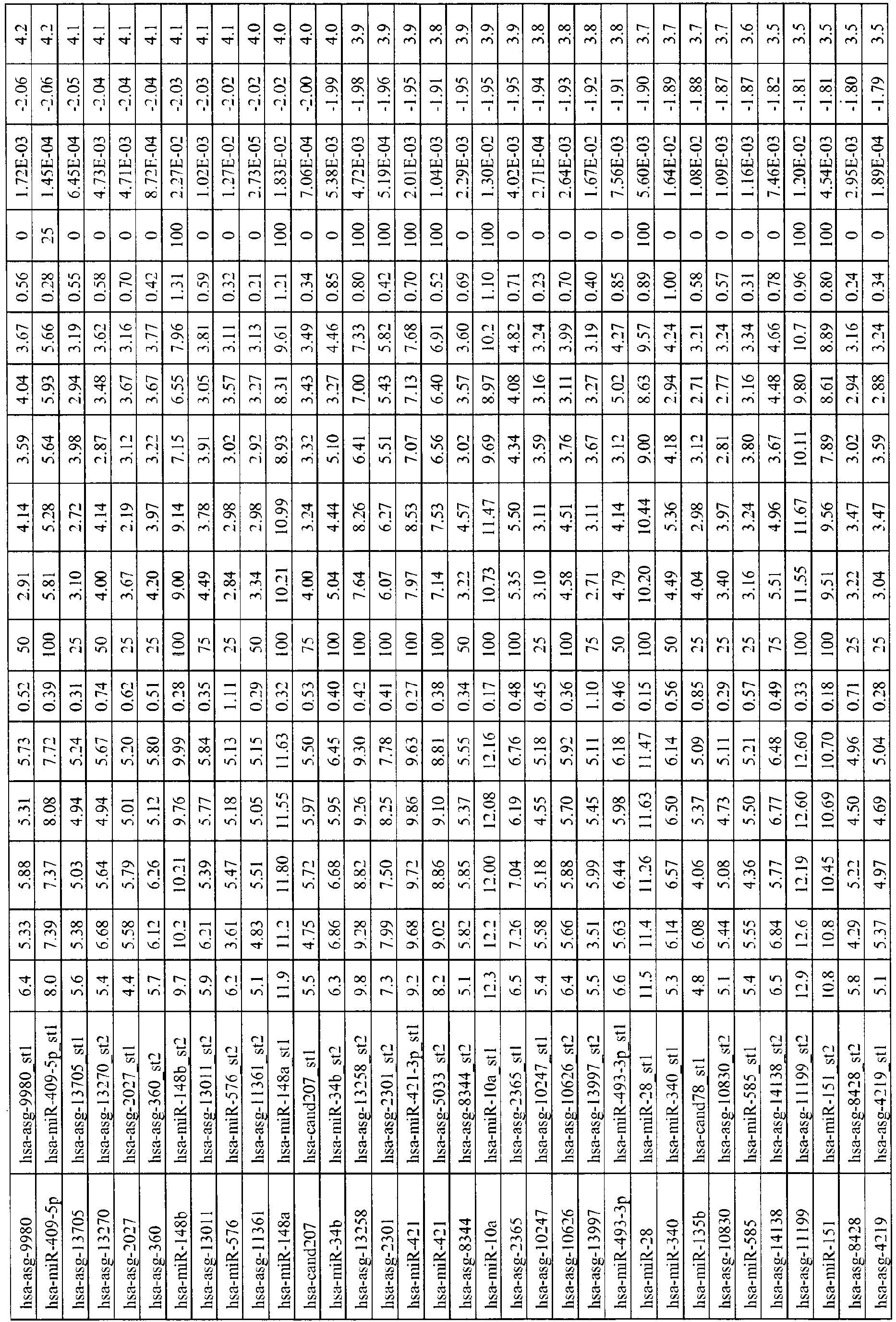

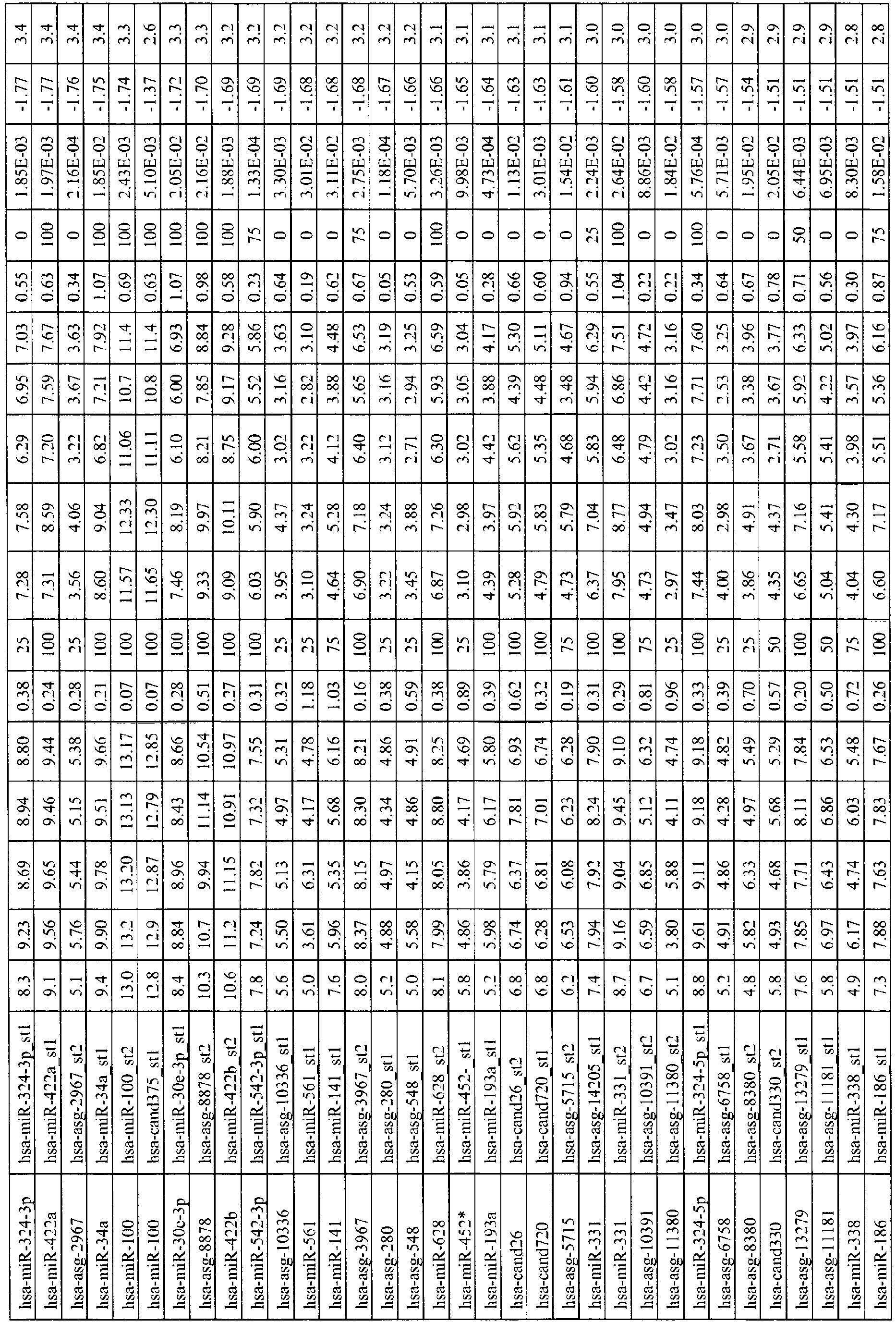

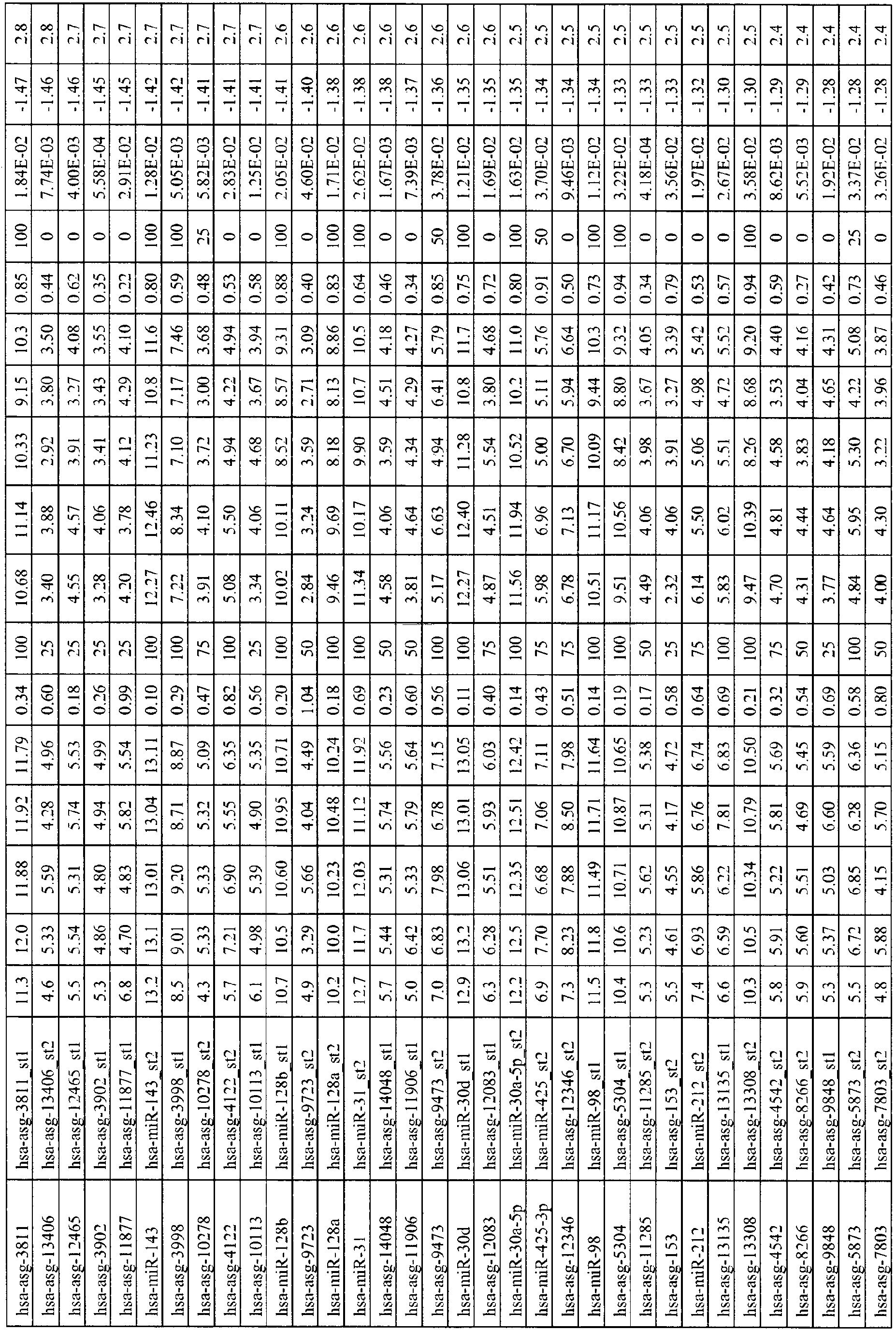

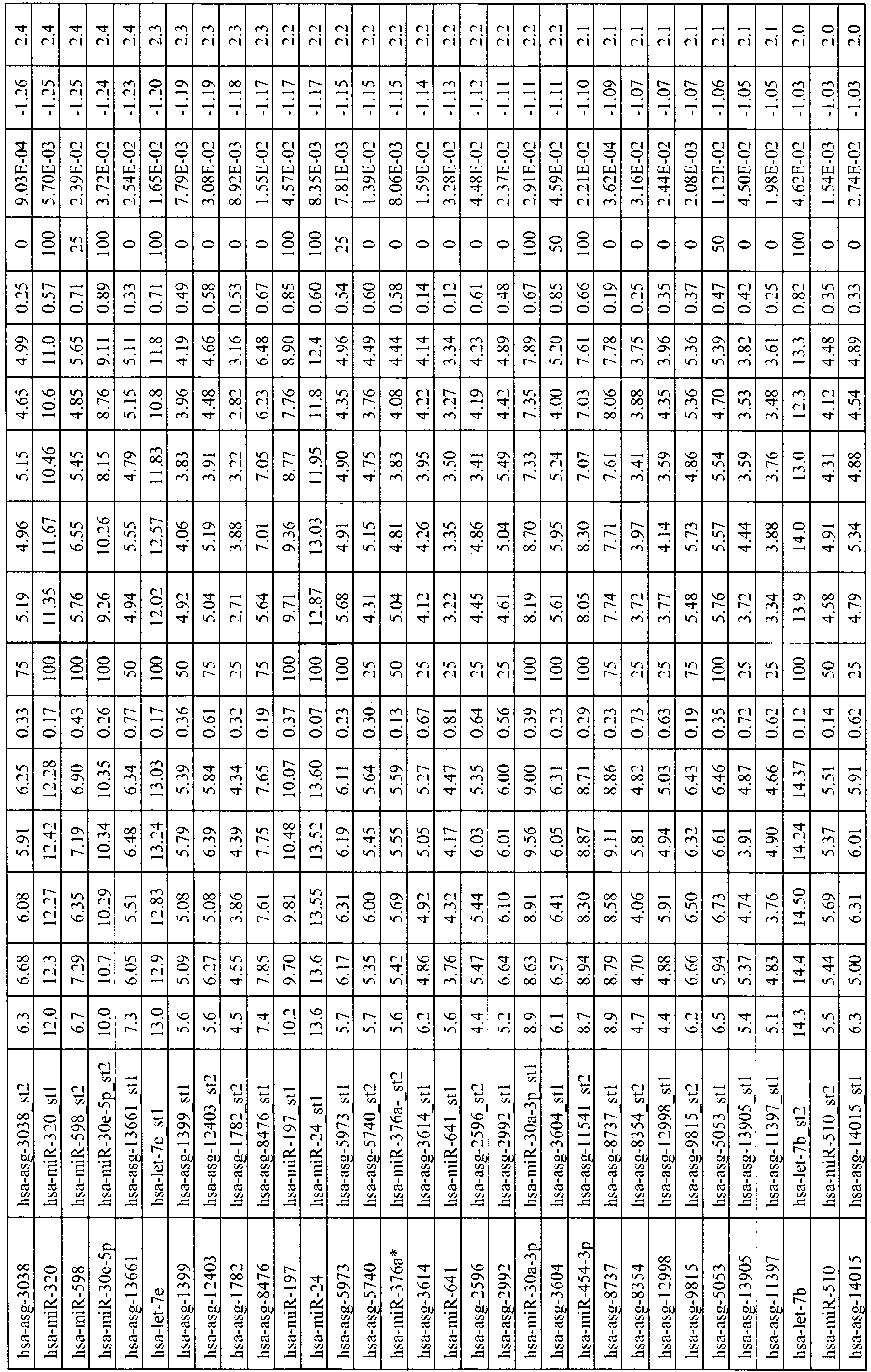

### EXAMPLE 3:

### MIRNAS DIFFERENTIALLY EXPRESSED BETWEEN CANCER-POSITIVE AND CANCER-NEGATIVE INGUINAL LYMPH NODES FROM CERVICAL CANCER PATIENTS

To identify miRNAs that may be useful markers for metastasis of cervical cancer to inguinal lymph nodes, the inventors identified miRNAs that are expressed at significantly higher levels in cancer-positive inguinal lymph nodes from cervical cancer patients than in cancer-negative inguinal lymph nodes.

miRNA expression was determined for four cancer-positive lymph nodes (LNpos1-4) and four cancer-negative lymph nodes (LNneg1-4) isolated from eight different cervical cancer patients (described in Example 1, Table 3). miRNAs were purified from formalin-fixed, paraffin-embedded (FFPE) lymph nodes as described in Example 1 and analyzed with microarrays as described in Example 2.

A total of 126 miRNAs (39 verified, 87 predicted) exhibited significant differential expression between cancer-negative lymph nodes (LNneg) and cancer-positive lymph nodes (LNpos), (*t*-test (LNpos vs LNneg) <0.05 and log2Diff (LNpos vs LNneg) ≤ -1 (Table 5)). Among the verified miRNAs, the average expression level of hsa-miR-205 was more than 1,000-fold lower in LNneg samples than in LNpos samples; the average expression levels of three miRNAs (hsa-miR-203, -200b, and -200a) were between 100- and 1,000-fold lower in LNneg samples; the average expression levels of two miRNAs (hsa-miR-429 and - 200c) were between 50- and 100-fold lower in LNneg samples; the average expression levels of six miRNAs (hsa-miR-224, -141, -200a*, -183, -605, and -182) were between 10- and 50-fold lower in LNneg samples; and the average expression levels of eight miRNAs (hsa-miR-9, -493-5p, -9*, -495, -576, -368, -452, and -189) were between 5- and 10-fold lower in LNneg samples than in LNpos samples. Among the 87 predicted miRNAs, the average expression levels of two (hsa-asg-14153_st2, and -7637_st2) were at least 45-fold lower in LNneg samples than in LNpos samples; the average expression levels of two other miRNAs (hsa-asg-2919_st1, and -12227_st1) were between 10- and 45-fold lower in LNneg samples; and the average expression levels of 14 miRNAs (hsa-asg-11715_st2, -11160_st2, -5119_st1, -1296_st1, -10131_st2, -14176_st1, -8174_st1, -2785_st2, -4978_st2, -9198_st1, -8428_set2,-9112_st2, -1596_st2, -12139_st2) were between 5- and 10-fold lower in LNneg samples than in LNpos samples.

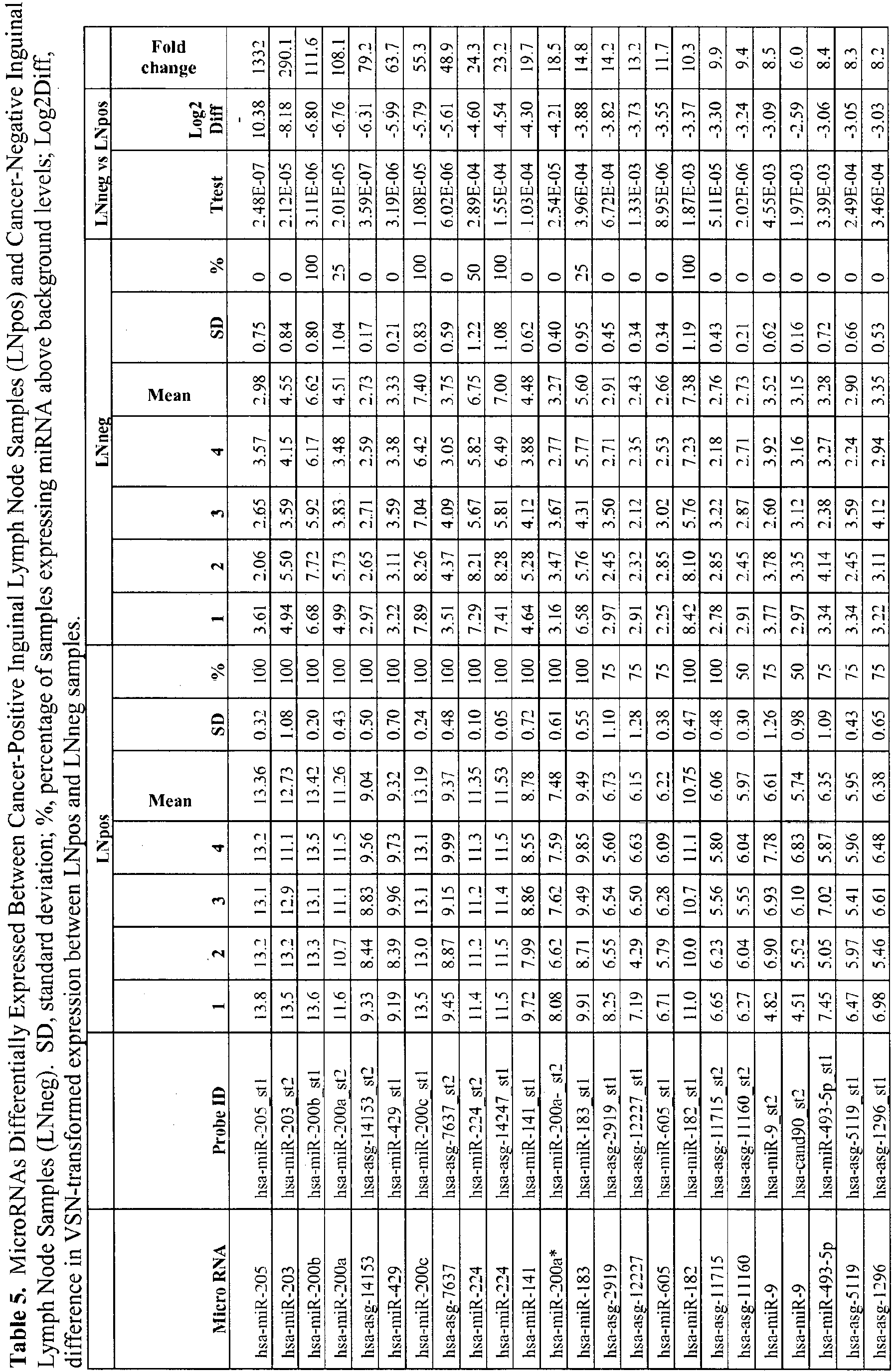

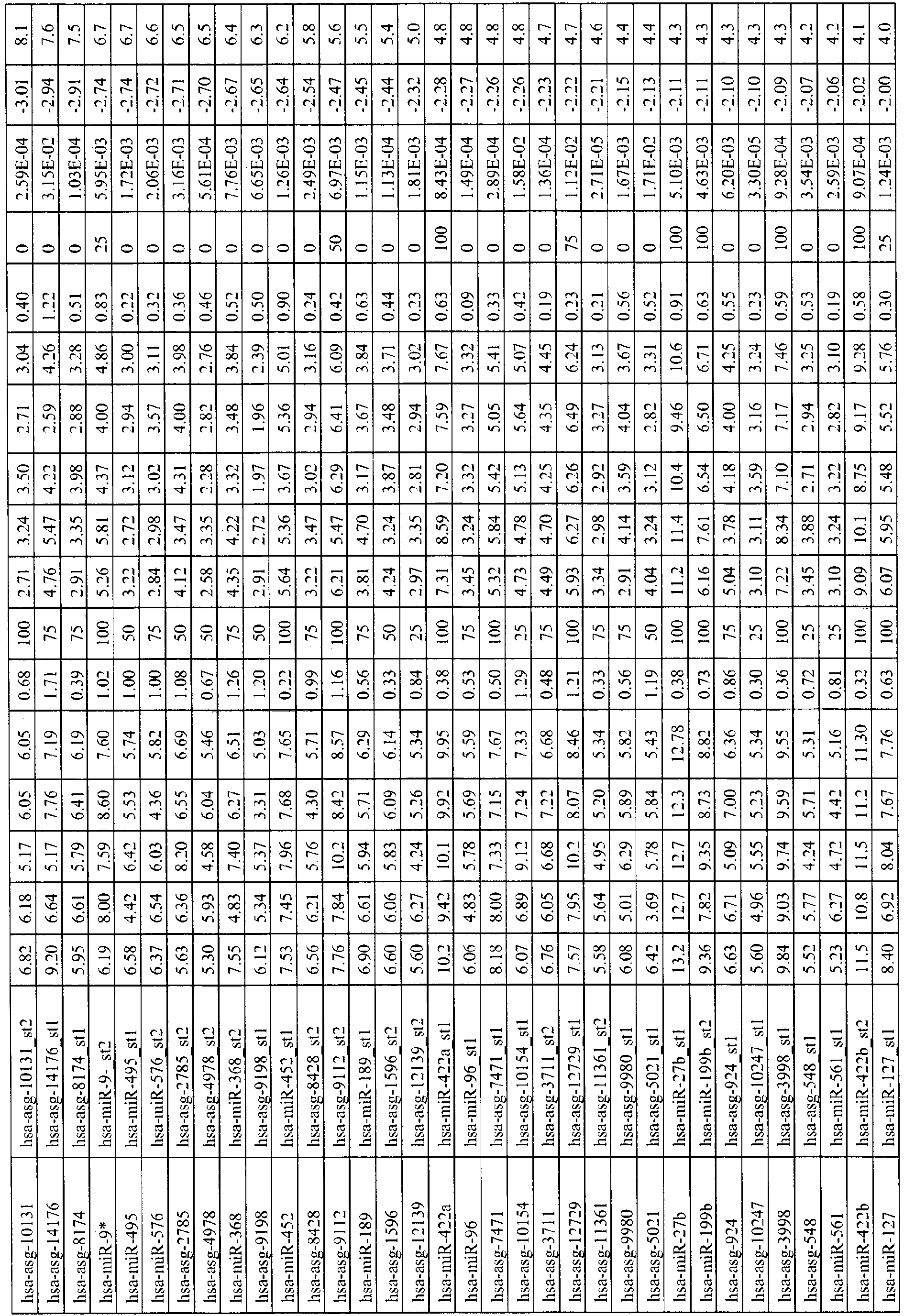

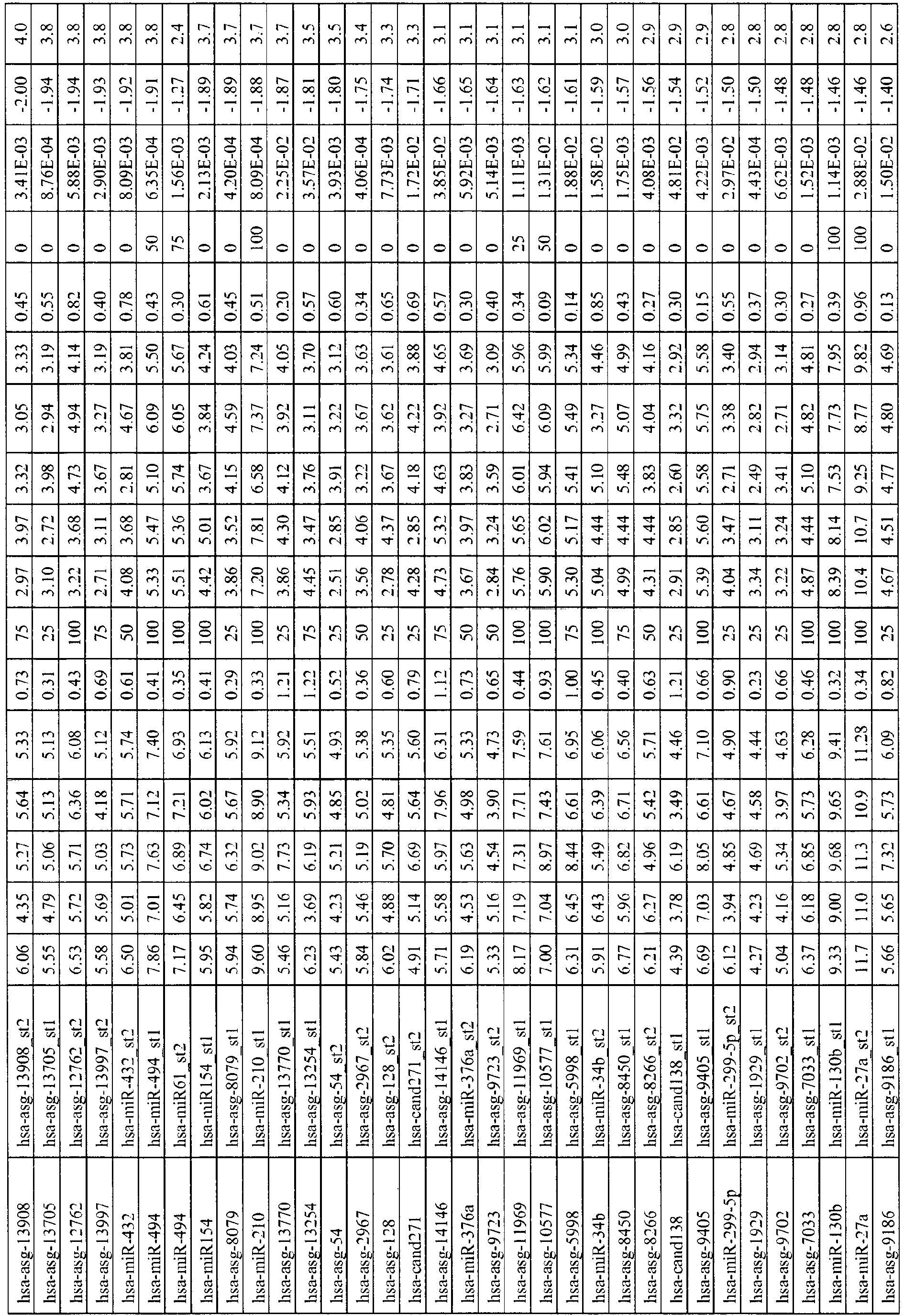

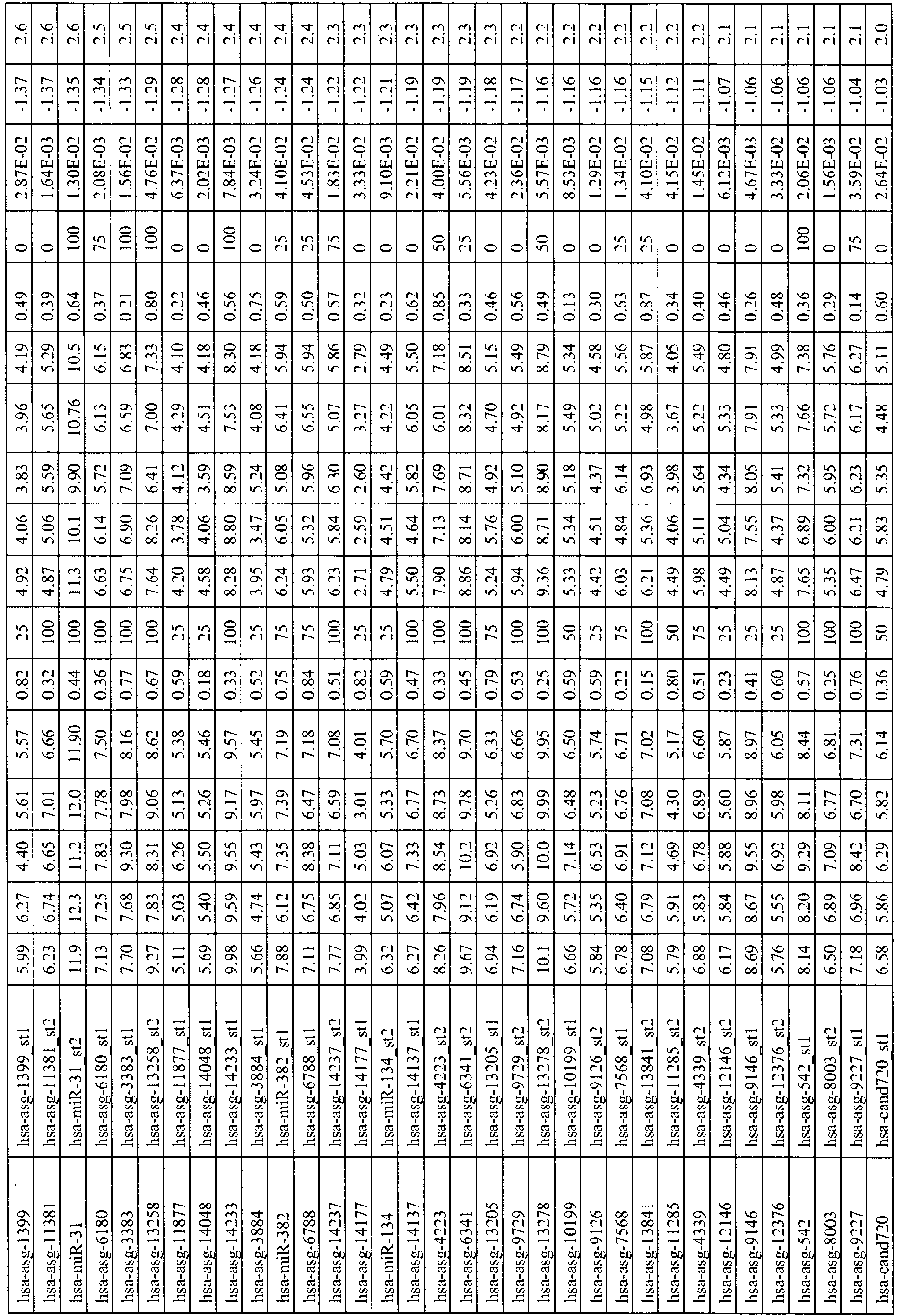

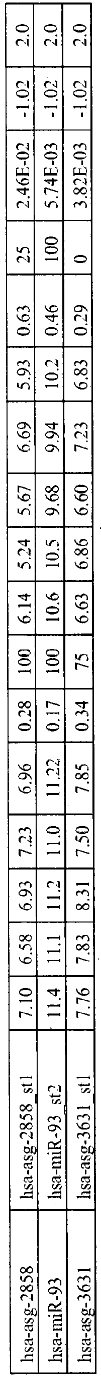

### EXAMPLE 4:

### COMMON MIRNAS DIFFERENTIALLY EXPRESSED BETWEEN NORMAL CERVIX SAMPLES AND CANCER-NEGATIVE INGUINAL LYMPH NODES AND BETWEEN CANCER-POSITIVE AND CANCER-NEGATIVE INGUINAL LYMPH NODES

The inventors determined the common miRNAs that are differentially expressed between normal cervix (NCX) samples and cancer-negative inguinal lymph nodes (LNneg) and between cancer-positive (LNpos) and cancer-negative (LNneg) inguinal lymph nodes. For this analysis, differentially expressed miRNAs were identified as having a p-value <0.05 and fold-change >2. These miRNAs represent particularly useful biomarkers for monitoring cervical cancer metastasis to inguinal lymph nodes.

The inventors found a total of 71 miRNAs to be differentially expressed between both NCX and LNneg samples and between LNpos and LNneg sample (Table 6.). Among these, the average expression levels of 26 miRNAs were at least 5-fold lower in LNneg samples than their average expression levels in either LNpos samples or NCX samples, corresponding to 18 verified miRNAs (hsa-miR-182, -183, -189, -200a, -200a*, -200b, -200c, -203, -205, -224, -368, -429, -452, -493-5p, -495, -605, -9, and -9*) and 8 predicted miRNAs (hsa-asg-8174_st1, -7637_st2, -14176_st1, -14153_st2, -1296_st1, -12227_st1, -11715_st2, and -10131_st2).

**Table 6. miRNAs differentially expressed between both normal cervix samples and cancer-negative inguinal lymph nodes and between cancer-positive and cancer-negative inguinal lymph nodes.**

| **miR Name** | **Fold Change NCX vs LNneg** | **Fold Change LNpos vs LNneg** |
|---|---|---|
| hsa-miR-205 | 413.8 | 1332.5 |
| hsa-miR-368 | 187.2 | 6.4 |
| hsa-miR-203 | 171.5 | 290.1 |
| hsa-miR-495 | 138.2 | 6.7 |
| hsa-asg-5021 | 97.3 | 4.4 |
| hsa-miR-200a | 80.6 | 108.1 |
| hsa-miR-299-5p | 78.3 | 2.8 |
| hsa-miR-493-5p | 76.2 | 8.4 |
| hsa-asg-13254 | 72.2 | 3.5 |
| hsa-miR-200b | 64.3 | 111.6 |
| hsa-miR-200c | 53.7 | 55.3 |
| hsa-miR-127 | 50.0 | 4.0 |
| hsa-miR-432 | 49.9 | 3.8 |
| hsa-asg-14153 | 42.2 | 79.2 |
| hsa-asg-14176 | 32.1 | 7.6 |
| hsa-miR-199b | 26.1 | 4.3 |
| hsa-miR-134 | 25.3 | 2.3 |
| hsa-miR-376a | 24.9 | 3.1 |
| hsa-asg-7637 | 24.3 | 48.9 |
| hsa-miR-189 | 24.1 | 5.5 |
| hsa-miR-429 | 23.7 | 63.7 |
| hsa-miR-494 | 20.8 | 3.8 |
| hsa-miR-224 | 17.7 | 24.3 |
| hsa-miR-382 | 17.4 | 2.4 |
| hsa-miR-9* | 14.9 | 6.7 |
| hsa-miR-9 | 12.4 | 8.5 |
| hsa-miR-452 | 12.2 | 6.2 |
| hsa-miR-605 | 10.6 | 11.7 |
| hsa-asg-10131 | 8.3 | 8.1 |
| hsa-miR-27b | 8.2 | 4.3 |
| hsa-miR-200a* | 7.6 | 18.5 |
| hsa-miR-183 | 7.6 | 14.8 |
| hsa-miR154 | 7.4 | 3.7 |
| hsa-asg-1296 | 7.3 | 8.2 |
| hsa-miR-182 | 7.1 | 10.3 |
| hsa-asg-7471 | 6.9 | 4.8 |
| hsa-asg-13205 | 6.6 | 2.3 |
| hsa-asg-12227 | 6.4 | 13.2 |
| hsa-asg-11715 | 5.5 | 9.9 |
| hsa-asg-924 | 5.3 | 4.3 |
| hsa-asg-14233 | 5.3 | 2.4 |
| hsa-miR-27a | 5.3 | 2.8 |
| hsa-asg-8174 | 5.2 | 7.5 |
| hsa-asg-14146 | 5.0 | 3.1 |
| hsa-asg-1596 | 4.7 | 5.4 |
| hsa-asg-9980 | 4.2 | 4.4 |
| hsa-asg-9198 | 4.2 | 6.3 |
| hsa-asg-12139 | 4.2 | 5.0 |
| hsa-asg-13705 | 4.1 | 3.8 |
| hsa-miR-576 | 4.1 | 6.6 |
| hsa-asg-11361 | 4.0 | 4.6 |
| hsa-miR-34b | 4.0 | 3.0 |
| hsa-asg-13258 | 3.9 | 2.5 |
| hsa-asg-13997 | 3.8 | 2.3 |
| hsa-asg-10247 | 3.8 | 4.3 |
| hsa-asg-8428 | 3.5 | 5.8 |
| hsa-asg-2967 | 3.4 | 3.4 |
| hsa-miR-422a | 3.4 | 4.8 |
| hsa-asg-548 | 3.2 | 4.2 |
| hsa-miR-561 | 3.2 | 4.2 |
| hsa-miR-422b | 3.2 | 4.1 |
| hsa-miR-141 | 3.2 | 19.7 |
| hsa-cand720 | 3.1 | 2.0 |
| hsa-asg-3998 | 2.7 | 4.3 |
| hsa-asg-11877 | 2.7 | 2.4 |
| hsa-asp-9723 | 2.6 | 3.1 |
| hsa-asp-14048 | 2.6 | 2.4 |
| hsa-miR-31 | 2.6 | 2.6 |
| hsa-asg-11285 | 2.5 | 2.2 |
| hsa-asg-8266 | 2.4 | 2.9 |
| hsa-asg-1399 | 2.3 | 2.6 |

### EXAMPLE 5:

### QRT-PCR VERIFICATION OF DIFFERENTIALLY EXPRESSED MICRO-RNAS IN CANCER-POSITIVE AND CANCER-NEGATIVE LYMPH NODES FROM CERVICAL CANCER PATIENTS

The inventors also evaluated the differential expression of selected miRNAs in LNpos and LNneg samples (described in Example 1, Table 3) by qRT-PCR. qRT-PCR reactions were performed using TaqMan® MicroRNA Assays (Applied Biosystems; Foster City, CA, USA) specific for eleven of the most differentially expressed miRNAs between LNpos and LNneg samples and between NCX vs LNneg samples (hsa-miR-205, -203, -429, - 200a, -200c, -141, -183, -182, -189, -200b and -224). Assays were performed according to the manufacturer's instructions with 15 ng of input total RNA from one of eight samples (LNpos 1-4, LNneg 1-4) and were incubated in a 7900HT Fast Real-Time PCR system (Applied Biosystems, Foster City, CA, USA). Initial data analysis was done using the 7500 Fast System SDS V2.3 software (Applied Biosystems). miR-16, whose expression level did not change between the different samples, was used for normalization.

Results of the qRT-PCR assays revealed that differences in expression levels of the 11 miRNAs, between LNpos and LNneg samples were conserved and were very similar to those observed by microarray analysis (FIGs. 1A - 1B).

These data demonstrate that both qRT-PCR and microarray analysis are effective methods for quantifying miRNA expression when evaluating metastasis of cervical cancer to inguinal lymph nodes.

### EXAMPLE 6:

### QRT-PCR VERIFICATION OF DIFFERENTIALLY EXPRESSED MICRO-RNAS BY ANALYSIS OF ADDITIONAL CANCER-POSITIVE AND CANCER-NEGATIVE LYMPH NODES FROM CERVICAL CANCER PATIENTS

A second set of FFPE tissue samples, distinct from those described in Table 3 above, was purchased from ProteoGenex. Samples were prepared from eight cancer-positive (LNpos5-12) and four cancer-negative (LNneg5-8) lymph nodes that were obtained from twelve different patients with cervical squamous cell carcinoma (cervical cancer) (Table 7). A pathology report and hematoxilin-eosin (H&E)-stained slide were available for each sample. H&E-stained slides were reviewed by a pathologist at Asuragen to confirm lymph node statuses as cancer cell-positive or cancer cell-negative.

**Table 7. Patient and tissue sample pathology information for lymph nodes from cervical cancer patients. Lymph nodes from four patients were cancer cell-negative (LNneg), and lymph nodes from eight patients were cancer cell-positive (LNpos). SCC, squamous cell carcinoma.**

| **Sample ID** | **Patient Age (yr) Ethnicity** | **Cancer Diagnosis** | **Cervix Tumor Histological Diagnosis** | **TNM Stage** (Sobin and Wittekind, 2002) | **AJCC Stage** (Greene, 2002) | **Lymph Node Metastases** |
|---|---|---|---|---|---|---|
| LNneg5 | 24 Caucasian | normal | squamous cell carcinoma moderately differentiated | T1a2N0M0 | IA2 | 0/4 |
| LNneg6 | 44 Caucasian | normal | squamous cell carcinoma moderately differentiated | T1b1N0M0 | IB1 | 0/10 |
| LNneg7 | 44 Caucasian | normal | squamous cell carcinoma moderately differentiated | T1b1N0M0 | IB1 | 0/6 |
| LNneg8 | 58 Caucasian | normal | squamous cell carcinoma poorly differentiated | T1B1N0M0 | IB1 | 0/8 |
| LNpos5 | 31 Caucasian | metastatic | squamous cell carcinoma moderately differentiated | T1B1N1M0 | IIIB | 2/5 |
| LNpos6 | 40 Caucasian | metastatic | squamous cell carcinoma moderately differentiated | T1B1N1M0 | IIIB | 1/5 |
| LNpos7 | 21 Caucasian | metastatic | squamous cell carcinoma poorly differentiated | T1B1N1M0 | IIIB | 2/9 |
| LNpos8 | 56 Caucasian | metastatic | squamous cell carcinoma moderately differentiated | T1b1N1M0 | IIIB | 3/3 |
| LNpos9 | 72 Caucasian | metastatic | squamous cell carcinoma poorly differentiated | T1b1N1M0 | IIIB | 2/8 |
| LNpos10 | 25 Caucasian | metastatic | squamous cell carcinoma moderately differentiated | T2bN1M1 | IVB | 2/7 & ovary |
| LNpos11 | 32 Caucasian | metastatic | squamous cell carcinoma poorly differentiated | T1B1N1M0 | IIIB | 3/10 |
| LNpos12 | 58 Caucasian | metastatic | squamous cell carcinoma moderately differentiated | T2bN1M0 | IIIB | 2/4 |

The differential expression of selected miRNAs was evaluated in LNpos5-12 and LNneg5-8 samples by qRT-PCR. qRT-PCR reactions were performed using TaqMan® MicroRNA Assays (Applied Biosystems) specific for eleven miRNAs (hsa-miR-205, -203, - 429, -200a, -200c, -141, -183, -182, -189, -200b, and -224) that are among those most differentially expressed between LNpos1-4 and LNneg1-4 samples and between NCX vs LNneg1-4 samples (Table 6). Assays were performed with 15 ng of input total RNA from one of twelve samples (LNpos 5-12, LNneg 5-8), and data were analyzed as described above in Example 5.

Differential expression was confirmed for seven of the eleven miRNAs tested (FIG. 2B). The seven miRNAs (miR-205, -203, -429, -200b, -200a, -200c, and -141) were expressed at levels that were at least 100-fold lower in the cancer-negative lymph node samples (LNneg5-8) than in the cancer-positive lymph node samples (LNpos5-12) and correspond to the same miRNAs that were identified as most differentially-expressed by qRT-PCR between lymph node samples LNpos1-4 and LNneg1-4 (FIG. 2A, FIG. 2D). Differential expression of four of the miRNAs was not observed by qRT-PCR quantification with this set of lymph node samples (LNpos5-12 and LNneg5-8). These four miRNAs were the least differentially-expressed between lymph nodes LNpos1-4 and LNneg1-4, with fold changes varying between 10- and 35-fold (FIG. 2A, FIG. 2D).

Quantification of these miRNAs by qRT-PCR in an independent set of lymph nodes from cervical cancer patients validates their differential expression in cancer-positive and cancer-negative lymph nodes. The seven miRNAs (miR-205, -203, -429, -200b, -200a, - 200c, and -141) represent useful markers for the identification of cervical tumor cells in lymph nodes of cervical cancer patients (FIG. 2C).

### EXAMPLE 7:

### IDENTIFICATION OF CLASSIFIERS FOR CANCER-POSITIVE LYMPH NODES IN PATIENTS WITH METASTATIC CERVICAL CARCINOMA

The inventors have demonstrated that miRNA quantification and expression analysis can differentiate cancer-positive and cancer-negative lymph nodes from the inguinal region of cervical cancer patients. To further demonstrate the utility of specific miRNA markers as diagnostic analytes, the inventors quantified specific combinations of miRNAs by qRT-PCR to determine if any such combinations enhanced the distinction between cancer-positive and cancer-negative lymph nodes from cancer patients. qRT-PCR assays were performed as described in Example 5 above. For miRNA combinations quantified by qRT-PCR, raw Ct values from the quantification of individual miRNAs were added then the Ct value of miR-16 was subtracted. For miRNA combinations quantified by microarray expression analysis, ΔH values for each individual miRNA were added. The inventors determined that quantification of several miRNA combinations (miR-205+miR-429; miR-205+miR-203; miR-429+miR-203; miR-425+miR-203+miR-205) enhances the distinction between cancer positive and cancer-negative lymph nodes from cervical cancer patients (FIG. 3).

These combinations serve as non-limiting examples. Combinations of qRT-PCR or microarray data for other microRNAs will serve to enhance the distinction between cancer-positive and cancer-negative lymph nodes. Combinations may include subtraction of average Ct values. In that case, if an even number of miRNAs is present in the combination, the subtraction of Ct values in a combination eliminates the need to normalize the data.

### EXAMPLE 8:

### DETECTION OF RNA FROM CERVICAL CANCER CELLS IN A BACKGROUND OF CANCER-NEGATIVE LYMPH NODE RNA

Routine, histological examination of lymph nodes for the presence of cancer metastasis utilizes light microscopic examination of formalin-fixed, paraffin-embedded (FFPE) lymph node tissue that has been stained with hematoxylin and eosin (H&E). The sensitivity of this technique for detecting cancer is approximately 1 cancer cell per 100 normal cells.

First, the inventors evaluated the sensitivity of detection of cervical cancer miRNA markers using qRT-PCR. Sensitivities of detection were determined for two synthetic miRNAs (hsa-miR-203 and hsa-miR-205) that were shown by microarray analysis to be overexpressed by ≥1,000 fold (>10 ΔCt) in cancer-positive lymph nodes (LNpos) when compared with their expression in cancer-negative lymph nodes (LNneg). Synthetic miRNAs (0-10⁸ copies) were mixed with 15 ng of total RNA from a cancer-negative lymph node, and qRT-PCR reactions specific for each miRNA were performed using TaqMan® MicroRNA Assays (Applied Biosystems). The results of these analyses (FIG. 4) indicate that the limit of detection of miR-205 and miR-203 by qRT-PCR in this assay of the invention is ∼1 cancer cell per ∼10, 000 cancer-negative lymph node cells.

Next, the inventors determined the sensitivities of detection for hsa-miR-205, - 429, -200a, -200c, and -141 following the serial 10-fold dilution (1:10; 1:100; 1:1,000; 1:10,000, 1:100,000, and 1:1,000,000) of total RNA from cervical cancer cells (TUM) into a background of total RNA (15 ng) from a cancer-negative lymph node (LNneg). qRT-PCR reactions specific for the each indicated miRNA were performed using TaqMan® MicroRNA Assays (Applied Biosystems). A ΔCt of at least 3.3 (10-fold) from that for the LNneg sample was required for a difference in Ct values to be considered statistically significant. Results of these analyses (FIG. 5A) indicate that the sensitivity of detection was the 1:100 dilution for miR-205, -429, and -200a and the 1:10 dilution for miR-200c and -141.

Finally, the inventors determined the sensitivities of detection for hsa-miR-205, - 429, -200a, -200c, and -141 following the serial 10-fold dilution (1:10; 1:100; 1:1,000; 1:10, 000, 1:100,000) of total RNA from a cancer-positive lymph node, containing -70% cancerous cells, into a background of total RNA (15 ng) from a cancer-negative lymph node. (FIG. 5B). Results of these analyses indicate that the sensitivity of detection was the 1:100 dilution for mir-205 and miR-429 and the 1:10 dilution for miR-200a, -200c, and -141.

### EXAMPLE 9:

### MELANOMA SAMPLES AND RNA ISOLATION

Three melanoma (MEL1-3) (Table 8), four cancer-positive lymph node (LNpos1A and LNpos2B-4B), and seven cancer-negative lymph node (LNneg1-7) (Table 9) frozen tissue samples were purchased from ProteoGenex (ProteoGenex, Inc.; Culver City, CA, USA). Melanoma samples were from the chest or shoulder regions of patients, and ten of eleven lymph nodes were axillary, to match the origin of the tumors. All the samples were from different patients. Total RNA isolation from the melanoma and lymph node tissue samples was performed using the *mir*Vana™ miRNA Isolation Kit (Ambion; cat. no. AM1560), following the manufacturer's protocol. Purified total RNA from all samples was quantified using a Nanodrop® ND-1000 (NanoDrop Technologies).

An independent set of three melanoma (MEL4-6) and four cancer-positive lymph node (LNpos5-8) formalin-fixed, paraffin-embedded (FFPE) tissue samples were purchased from ProteoGenex (ProteoGenex, Inc.; Culver City, CA, USA). Total RNA isolation from these samples was performed using the RecoverAll™ Total Nucleic Isolation Kit (cat. No. AM 1975; Ambion, Inc.; Austin, TX, USA) as described above in Example 1. Purified total RNA was quantified using a Nanodrop® ND-1000 (Nanodrop Technologies; Wilmington, DE, USA). A hematoxilin-eosin (H&E)-stained slide from each sample was reviewed by a pathologist at Asuragen who confirmed the presence of cancer cells in the lymph nodes.

**Table 8. Patient and tissue pathology information for melanoma tissue samples. TNM Stage (Sobin and Wittekind, 2002); Clark's Level (Clark et al., 1969); Breslow Micro-staging (Breslow, 1970). MEL1-3 were provided as frozen biopsies. MEL4-6 were provided as FFPE samples. LN Mets, number of lymph nodes with cancer metastasis/total number of lymph nodes taken.**

| **Sample** | **Patient Age (yr) Sex Ethnicity** | **Histological Diagnosis** | **TNM Stage** | **Clinical Stage Group** | **LN Mets** | **Clark's Level** | **Breslow Micro-Staging** |
|---|---|---|---|---|---|---|---|
| MEL1 | 70 Male Caucasian | pigmented melanoma, epithelia cell type, left shoulder | T3aN0M0 | IIA | No | IV | 4mm |
| MEL2 | 47 Male Caucasian | pigmented melanoma, epithelioid cell type, anterior chest wall | T4bN3M0 | III | 7/9 | V | 20 mm |
| MEL3 | 48 Female Caucasian | poorly pigmented melanoma, spindle cell type, scapula | T4bN2M0 | III | 2/9 | II | 8 mm |
| MEL4 | 17 Female Caucasian | melanoma, epithelioid cell type | T4aN0M0 | IIB | No | V | 6 mm |
| MEL5 | 37 Female Caucasian | poorly pigmented melanoma, epithelioid cell type, shoulder | T4bN1M0 | IIB | 1/9 | V | 11 mm |
| MEL6 | 67 Female Caucasian | poorly pigmented, malignant nodular carcinoma,, epithelioid type, shoulder | T4bN0M0 | IIC | No | IV | 7 mm |

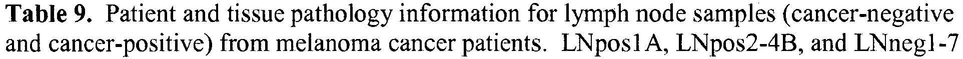

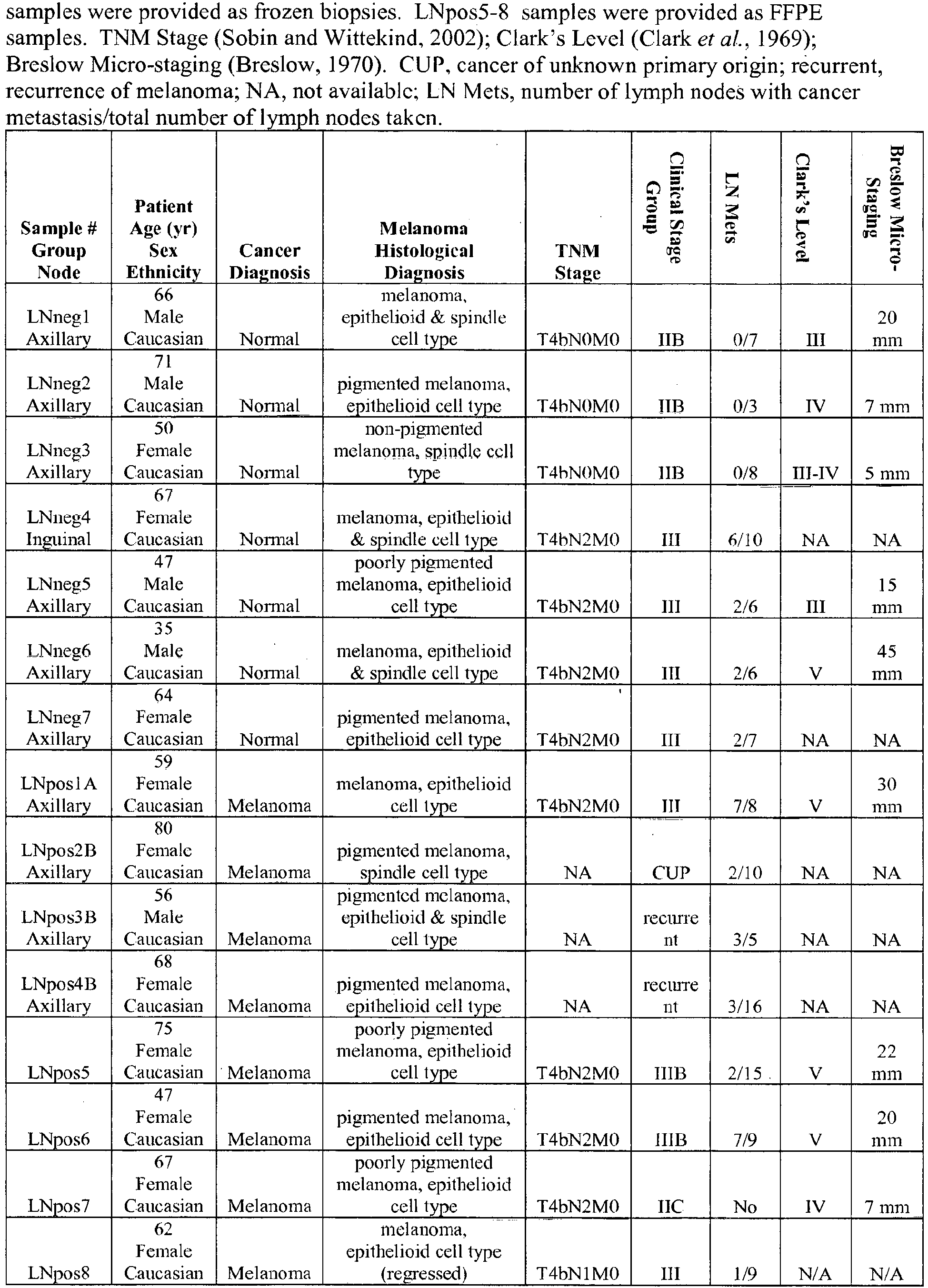

### EXAMPLE 10:

### IDENTIFICATION OF MIRNAS DIFFERENTIALLY EXPRESSED BETWEEN MELANOMA SAMPLES AND CANCER-NEGATIVE LYMPH NODE SAMPLES

To identify miRNAs that represent useful markers for metastasis of melanoma cancers to lymph nodes, the inventors identified miRNAs that are expressed at significantly higher levels in melanoma samples from patients with melanoma than in cancer-negative lymph nodes from patients with melanoma.

miRNA expression was determined for two melanoma samples (MEL1, MEL3, described in Example 9 and Table 8 above) and seven cancer-negative lymph nodes (LNnegl-7, described in Example 9 and Table 9 above). For each tissue sample, microRNA-containing fractions were purified from 10 µg of total RNA using a flashPAGE™ Fractionation Apparatus (Ambion) and analyzed with microarrays as described above in Example 2.

The inventors identified 90 human miRNAs (34 verified and 56 predicted) with average expression levels that were significantly higher in the two melanoma (MEL) samples than in cancer-negative lymph nodes (LNneg) (log2Diff(MEL vs LNneg)≥1 and p-value<0.05) (Table 10). Among the verified miRNAs, the average expression level of hsa-miR-205 was 260-fold higher in MEL samples than in LNneg; the average expression levels of four miRNAs (hsa-miR-203, -211, -509, and -513) were between 60- and 160-fold higher in MEL samples than in LNneg samples; four miRNA (hsa-miR-200c, -132, -506, and -508) had average expression levels between 10- and 15- fold higher in MEL than in LNneg samples, and 11 miRNAs had average expression levels between 5- and 10-fold higher in MEL samples (Table 10). In addition, 19 miRNAs had average expression levels that were lower in MEL samples than in LNneg samples. Among these, one (hsa-miR-1) was expressed at a level at least 5-fold lower in MEL samples (Table 10). Among the predicted miRNAs, one (hsa-asg-5638_st2) was expressed at a level more than 100-fold higher in MEL samples compared to LNneg samples, and seven miRNAs (hsa-asg-11338_st2, -5630_st2,-13997_st2, -8428_st2, -9186_st1, -1296_st1, and -9723_st2) had average expression levels between 5- and 25- fold higher in MEL samples (Table 10).

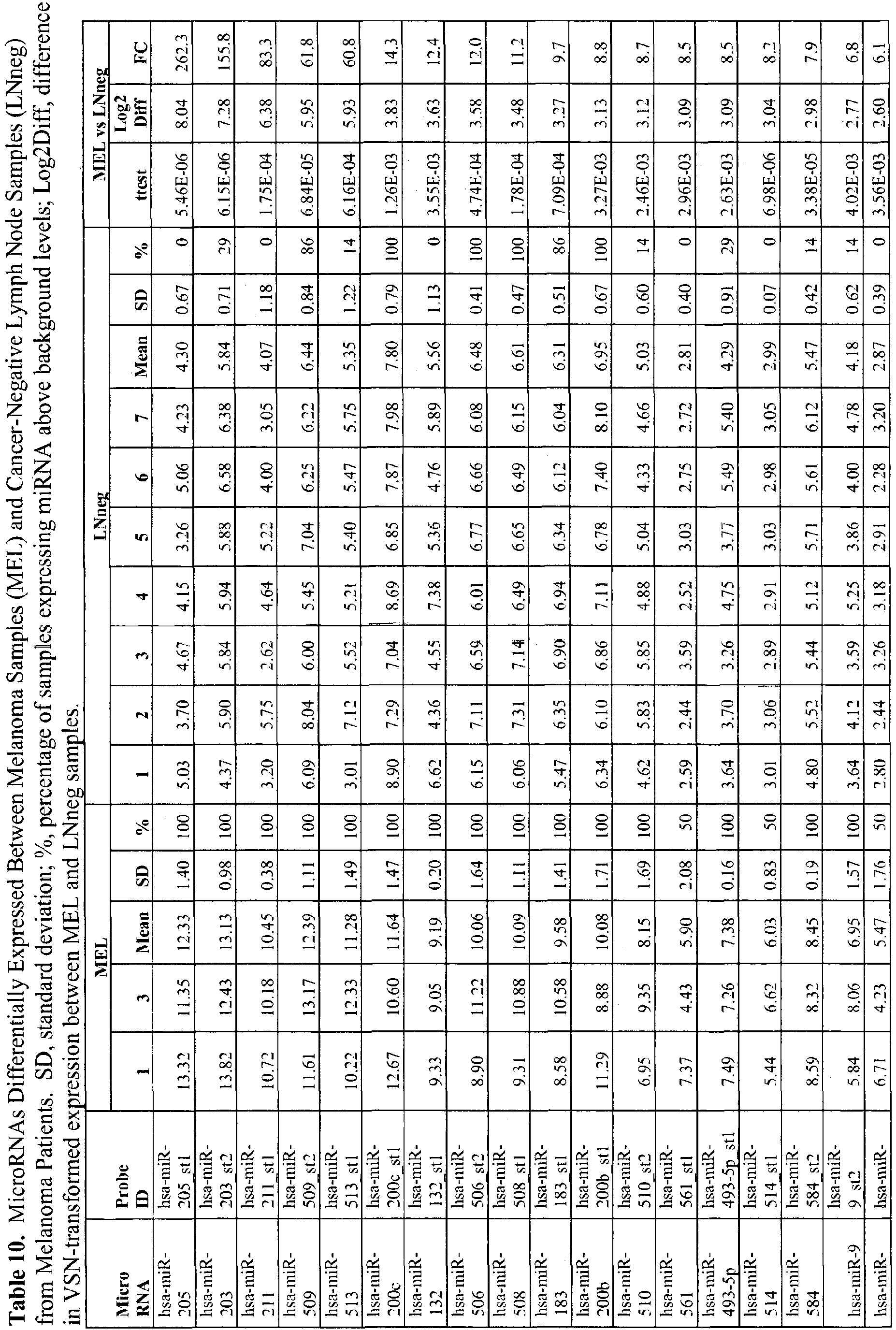

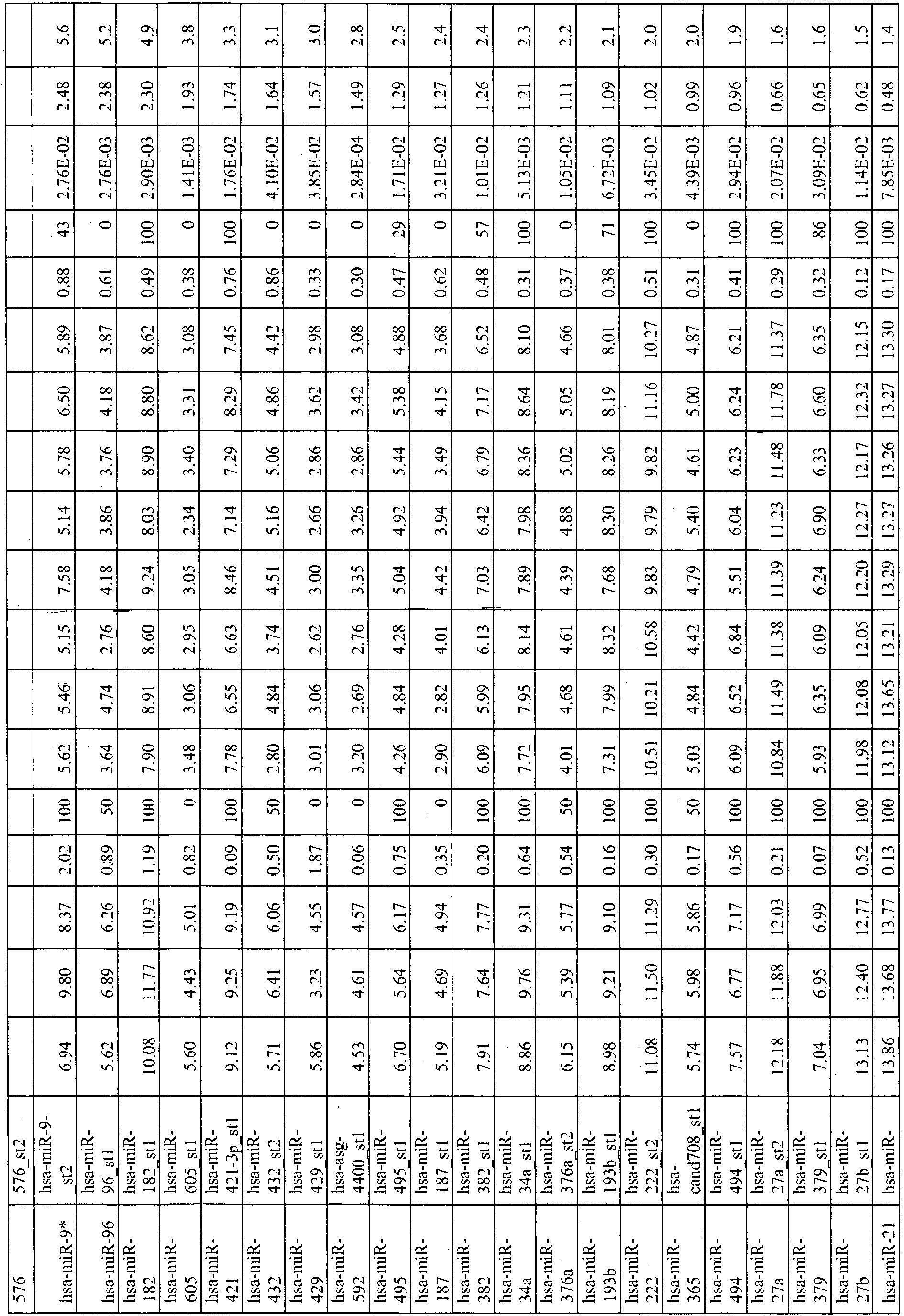

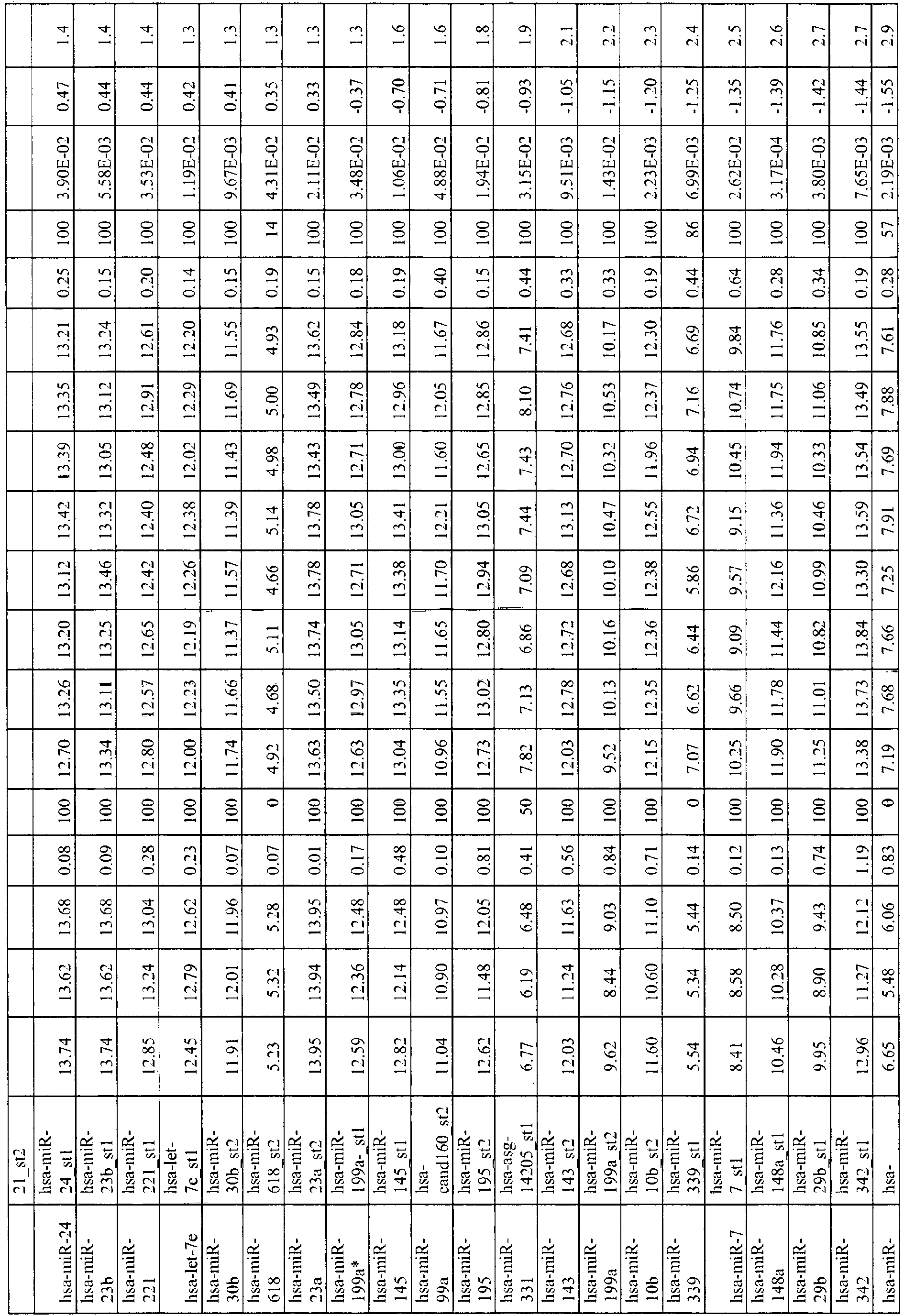

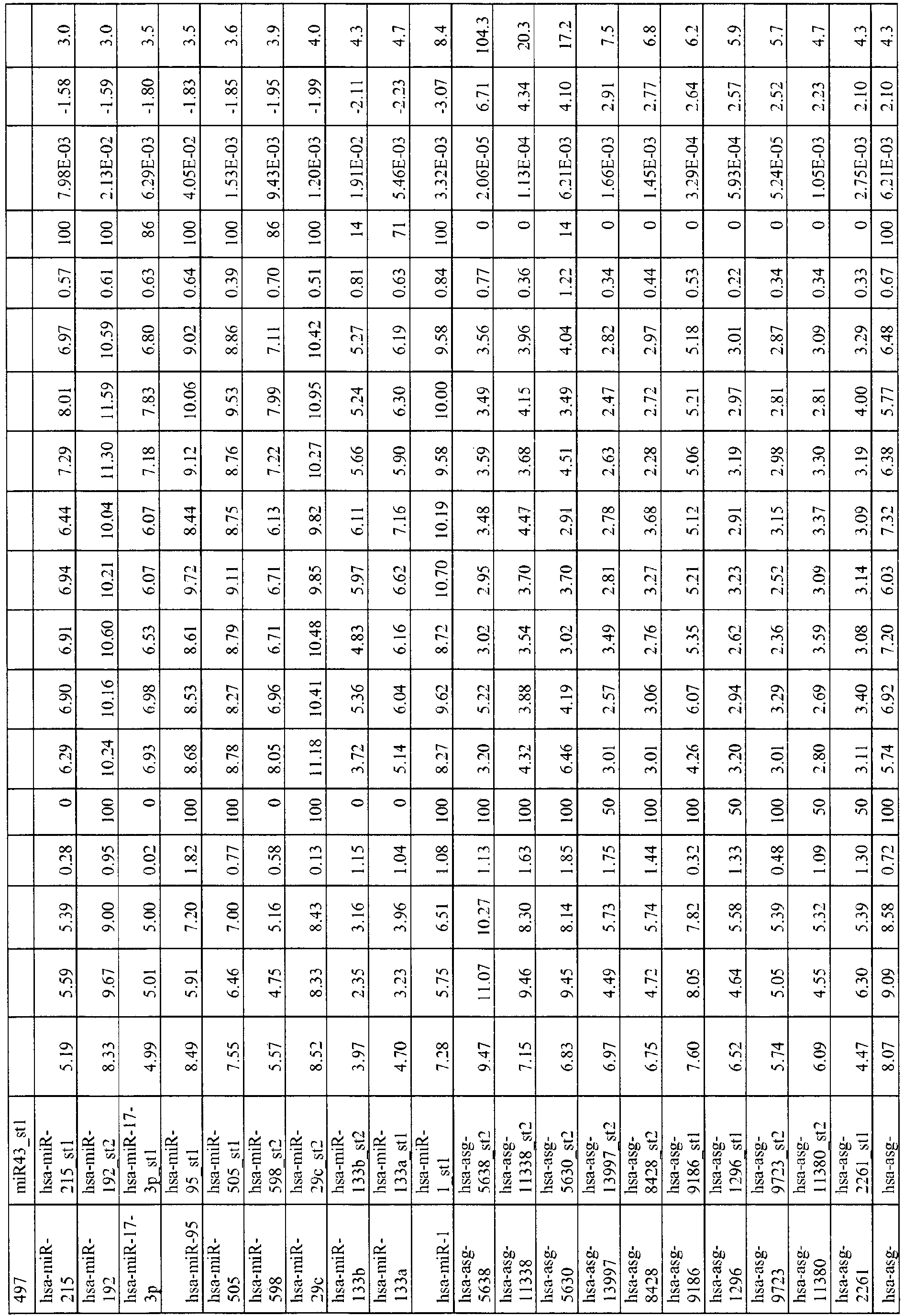

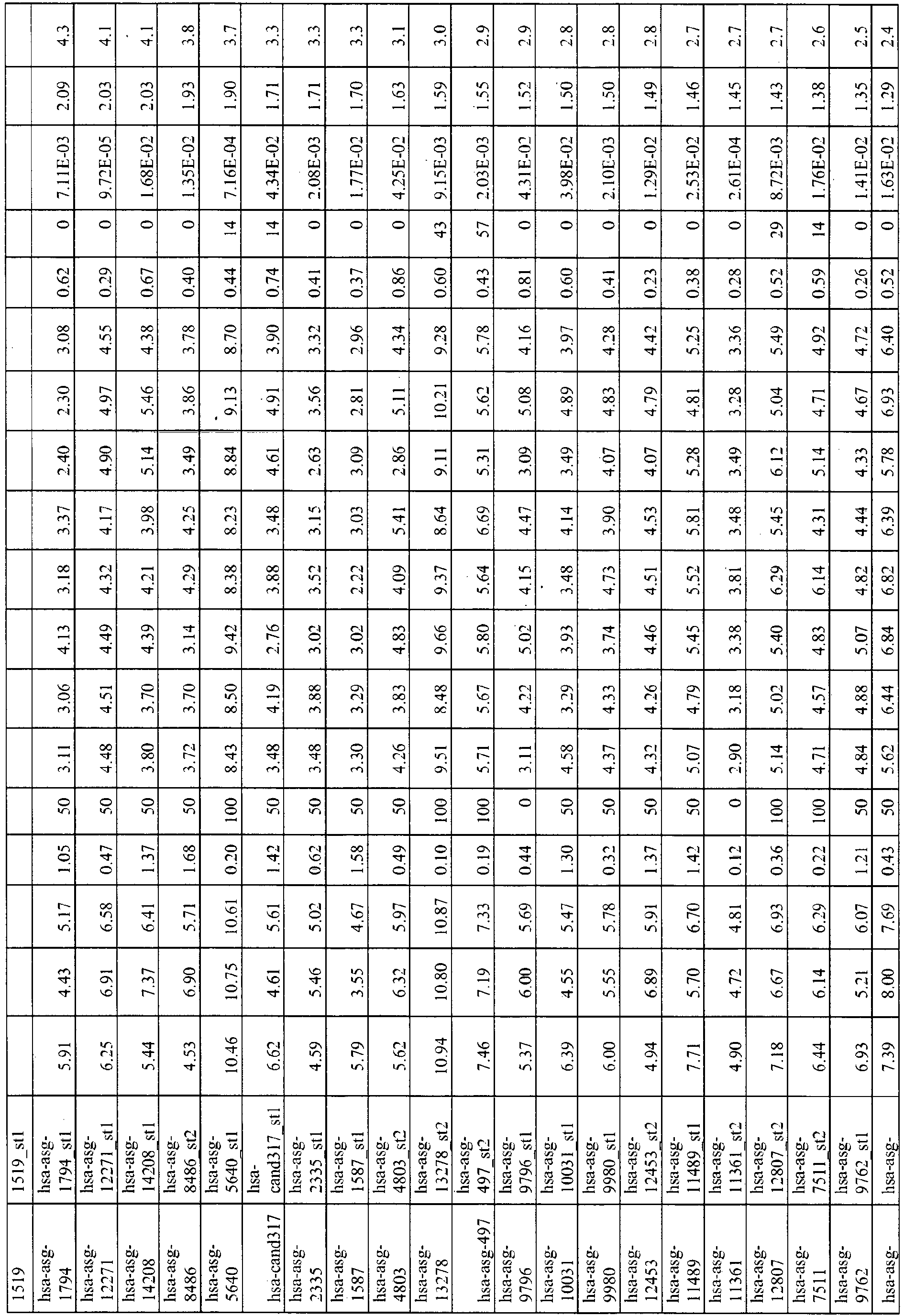

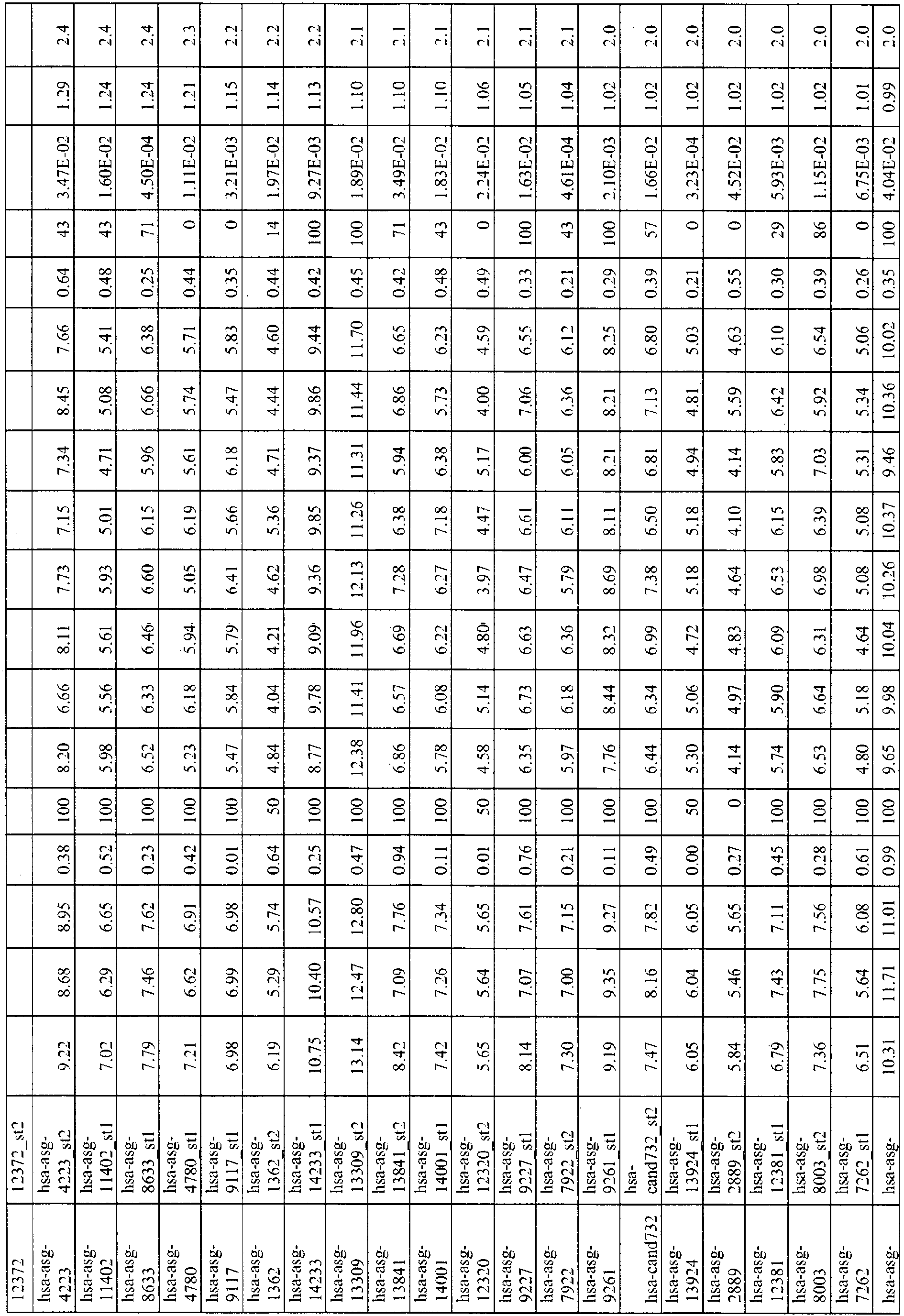

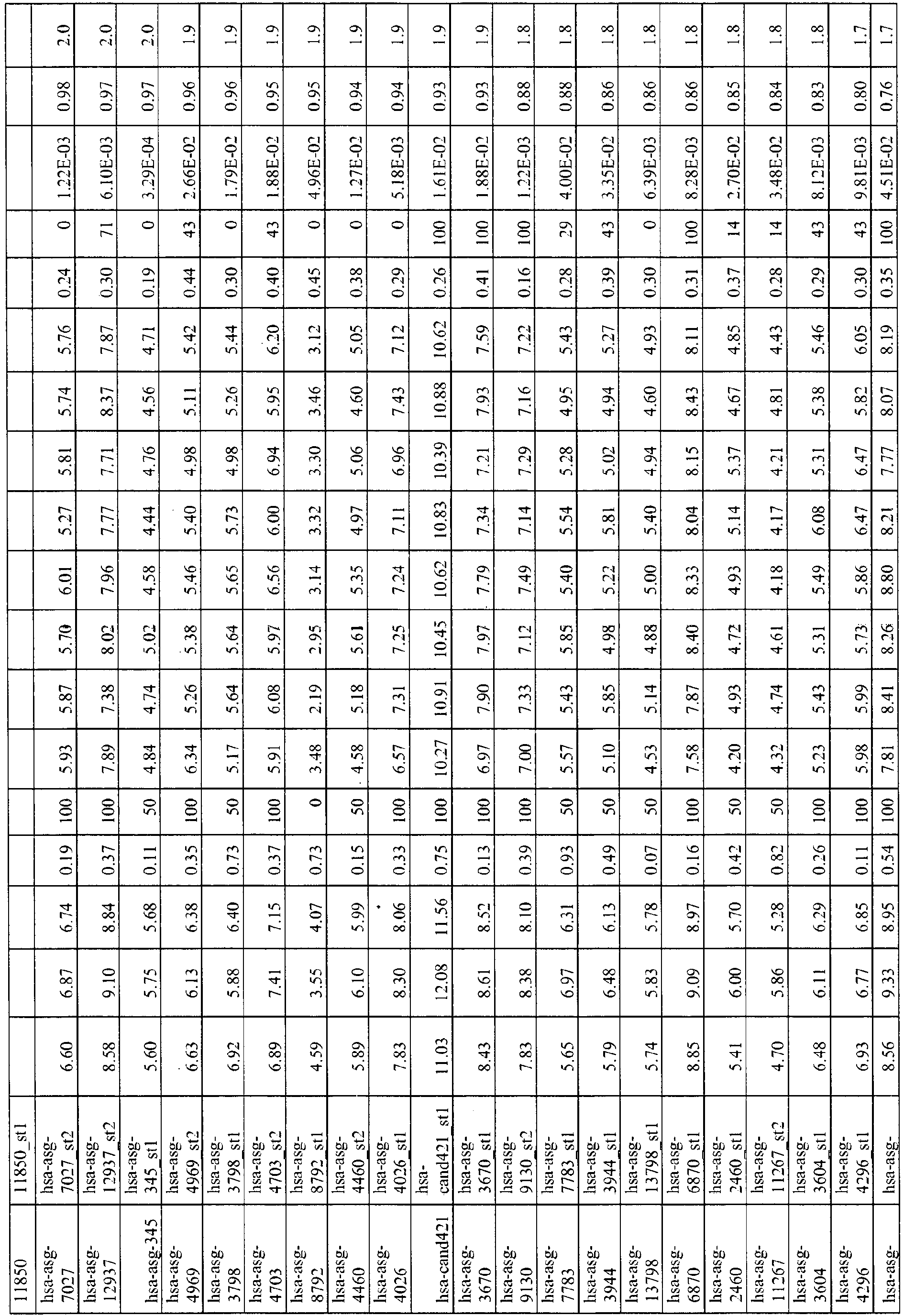

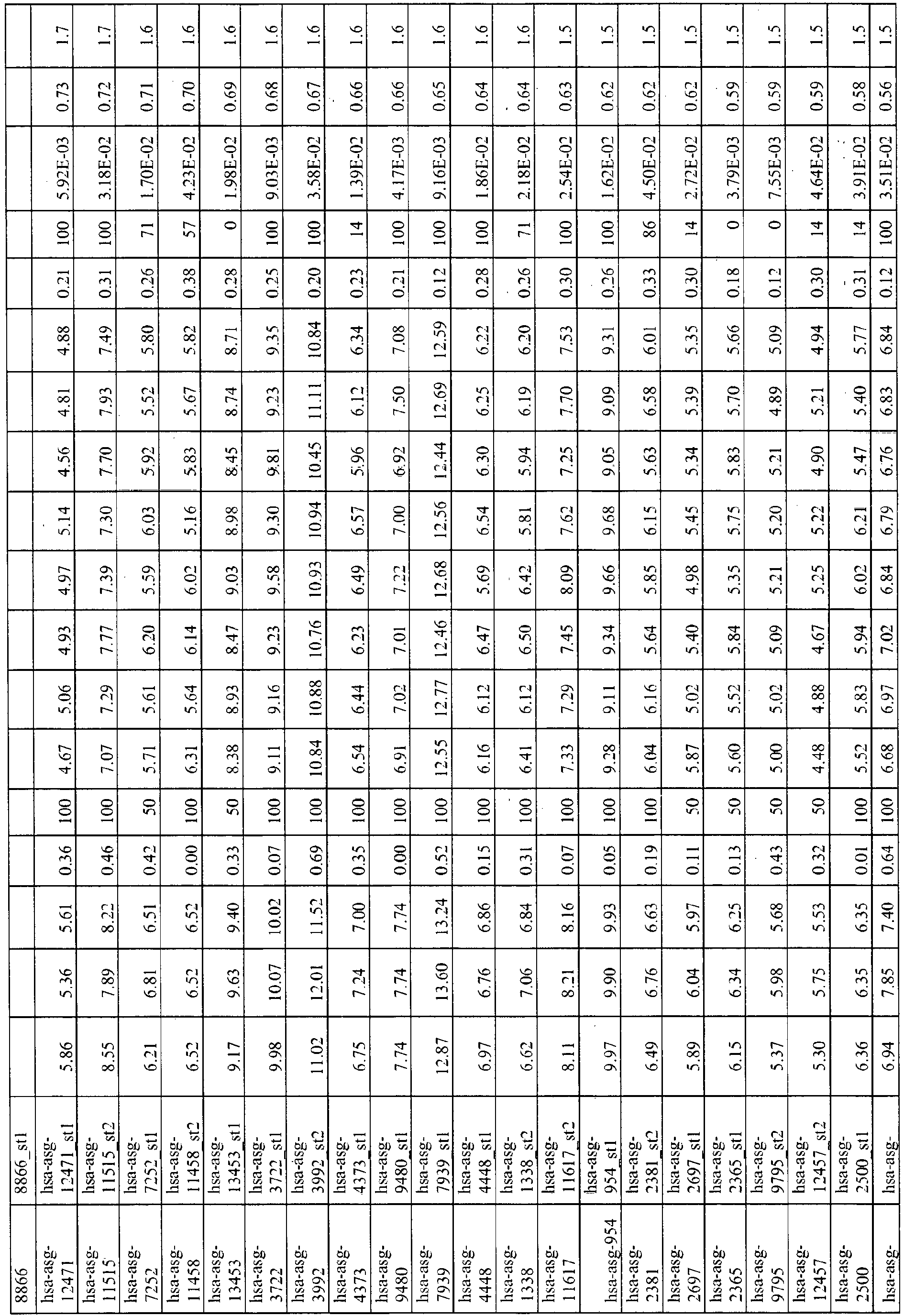

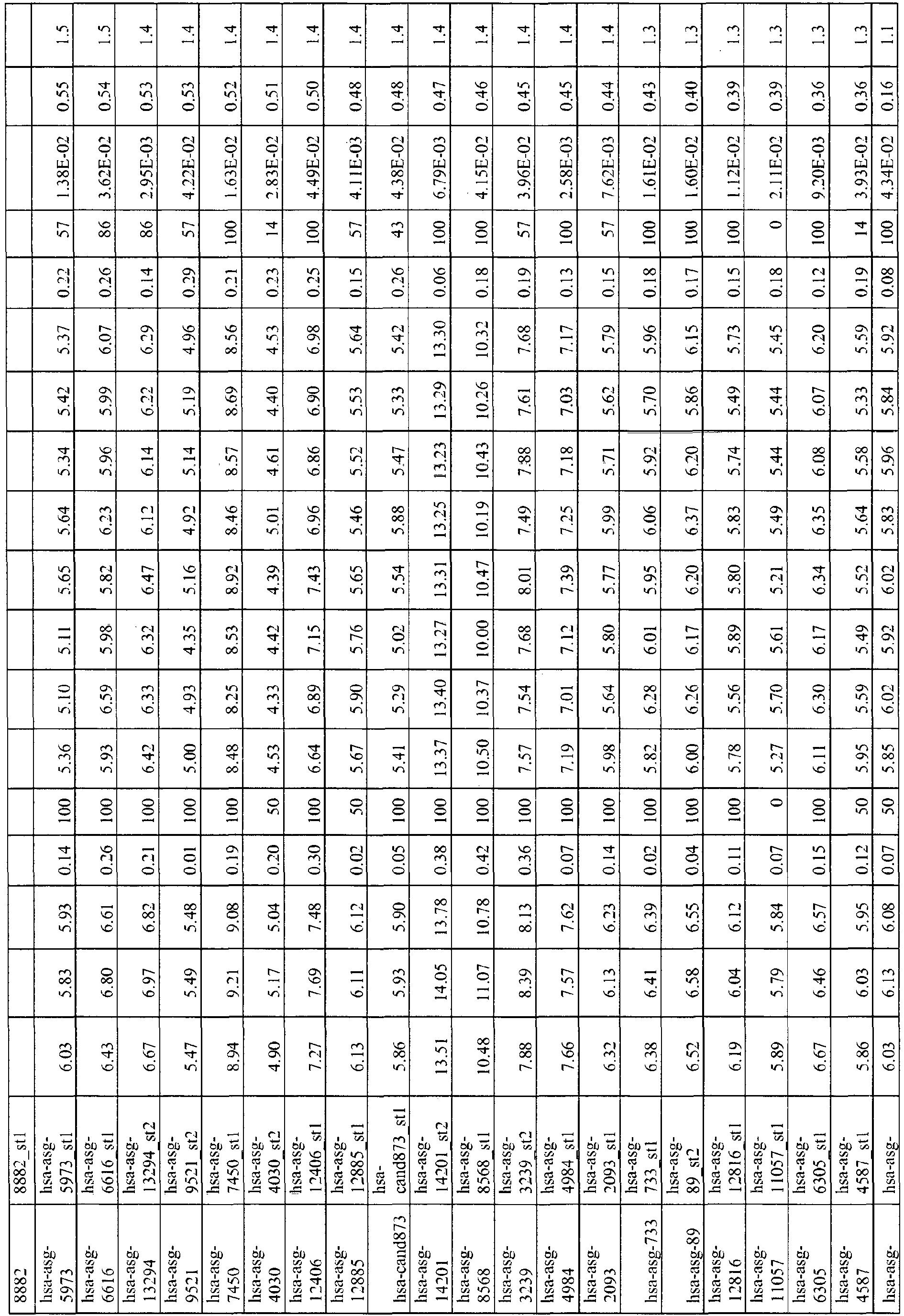

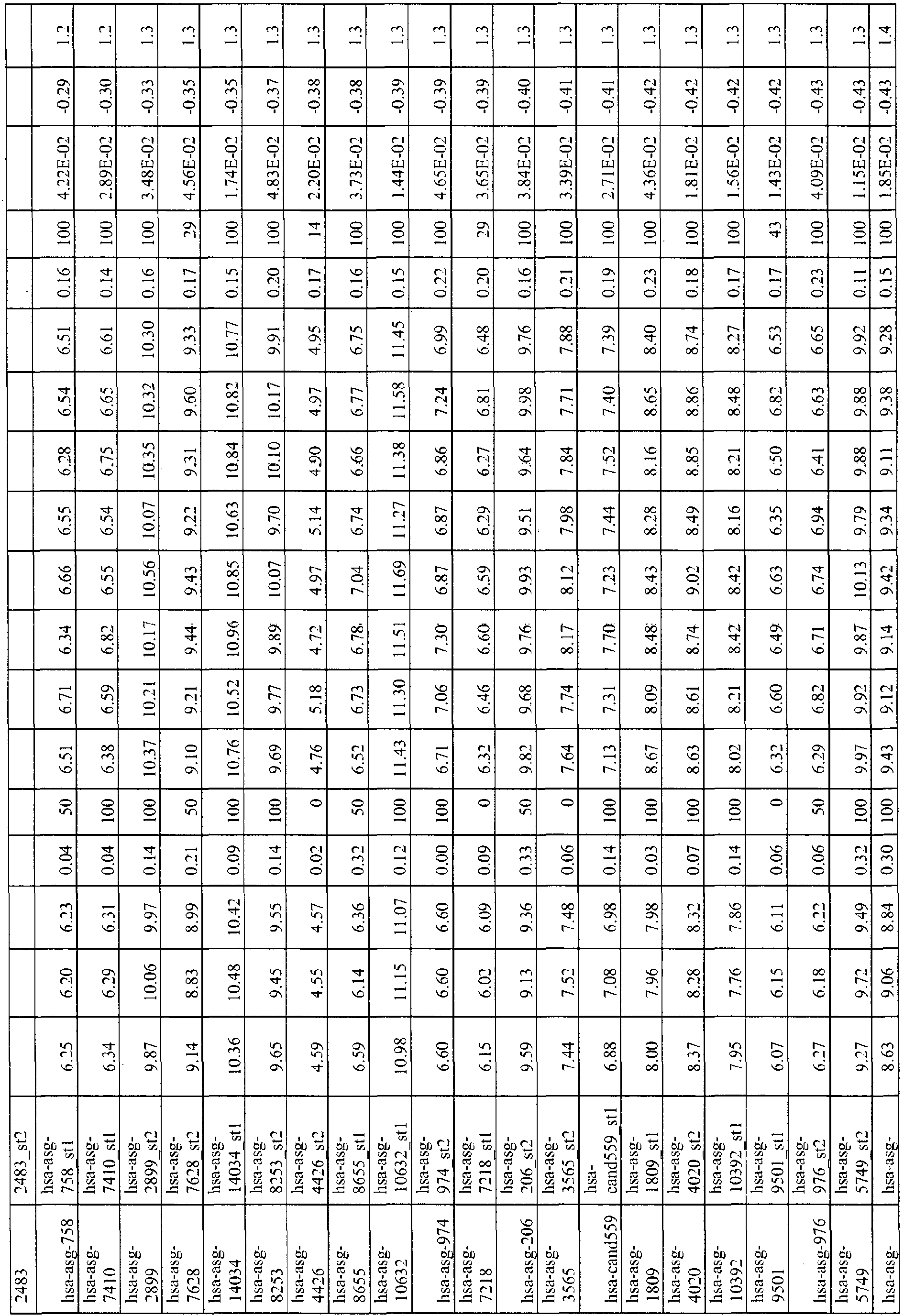

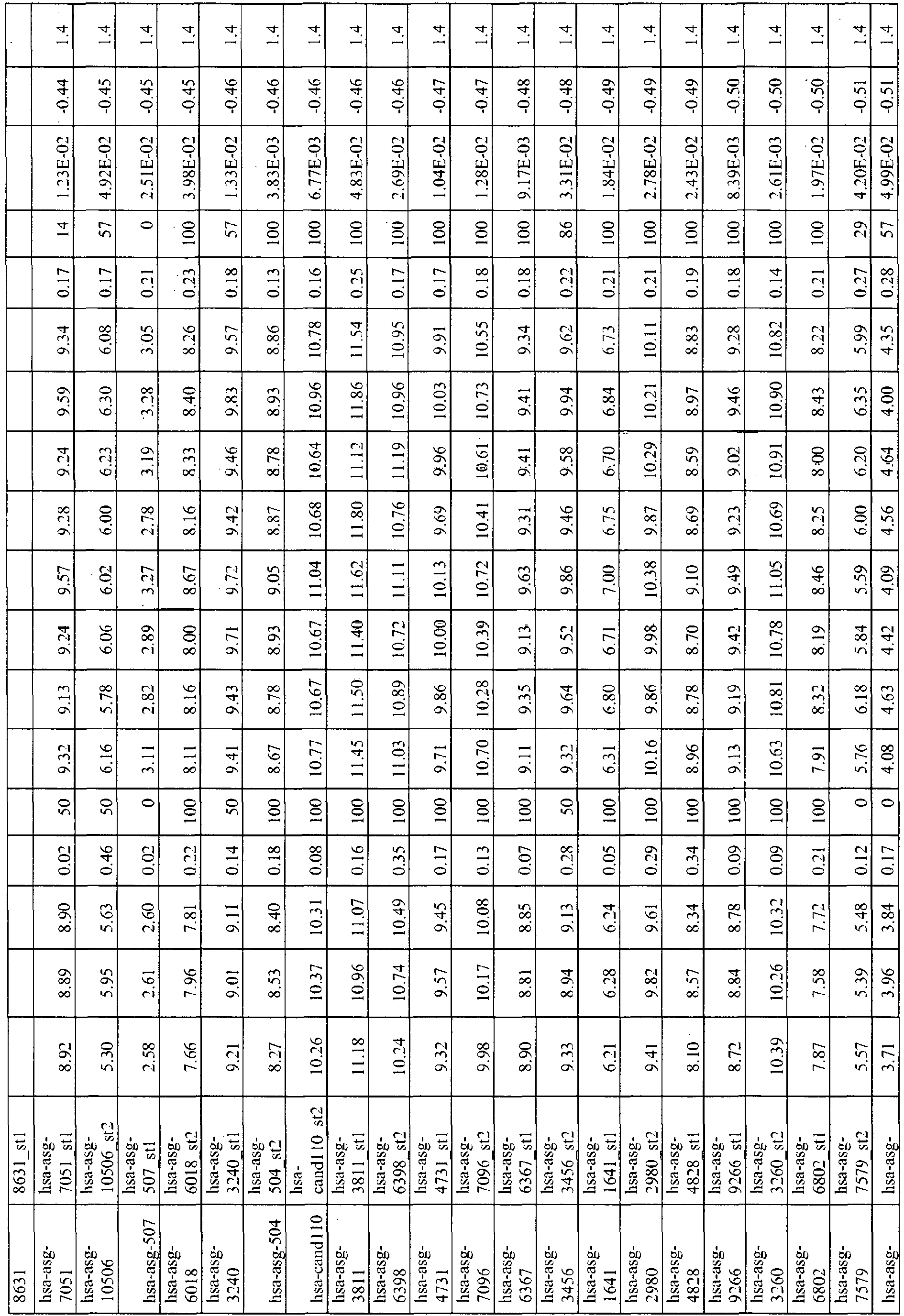

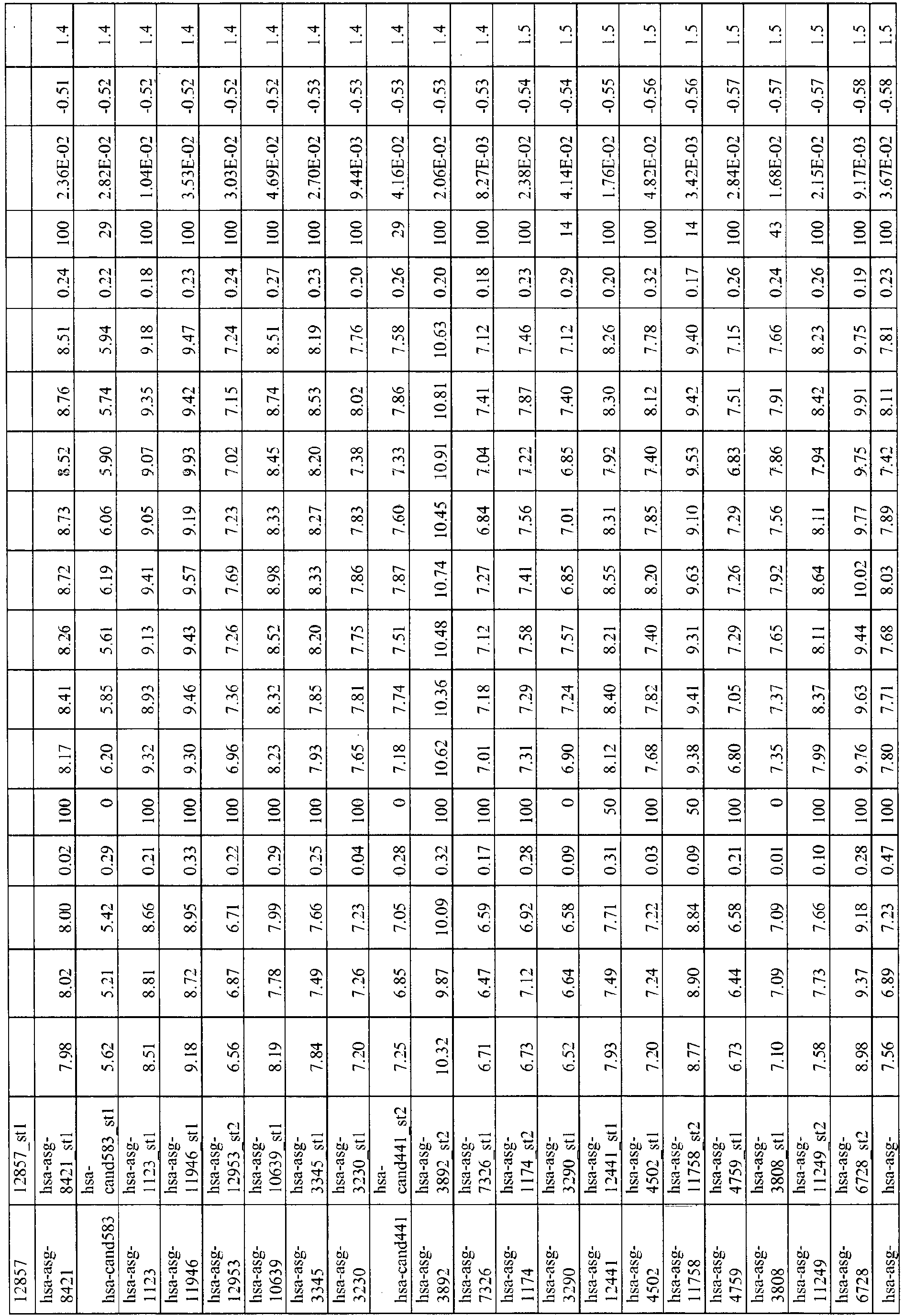

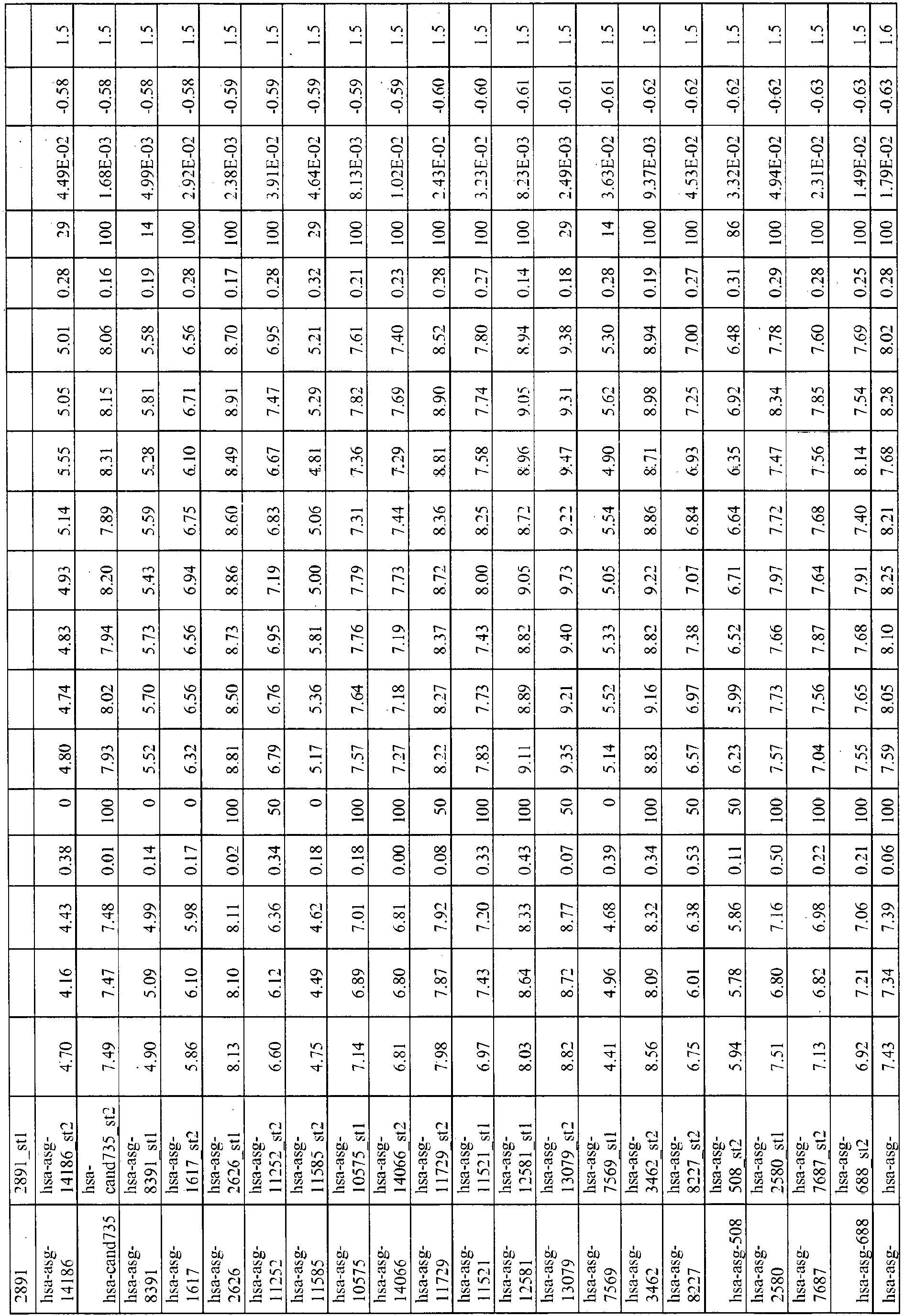

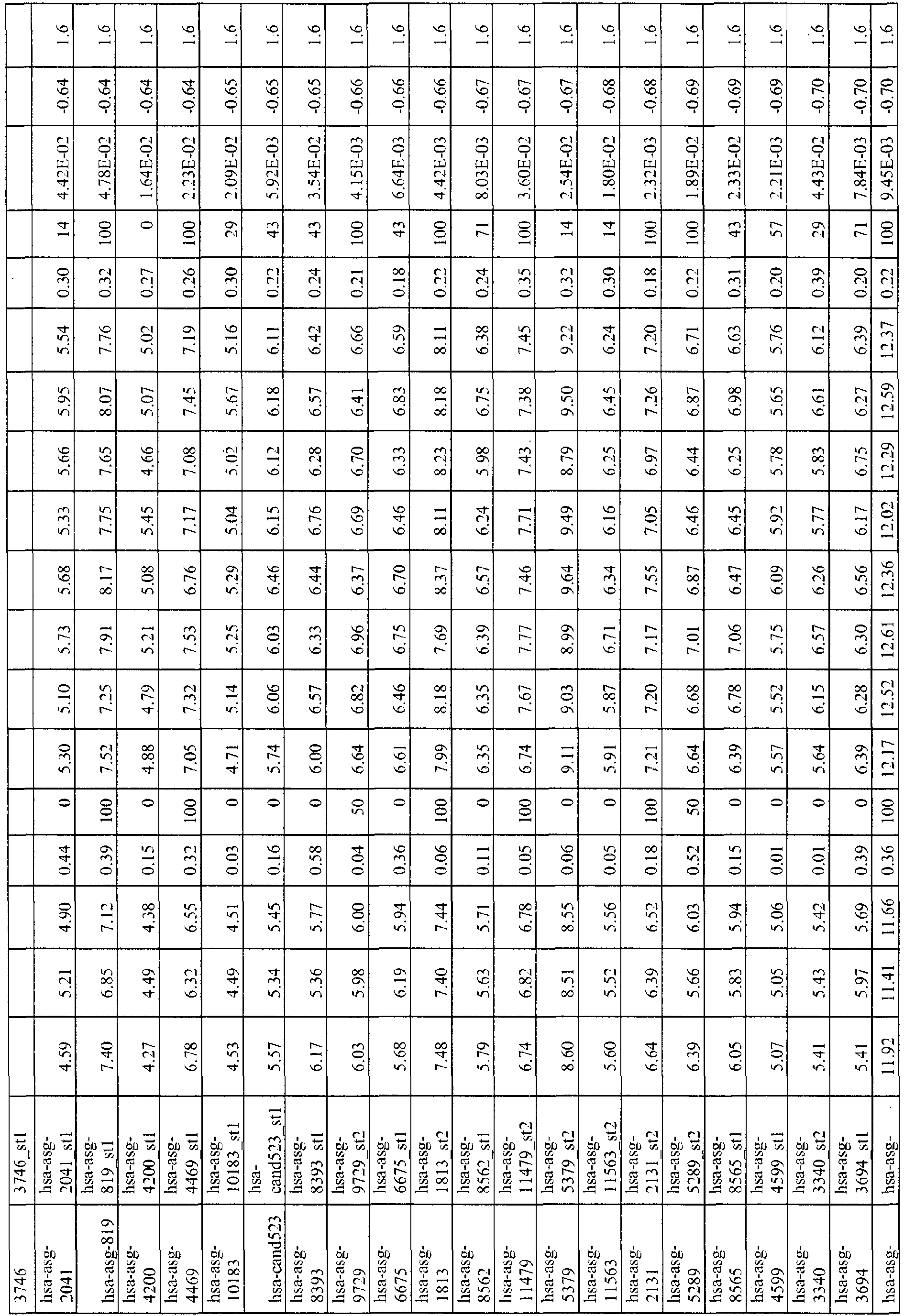

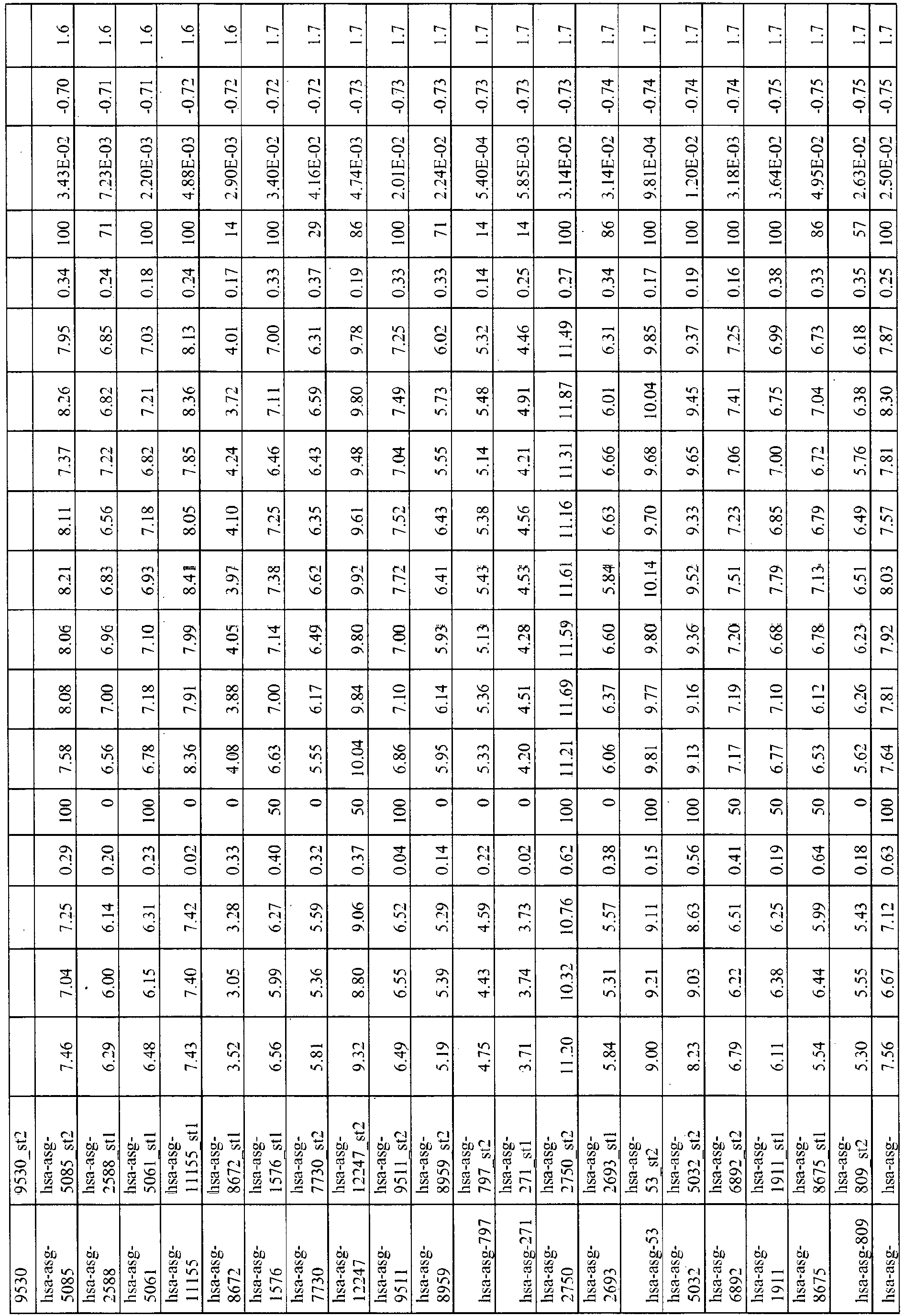

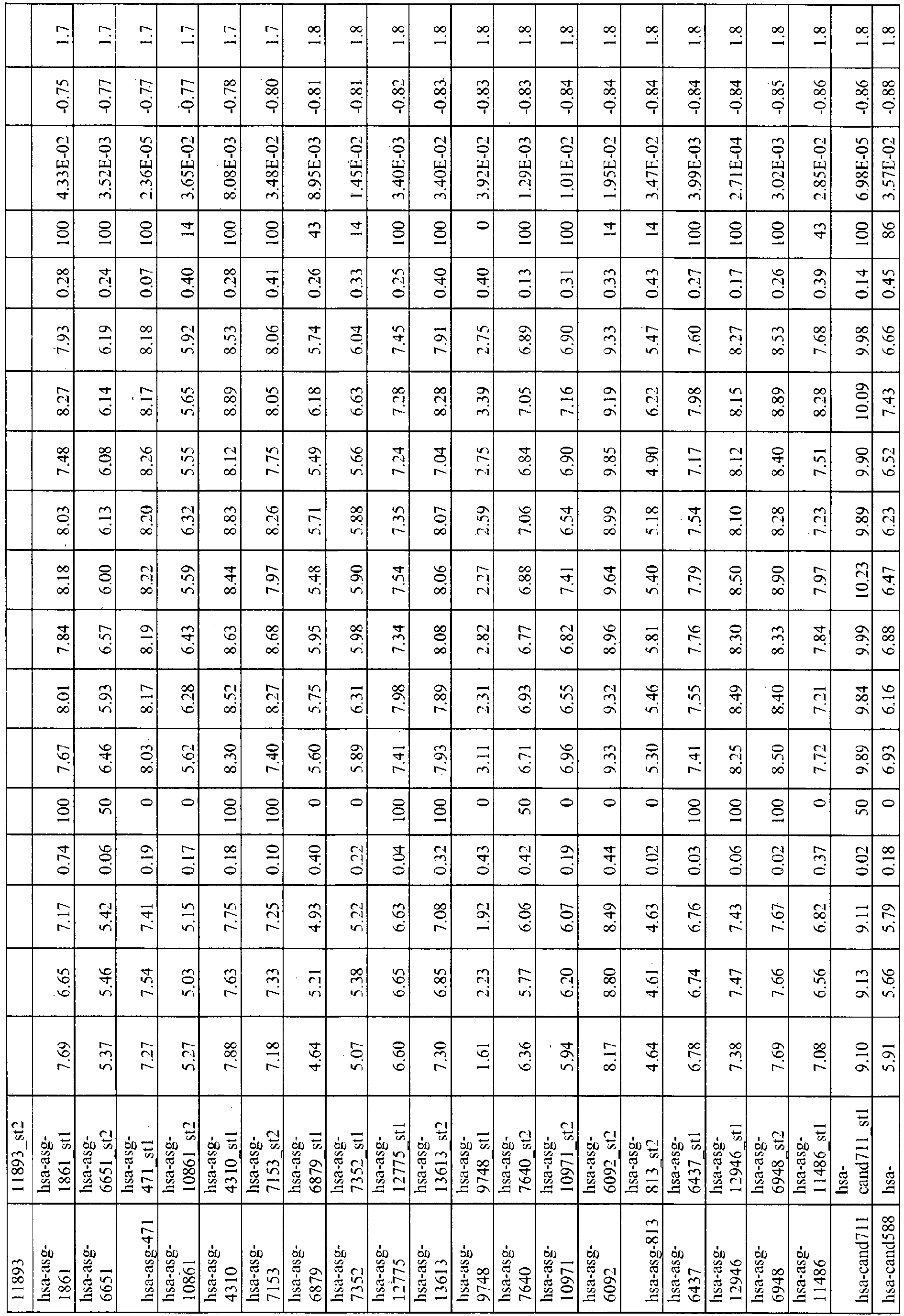

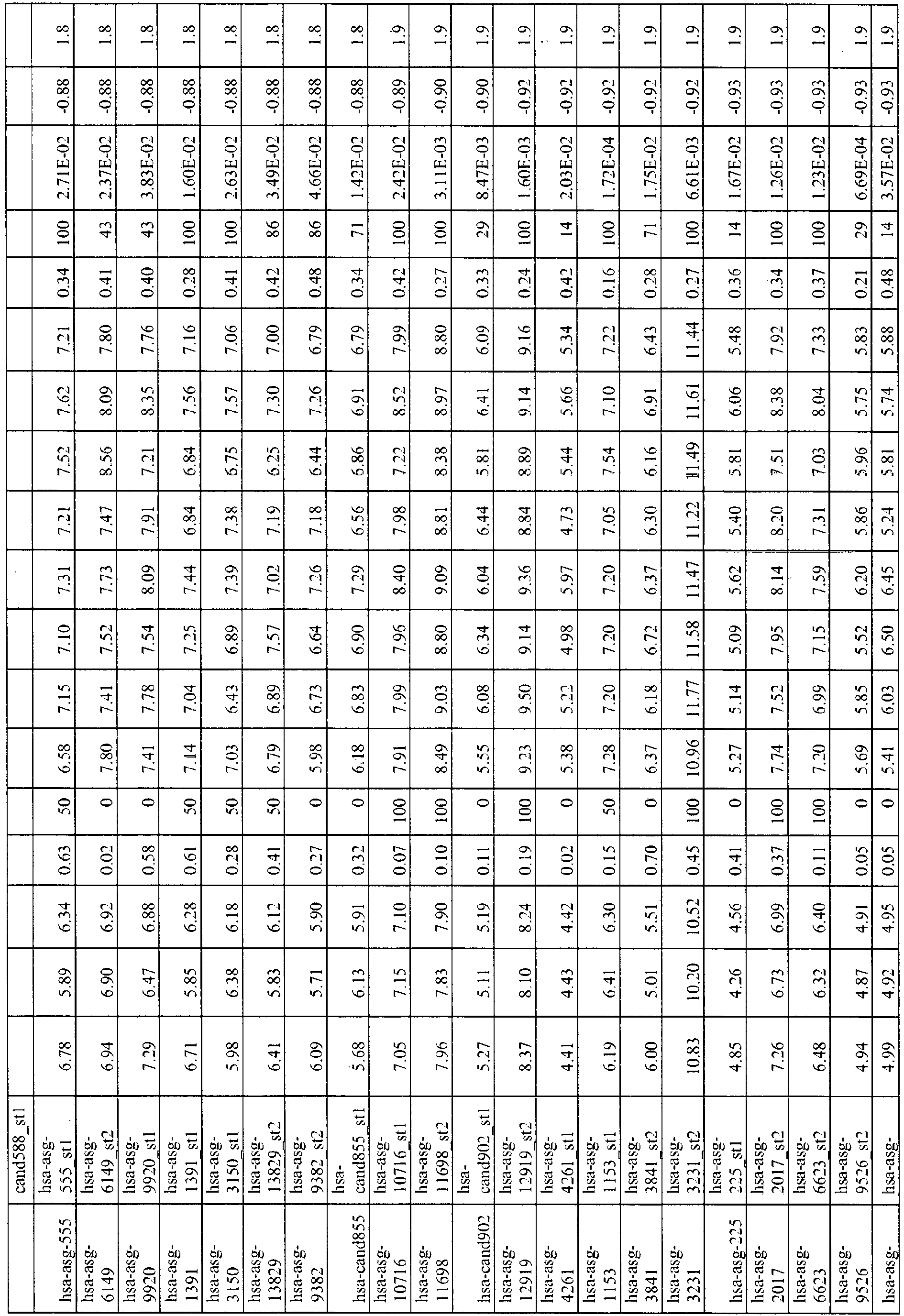

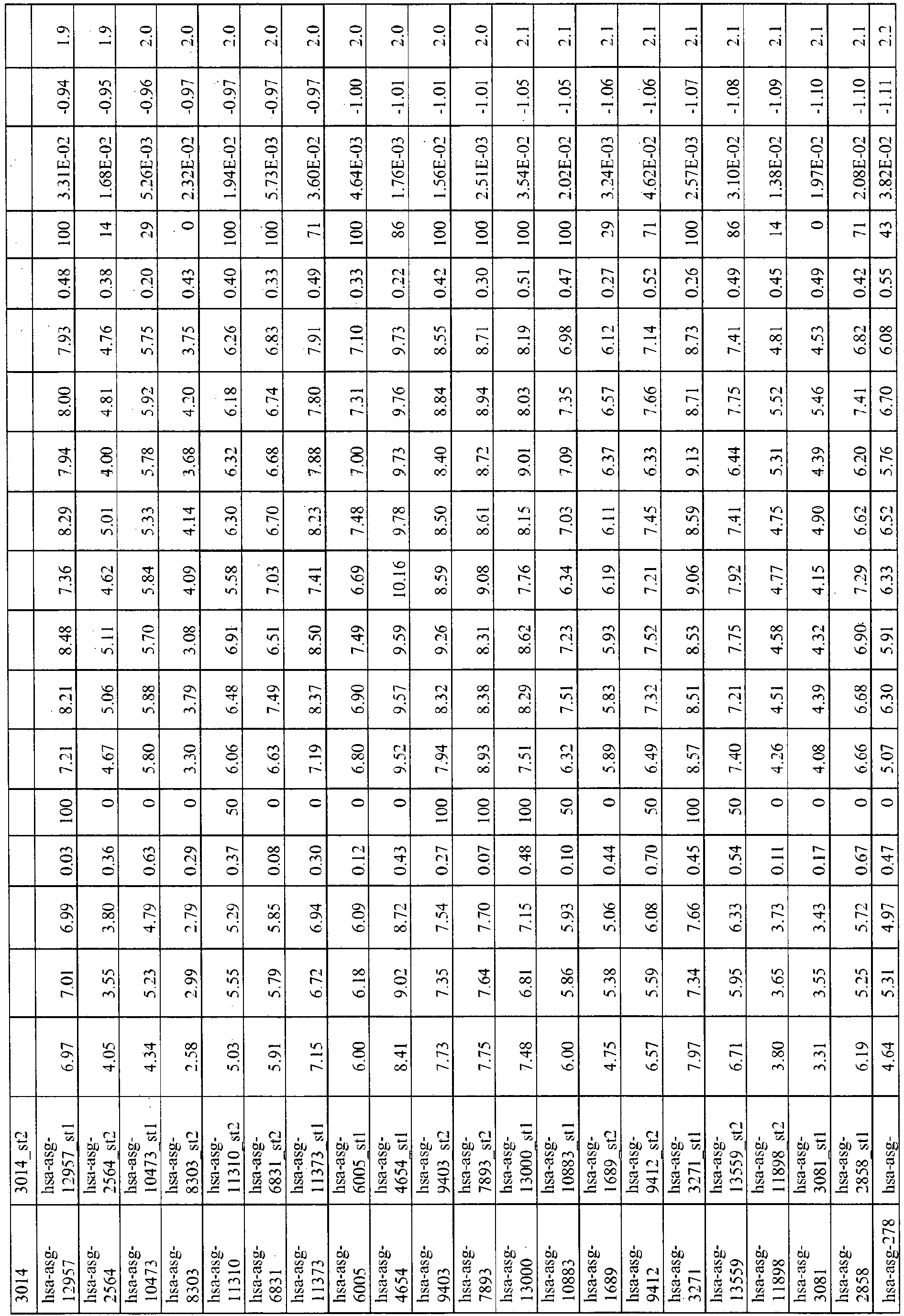

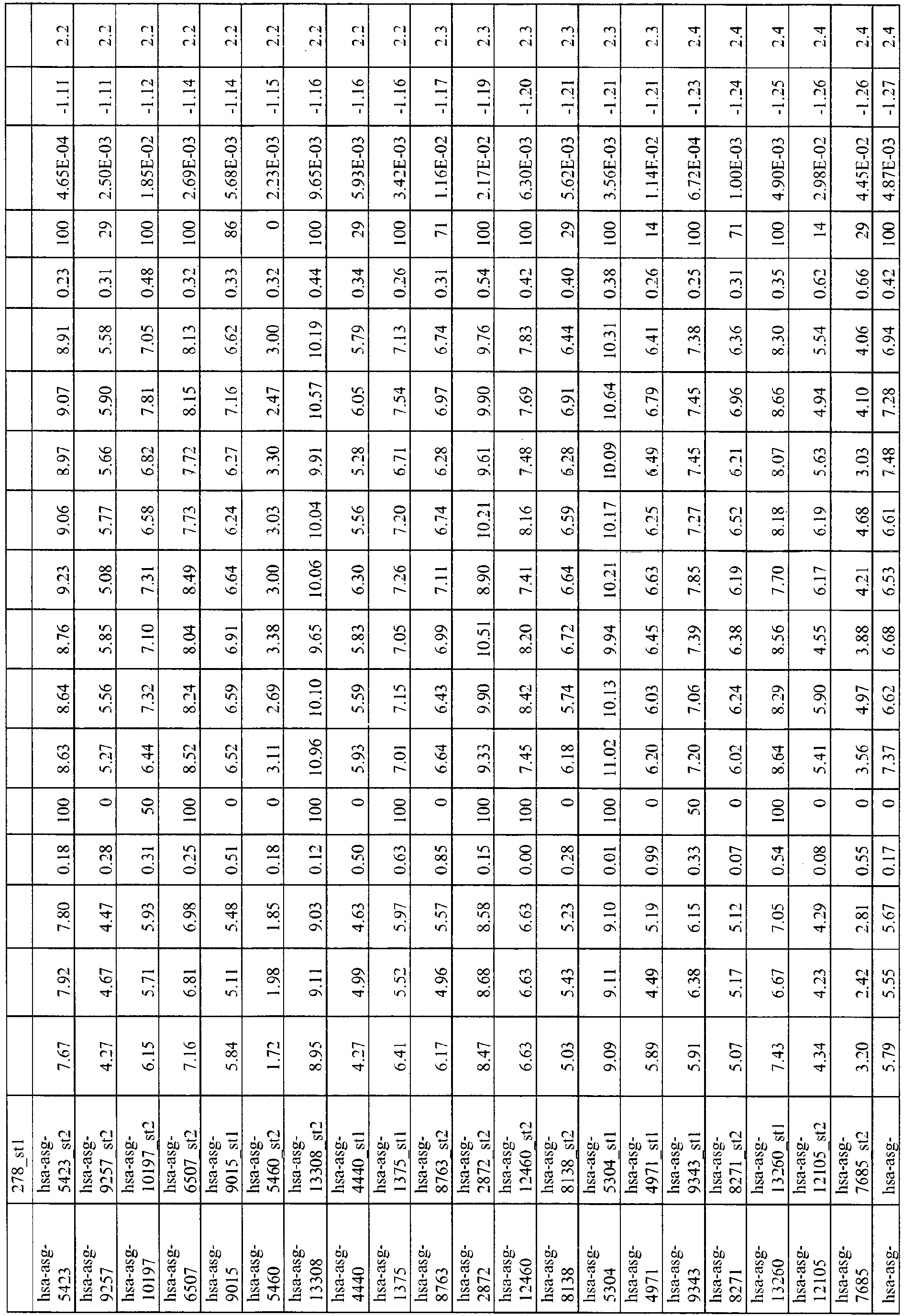

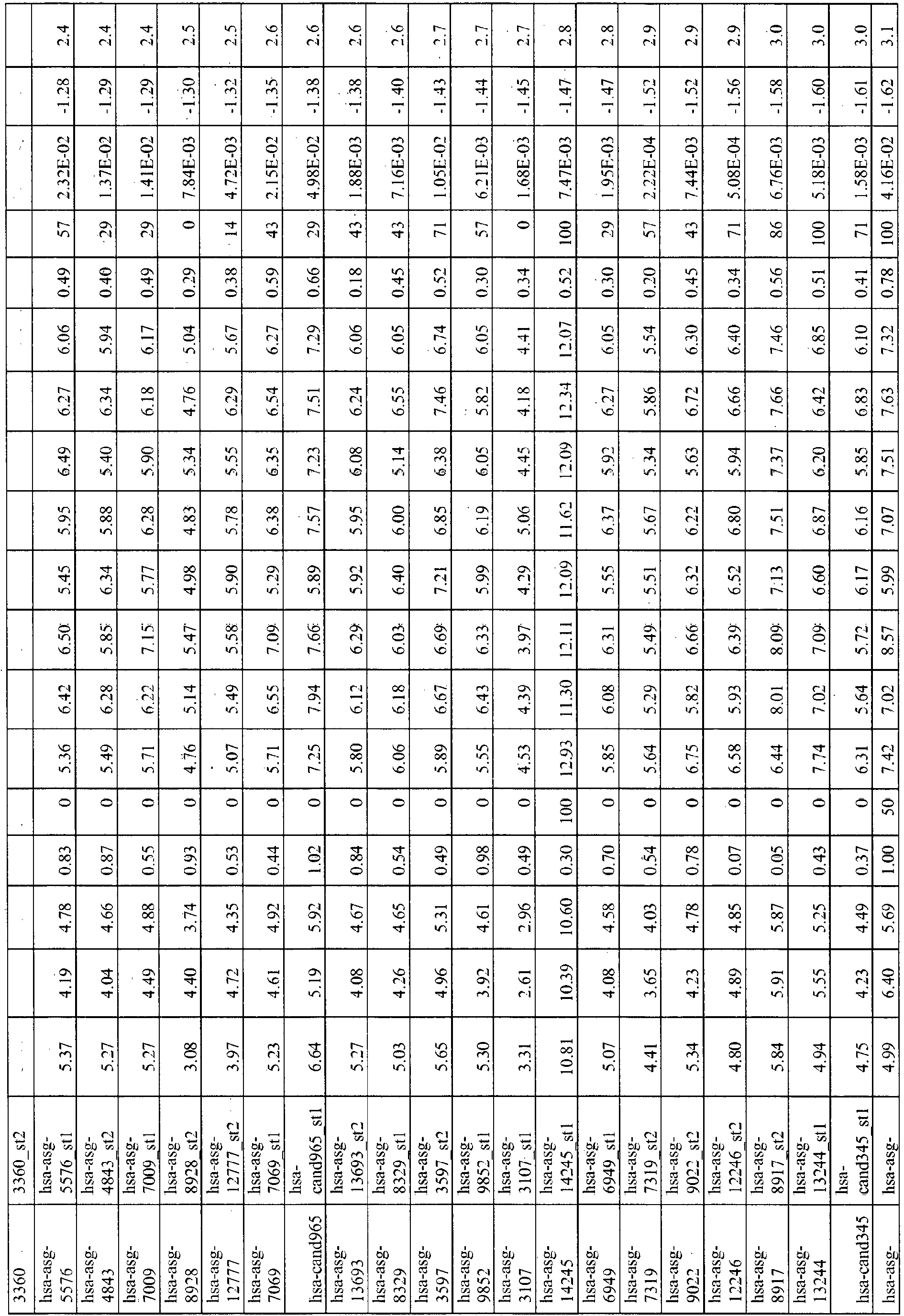

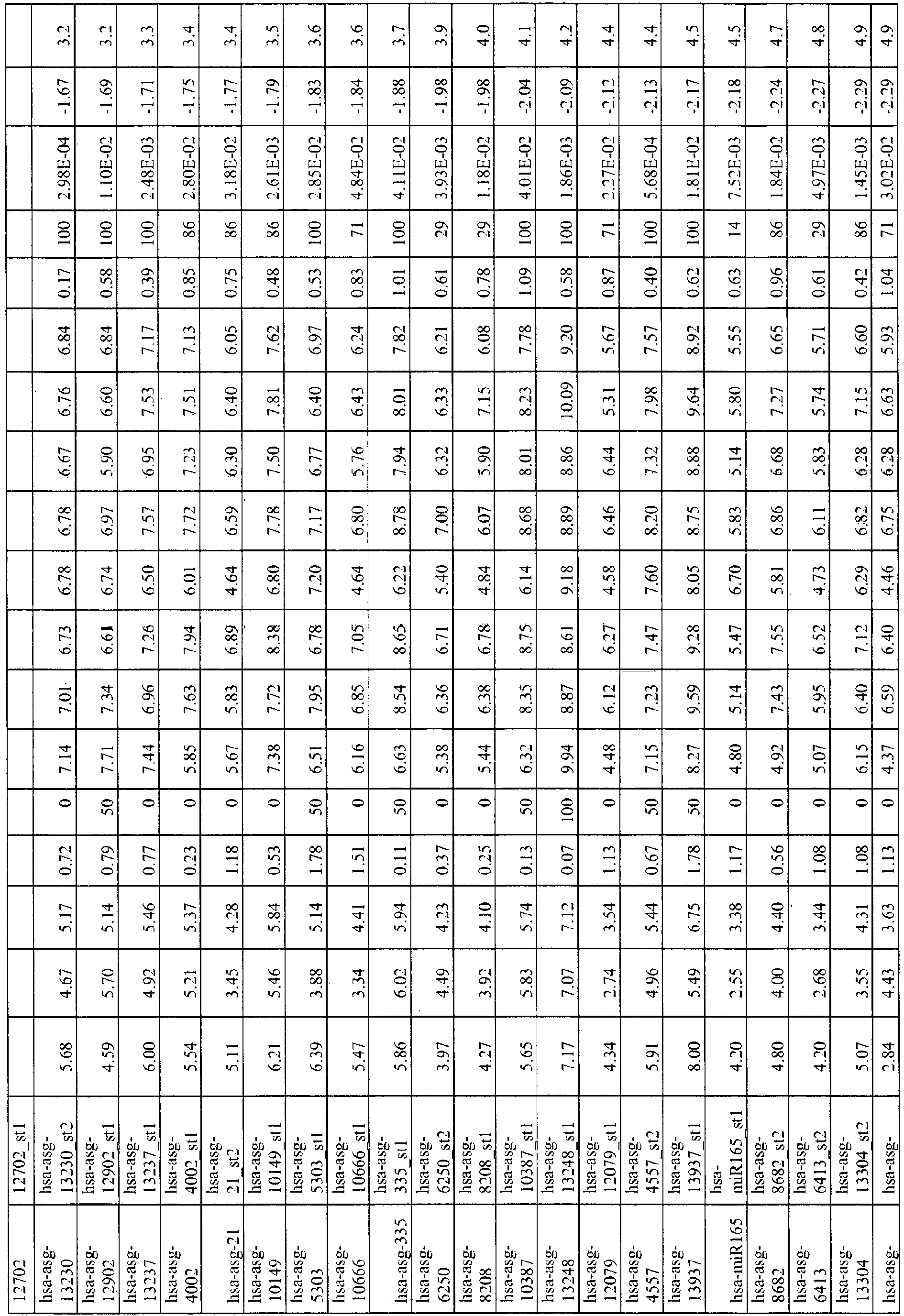

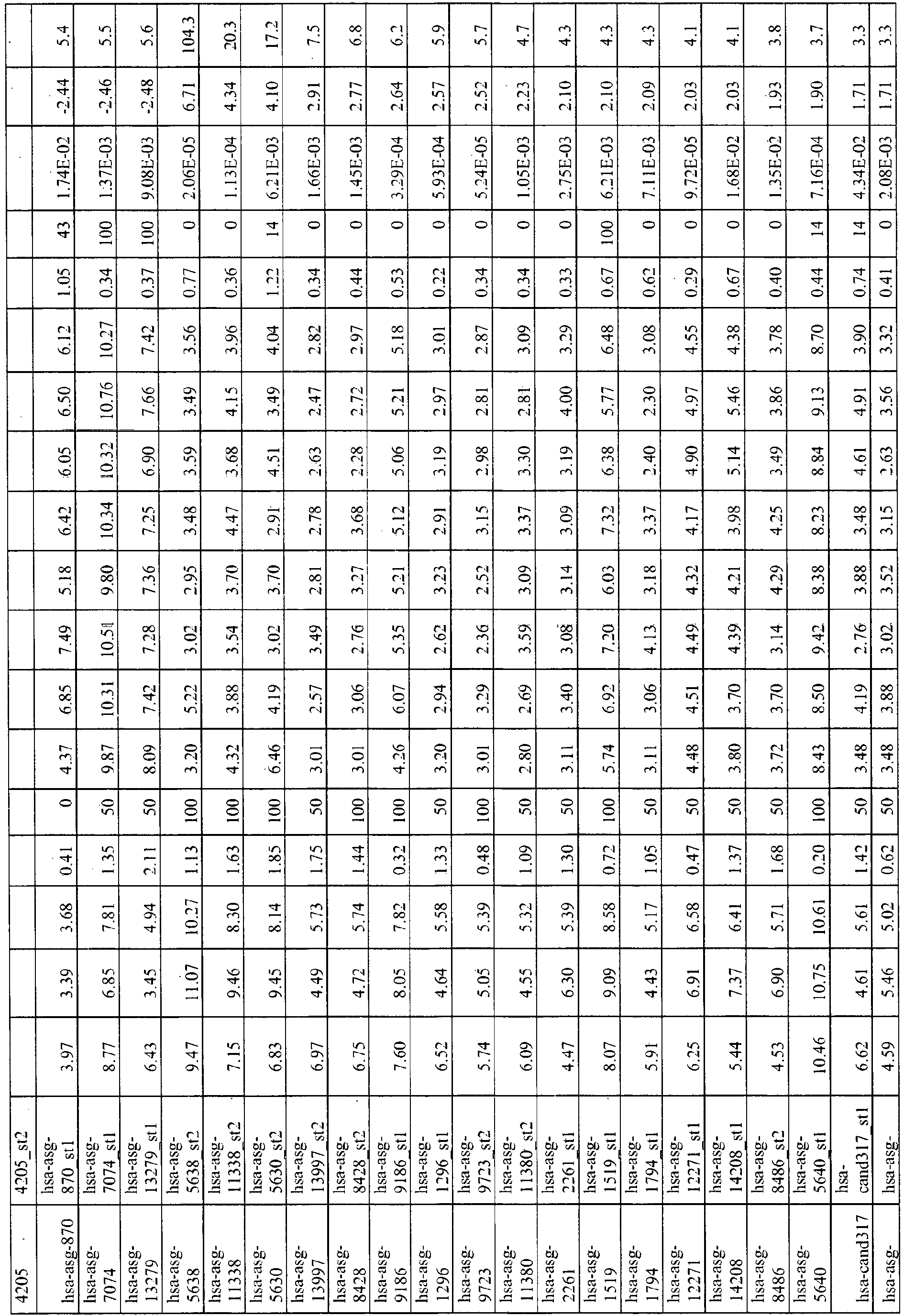

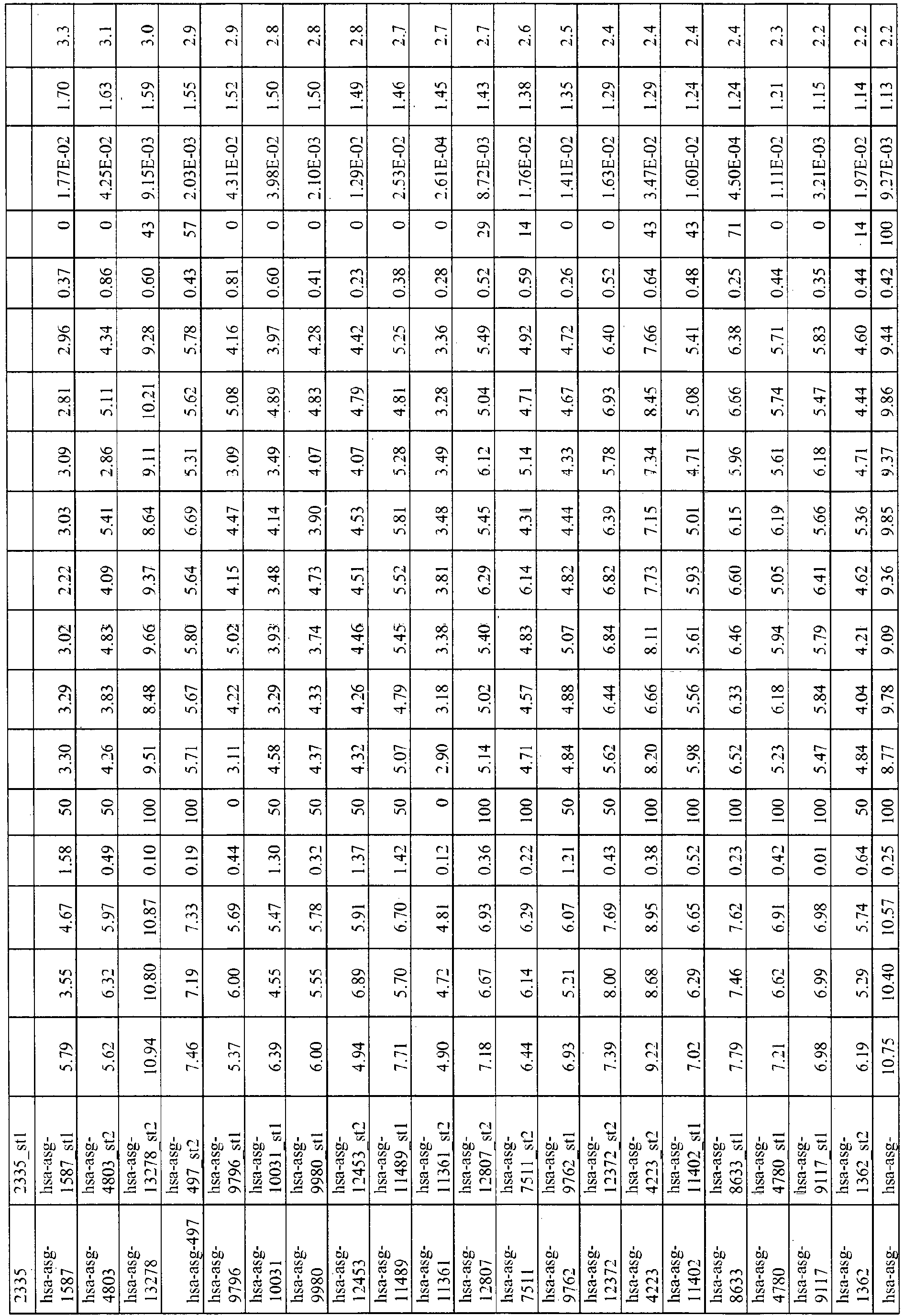

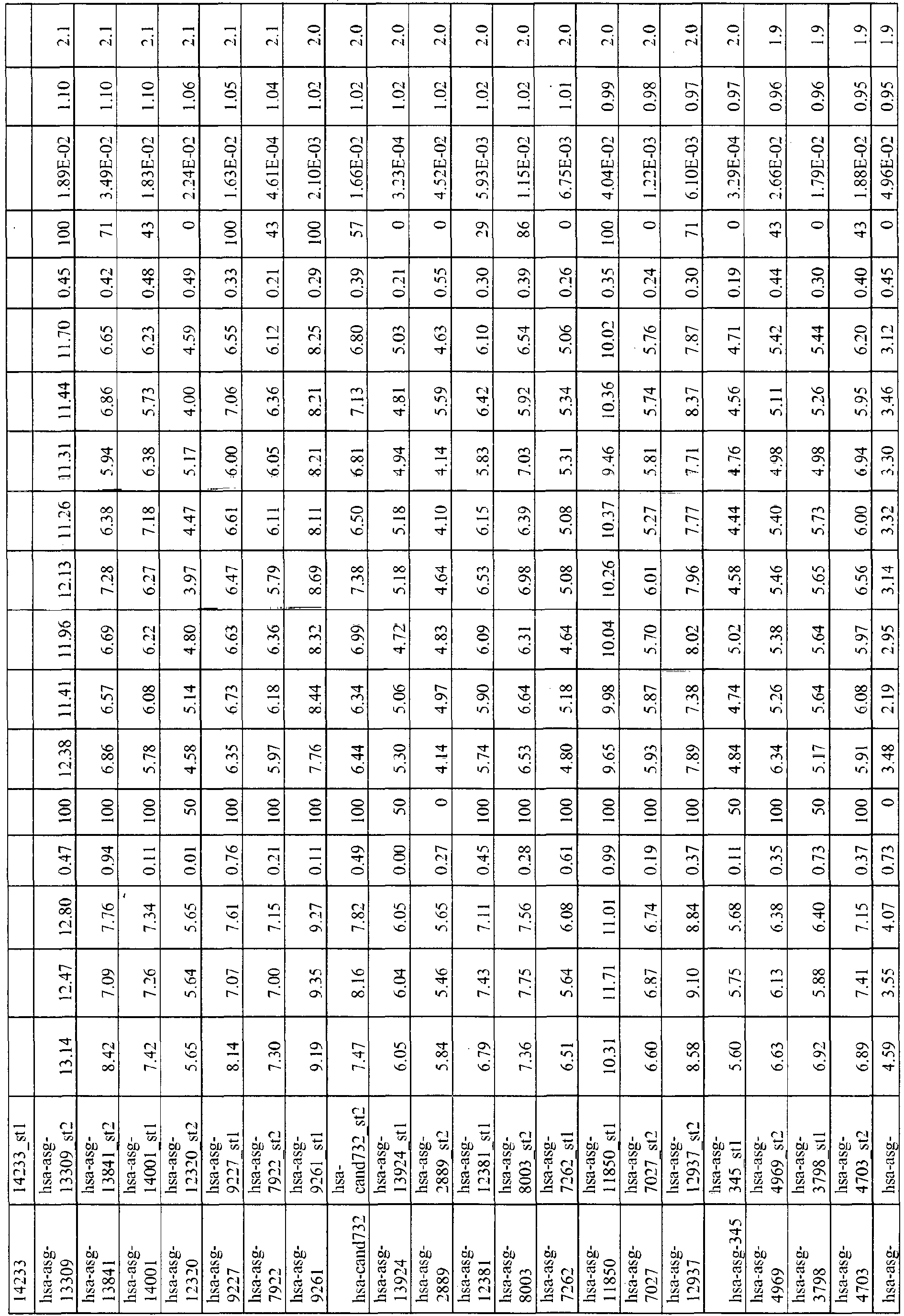

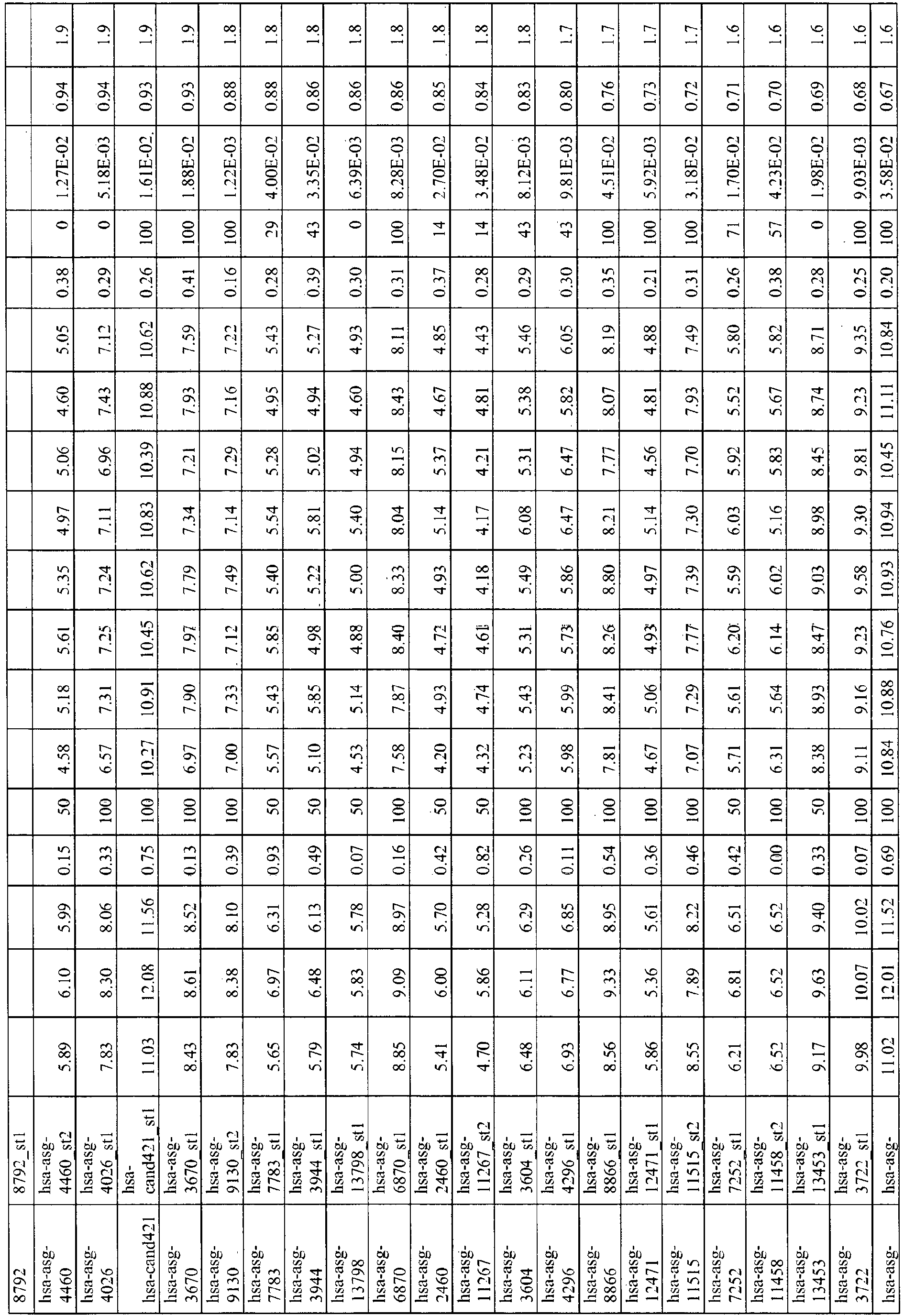

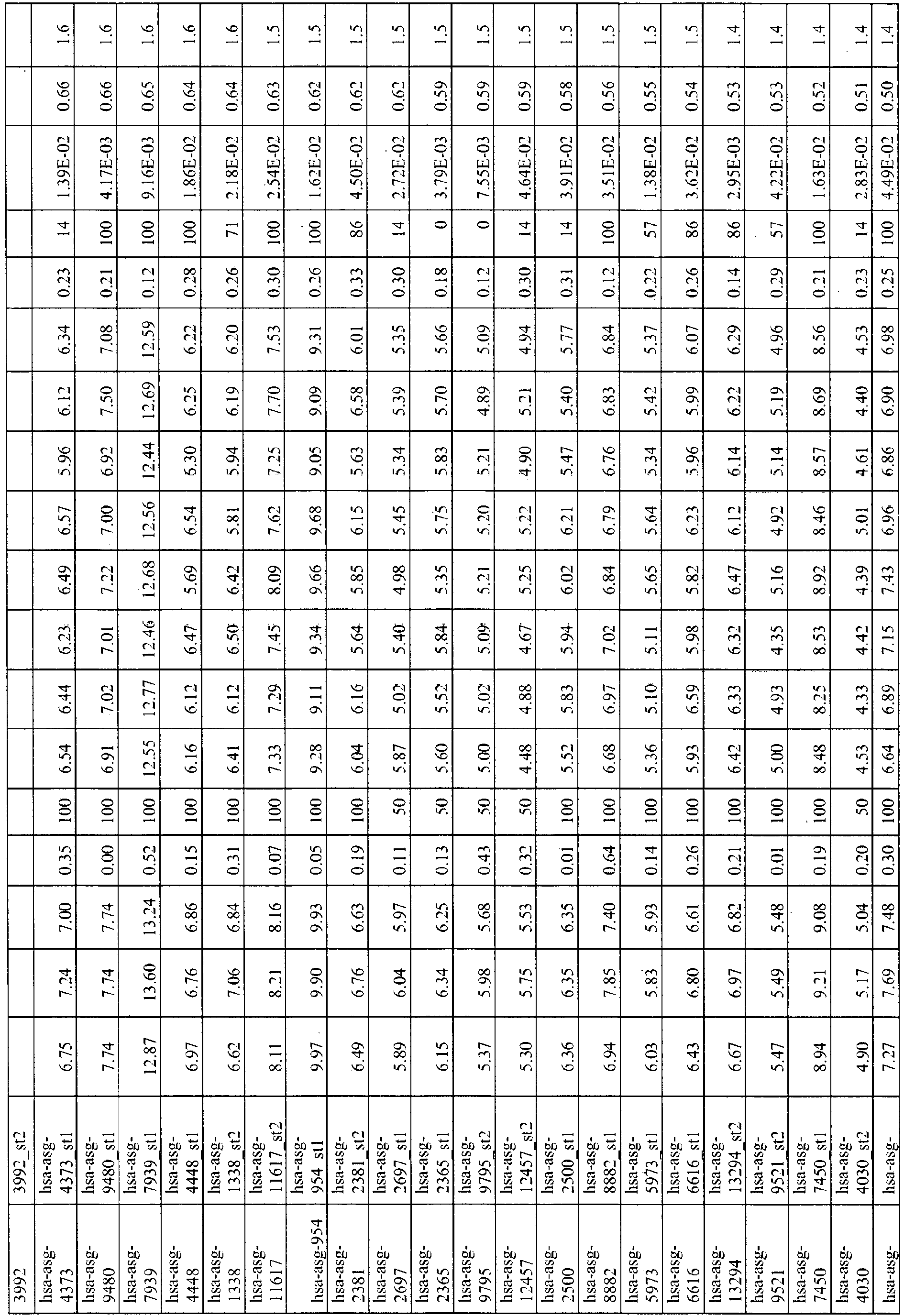

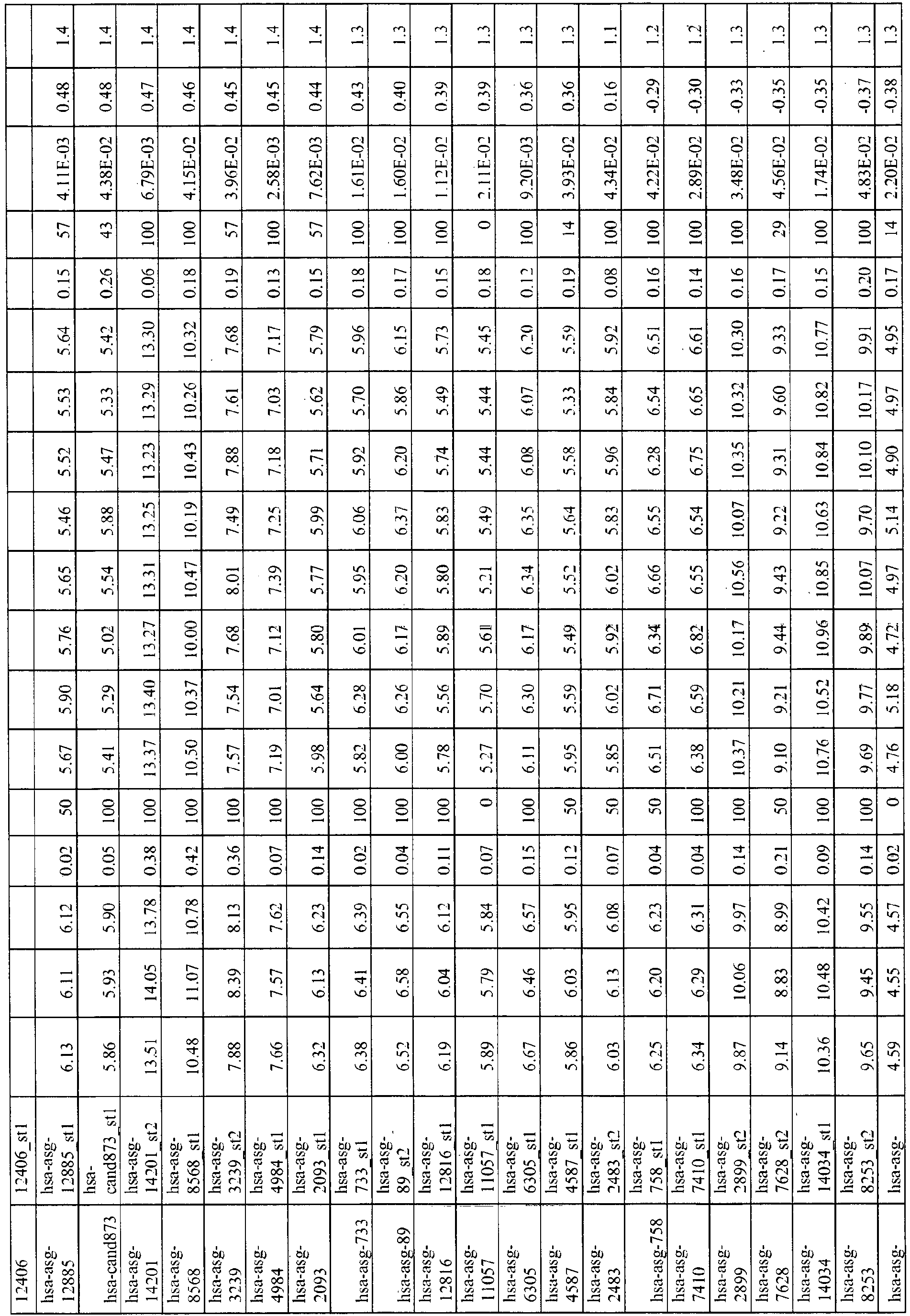

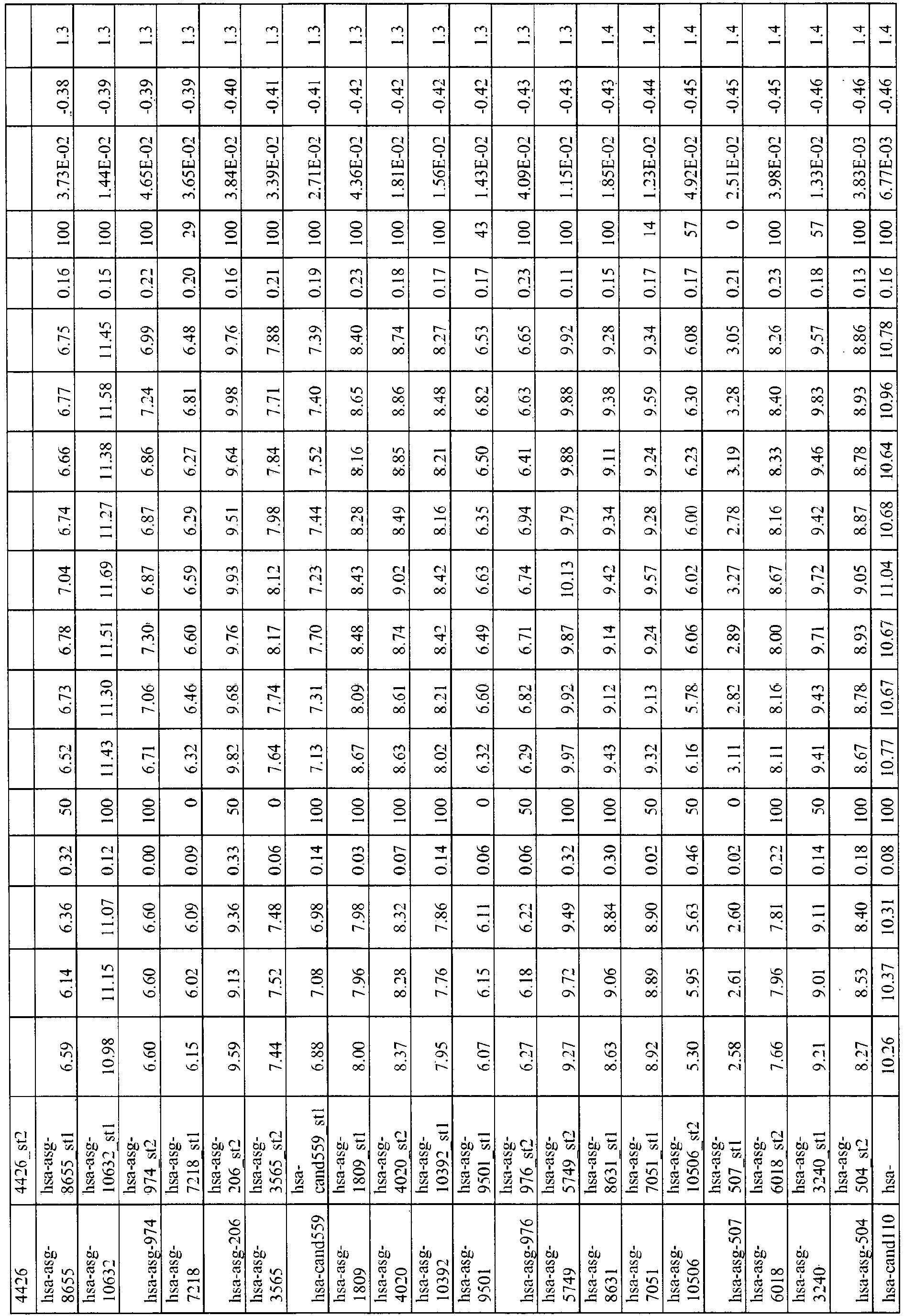

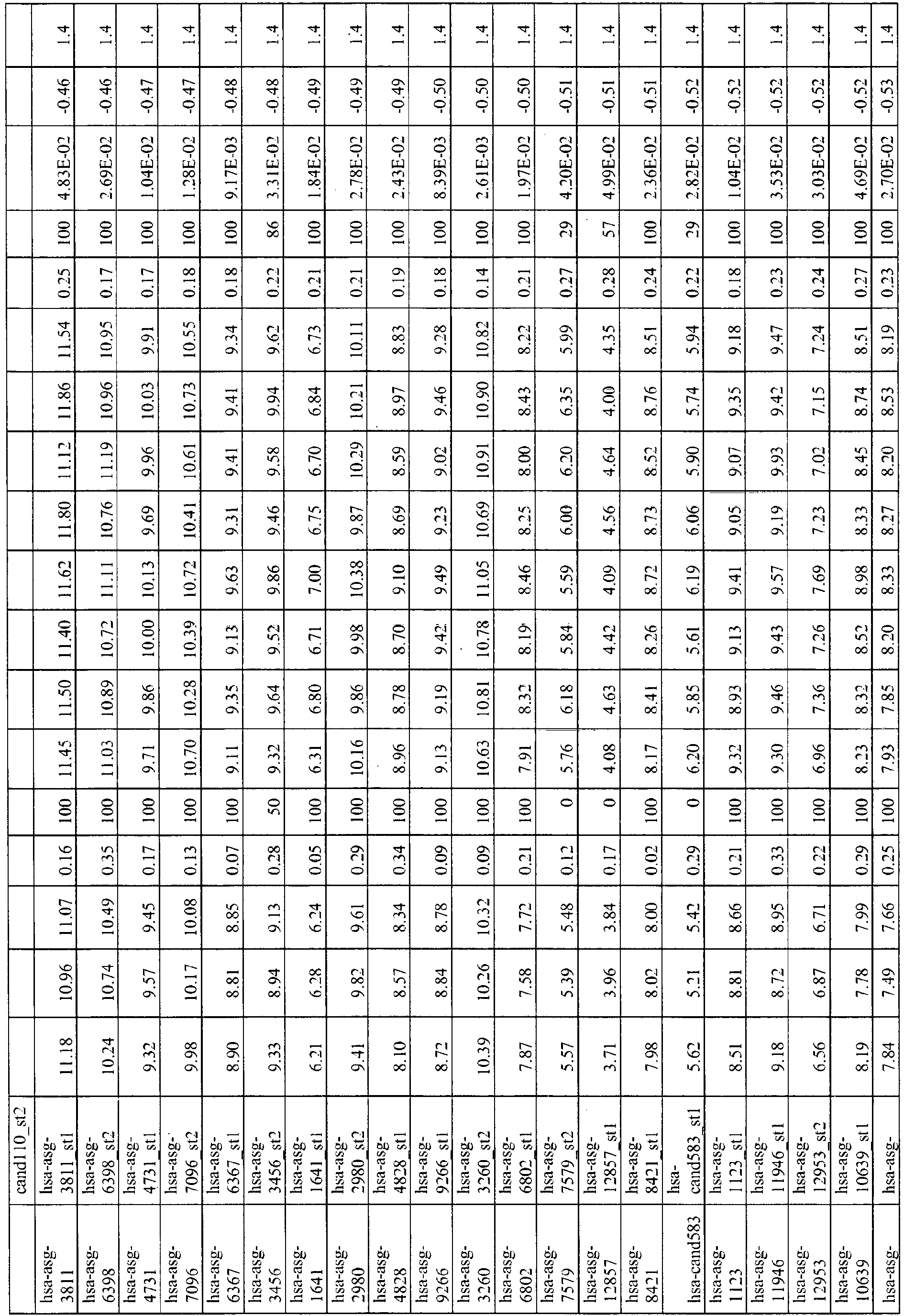

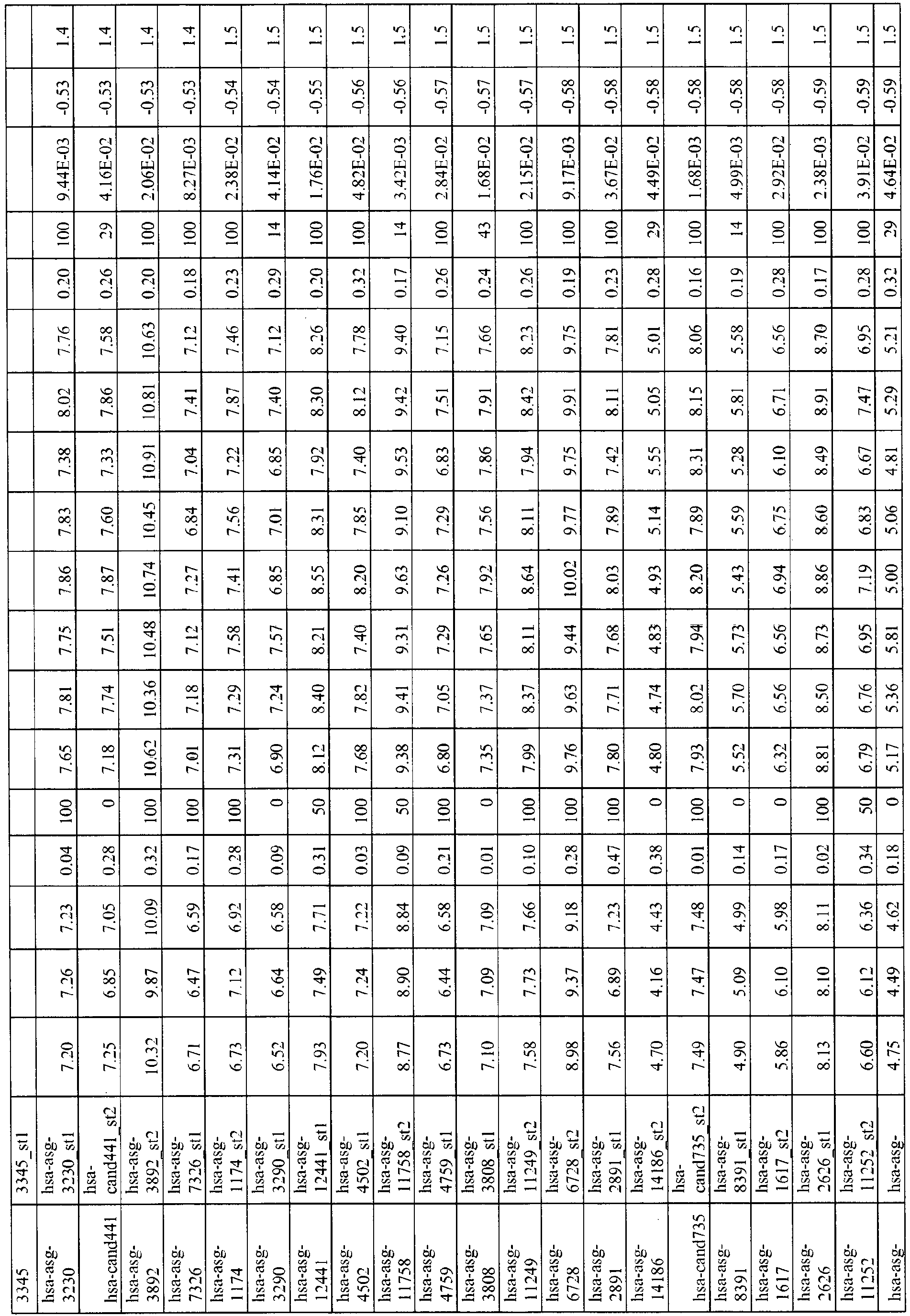

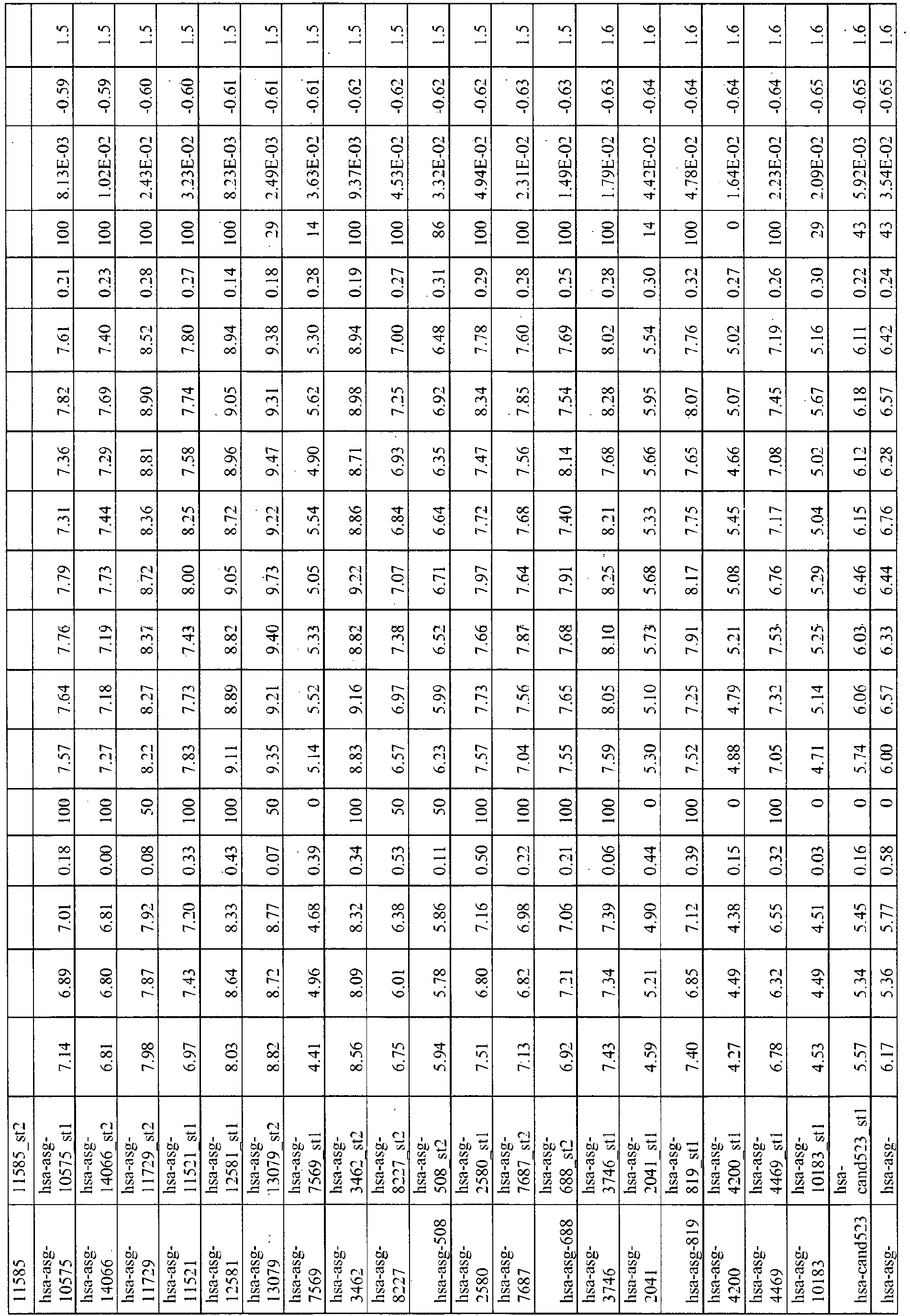

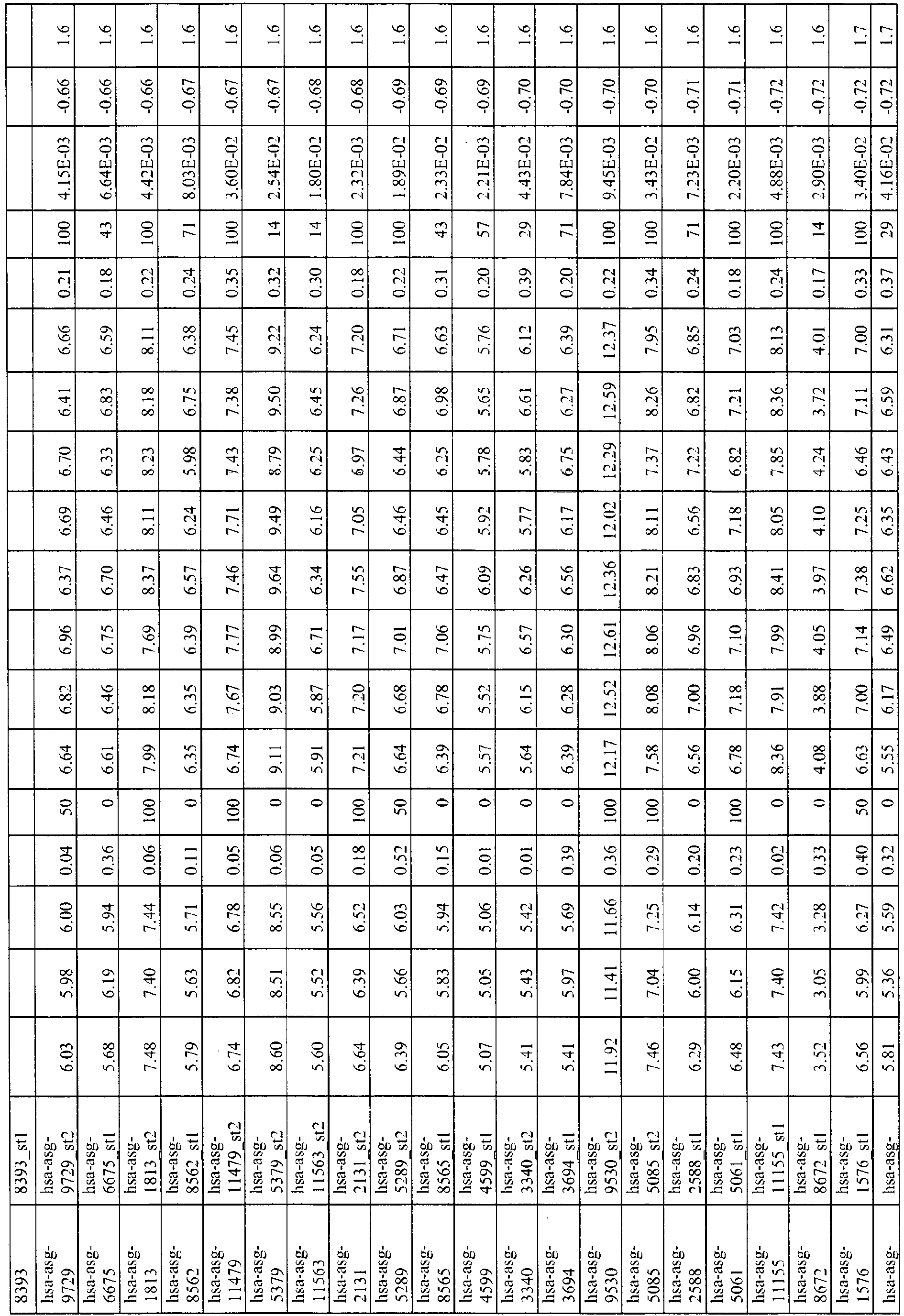

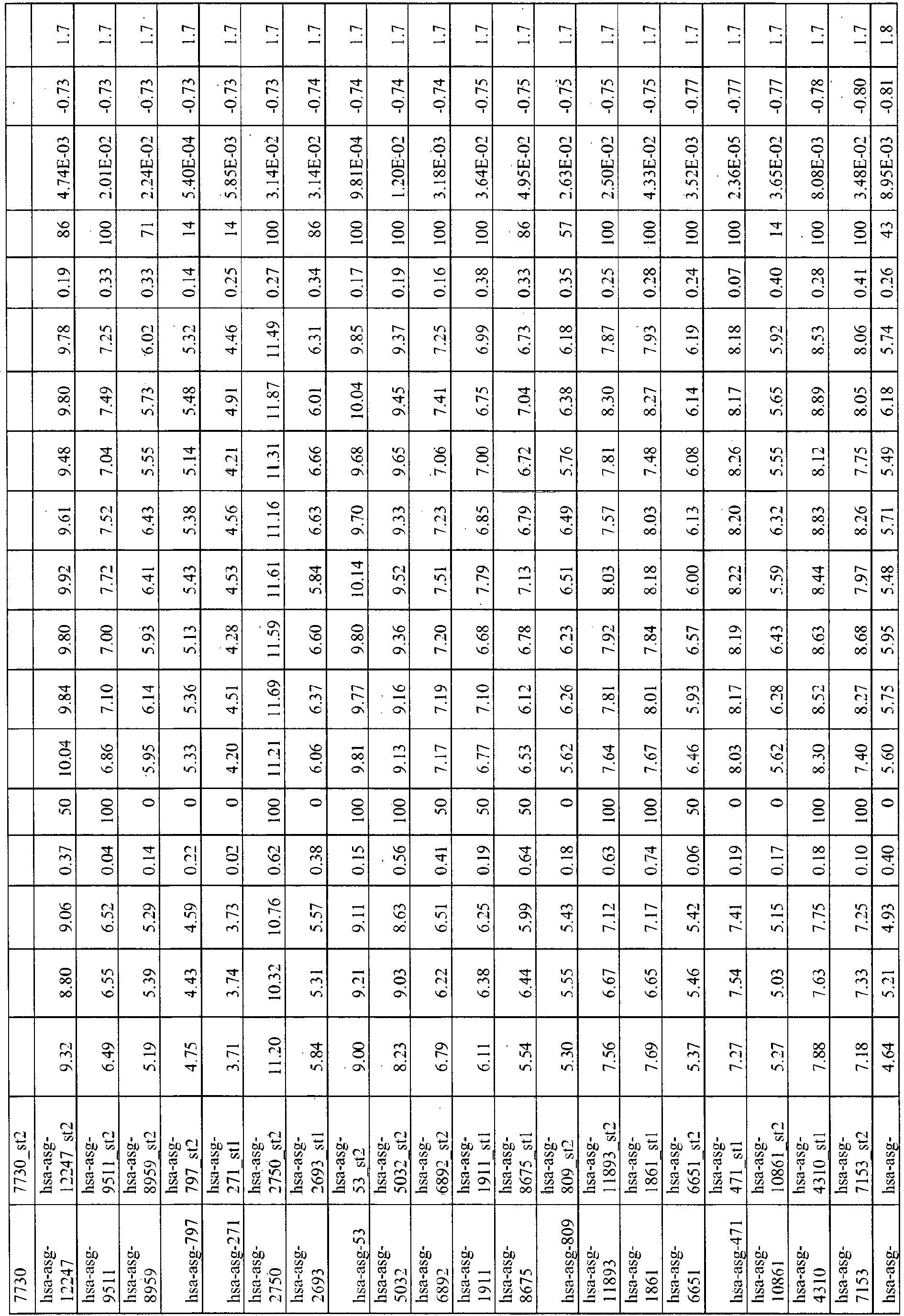

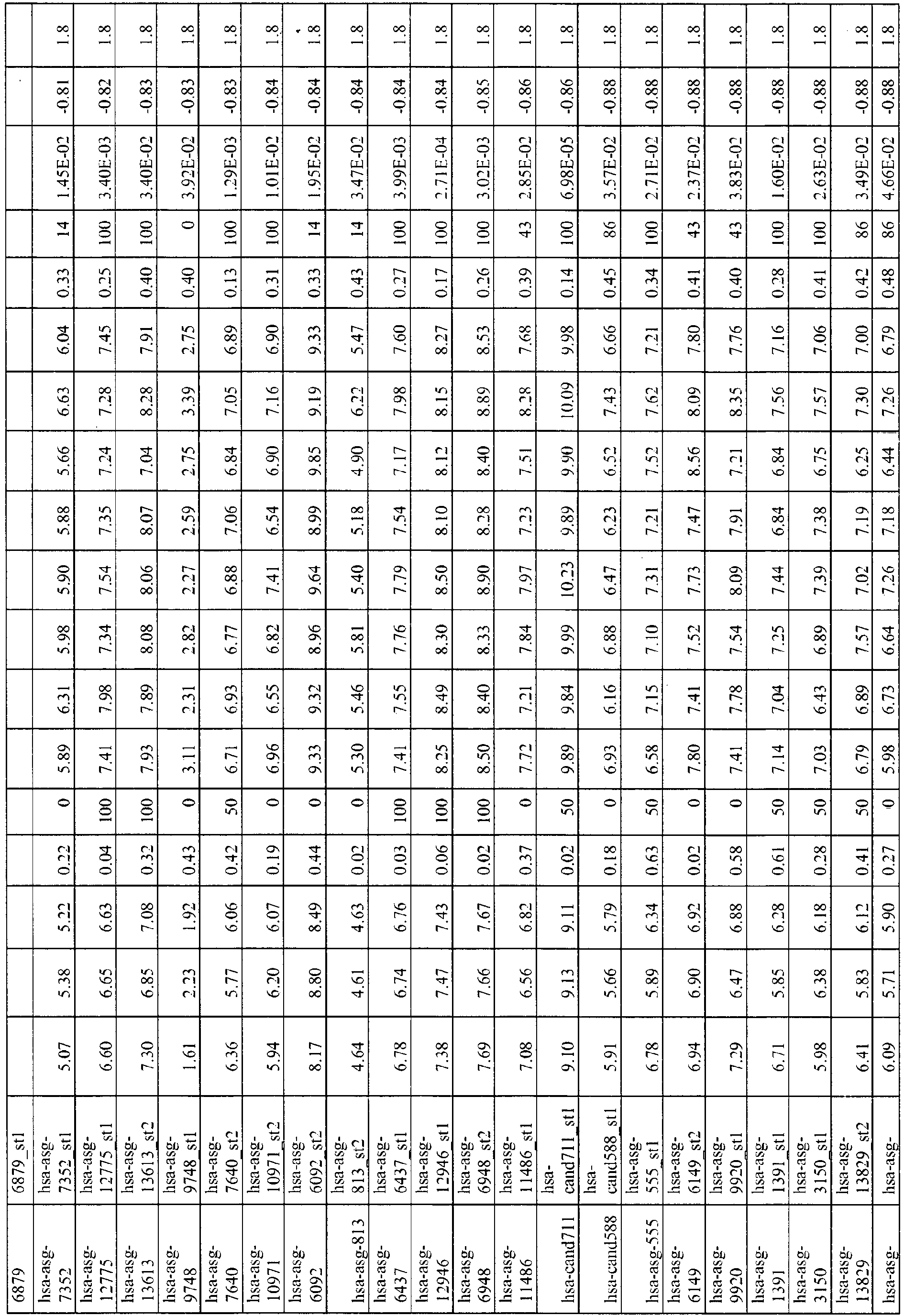

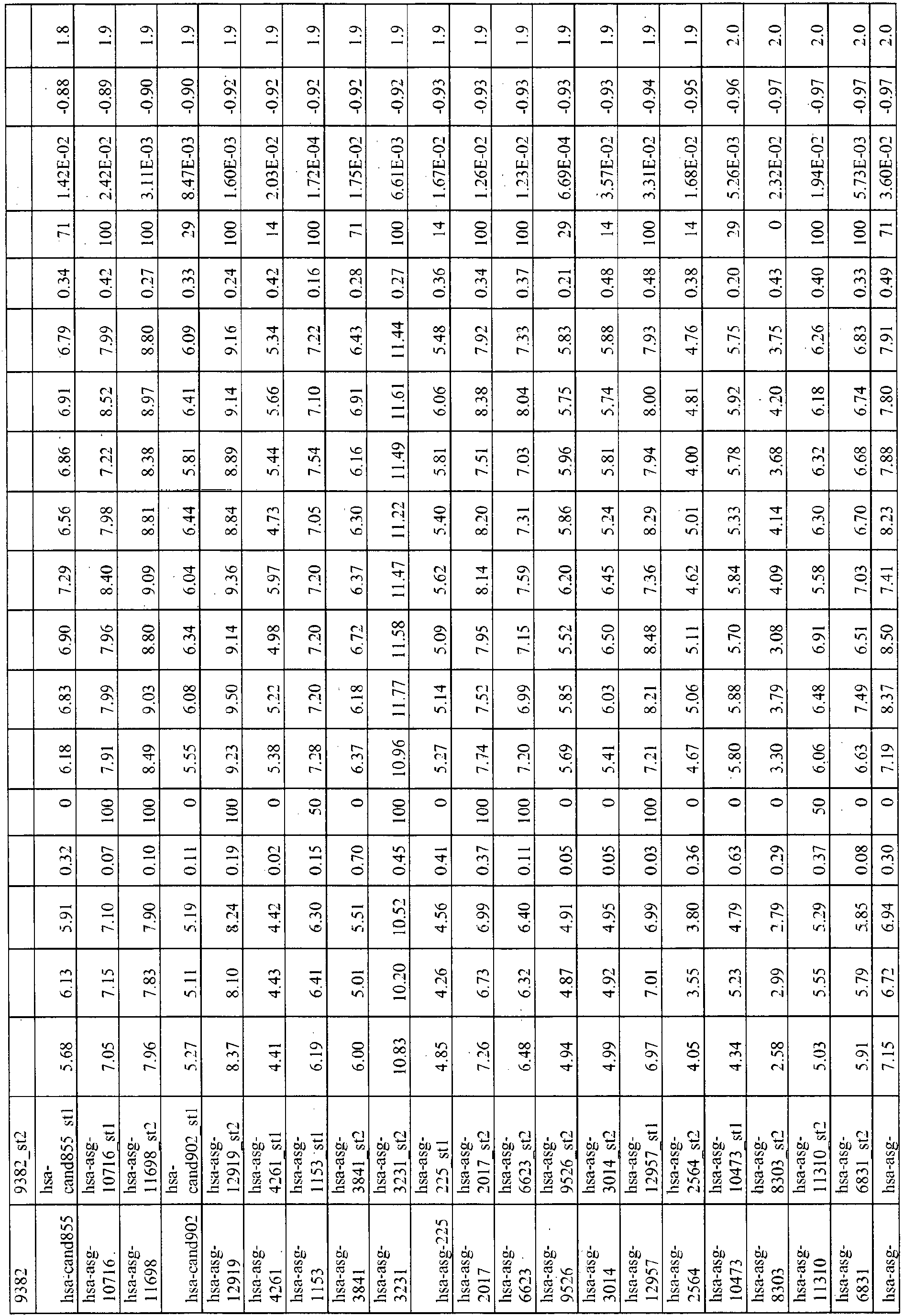

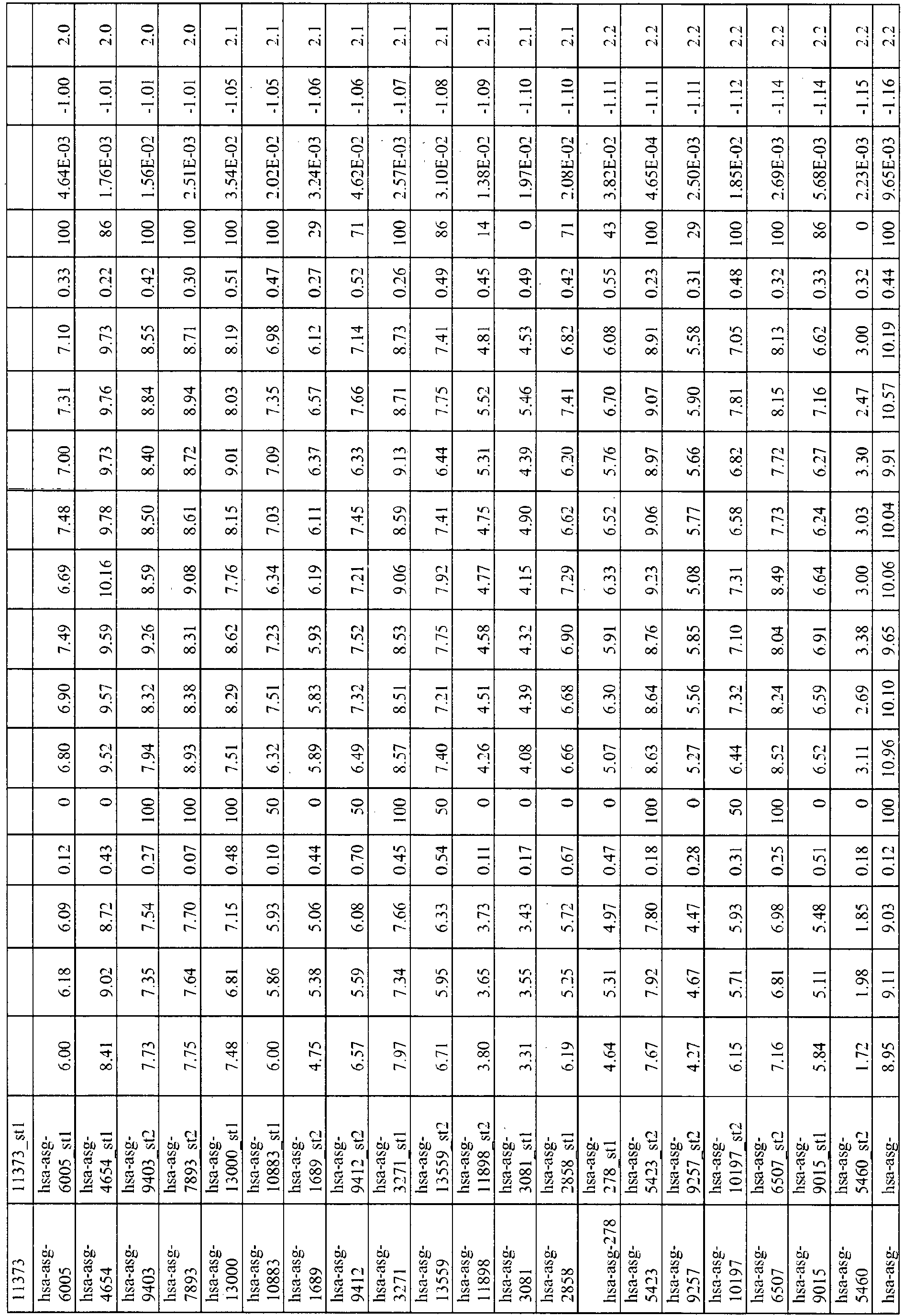

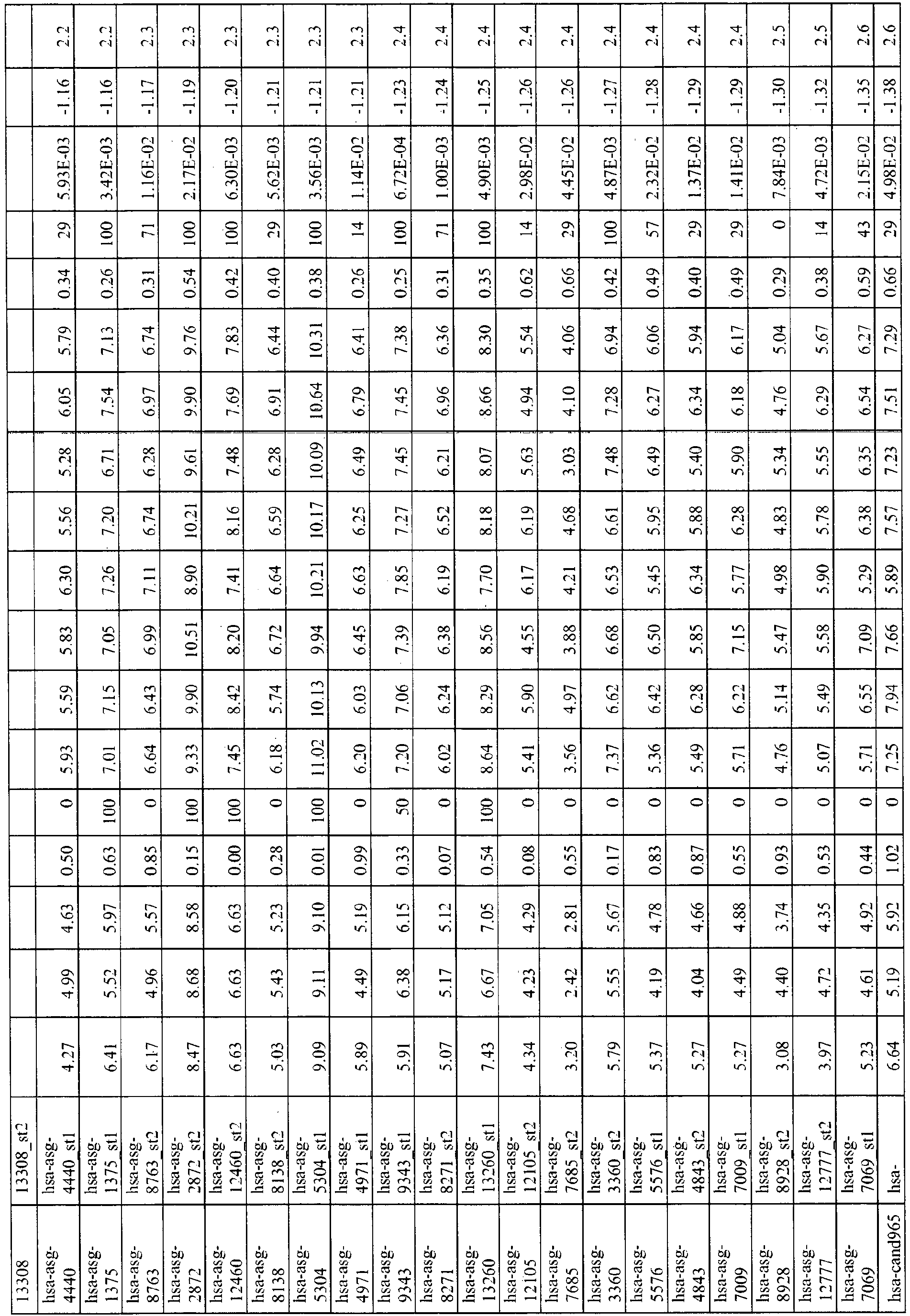

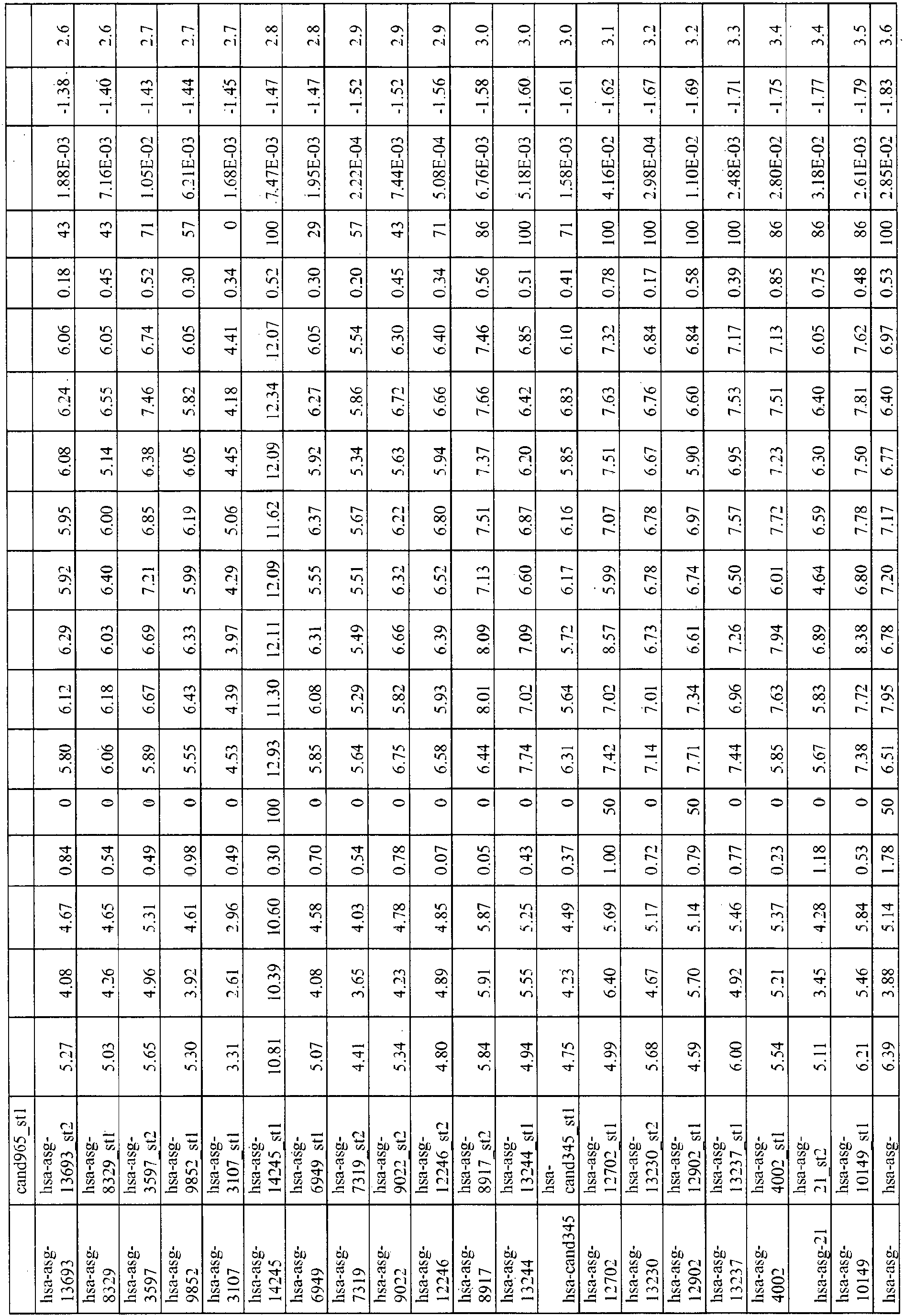

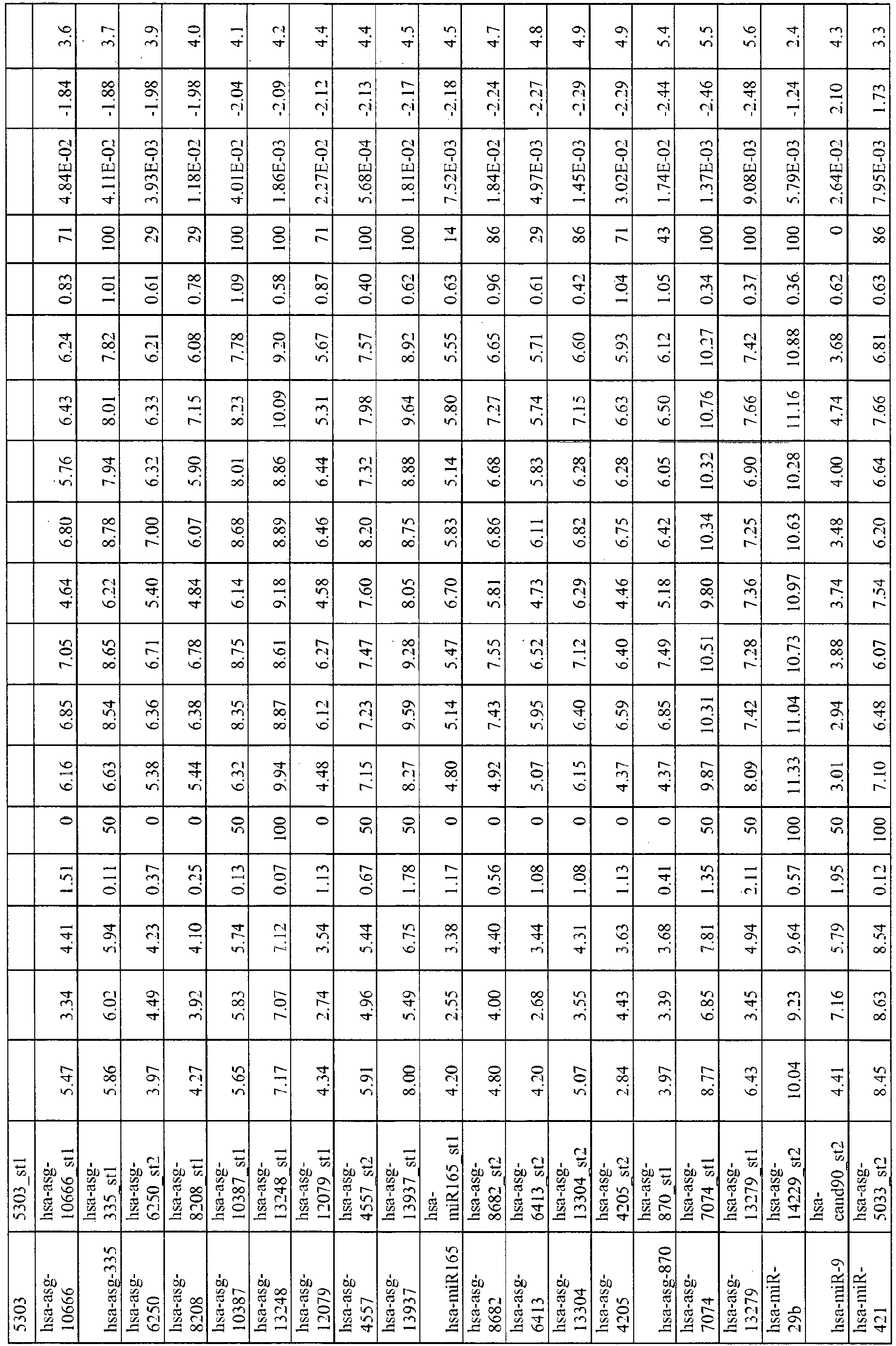

### EXAMPLE 11:

### MIRNAS DIFFERENTIALLY EXPRESSED BETWEEN CANCER-POSITIVE AND CANCER-NEGATIVE LYMPH NODES FROM MELANOMA PATIENTS

To identify miRNAs that are useful markers for metastasis of melanoma to lymph nodes, the inventors identified miRNAs that are expressed at significantly higher levels in cancer-positive lymph nodes (LNpos, frozen biopsy specimens, Table 9) than in cancer-negative lymph nodes (LNneg1-7, frozen biopsy specimens, Table 9), from melanoma patients. miRNAs were purified and expression was analyzed with microarrays as described above in Example 2. miRNA expression profiles were established for three positive lymph nodes (LNpos1A, LNpos2B, LNpos3B) and compared with expression profiles from the seven cancer-negative lymph nodes (LNneg1-7) and expression profiles from two melanoma (MEL1, MEL3) samples. Pearson correlation analyses indicated that the correlation between arrays was higher between the LNpos and the MEL samples than between the LNpos and LNneg samples (data not shown). Further analysis also revealed significant differences between the miRNA expression profiles of one positive lymph node (LNpos1A) and those of the two other positive lymph nodes (LNpos2B and 3B) for a subset of miRNAs (data not shown). Therefore, data were reanalyzed with the positive lymph nodes separated into two distinct groups for the analysis (LNposA for LNpos1A and LNposB for LNpos2B and 3B). Under these new conditions, hierarchical clustering analysis, as well as principal component analysis on the significantly differentially expressed miRNAs between the four different groups of samples confirmed the heterogeneity between LNposA and LNposB (FIG. 6). The existence of two subgroups within the positive lymph node samples was confirmed by further real time RT-PCR analysis. The LNpos1A sample was from a patient with a primary melanoma and regional metastasis, but no distant metastasis. The melanoma was invasive through the reticular dermis into the subcutaneous tissue (Clark's level V), and seven of eight lymph nodes from the patient were cancer-positive. The LNpos3B sample was from a patient with recurrent melanoma, and the LNpos2B sample was from a patient with a cancer of unknown primary origin (CUP). As the histopathological reports of the primary tumors were very similar in all cases (Table 9), these data suggest that recurrent melanoma and CUP differ from first-step metastatic tumors at the molecular level.

As a consequence of the heterogeneity within the positive lymph nodes, comparisons of miRNA expression between cancer-positive and cancer-negative lymph nodes were made separately with the two LNpos subgroups, LNposA (Table 11) and LNposB (Table 12).

Seventy miRNAs (36 verified and 34 predicted) were determined to have significantly higher average expression levels in LNpos1A sample as compared to their average expression levels in LNneg samples (log2Diff (LNpos1A vs LNneg) ≥1 and t-test (LNpos1A vs LNneg) <0.05) (Table 11). Among the verified miRNAs, hsa-miR-211 and - 375 were expressed between 90- and 140-fold higher in LNpos1A than in LNneg; seven miRNAs (hsa-miR-203, -200b, -200c, -509, -513, -429, and -767-5p) were expressed at levels between 20- and 50- fold higher in LNpos1A than in LNneg; and 15 miRNAs had average expression levels between 5- and 20- fold higher in LNpos1A compared to LNneg (Table 11). In addition, 14 miRNAs had average expression levels between 2- and 5- fold lower in LNpos1A than in LNneg. Among the predicted miRNAs, three (hsa-asg-5638_st2, - 1296_st1, -7637_st2) were expressed at levels at least 10-fold higher in LNpos1A than in LNneg; and four miRNAs (hsa-asg-4803_st2, -11338_st2, -1519_st1, and -14208_st1) were expressed at levels between 5- and 10- fold higher in LNpos1A than in LNneg samples (Table 11).

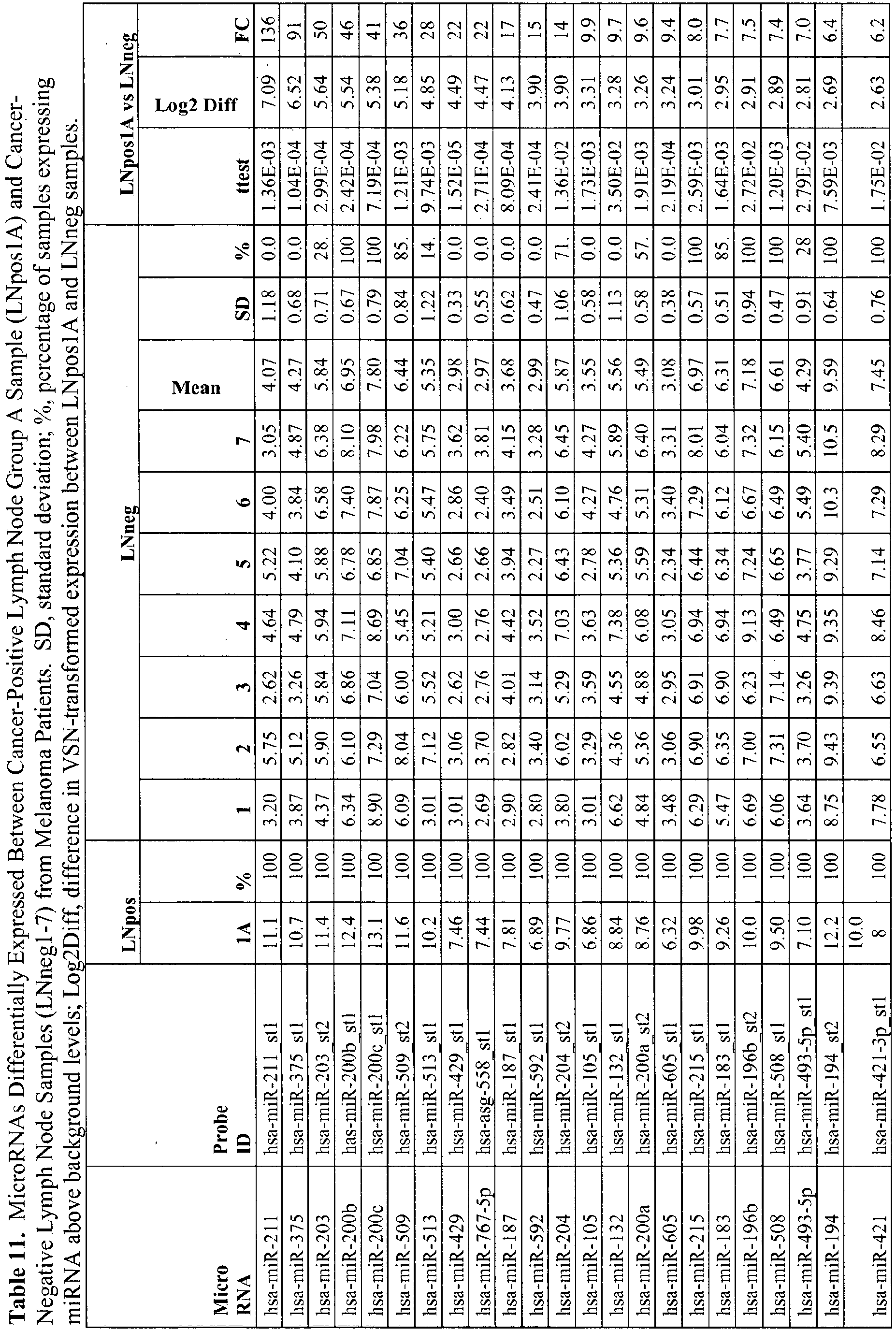

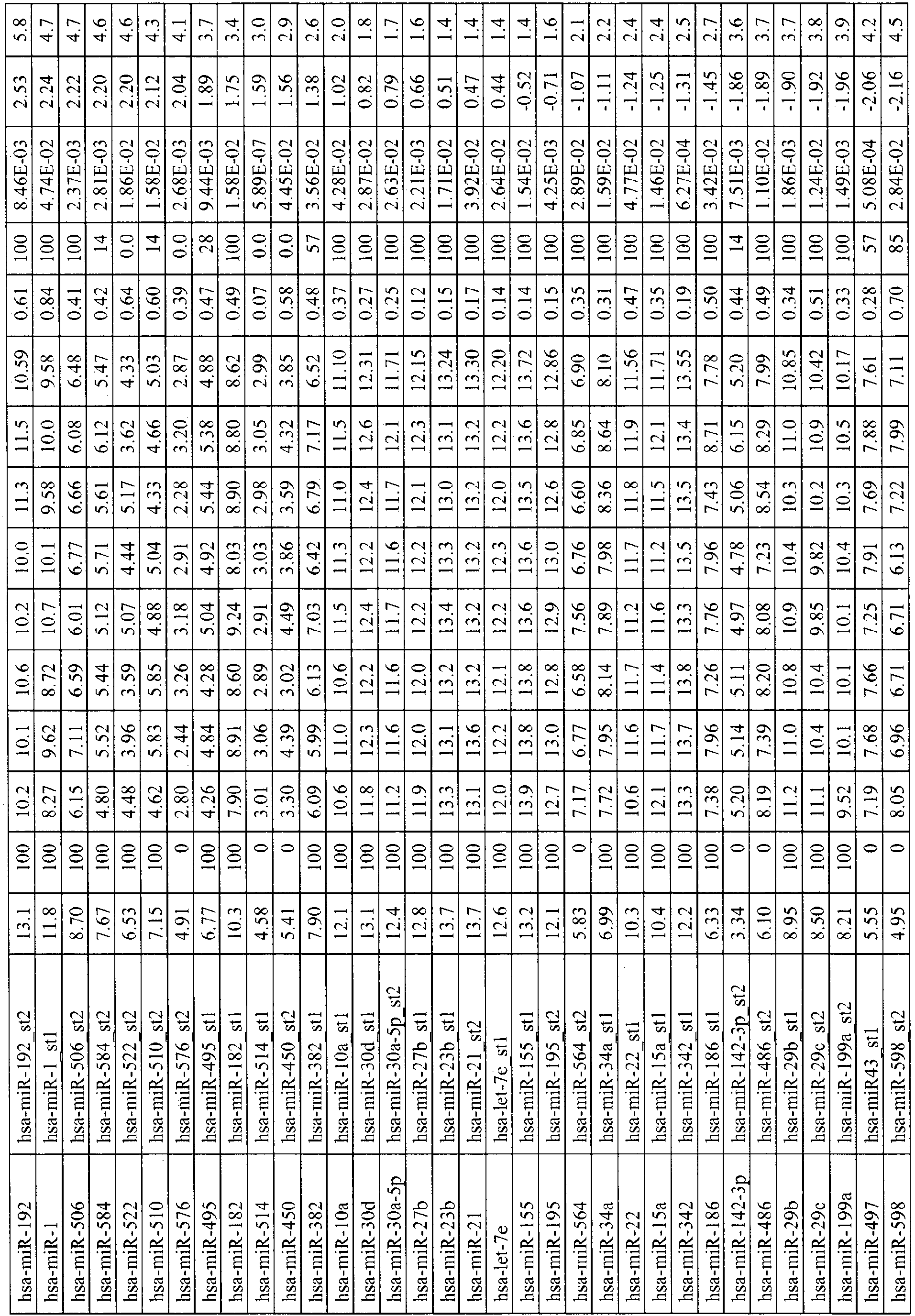

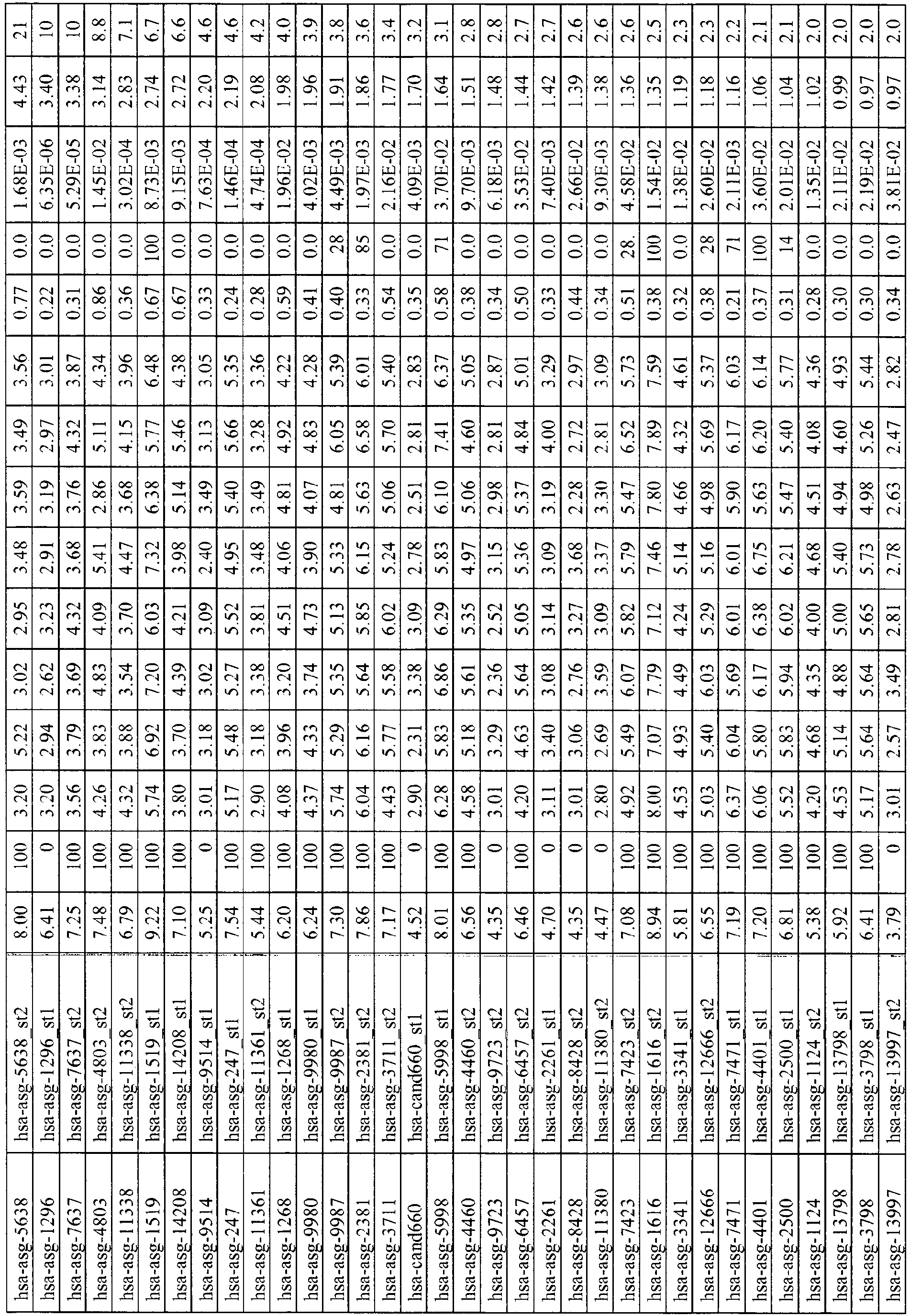

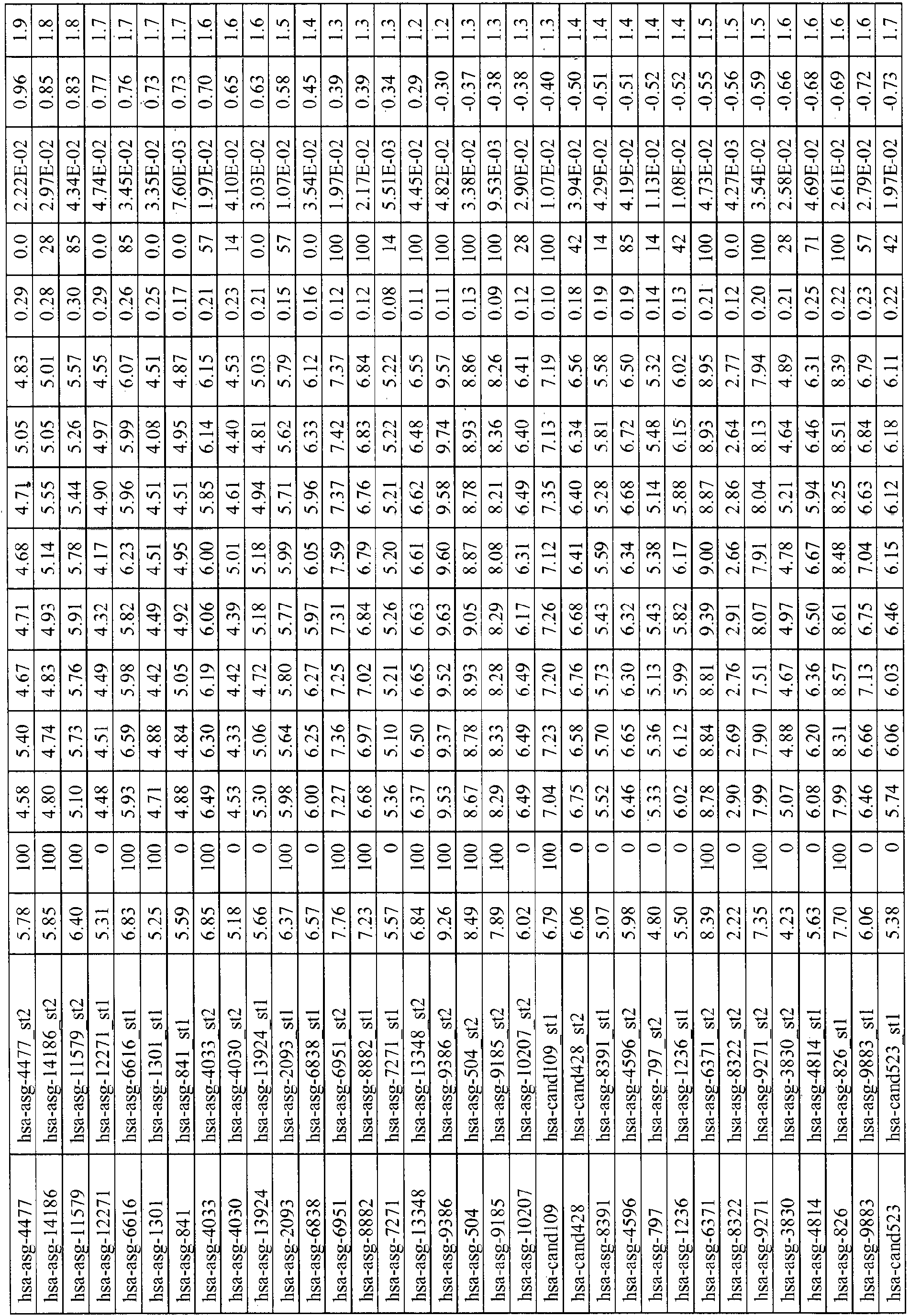

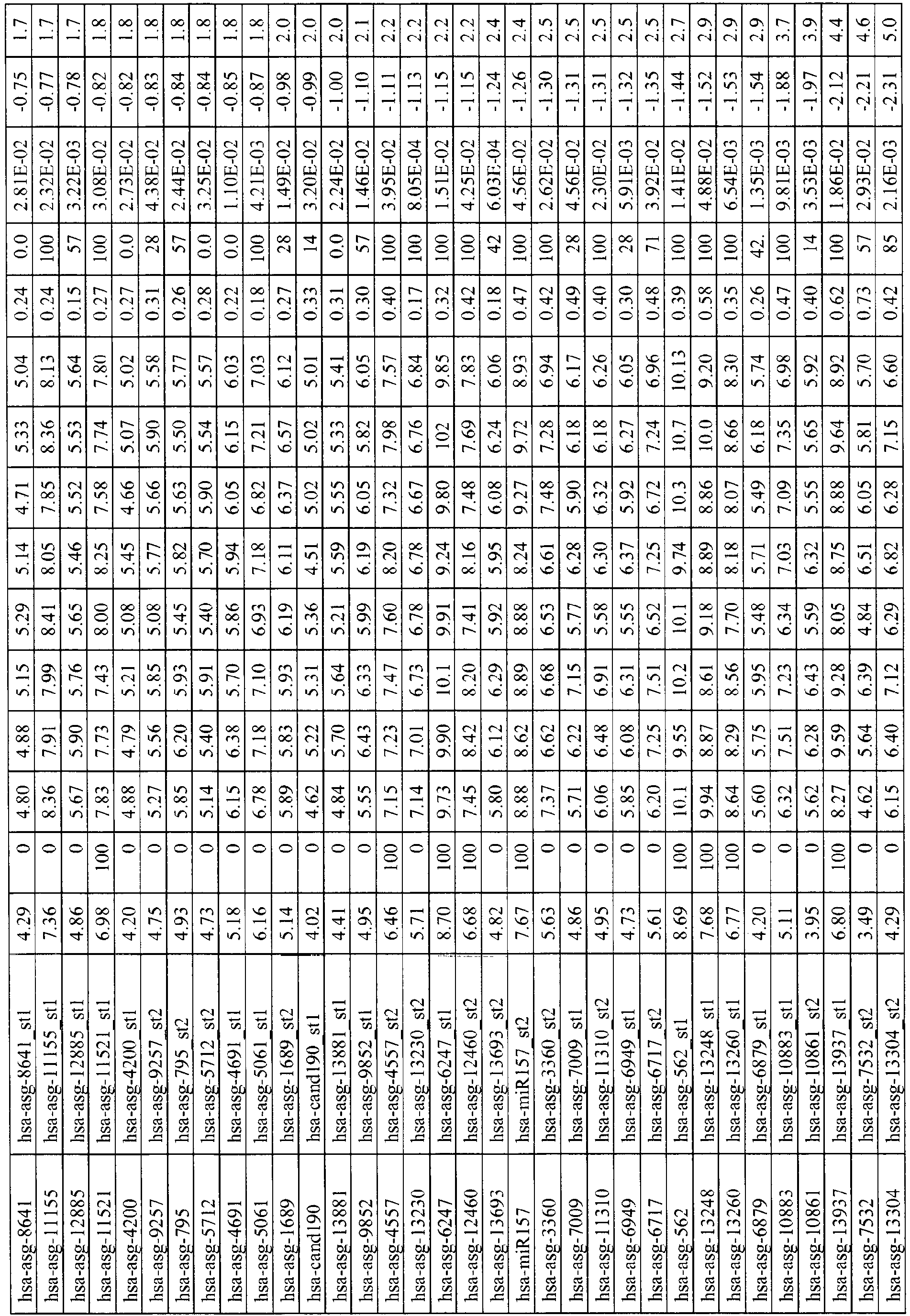

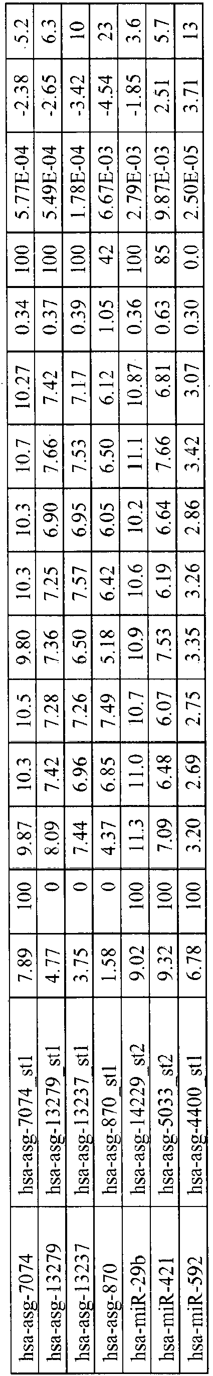

A comparison of miRNA expression between LNneg and LNposB (LNpos2B and LN pos3B) samples is shown in Table 12. Thirty six human miRNAs (13 verified and 23 predicted) were determined to have significantly higher average expression levels in LNposB samples than in LNneg (log2Diff(LNposB vs LNneg)≥1, t-test(LNposB vs LNneg)<0.05). Among the verified miRNAs, three (hsa-miR-211, -513, and -509) were expressed at levels between 25- and 52-fold higher in LNposB than in LNneg, and four miRNAs (hsa-miR-584, - 204, -132, and -506) were expressed at levels between 5- and 8- fold higher in LNposB samples. In addition, 85 miRNAs were expressed at levels at least 2-fold lower in LNposB samples. Among these, five miRNAs (hsa-miR-31, -1, -335, -85, and -192) had average expression levels between 15- and 25- fold lower in LNposB samples than in LNneg samples (Table 12). Among the 23 differentially expressed predicted miRNAs, hsa-asg-5638_st2 was expressed at levels at least 18-fold higher in LNposB than in LNneg, and four miRNAs (hsaasg-5630_st2, -11338_st2, -9186_st1, and -1946_st1) were expressed at levels between 5-and 8-fold higher in LNposB than in LNneg samples (Table 12).

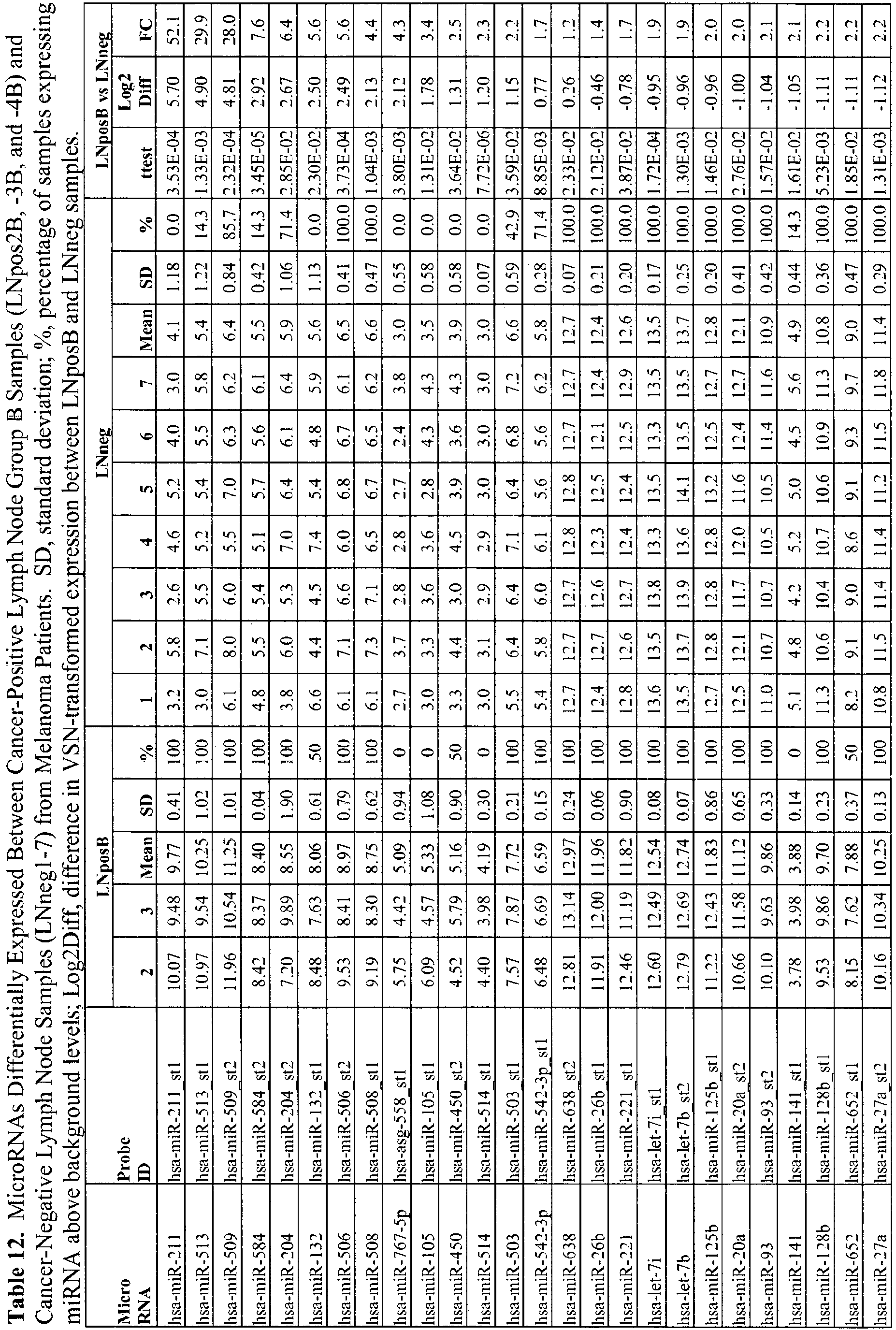

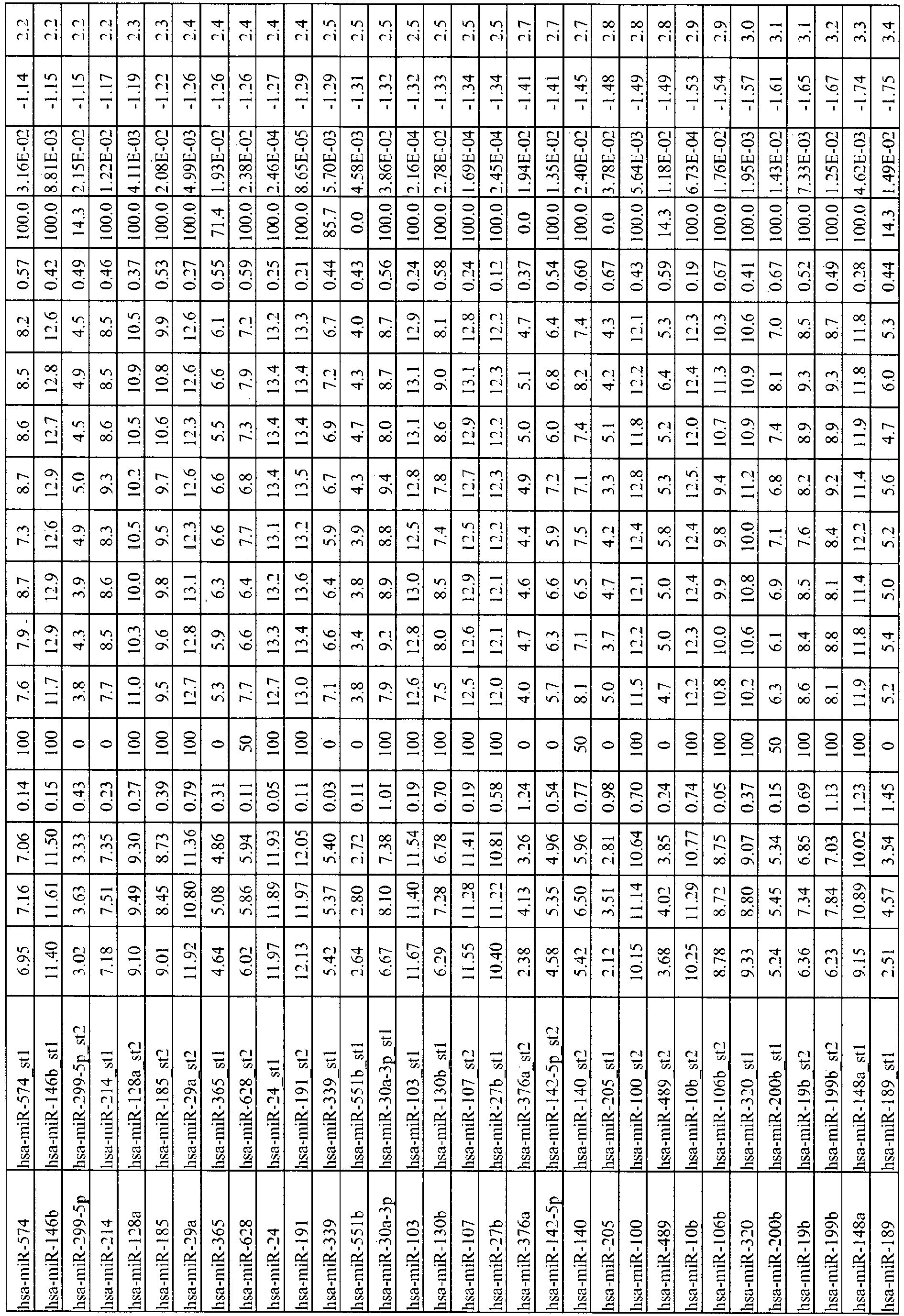

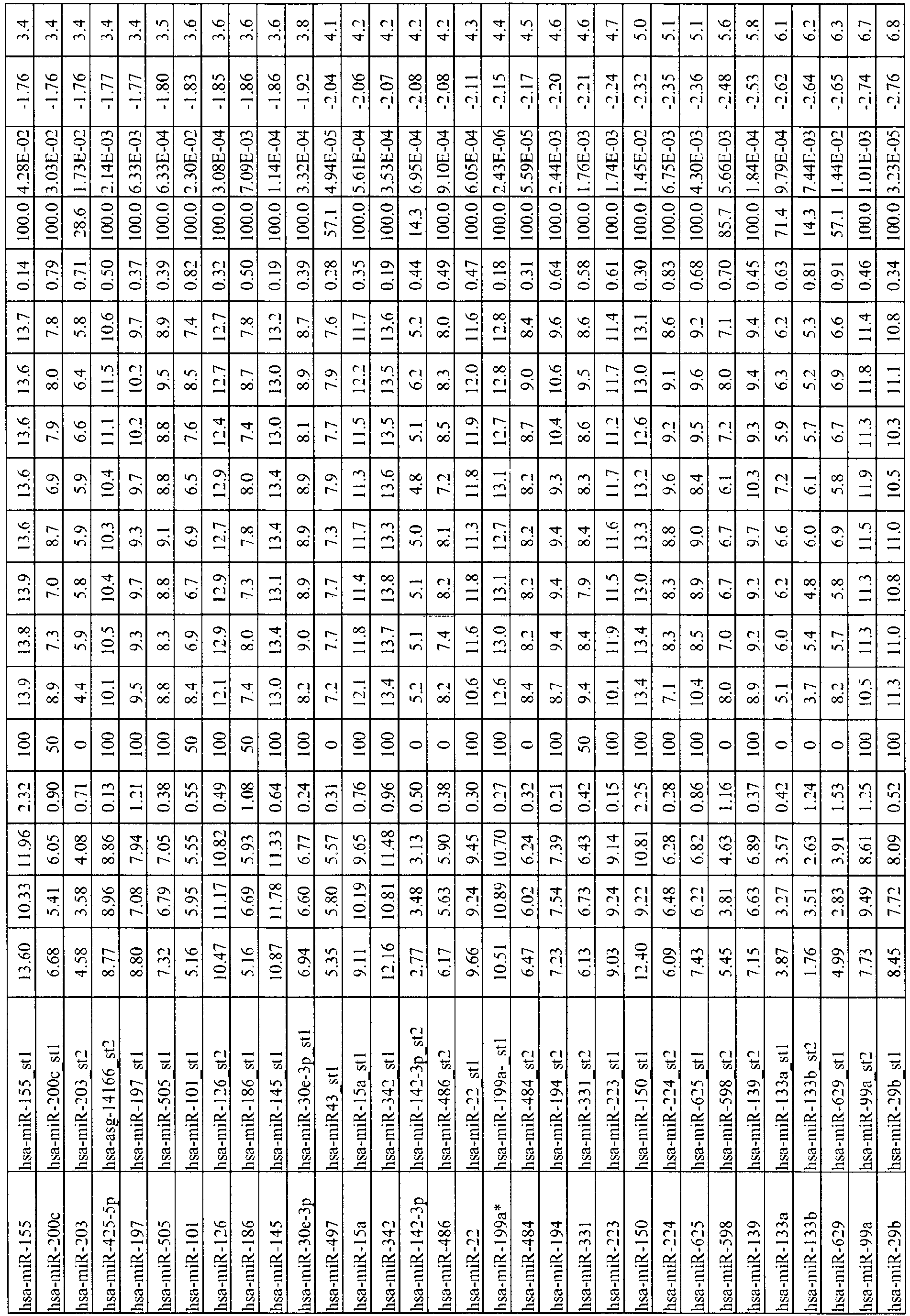

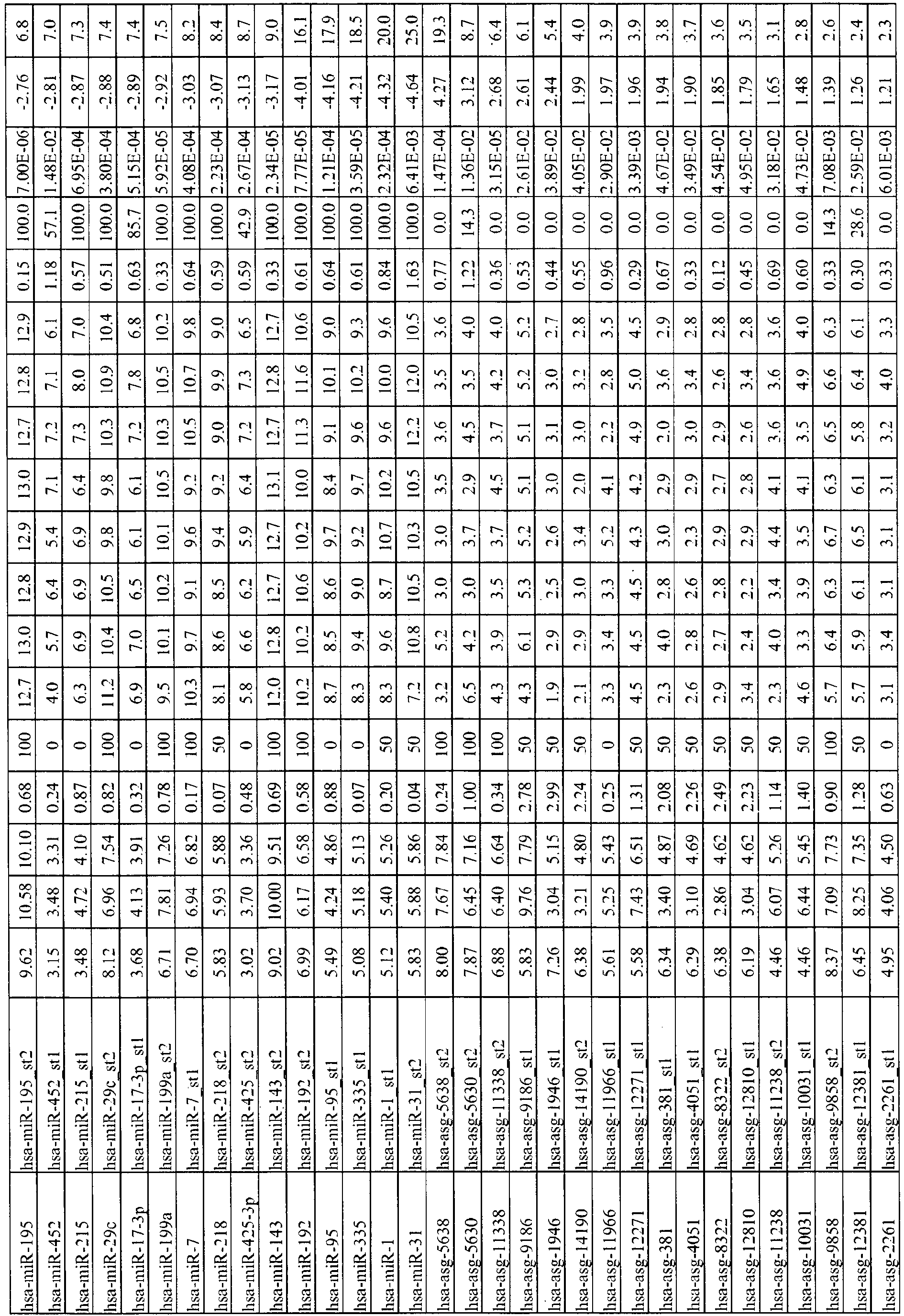

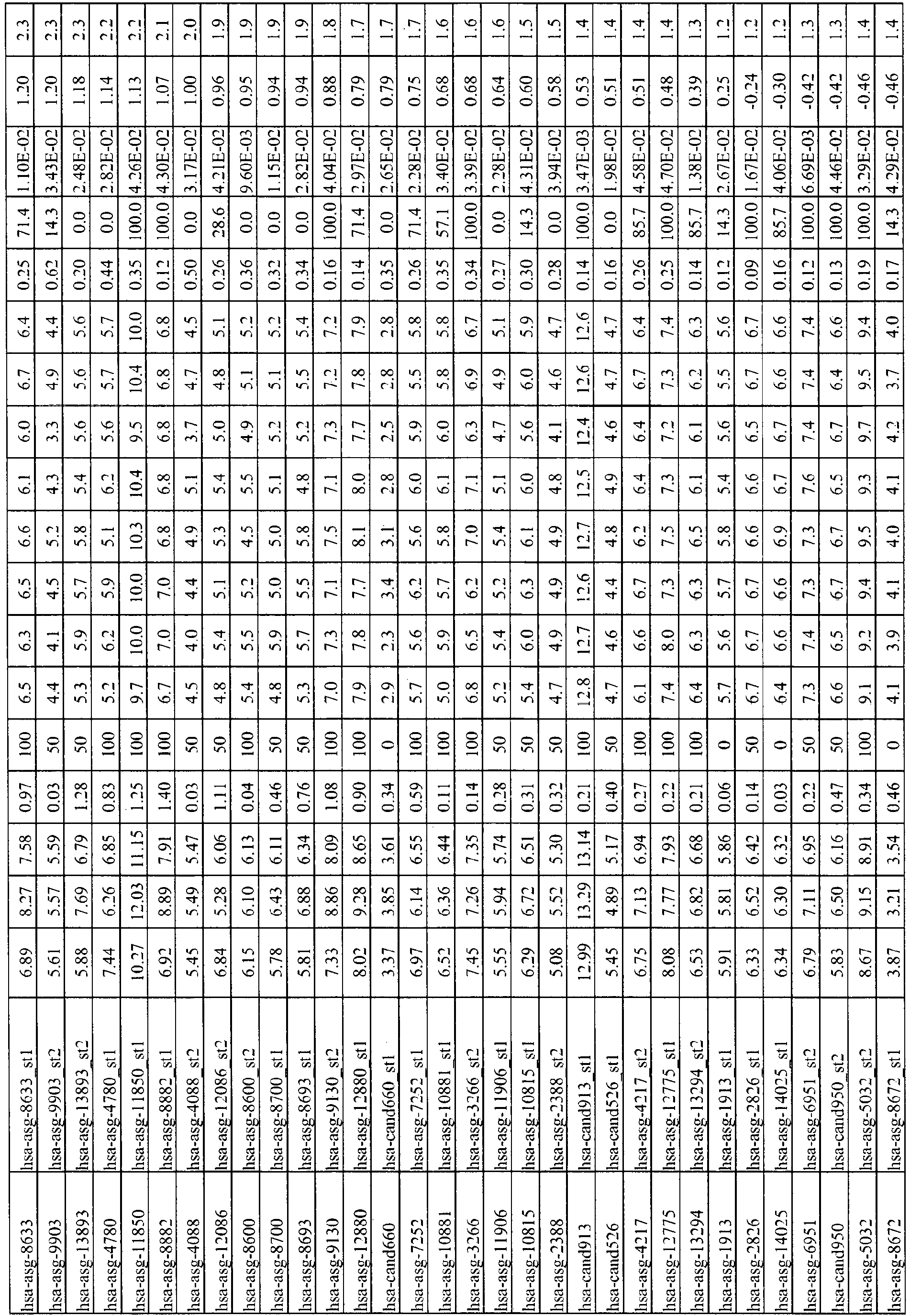

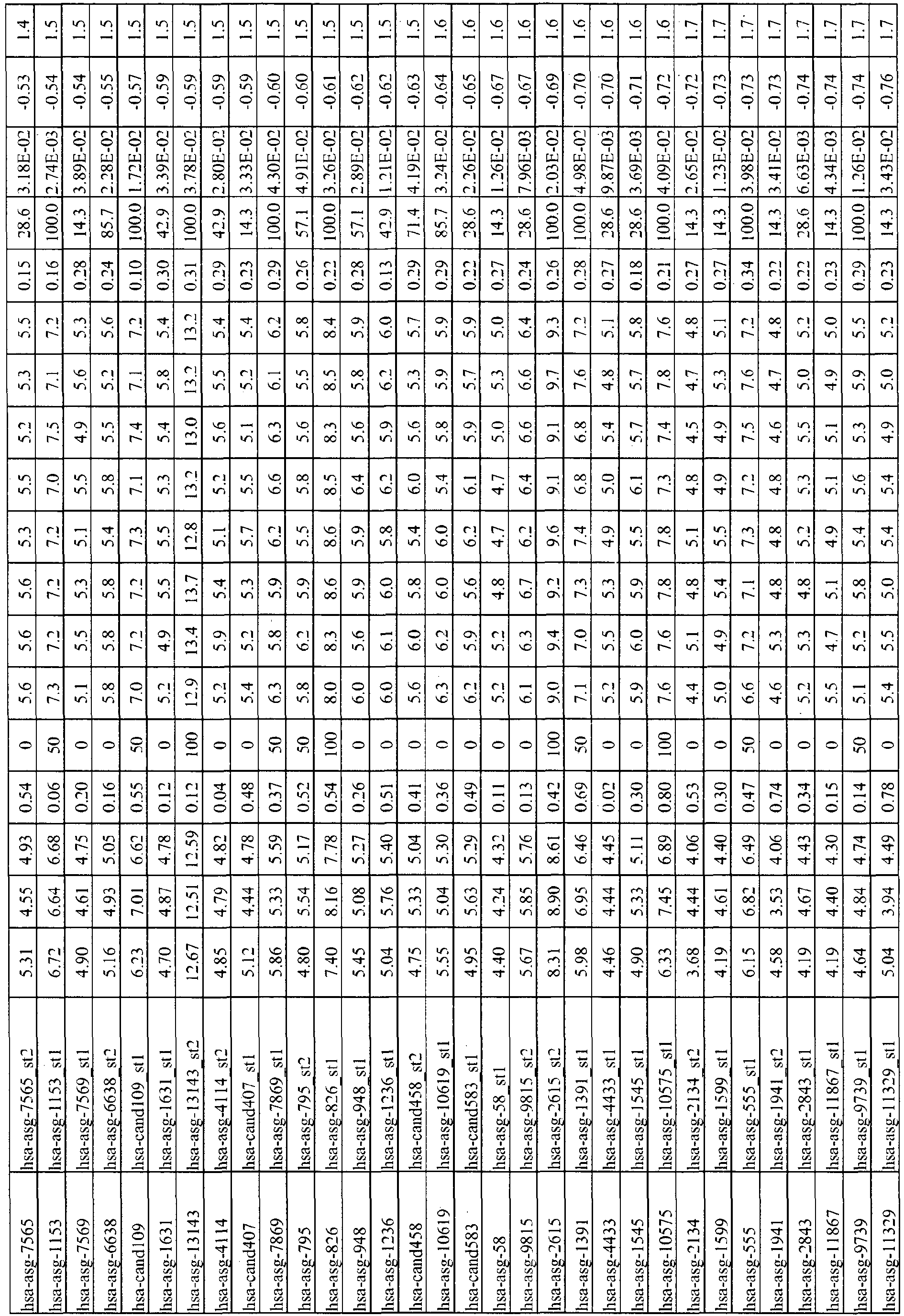

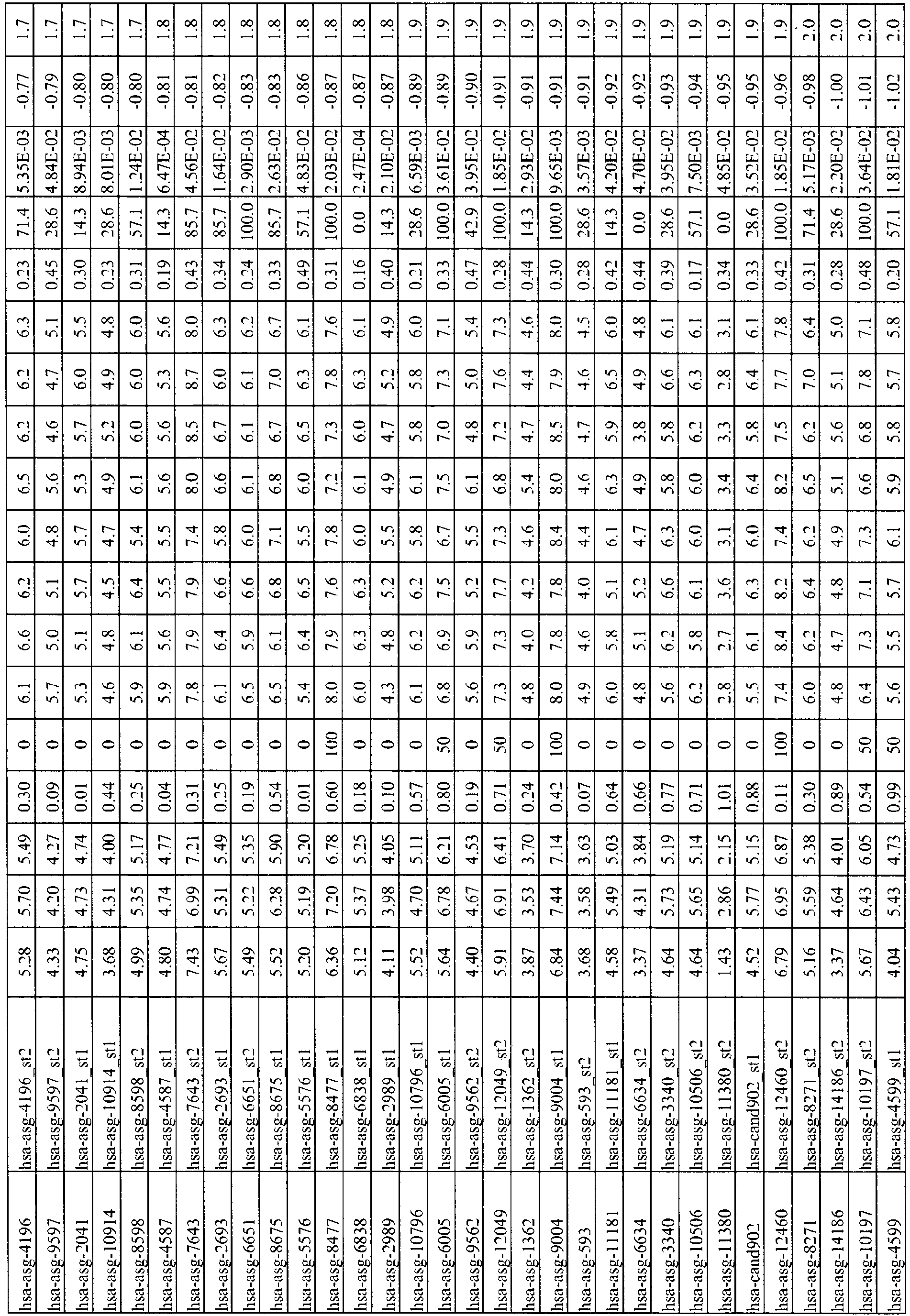

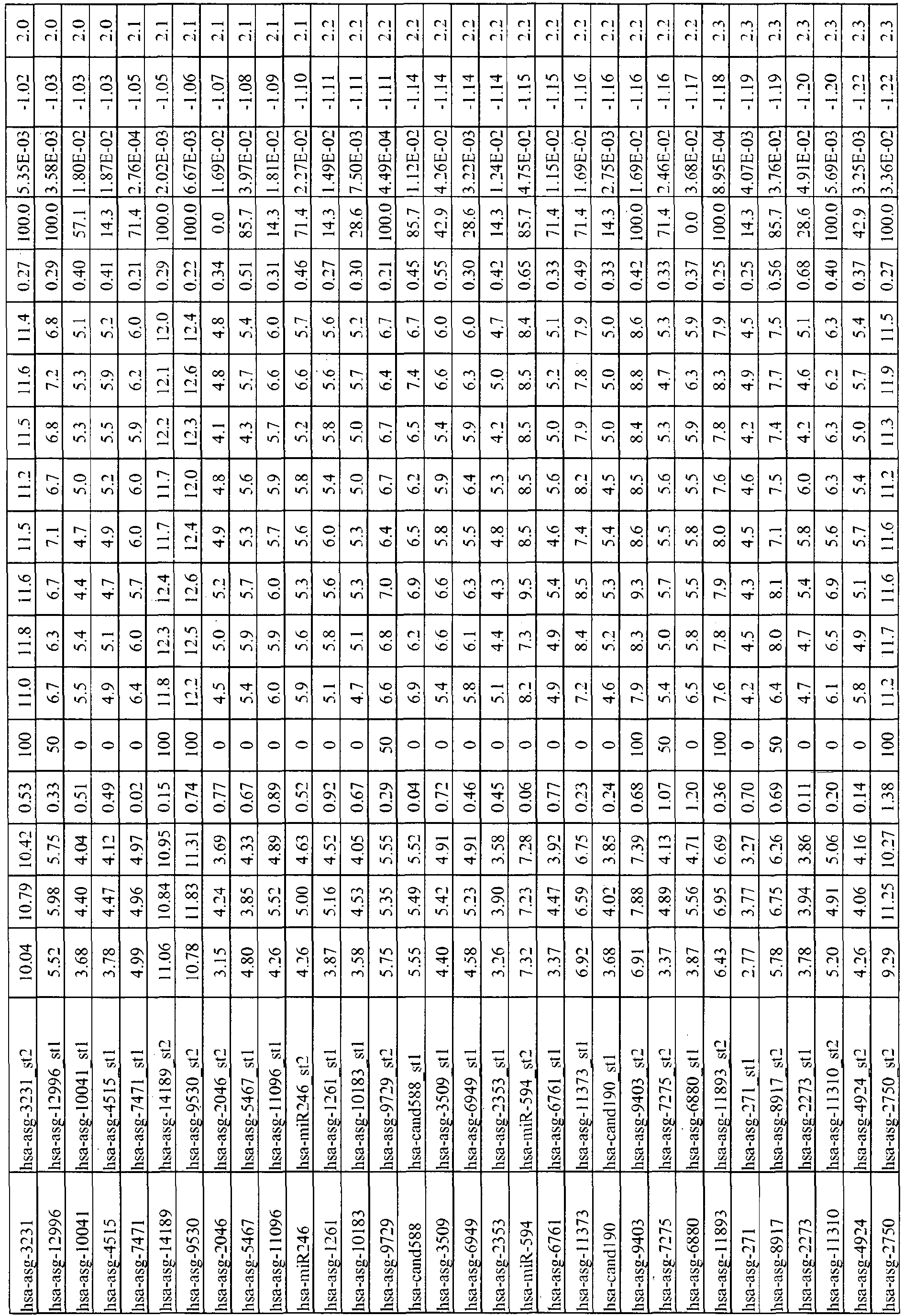

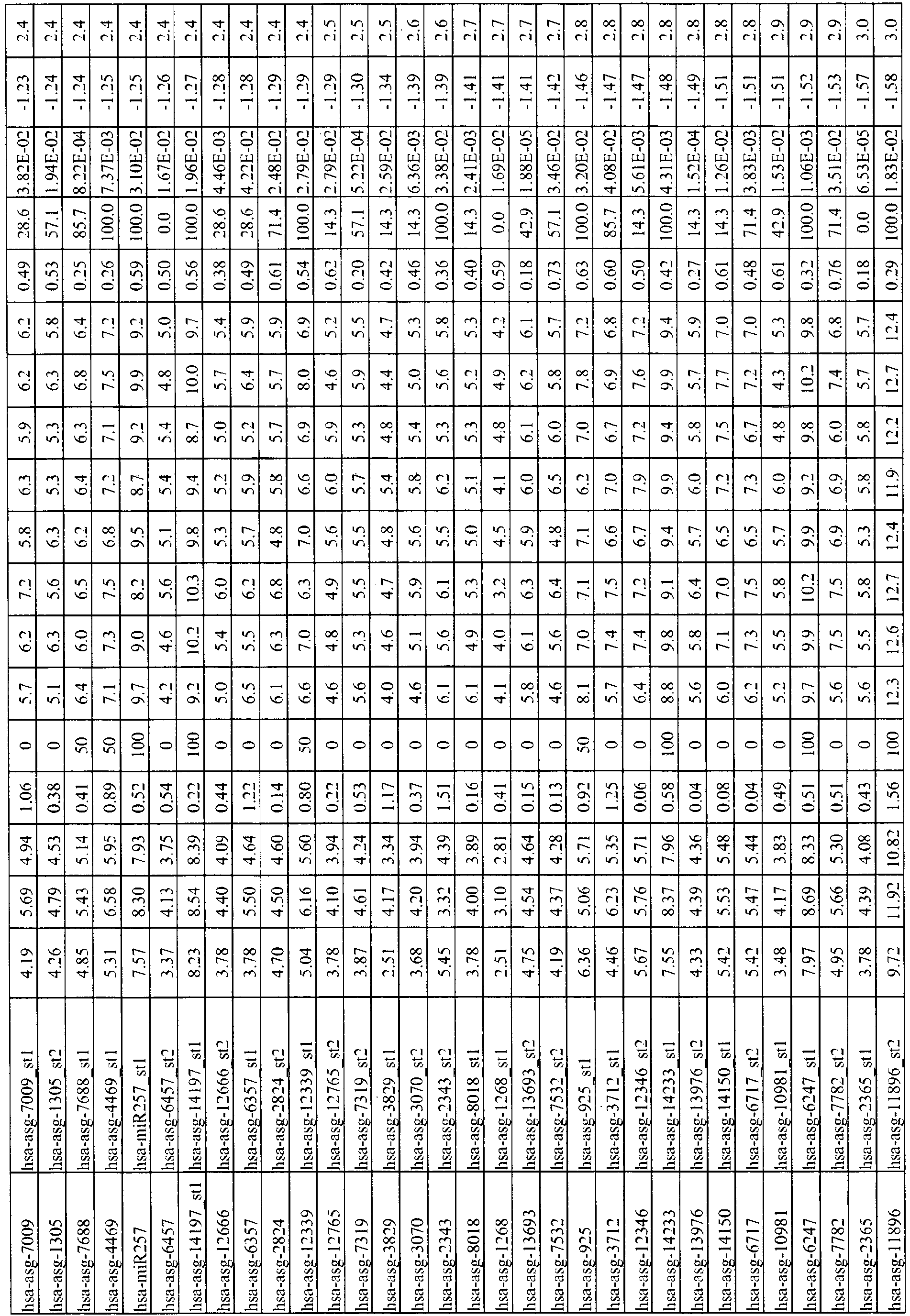

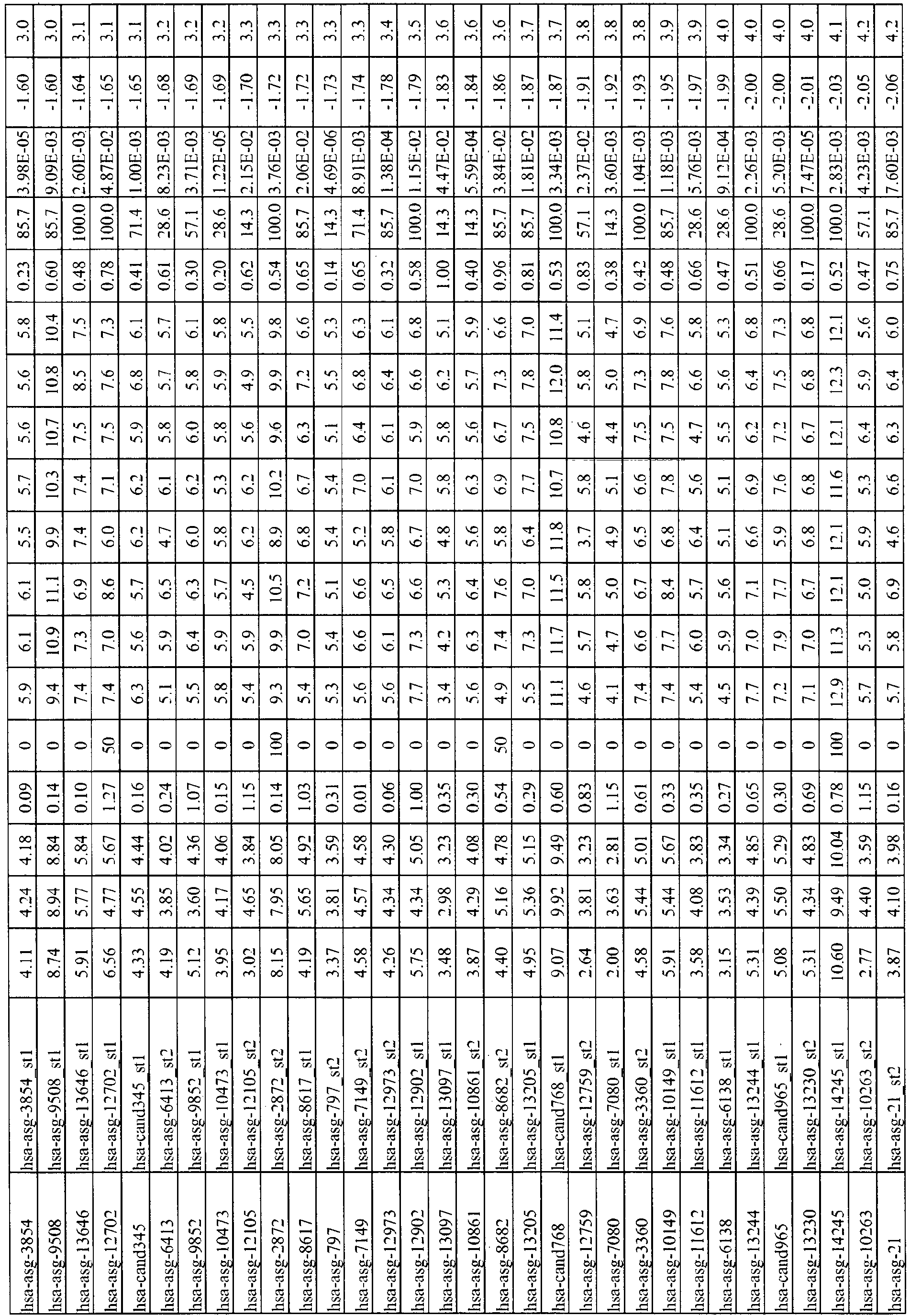

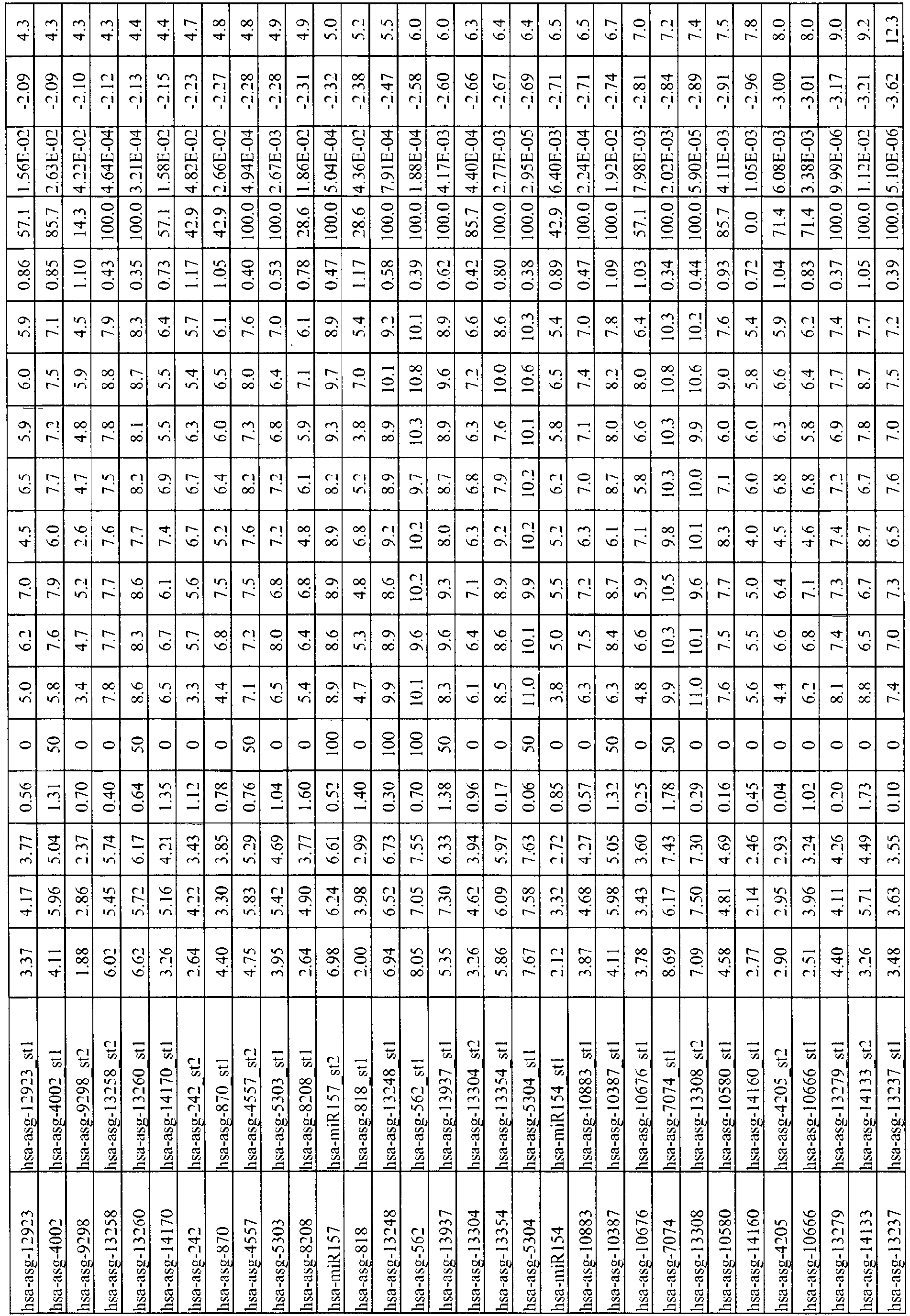

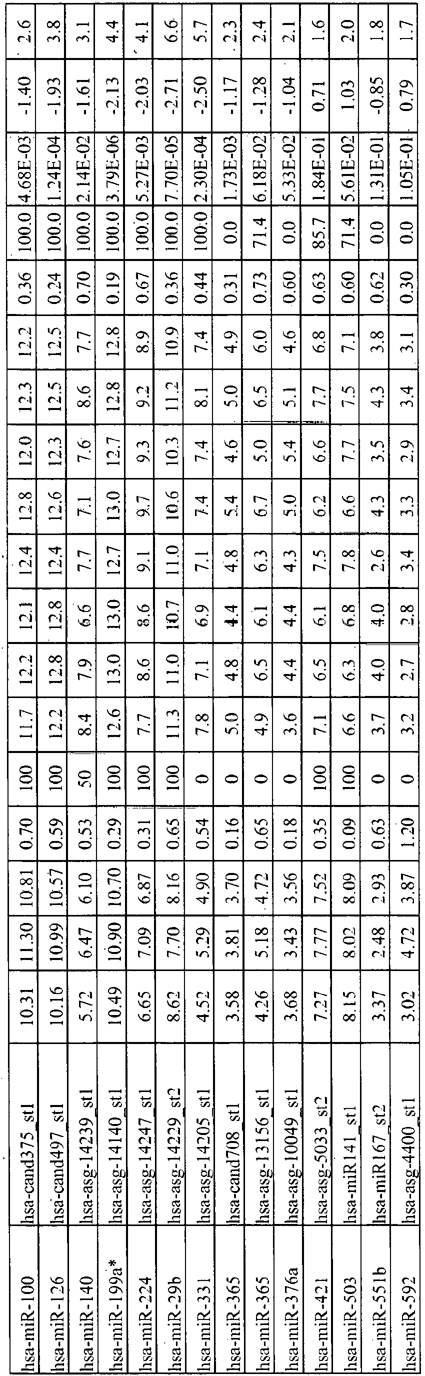

### EXAMPLE 12:

### MIRNAS DIFFERENTIALLY EXPRESSED BETWEEN MELANOMA SAMPLES AND LNNEG AND BETWEEN LNPOS AND LNNEG FROM MELANOMA PATIENTS

The inventors identified the common miRNAs that are differentially expressed between melanoma (MEL) samples and cancer-negative lymph nodes (LNneg) and between cancer-positive (LNpos) and cancer-negative (LNneg) lymph nodes from melanoma patients. For this analysis, differentially expressed miRNAs were identified as having a p-value <0.05 and fold-change >2. These miRNAs represent particularly useful biomarkers for monitoring melanoma metastasis to lymph nodes.

The inventors found 21 miRNAs to be differentially expressed between both MEL and LNneg samples (Table 10) and between LNpos1A and LNneg samples (Table 11). The miRNAs include hsa-miR-211, -203, -200b, -200c, -509, -513, -429, -187, -132, -605, =183, - 508, -493-5p, 421-3p, -506, -584, -576, -495, -182, -514, and -382). Among these, four miRNAs (hsa-miR-203, -211, -509, and -513) had average expression levels between 20- and 160-fold lower in LNneg samples compared to either MEL (Table 10) or LNpos1A samples (Table 11). Among the predicted miRNAs, the inventors identified three (hsa-asg-5638_st2, -11338_st2, and -1296_st1) that were expressed at levels between 5- and 110-fold lower in LNneg samples compared to either MEL (Table 10)or LNpos1A samples (Table 11).

The inventors found that eight verified miRNAs (hsa-miR-211, -513, -509, -584, - 132, -506, -508, and -514) were expressed at lower levels in LNneg samples compared to either MEL (Table 10) or LNposB (Table 12) samples. Among these, three miRNAs (hsa-miR-211, -513, and -509) had average expression levels between 20- and 85- fold lower in LNneg samples than in MEL (Table 10) or LNposB (Table 12) samples. Among the predicted miRNAs, eleven (hsa-asg-5638_st2, 5630_st2, -11338_st2, -9186_st1, -12271_st1, -10031_st1, -12381_st1, -2261_st1, -8633_st1, -4780_st1, and -11850_st1) were expressed at lower levels in LNneg samples compared to MEL (Table 10) or LNposB (Table 12) samples. Among these, four miRNAs (hsa-asg-5638_st2, -5630_st2, -11338_st2, and -9186_st1) had average expression levels between 5-and 100-fold lower in LNneg samples than in MEL (Table 10) or LNposB (Table 12) samples.

Together, the inventors found a total of eight verified miRNAs (hsa-miR-211, - 513, -509, -584, -132, -506, -508, and -514) that were expressed at lower levels in LNneg samples compared to either MEL (Table 10), LNpos1A (Table 11), or LNposB (Table 12) samples. Among these, four miRNAs (hsa-miR-211, -513, -509, and -132) had average expression levels between 5- and 140- fold lower in LNneg samples than in MEL, LNpos1A, or LNposB samples. The inventors also identified two predicted miRNAs (hsa-asg5638_st2, and -11338_st2) that had average expression levels between 5- and 25- fold lower in LNneg samples than in either MEL, LNpos1A, or LNposB samples.

### EXAMPLE 13:

### QRT-PCR VERIFICATION OF DIFFERENTIALLY EXPRESSED MICRO-RNAS IN MELANOMA TISSUES AND LYMPH NODES FROM MELANOMA PATIENTS

The inventors also evaluated the differential expression of 13 selected miRNAs in MEL, LNpos, and LNneg frozen biopsy samples (Table 8, Table 9) by qRT-PCR. qRT-PCR reactions were performed using TaqMan® MicroRNA Assays (Applied Biosystems; Foster City, CA, USA) specific for thirteen of the most differentially expressed miRNAs between LNposA/LNposB and LNneg samples and/or between MEL and LNneg samples (hsa-miR-205, -203, -211, -509, -200c, -506, -508, -200b, -514, -584, -429, -592, and -375). Assays were performed according to the manufacturer's instructions with 15 ng of input total RNA from one of 12 samples (MEL1, MEL 3, LNneg1-7, LNpos1A, LNpos2B, LNpos3B) (Table 8, Table 9) and were incubated in a 7900HT Fast Real-Time PCR system (Applied Biosystems, Foster City, CA, USA). Initial data analysis was done using a 7500 Fast System SDS V2.3 software (Applied Biosystems). miR-16, whose expression level did not change between the different samples, was used for normalization. Results of qRT-PCR expression analysis for the thirteen miRNAs were compared with those obtained from microarray expression analysis (FIGs.7-9).

qRT-PCR quantification confirmed the differential expression of ten miRNAs (hsa-miR-205, -203, -211, -200c, -508, -509, -584, -514, -200b, and -506) between MEL and LNneg samples (FIGs. 7A-7C). These results are consistent with the results obtained after array analysis (FIGs. 7A-7C).

Upon qRT-PCR analysis of the thirteen miRNAs in LNpos1A and LNneg samples, twelve were found to be expressed at significantly lower levels in the LNneg samples and one (hsa-miR-205) was not. These results correspond to those observed with microarray quantification (FIGs. 8A-8C).

Upon qRT-PCR analysis of the thirteen miRNAs in LNposB and LNneg samples, six (hsa-miR-211, -509, -506, -508, -514, and -584) were found to be expressed at significantly lower levels in the LNneg samples (FIGs. 9A-9C). As observed for the LNpos1A and LNneg samples, miR-205 was not found to be expressed at significantly different levels between LNposB and LNneg samples. As observed with microarray analysis, miR-429, -592, and -375 were not expressed at significantly different levels in LNposB and LNneg samples when quantified by qRT-PCR. This is in contrast to what was observed for these miRNAs in the LNposA and LNneg samples. In addition, three miRNAs, miR-203, - 200b, and -200c, were not found differentially expressed between LNposB and LNneg samples, although these miRNAs were found differentially expressed between LNposA and LNneg samples (FIGs 8A-8C and FIGs. 9A-9C). These observations suggest that qRT-PCR quantification of miR-200c, -203, -200b and -375 is a useful assay for distinguishing LNposA group lymph nodes from LNposB group lymph nodes.

These data demonstrate that both qRT-PCR and microarray analysis are effective methods for quantifying miRNA expression when evaluating metastasis of melanoma to lymph nodes.

### EXAMPLE 14:

### MICRORNAS DIFFERENTIALLY EXPRESSED BETWEEN MELANOMA SAMPLES AND LNPOS FROM MELANOMA PATIENTS

Cancer cells present in lymph nodes are considered to be cells arising from a primary tumor, which have metastasized to that node. In the case of melanoma, a positive lymph node is defined by the AJCC as one containing an aggregate of melanoma cells 2 mm or greater in size. It is expected that miRNA expression in a primary melanoma tumor and in metastatic inguinal lymph nodes share similarities. However, miRNA expression differences may also exist as the consequence of genetic alterations linked to the cancer cells' ability to metastasize. The differences in miRNA expression between the primary melanoma and the metastatic melanoma cells in lymph nodes represent clinically significant surrogate markers for the propensity of a primary lesion to metastasize. Such differentially expressed miRNAs also represent targets for replacement therapy. To identify any such differences, the inventors compared miRNA expression between three primary melanoma tumors (MEL) and cancer-positive lymph nodes (LNposA, LNposB), using frozen biopsy specimens (Table 8, Table 9).

The inventors identified 8 miRNAs (2 verified, 6 predicted) that were expressed at significantly lower levels in the LNpos1A sample than in the MEL samples (p-value<0.05) with at least a 2-fold-change in expression level (log2Diff (MEL vs LNpos1A) ≥1) (Table 13). Interestingly, hsa-miR-205 was not found significantly differentially expressed by array analysis despite a differential expression of at least 182-fold between MEL samples and the LNpos1A sample. This result may be due to the presence of only one sample in the LNposA group and the result of a lack of power of the analysis. qRT-PCR analysis, however, confirmed the differential expression between the LNpos1A sample and the MEL samples (FIG- 8A-8C). In addition, a total of 14 miRNAs (5 verified and 9 predicted) were identified that were expressed at lower levels in the MEL samples compared to the LNpos1A sample (Table 13). Among these, four miRNAs (hsa-miR-215, -592, -518c*, and -522) had average levels of expression between 5- and 25- fold lower in the MEL samples compared to the LNpos1A sample (Table 13).

The inventors also identified 55 miRNAs (29 verified, 26 predicted) with significant differential expression between melanoma samples and the LNposB group of cancer-positive lymph nodes (p-value<0.05 and log2Diff (MEL vs LNposB) ≥1) (Table 14). Among the verified miRNAs, two (hsa-miR-205 and -203) had average expression levels at least 500-fold higher in MEL samples than in LNposB samples and five miRNAs (hsa-miR-31, -200c, -218, -223, and -493-5p) had average expression levels between 5- and 60- fold higher in MEL than in LNposB samples (Table 14). Among the 26 predicted miRNAs, only one (hsa-asg-14160_st1) was expressed at least 10-fold higher in MEL samples than in LNposB samples (Table 14). No verified miRNA was found expressed at a lower level in MEL samples compared to LNposB samples. Only three predicted miRNAs had average expression levels lower by 2- 3- fold in MEL than in LNposB samples (Table 14).

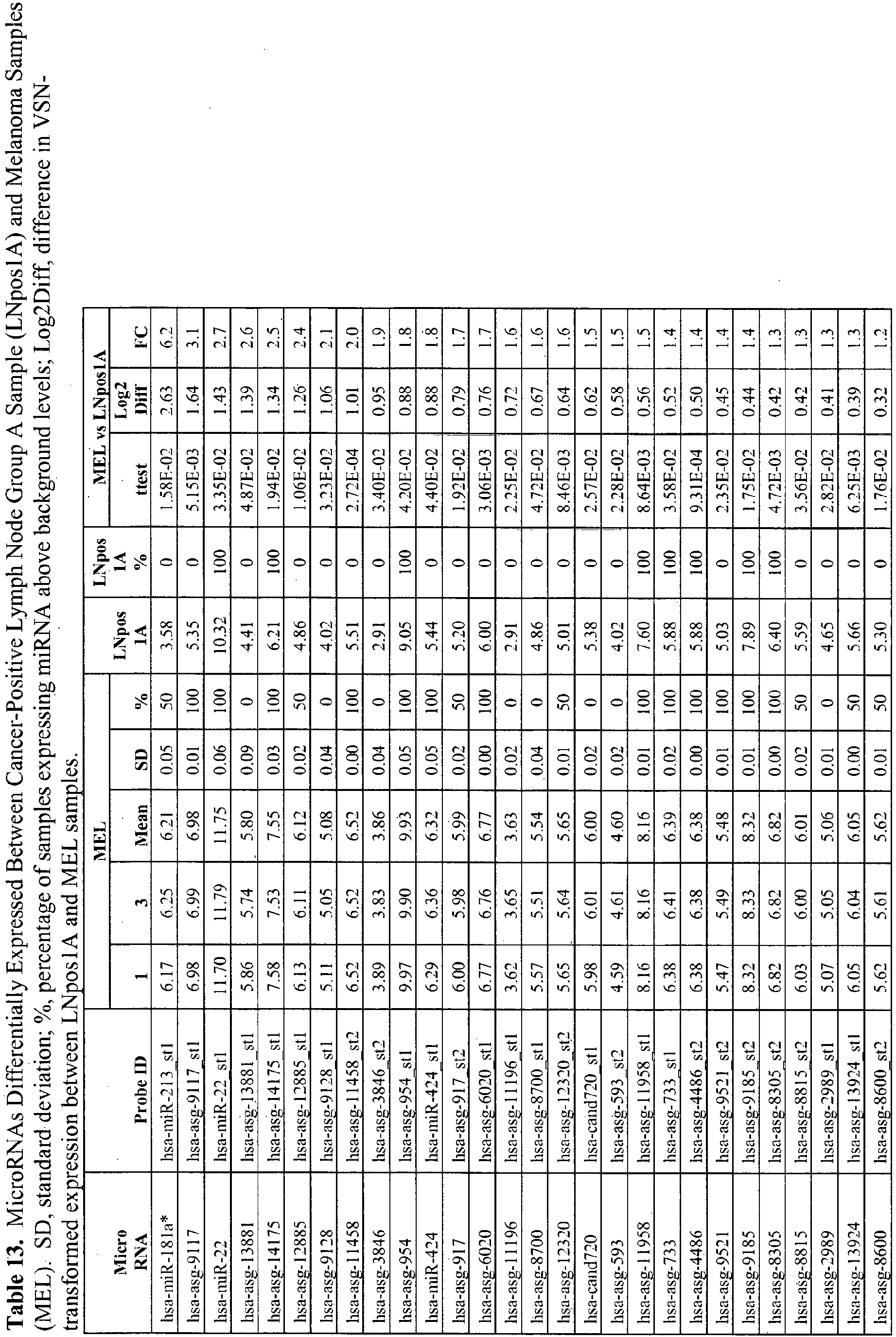

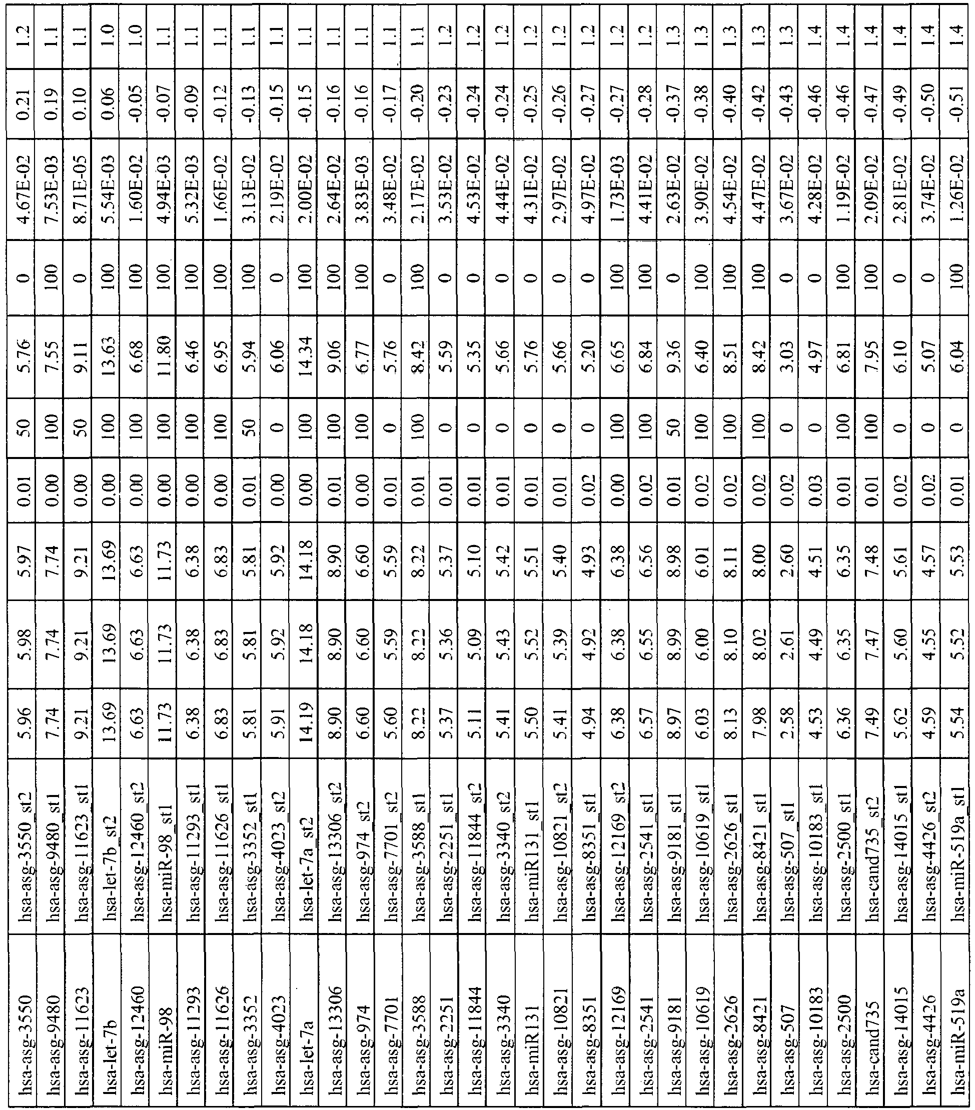

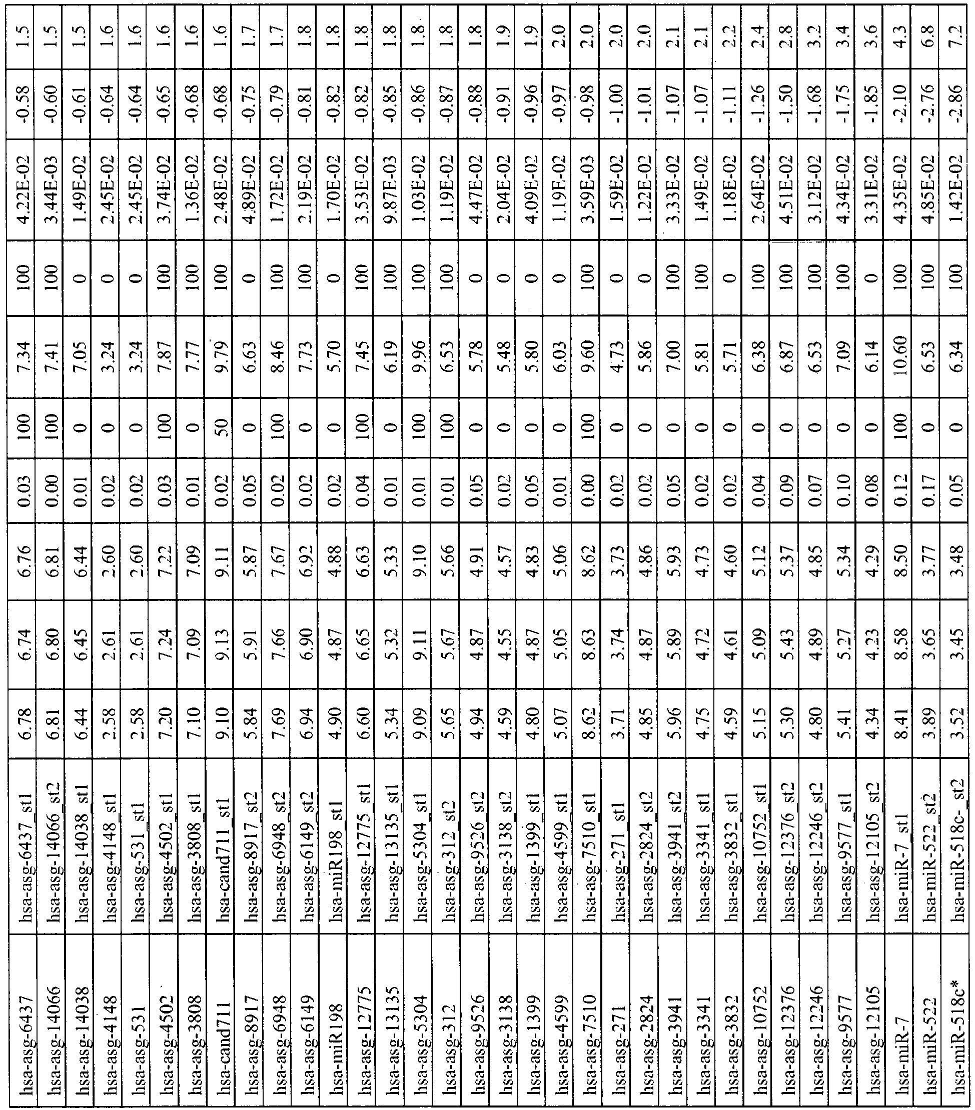

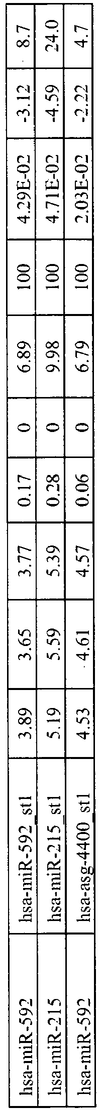

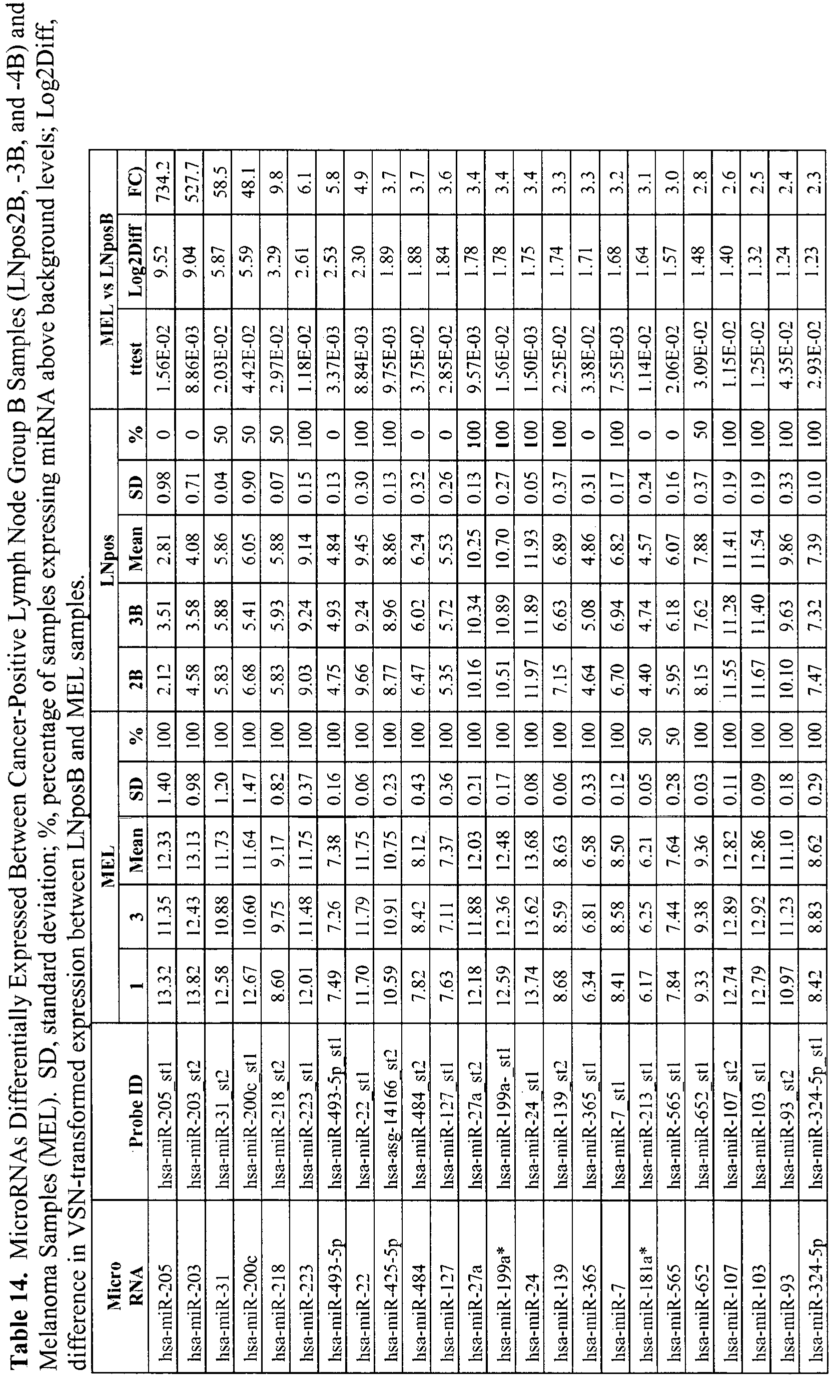

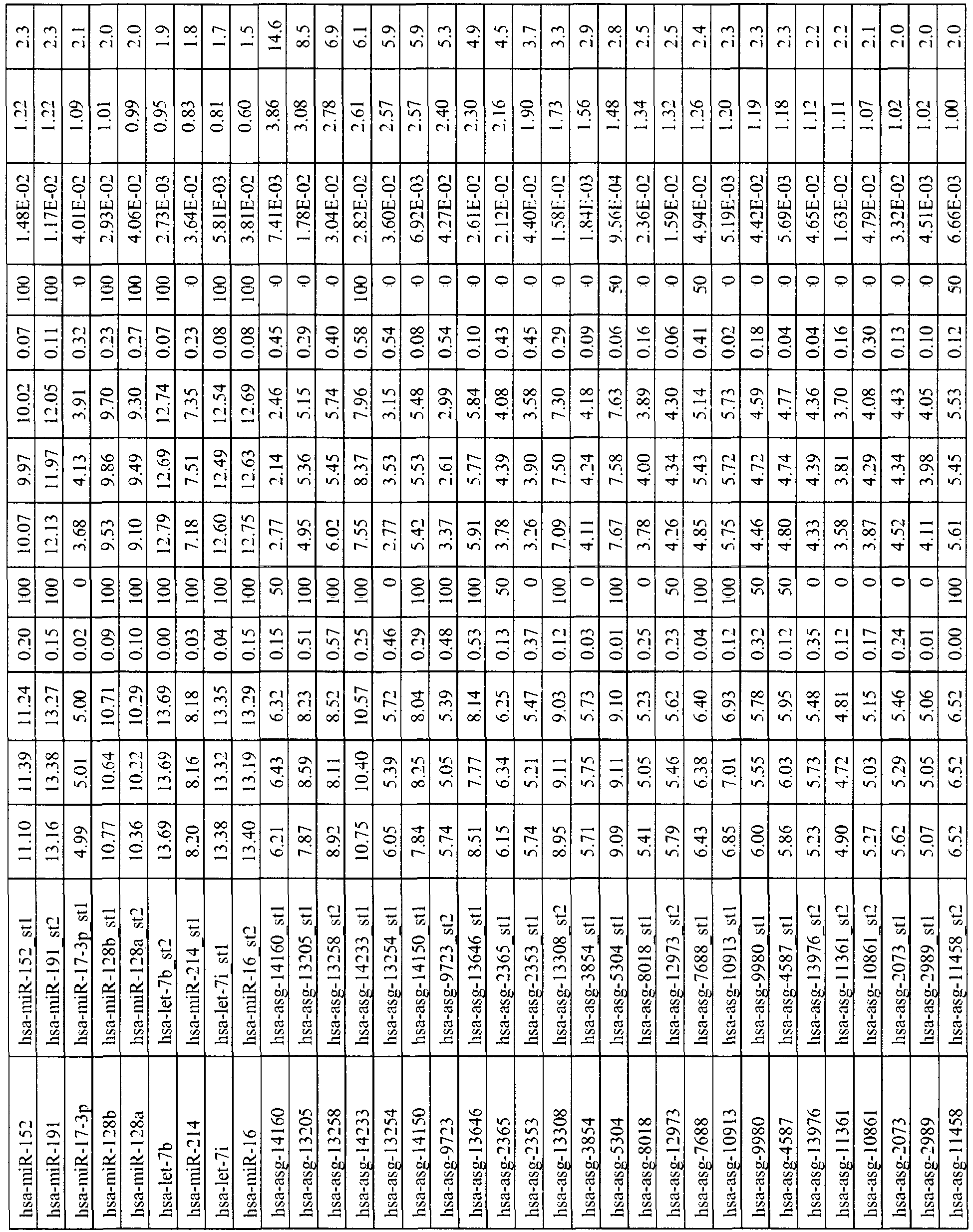

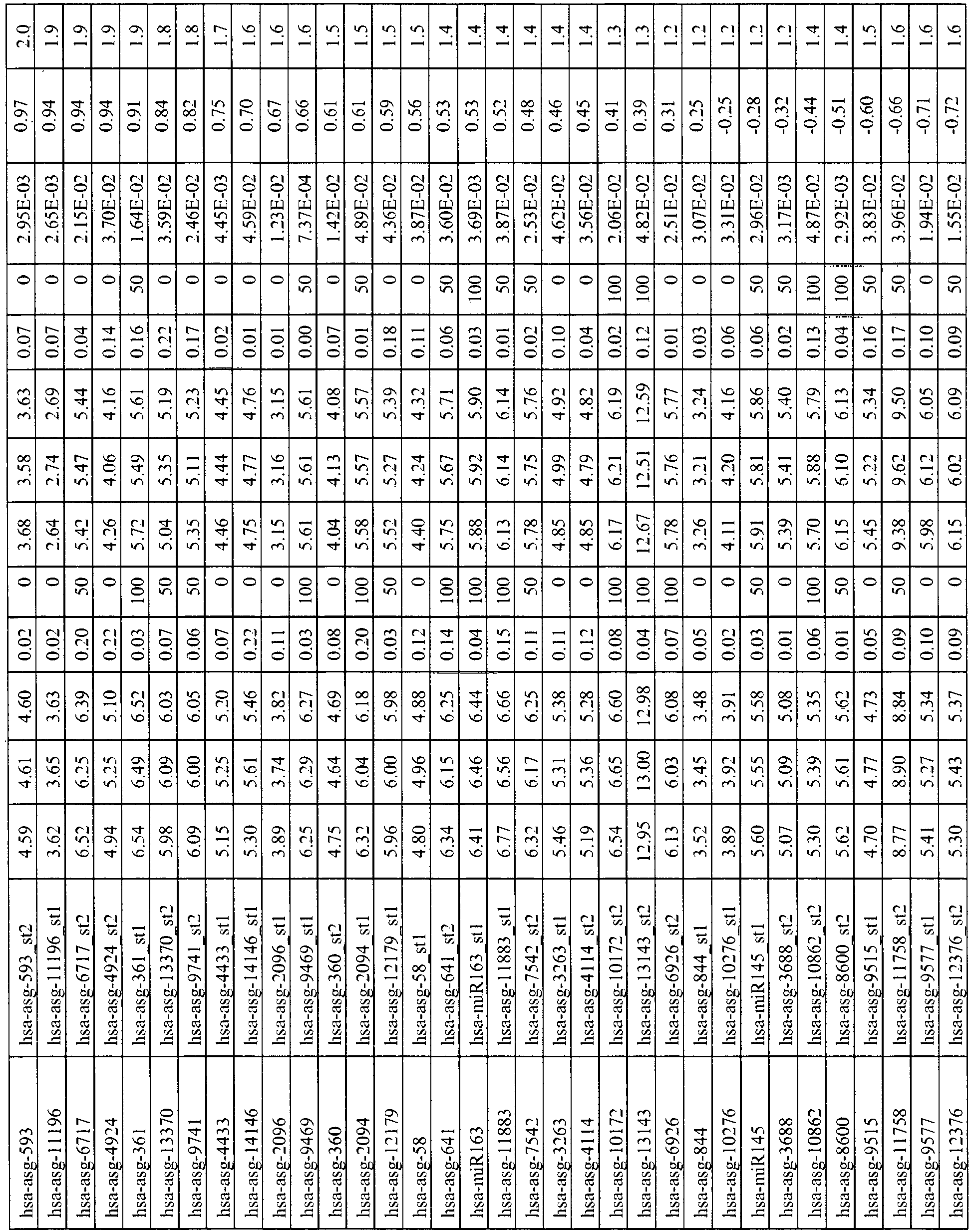

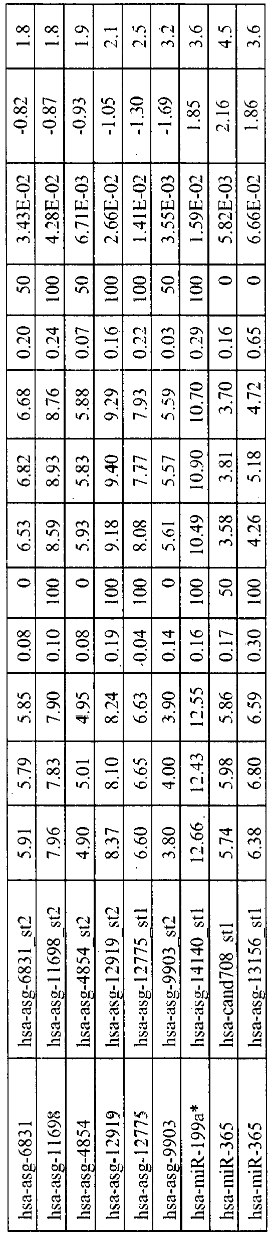

### EXAMPLE 15:

### COLON CARCINOMA SAMPLES AND RNA ISOLATION

FirstChoice® human total RNA from nine normal (cancer-negative) lymph node (cat#AM7894) and four normal colon (cat# AM7986) specimens was purchased from Ambion (Ambion, Inc). Seven cancer-positive lymph nodes from colon cancer patients were purchased from ProteoGenex (ProteoGenex, Inc.) (Table 15). Total RNA isolation was performed, using the mirVana miRNA Isolation Kit (Ambion, Inc.), following the manufacturer's protocol. Purified total RNA was quantified using a Nanodrop® ND-1000 (Nanodrop Technologies).

**Table 15. Patient and tissue pathology information for seven cancer-positive lymph node samples from patients with metastatic colon cancer. TNM Stage (Sobin and Wittekind, 2002).**

| **Sample** | **Age (yr)/Sex, Ethnicity** | **Histological Diagnosis** | **TNM Stage** | **Overall Stage Group** | **Lymph Node Metastases** |
|---|---|---|---|---|---|
| LNpos4 | 55/Male Caucasian | adenocarcinoma moderately to poorly differentiated | T3N2M0 | III | 6/8 lymph nodes |
| LNpos5 | 64/Male Caucasian | adenocarcinoma moderately differentiated | T3N1M1 | IV | 2/6 lymph nodes |
| LNpos6 | 74/Male Caucasian | adenocarcinoma moderately differentiated | T3N2M0 | III | 4/6 lymph nodes |
| LNpos7 | 73/Male Caucasian | adenocarcinoma moderately to poorly differentiated | T3N1M0 | III | 2/6 lymph nodes |
| LNpos8 | 59/Female Caucasian | adenocarcinoma well to poorly differentiated | T3N1M0 | III | 3/5 lymph nodes |
| LNpos9 | 75/Female Caucasian | adenocarcinoma moderately differentiated | T3N2M0 | III | 4/4 lymph nodes |
| LNpos12 | 67/female Caucasian | adenocarcinoma well to moderately differentiated | T3N2M1 | IV | 4/4 lymph nodes |

### EXAMPLE 16:

### MIRNAS DIFFERENTALLY EXPRESSED BETWEEN NORMAL COLON TISSUE AND CANCER-NEGATIVE LYMPH NODES

To identify miRNAs that may be useful markers for metastasis of colon cancer to lymph nodes, the inventors identified miRNAs that are expressed at significantly higher levels in normal colon tissue samples than in cancer-negative lymph node samples.

miRNAs that are differentially expressed between normal colon (NCo) samples and cancer-negative lymph node (LNneg) samples, both described above in Example 15, were determined using one of two microarray platforms: *mir*Vana™ miRNA Bioarrays V1.0 (Ambion) or the DiscovArray™ miRNA Expression Service (Asuragen). The DiscovArray™ miRNA Expression Service uses the miRChip V1.0 (described in Example 2 above).

***mirV*ana Bioarrays.** The *mir*Vana V1.0 Bioarrays contain 377 individual miRNA probes, including 281 human miRNAs from Sanger database (V.6), 33 novel human miRNAs (Ambi-miRs), and 63 mouse or rat miRNAs from the miRBase Sequence Database (microrna.sanger.ac.uk) (Griffiths-Jones *et al.,* 2006). miRNA expression analysis was performed as described previously(Shingara *et al.,* 2005), with the following modifications. microRNA-containing fractions were purified from 10 µg of total RNA, using a flashPAGE™ Fractionation Apparatus (Ambion). Purified small RNAs were labeled with Cy5 (GE Healthcare Life Sciences, Piscataway, NJ, USA) and hybridized to microarrays according to the manufacturer's instructions. Following hybridization, the arrays were scanned using the Axon® GenePix 400B scanner and associated GenePix software (Molecular Devices Corporation, Sunnyvale, CA).

***mir*Vana Bioarray data processing.** Raw array data were normalized with the variance stabilization method (VSN) (Huber *et al.,* 2002). VSN is a global normalization process that stabilizes the variance evenly across the entire range of expression and utilizes calibration of signal followed by transformation of data to a generalized natural logarithmic space *in lieu* of the traditional logarithm base 2 transformation. Absolute values and differences in VSN-transformed expression are denoted by H and ΔH, respectively, and were used for all subsequent data analyses. Differences in normalized expression values between samples (ΔH) can be transformed to a generalized fold-change via exponentiation base e. These values will exhibit a compression for small differences in expression. For each array, the minimum observable threshold was determined by examining the foreground minus background median intensities for 'EMPTY' spots. The minimum threshold was defined as 5% symmetric trimmed mean plus 2 standard deviations across all 'EMPTY' spots on an individual array. For an overview of miRNA array processing and analysis, see Davison *et al.,* (2006).

**Additional data analyses. A** one-way analysis of variance (ANOVA) model was used to test the hypothesis that there was no difference in expression between groups of samples for each miRNA on the array. Pair-wise comparisons for differentially expressed miRNAs identified by ANOVA were then performed to measure relative differences. For each pair of sample types (normal colon and lymph nodes), a two-sample *t*-test assuming equal variance was carried out for every miRNA and multiplicity correction was included to control the false discovery rate at 0.05% (Davison *et al.,* 2006).

**miRChip Microarrays.** microRNA purification and labeling and miRChip expression analysis were performed as described above in Example 2.

In a preliminary study, miRNA expression was determined for three normal colon (NCo) samples and three cancer-negative lymph node (LNneg) samples, using the *mir*Vana Bioarray platform. The inventors identified 20 miRNAs whose average expression levels in the three cancer-negative lymph node samples were significantly lower than their average expression levels in the normal colon samples (p-value <0.05; fold change ≥5) (Table Co2 ). Among these, six miRNAs (hsa-miR-200b, -200a, -200c, -141, -215, and -375) had average expression levels that were at least 100-fold lower in the cancer-negative lymph node sample. Seven miRNAs (hsa-miR-192, -194, -429, -203, -187, and -1 and ambi-miR-7072) had average expression levels that were between 10- and 100-fold lower in the cancer-negative lymph node samples. Seven other miRNAs had average expression levels that were between 5- and 10-fold lower in cancer-negative lymph node samples.

**Table 16. miRNAs identified with mirVana Bioarrays as being differentially expressed between normal colon (NCo) and cancer-negative lymph nodes (LNneg).**

| **microRNA** | **% NCo** | **% LNneg** | **Mean NCo** | **Mean LNneg** | **ΔH NCo vs LNneg** | **Fold Change** | **p-value NCo vs LNneg** |
|---|---|---|---|---|---|---|---|
| hsa-miR-200b | 100 | 33 | 9.47 | 3.14 | 6.33 | 561 | 1.69E-06 |
| hsa-miR-200a | 100 | 0 | 8.26 | 2.02 | 6.23 | 509 | 2.83E-04 |
| hsa-miR-200c | 100 | 100 | 9.70 | 4.75 | 4.95 | 142 | 6.41E-05 |
| hsa-miR-141 | 100 | 33 | 8.05 | 3.13 | 4.93 | 138 | 9.94E-04 |
| hsa-miR-215 | 100 | 67 | 8.25 | 3.38 | 4.87 | 130 | 8.07E-03 |
| hsa-miR-375 | 100 | 0 | 7.00 | 2.26 | 4.73 | 114 | 1.81E-04 |
| hsa-miR-193 | 100 | 100 | 10.38 | 5.78 | 4.60 | 99 | 7.28E-04 |
| hsa-miR-194 | 100 | 67 | 10.25 | 6.17 | 4.08 | 59 | 2.46E-03 |
| hsa-miR-429 | 100 | 0 | 7.26 | 3.29 | 3.97 | 53 | 1.35E-05 |
| hsa-miR-203 | 100 | 0 | 6.79 | 3.29 | 3.49 | 33 | 8.15E-05 |
| hsa-miR-187 | 100 | 100 | 5.65 | 2.28 | 3.37 | 29 | 7.41E-04 |
| ambi-miR-7072 | 100 | 33 | 4.82 | 2.11 | 2.71 | 15 | 1.05E-03 |
| hsa-miR-1 | 100 | 100 | 8.37 | 5.79 | 2.58 | 13 | 6.87E-03 |
| hsa-miR-133a | 67 | 0 | 8.94 | 6.88 | 2.05 | 8 | 2.40E-03 |
| hsa-miR-9 | 67 | 0 | 3.96 | 1.99 | 1.97 | 7 | 2.10E-02 |
| hsa-miR-137 | 100 | 100 | 3.90 | 1.98 | 1.92 | 7 | 9.29E-03 |
| hsa-miR-10a | 100 | 100 | 9.56 | 7.72 | 1.84 | 6 | 8.60E-04 |
| Hsa-miR-422a | 100 | 100 | 7.95 | 6.37 | 1.57 | 5 | 6.78E-04 |
| hsa-miR-124a | 100 | 0 | 4.14 | 2.58 | 1.57 | 5 | 8.34E-03 |
| hsa-miR-422b | 100 | 100 | 9.00 | 7.44 | 1.57 | 5 | 1.93E-04 |

The miRNAs identified as differentially expressed between normal colon samples and cancer-negative lymph nodes represent useful biomarkers for indicating the presence of metastatic colon cancer cells in lymph nodes.

Independently from this experiment, the inventors also performed miRNA expression profiling and comparison with four stage III (T3N2MX) colon cancer samples and four normal colon samples, using the same microarray platform. The results revealed that none of the miRNAs, identified as differentially expressed between these normal colon and colon cancer samples were among the miRNAs most differentially expressed between cancer-negative lymph nodes and colon cancer-positive lymph nodes (data not shown). This observation suggests that, at least in the case of colon cancer, the most differentially expressed miRNAs between normal colon and normal lymph node samples are also representative of the most differentially expressed miRNAs between cancer-positive lymph nodes and cancer-negative lymph nodes.

Next, using the miRChip microarray, the inventors determined miRNA expression for four normal colon samples and nine cancer-negative lymph nodes (including the three normal colon samples and the three cancer-negative lymph nodes analyzed on *mir*Vana Bioarrays in the preliminary study described immediately above).

The inventors identified 200 miRNAs whose average expression levels were significantly different between the normal colon samples (NCo) and the cancer-negative lymph node samples (LNneg) (*t*-test (LNneg vs NCo) <0.05) (Table 17). Fifty of those miRNAs (21 verified, 29 predicted) had average expression levels that were at least 5-fold lower in the cancer-negative lymph node samples than in the normal colon samples (LNneg vs NCo p-value<0.05; log2Diff NCo vs LNneg ≥ 2.35). Among the verified miRNAs, hsa-miR-375 and -429 had average expression levels that were at least 100-fold lower in cancer-negative lymph node samples. Hsa-miR-215 and-200a had average expression levels that were between 50-and 70-fold lower in cancer-negative lymph node samples. Ten miRNAs (hsa-miR-200b, -200c, -141, -194, -203, -200a*, 605, -192, -187, and -133b) had average expression levels that were 10- to 50-fold lower in cancer-negative lymph node samples. Seven miRNAs (hsa-miR-133a, -338, -378, -9, -487b, -495, and -582) had average expression levels that were between 5- and 10-fold lower in cancer-negative lymph node samples. Among the predicted miRNAs, seven (hsa-asg-14153_st2, -14182_st1, -7637_st2, 11715_st2, -12227_st1, -924_st1 and -10131_st2) had average expression levels that were between 10-and 53-fold lower in cancer-negative lymph node samples than in normal colon samples. Twenty-two predicted miRNAs had average expression levels that were between 5- and 10-fold lower in cancer-negative lymph node samples than in normal colon samples.

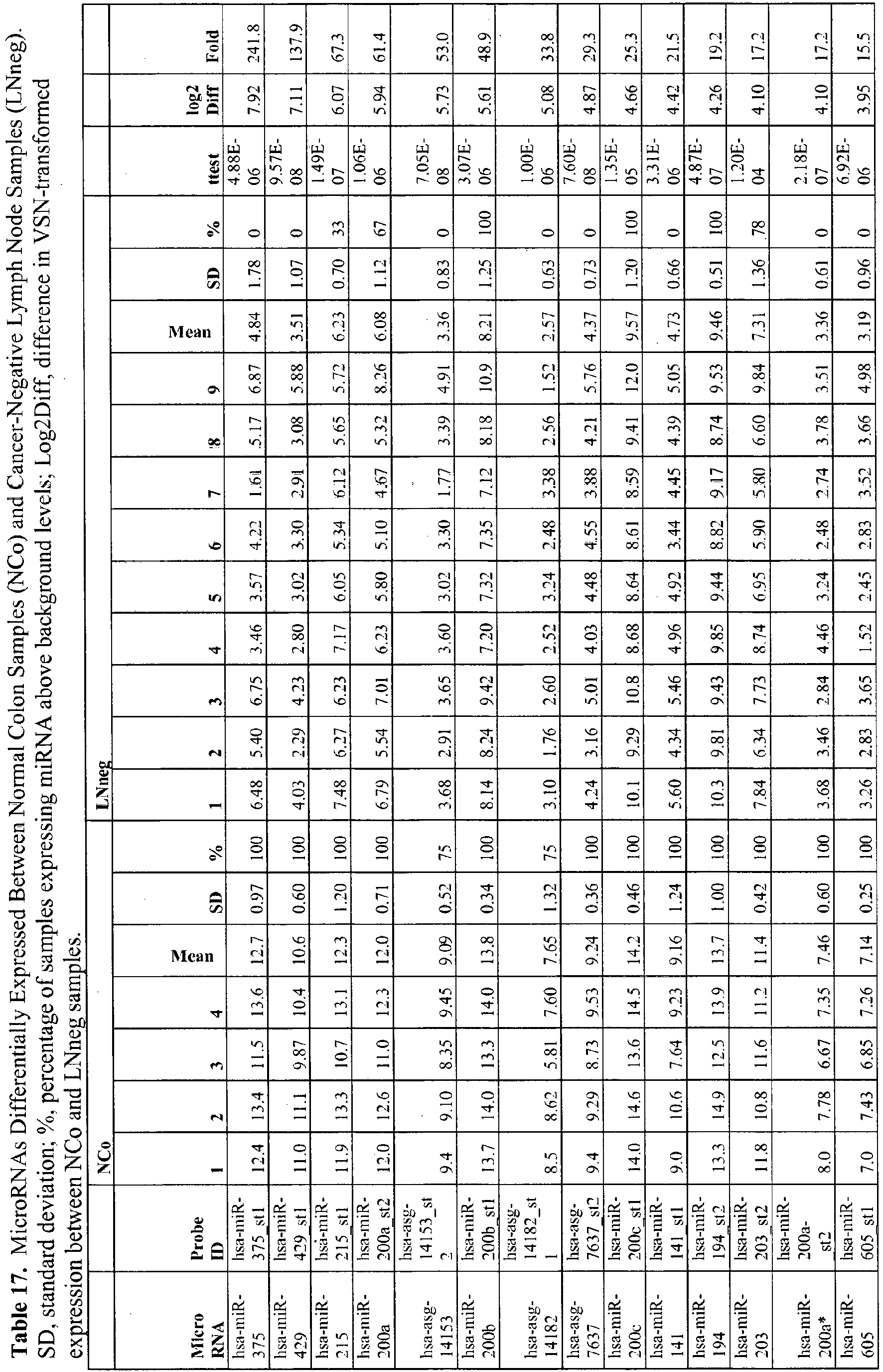

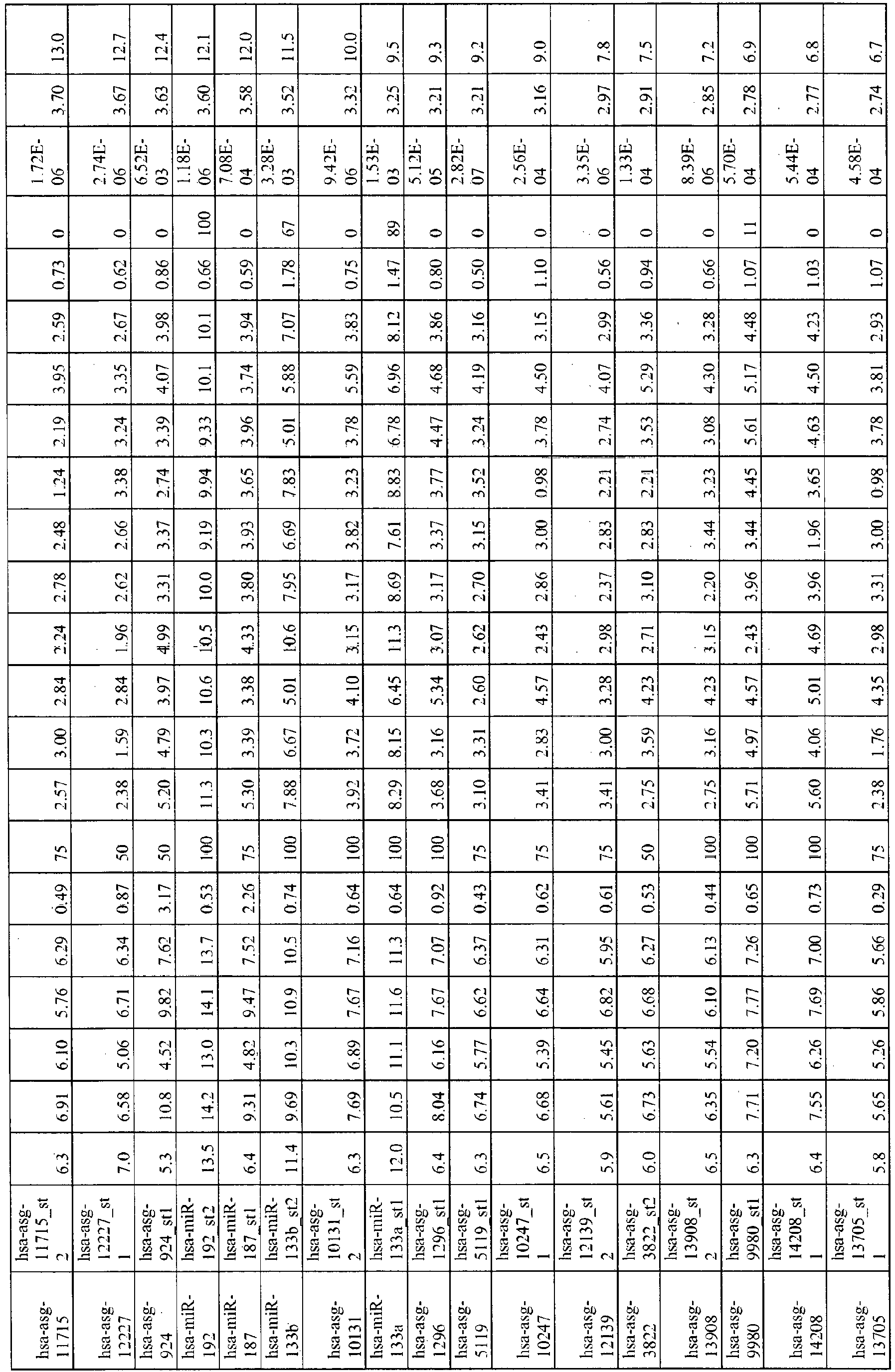

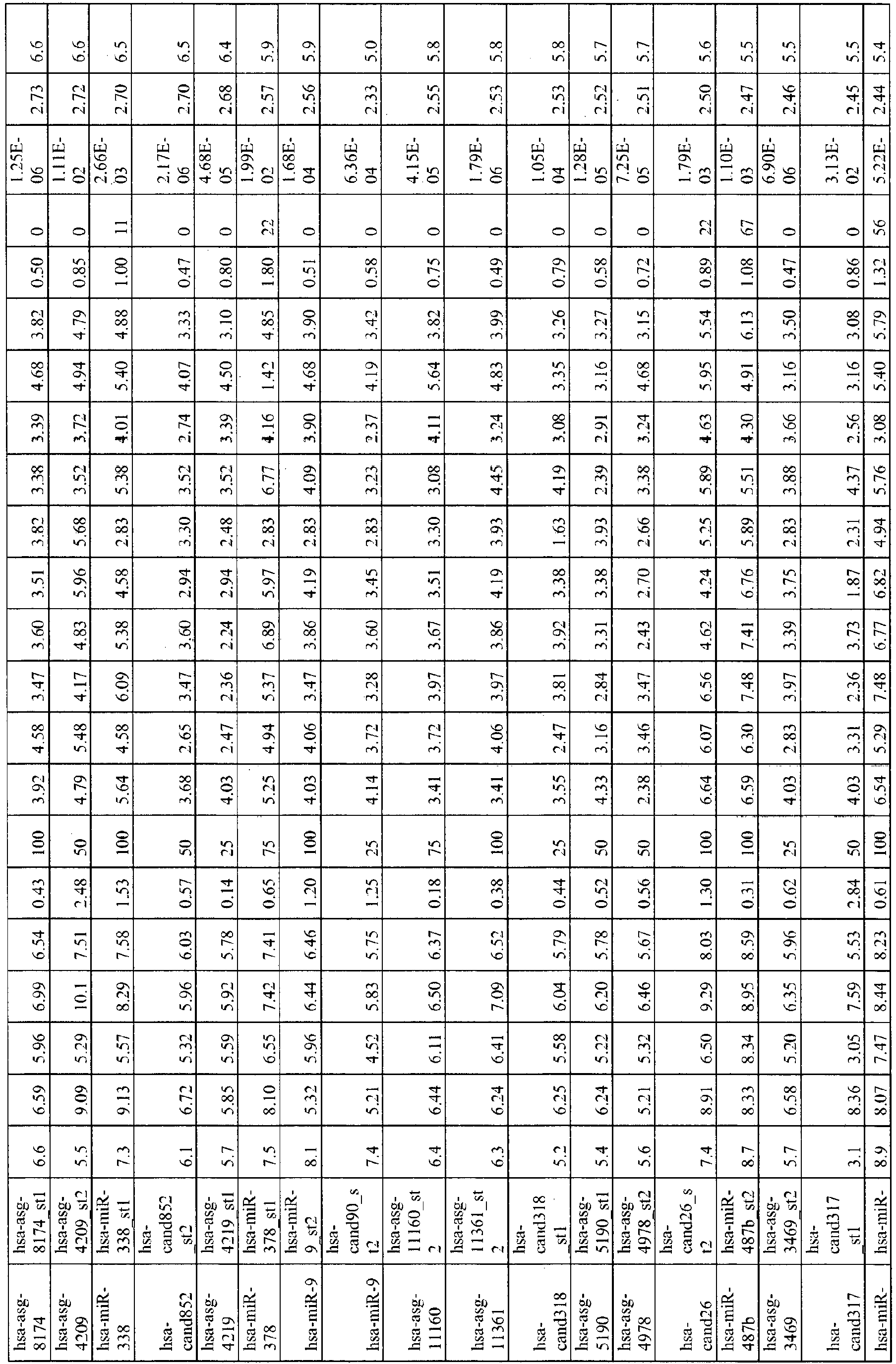

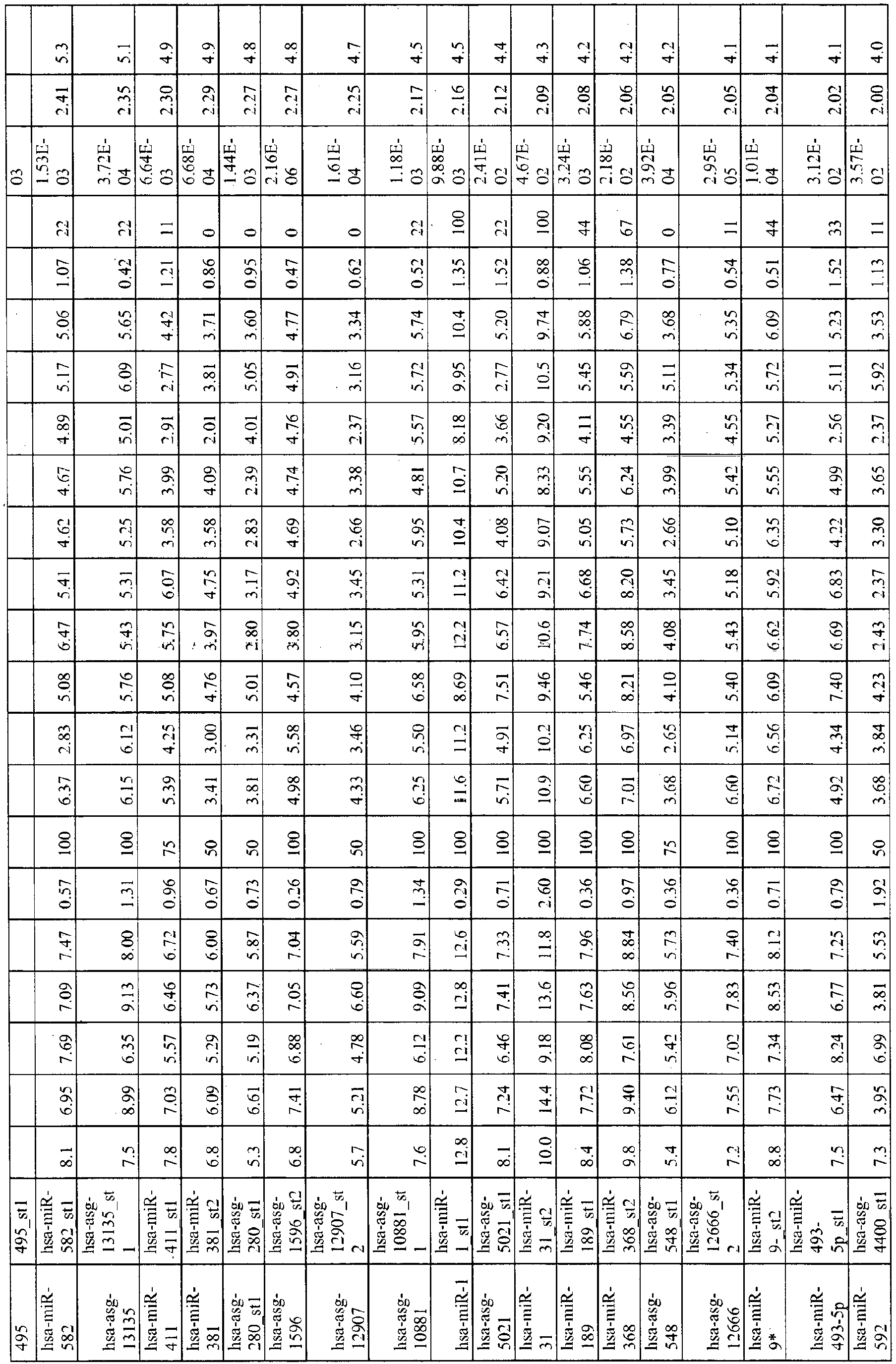

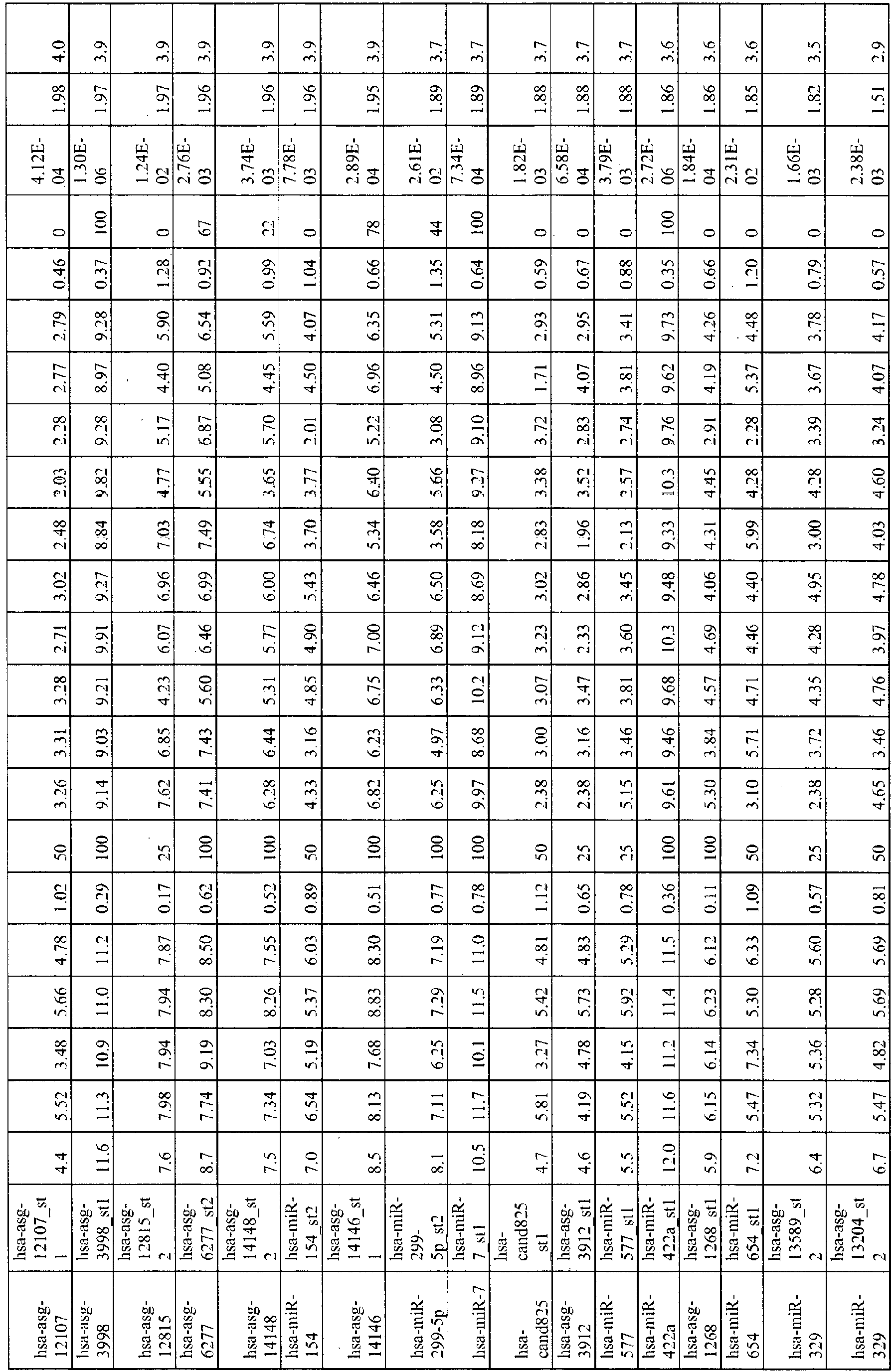

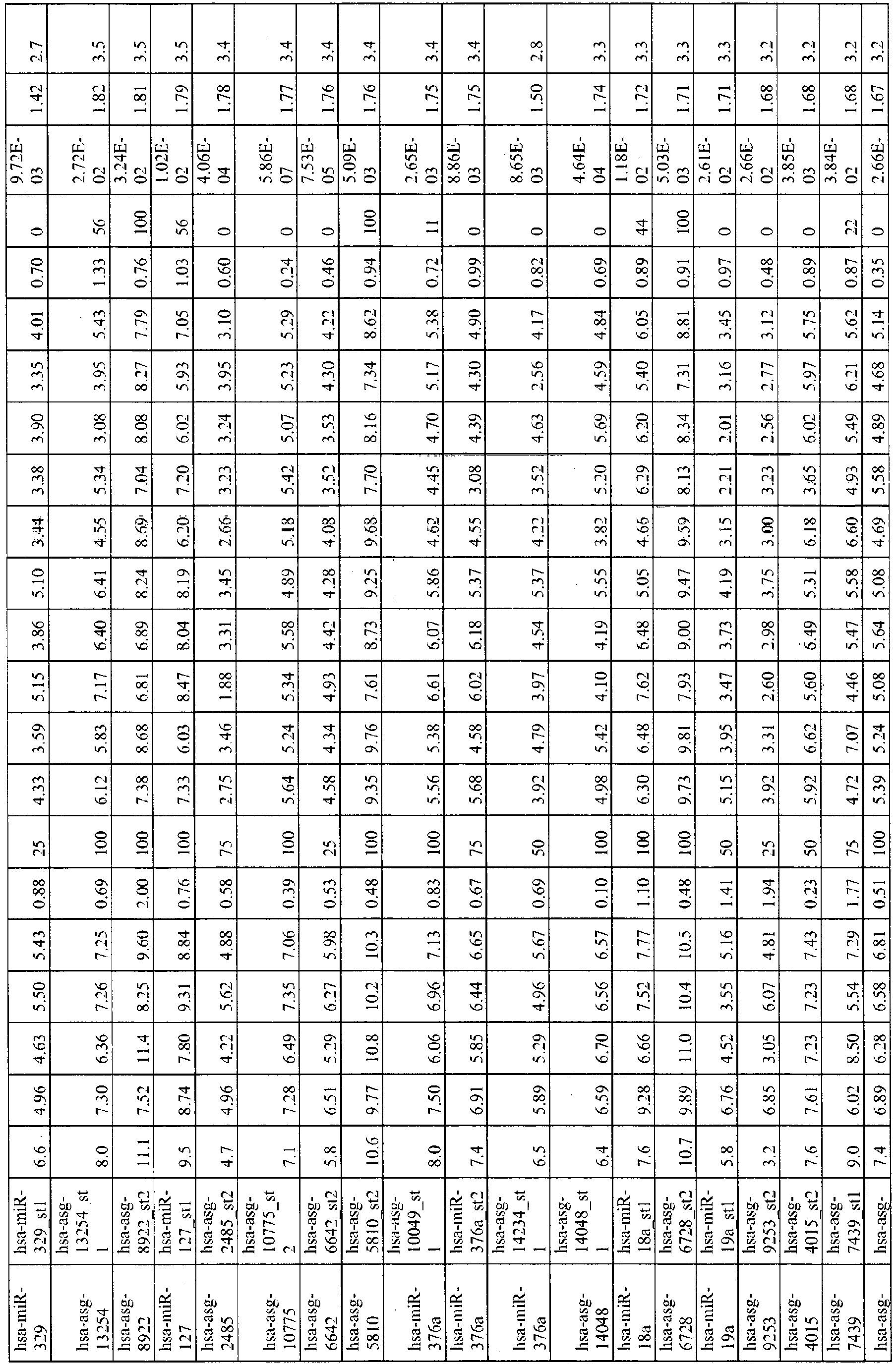

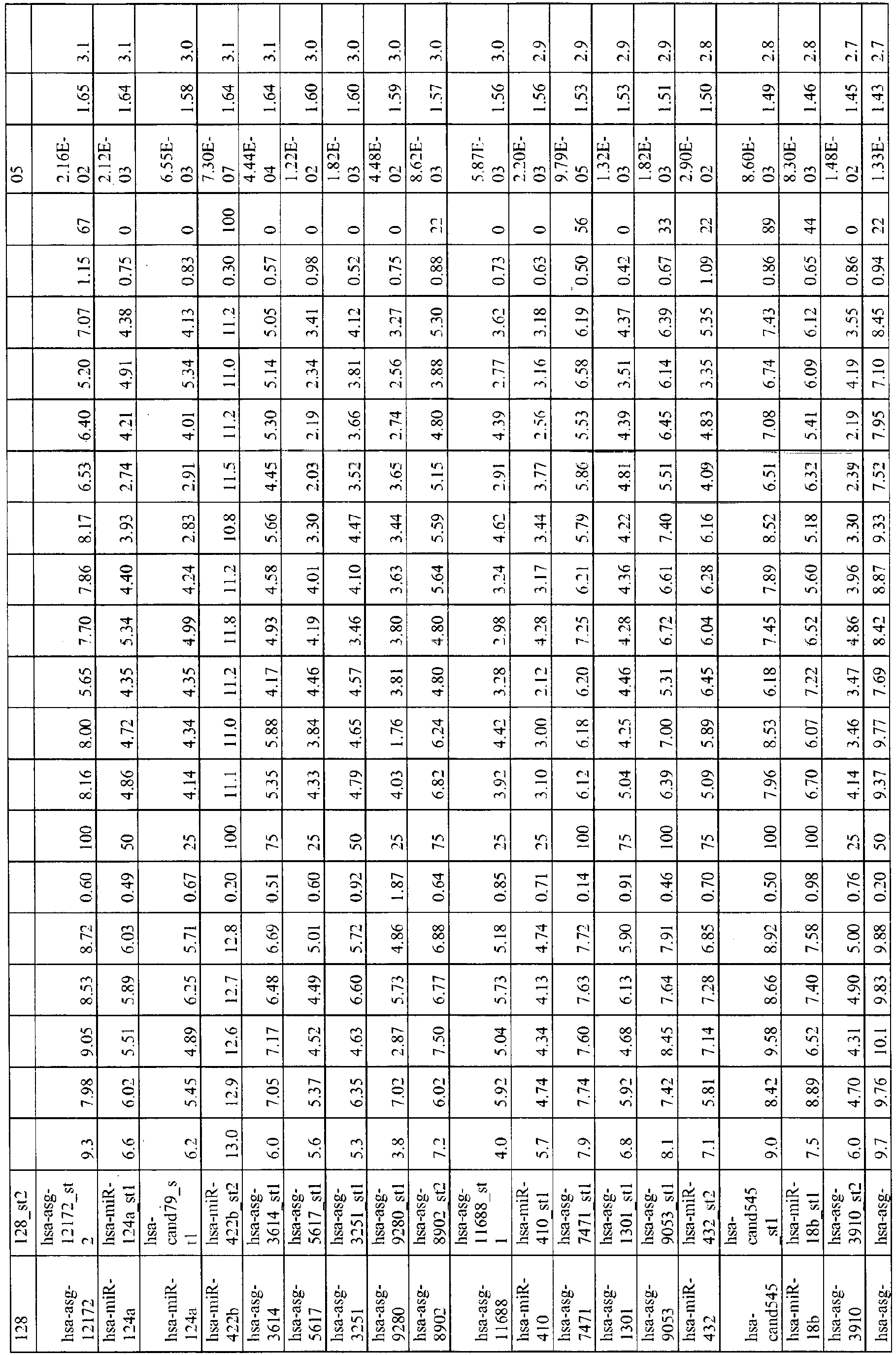

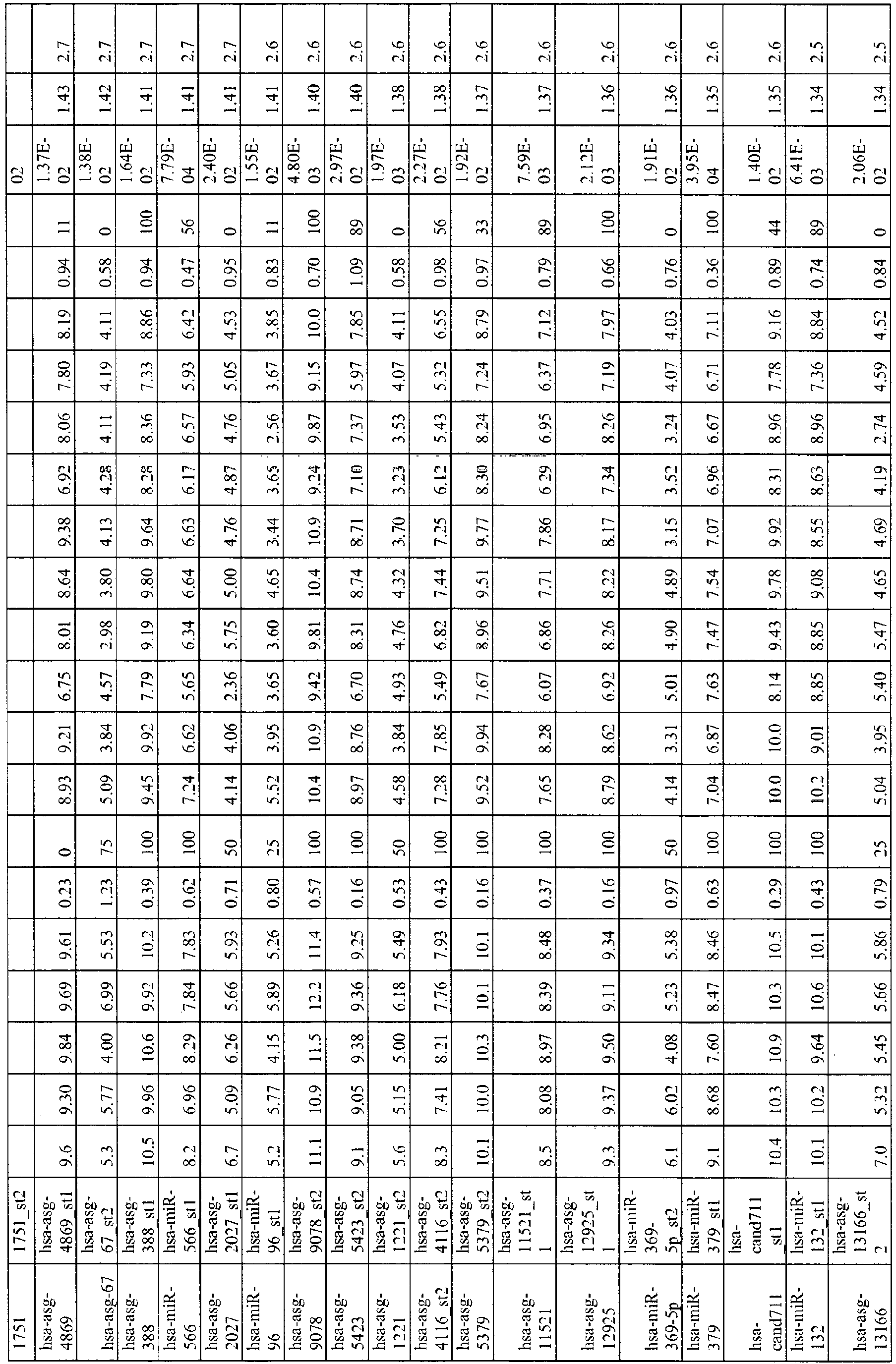

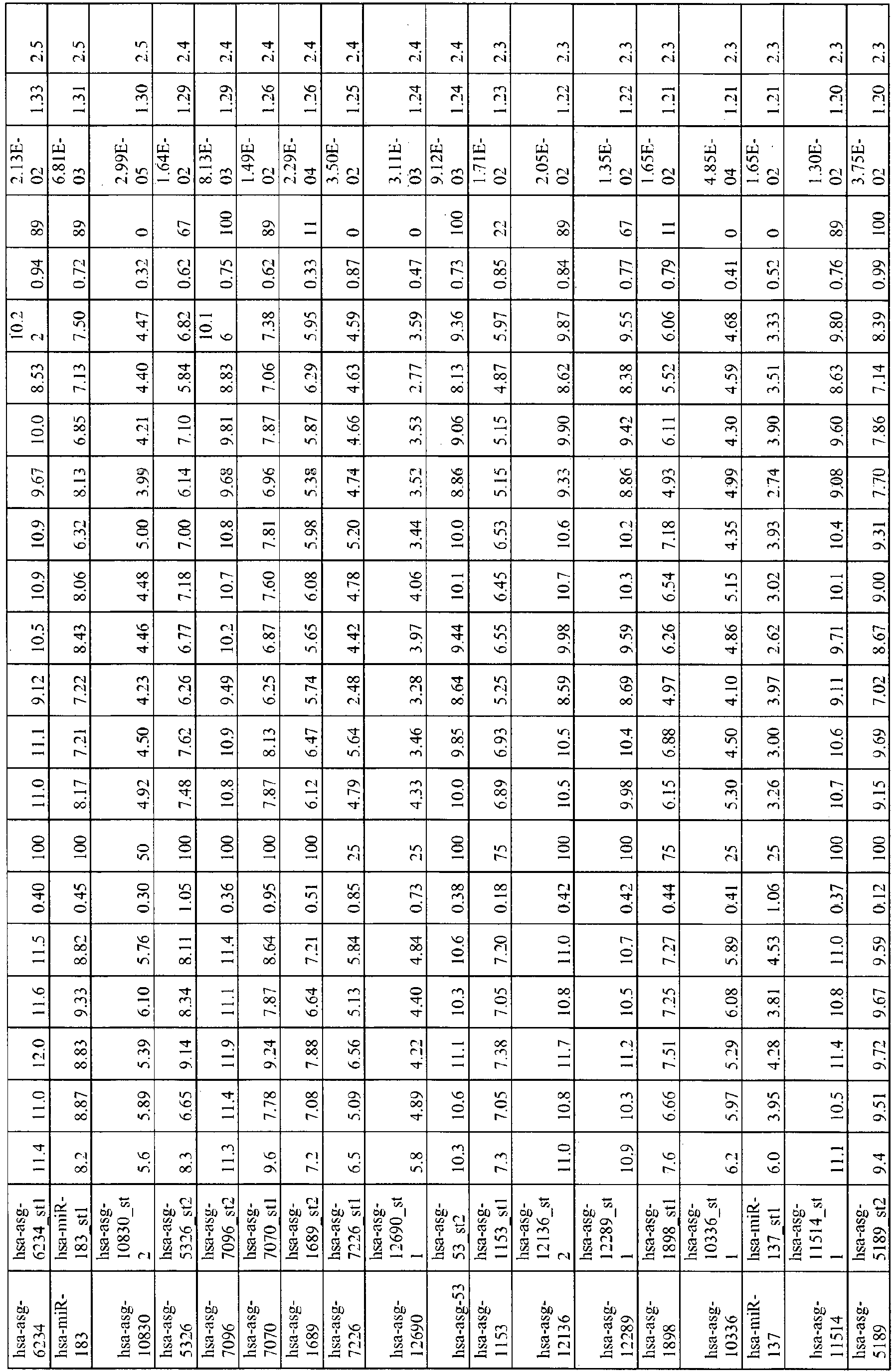

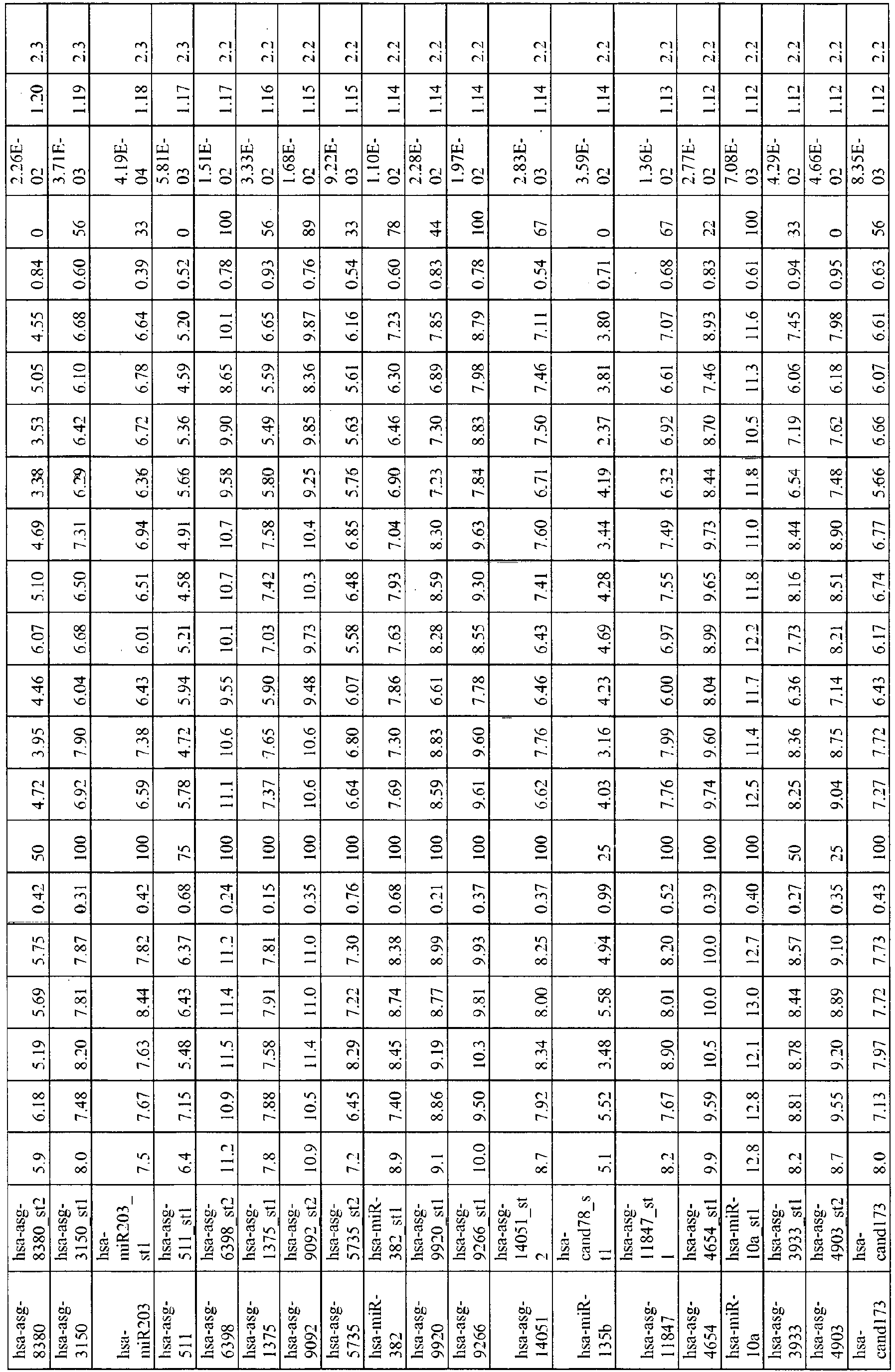

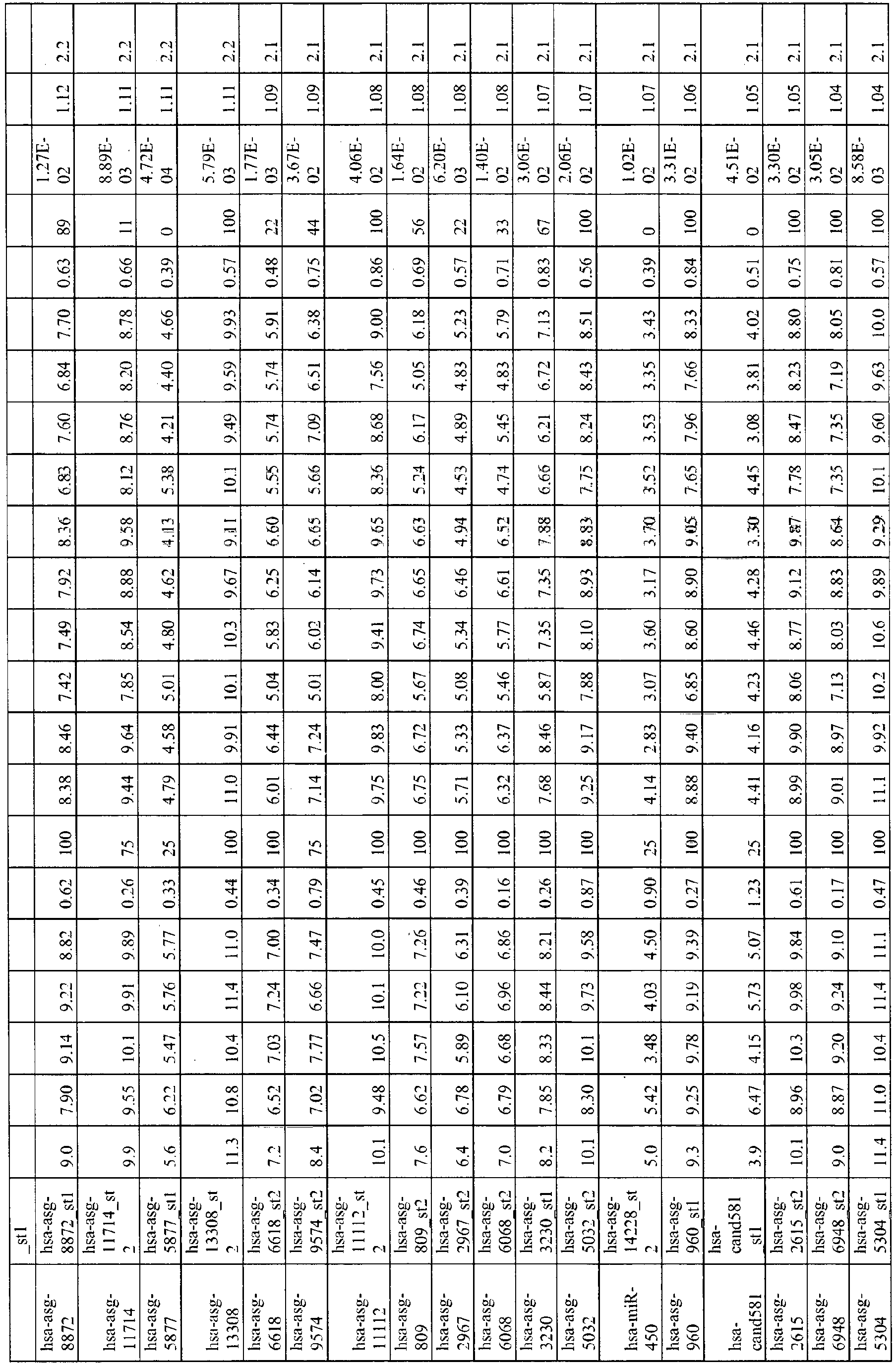

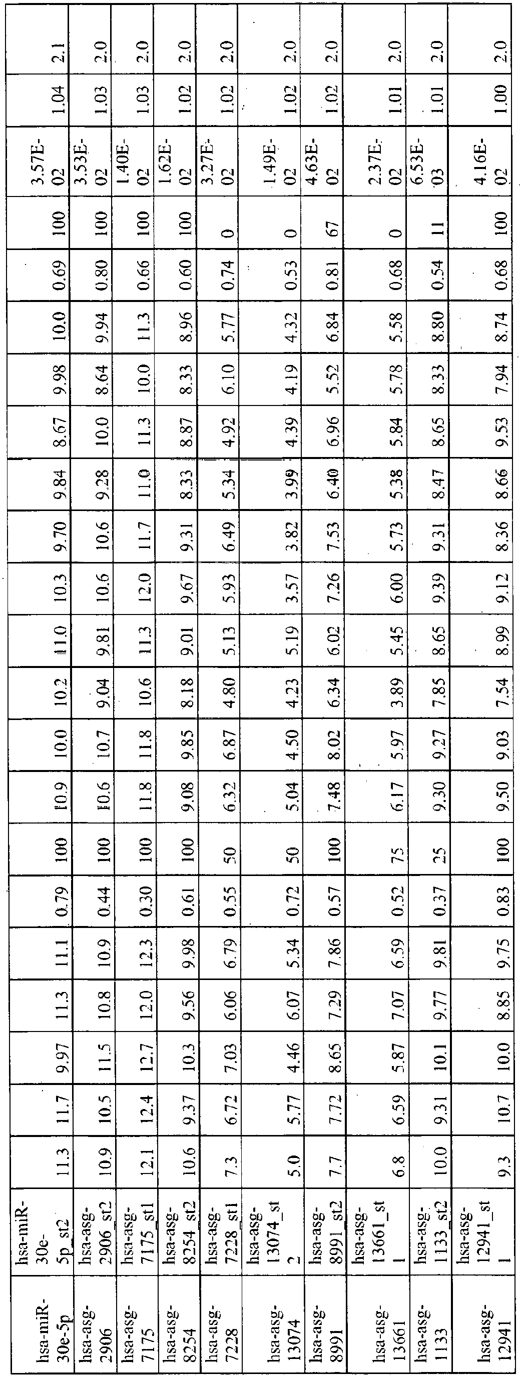

A comparison of results from the two different microarray platforms revealed that most of the miRNAs identified as differentially expressed with the *mir*Vana Bioarray platform were among the differentially expressed miRNAs identified on the miRChip microarray platform and include hsa-miR-200b, -200a, -200c, -141, -215, -375, -192, -194, - 429, -203, -187, 133a, and -9 (Table 16 and Table 17 above). Fold change values for these microRNAs differed between the two platforms, reflecting differences in the probes, samples, and data analysis tools. In contrast, seven miRNAs (hsa-miR-1, -137, -10a, -422a, -124a, and -422b and ambi-miR-7072-corresponding to hsa-asg-13254 on the miRChip), identified on the *mir*Vana Bioarray platform as significantly under-expressed in LNneg samples (Table 16), were not found to be significantly differentially expressed on the miRChip microarray platform. This is because the fold changes observed for these miRNAs on the miRChip platform were less than the 5-fold cut-off used for those data. The inventors identified three additional miRNAs (hsa-miR-605, -200a* and -133b) that were reported as significantly differentially expressed on the miRChip platform for which no probes were present on the *mir*Vana Bioarray platform.

### EXAMPLE 17

### QRT-PCR VERIFICATION OF DIFFERENTIALLY EXPRESSED MICRO-RNAS IN LYMPH NODES FROM COLON CANCER PATIENTS

The inventors evaluated the differential expression of selected miRNAs in seven cancer-positive lymph nodes from colon cancer patients (LNpos) and in seven control cancer-negative lymph nodes (LNneg, Example 15) by qRT-PCR. The LNpos samples were from patients with histologically-identified stage III colon cancer (T3N1M0 or T3N1M1-Table 15). qRT-PCR reactions were performed using TaqMan® MicroRNA Assays (Applied Biosystems; Foster City, CA, USA) specific for fifteen of the most differentially expressed miRNAs between normal colon (NCo) and cancer-negative lymph node (LNneg) samples (hsa-miR-1, -9, -141, -187, -192, -194, -200a, -200b, -200c, -203, -215, -375, -429, -566, and -605). Assays were performed according to the manufacturer's instructions with 15 ng of input total RNA from one of the fourteen samples and were incubated in a 7900HT Fast Real-Time PCR system (Applied Biosystems, Foster City, CA, USA). Initial data analysis was done using the 7500 Fast System SDS V2.3 software (Applied Biosystems). miR-16, whose expression level did not change between the different samples, was used for normalization. Equivalent *t*-tests were performed on the Cₜ values for individual qRT-PCR reaction without correction for multiple testing.

Significant differential expression between cancer-positive and cancer-negative lymph nodes was demonstrated by qRT-PCR quantification for ten of the fifteen miRNAs (miR-429, -200b, -141, -200a, -200c, -375, -203, -194, -192, -and -215) with fold changes varying between 760 fold (miR-429) and 77 fold (miR-215) (FIG. 10).

These data demonstrate that qRT-PCR quantification of selected miRNAs is an effective method for quantifying miRNA expression when evaluating metastasis of colon cancer to lymph nodes.

### EXAMPLE 18:

### IDENTIFICATION OF CLASSIFIERS FOR CANCER-POSITIVE LYMPH NODES IN PATIENTSWITH METASTATIC COLON CANCER

The inventors have demonstrated that miRNA quantification and expression analysis can differentiate cancer-positive lymph nodes from patients with colon cancer and control cancer-negative lymph nodes. To further demonstrate the utility of specific miRNA markers as diagnostic analytes, the inventors quantified specific combinations of miRNAs by qRT-PCR to determine if any such combinations enhanced the distinction between cancer-positive and cancer-negative lymph nodes. qRT-PCR assays were performed as described in Example 17 above. For miRNA combinations quantified by qRT-PCR, normalized Ct values (miRNACt - miR-16Ct) from the quantification of individual miRNAs were added,. The inventors determined that quantification of several miRNA combinations (miR-200b+miR-429; miR-200b+miR-203; miR-203+miR-429; miR-200b+miR-203+miR-429; miR-200a+miR-203+miR-429) enhances the distinction between cancer-positive and cancer-negative lymph nodes (FIG. 11).

These combinations serve as non-limiting examples. Combinations of qRT-PCR or microarray data for other microRNAs will serve to enhance the distinction between cancer-positive and cancer-negative lymph nodes. Interestingly, some miRNA combinations, such as miR-203+miR-429, can also be used as a classifier for the identification of metastatic lymph nodes in cervical cancer patients, in breast cancer patients, or in a subset of melanoma patients, underscoring the potential broad utility of these markers in cancer staging.

### EXAMPLE 19:

### MIRNAS DIFFERENTALLY EXPRESSED BETWEEN NORMAL BREAST TISSUE AND NORMAL LYMPH NODES

To identify miRNAs that may be useful markers for metastasis of cancerous breast cells to lymph nodes, the inventors identified miRNAs that are expressed at significantly higher levels in normal breast tissue than in cancer-negative lymph nodes.

Total RNA, purified from three separate normal human breast tissue samples (FirstChoice^{®} Human Breast Total RNA, cat. no. AM6952; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 872 miRNAs (87 verified, 785 predicted) with significant differential expression between normal breast tissues (NBR) and normal lymph nodes (LNneg) (log2Diff breast vs LNneg ≥1 and p-value<0.05) (Table 18). The average expression level of hsa-miR-205 was 196-fold higher in breast tissues compared with its average expression level in the normal lymph node samples. Among the verified miRNAs, thirteen miRNAs (hsa-miR-429, -200b, -411, -200c, -517a, -200a, -432, -368, -551b, -539, - 452*, -376a, and -487b) had average expression levels that were 10- to 30-fold higher in breast tissue samples, and 26 miRNAs had average expression levels that were 5- to 10-fold higher in breast tissue samples. Among the predicted miRNAs, seven (hsa-asg-4209_st2, - 3983_ st2, -14160_st1, -13922_st1, -4803_st2, -5021_st1, and miR154_st1) had average expression levels that were between 10- and 20-fold higher in breast tissues, and 76 miRNAs had average expression levels that were between 5- and 10- fold higher in breast tissues.

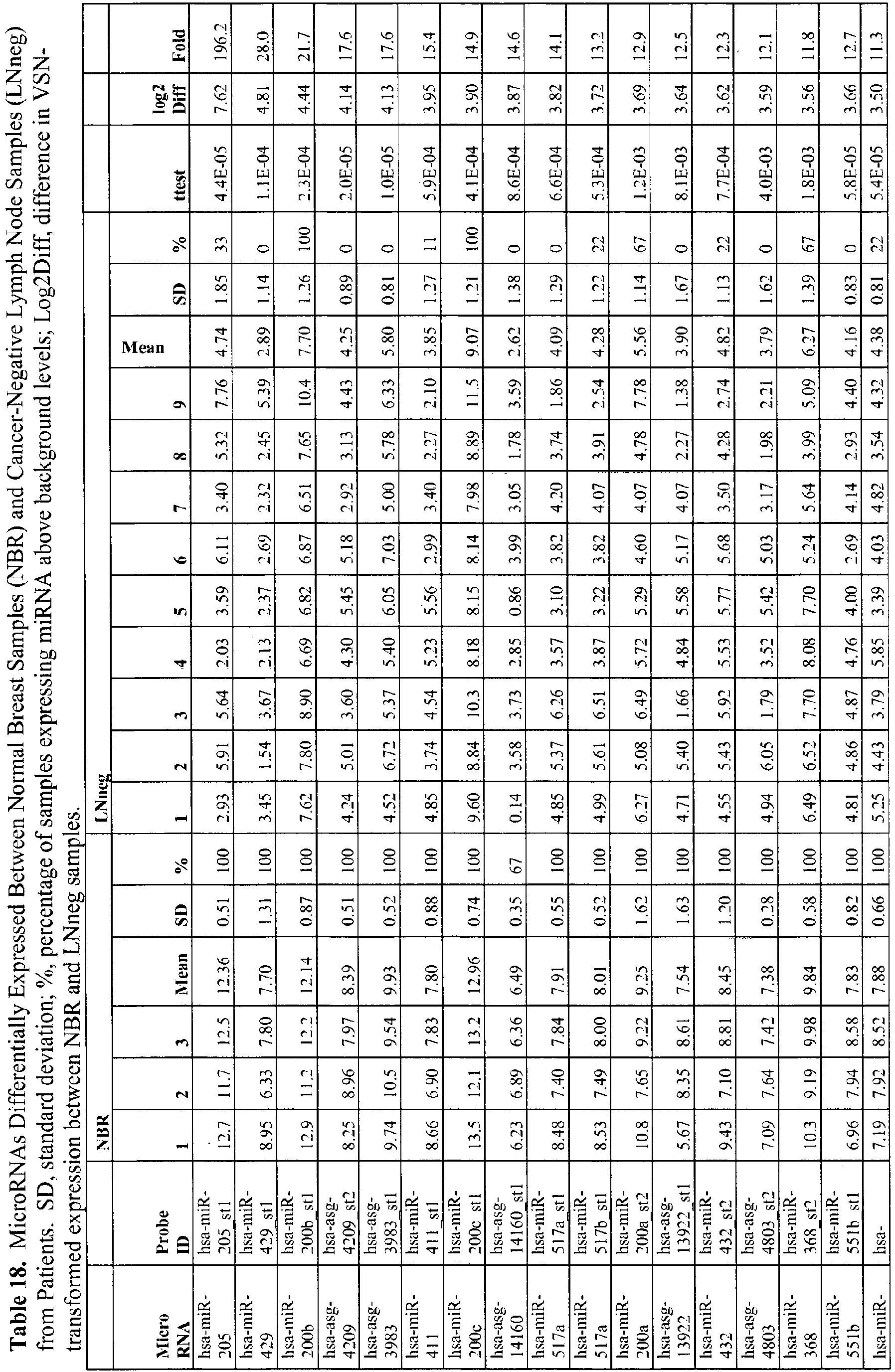

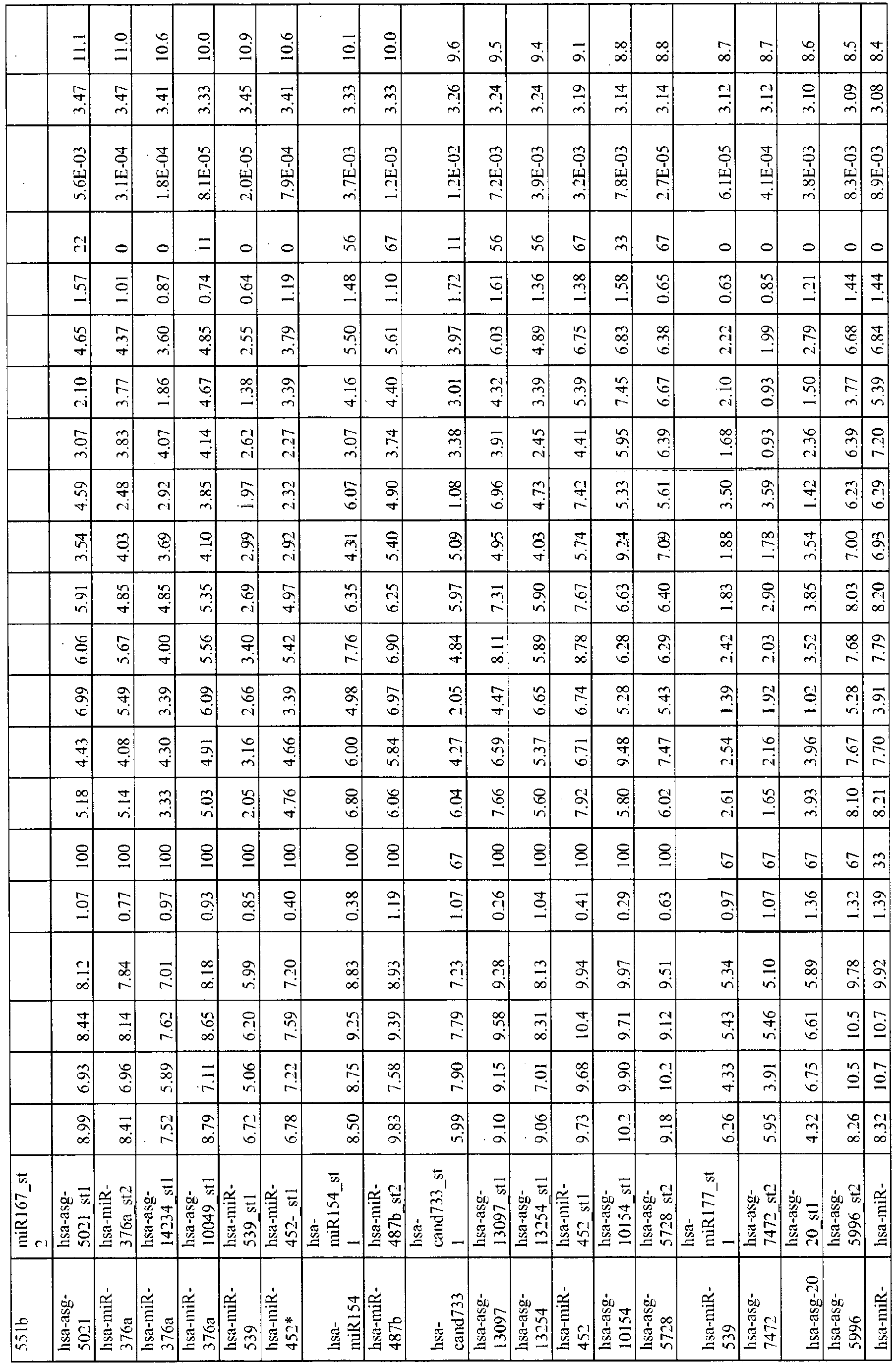

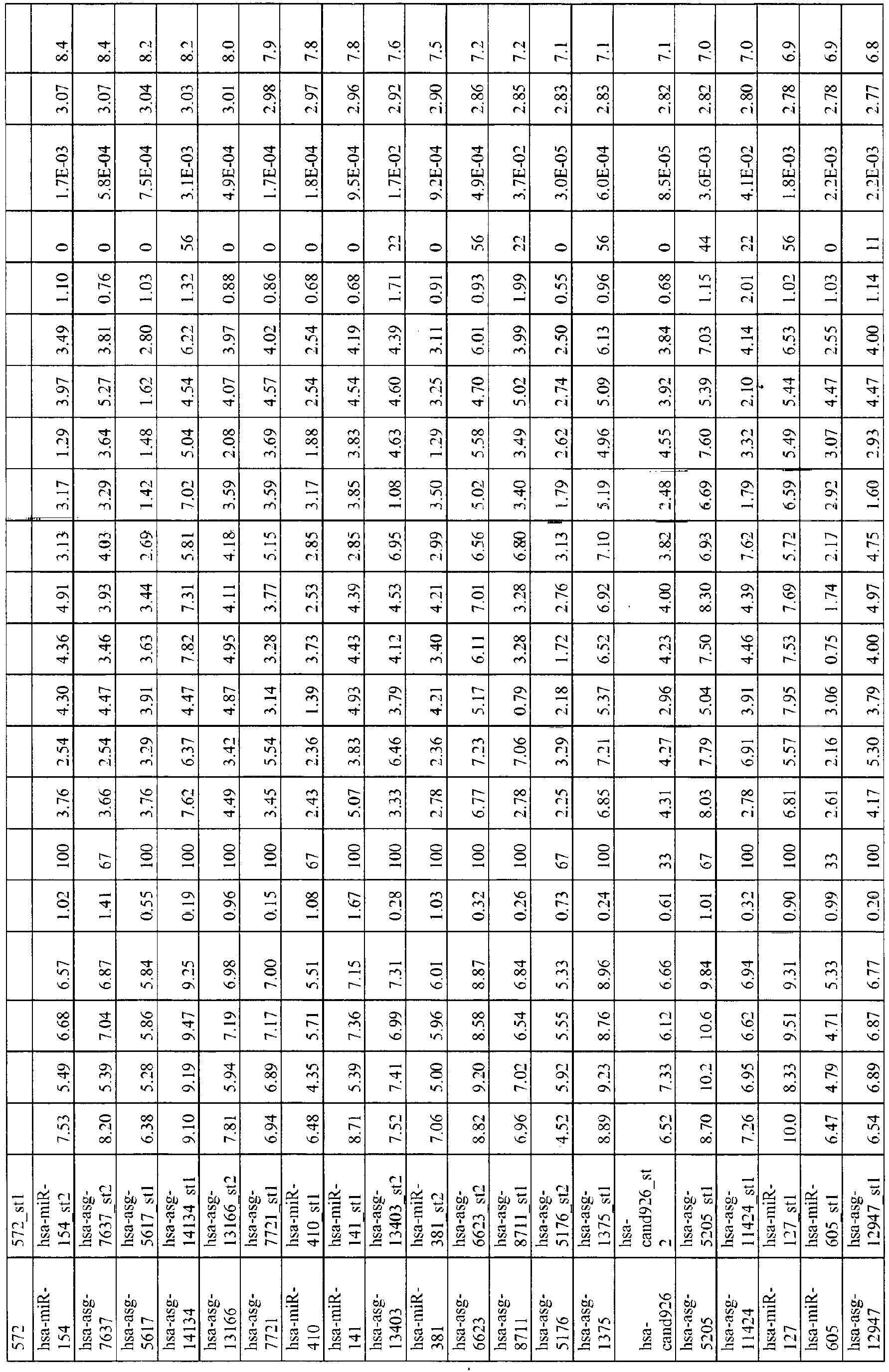

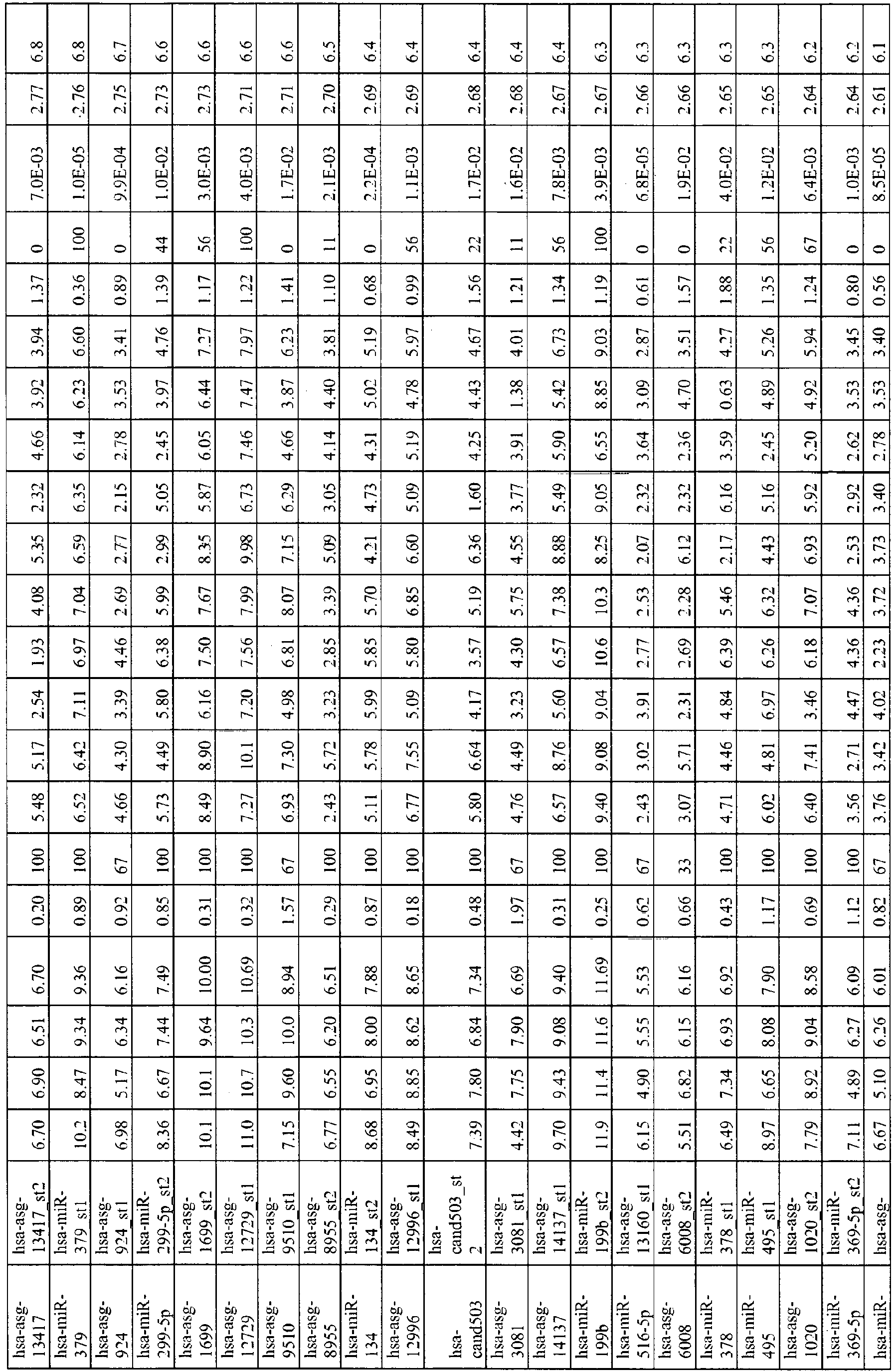

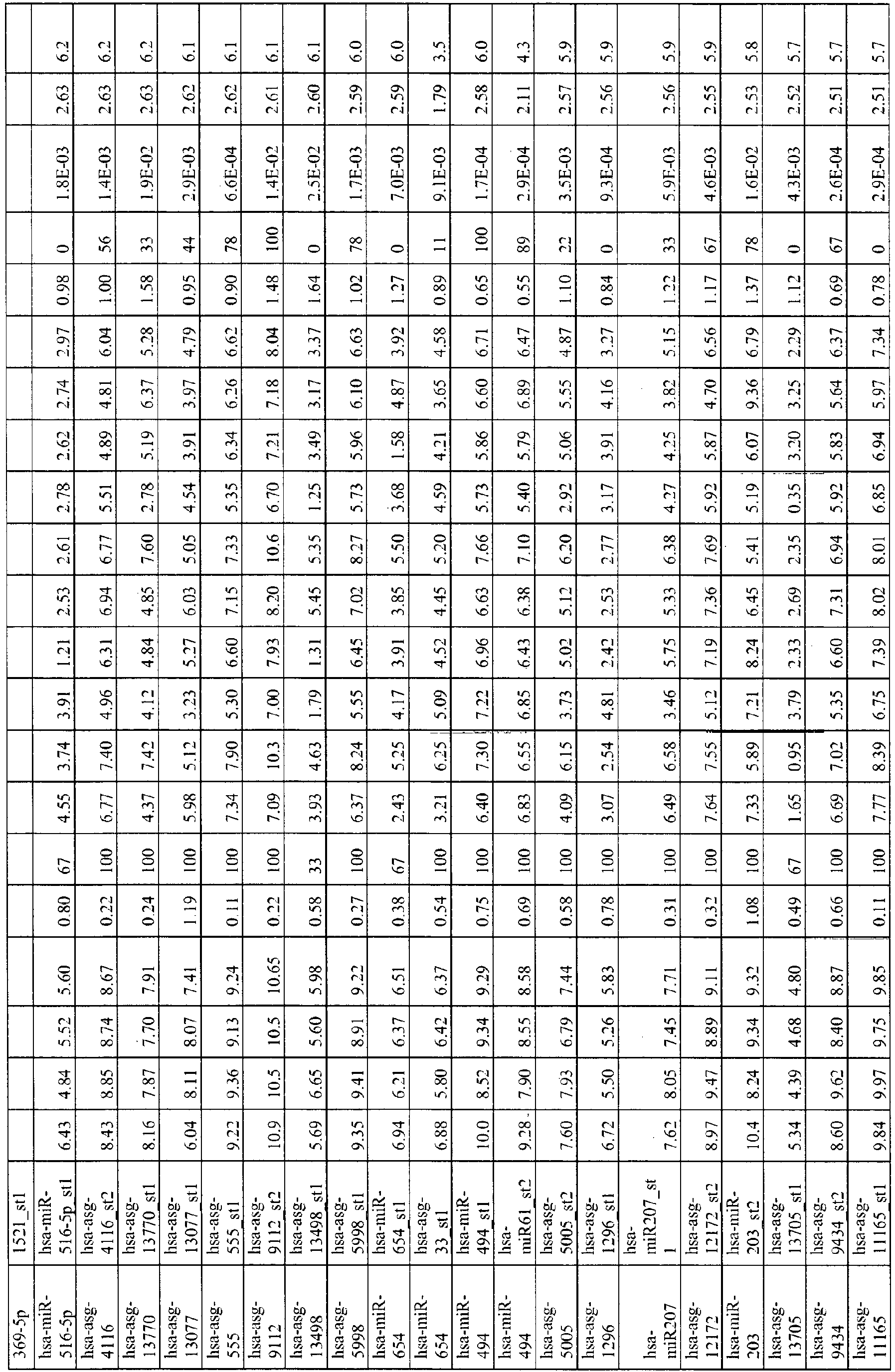

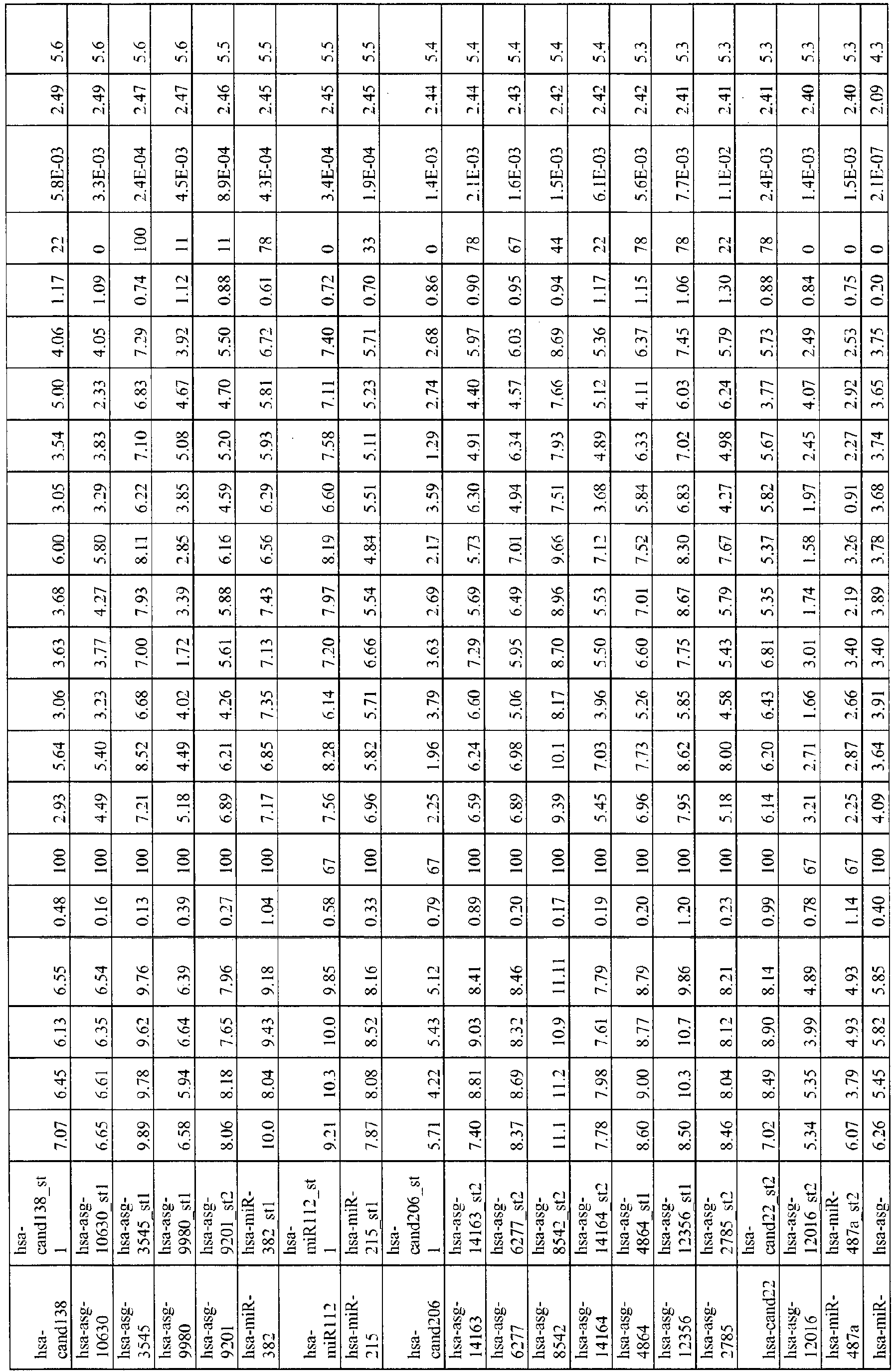

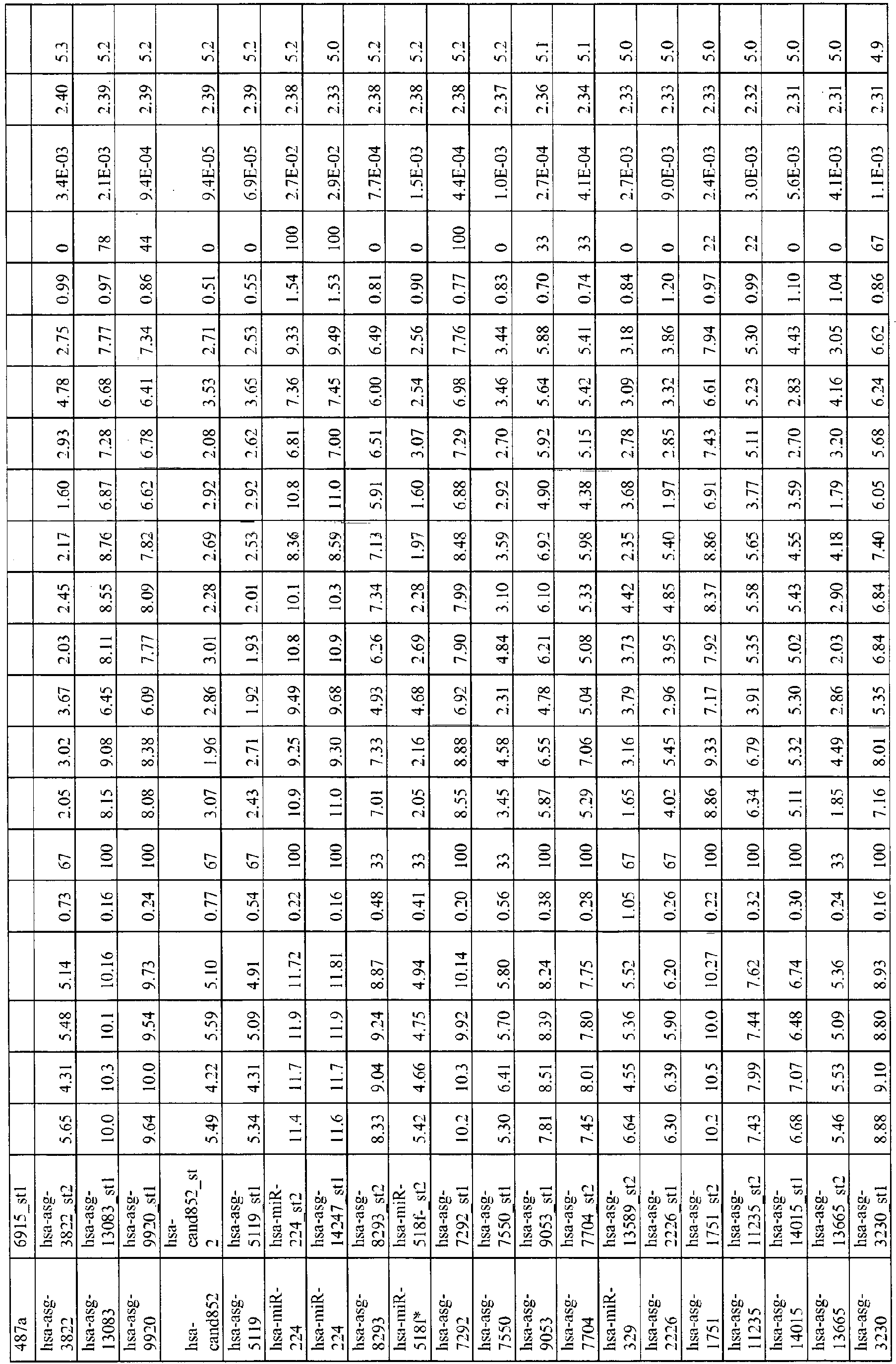

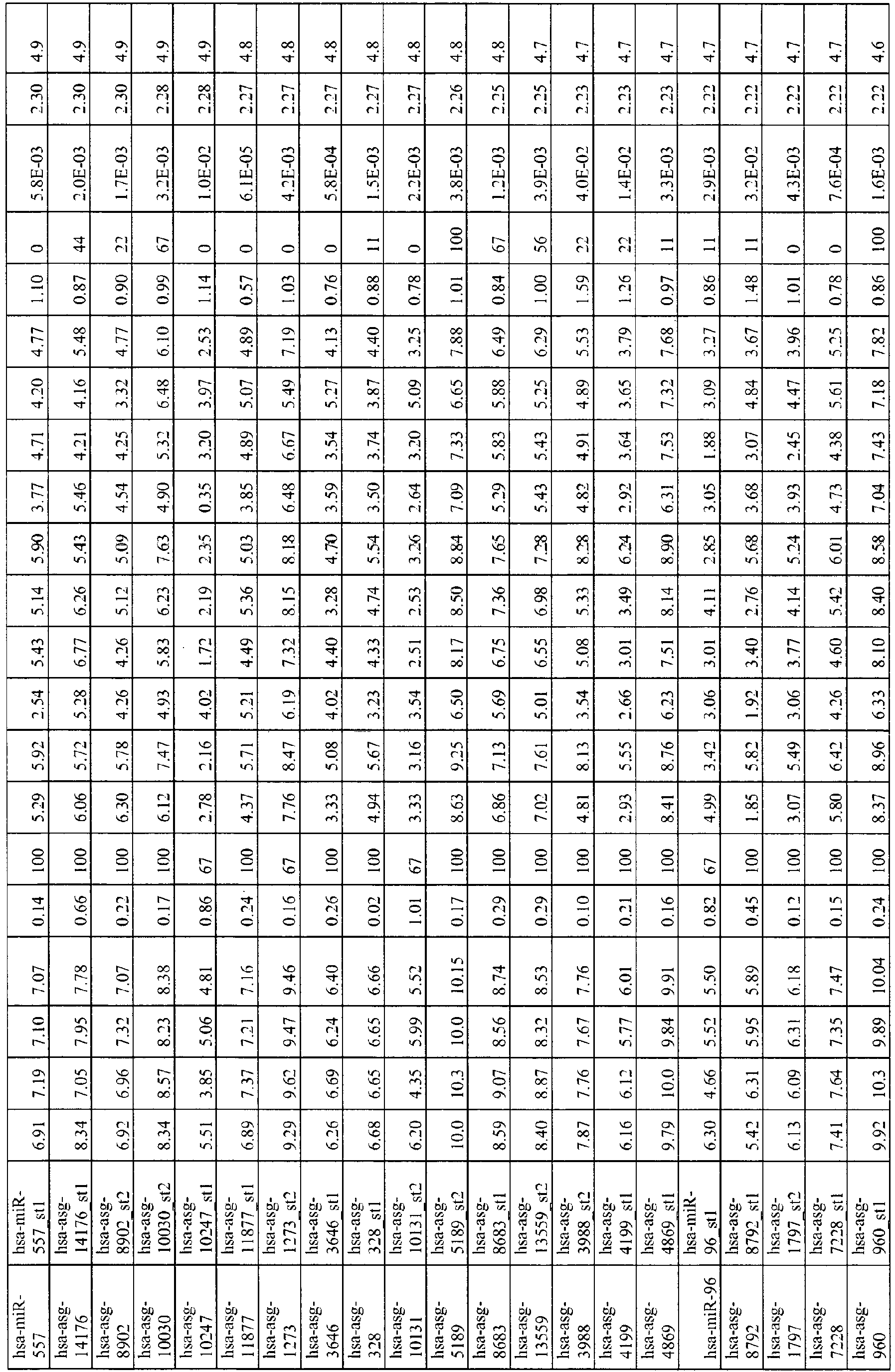

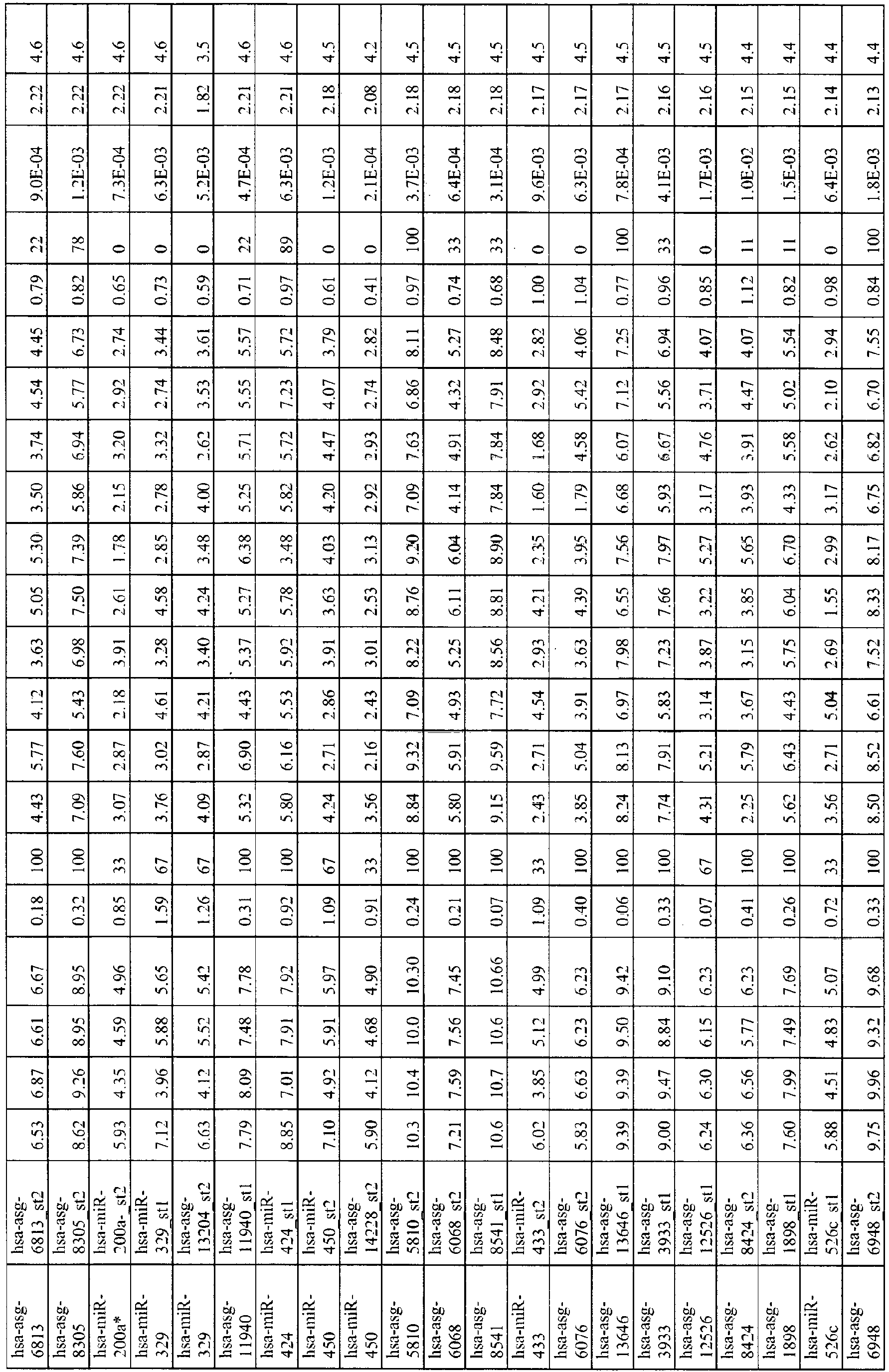

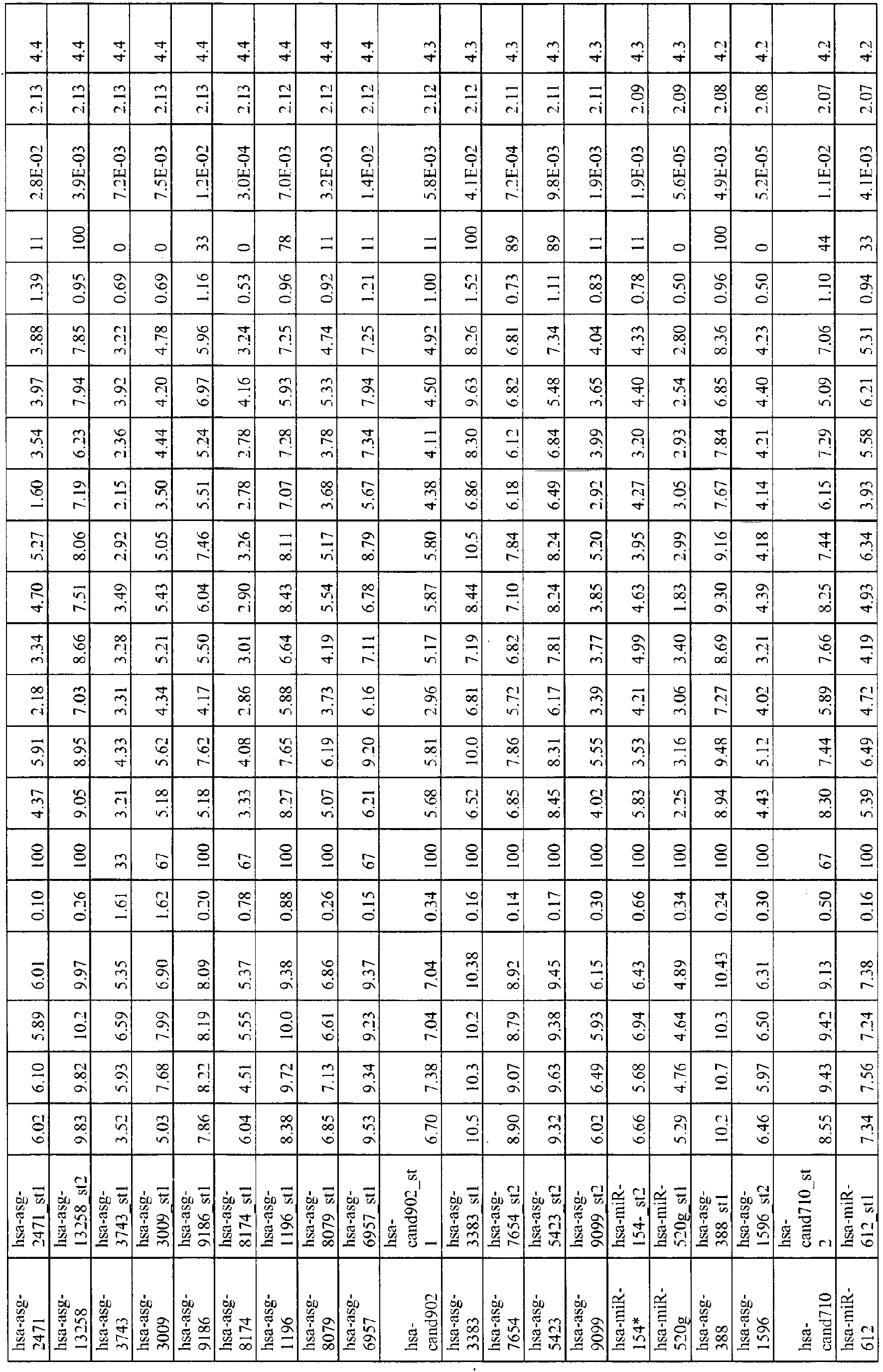

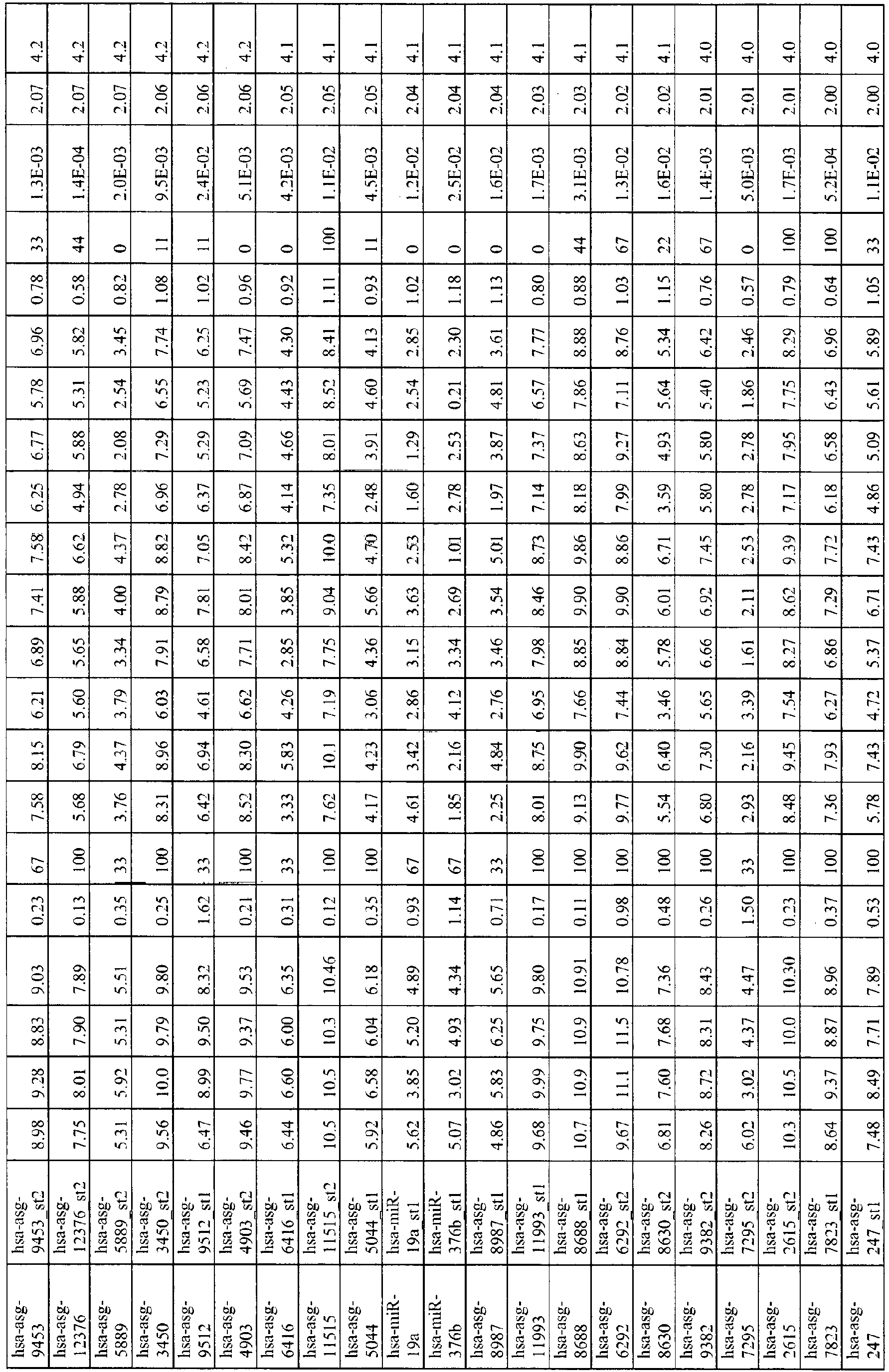

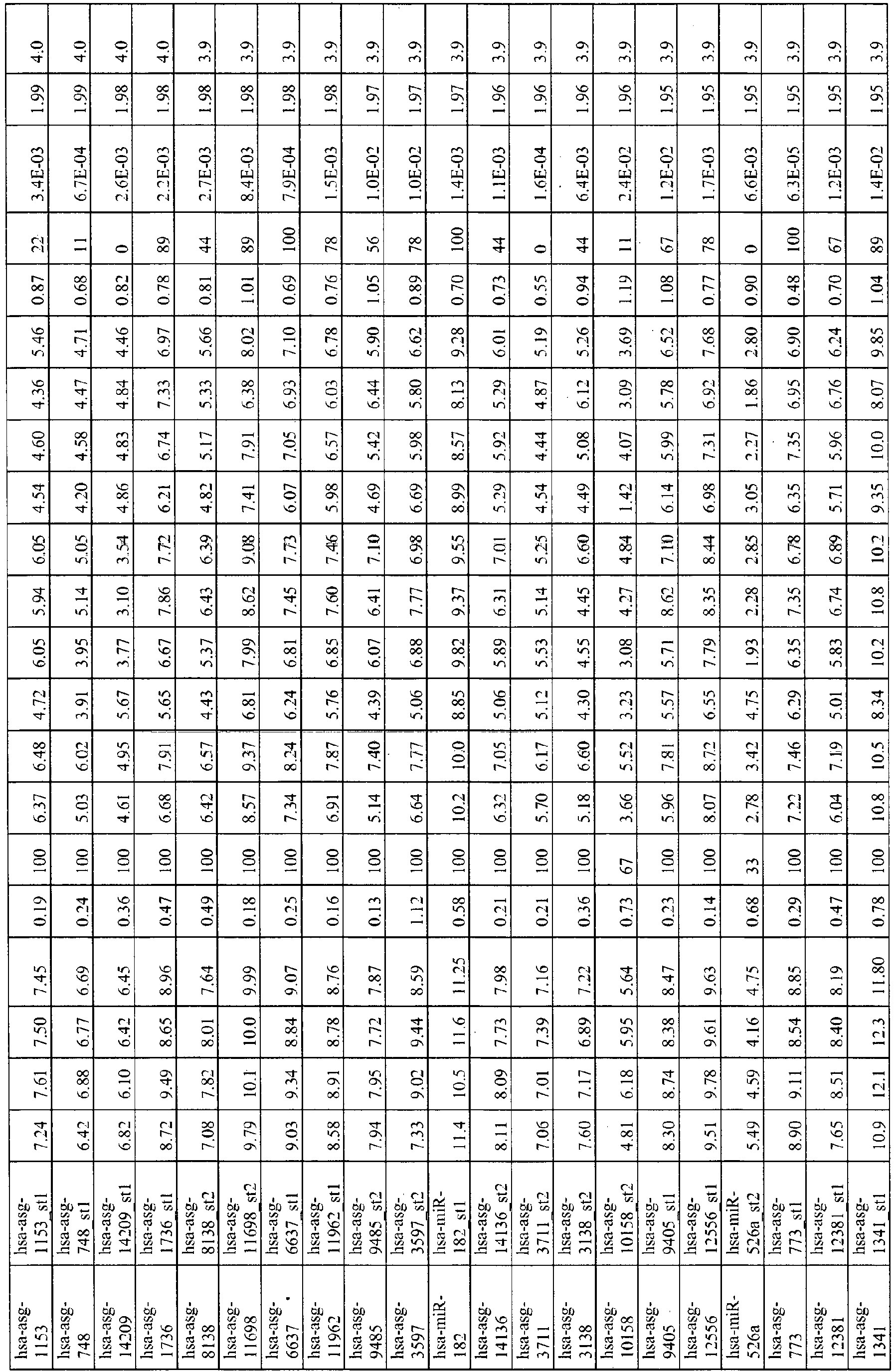

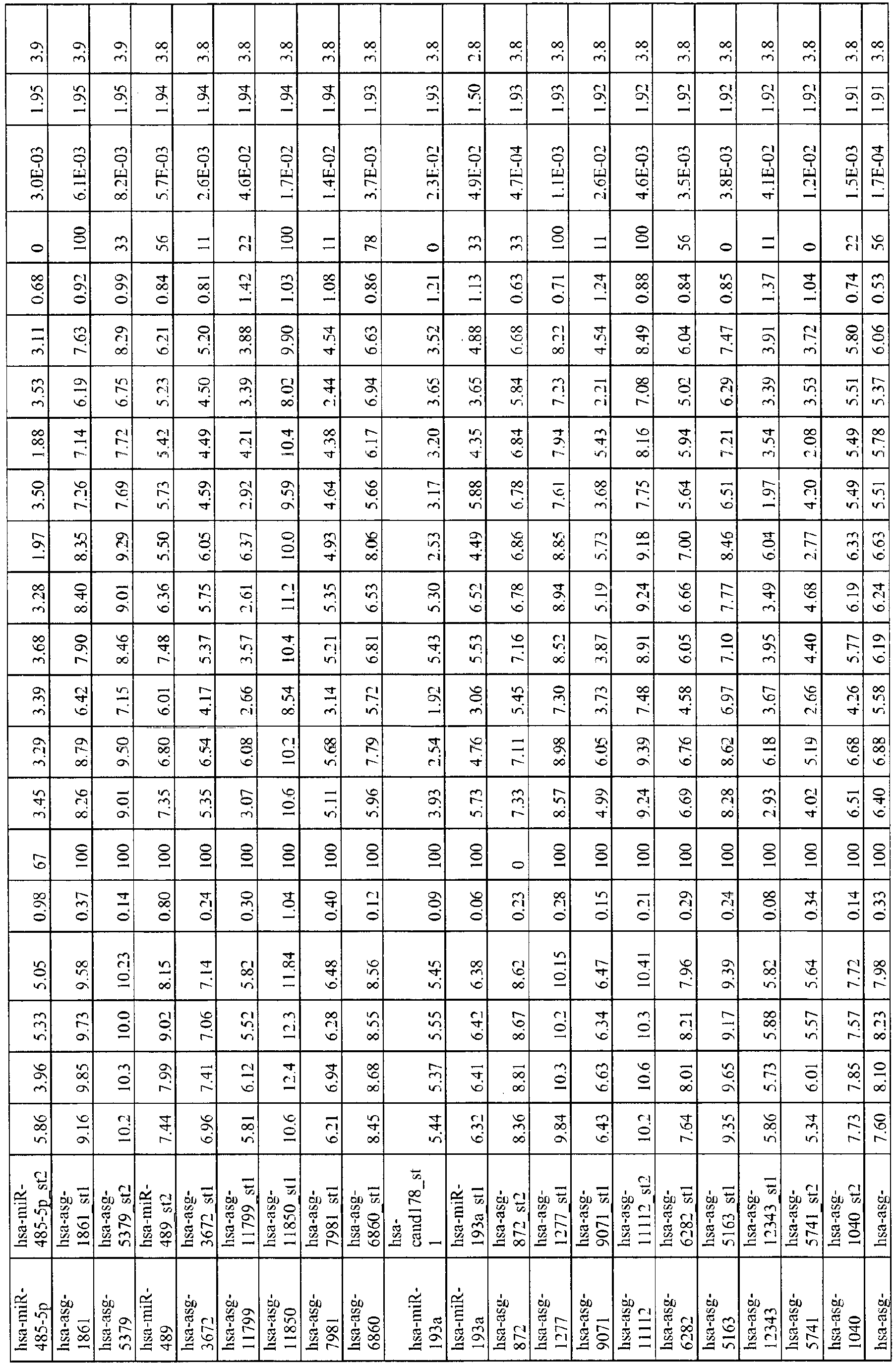

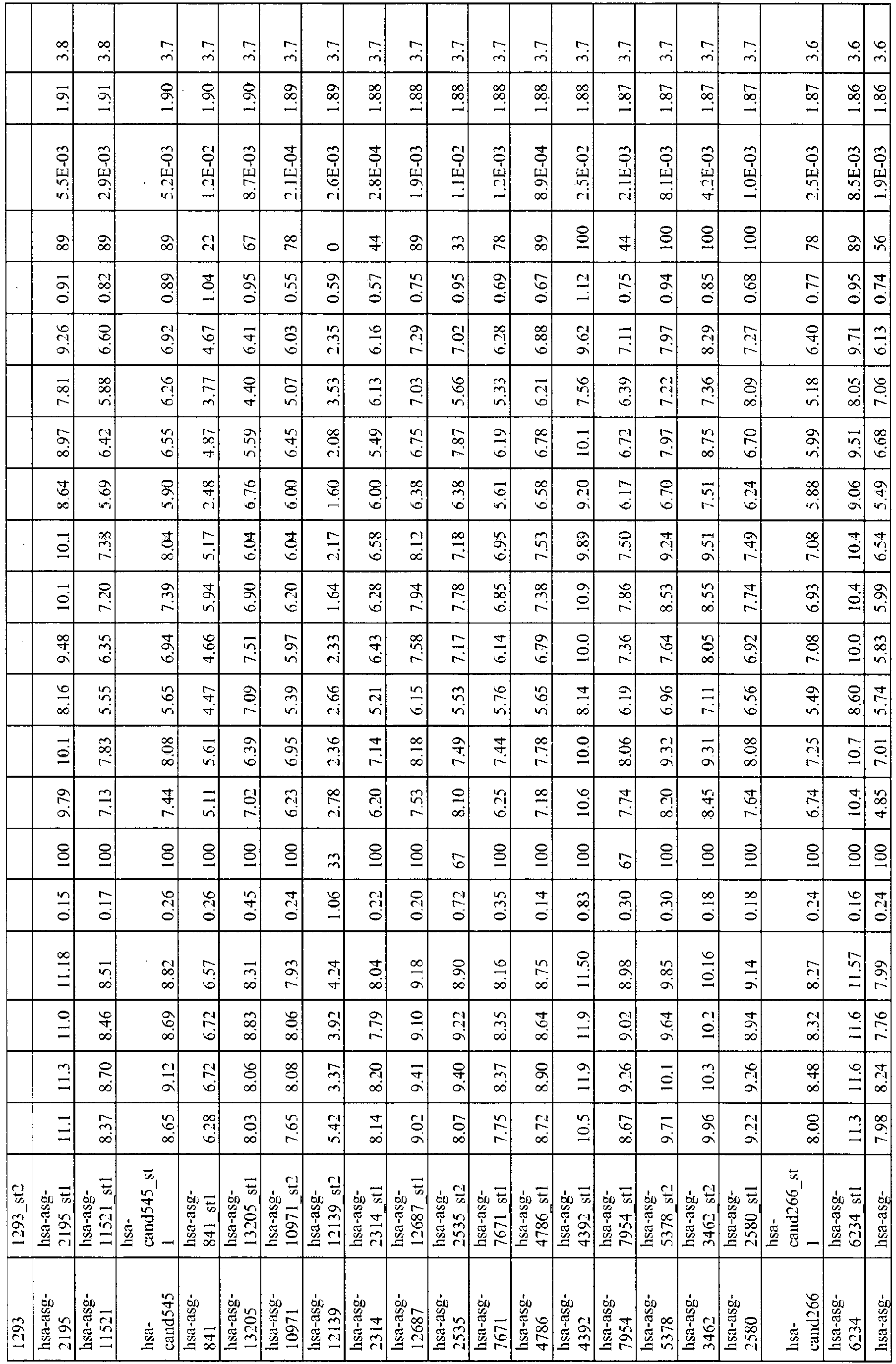

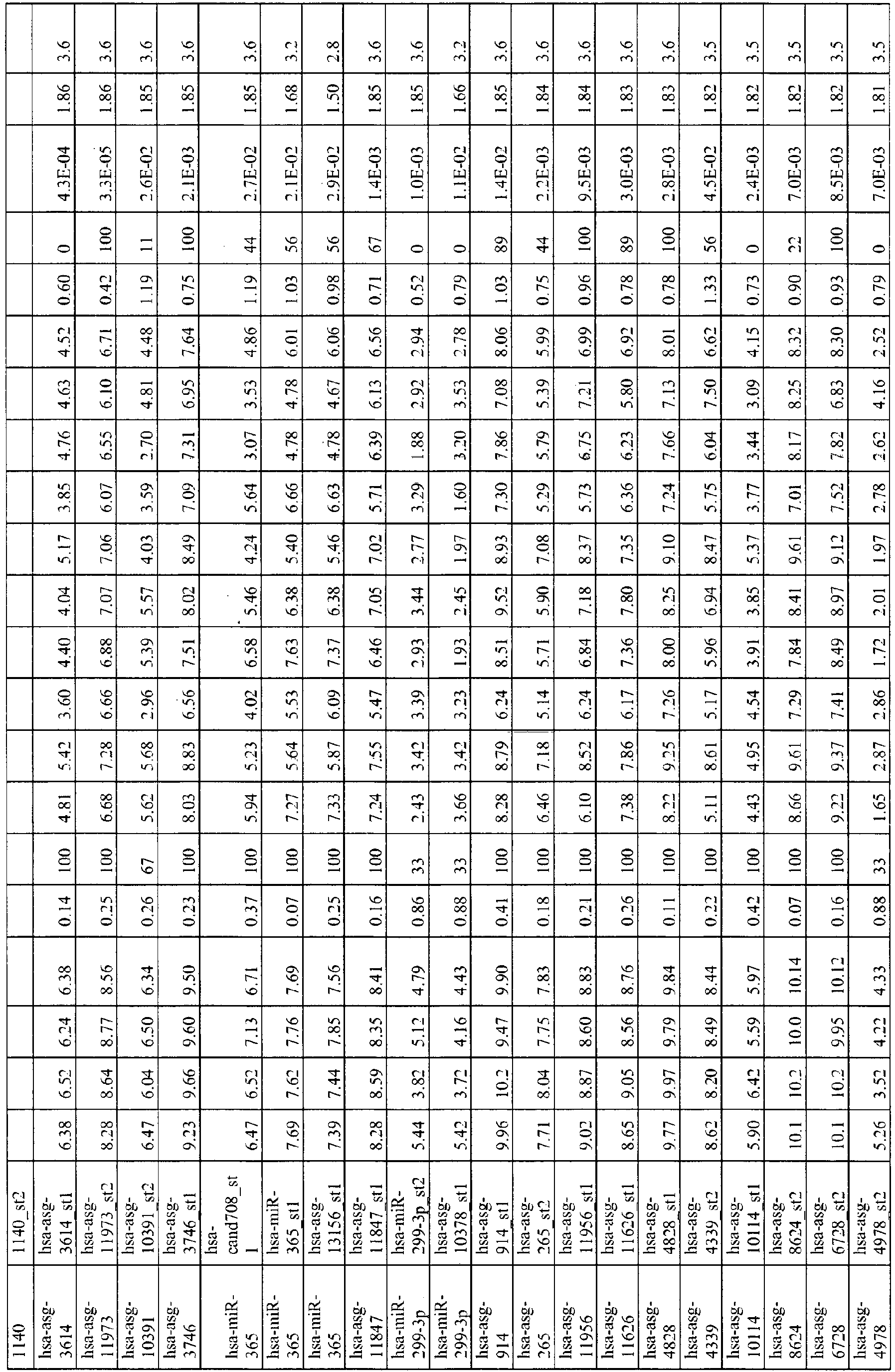

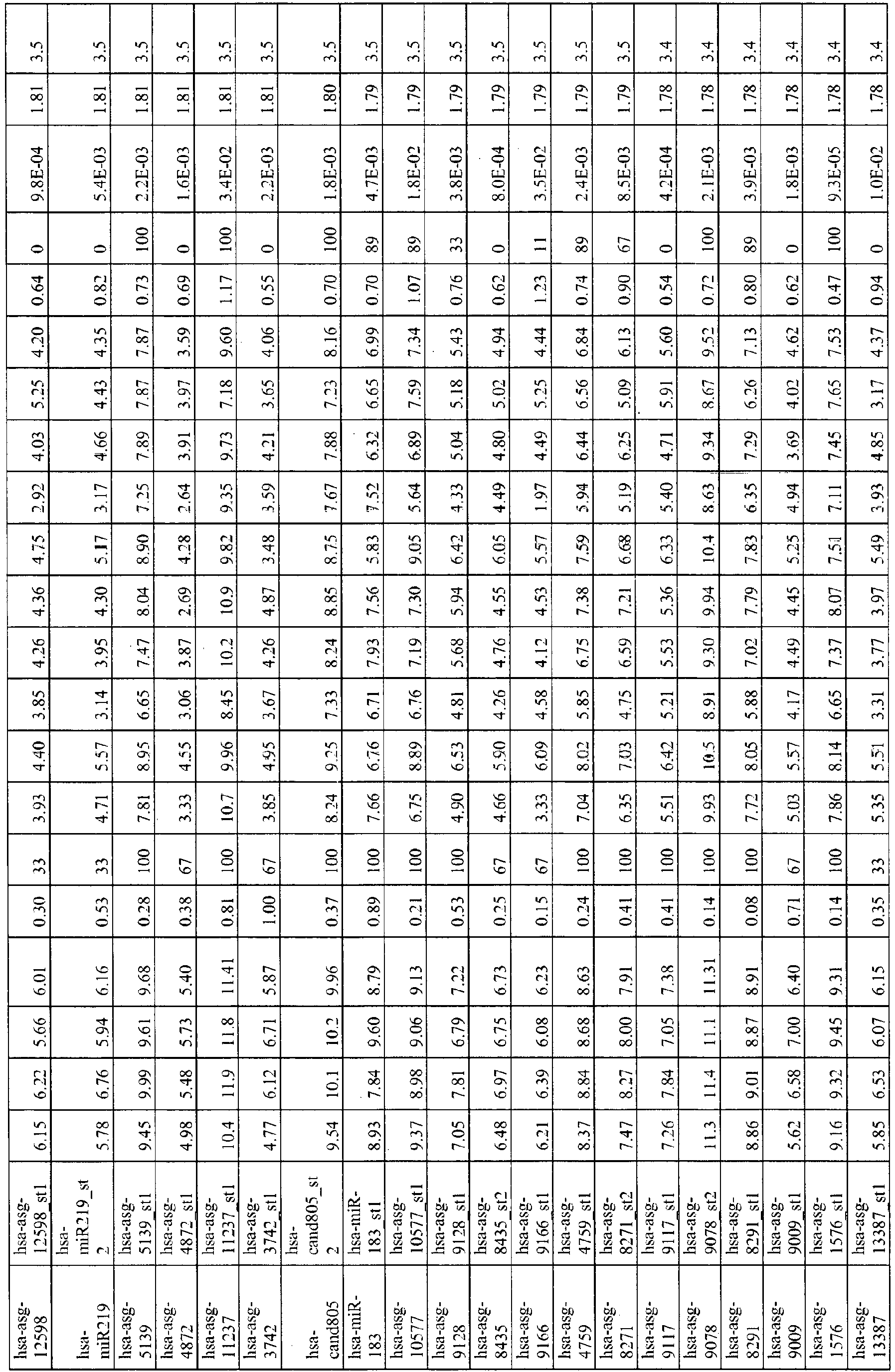

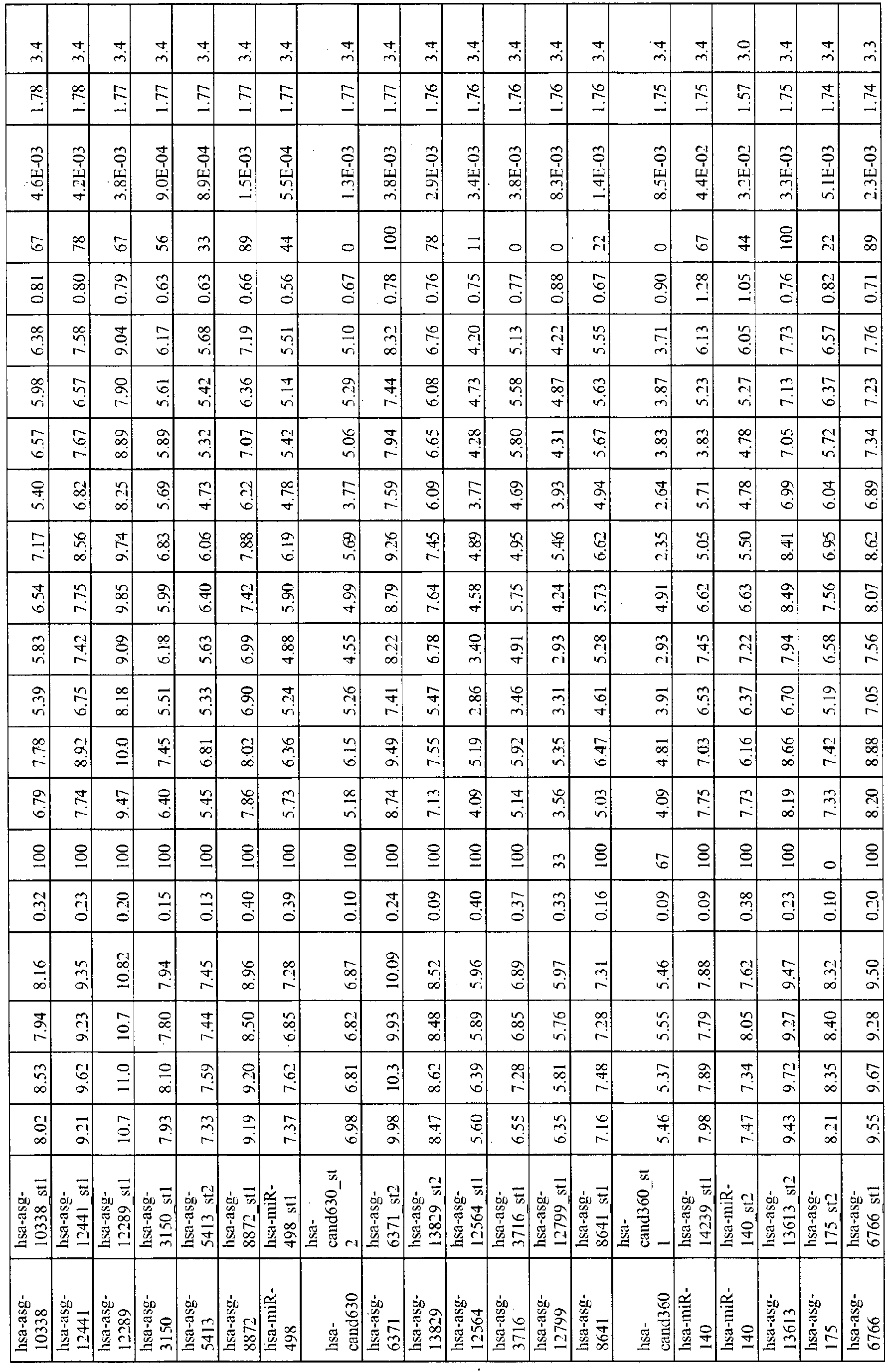

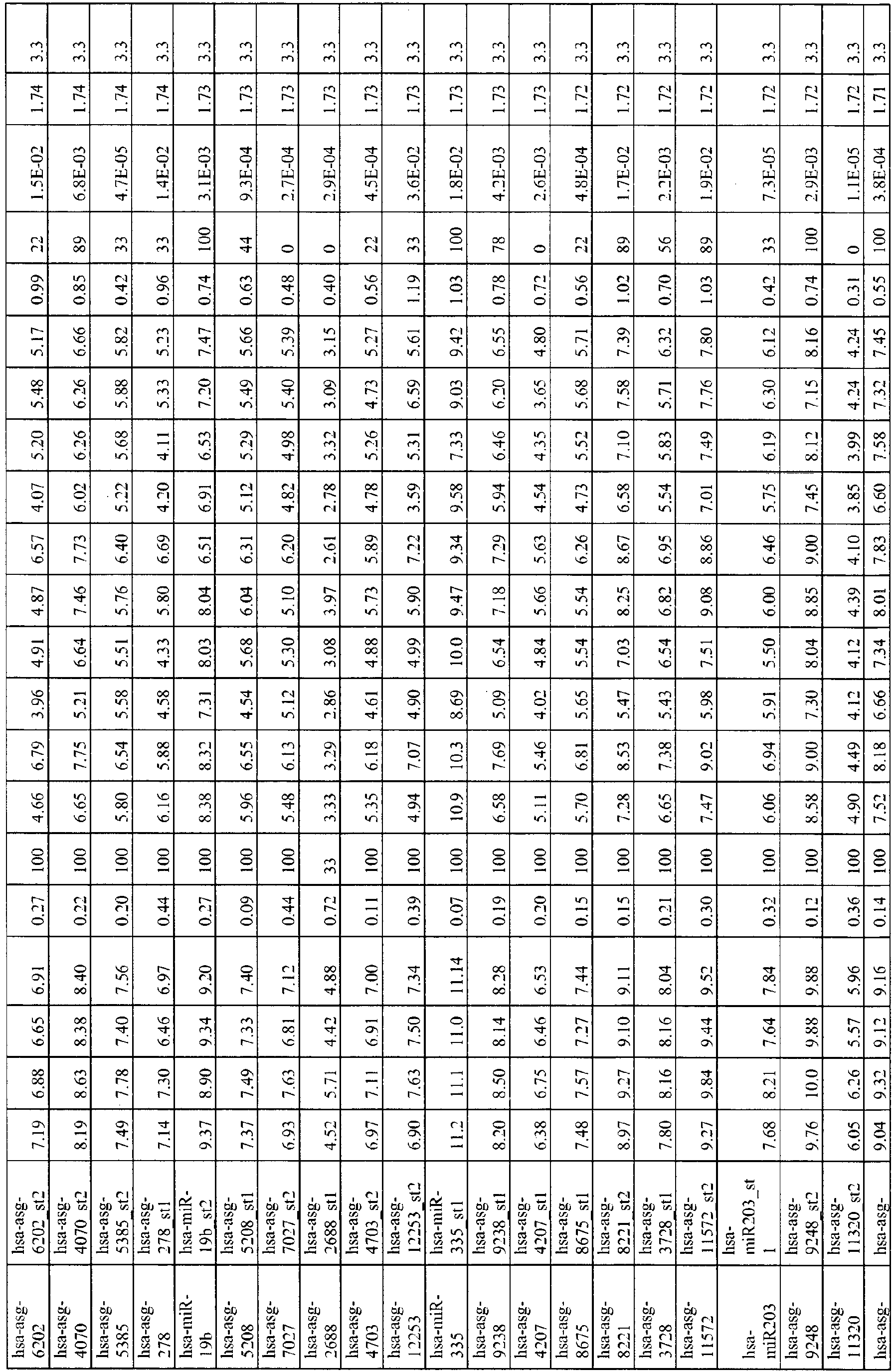

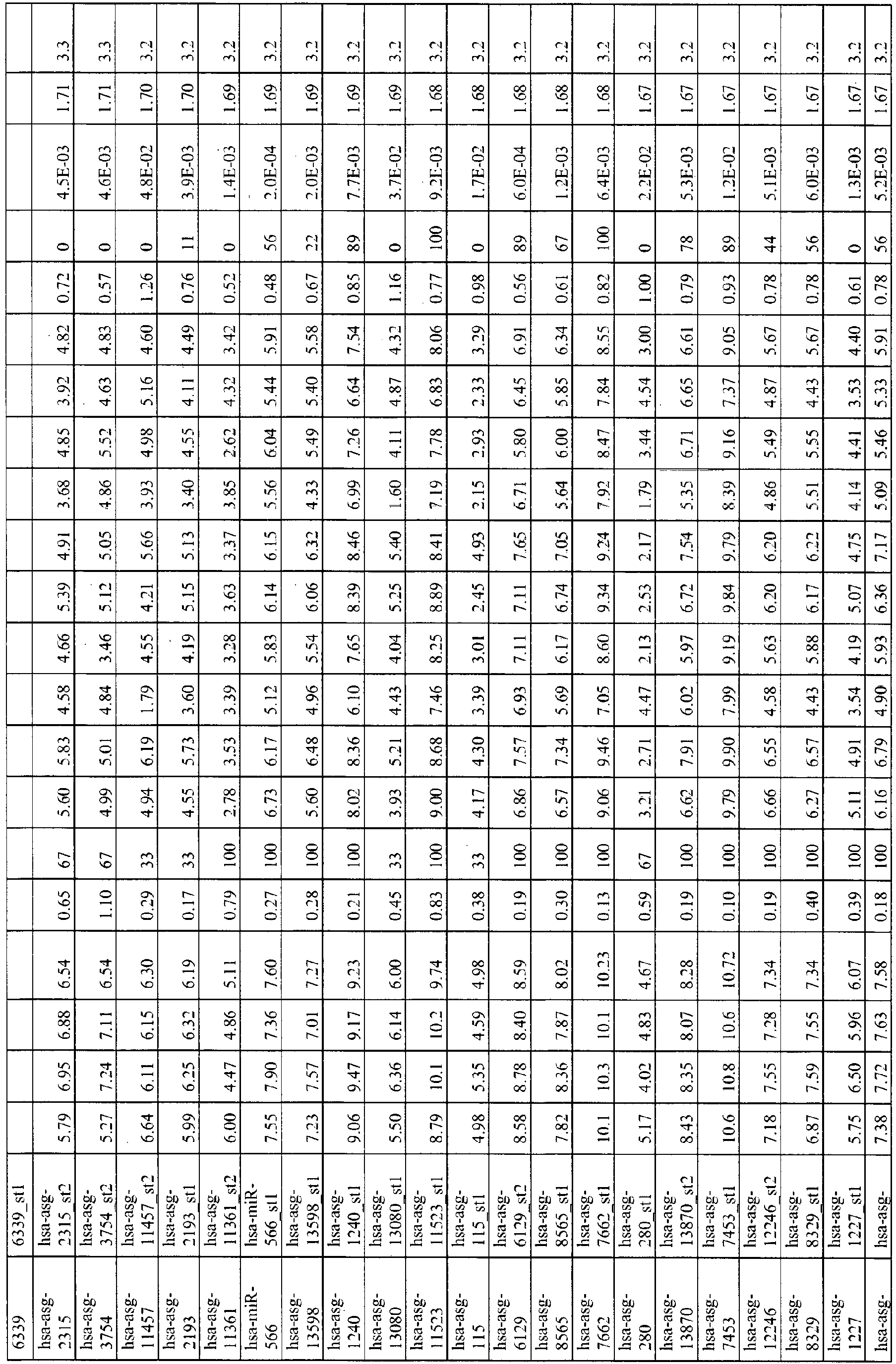

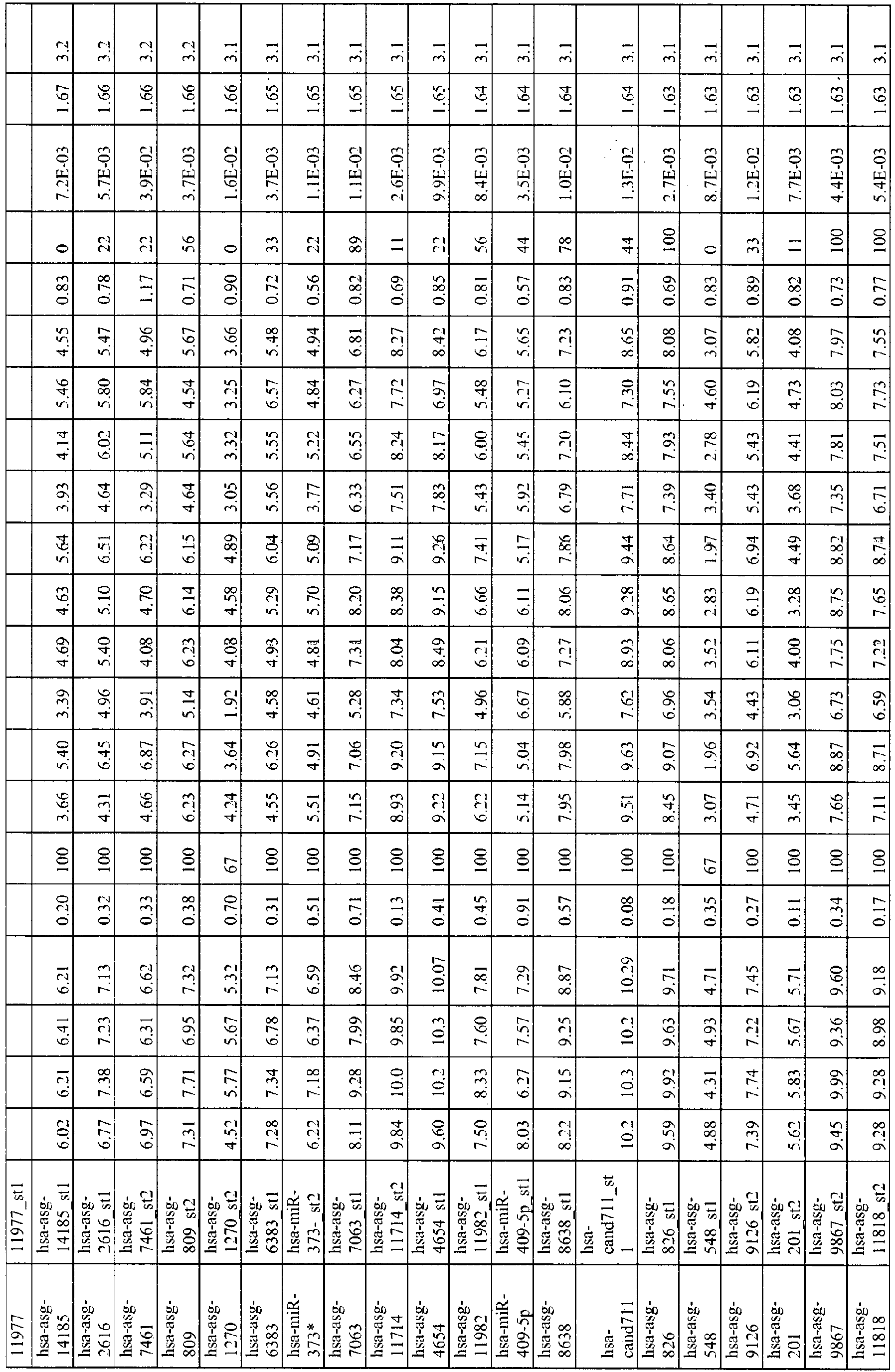

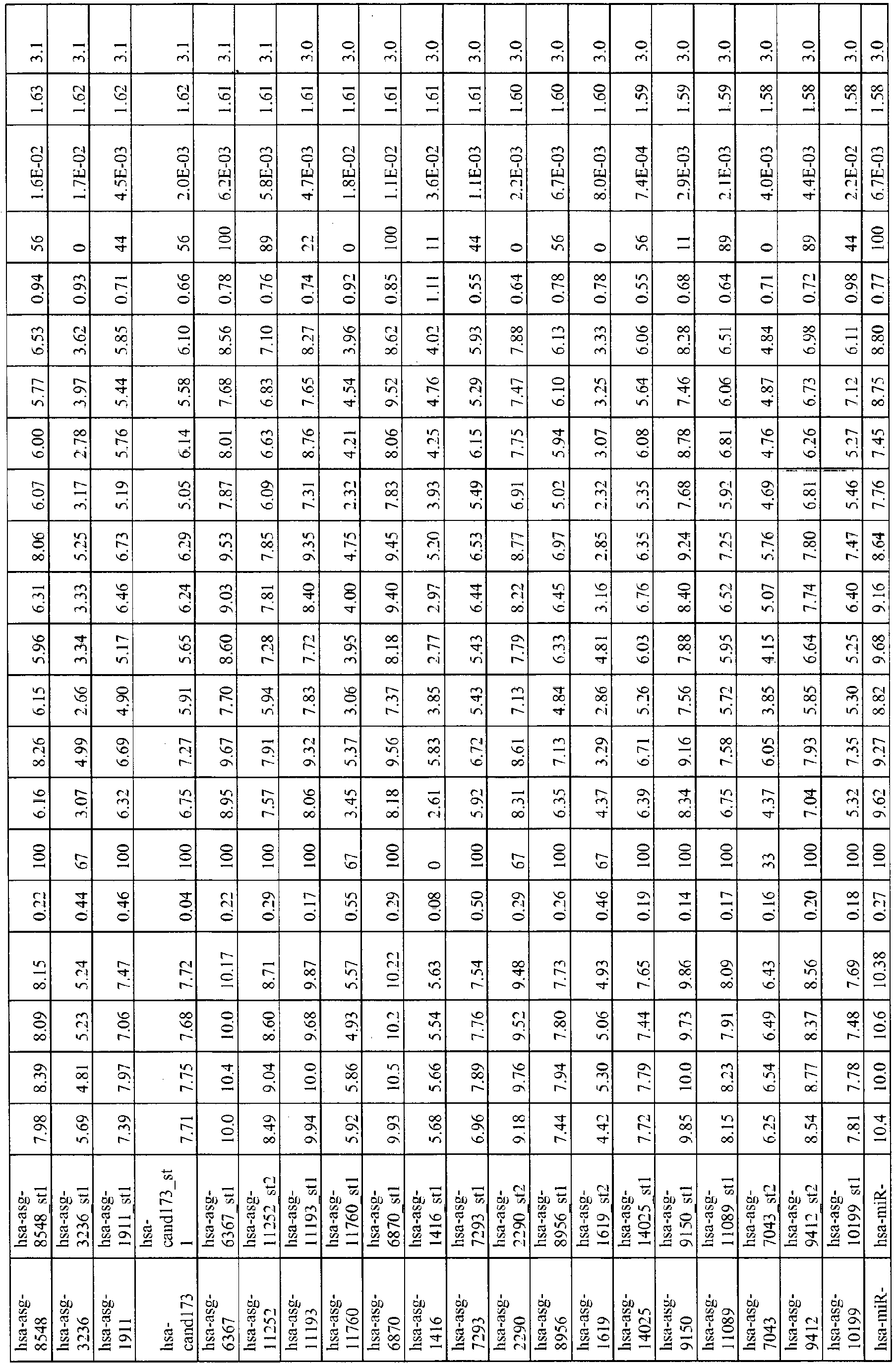

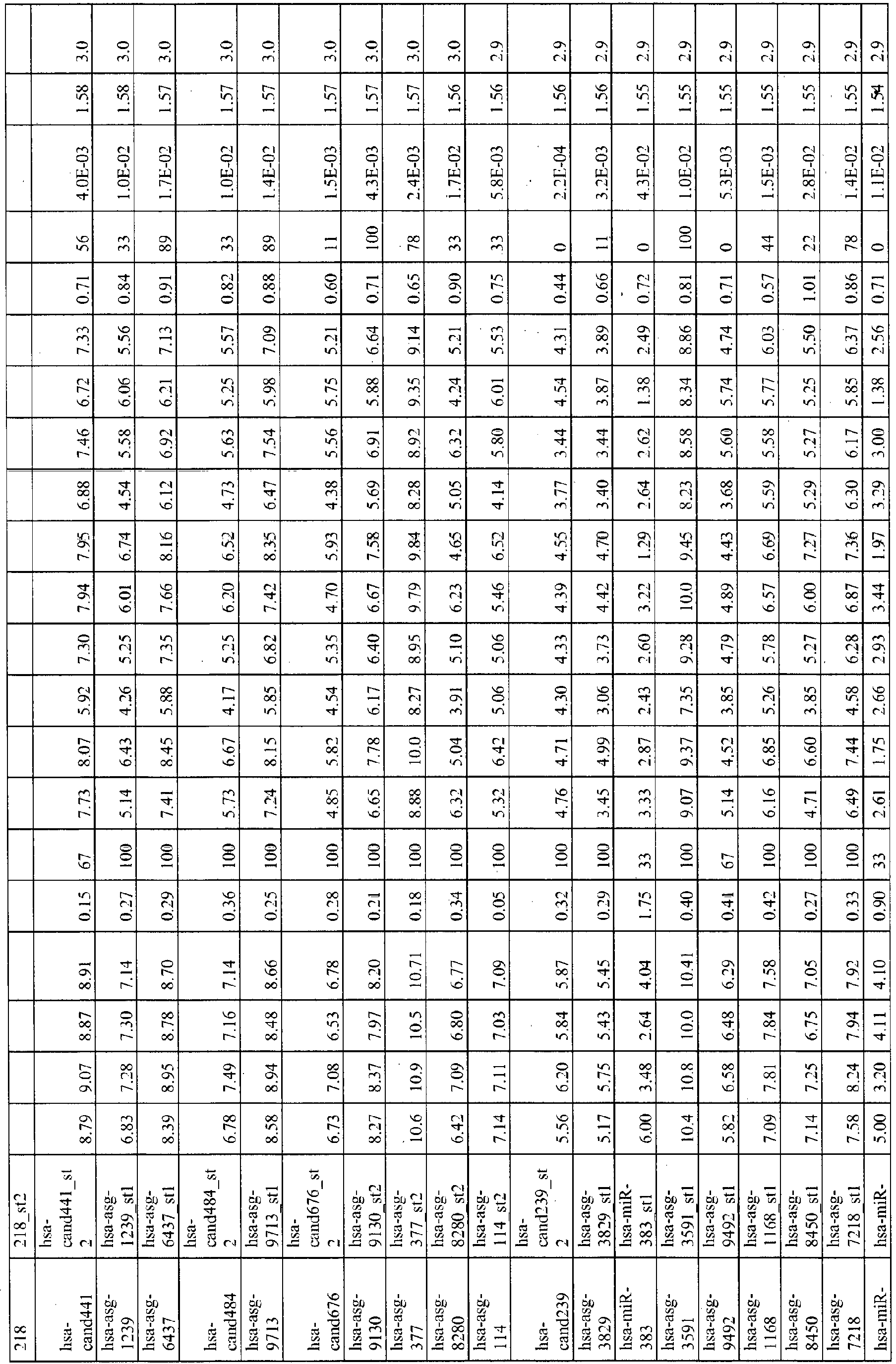

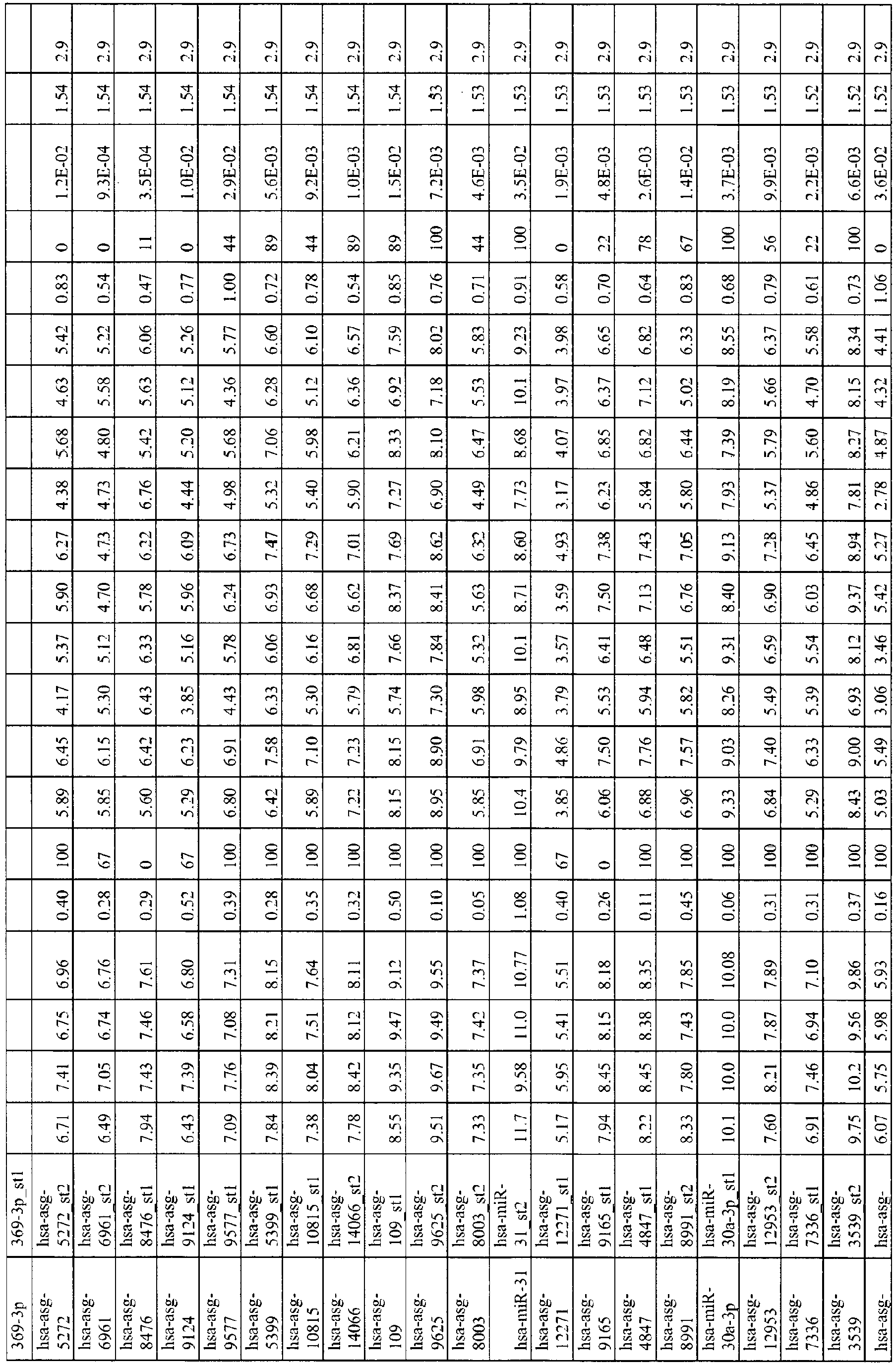

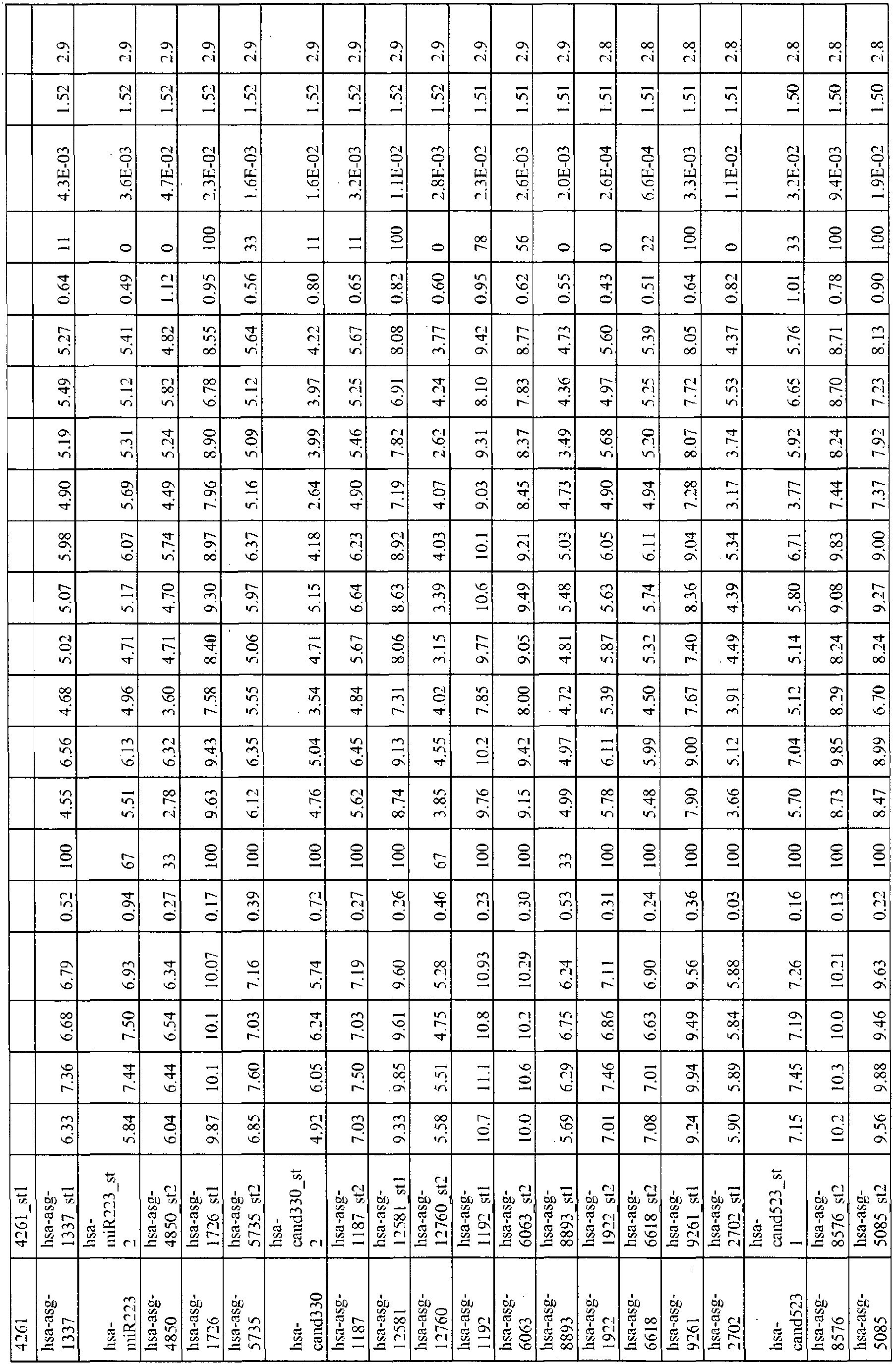

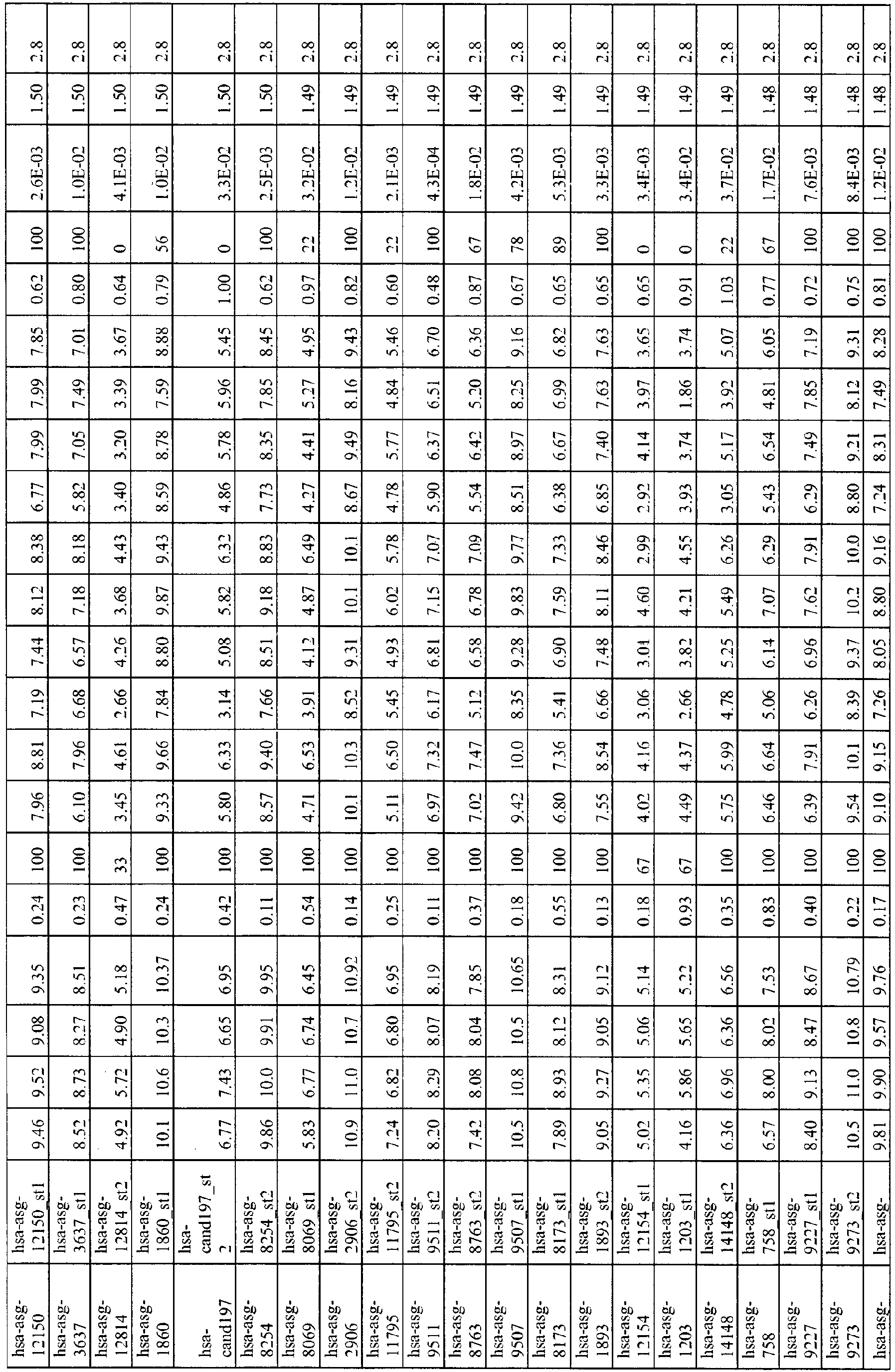

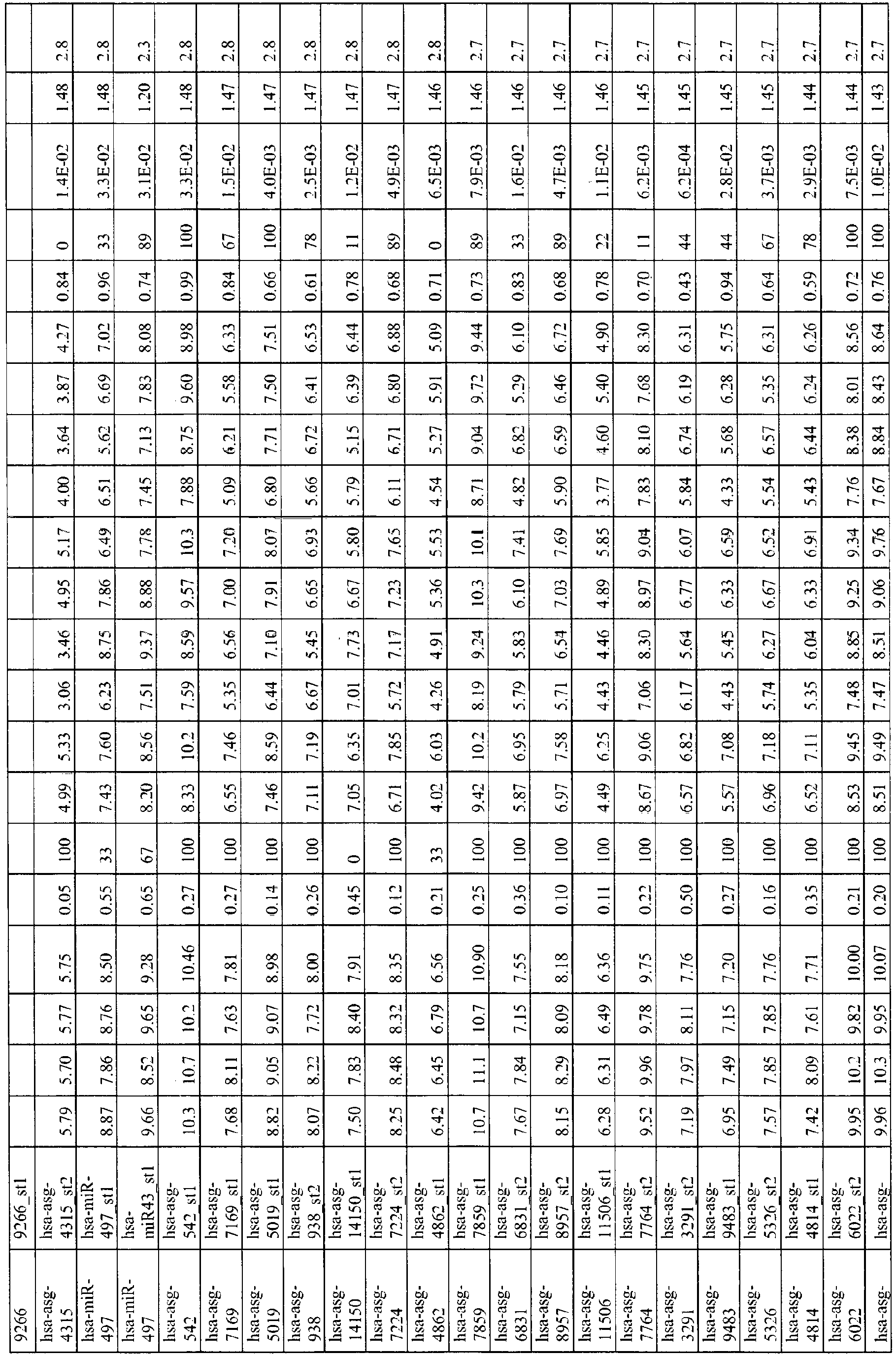

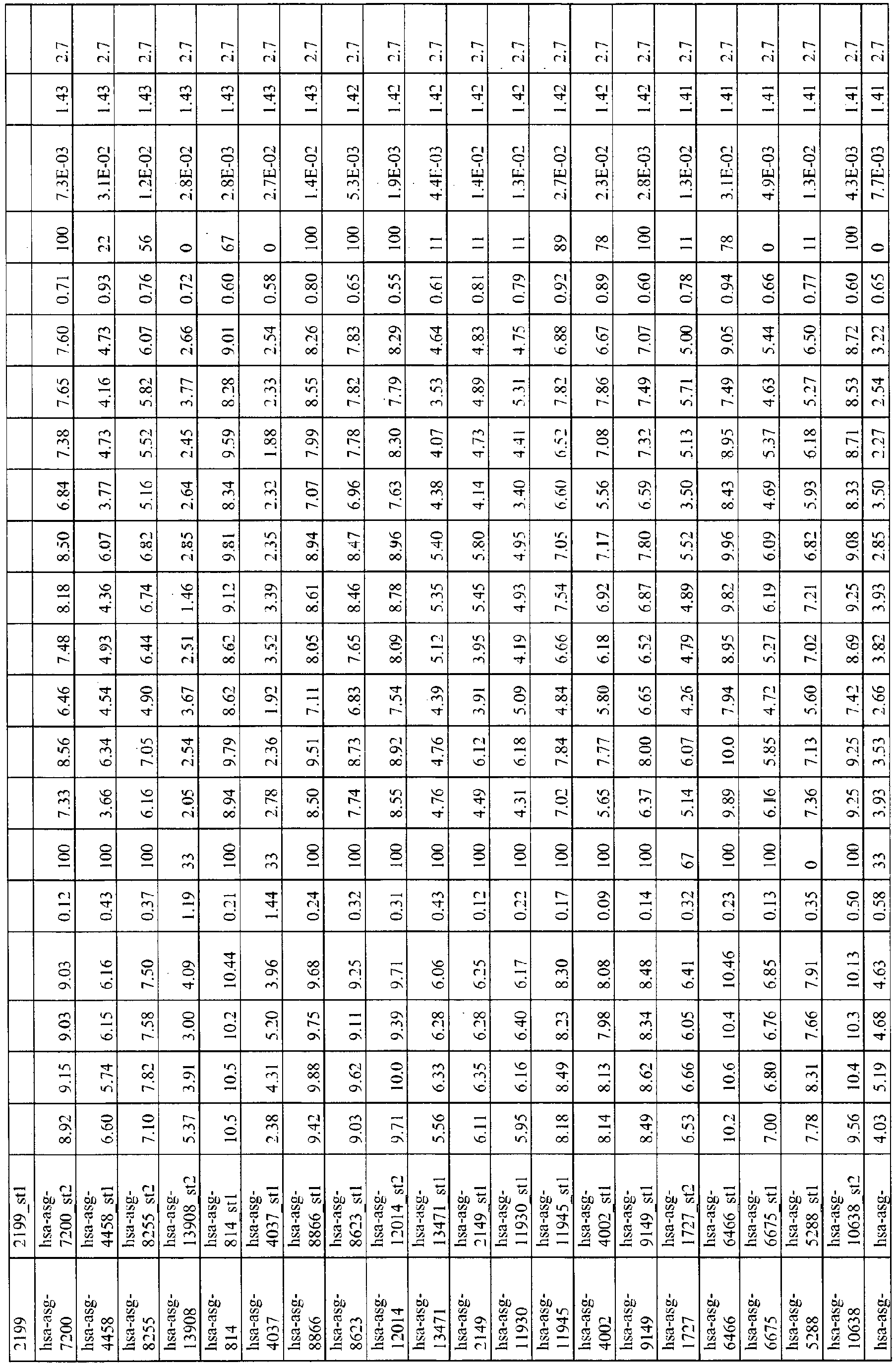

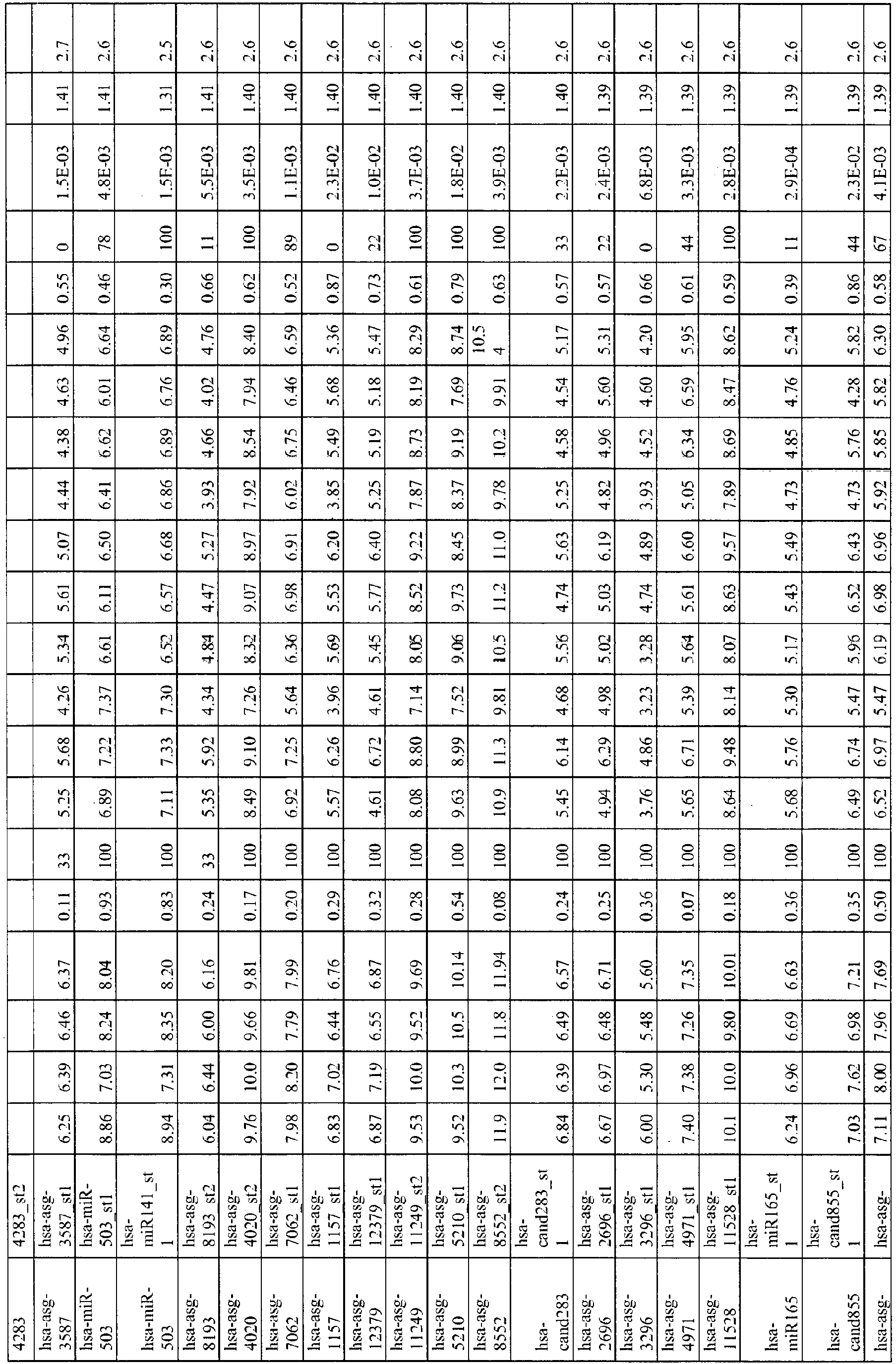

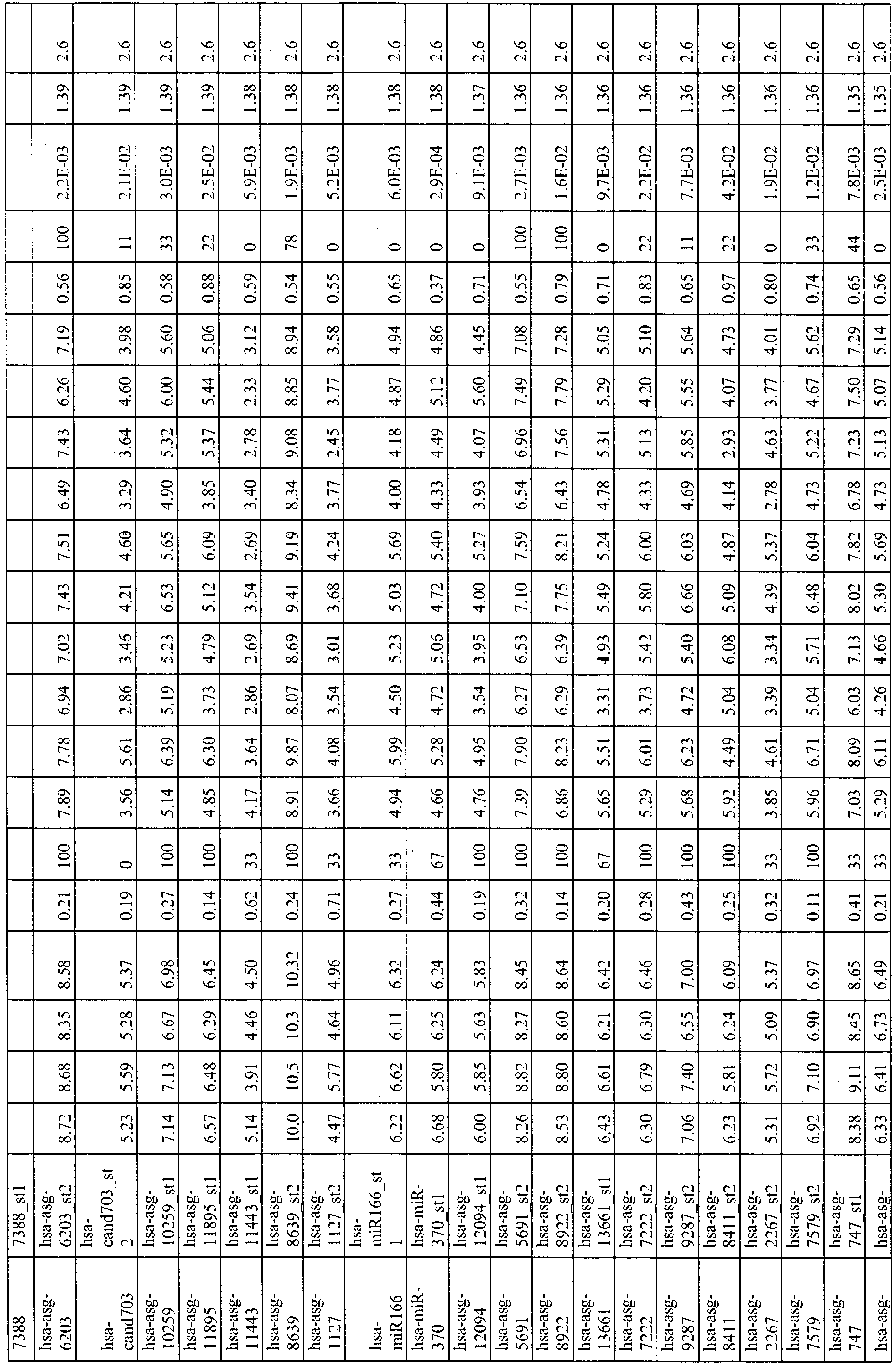

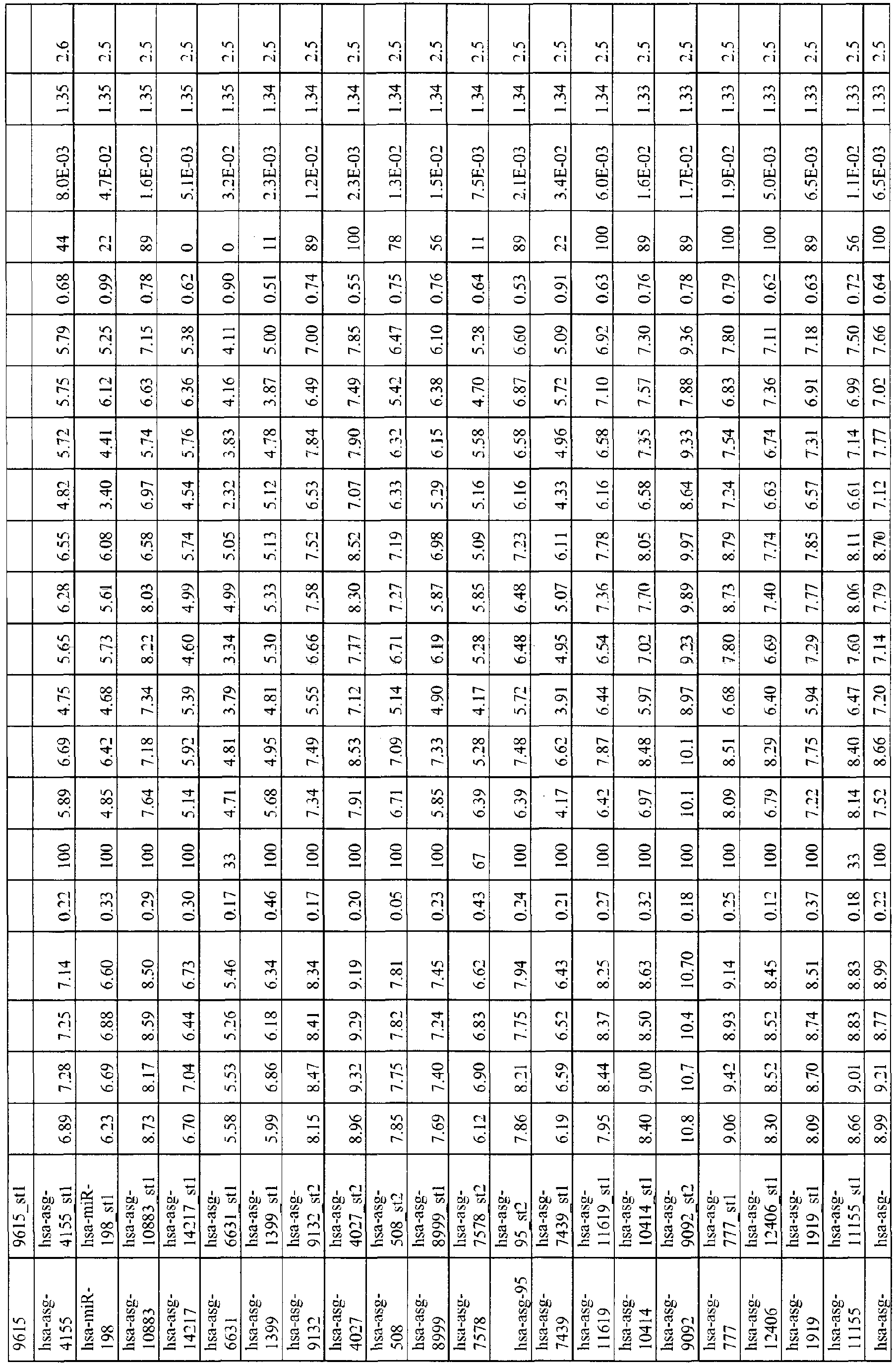

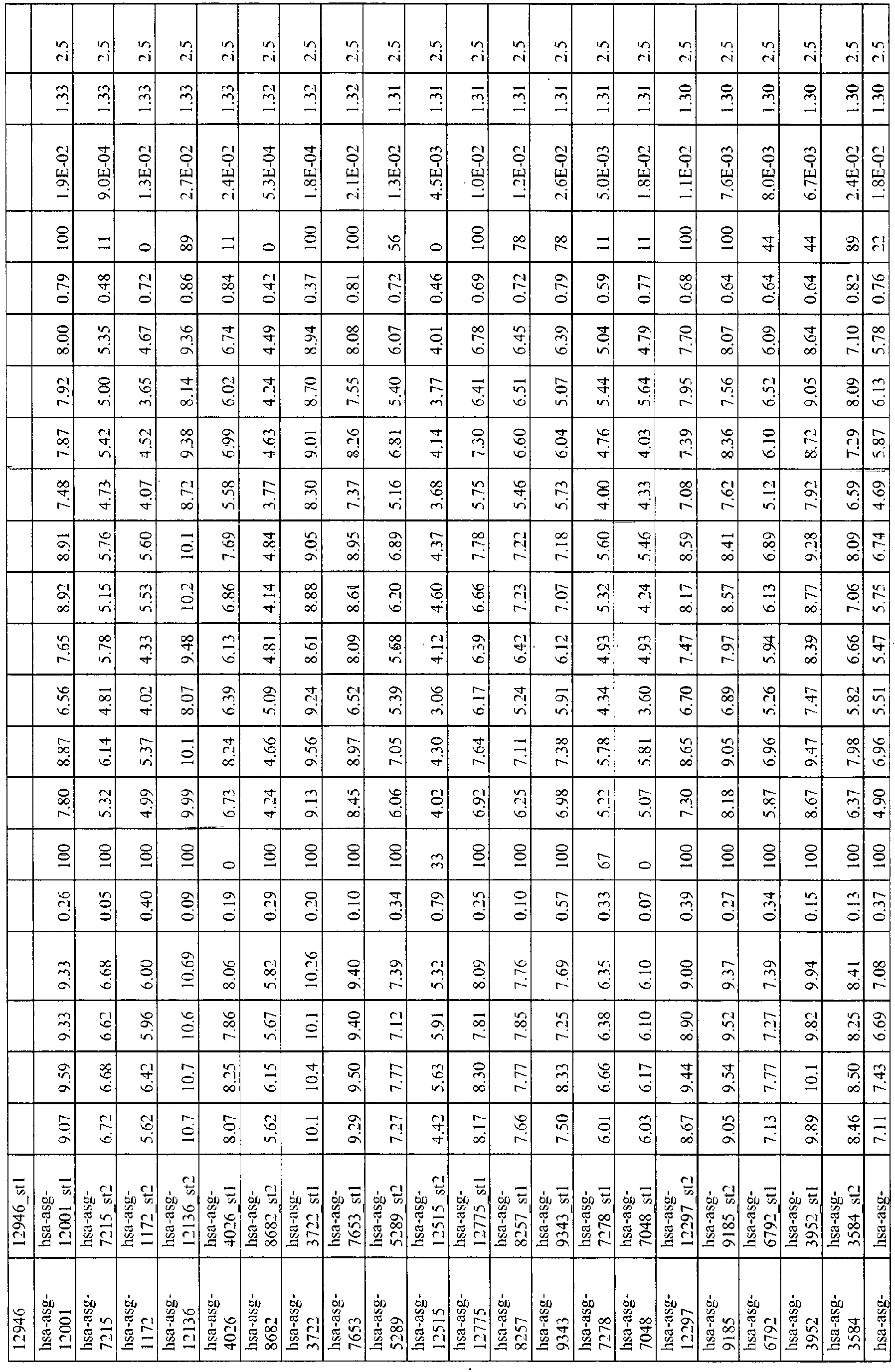

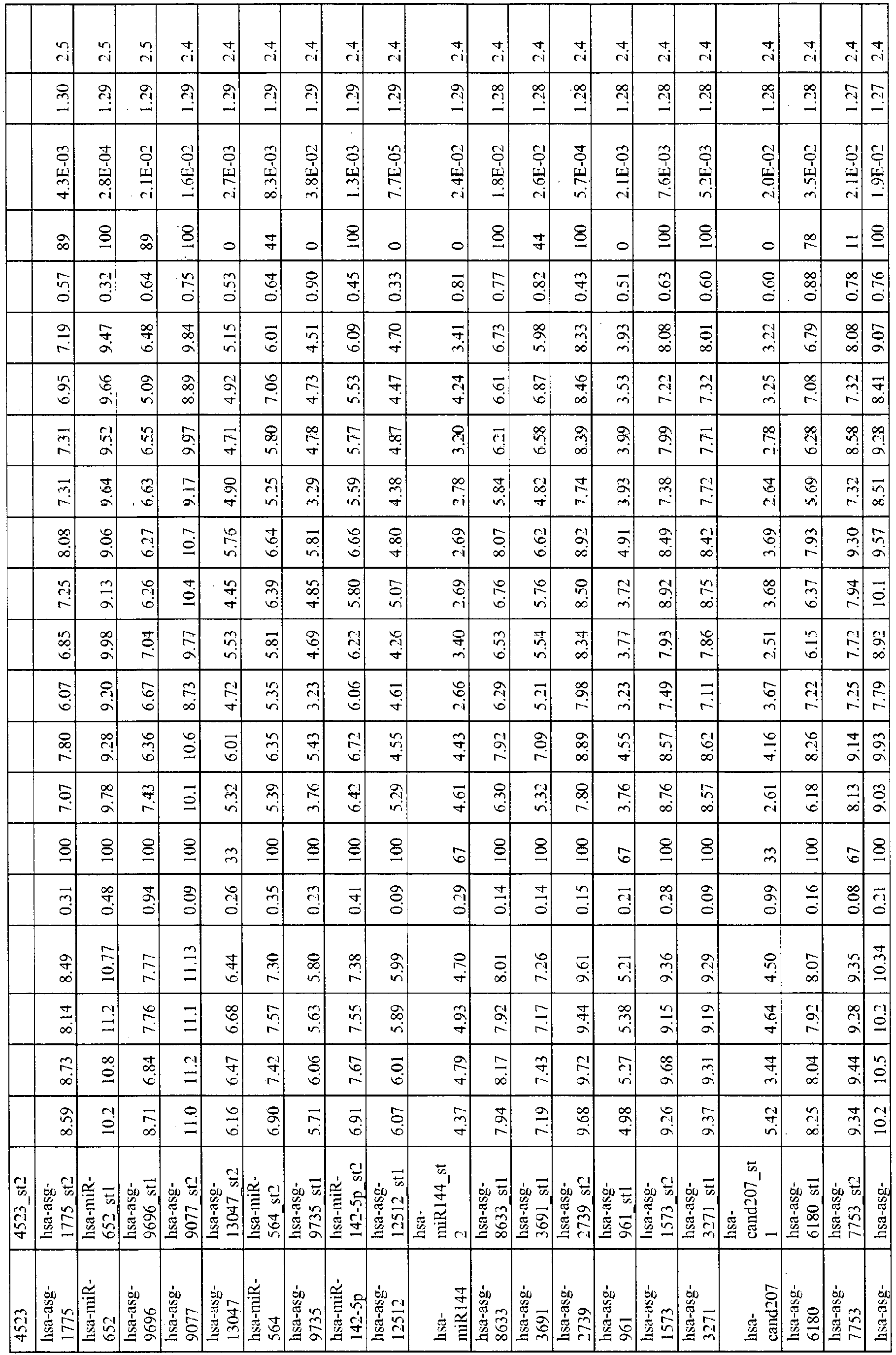

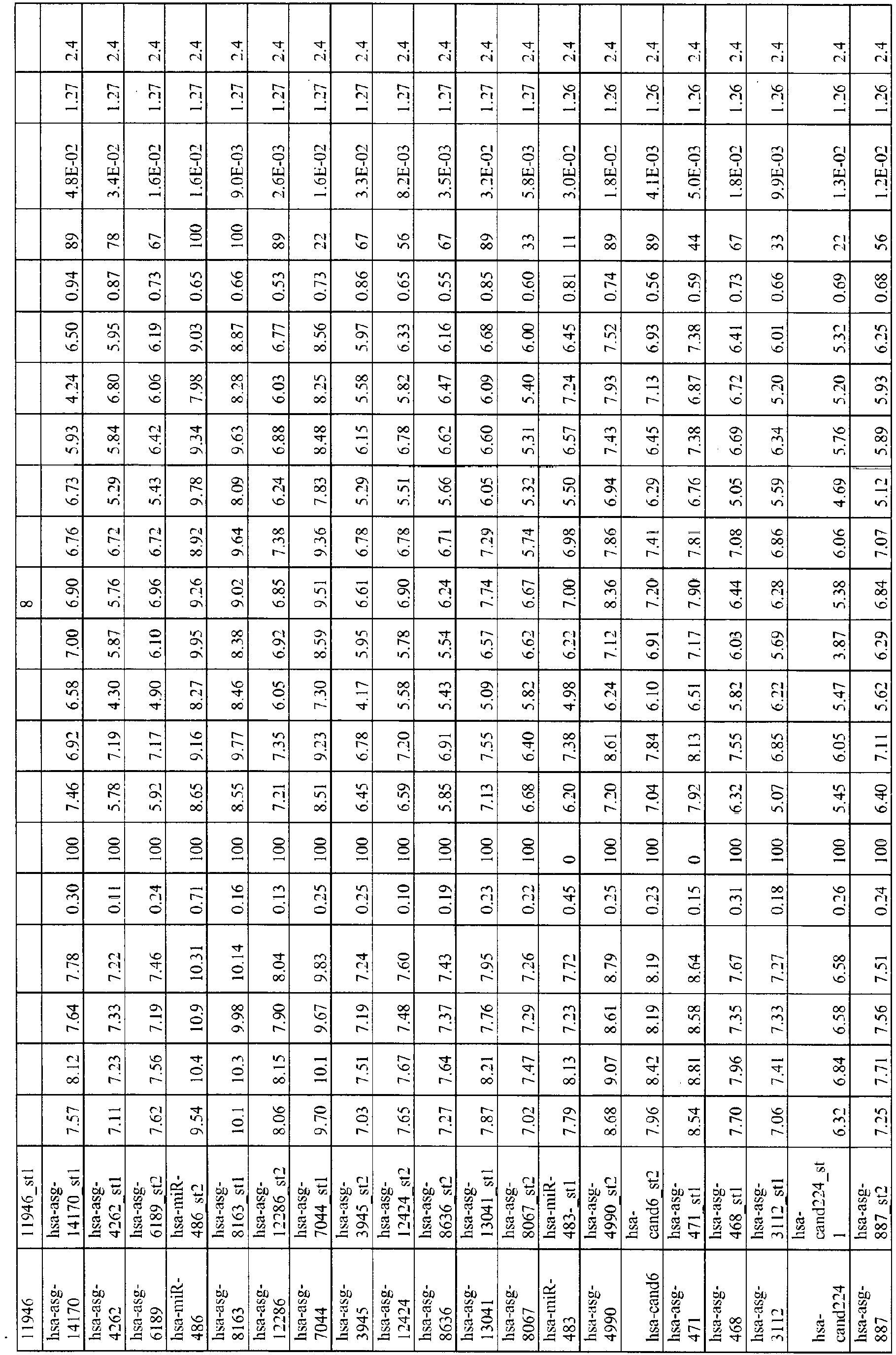

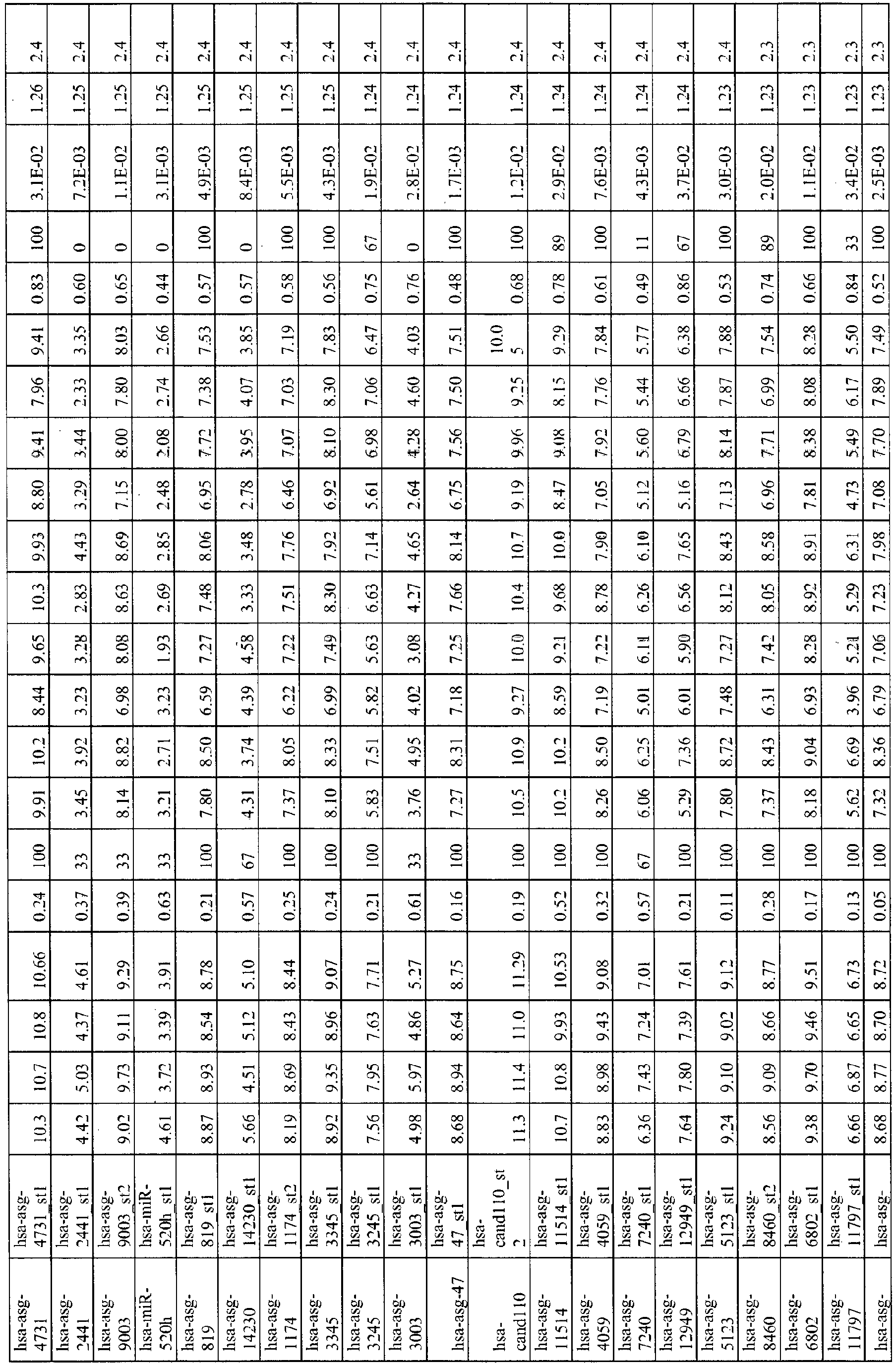

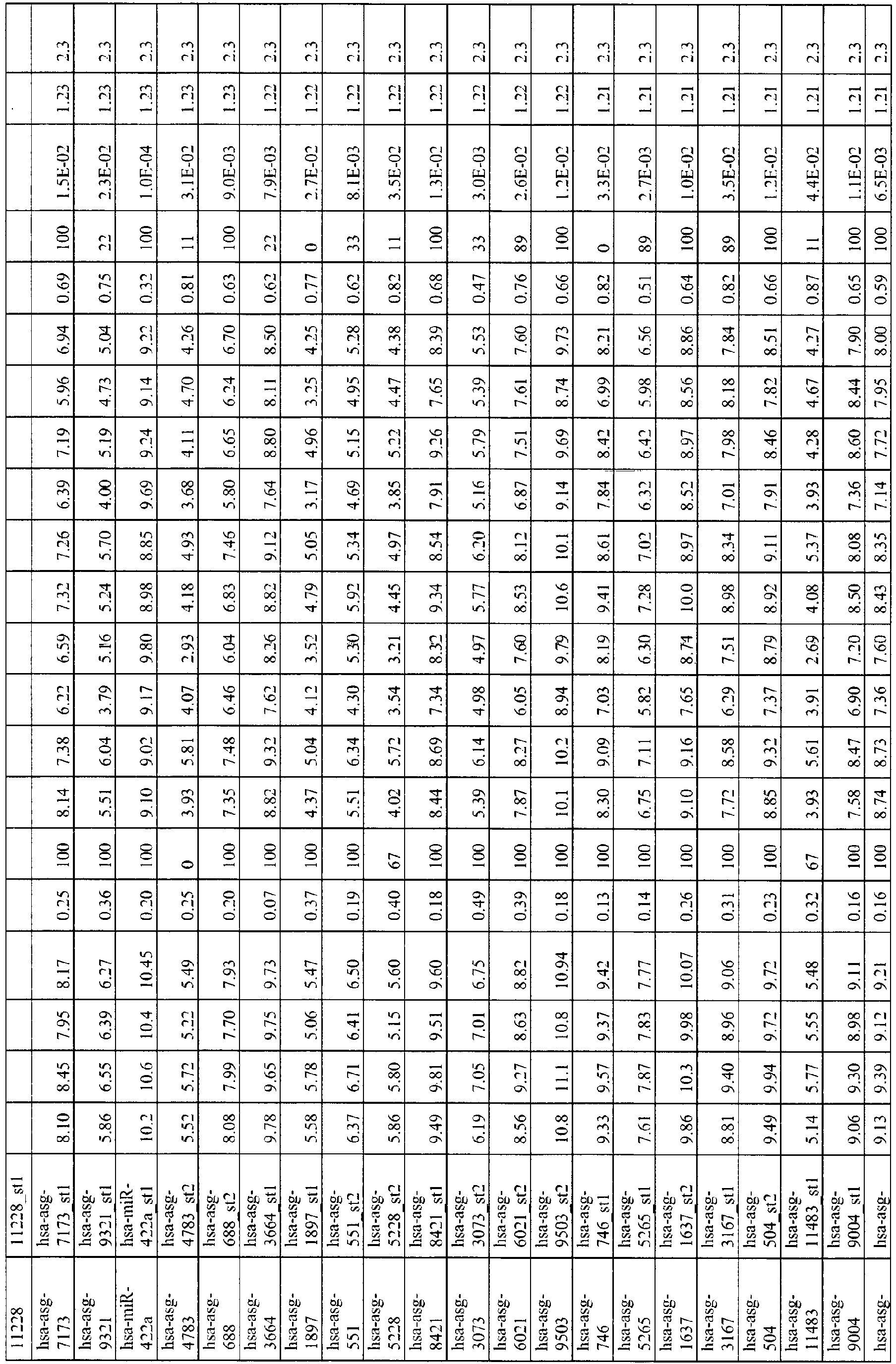

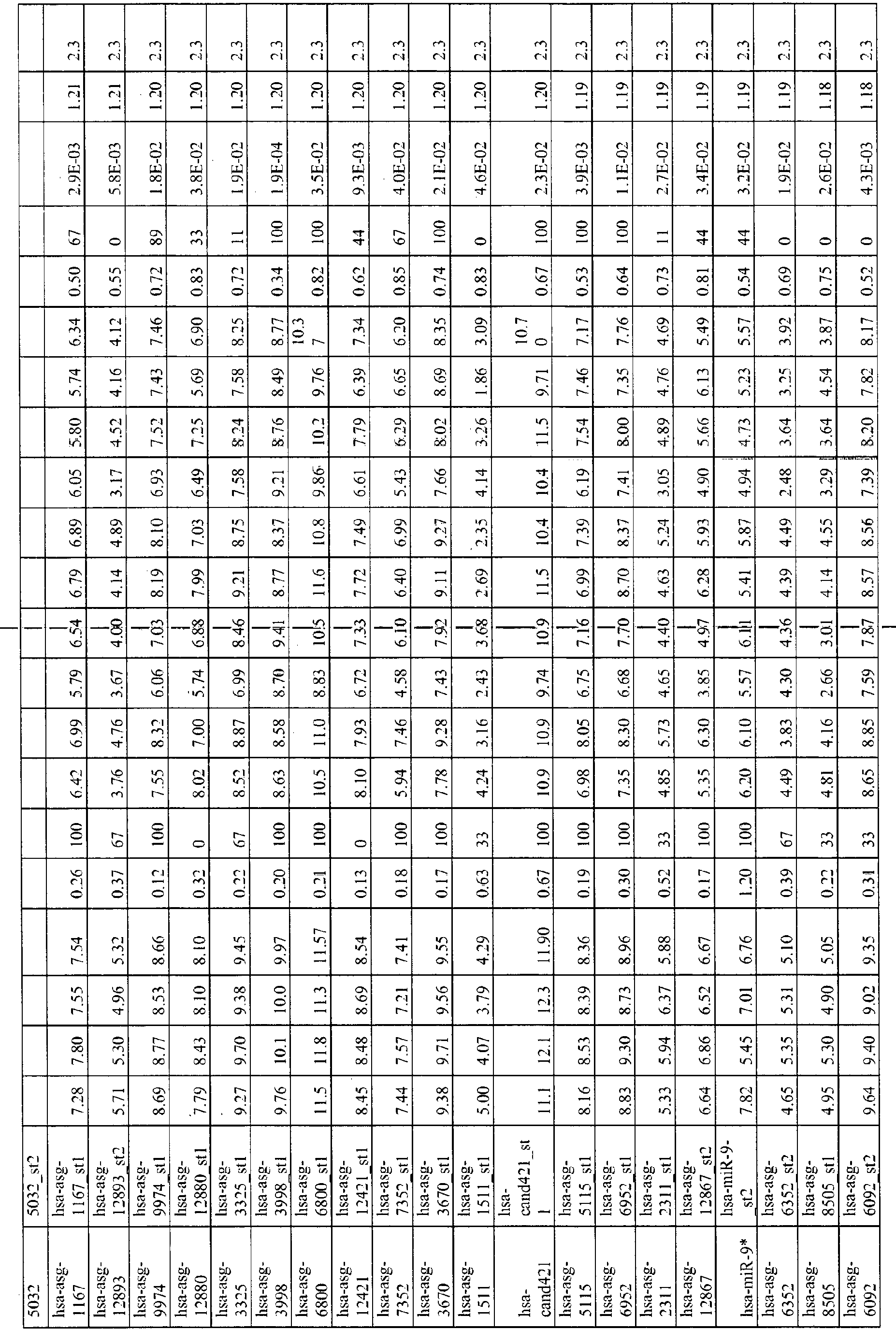

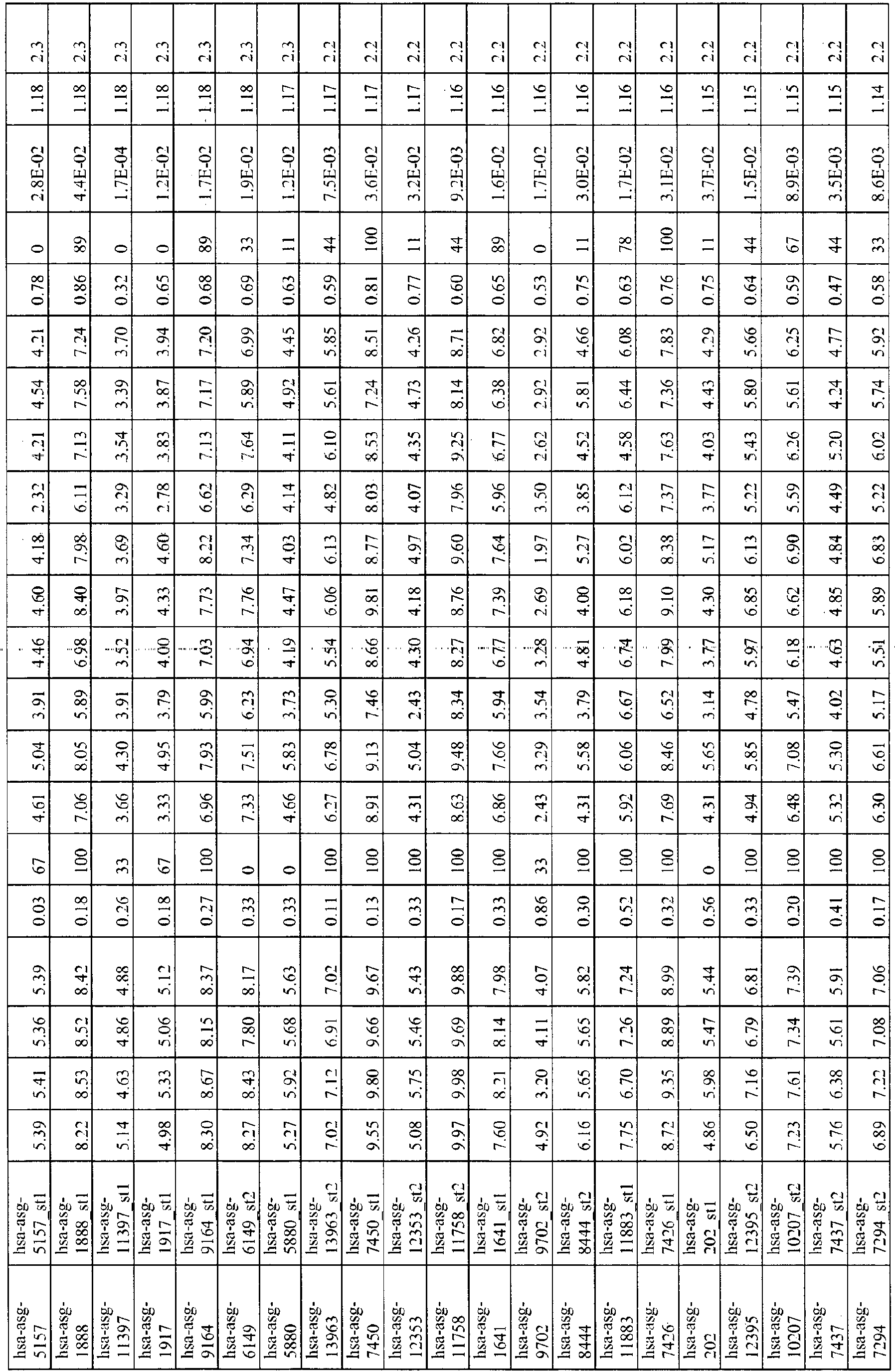

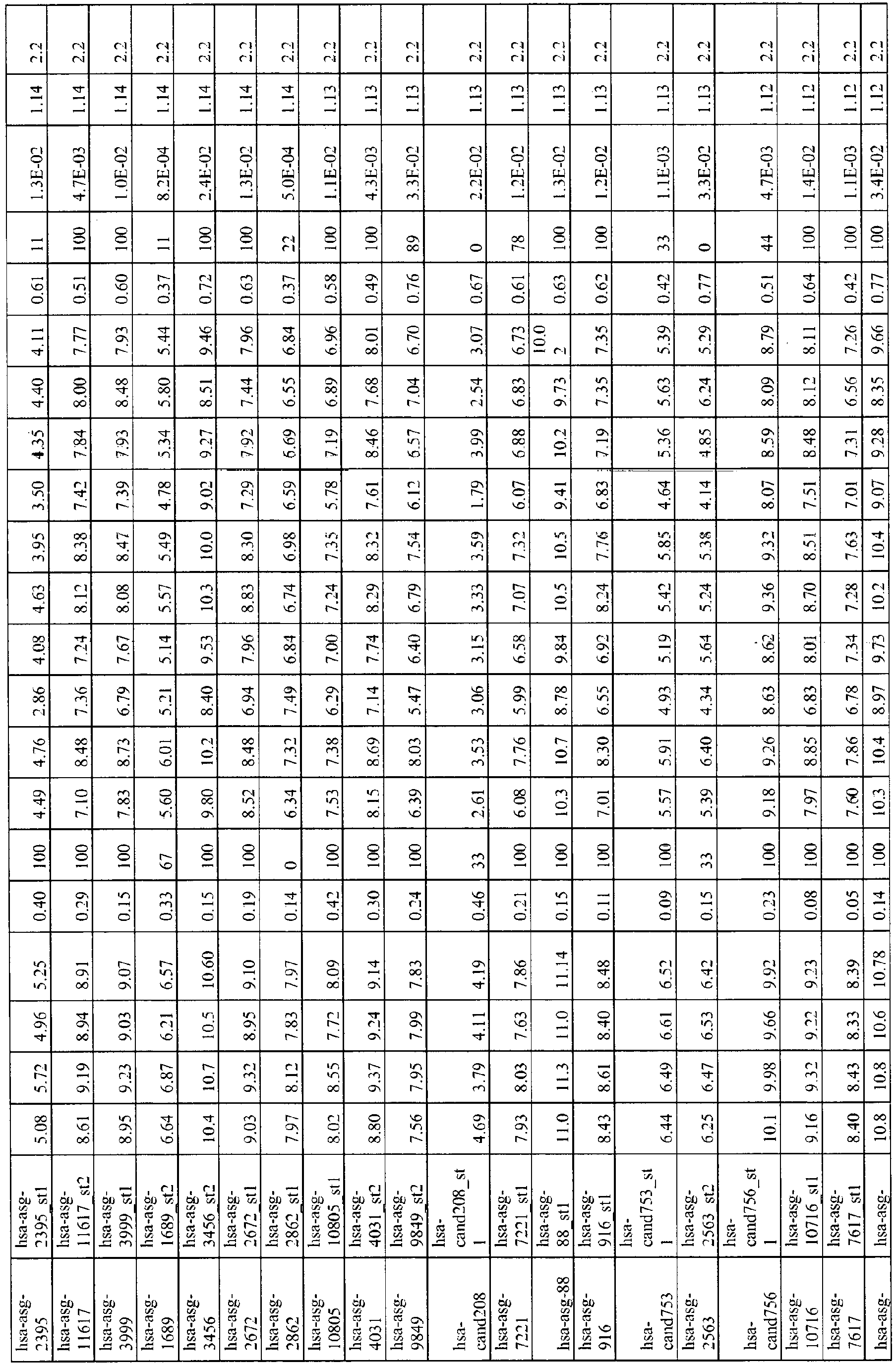

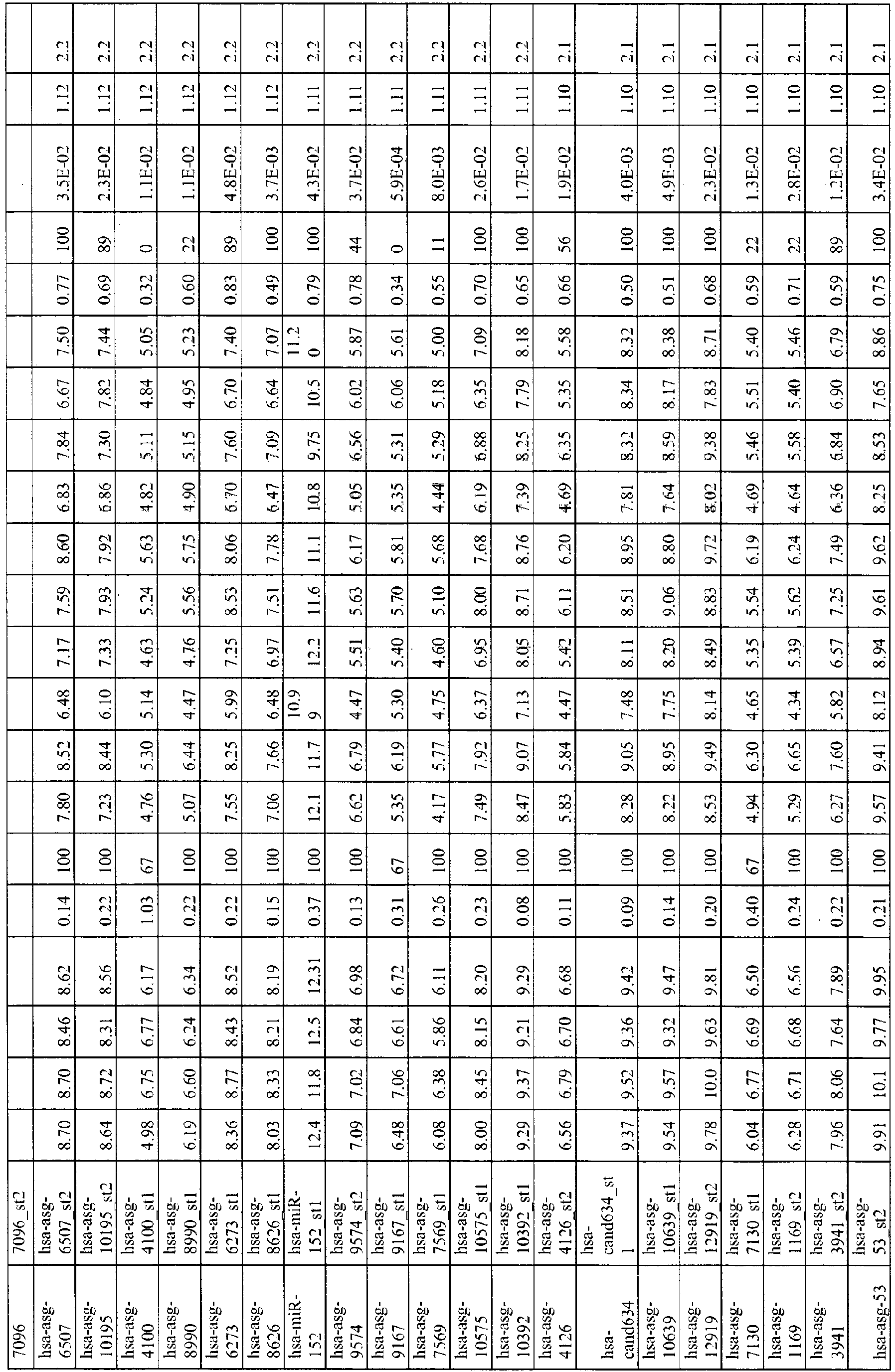

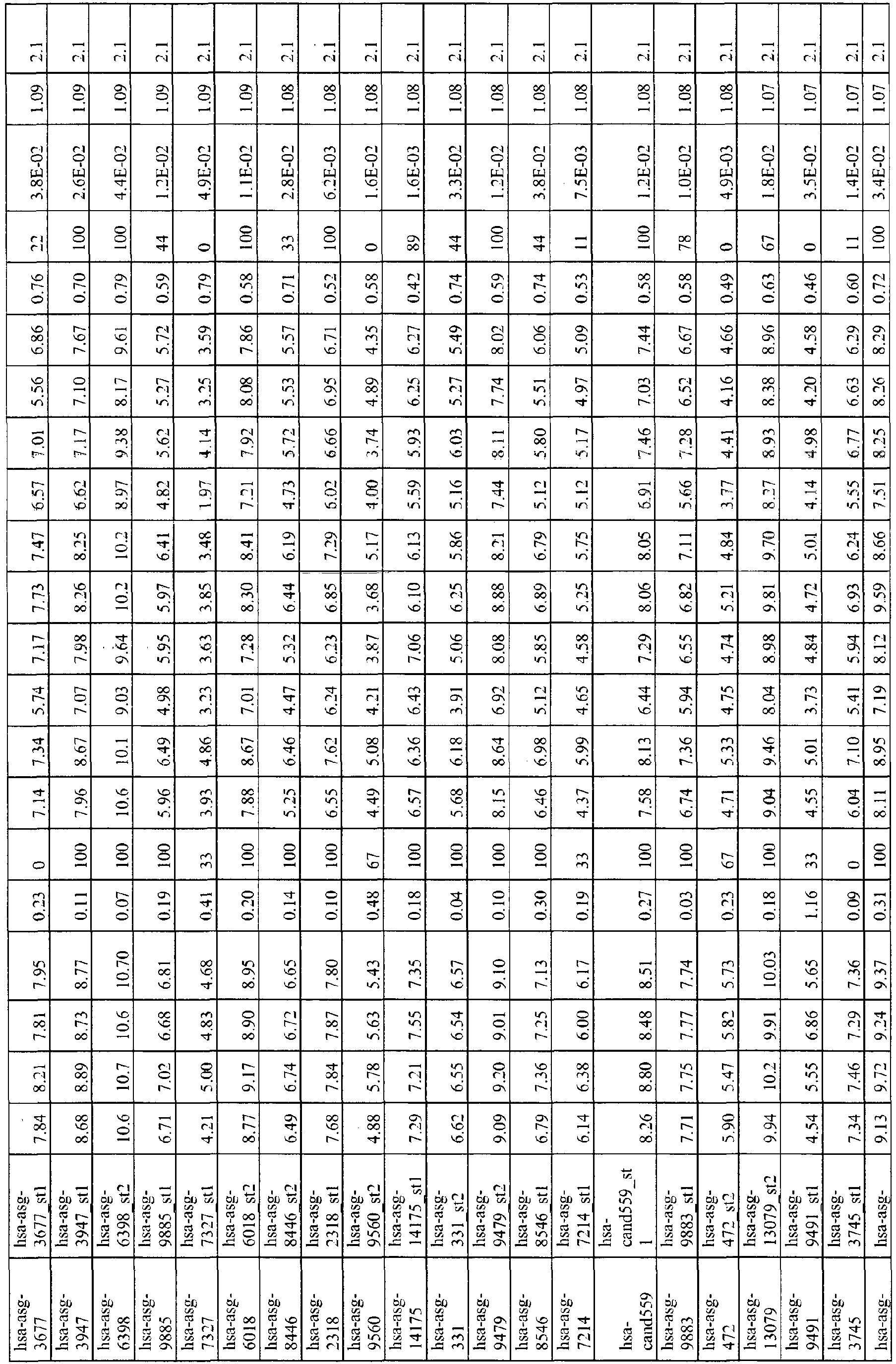

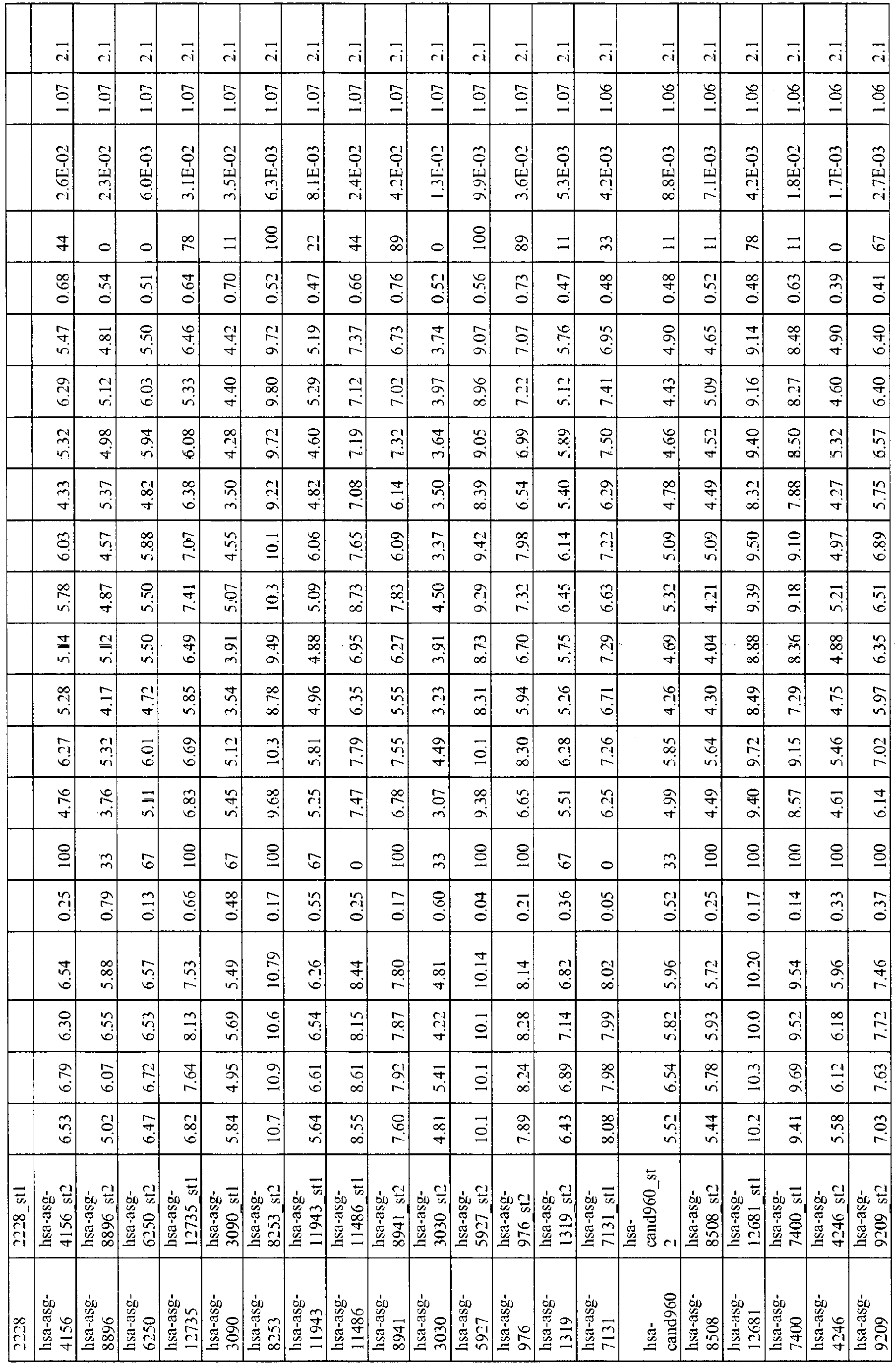

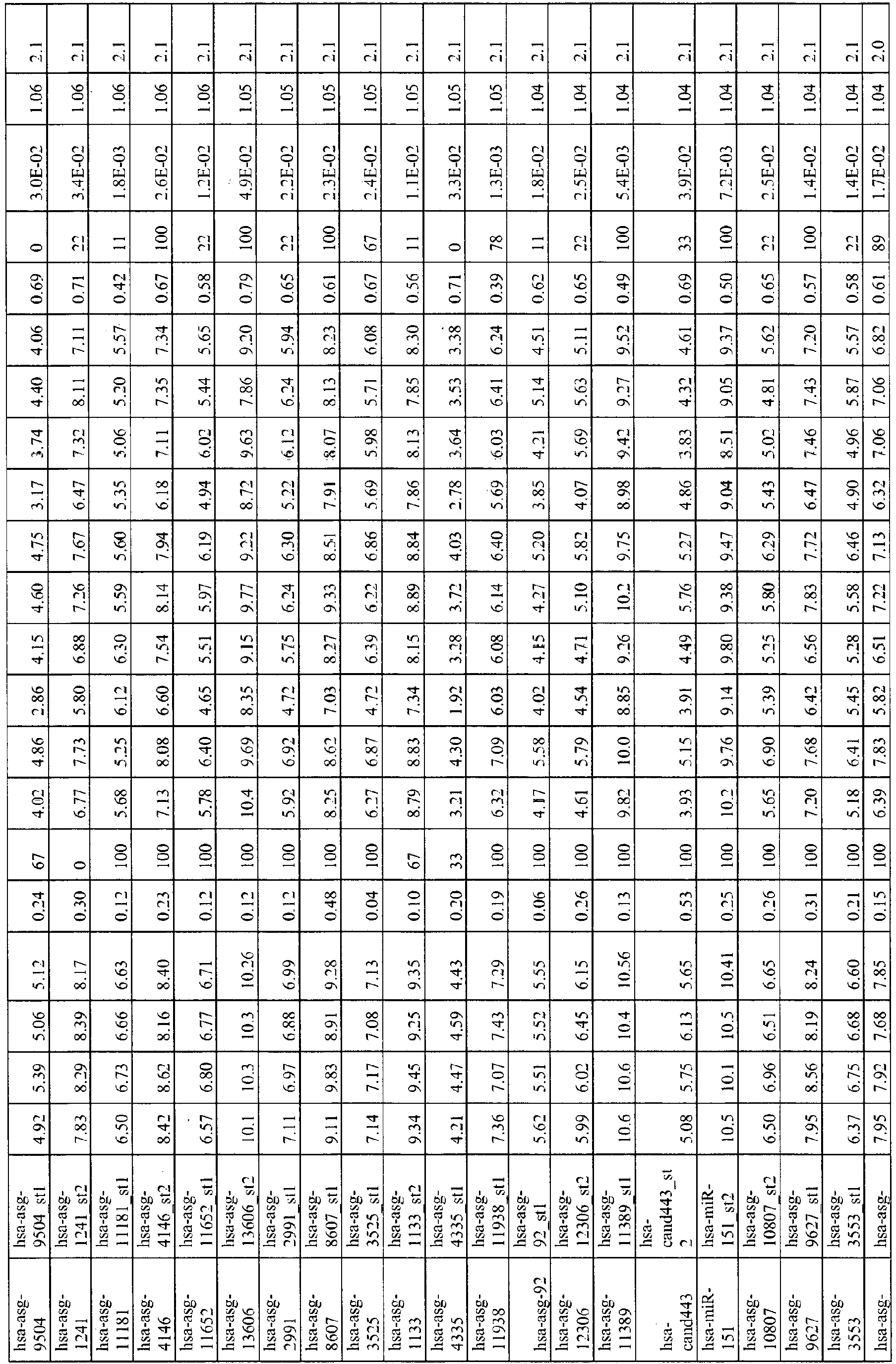

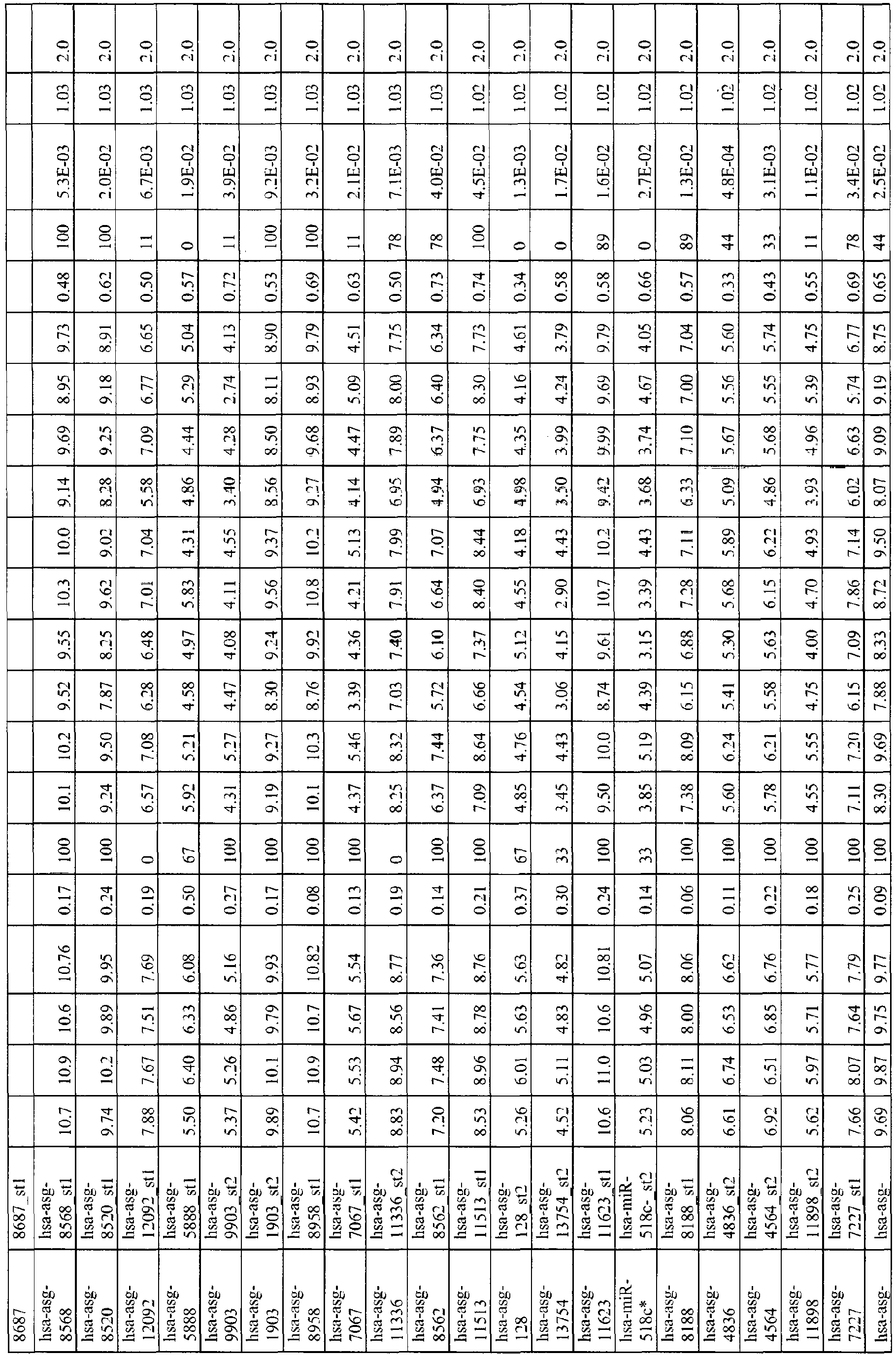

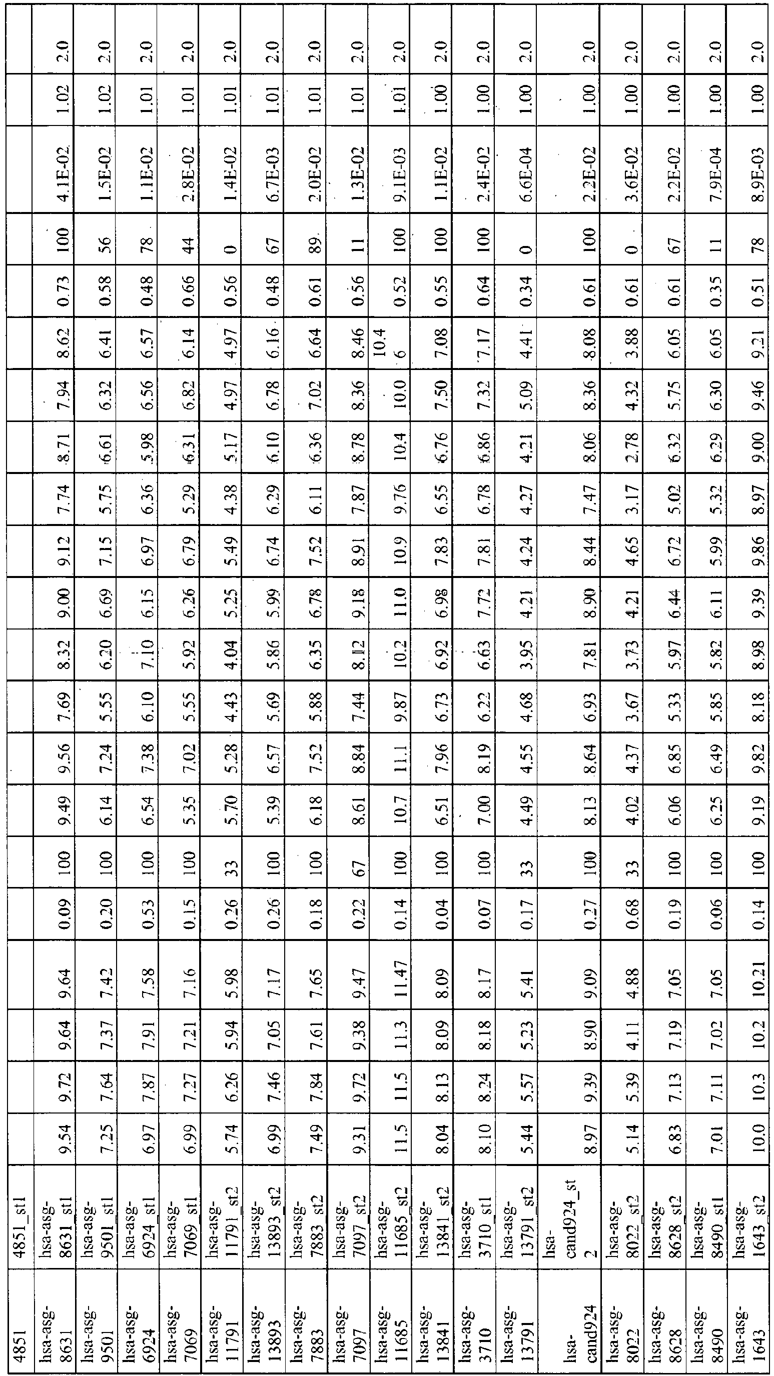

### EXAMPLE 20:

### MIRNAS DIFFERENTALLY EXPRESSED BETWEEN NORMAL BLADDER TISSUE AND NORMAL LYMPH NODES

To identify miRNAs that may be useful markers for metastasis of cancerous bladder cells to lymph nodes, the inventors identified miRNAs that are expressed at significantly higher levels in normal bladder tissue than in cancer-negative lymph nodes.

Total RNA, purified from three separate normal human bladder tissue samples (FirstChoice^{®} Human Bladder Total RNA, cat. no. AM7990; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 303 miRNAs (142 verified, 161 predicted) with significant differential expression between normal bladder tissue samples and normal lymph nodes (log2Diff bladder vs LN neg ≥1 and p-value<0.05) (Table 19). Among the verified miRNAs, hsa-miR-205 and hsa-miR-187 had average expression levels that were more than 100-fold higher in bladder tissues; hsa-miR-429 and hsa-miR-133b had average expression levels that were more than 50-fold higher in bladder tissues; 21 miRNAs had average expression levels that were between 10- and 40-fold higher in bladder tissue samples; and 41 miRNAs had average expression levels that were between 5- and 10-fold higher in bladder tissue samples. Among the predicted miRNAs, hsa-asg-6845_st2 had an average expression level that was over 50-fold higher in bladder tissue samples; 11 miRNAs had average expression levels that were between 10- and 20- fold higher in bladder samples; and 43 miRNAs had average expression levels that were between 5- and 10-fold higher in bladder tissue samples..

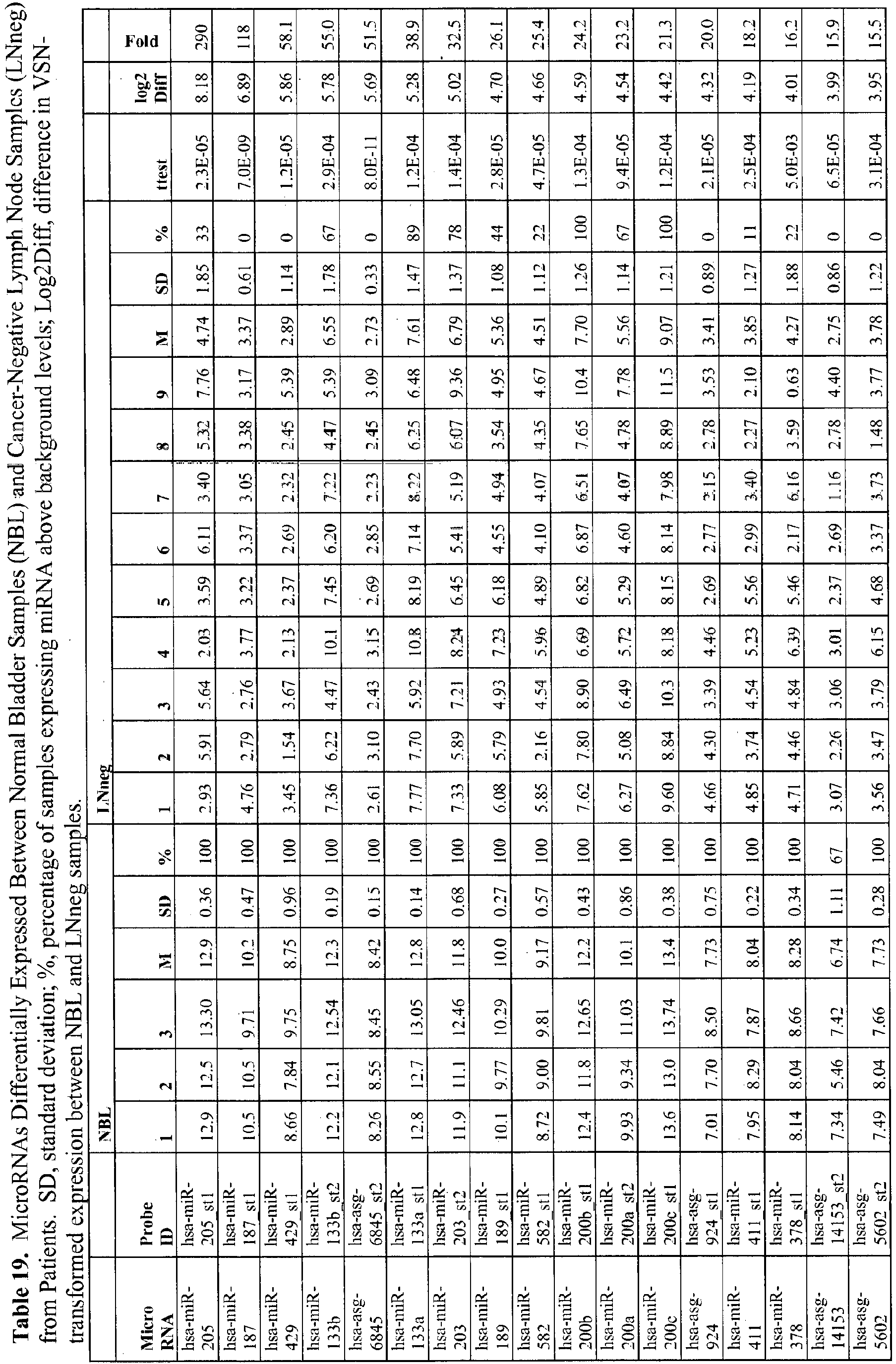

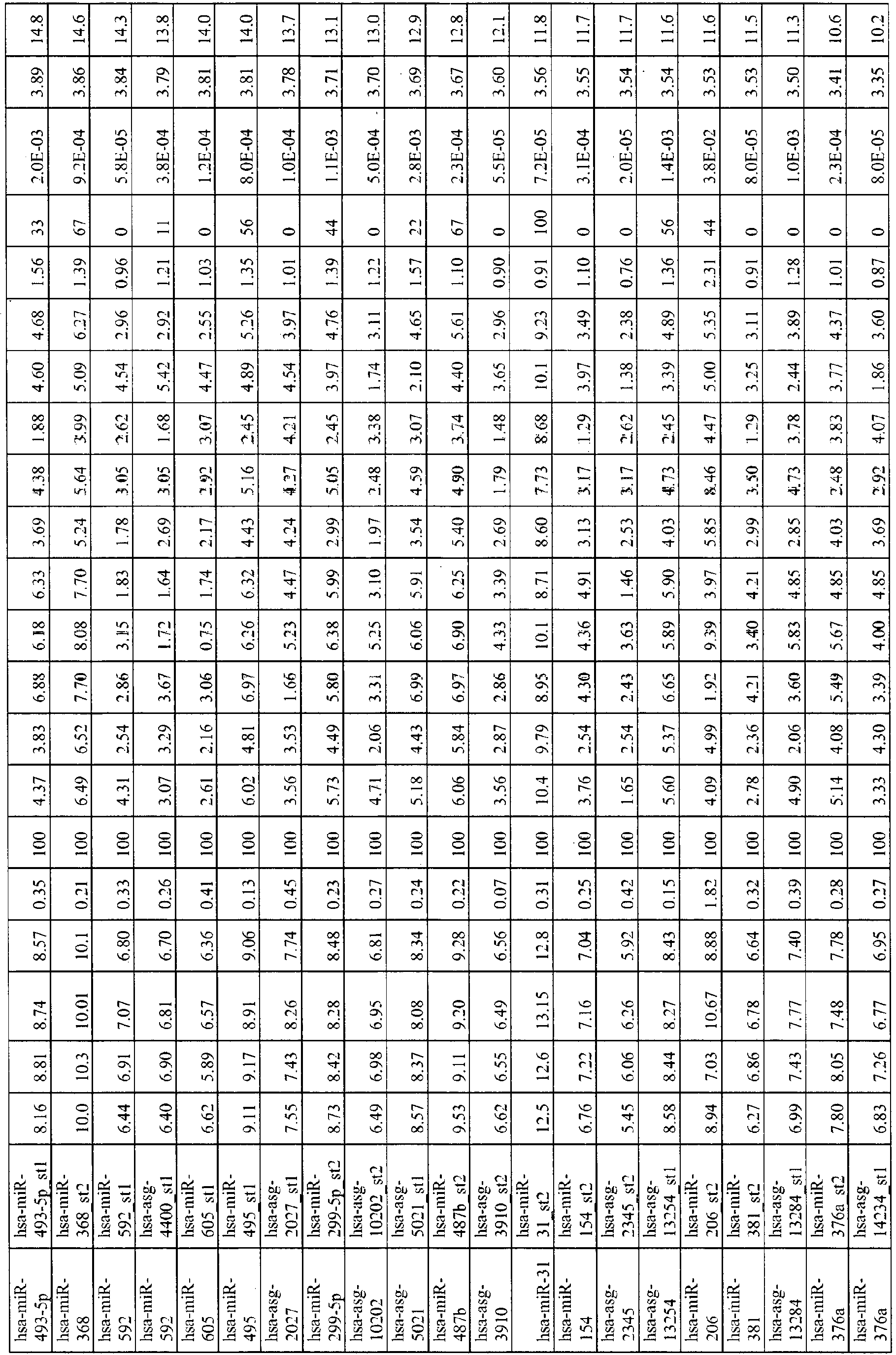

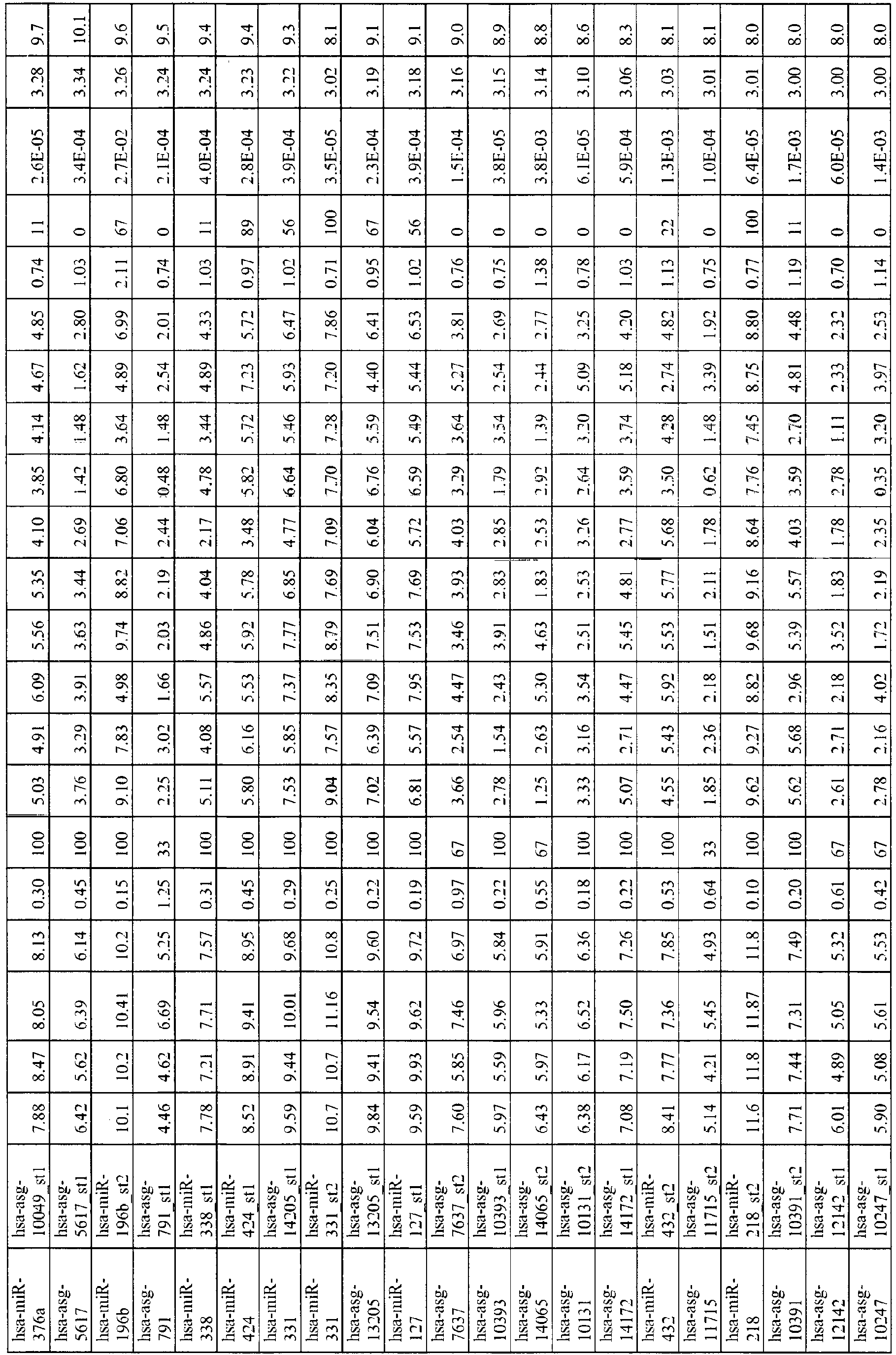

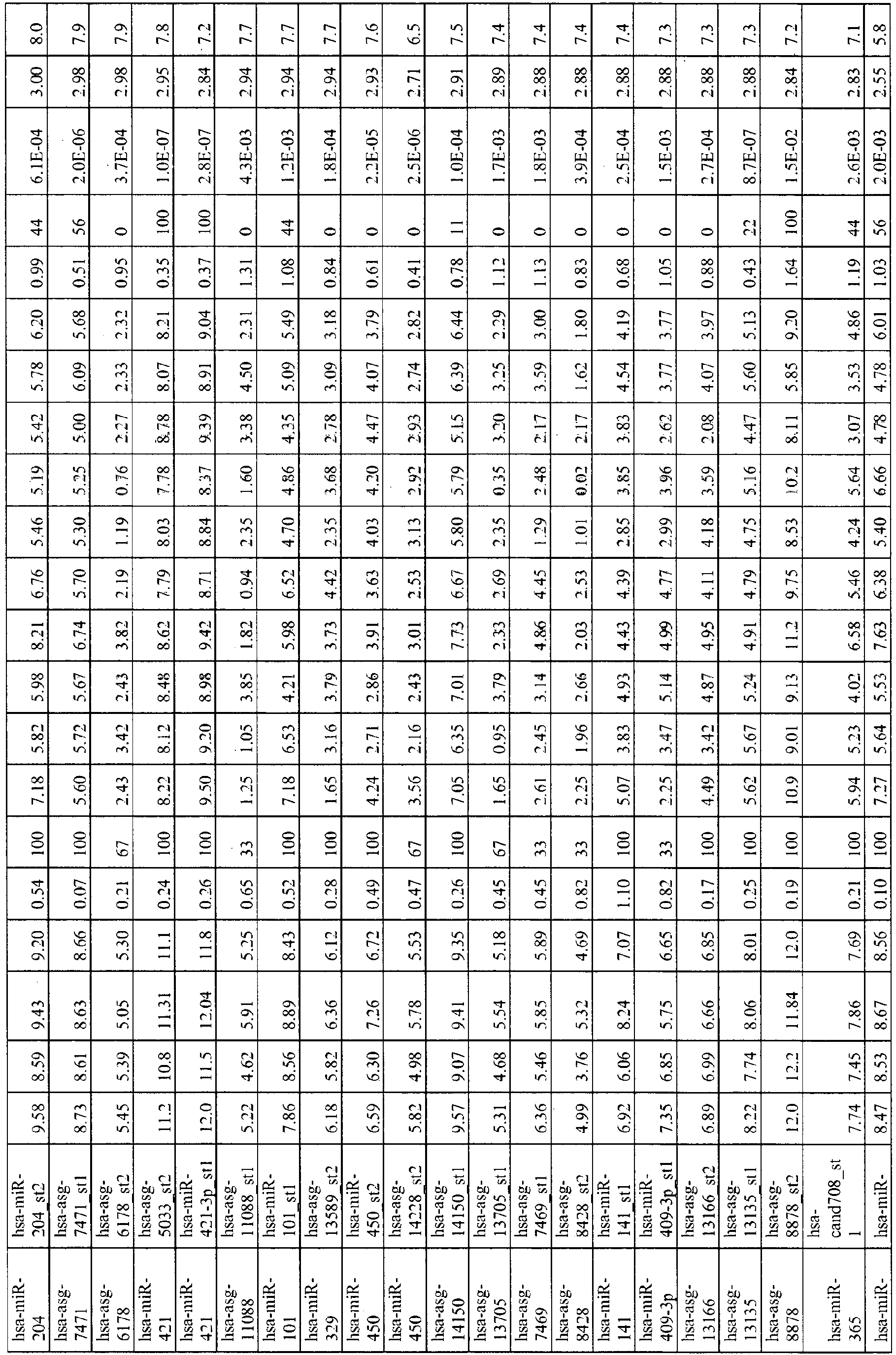

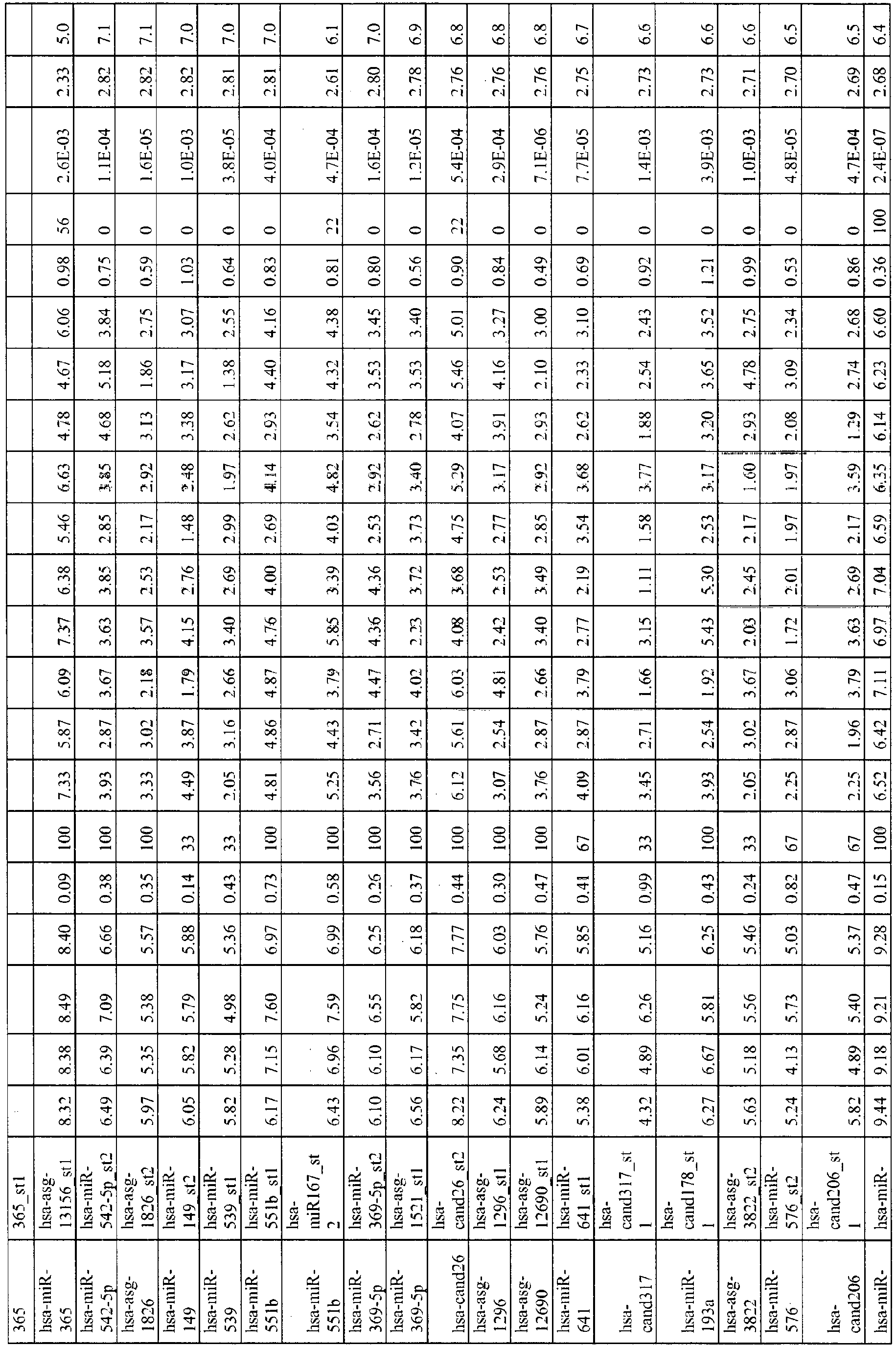

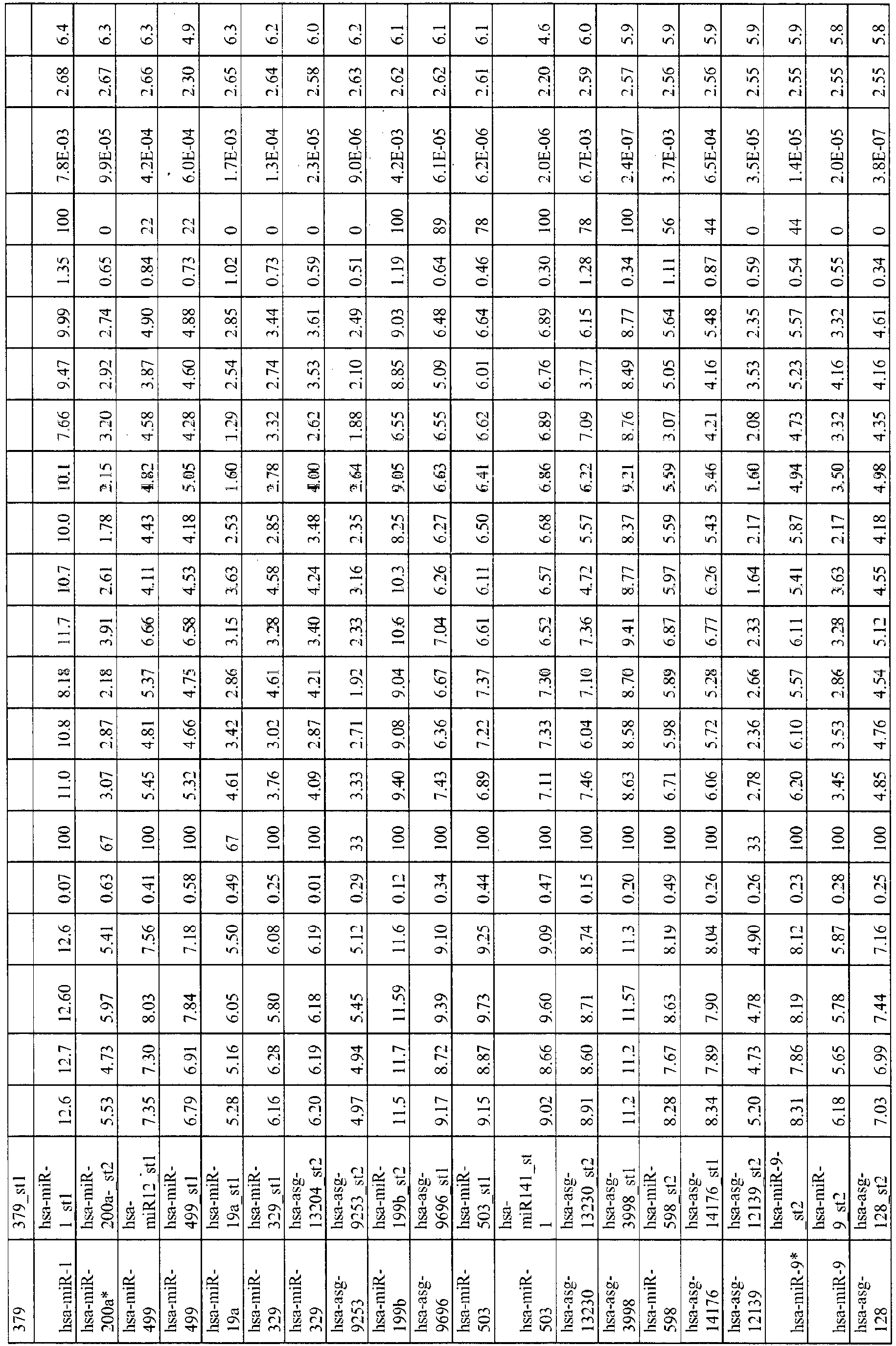

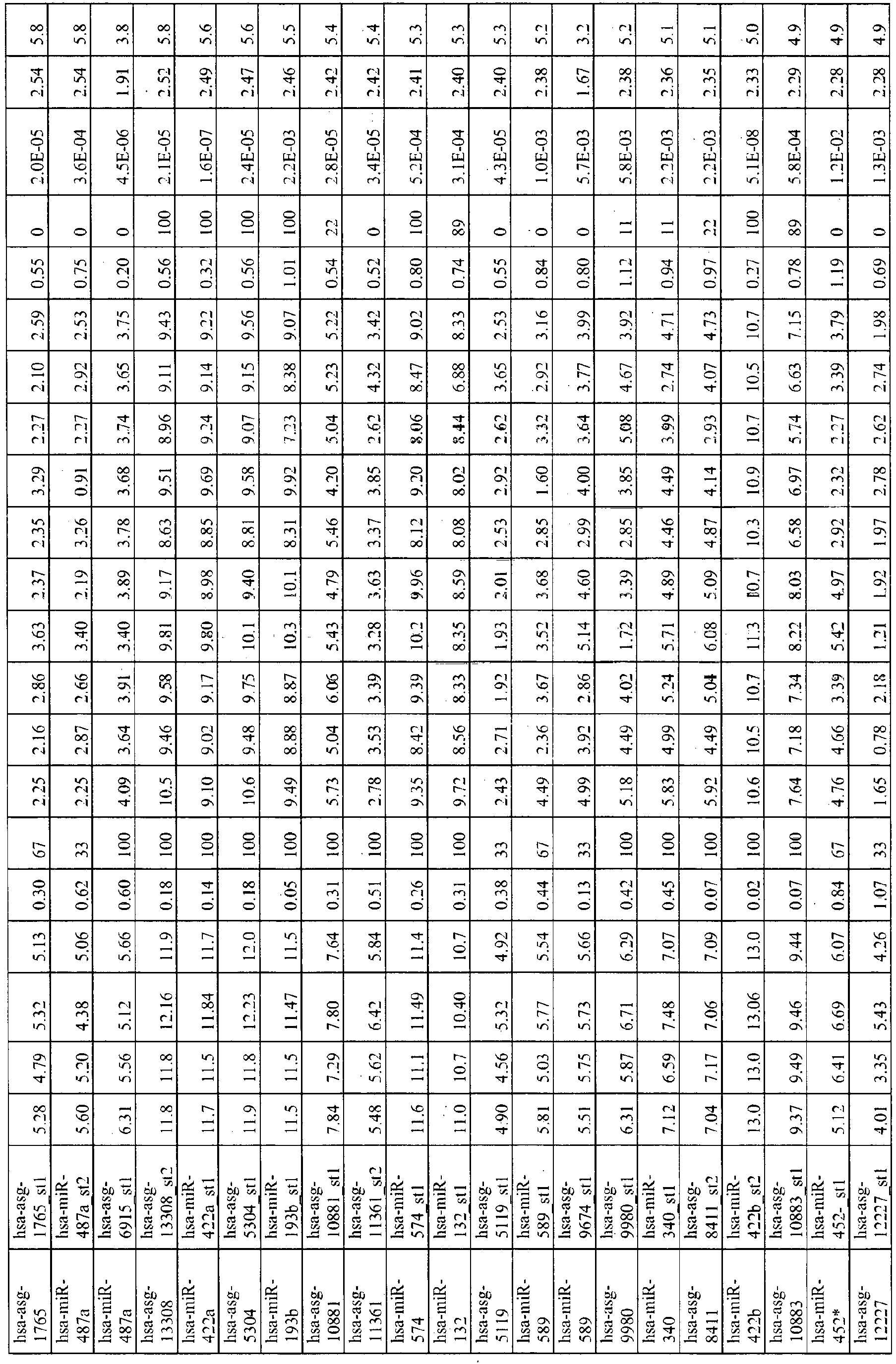

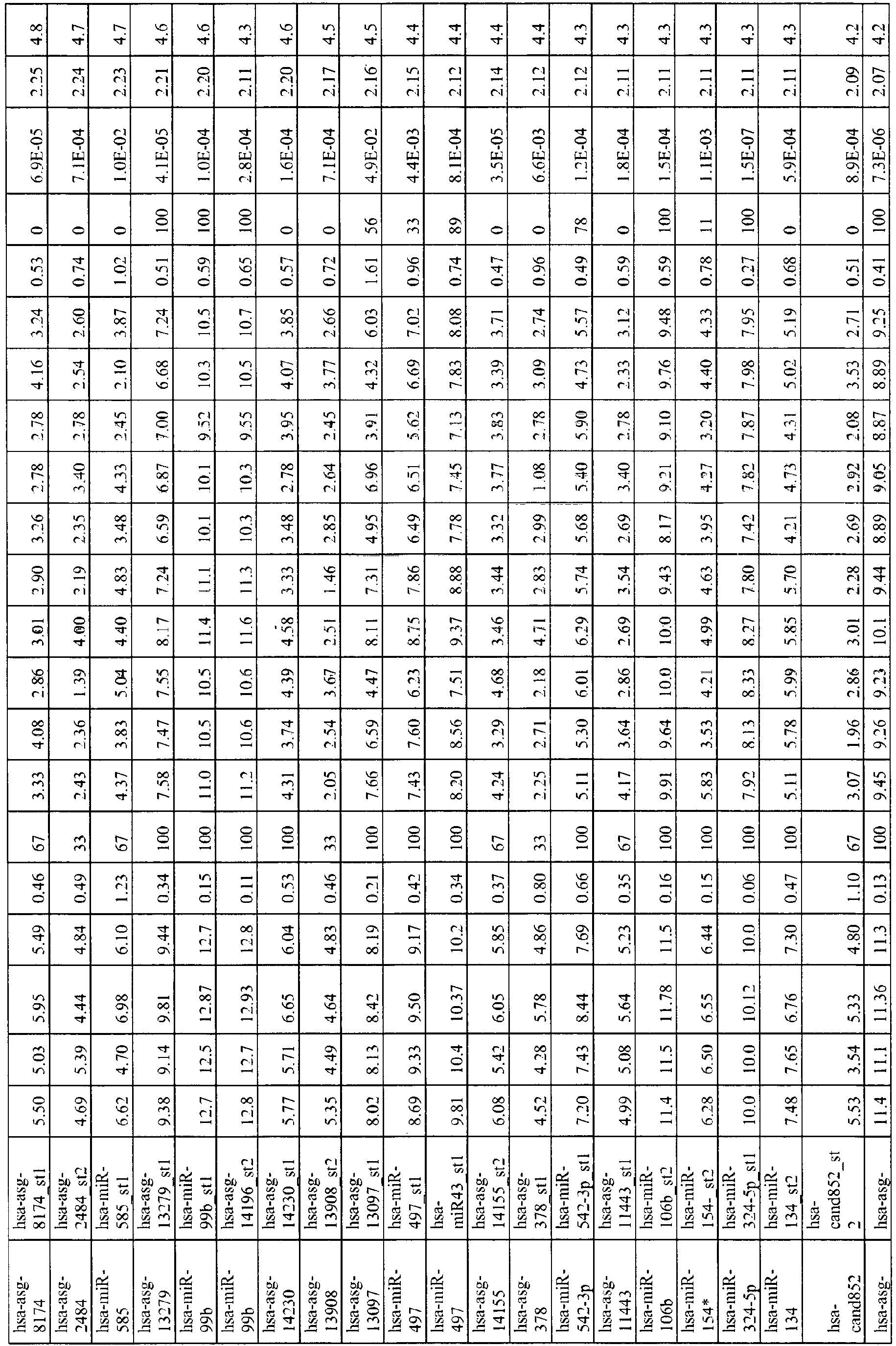

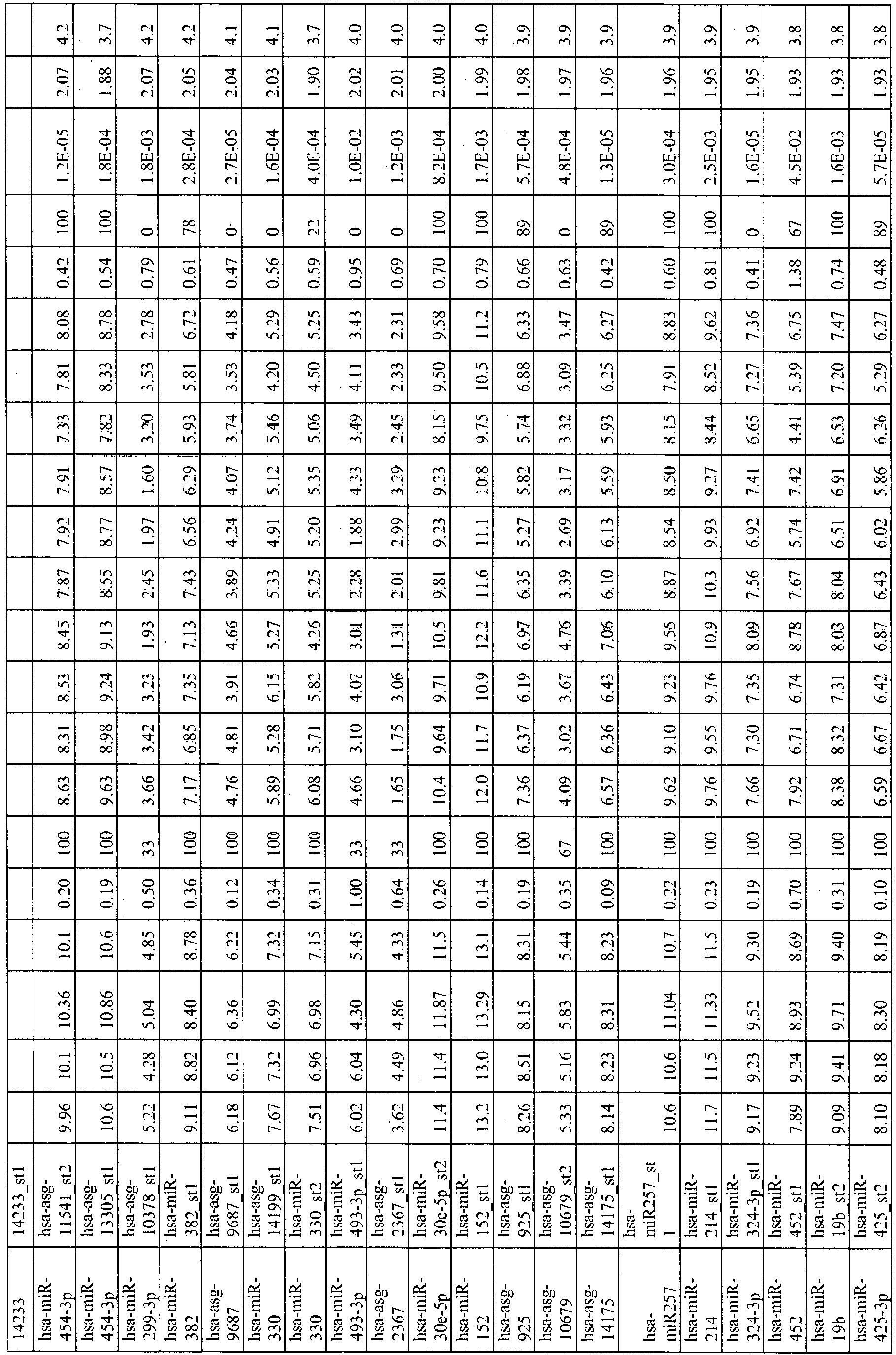

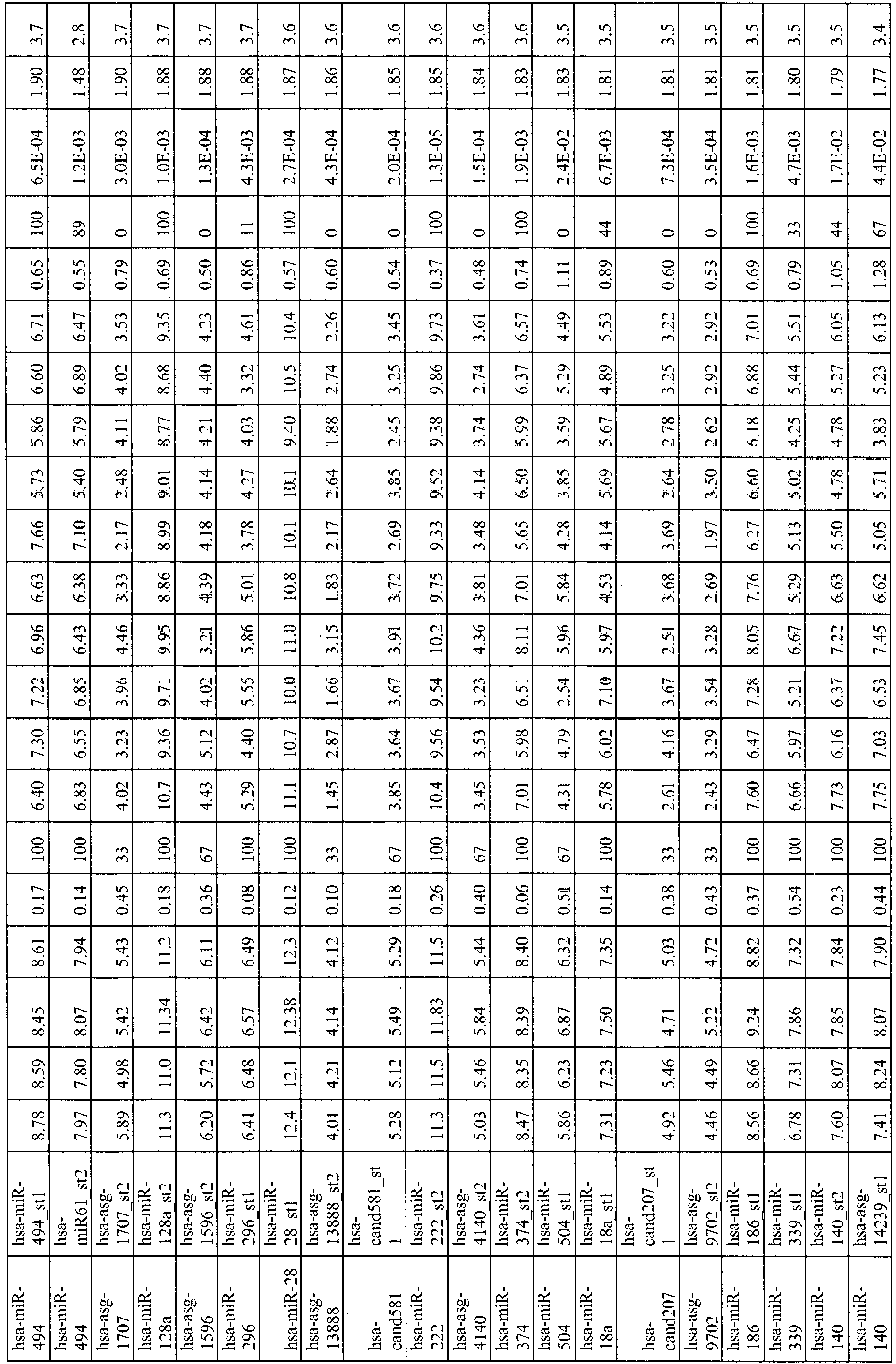

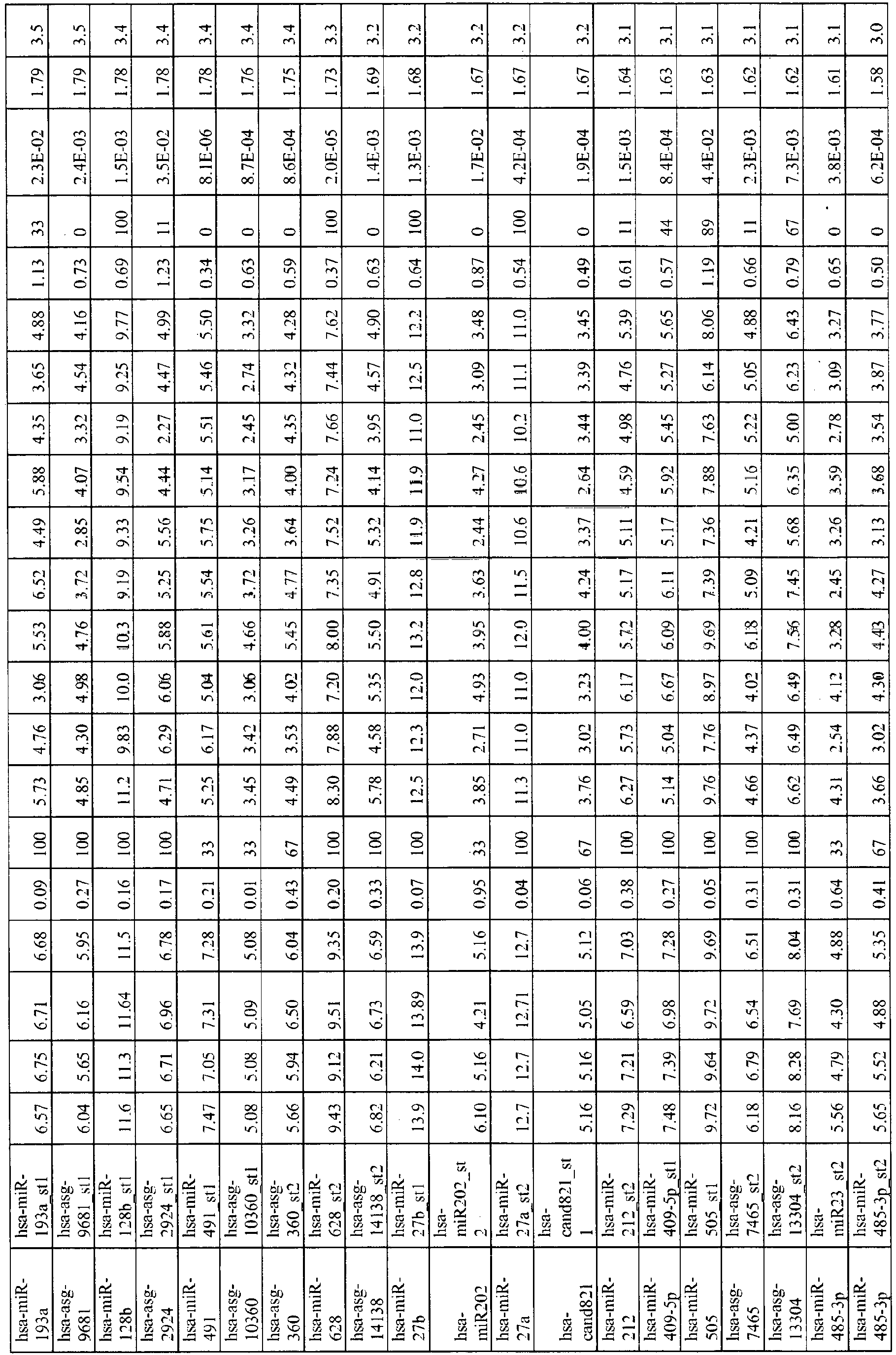

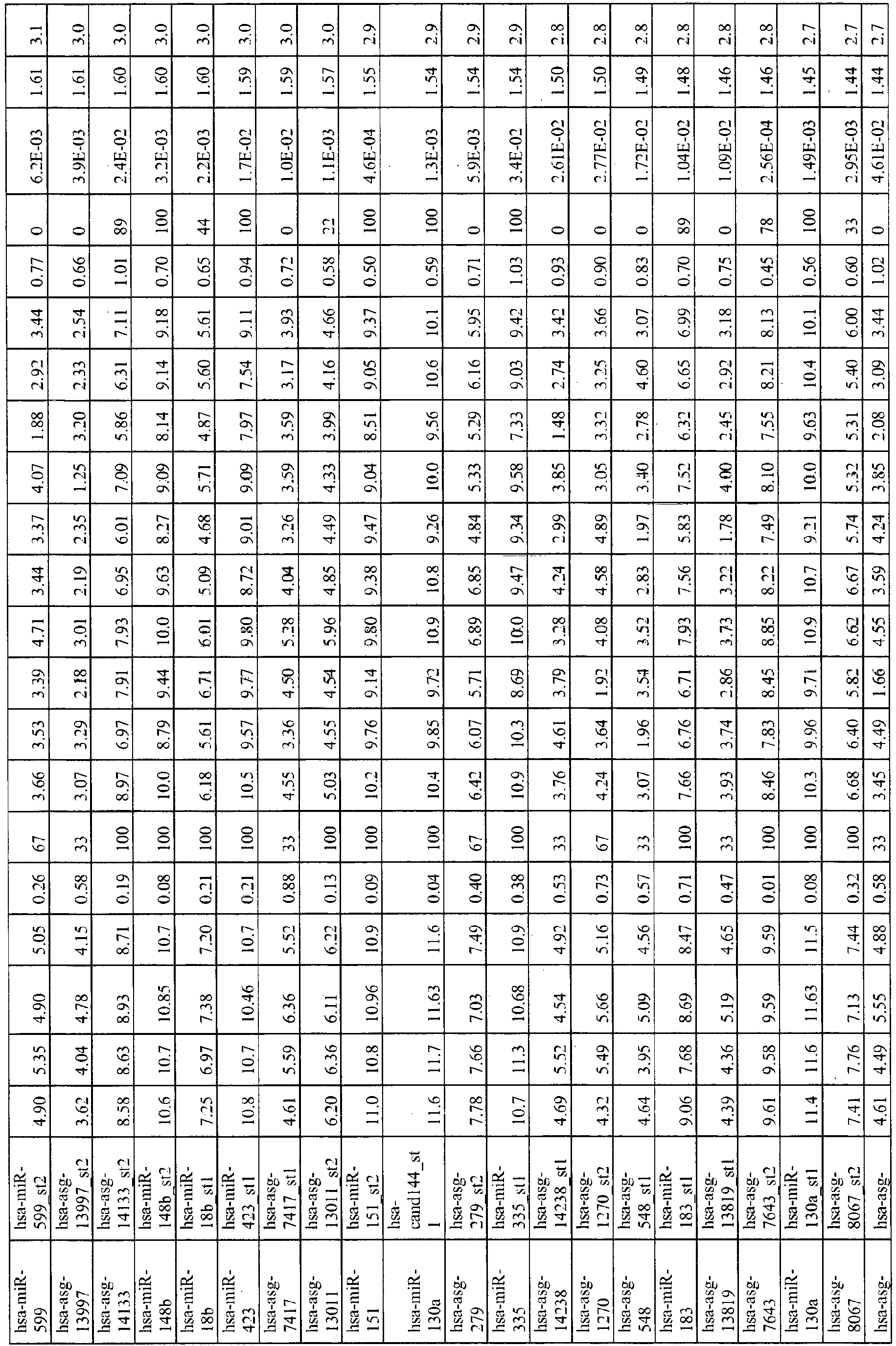

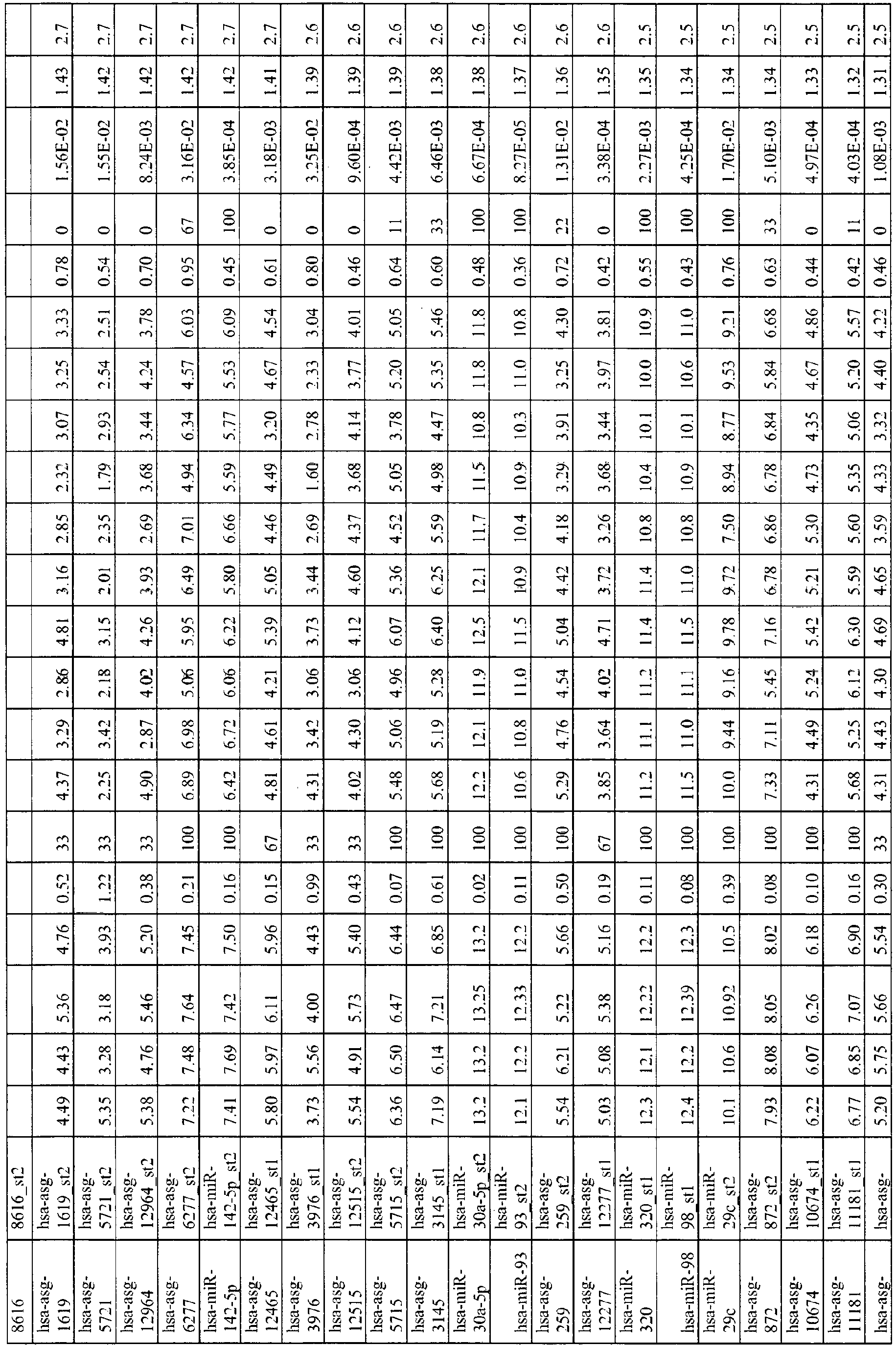

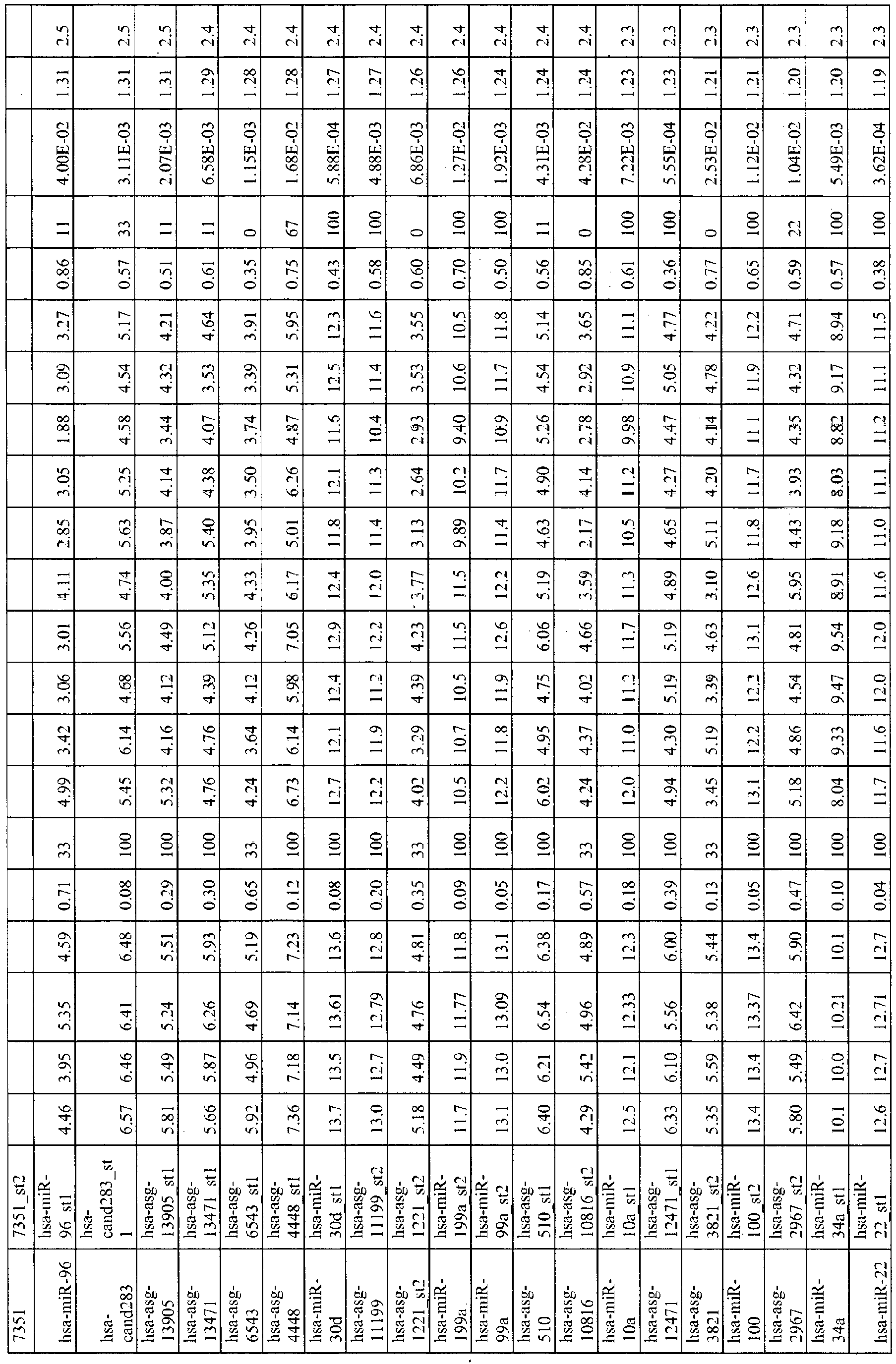

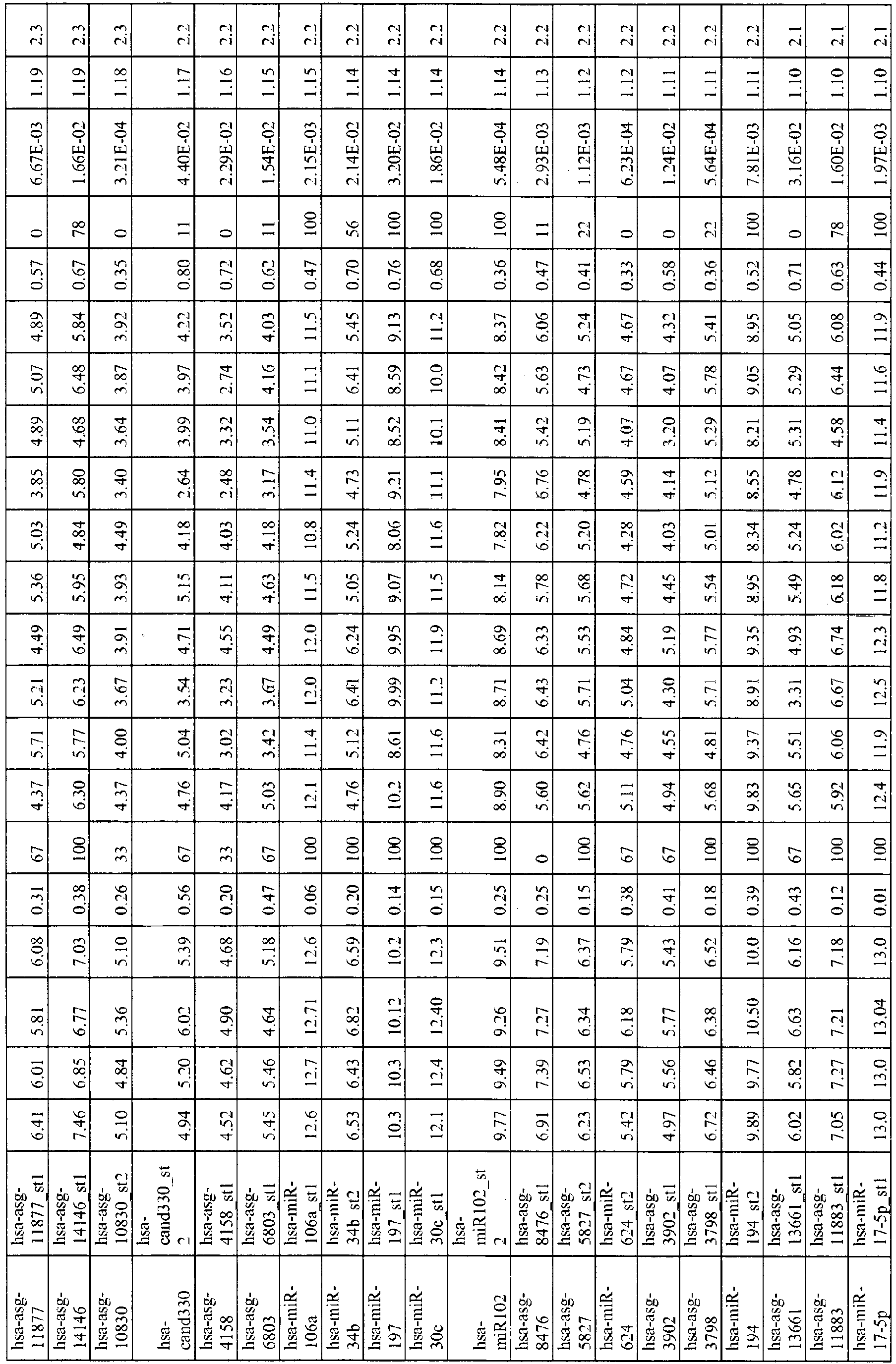

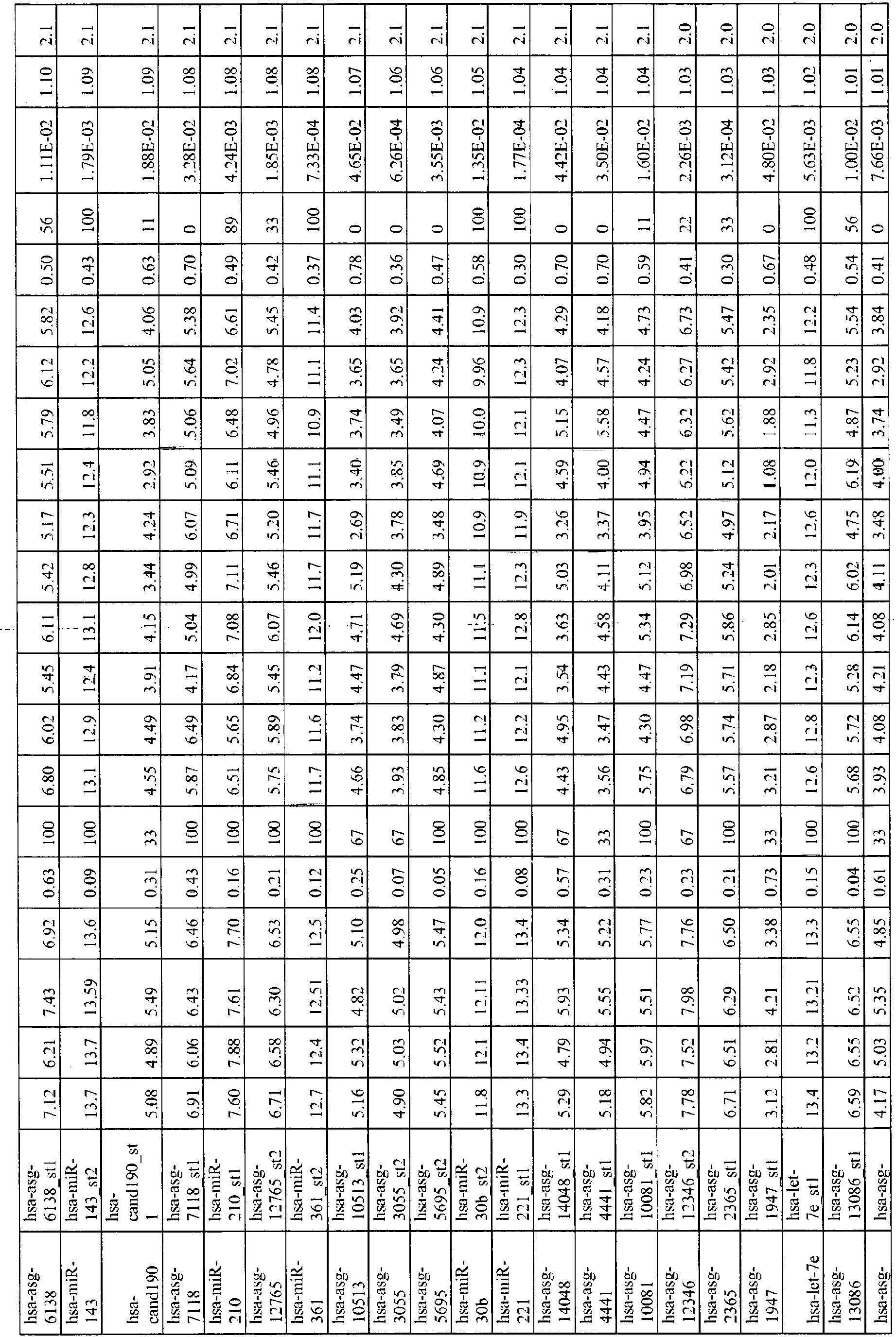

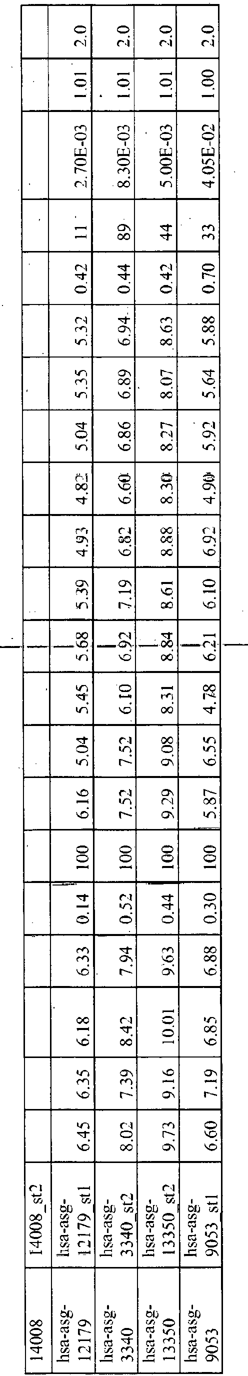

### EXAMPLE 21:

### MIRNAS DIFFERENTALLY EXPRESSED BETWEEN NORMAL ESOPHAGUS TISSUE AND NORMAL LYMPH NODES

To identify miRNAs that may be useful markers for metastasis of cancerous esophagus cells to lymph nodes, the inventors identified miRNAs that are expressed at significantly higher levels in normal esophagus tissue than in cancer-negative lymph nodes.

Total RNA, purified from three separate normal human esophagus tissue samples (FirstChoice^{®} Human Esophagus Total RNA, cat. no. AM6842; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 238 miRNAs (93 verified, 145 predicted) with significant differential expression between normal esophagus tissue samples and normal lymph nodes (log2Diff esophagus vs LN neg ≥1 and p-value<0.05) (Table 20). Among the verified miRNAs, hsa-miR-205, -206 and -187 had average expression levels that were more than 100-fold higher in esophagus samples than in normal lymph node samples; hsa-miR-203, - 133b, and -429 had average expression levels that were more than 50-fold higher in esophagus samples, 10 additional miRNA had average expression levels that were between 10- and 50-fold higher in esophagus samples; and 22 other miRNAs had average expression levels that were between 5- and 10-fold higher in esophagus samples. Among the predicted miRNAs, hsa-asg-8428_st2 and -14153_st2 had average expression levels that were over 20-fold higher in esophagus samples than in normal lymph nodes. Seven miRNAs had average expression levels that were between 10- and 20-fold higher in esophagus compared to lymph node samples.

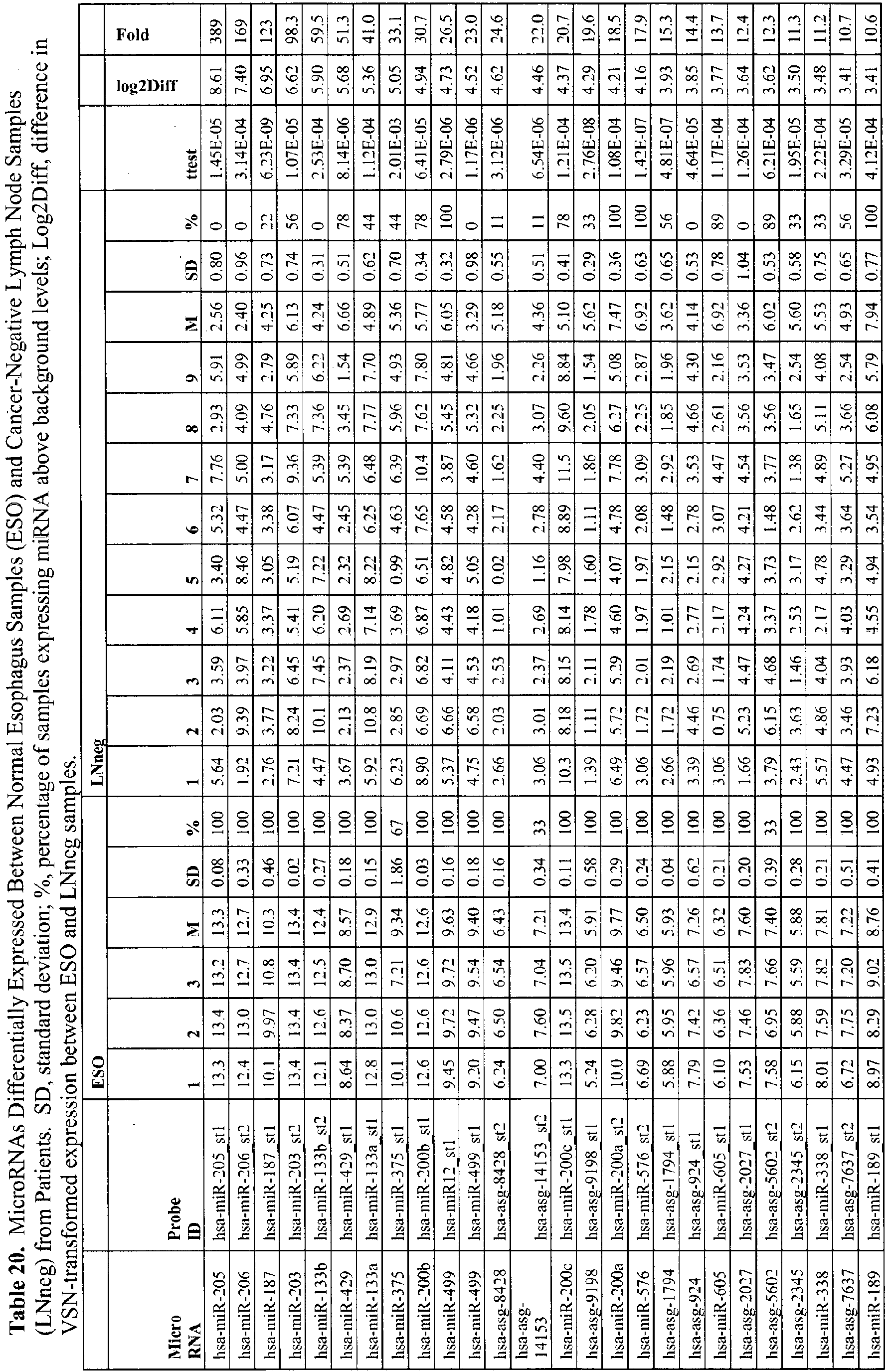

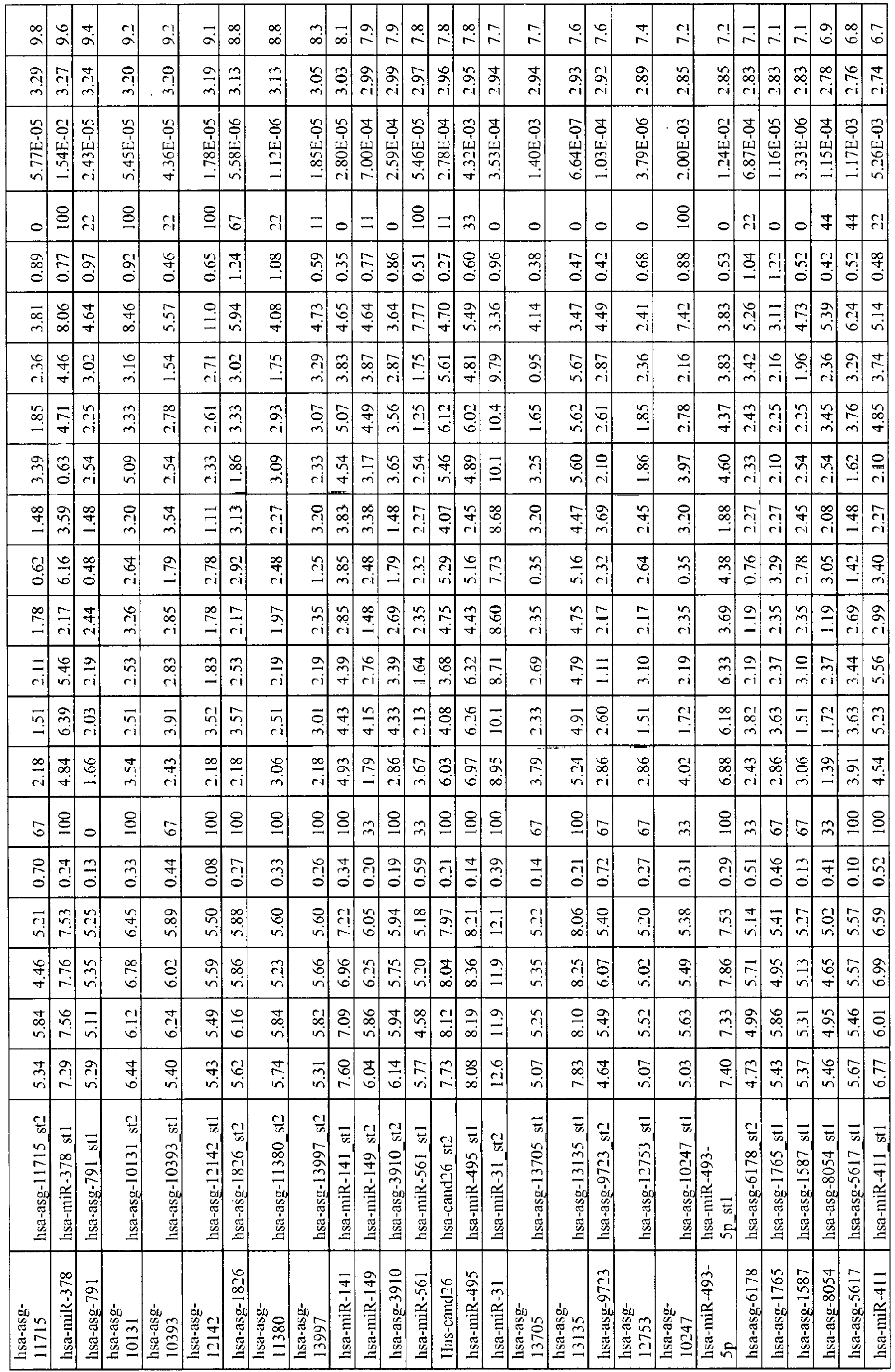

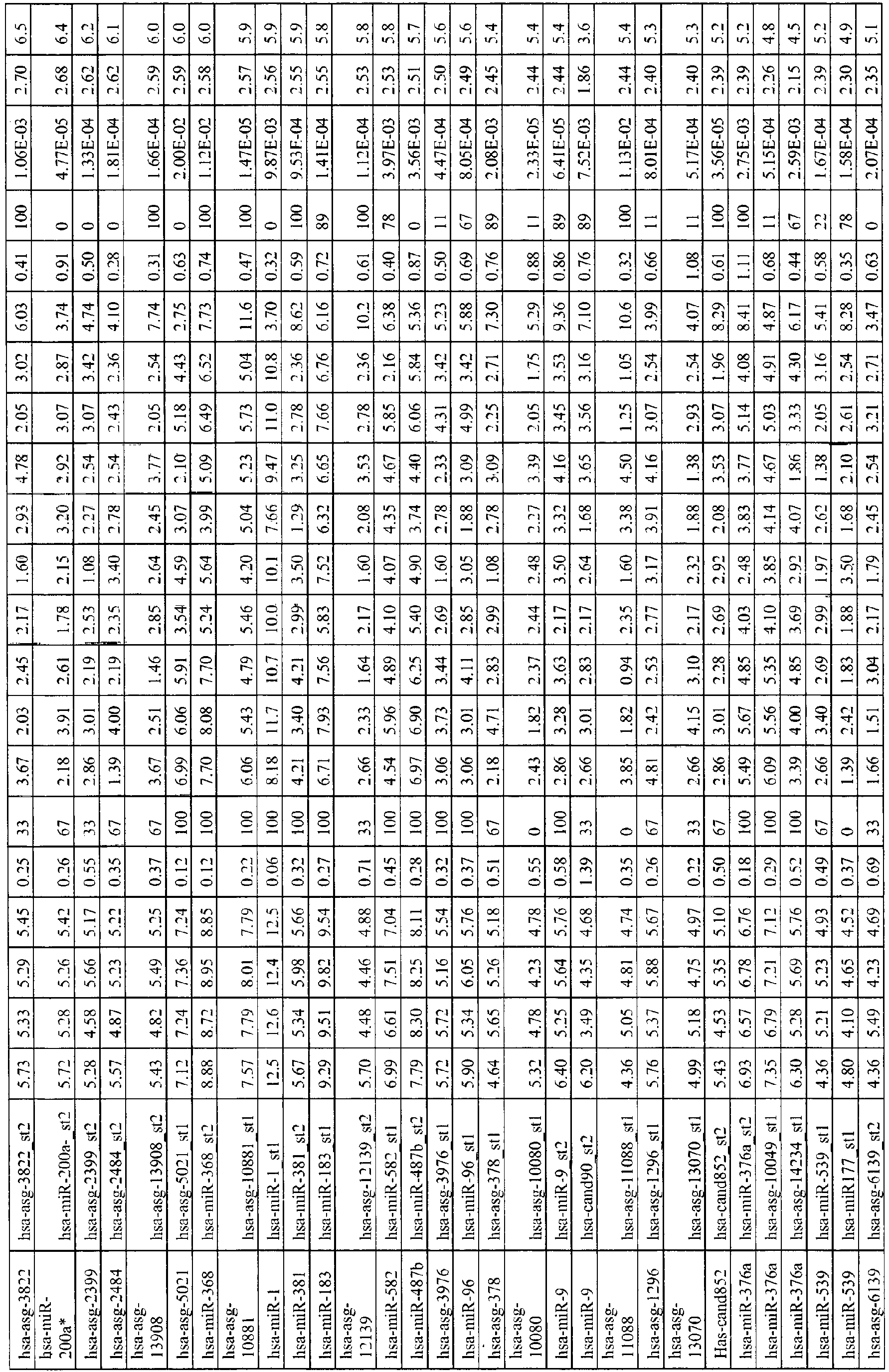

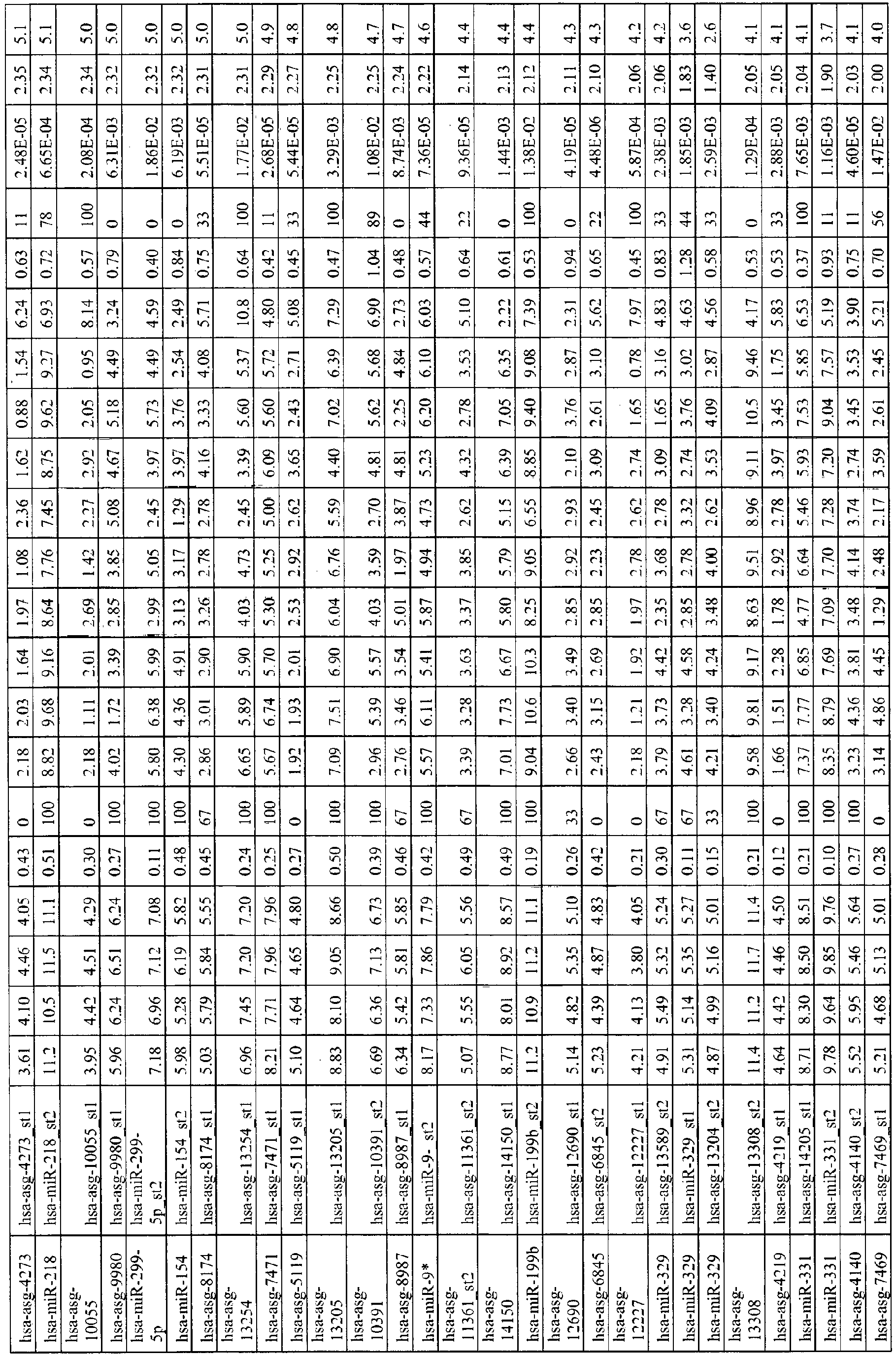

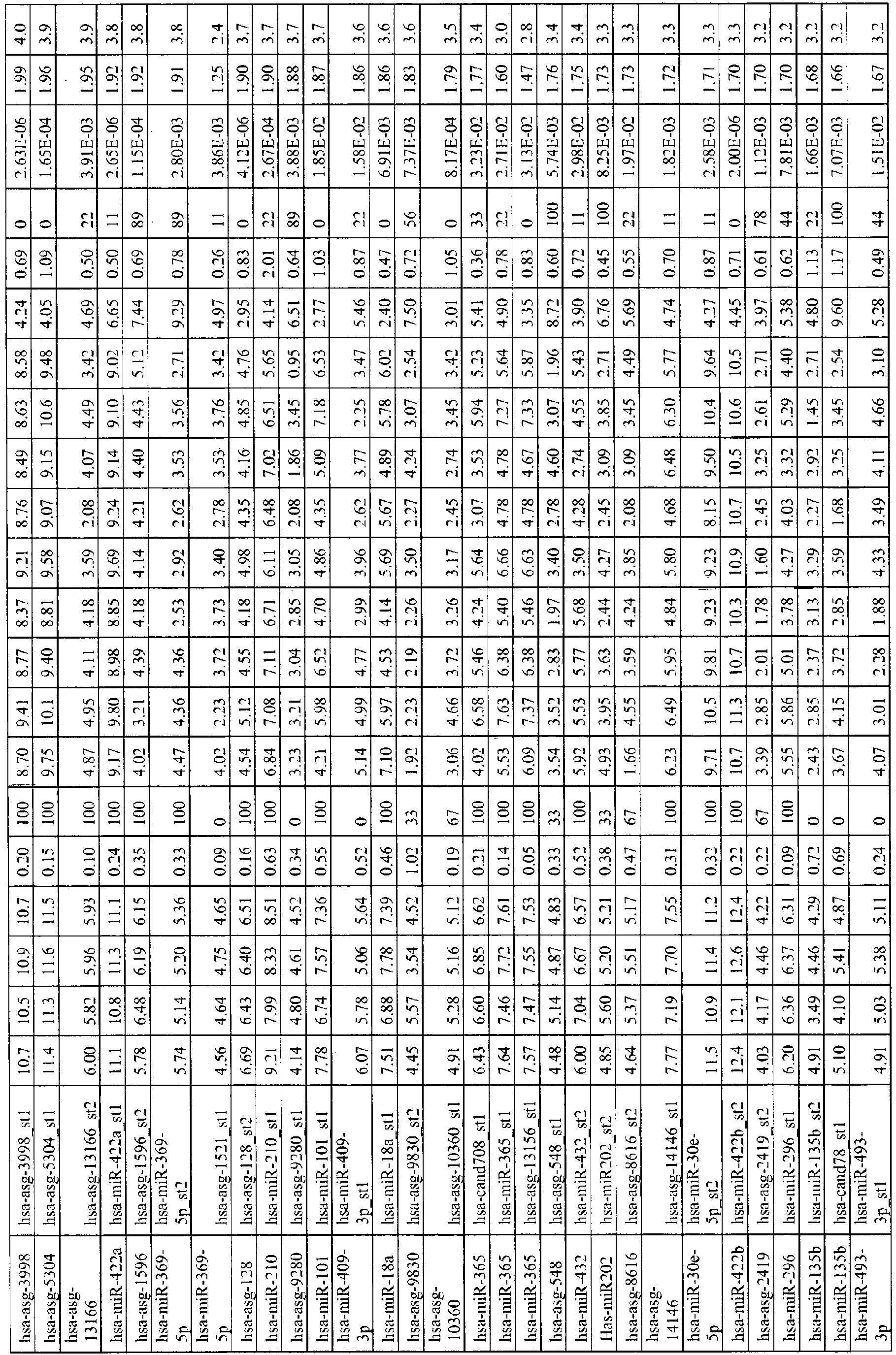

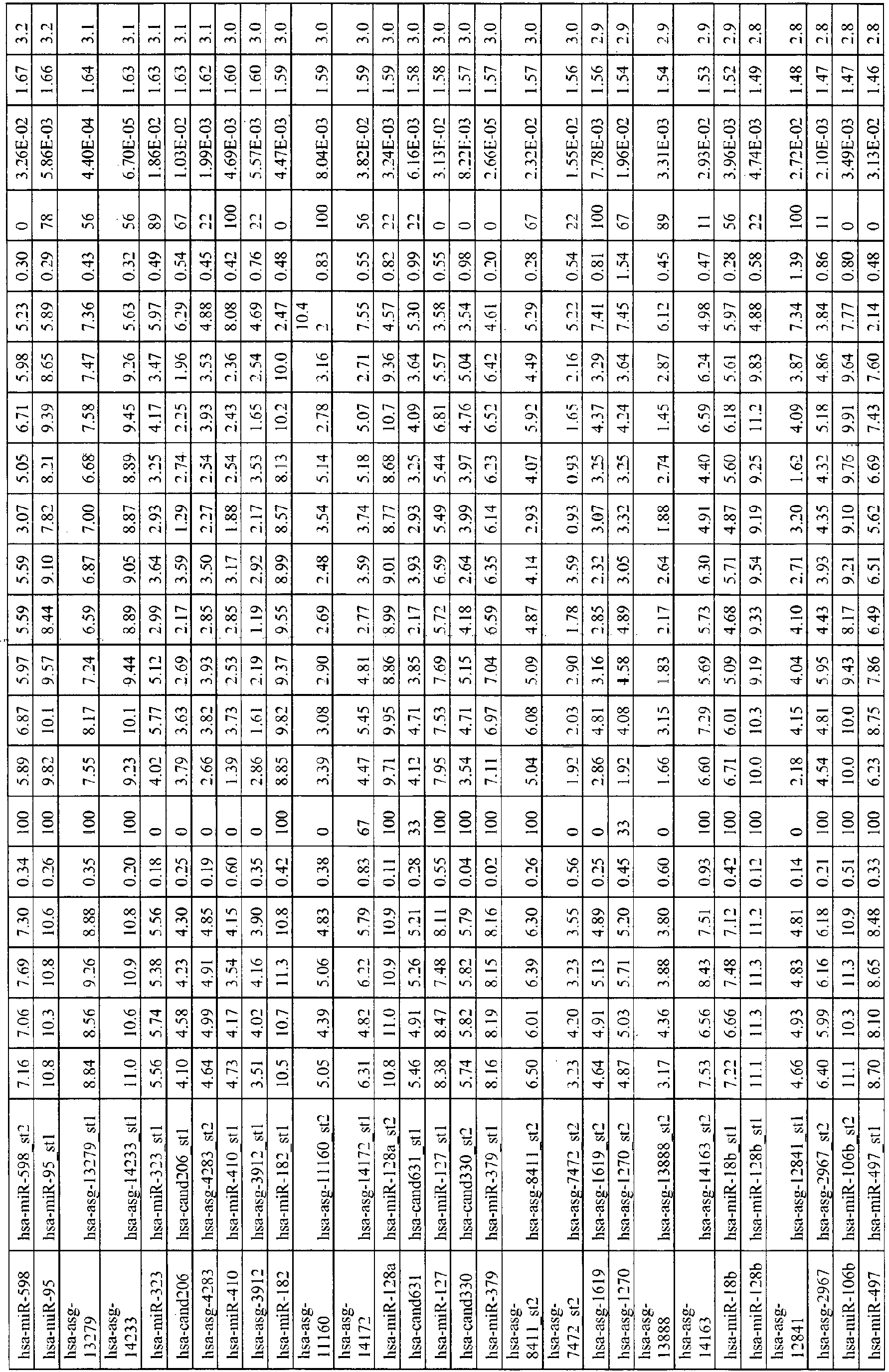

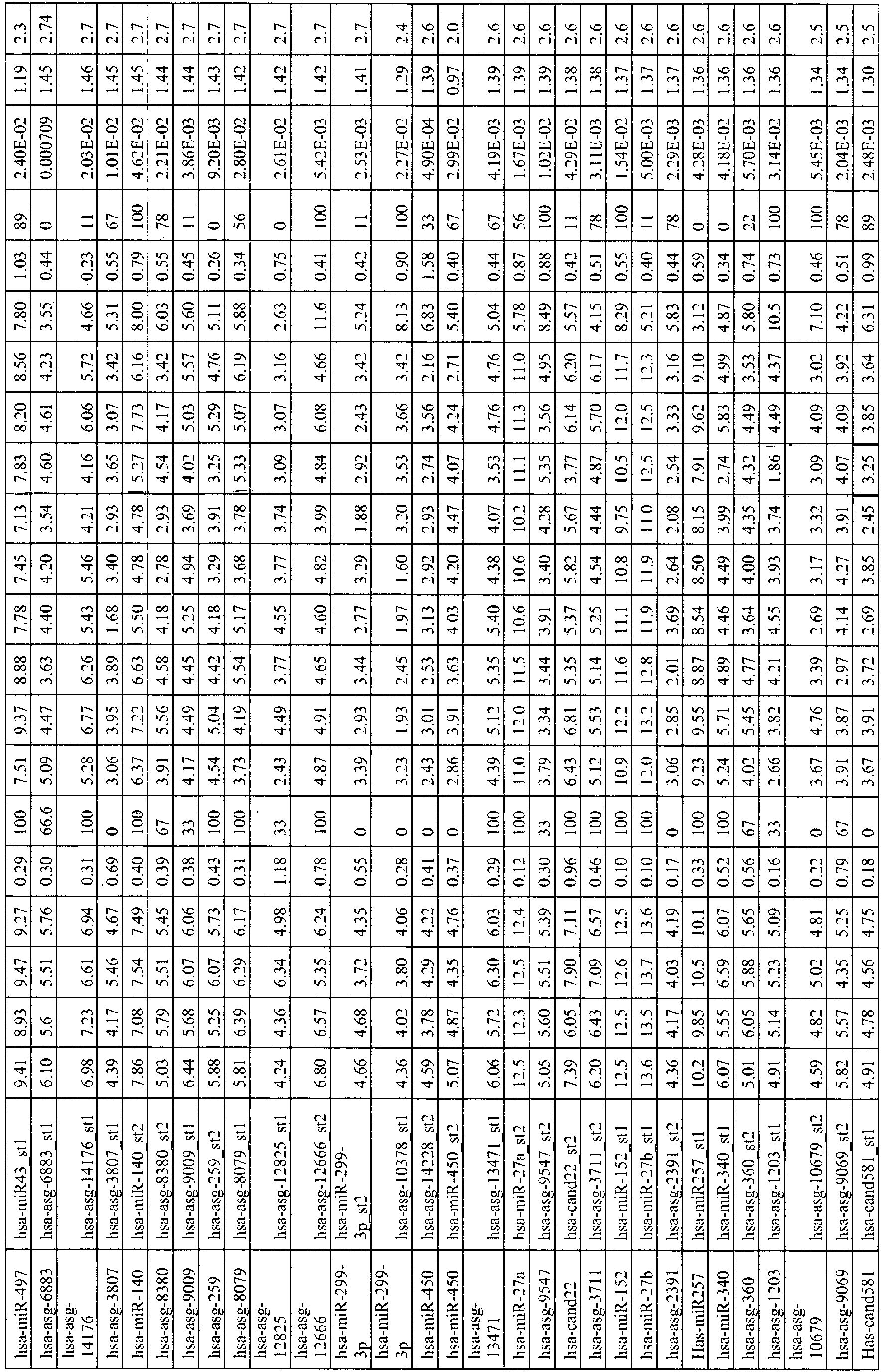

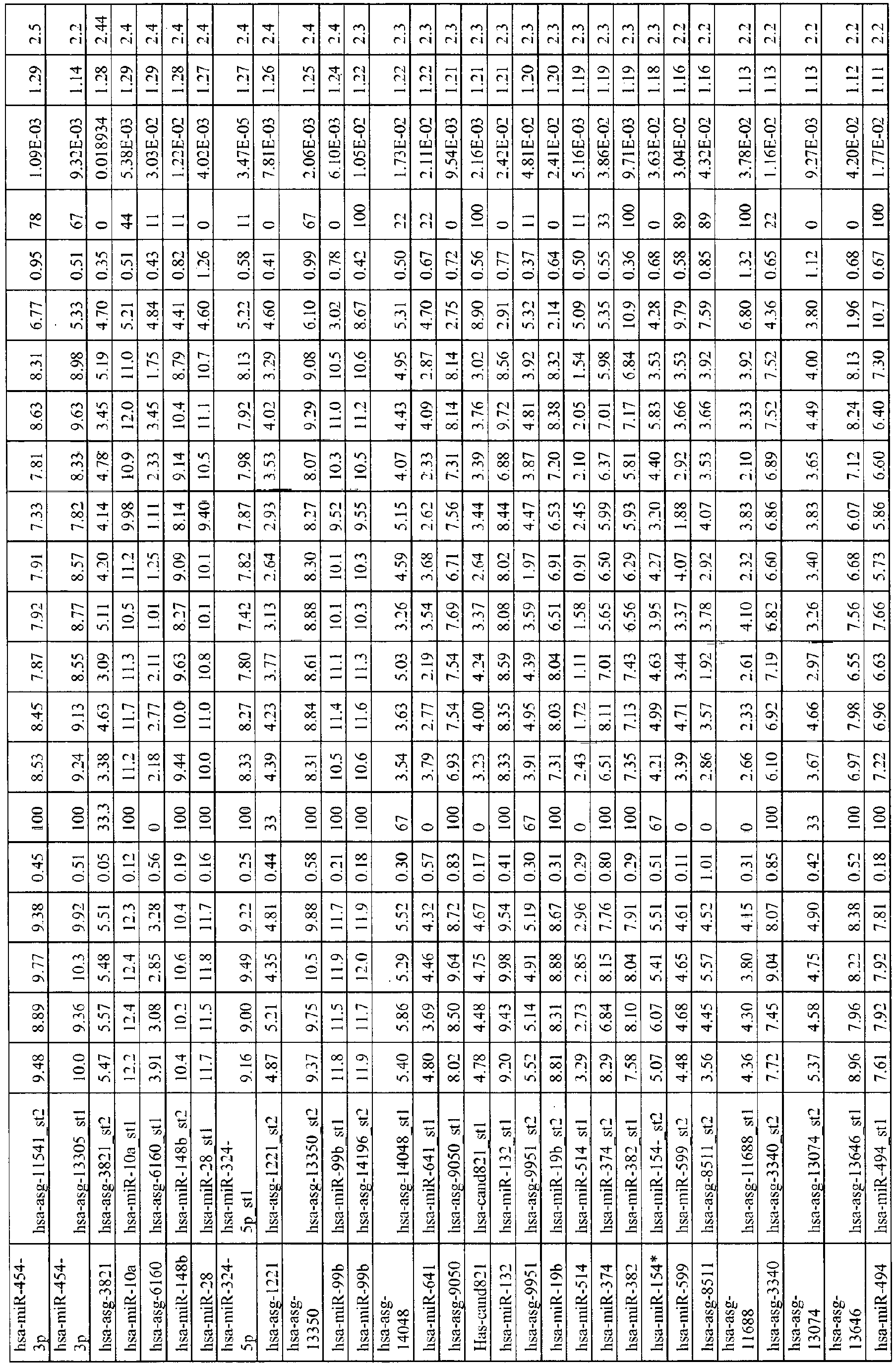

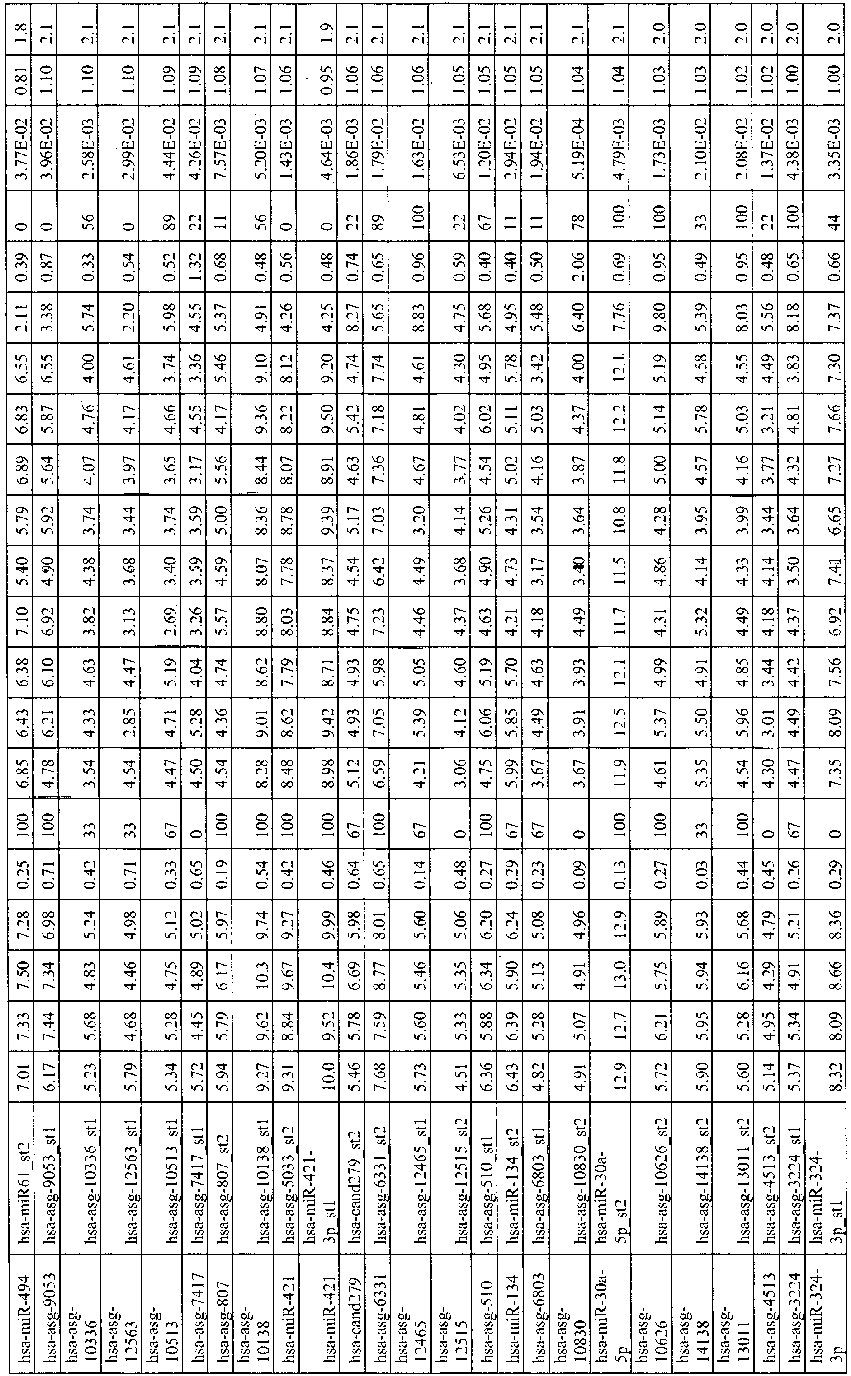

### EXAMPLE 22:

### MIRNAS_DIFFERENTALLY EXPRESSED BETWEEN NORMAL LIVER TISSUE AND NORMAL LYMPH NODES

To identify miRNAs that may be useful markers for metastasis of cancerous liver cells to lymph nodes, the inventors identified miRNAs that are expressed at significantly higher levels in normal liver tissue than in cancer-negative lymph nodes.

Total RNA, purified from three separate normal human liver tissue samples (FirstChoice^{®} Human Liver Total RNA, cat. no. AM7960; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 198 miRNAs (51 verified, 147 predicted) with significant differential expression between normal liver and normal lymph node samples (log2Diff liver vs LN neg ≥1 and p-value<0.05) (Table 21). Among the verified miRNAs, hsa-miR-122a had an average expression level over 300-fold higher in liver samples; hsa-miR-375 had an average expression level over 50-fold higher in liver samples; four miRNAs (hsa-miR-215, - 194, -483, and -192) had average expression levels that were between 15- and 25-fold higher in liver samples; and nine miRNAs (hsa-miR-200b, -592, -429, -139, -203, -200a, -19a, - 148a, and -493-5p) had average expression levels that were between 5- and 10-fold higher in liver samples. Among the predicted miRNAs, hsa-asg-14182_st1 had an average expression level over 40-fold higher in liver samples; eight miRNAs (hsa-asg-1035_st2, -9663_st1, - 11391_st1, -3922_st2, -2303_st1, -14208_st1, -11425_st1, -and -8128_st1) had average expression levels that were between 10- and 30- fold higher in liver samples; and 30 additional miRNAs had average expression levels that were between 5- and 10-fold higher in liver samples than in normal lymph node samples.

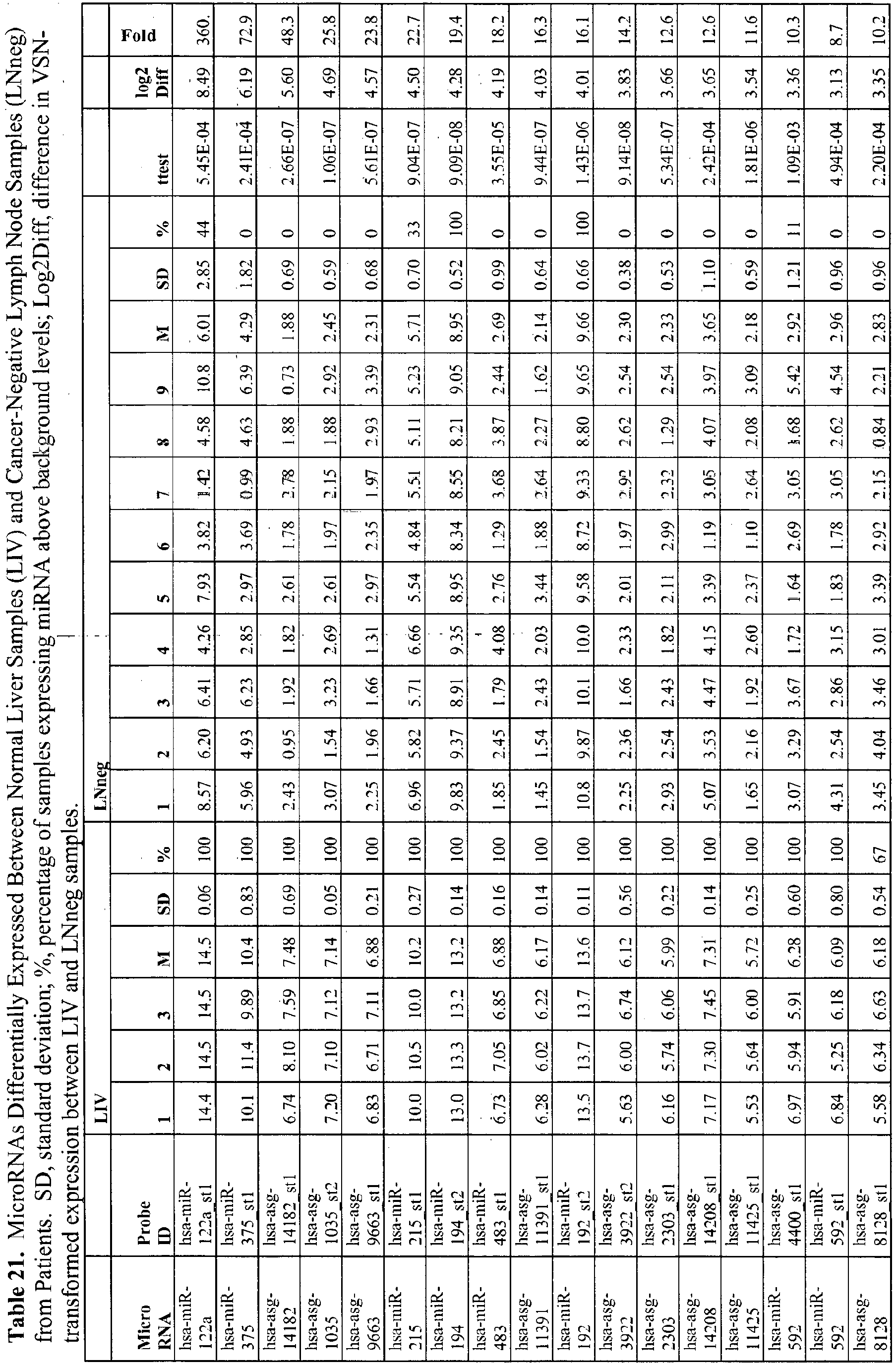

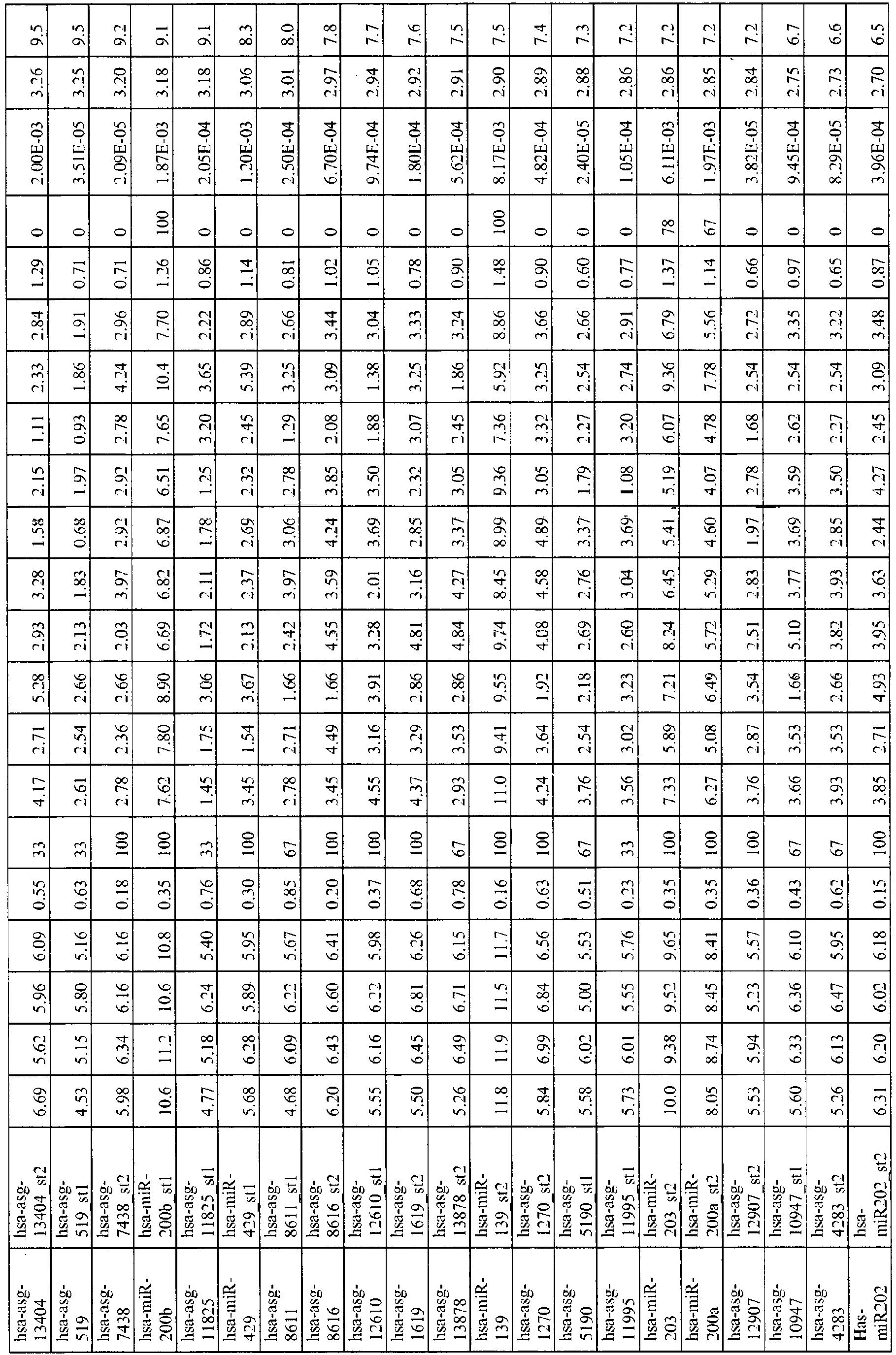

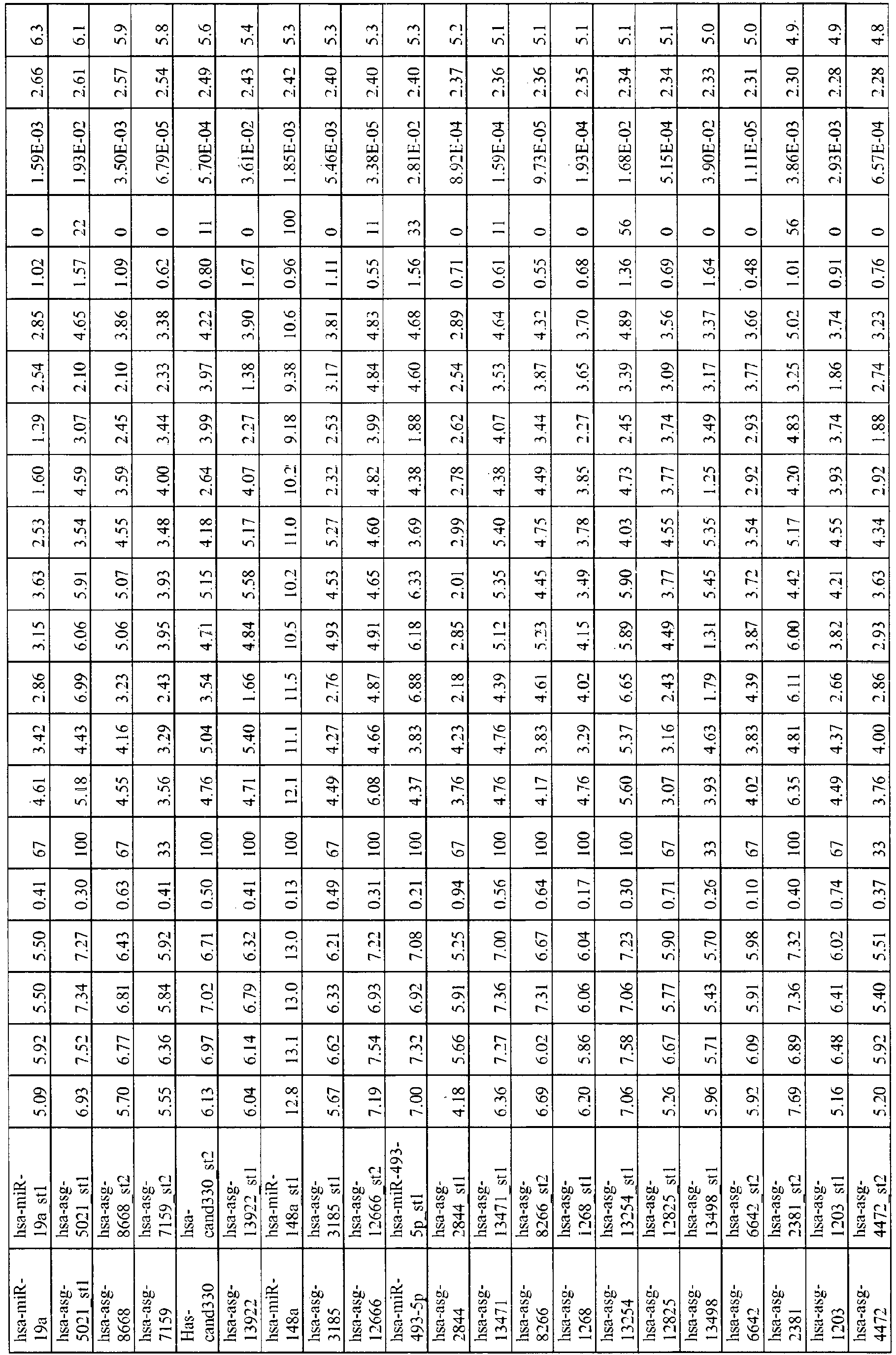

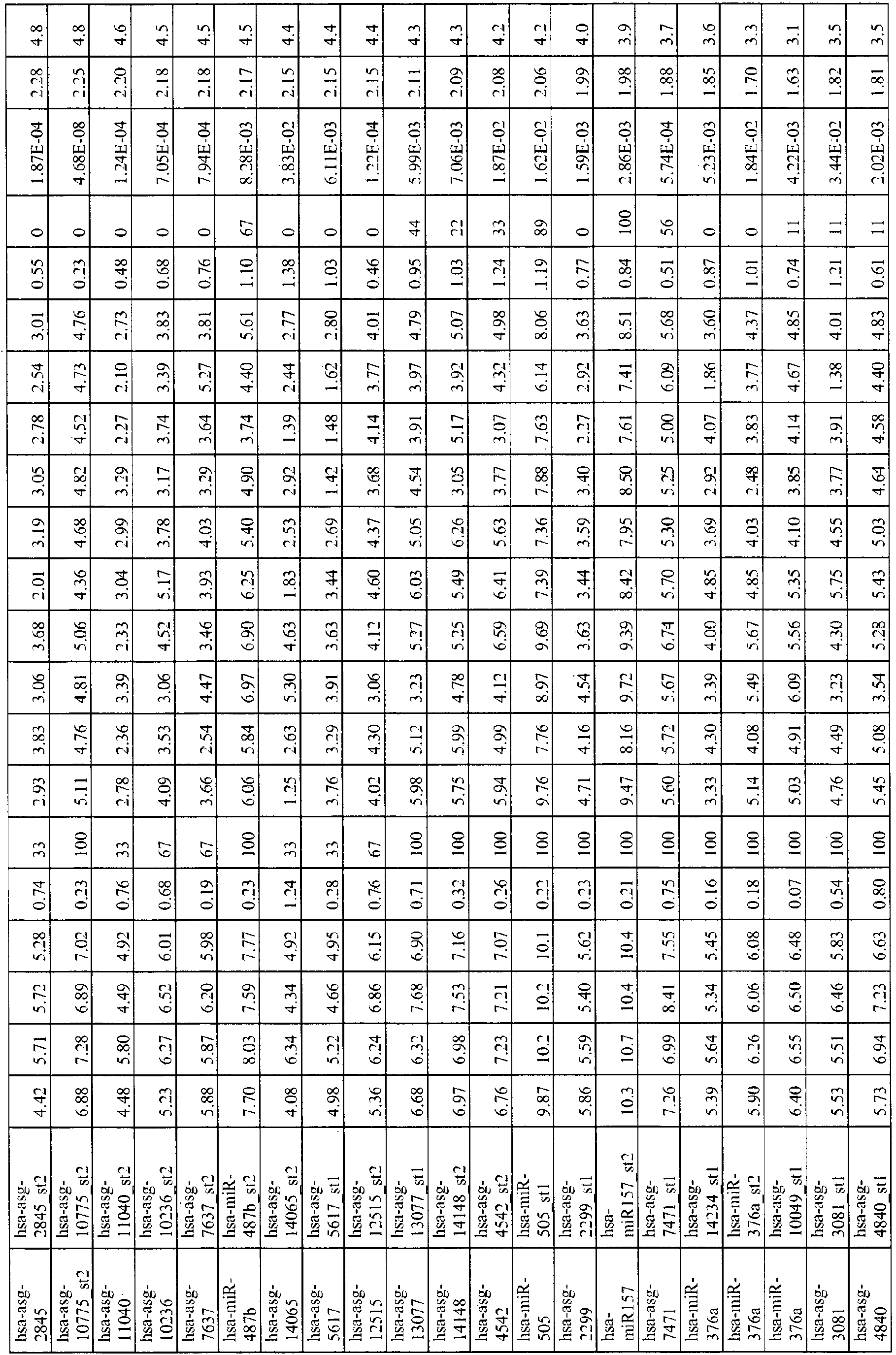

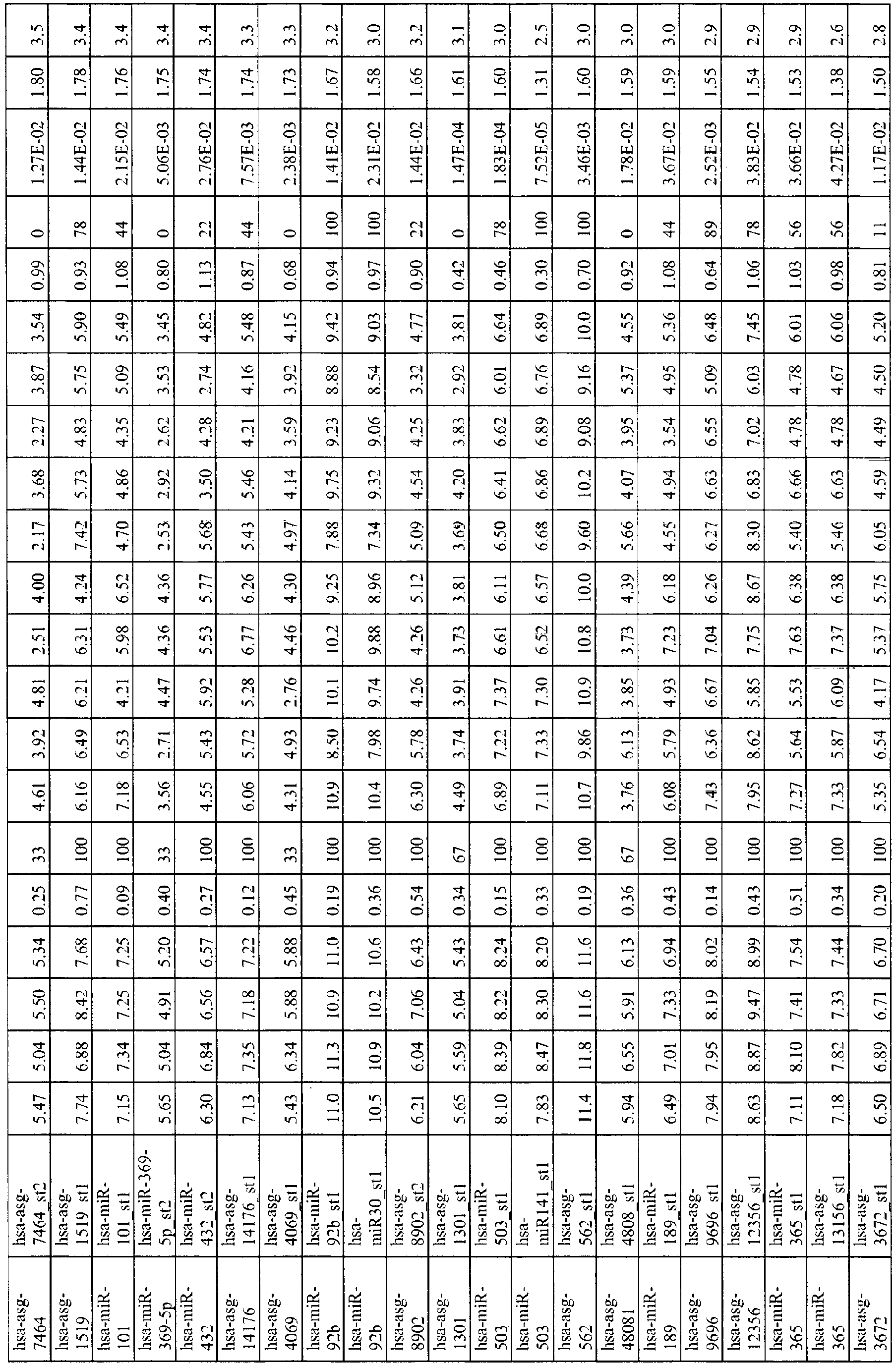

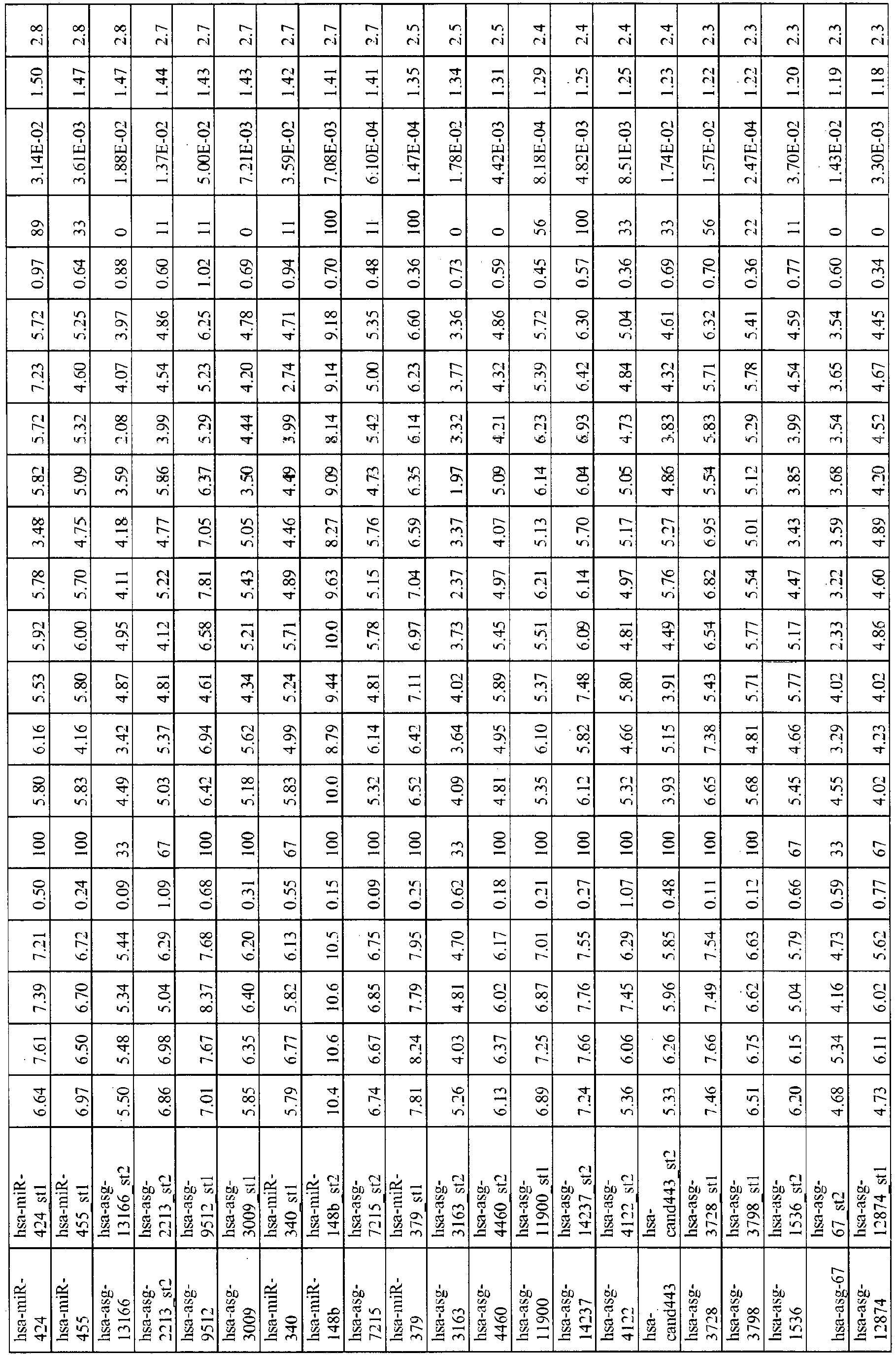

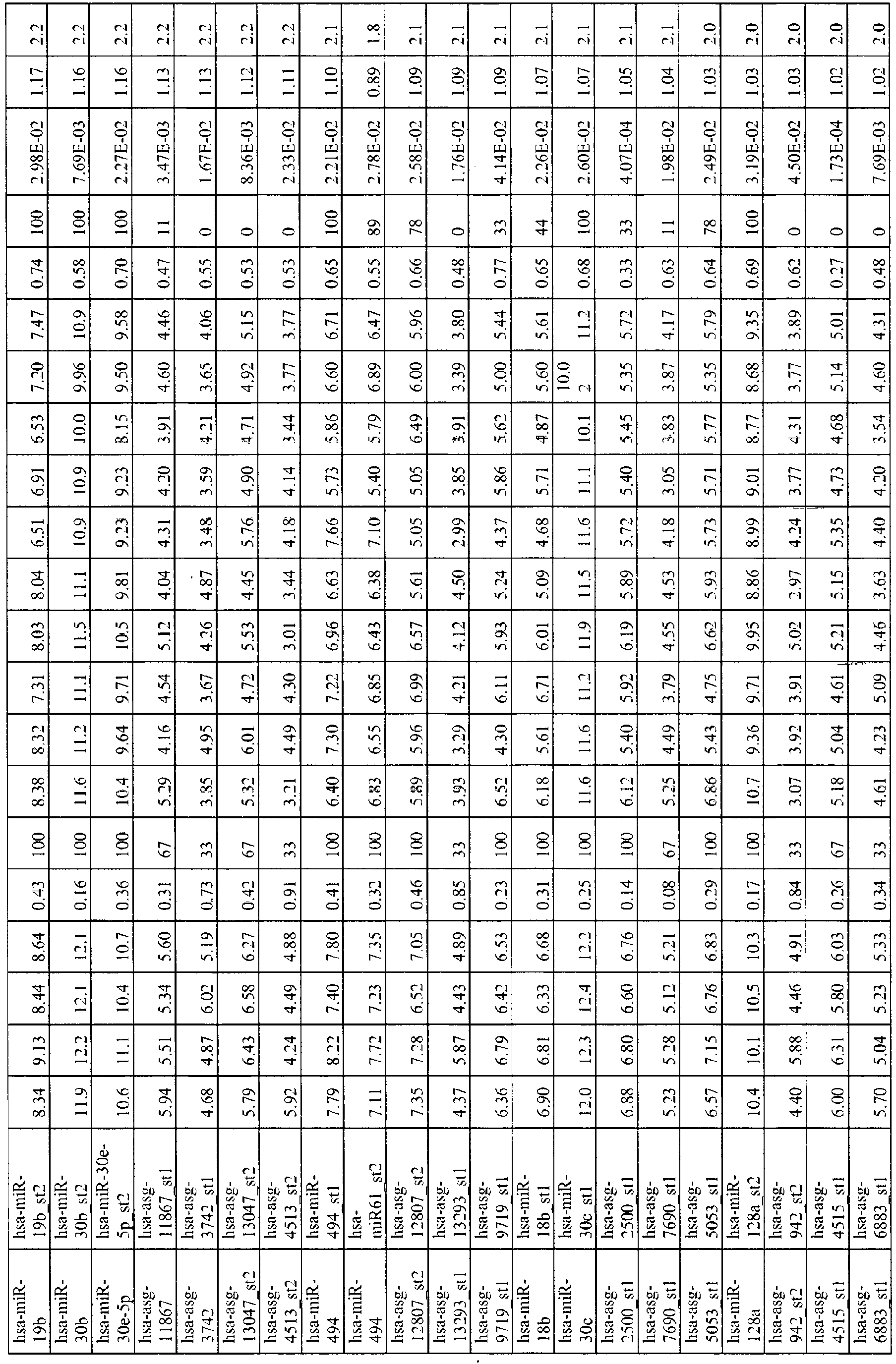

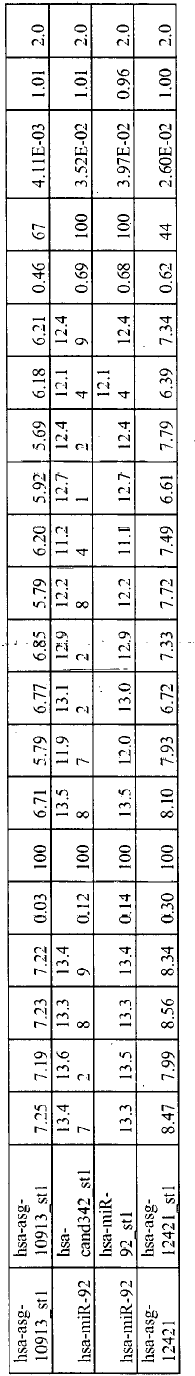

### EXAMPLE 23:

### MIRNAS DIFFERENTALLY EXPRESSED BETWEEN NORMAL OVARY TISSUE AND NORMAL LYMPH NODES

To identify miRNAs that may be useful markers for metastasis of cancerous ovary cells to lymph nodes, the inventors identified miRNAs that are expressed at significantly higher levels in normal ovary tissue than in cancer-negative lymph nodes.

Total RNA, purified from three separate normal human ovary tissue samples (FirstChoice^{®} Human Ovary Total RNA, cat. no. AM6974; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 239 miRNAs (114 verified, 125 predicted) with significant differential expression between normal ovary tissue samples and normal lymph nodes (|log2Diff| ovary vs LN neg ≥1 and p-value<0.05) (Table 22). Among the verified miRNAs, hsa-miR-383 had an average expression level over 100-fold higher in ovary samples; hsa-miR-513, -509, and, -493-5p had average expression levels that were more than 50-fold higher in ovary samples; 15 miRNAs had average expression levels that were between 20- and 50-fold higher in ovary samples; 18 miRNAs had average expression levels that were between 10- and 20-fold higher in ovary samples; and 28 miRNAs had average expression levels that were between 5- and 10-fold higher in ovary tissue samples than in normal lymph nodes. Among the predicted miRNAs, hsa-asg-5638_st2 had an average expression level over 100-fold higher in ovary samples; hsa-asg-13404_st2 had an average expression level over 50-fold higher in ovary samples; nine miRNAs had average expression levels that were between 20- and 50-fold higher in ovary samples; 10 miRNAs had average expression levels that were between 10-and 20-fold higher in ovary samples; and 23 miRNAs had average expression levels that were between 5- and 10-fold higher in ovary tissue samples than in normal lymph nodes.

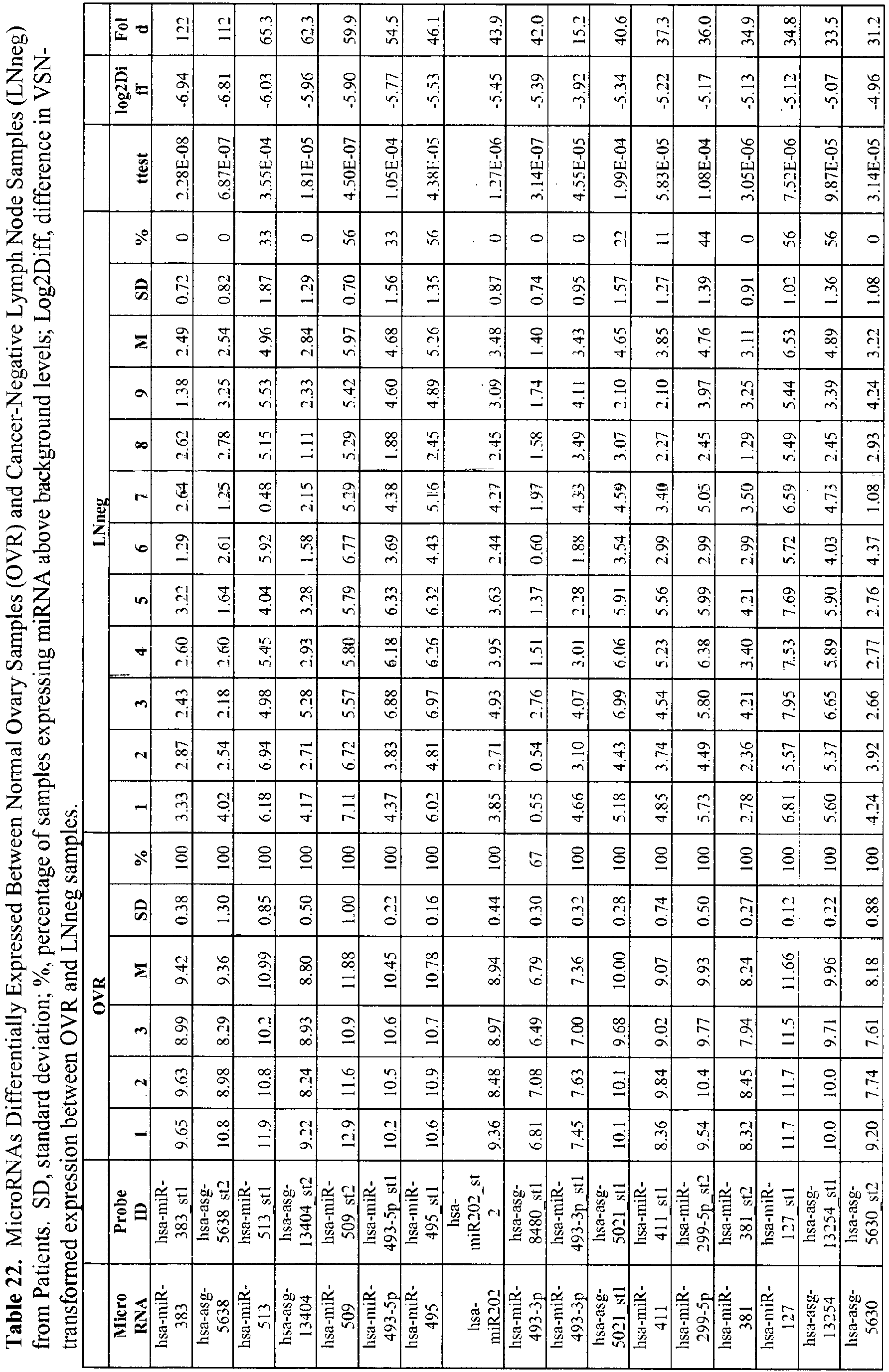

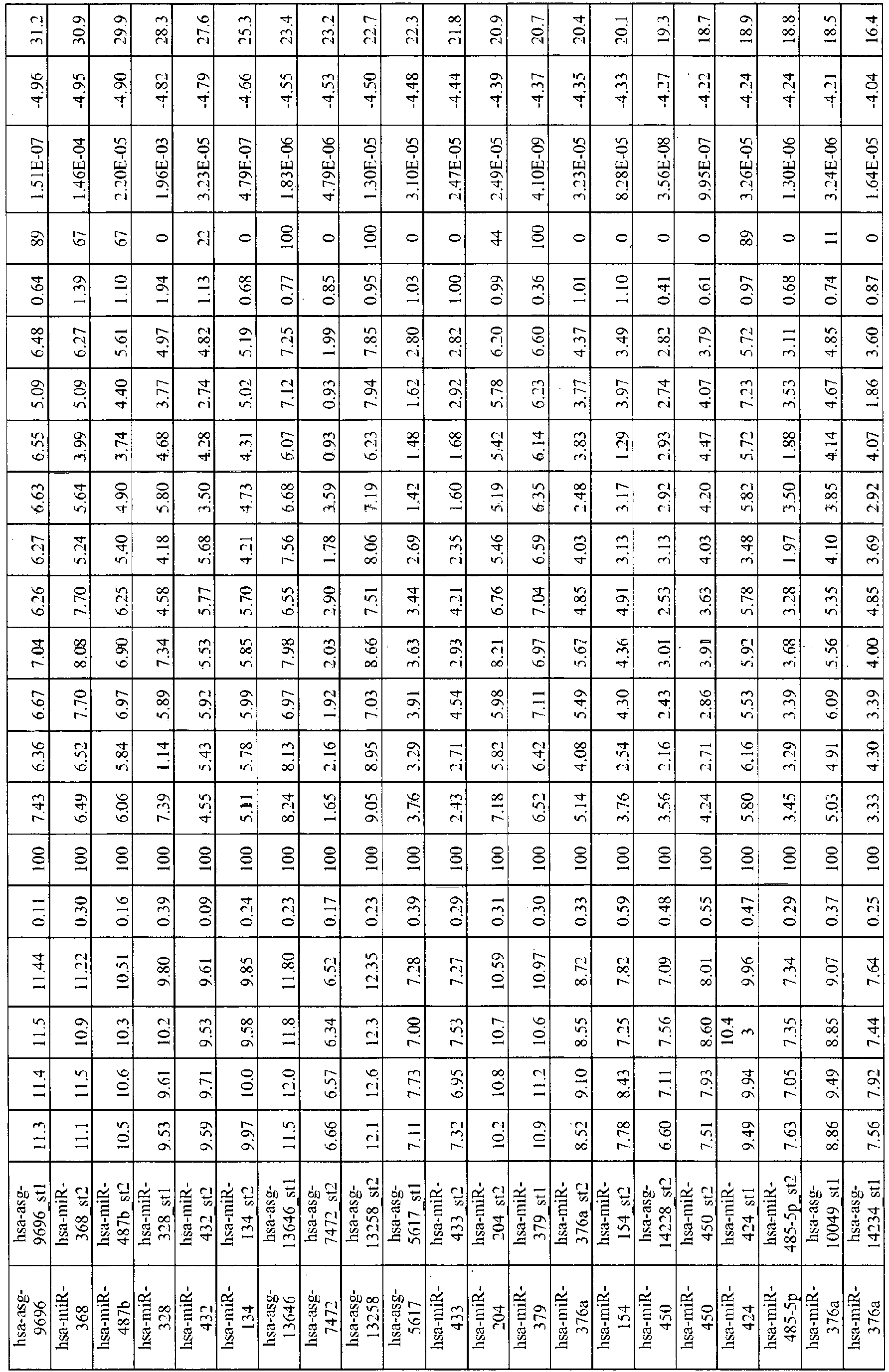

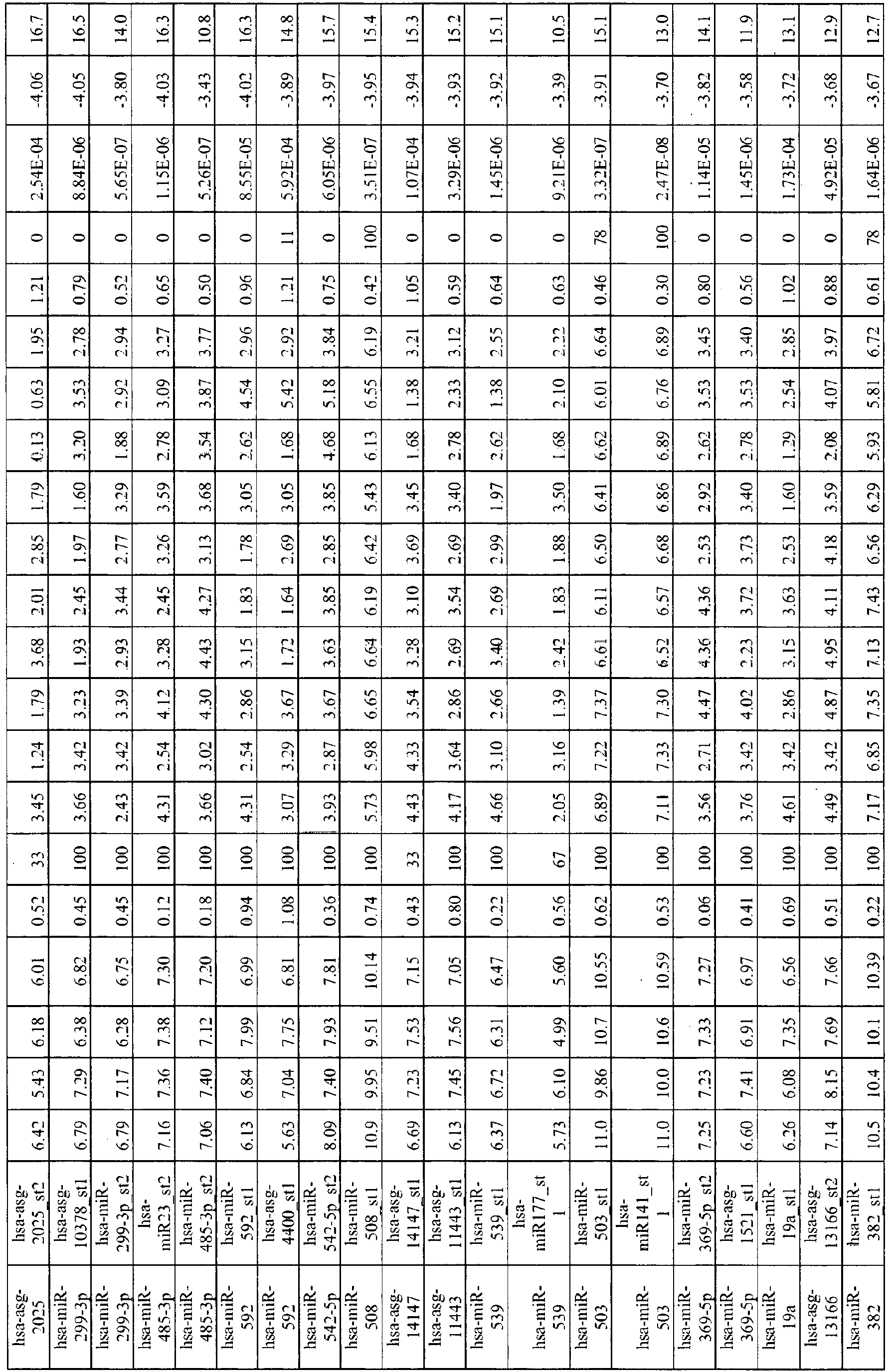

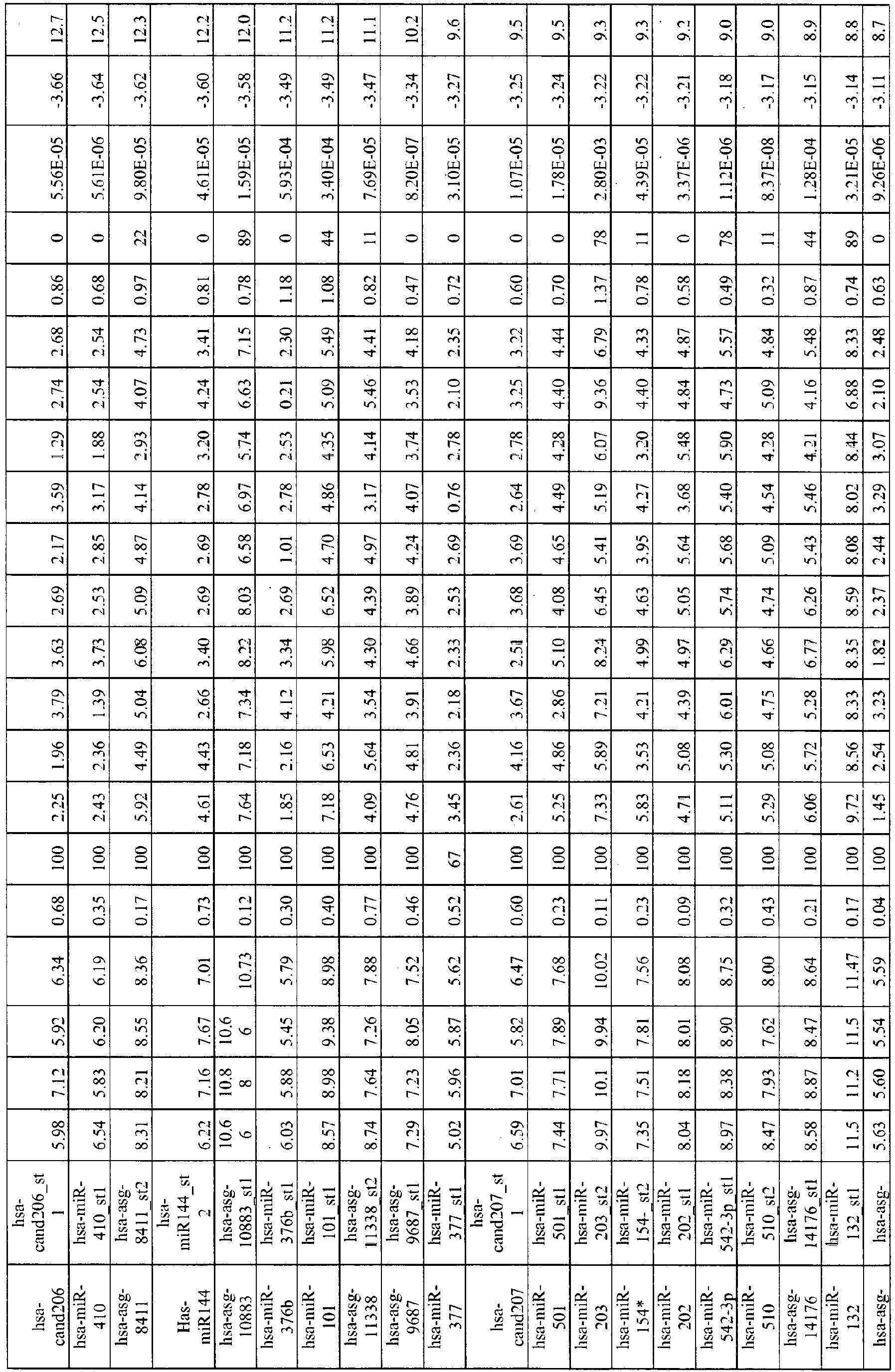

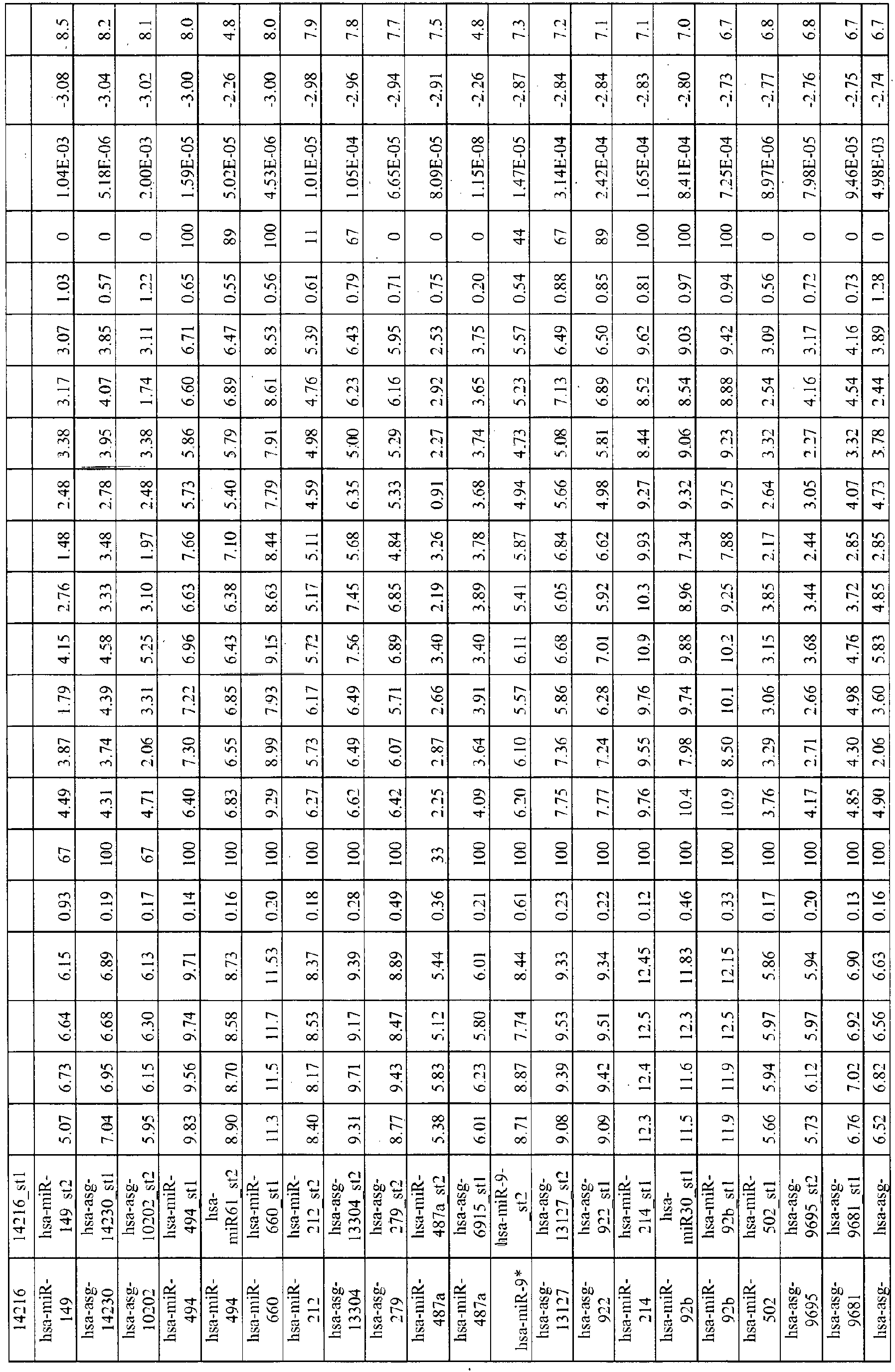

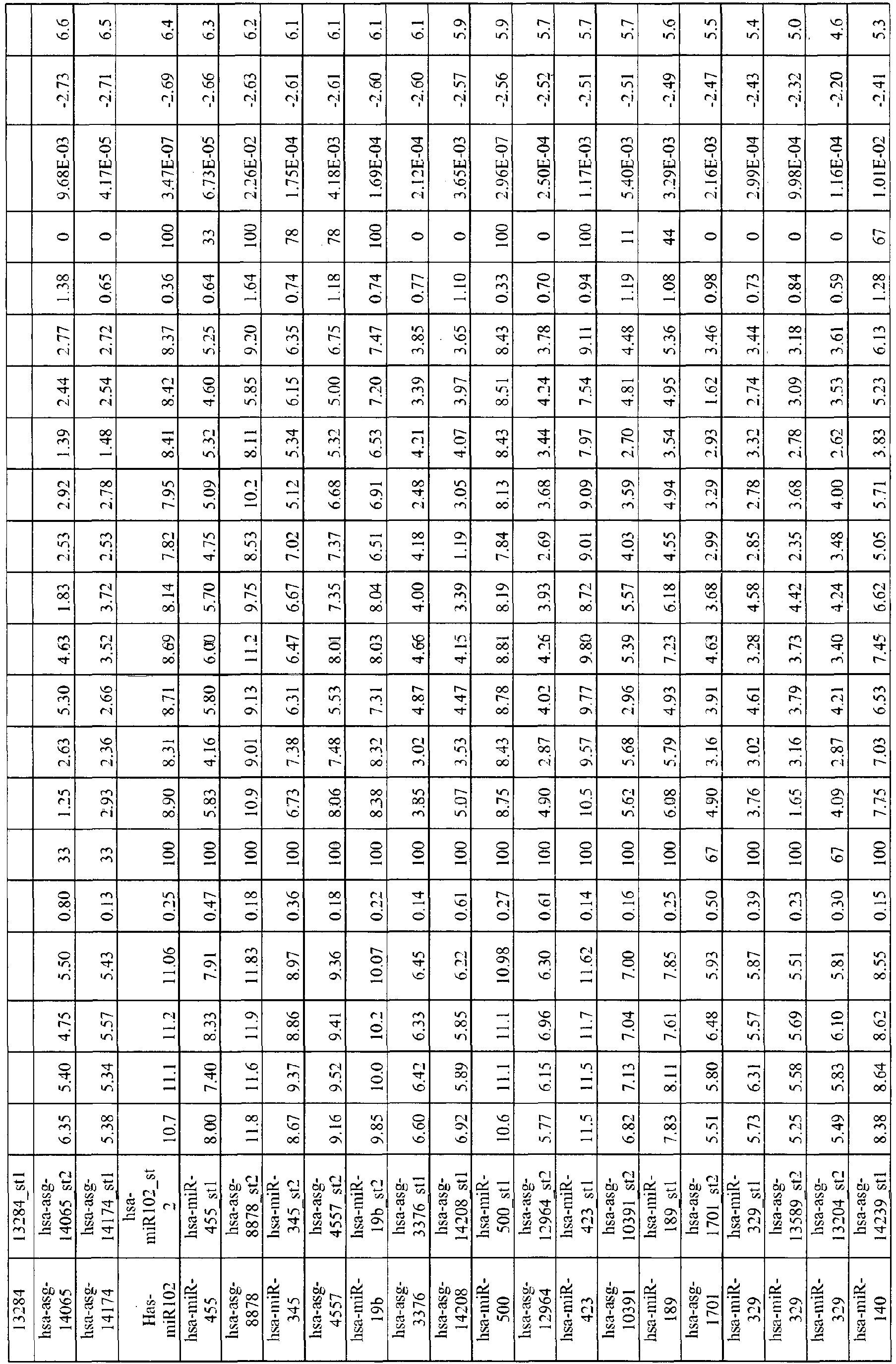

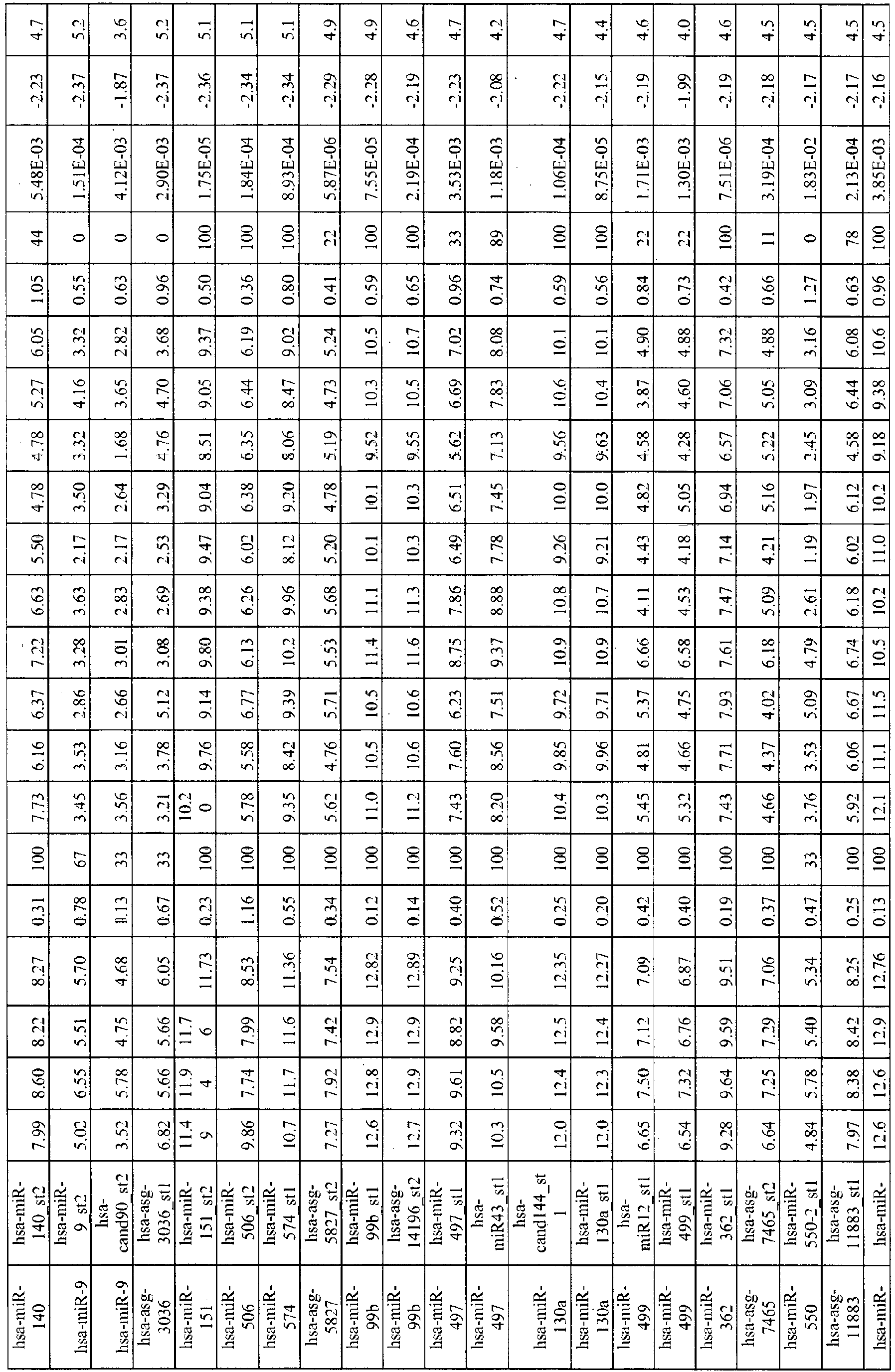

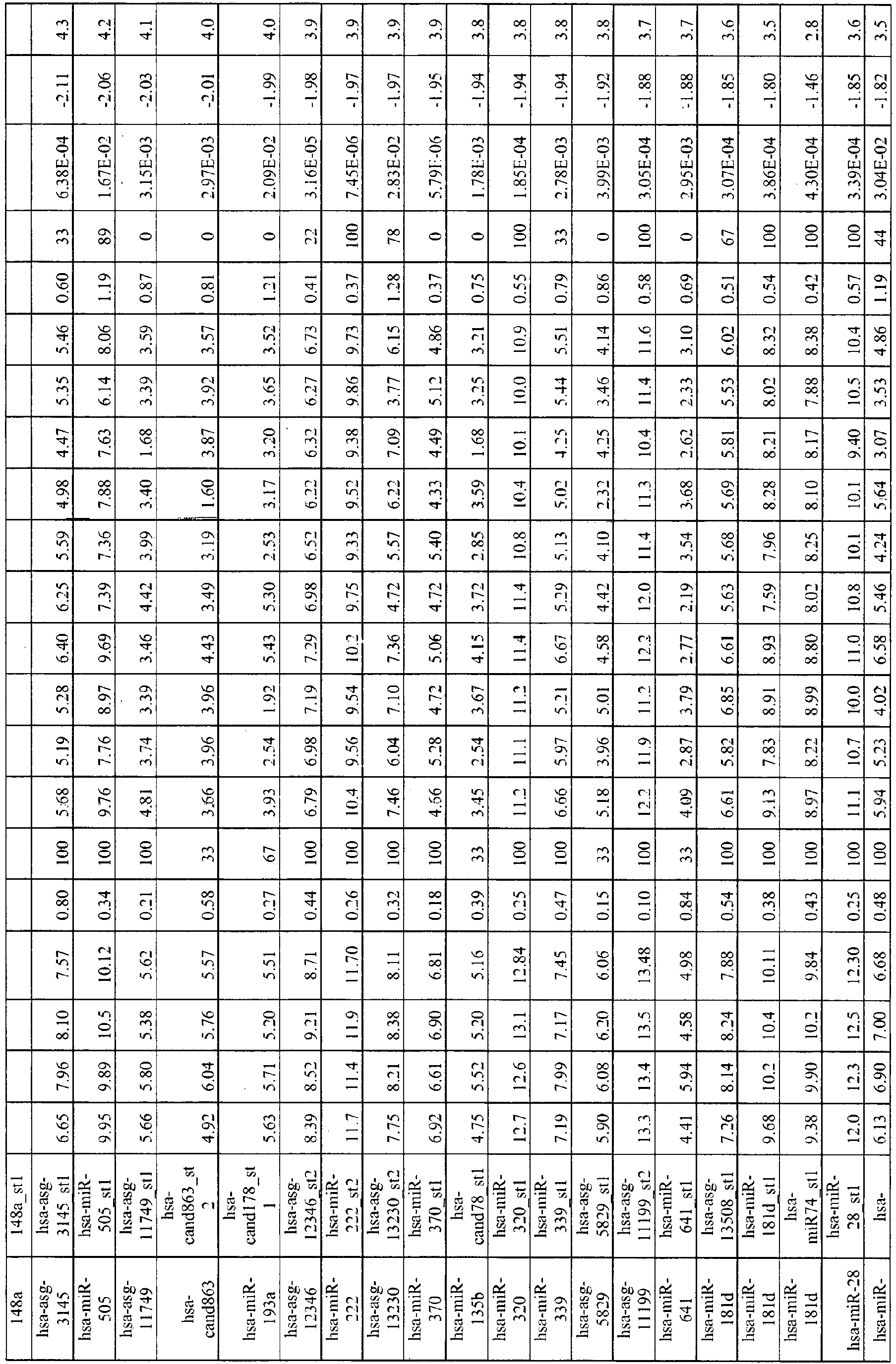

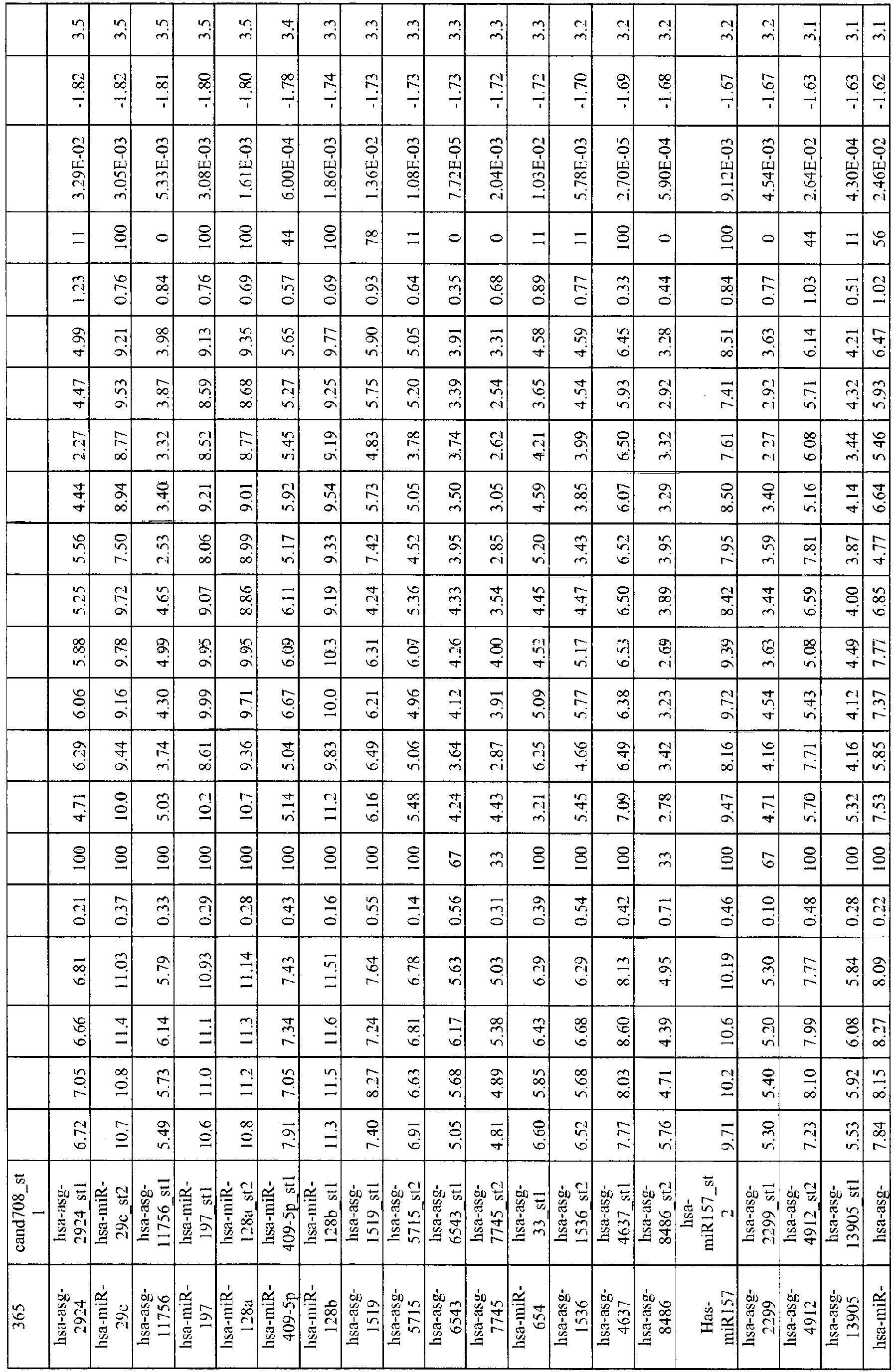

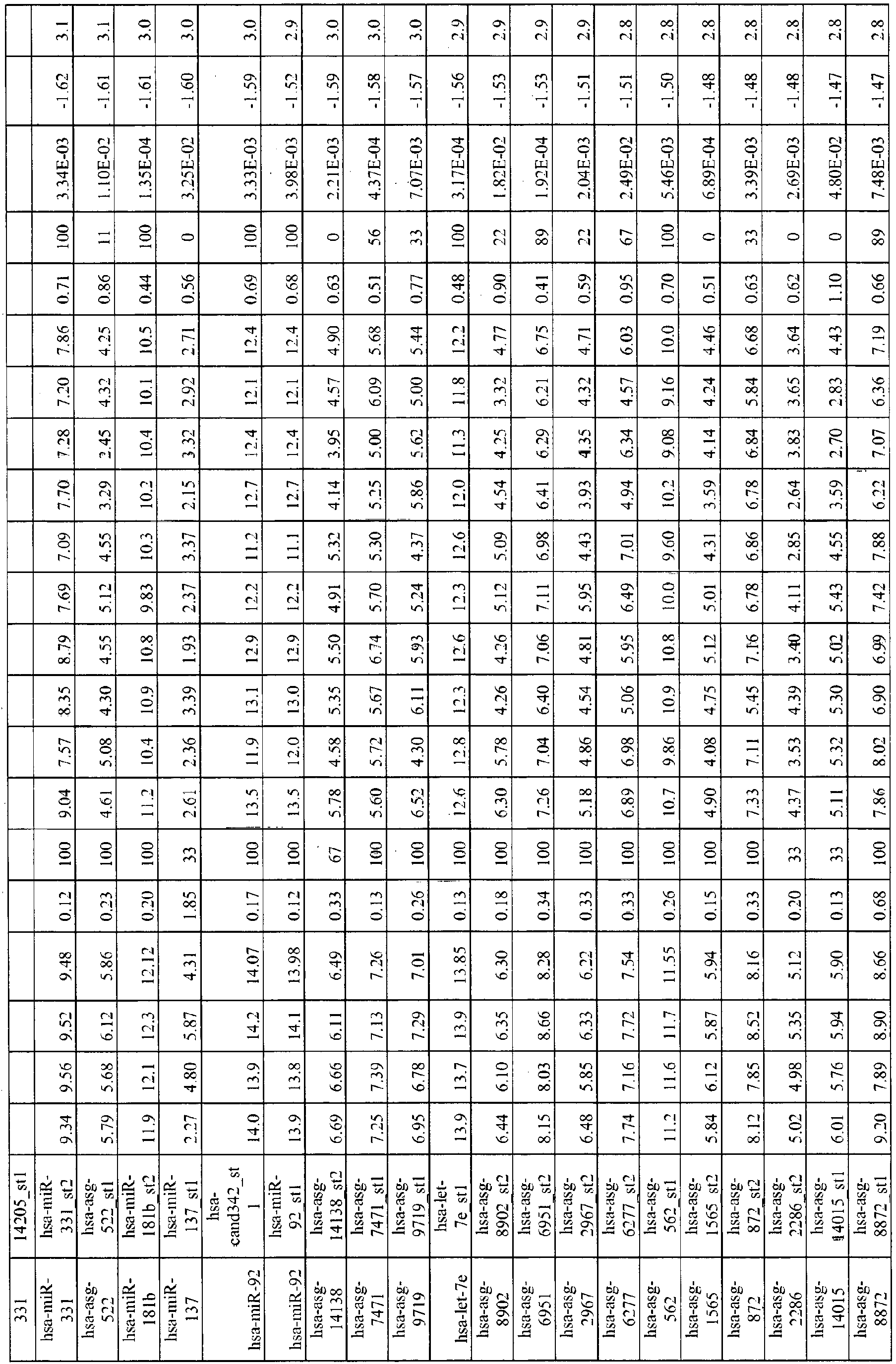

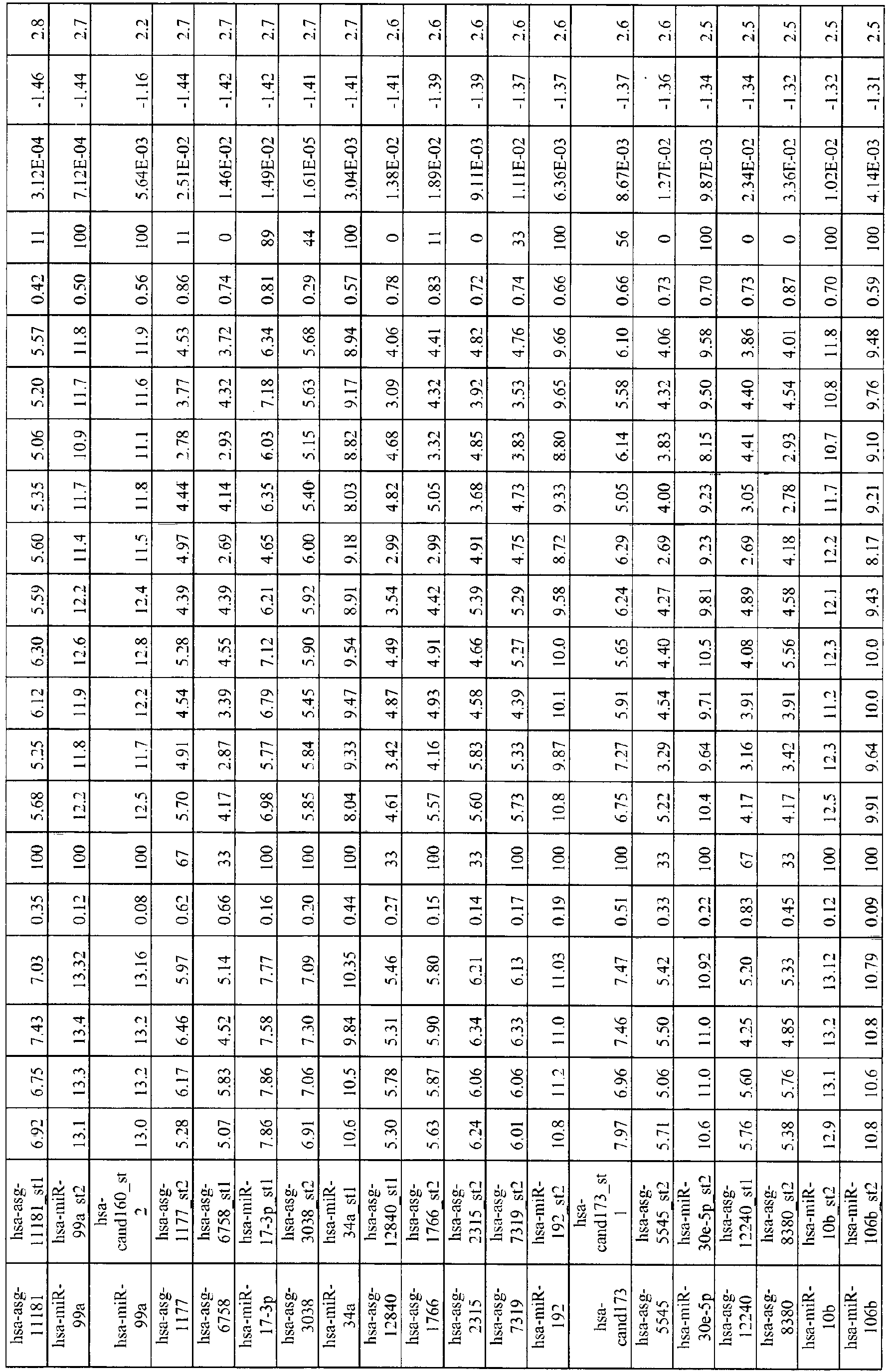

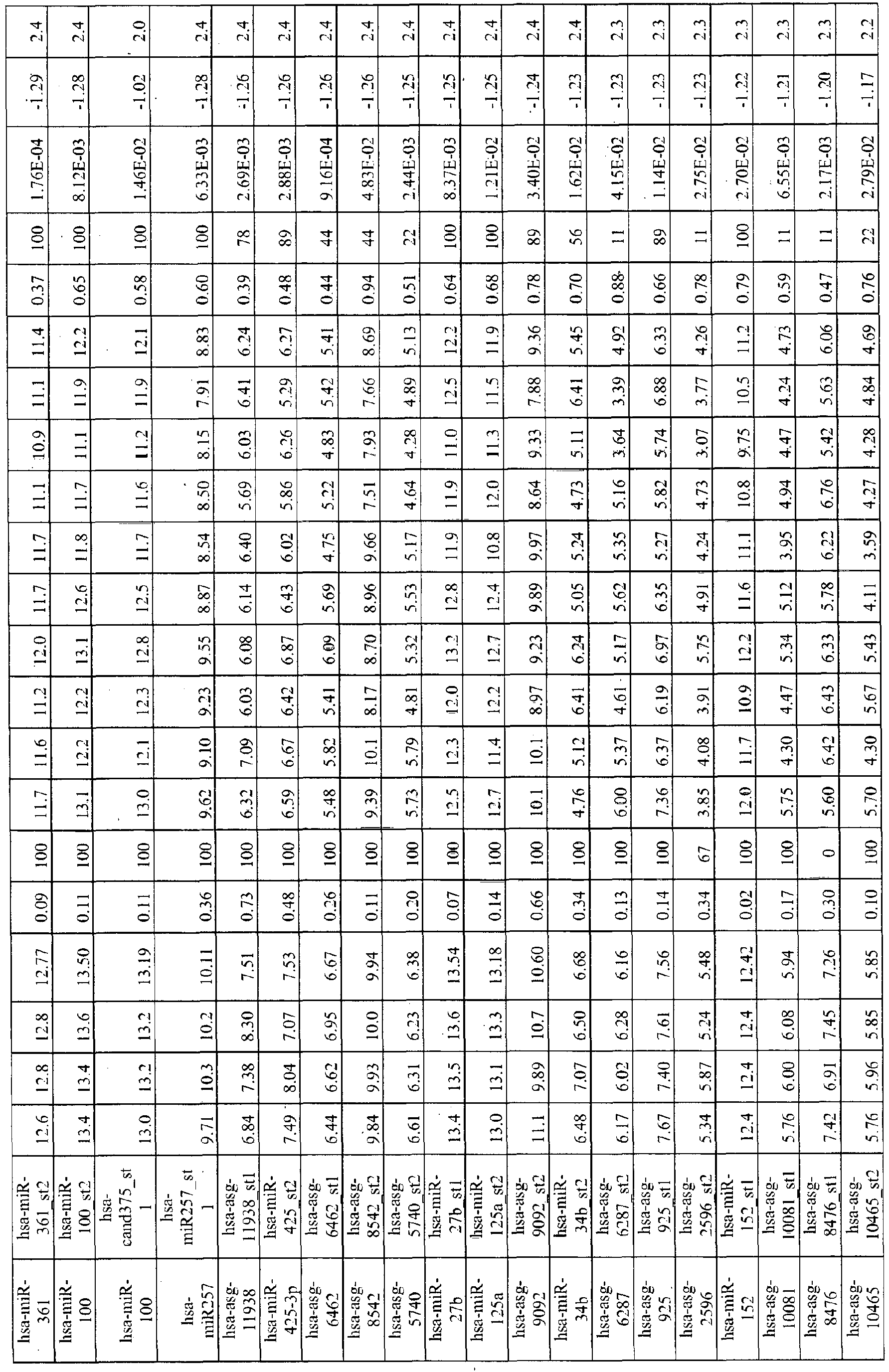

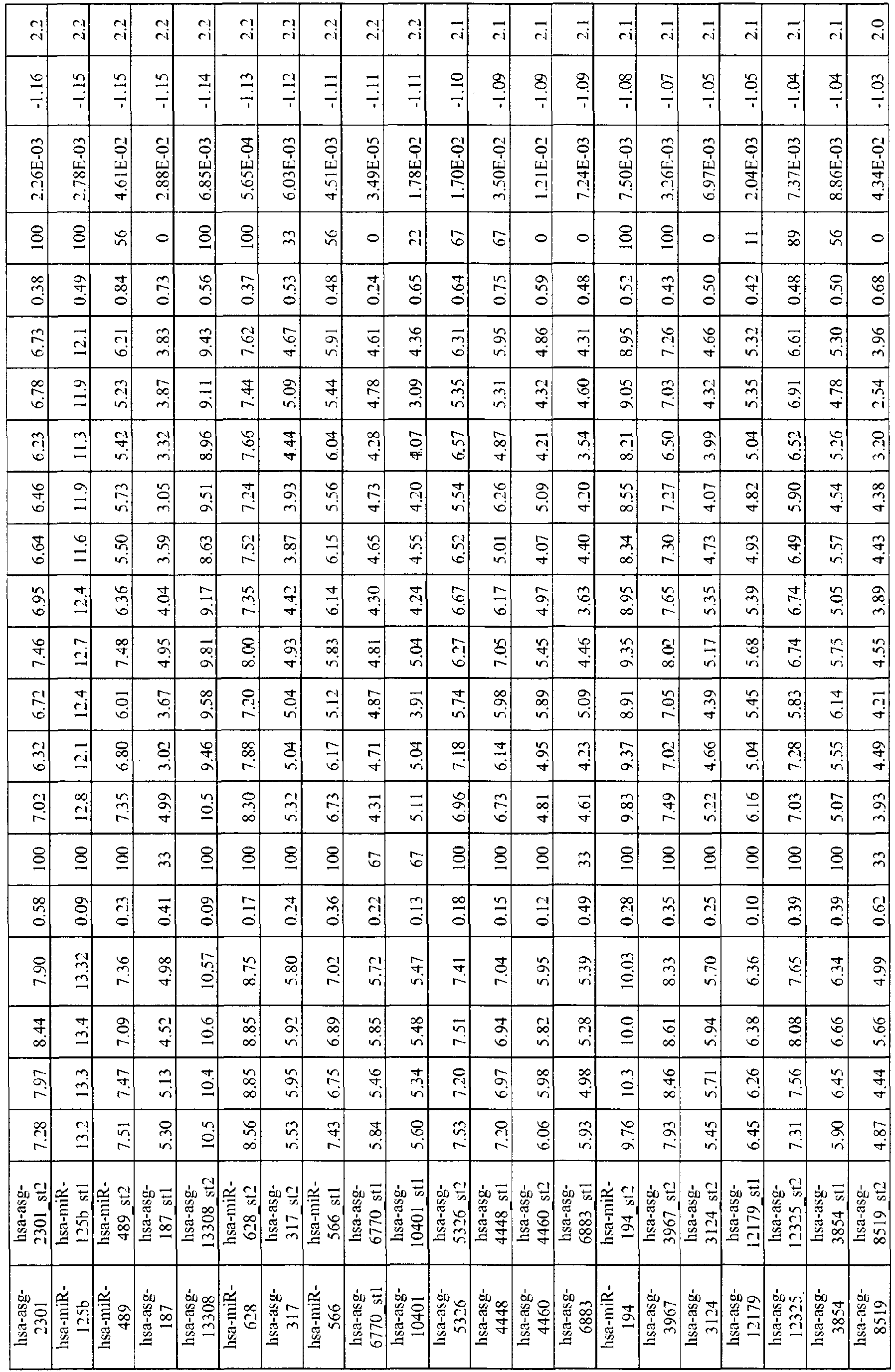

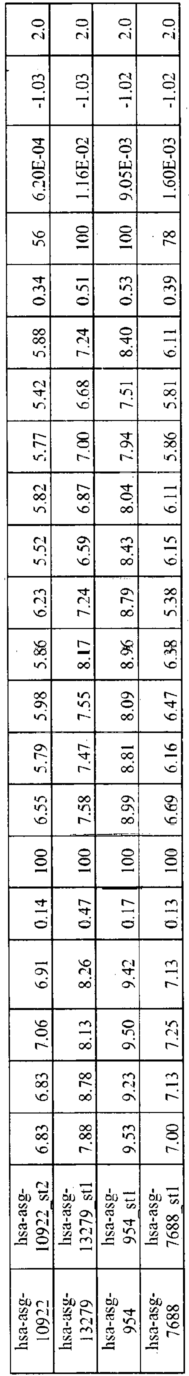

### EXAMPLE 24:

### MIRNAS DIFFERENTIALLY EXPRESSED BETWEEN NORMAL PROSTATE TISSUE AND NORMAL LYMPH NODES

To identify miRNA that may be useful markers for metastasis of cancerous prostate cells to lymph nodes, the inventors identified miRNAs that are expressed at significantly higher levels in normal prostate tissue than in cancer-negative lymph nodes.

Total RNA, purified from three separate normal human prostate tissue samples (FirstChoice^{®} Human Prostate Total RNA, cat. no. AM7988; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 159 miRNAs (72 verified, 87 predicted) with significant differential expression between normal prostate and normal lymph nodes samples (|log2Diff| prostate vs LN neg ≥1 and p-value<0.05) (Table 23). Among the verified miRNAs, hsa-miR-205 and -375 had average expression levels over 100-fold higher in prostate tissue samples; hsa-miR-429, -206 and -187 had average expression levels over 50-fold higher in prostate samples; 13 miRNAs (hsa-miR-200b, -200a, -133b, -200c, -133a, - 203, -493-5p, -605, -487b, -141, -96, -495, and -149) had average expression levels that were between 10- and 40-fold higher in prostate samples; and 18 miRNAs had average expression levels that were between 5- and 10-fold higher in prostate samples than in normal lymph nodes. Among the predicted miRNAs, hsa-asg-14153_st2 had an average expression level that was over 30-fold higher in prostate samples; hsa-asg-7637_st2, -4915_st1 and 5021_st1) had average expression levels that were between 10-and 20-fold higher in prostate samples; and 28 miRNAs had average expression levels that were between 5- and 10-fold higher in prostate tissue samples than in normal lymph nodes.

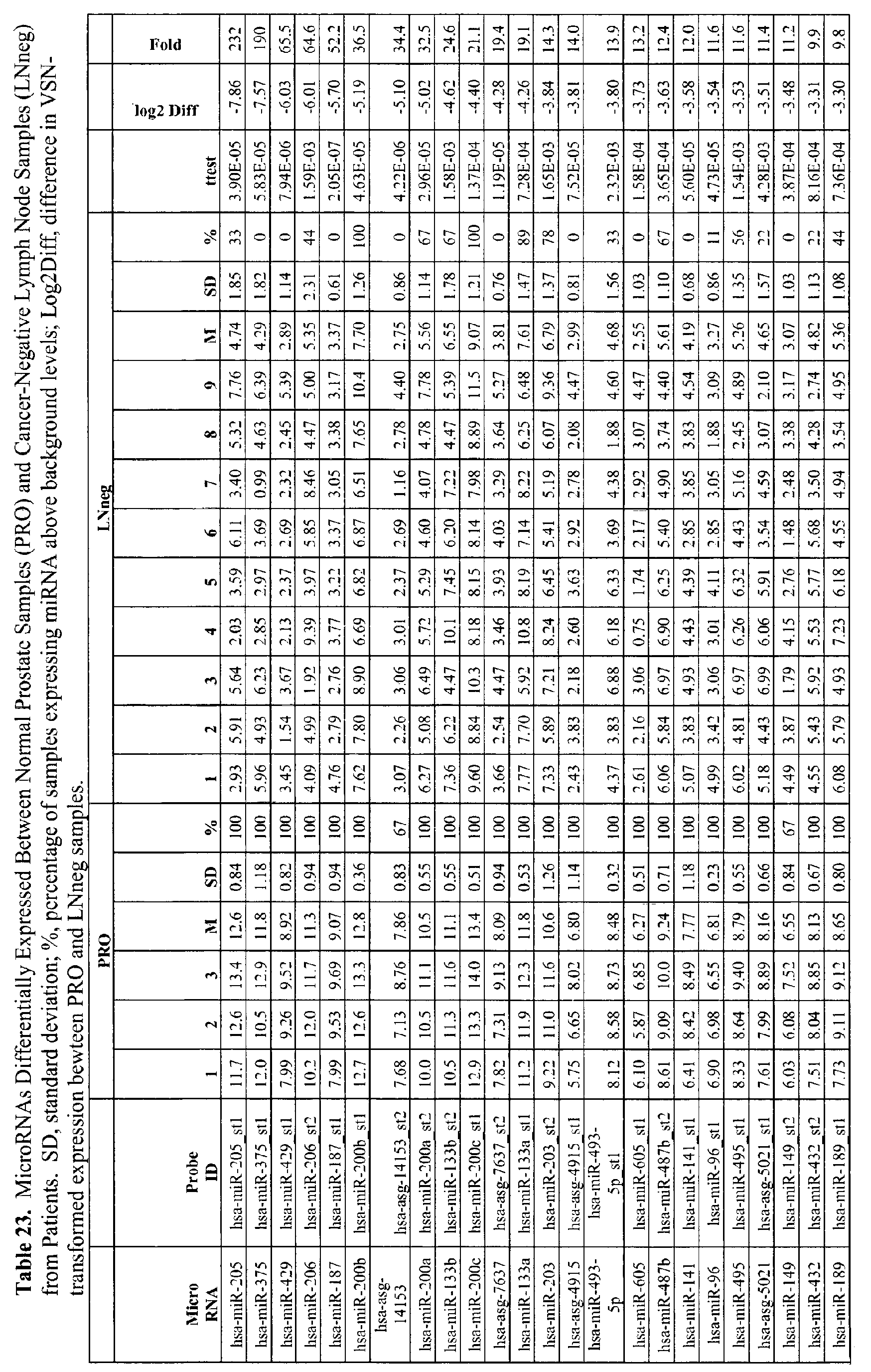

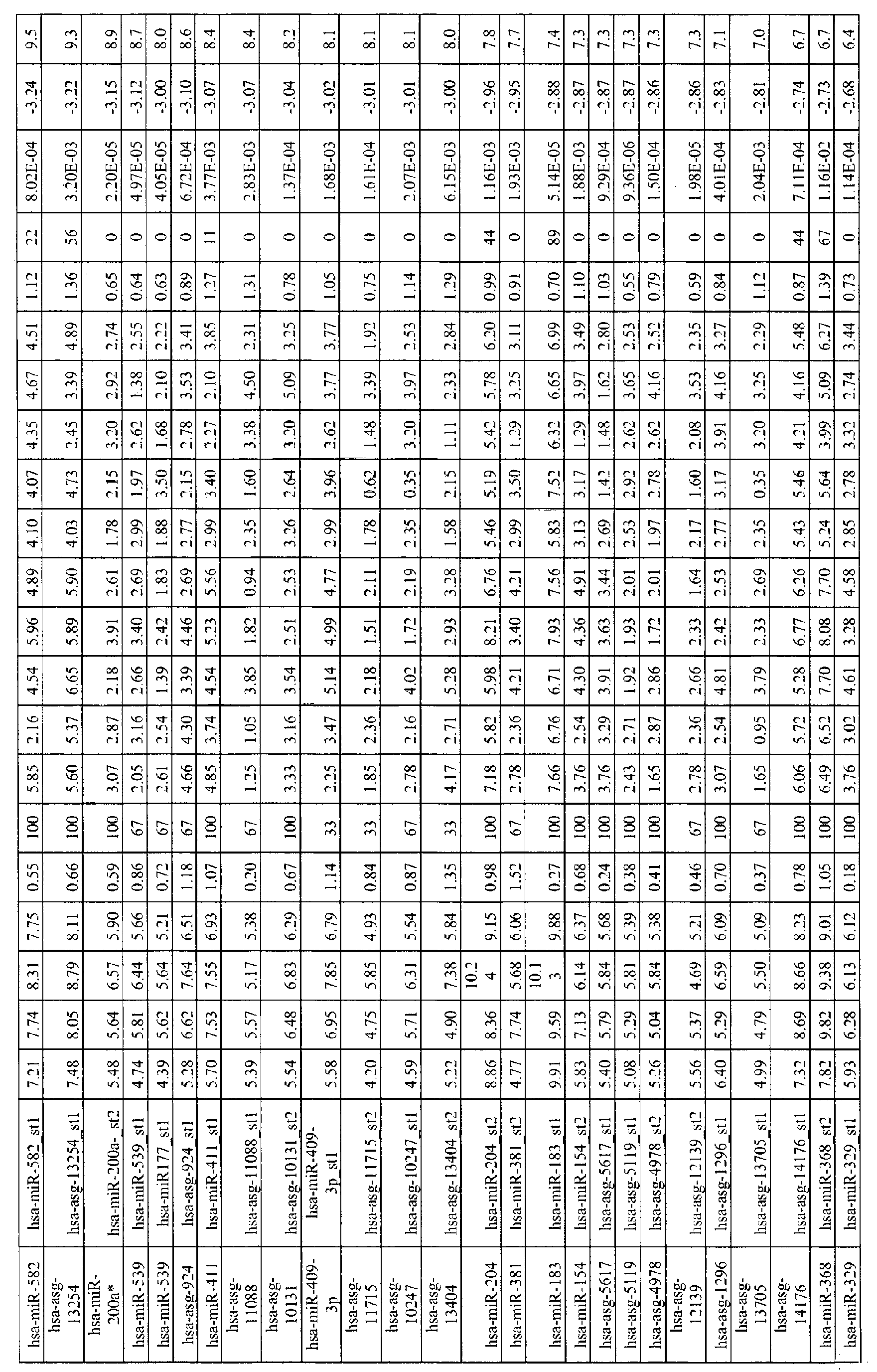

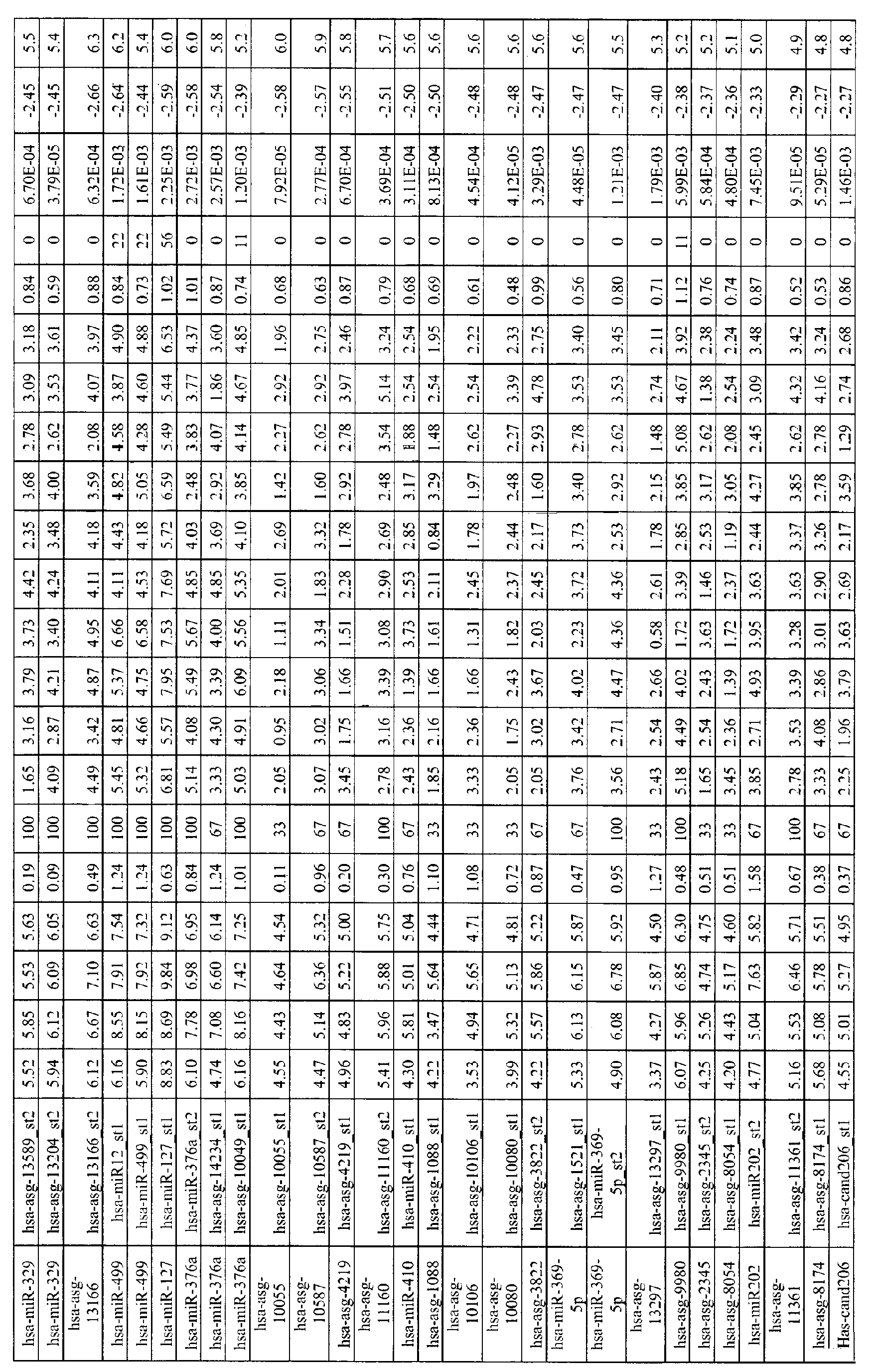

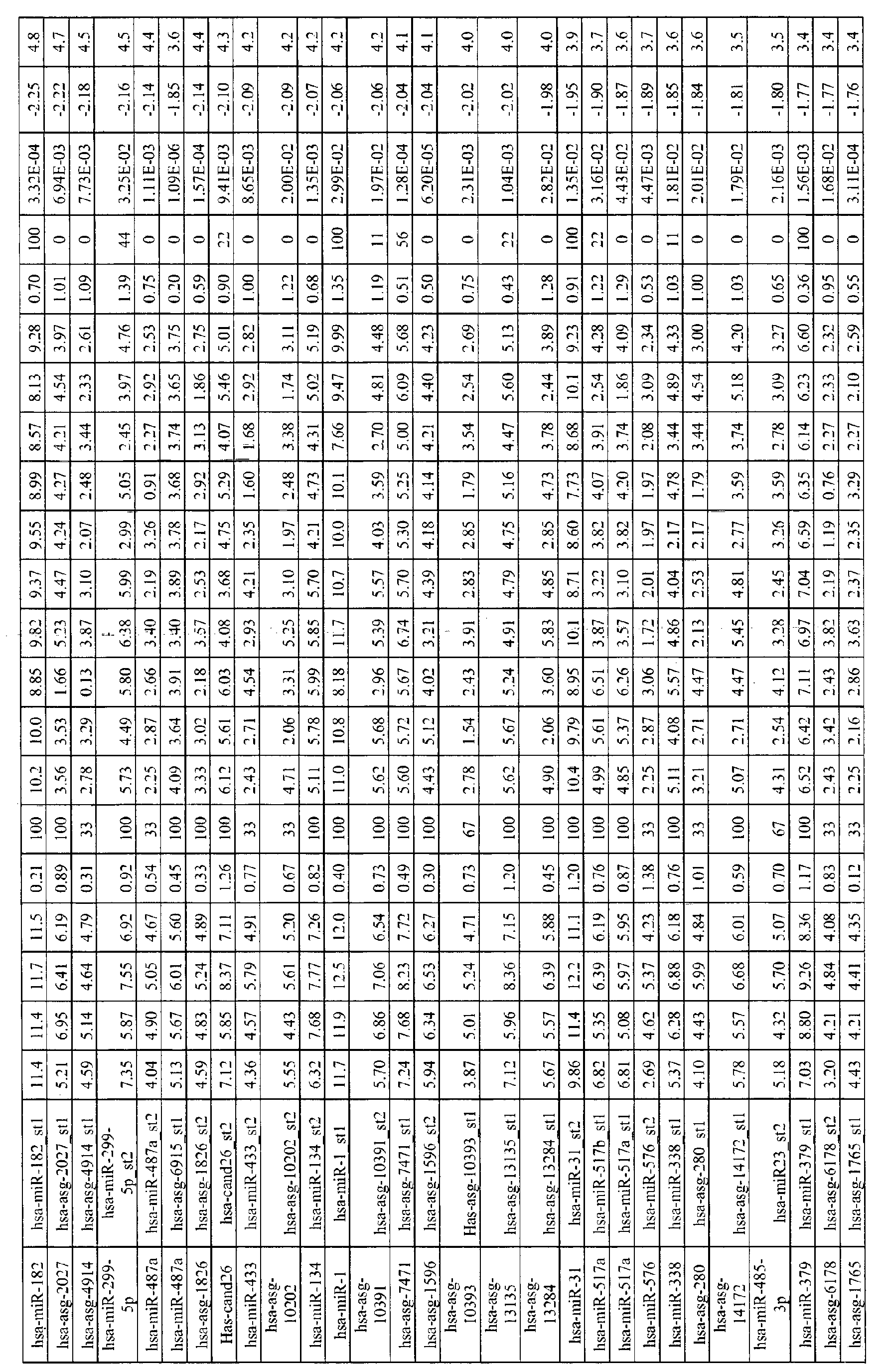

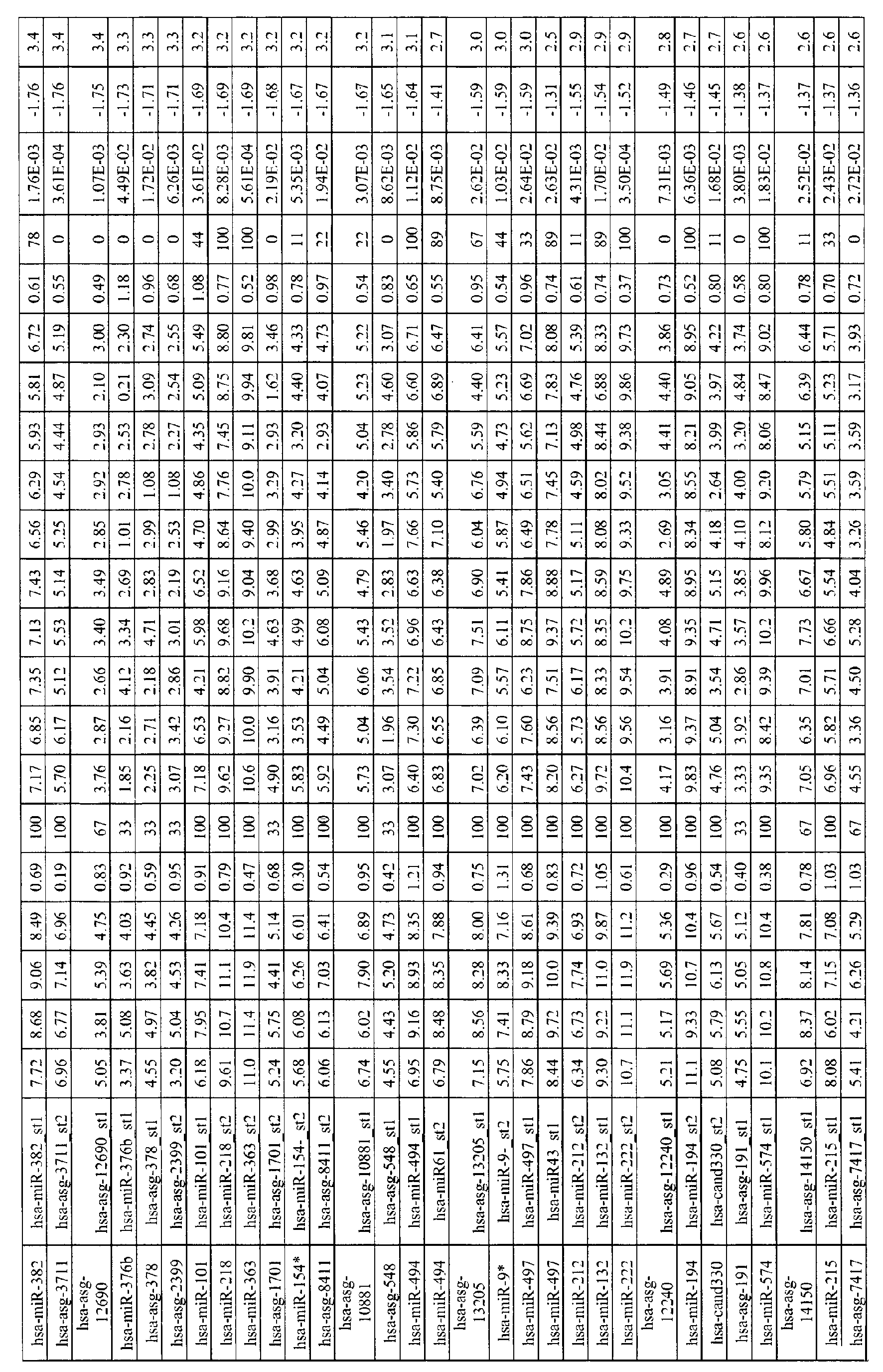

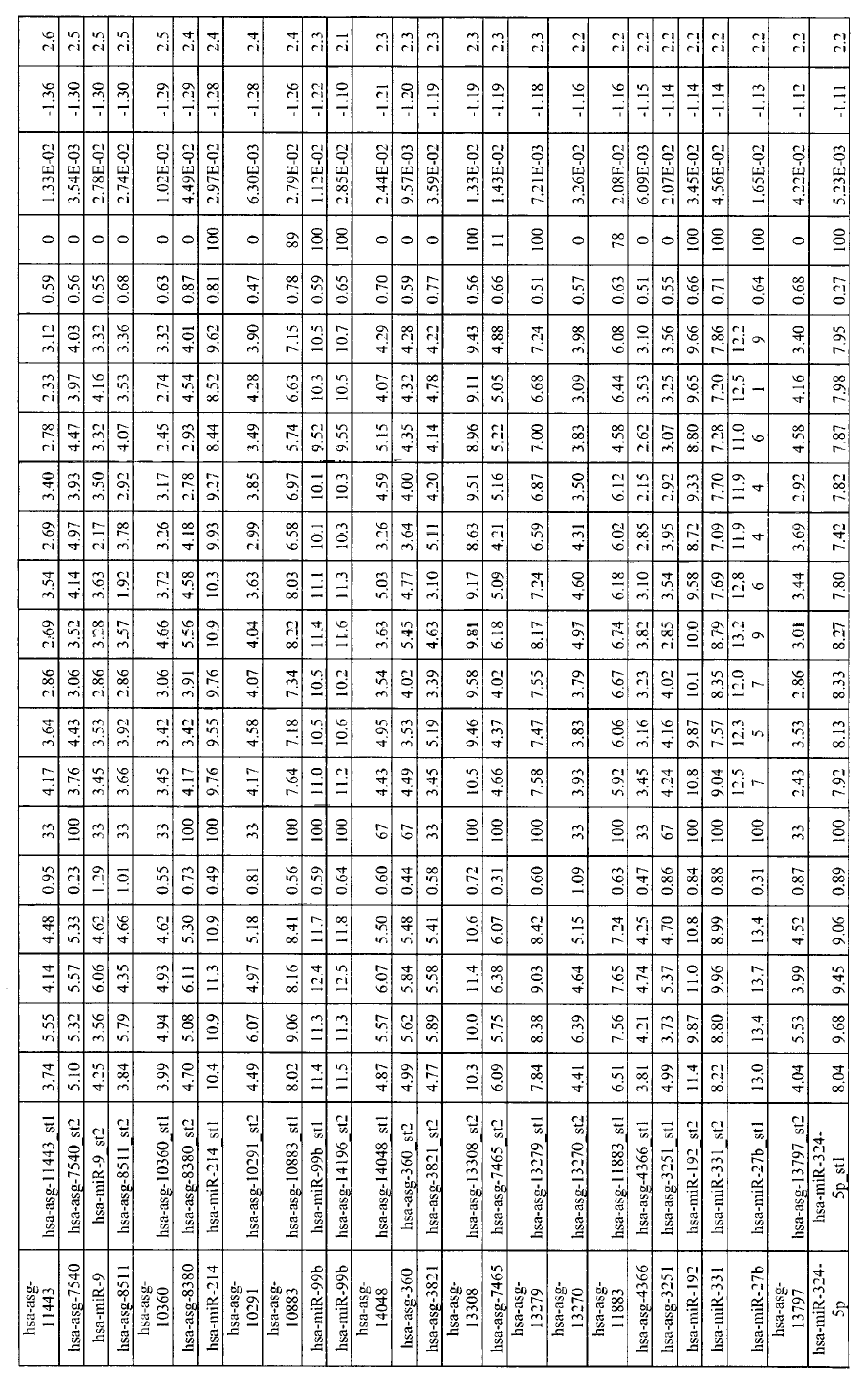

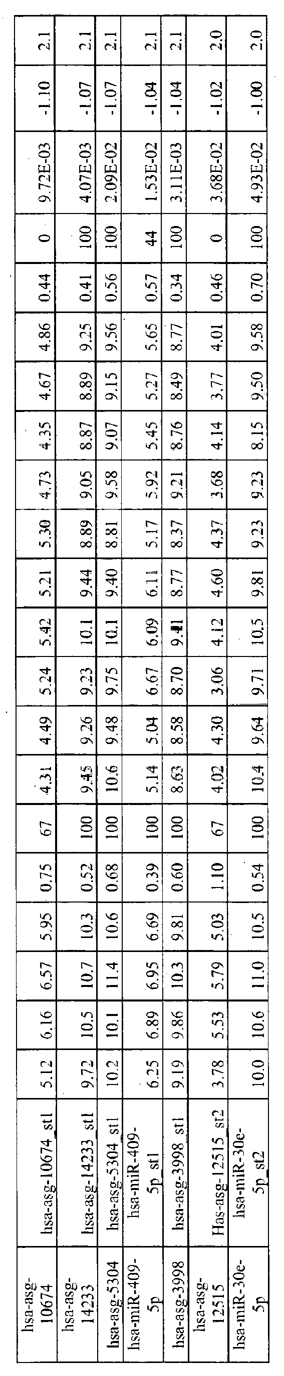

### EXAMPLE 25:

### MIRNAS DIFFERENTIALLY EXPRESSED BETWEEN NORMAL LUNG TISSUE AND NORMAL LYMPH NODES

To identify miRNAs that may be useful markers for metastasis of cancerous lung cells to lymph nodes, the inventors identified miRNAs that are expressed at significantly higher levels in normal lung tissue than in cancer-negative lymph nodes.

Total RNA, purified from three separate normal human lung tissue samples (FirstChoice^{®} Human Lung Total RNA, cat. no. AM7968; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 182 miRNAs (72 verified, 110 predicted) with significant differential expression between normal lung and normal lymph nodes (log2Diff lung vs LN neg ≥1 and p-value<0.05) (Table 24). Among the verified miRNAs, hsa-miR-375 had an average expression level over 100-fold higher in lung tissue samples; hsa-miR-429, -205 and -203) had average expression levels that were over 50-fold higher in lung samples; 6 miRNAs (hsa-miR-34c, -200a, -200b, -200c, -184 and-141) had average expression levels that were between 20- and 50-fold higher in lung samples; 4 miRNAs (hsa-miR-449, -605, - 338 and -187) had average expression levels that were between 10- and 20-fold higher in lung samples; and 11 miRNAs had average expression levels that were between 5- and 10-fold higher in lung samples compared to normal lymph nodes. Among the predicted miRNAs, hsa-asg-13231_st1 had an average expression level that was over 50-fold higher in lung samples; hsa-asg-4439_st2 and hsa-cand150_st2 had average expression levels that were between 25-and 45-fold higher in lung samples; 6 miRNAs had average expression levels that were between 10- and 20-fold higher in lung samples; and 18 miRNAs had average expression levels that were between 5- and 10-fold higher in lung tissue samples compared to normal lymph nodes.

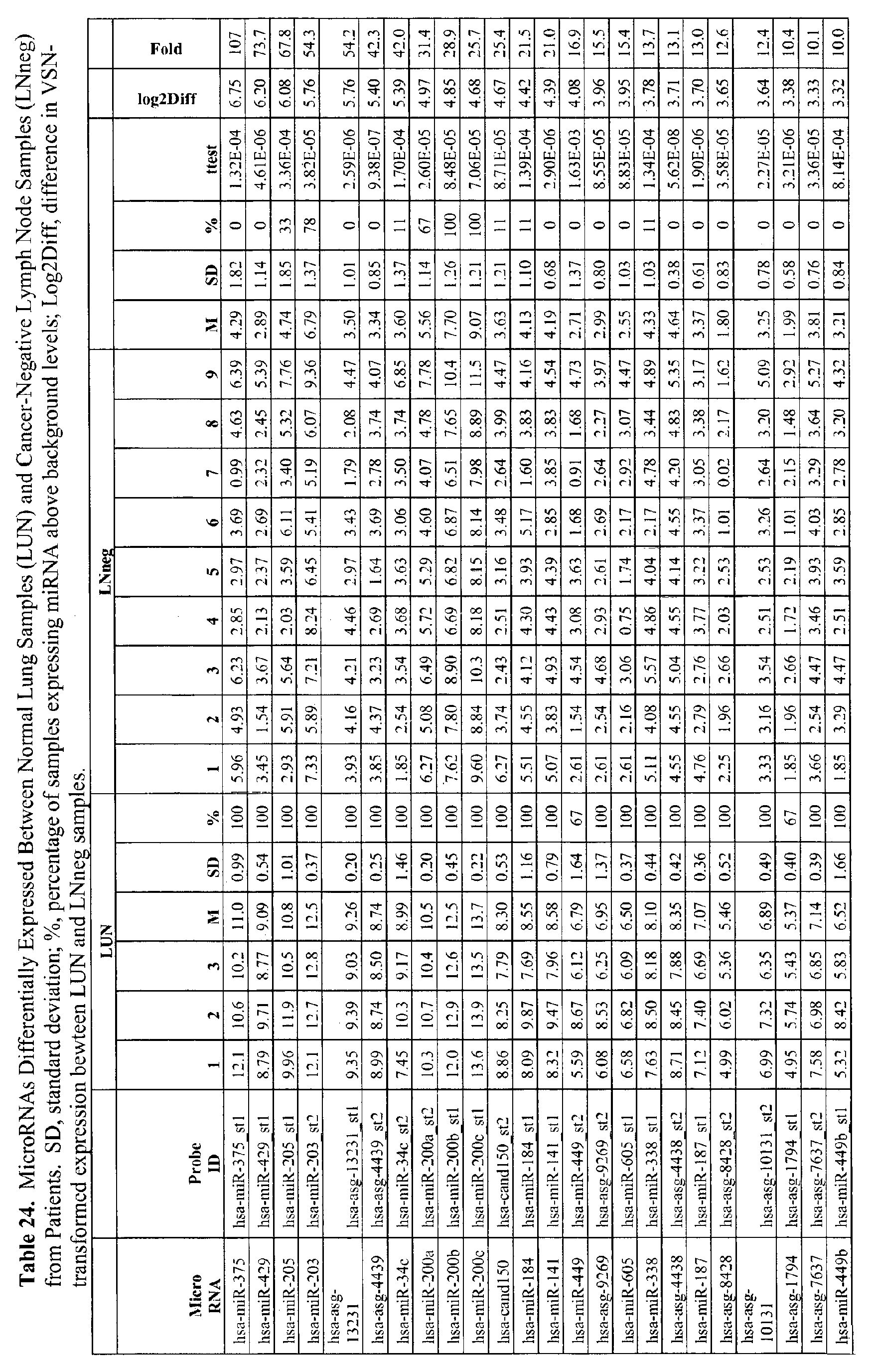

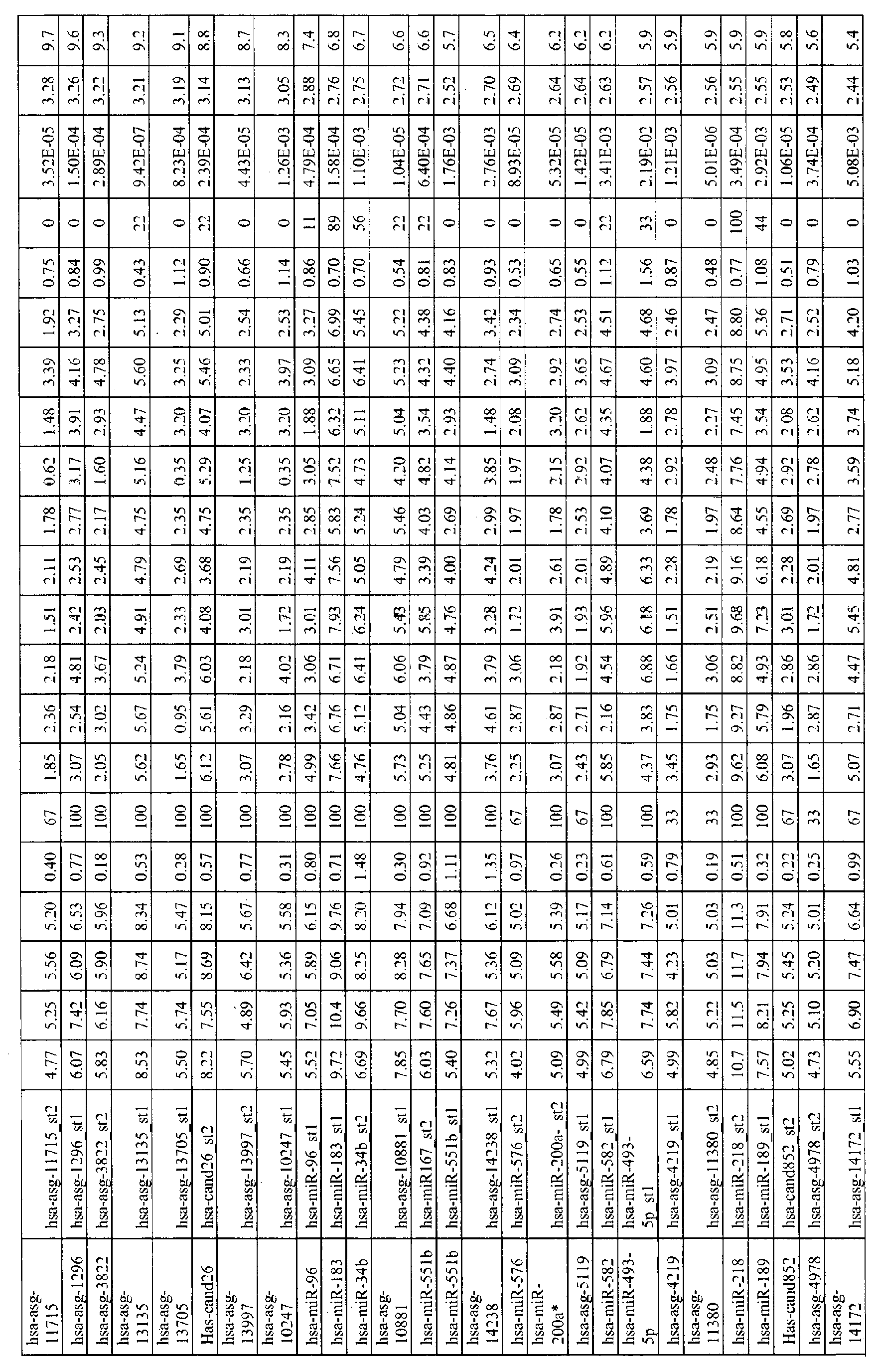

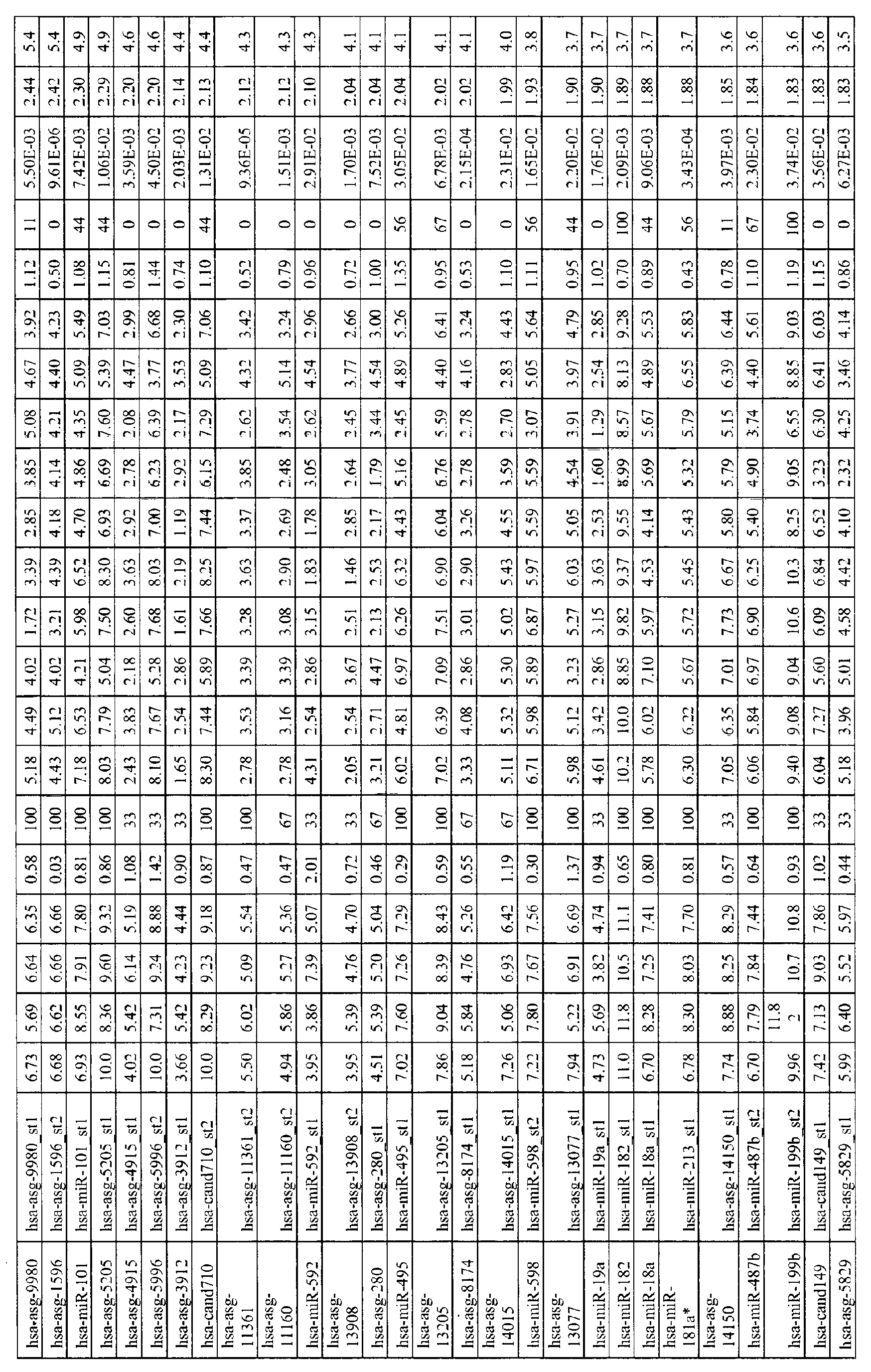

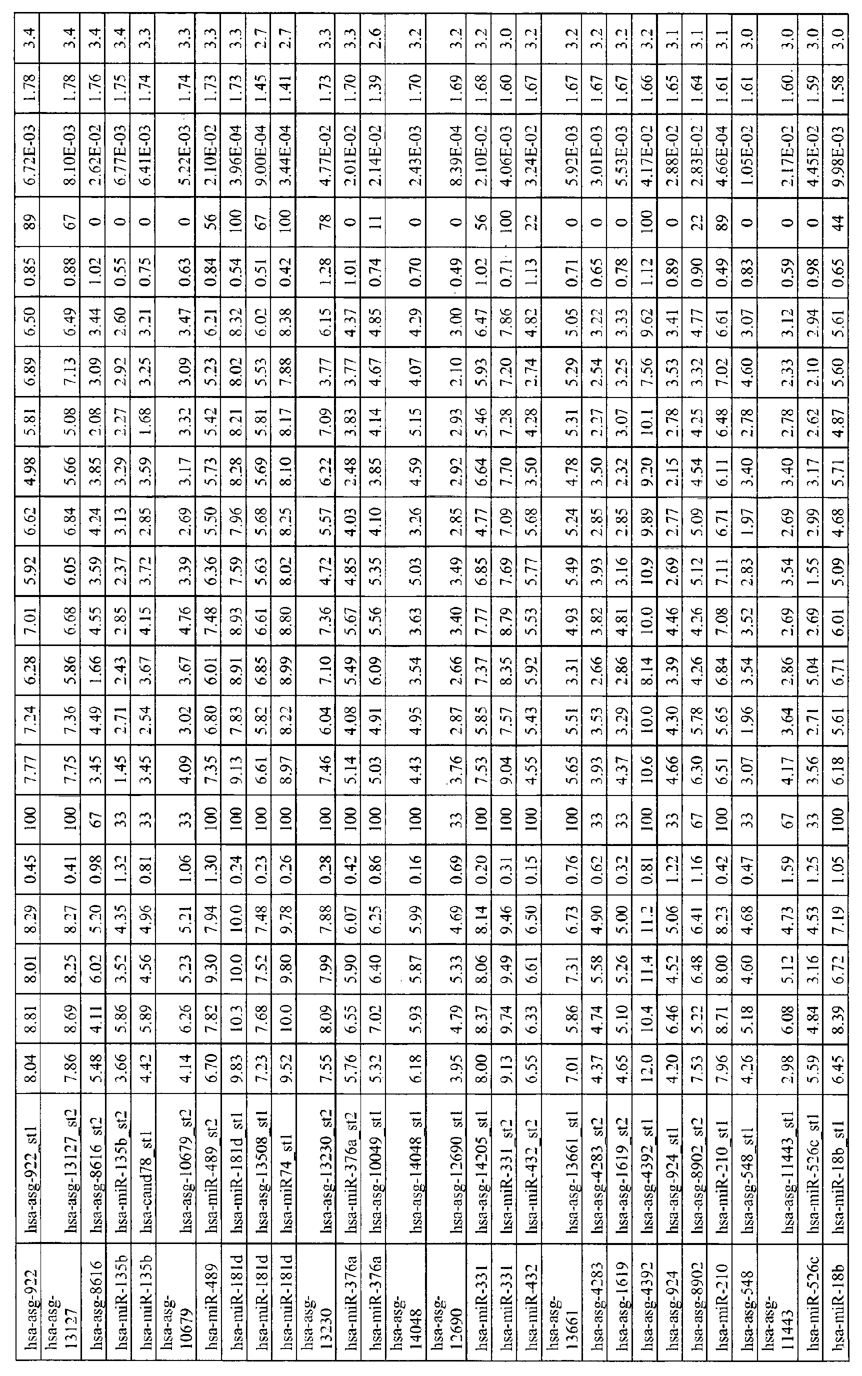

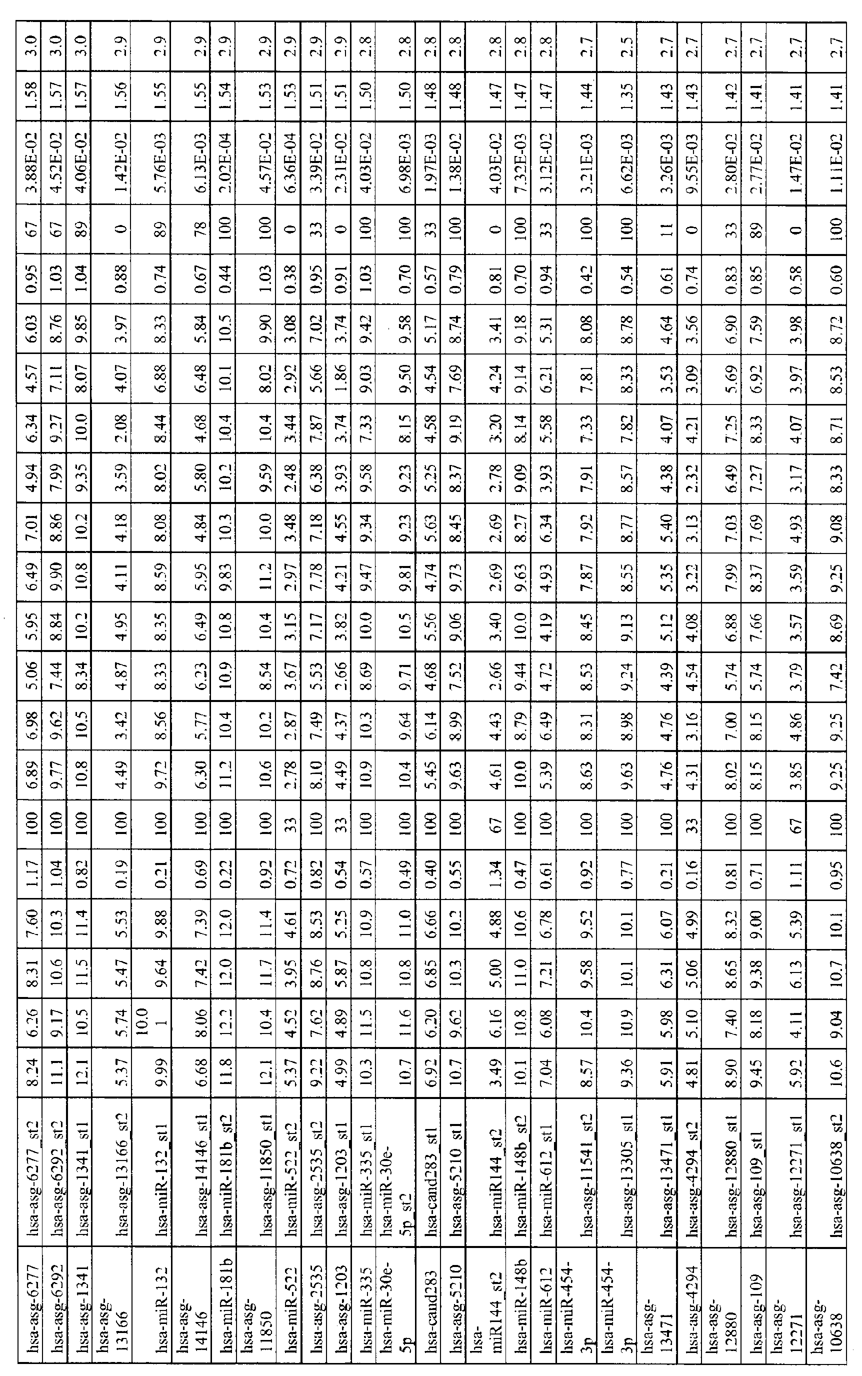

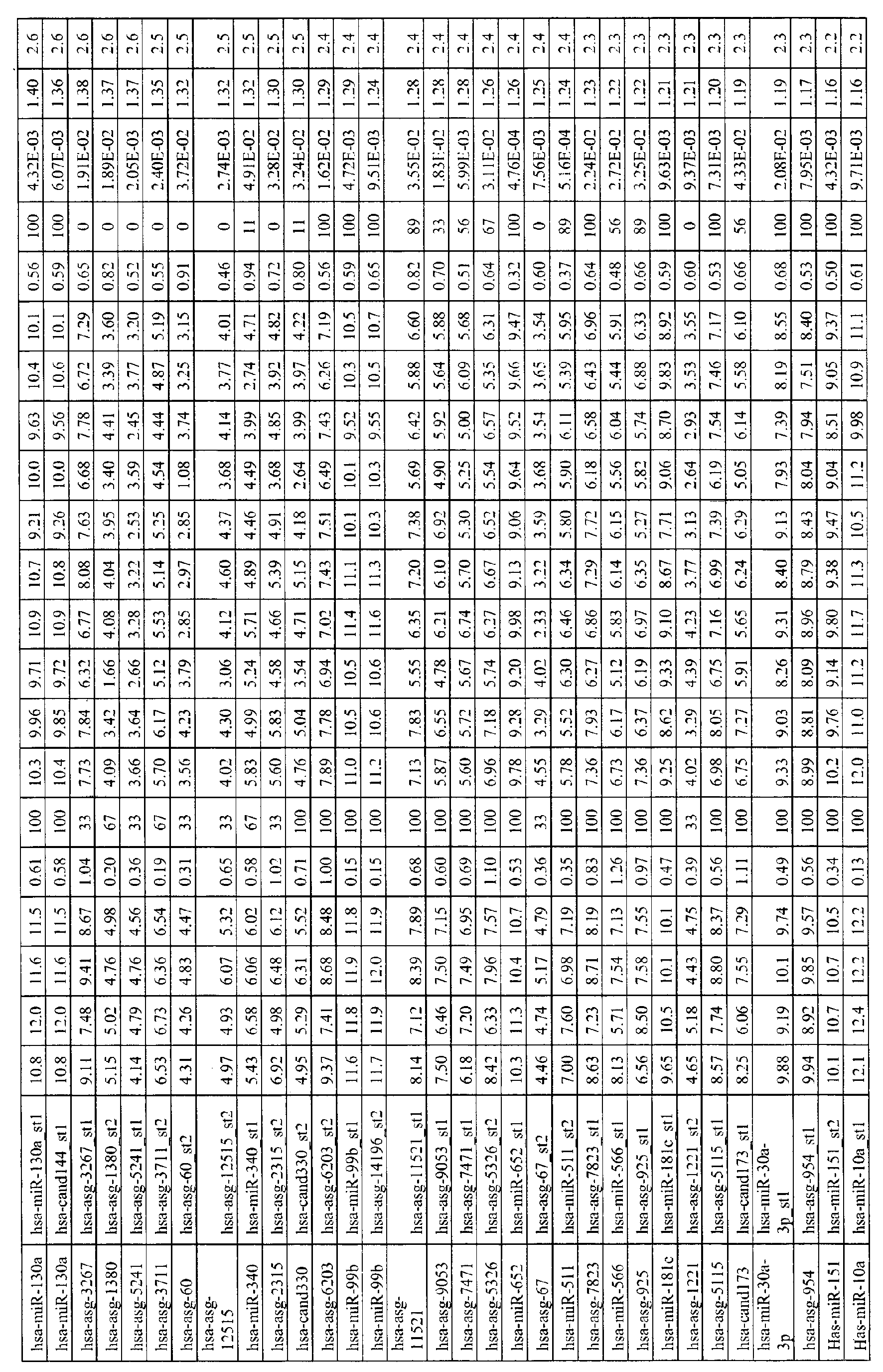

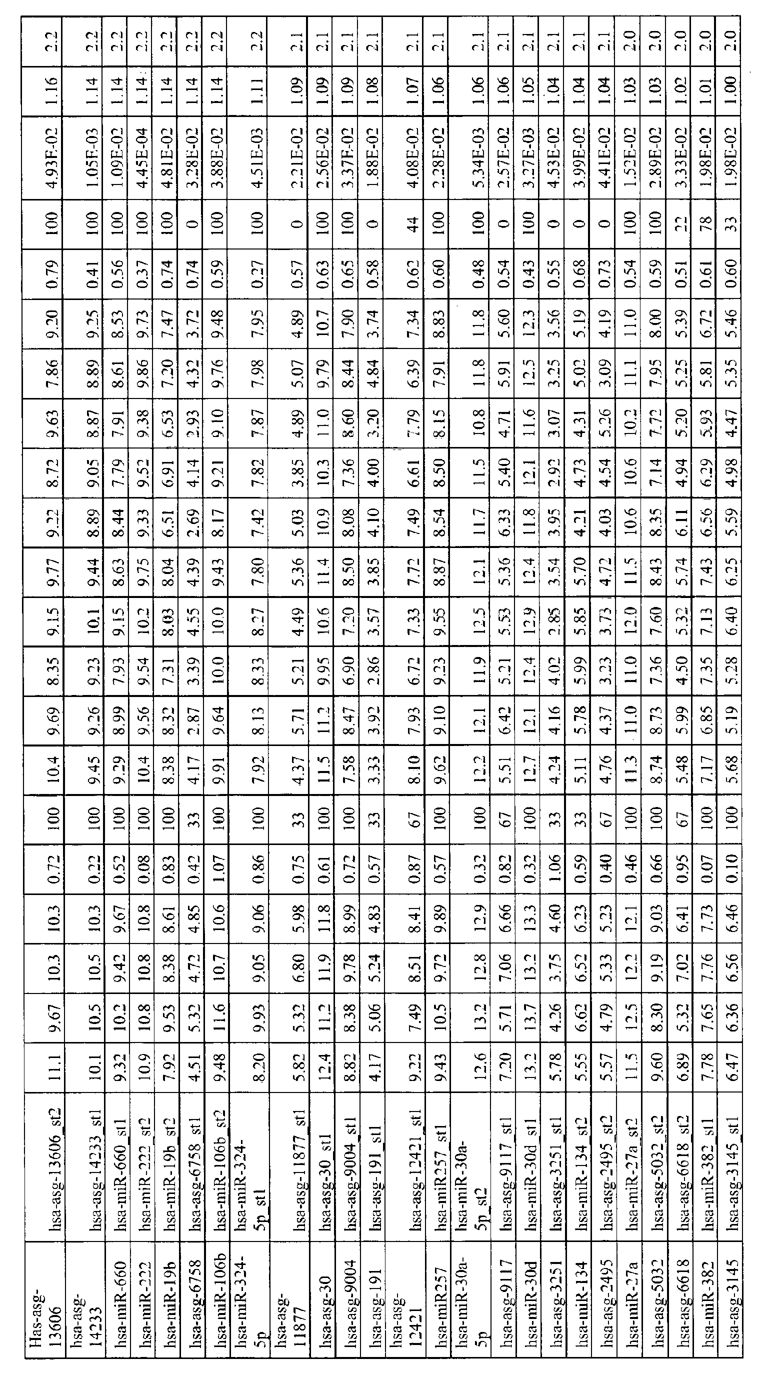

### EXAMPLE 26:

### MIRNAS DIFFERENTALLY EXPRESSED BETWEEN NORMAL PANCREAS TISSUE AND NORMAL LYMPH NODES

To identify miRNAs that may be useful markers for metastasis of cancerous pancreas cells to lymph nodes, the inventors identified miRNAs that are expressed at significantly higher levels in normal pancreas tissue than in cancer-negative lymph nodes.

Total RNA, purified from two separate normal human pancreas tissue samples (FirstChoice^{®} Human Pancreas Total RNA, cat. no. AM7954 Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 195 miRNAs (62 verified, 133 predicted) with significant differential expression between normal pancreas and normal lymph node samples samples (|log2Diff| | pancreas vs LN neg ≥1 and p-value<0.05) (Table 25). Among the predicted miRNAs, hsa-miR-375 and -217 had average expression levels over 800-fold higher in the pancreas samples; hsa-miR-216 and -429 had average expression levels that were between 100- and 200-fold higher in pancreas; hsa-miR-200a, -141 and -200b had average expression levels that were between 50- and 75-fold higher in pancreas; hsa-miR-200c, -96, -605, and - 802) had average expression levels that were between 20- and 40-fold higher in pancreas; 15 miRNAs had average expression levels that were between 10- and 20-fold higher in pancreas; and 20 miRNAs had average expression levels that were between 5- and 10-fold higher in pancreas samples than in normal lymph nodes. Among the predicted miRNAs, hsa-asg-7615_st2, -4913_st1, -14153_st2, and-5618_st1 had average expression levels that were between 50- and 80-fold higher in pancreas samples; 24 miRNA had average expression levels that were between 10-and 40-fold higher in pancreas; and 57 miRNAs had average expression levels that were between 5- and 10-fold higher in pancreas samples than in normal lymph nodes.

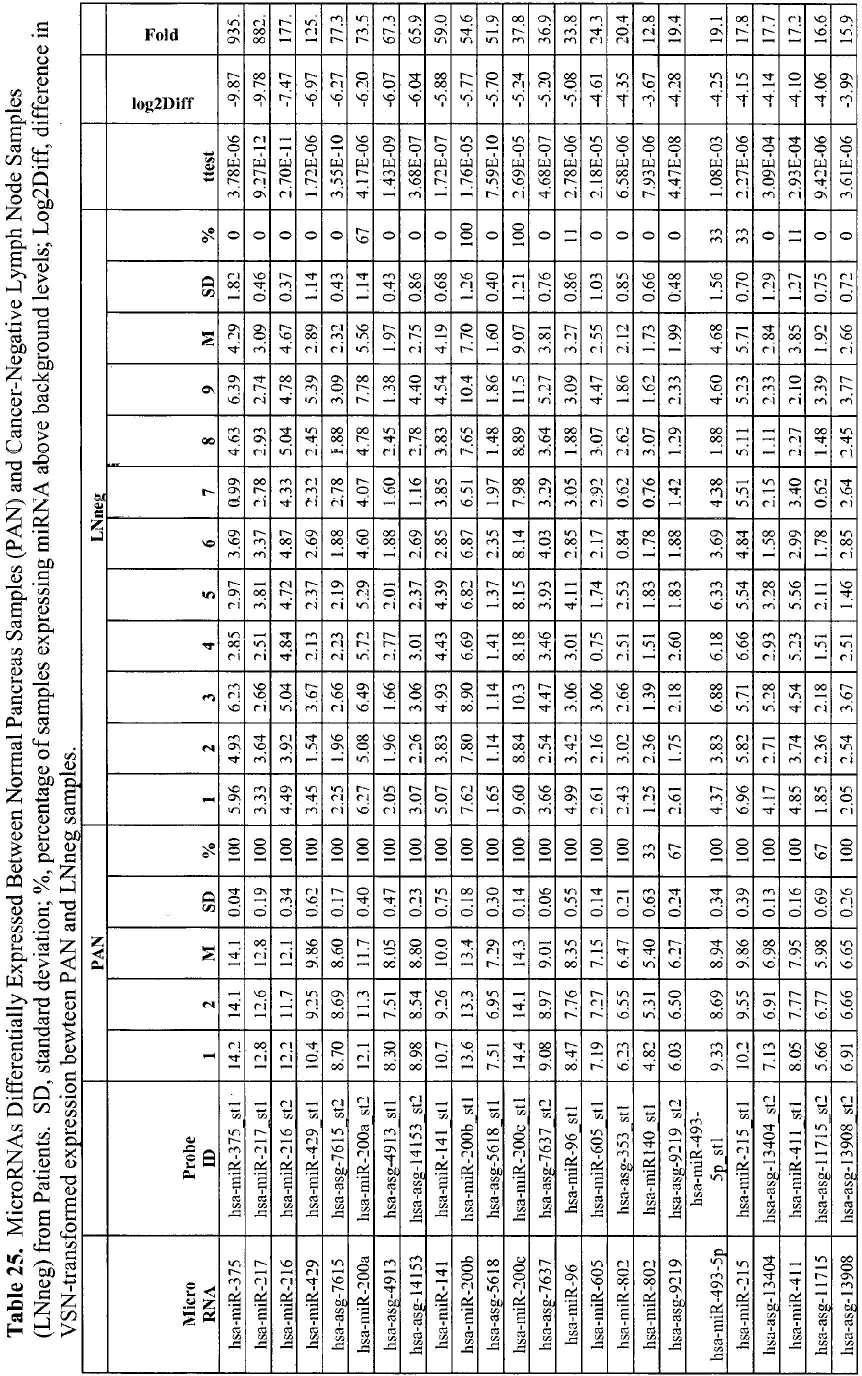

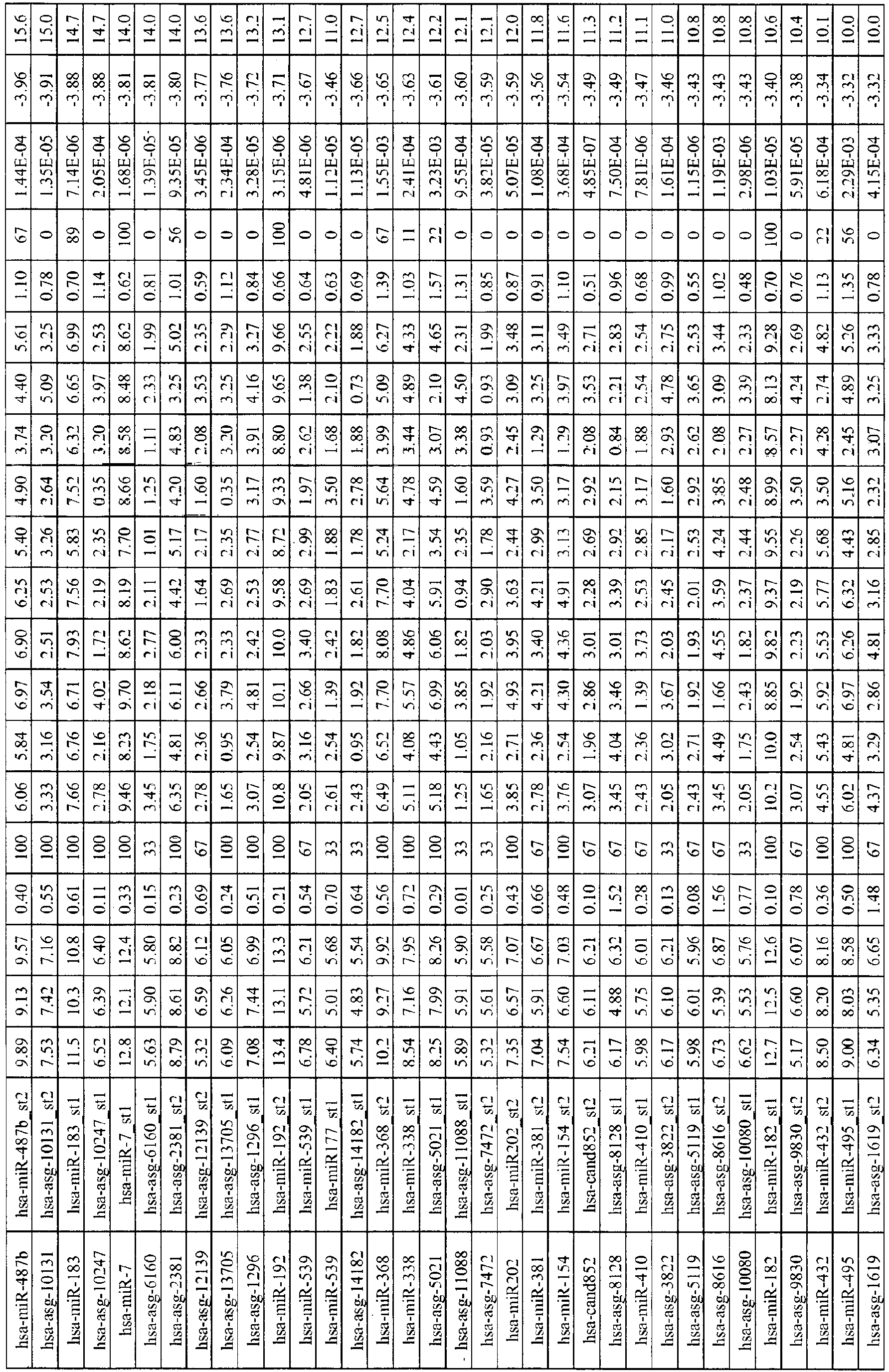

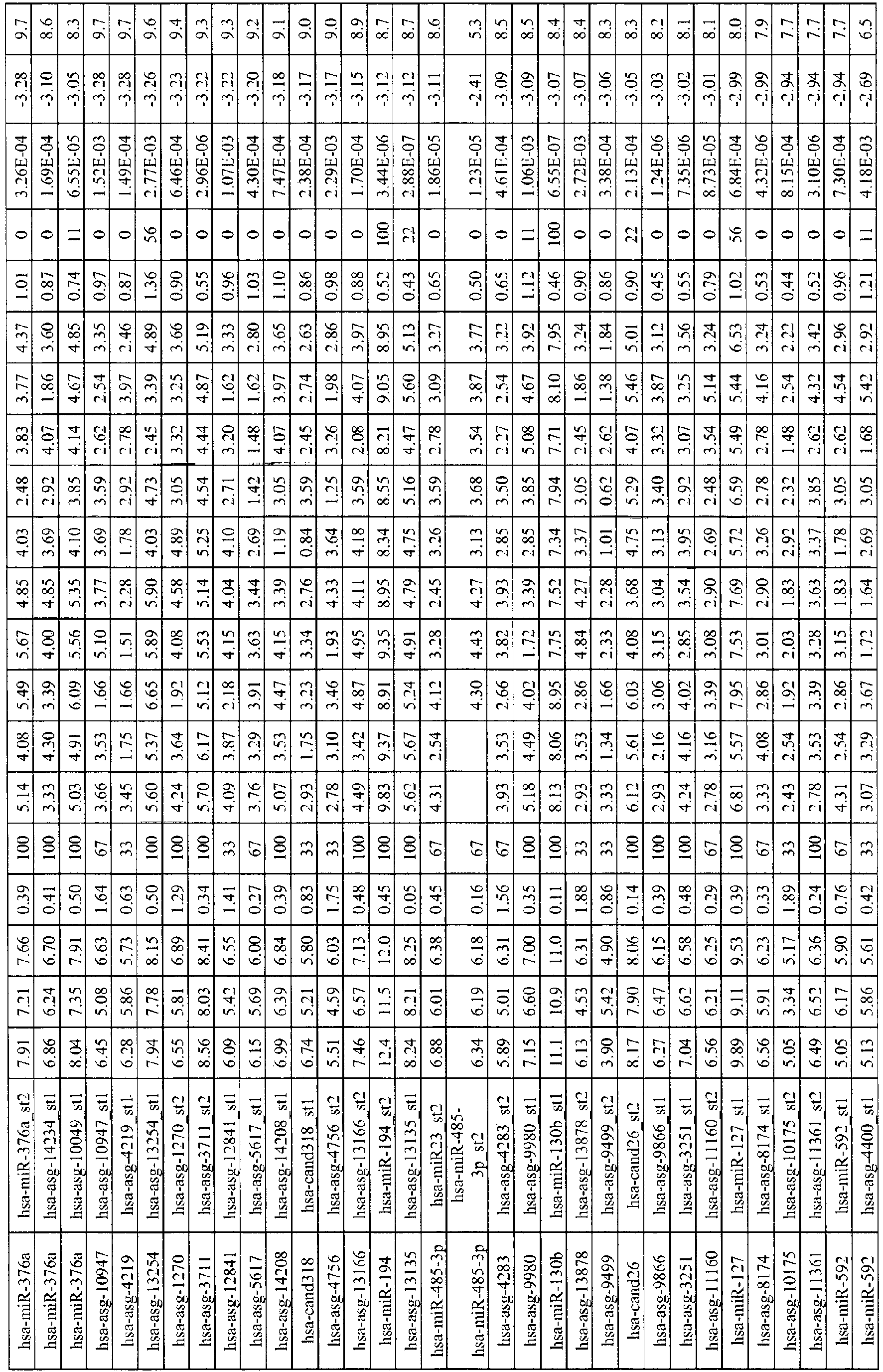

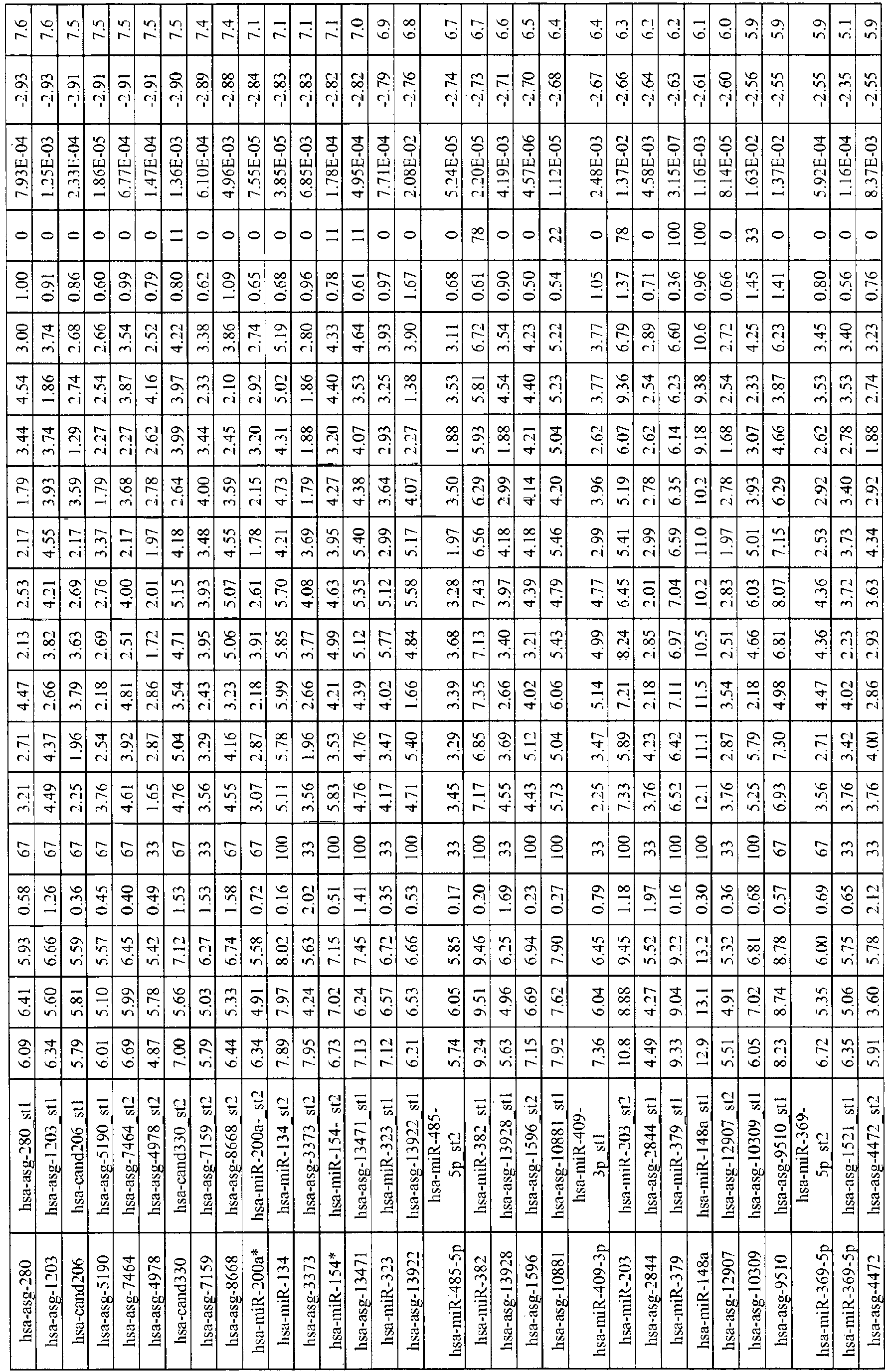

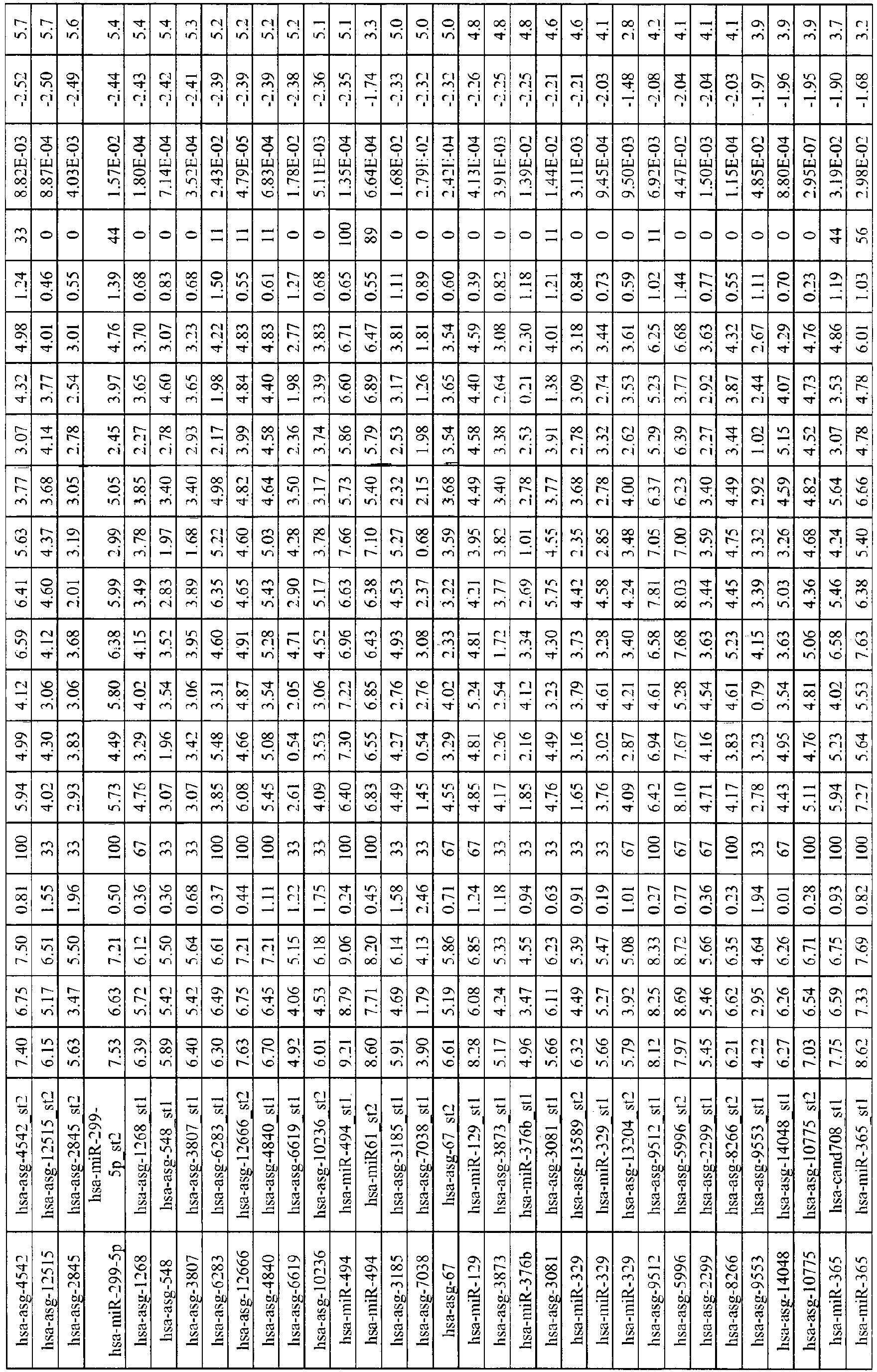

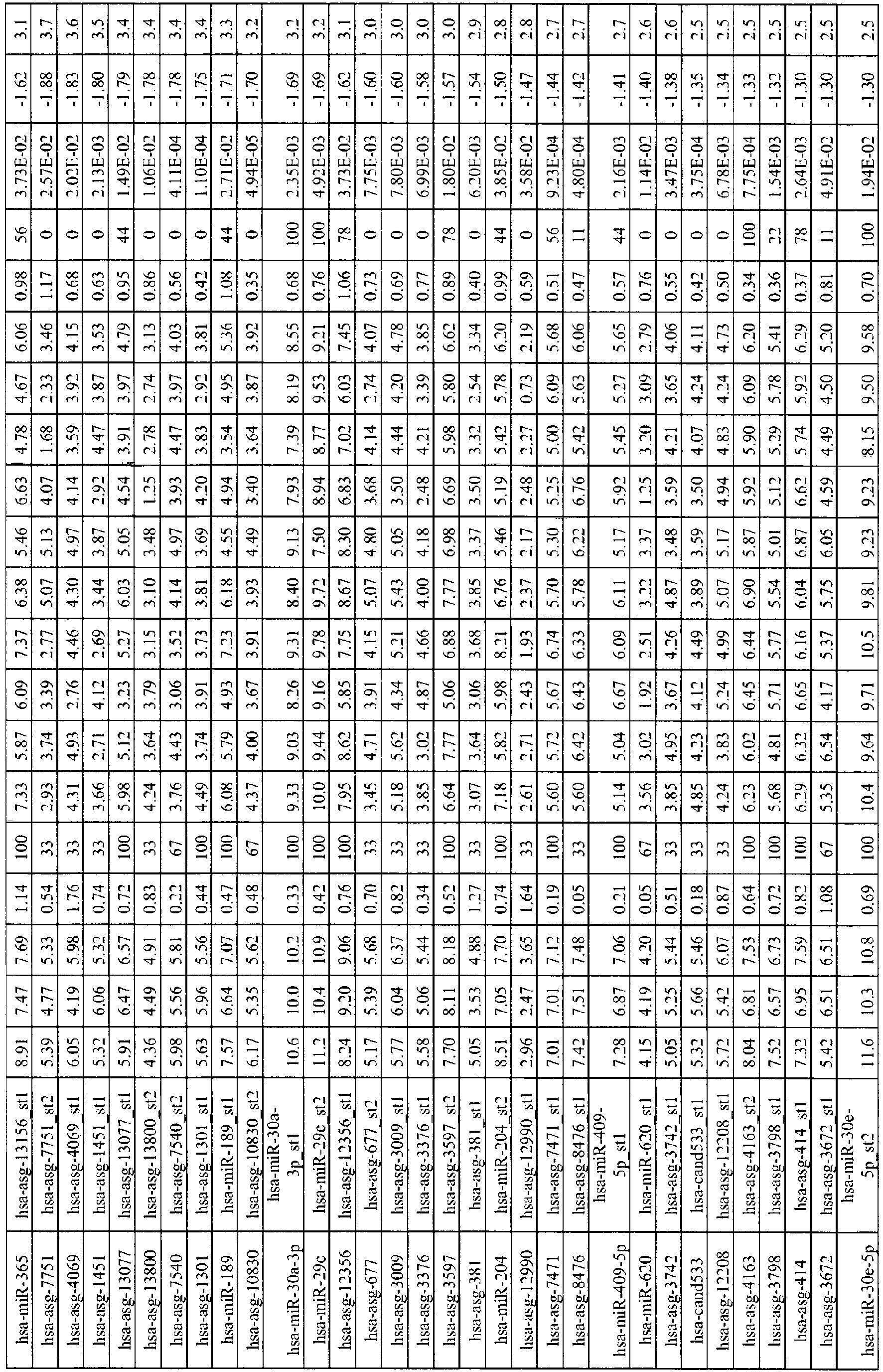

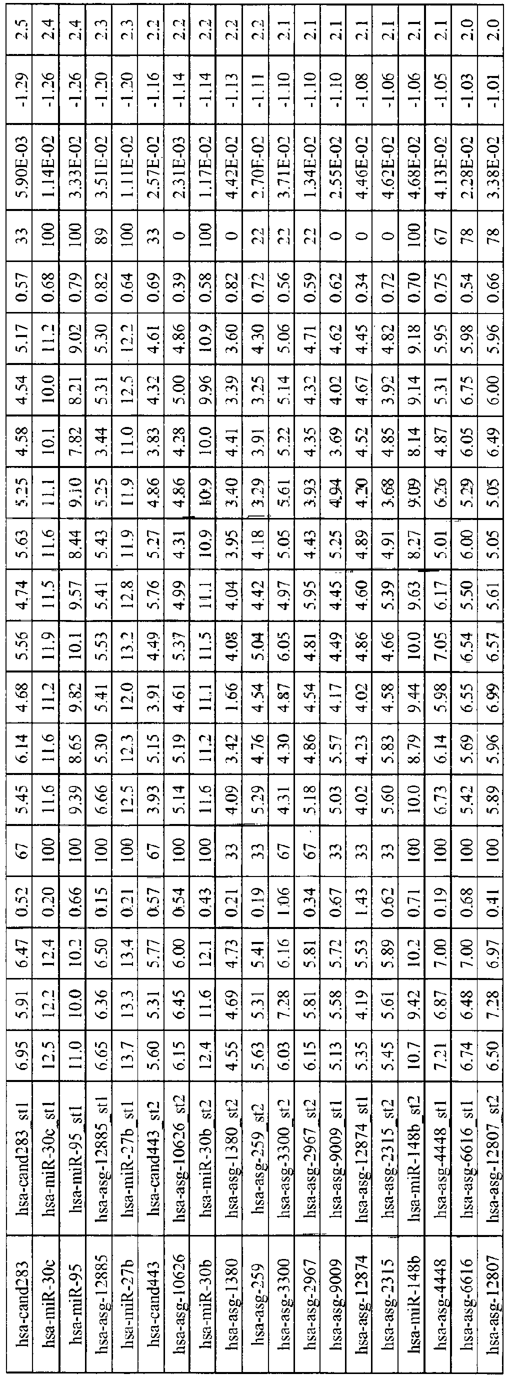

### EXAMPLE 27:

### MIRNAS DIFFERENTALLY EXPRESSED BETWEEN NORMAL THYROID TISSUE AND NORMAL LYMPH NODES

To identify miRNAs that may be useful markers for metastasis of cancerous thyroid cells to lymph nodes, the inventors identified miRNAs that are expressed at significantly higher levels in normal thyroid tissue than in cancer-negative lymph nodes.

Total RNA, purified from one normal human thyroid sample (FirstChoice^{®} Human Thyroid Total RNA, cat. no. AM6872; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 362 miRNA (31 verified, 331 predicted) with significant differential expression between the normal thyroid sample and the normal lymph nodes (|log2Diff| I thyroid vs LN neg ≥1 and p-value<0.05) (Table 26). Among the verified miRNAs, hsa-miR-206 was expressed more than 300-fold higher in the thyroid tissue; hsa-miR-133b, -499, and -133a were expressed between 50- and 100-fold higher in the thyroid sample; eleven miRNAs were expressed between 10- and 50-fold higher in the thyroid sample; and seven miRNAs were expressed between 5- and 10-fold higher in the thyroid tissue than in the normal lymph nodes. Among the predicted miRNAs, hsa-asg-4915_st1 and -2027_st1 were expressed between 25- and 40-fold higher in the thyroid tissue sample; 19 miRNAs were expressed between 10-and 20-fold higher in the thyroid sample; and 112 miRNAs were expressed between 5- and 10-fold higher in the thyroid sample than in normal lymph nodes.

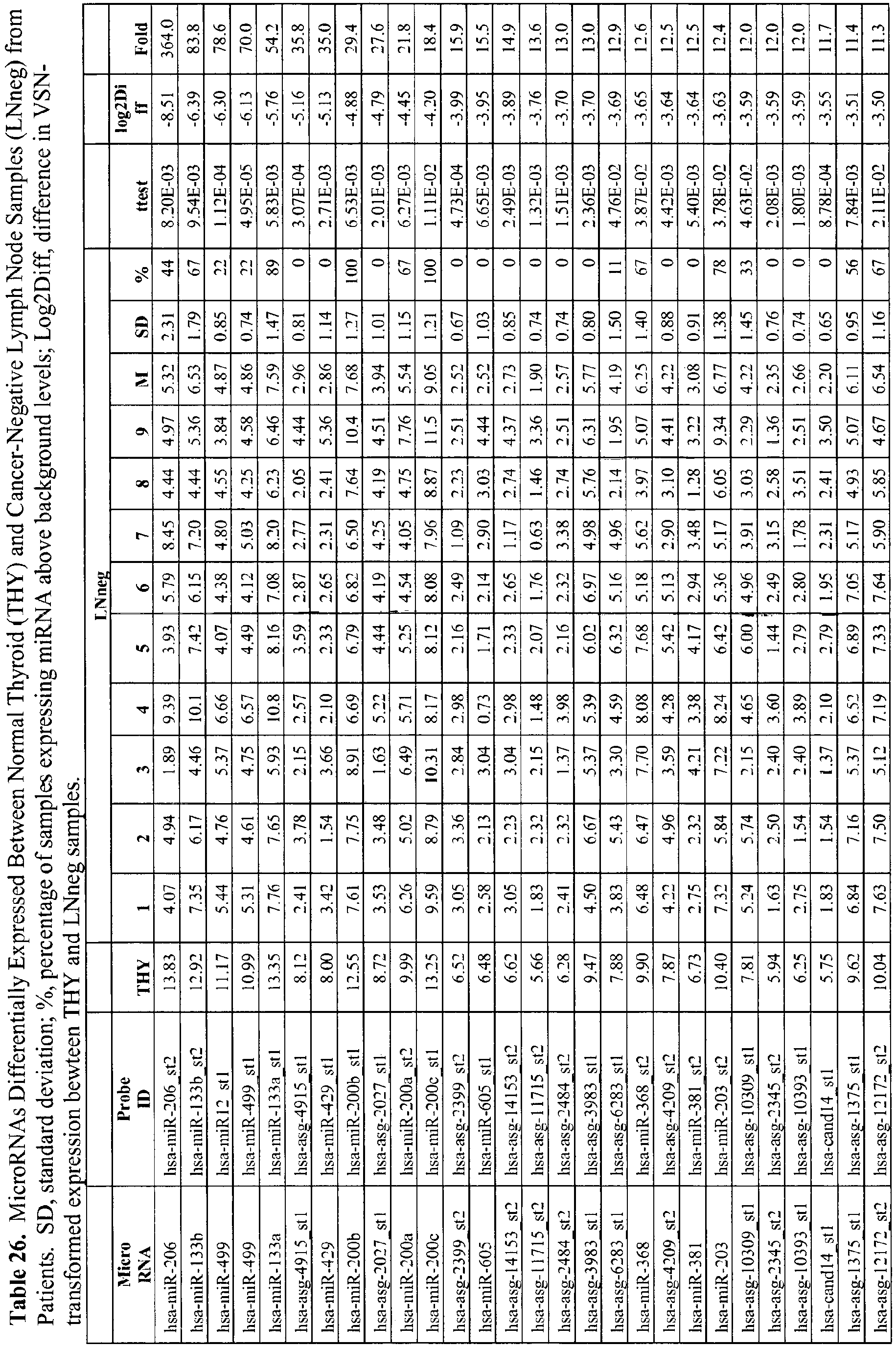

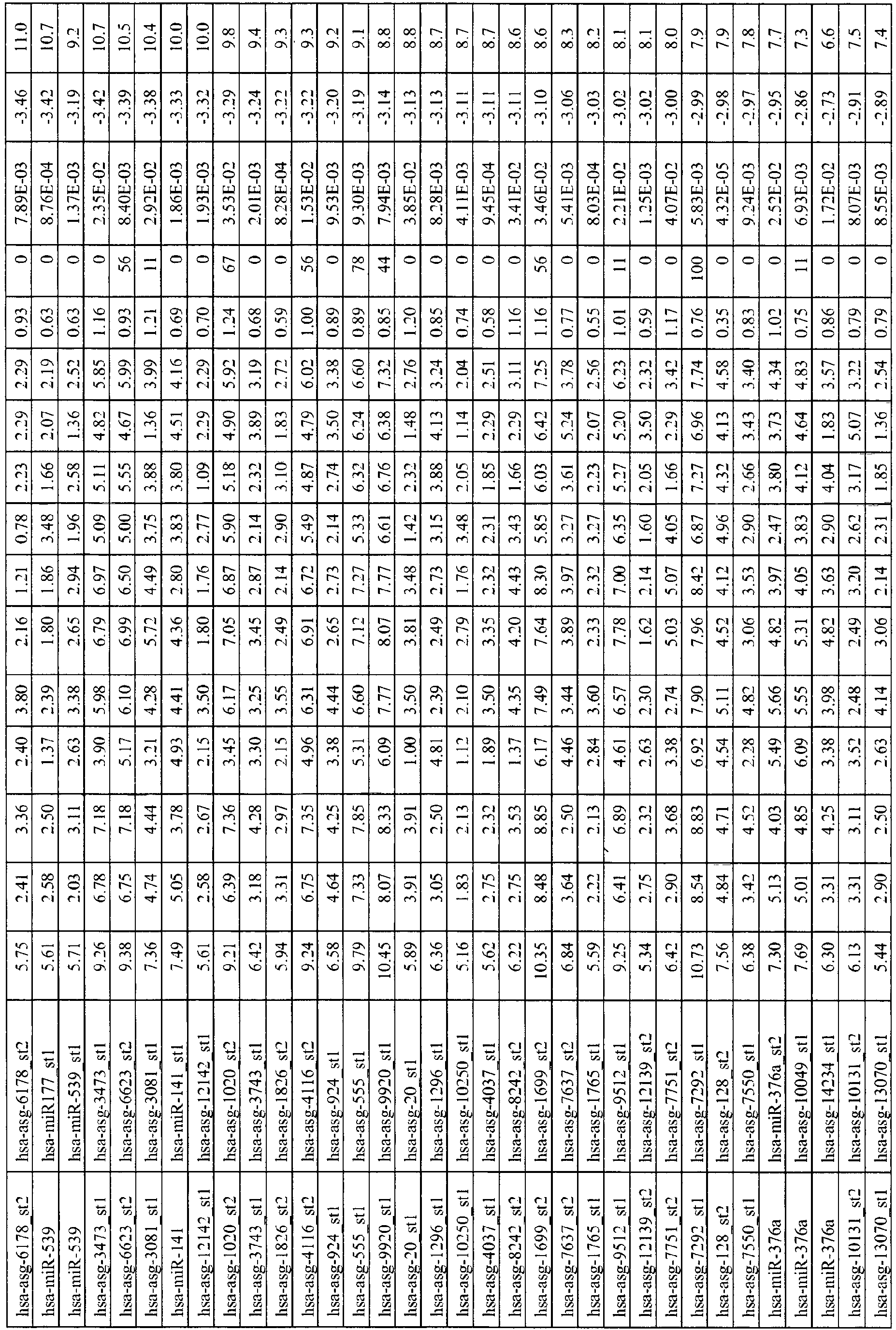

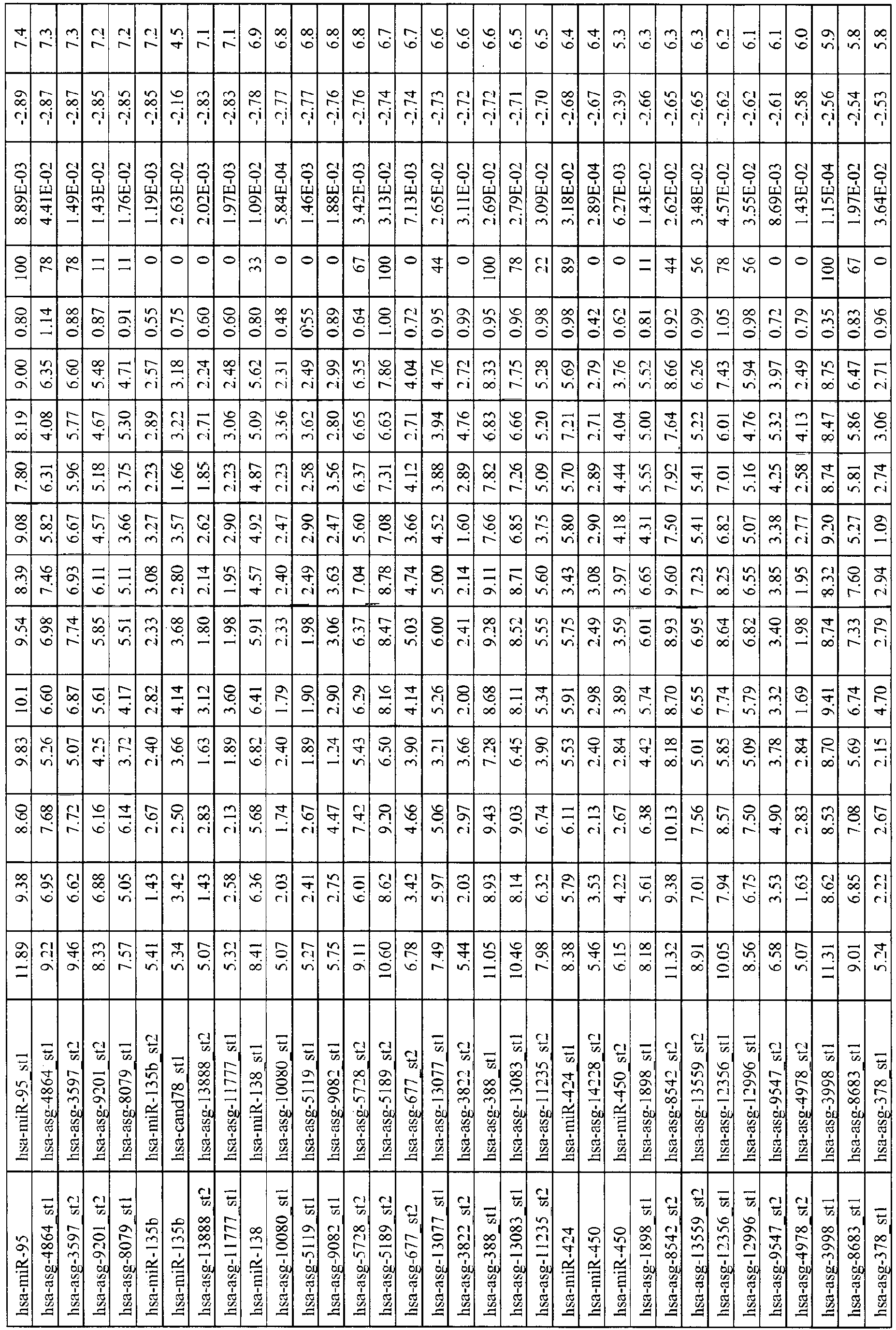

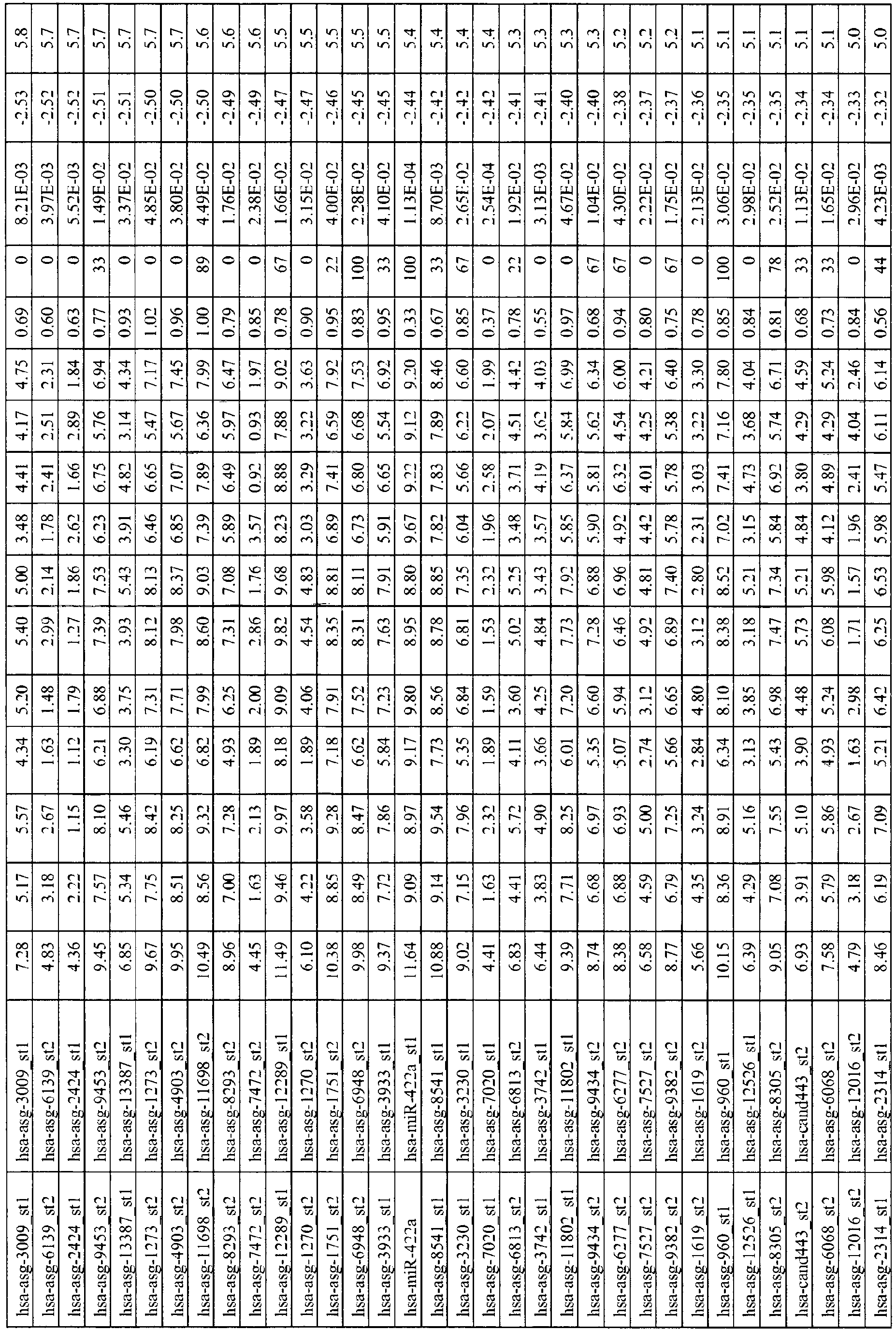

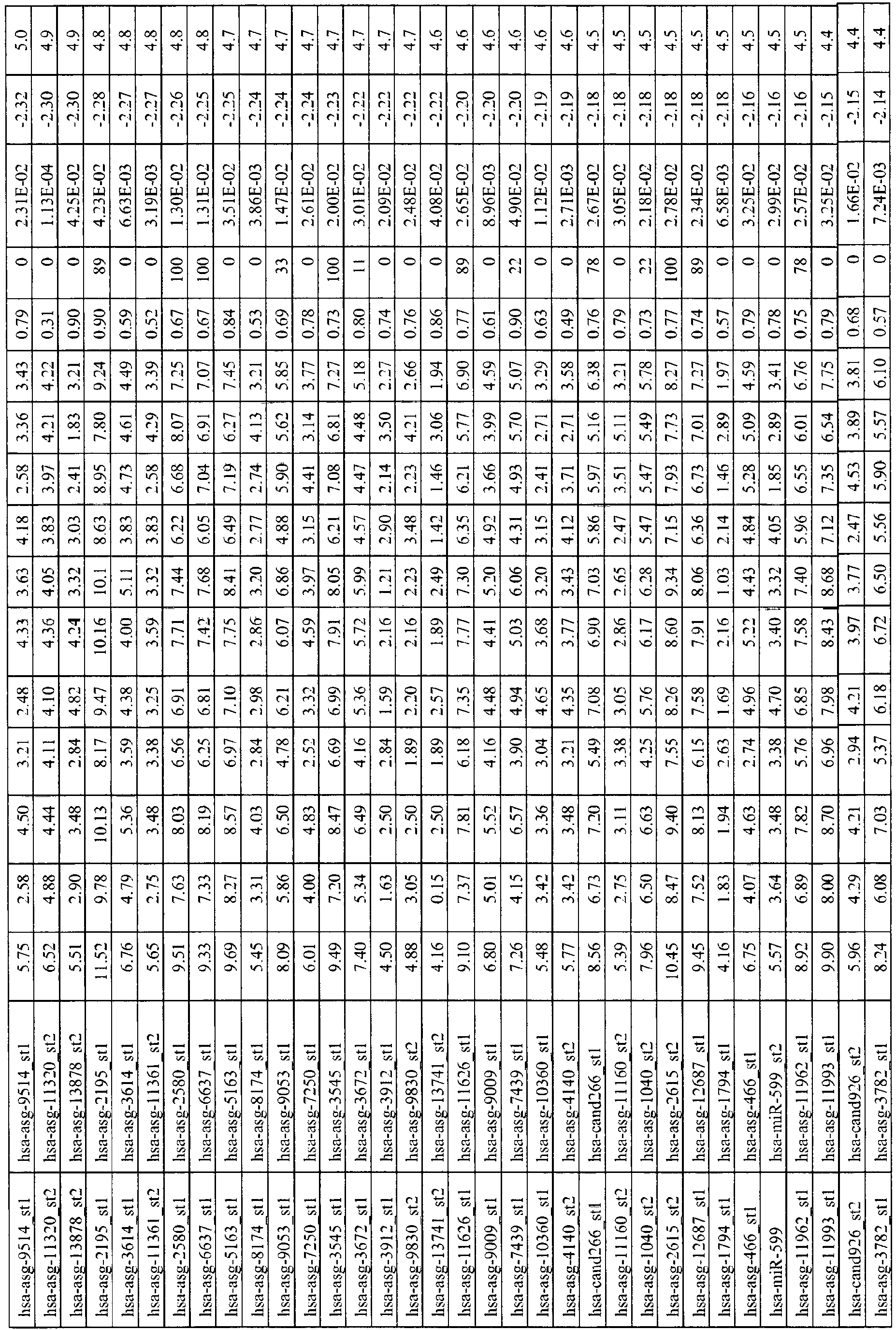

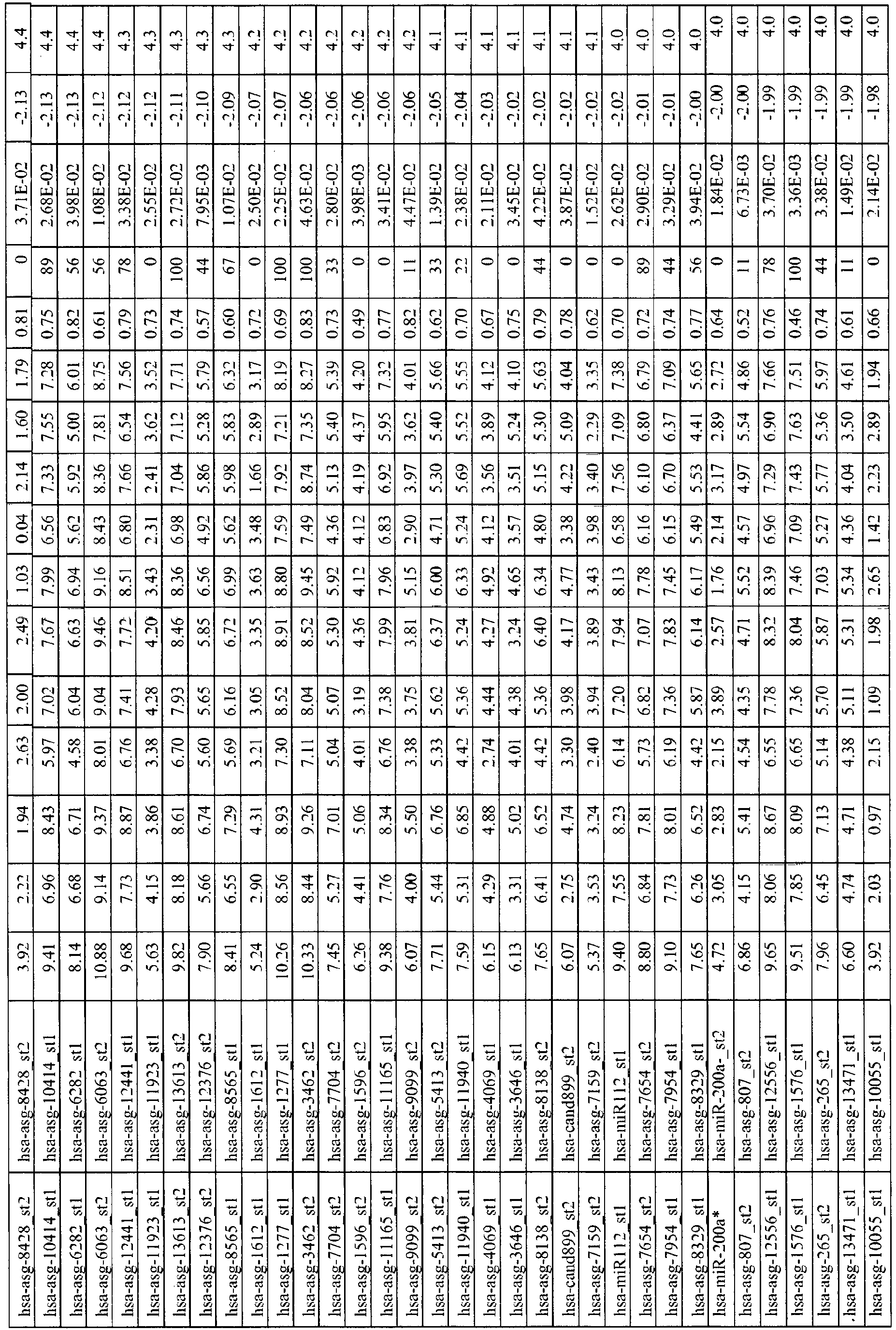

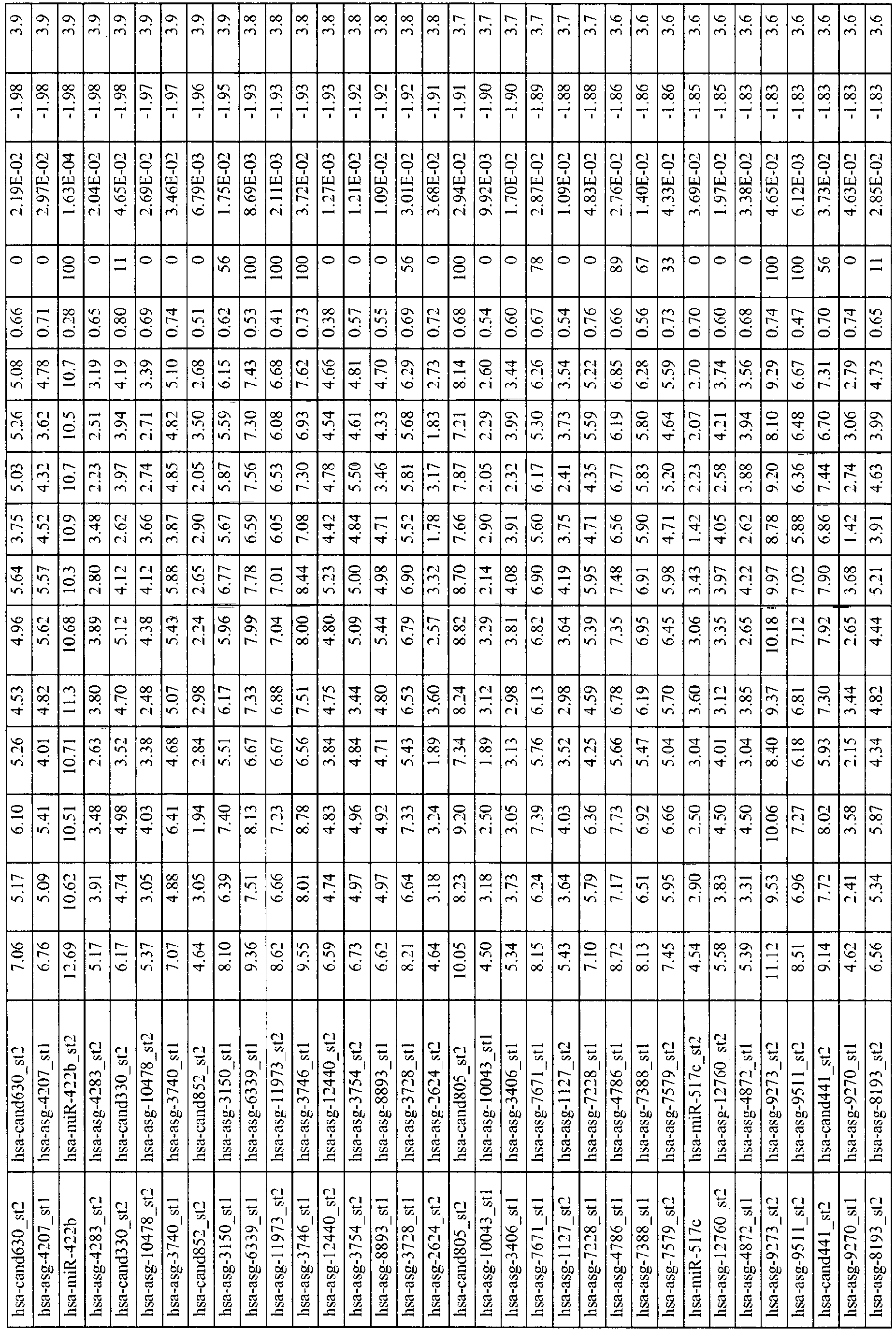

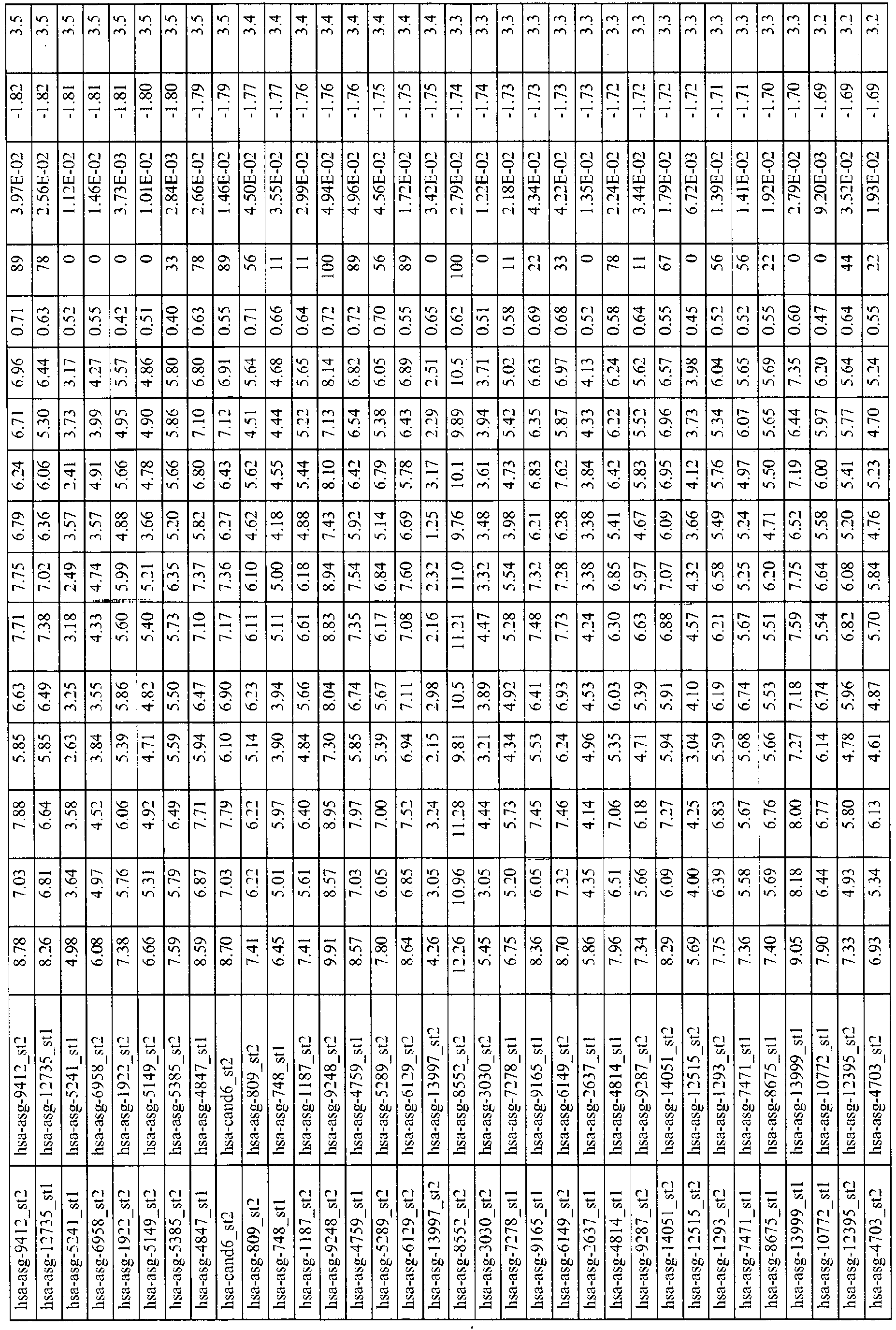

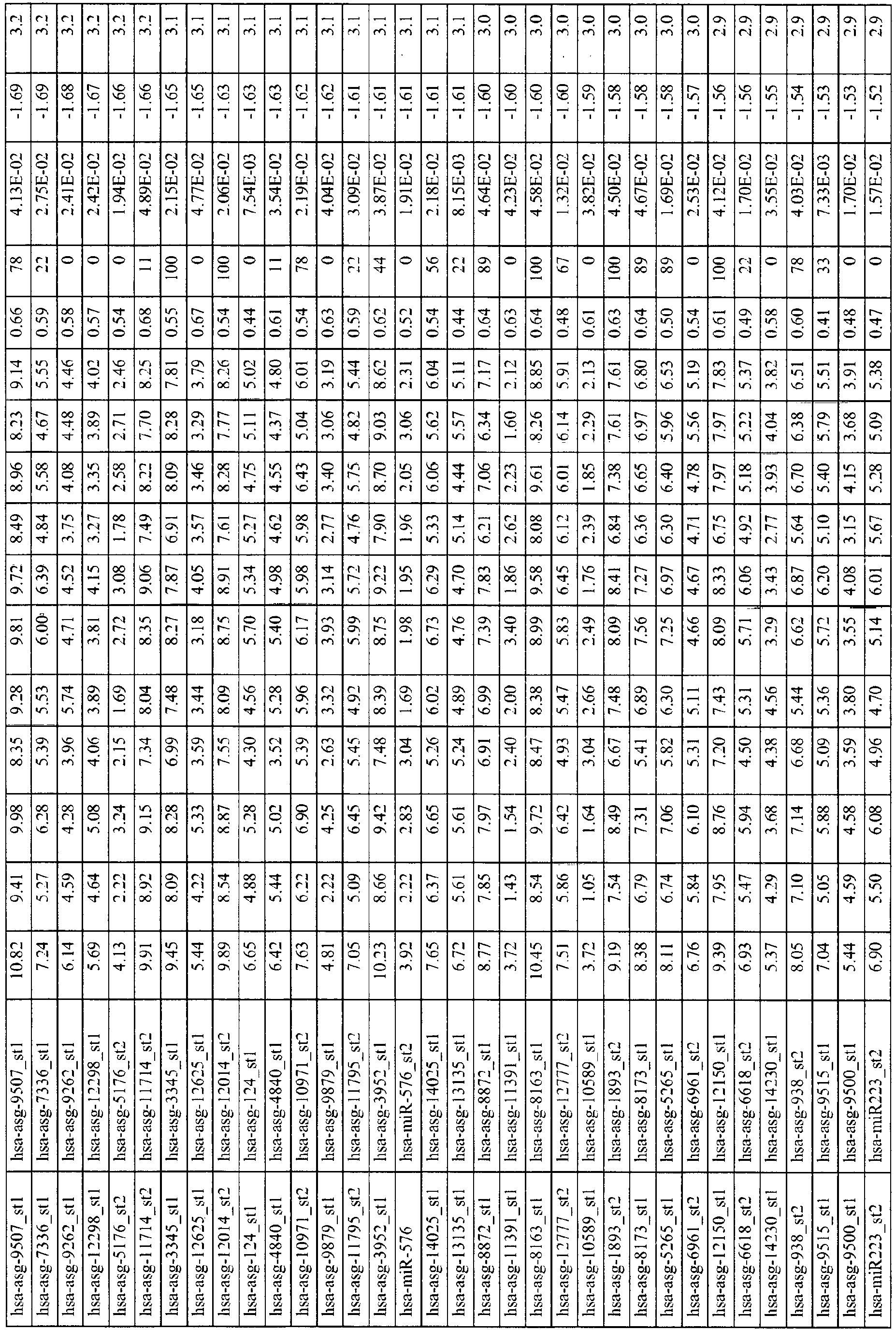

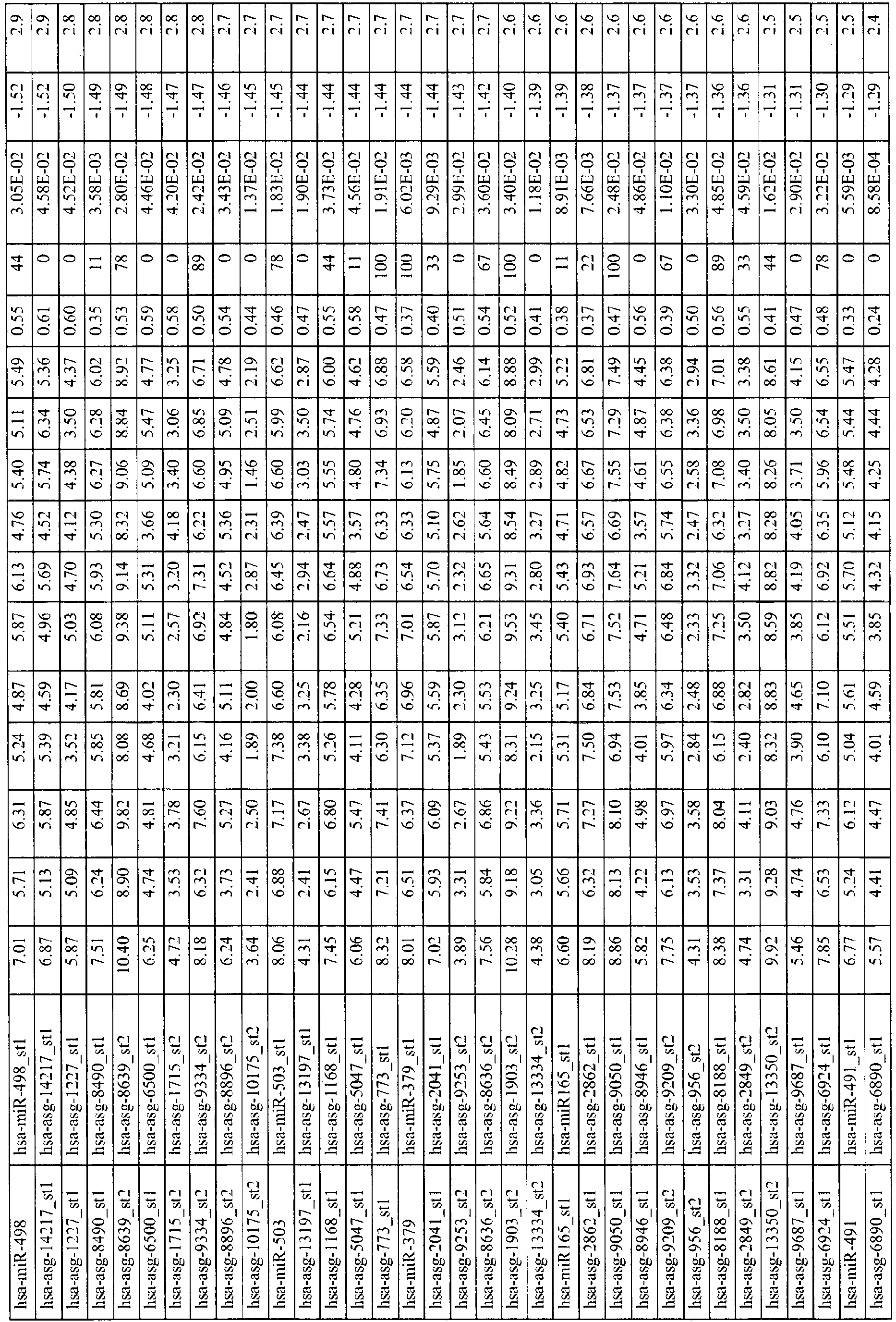

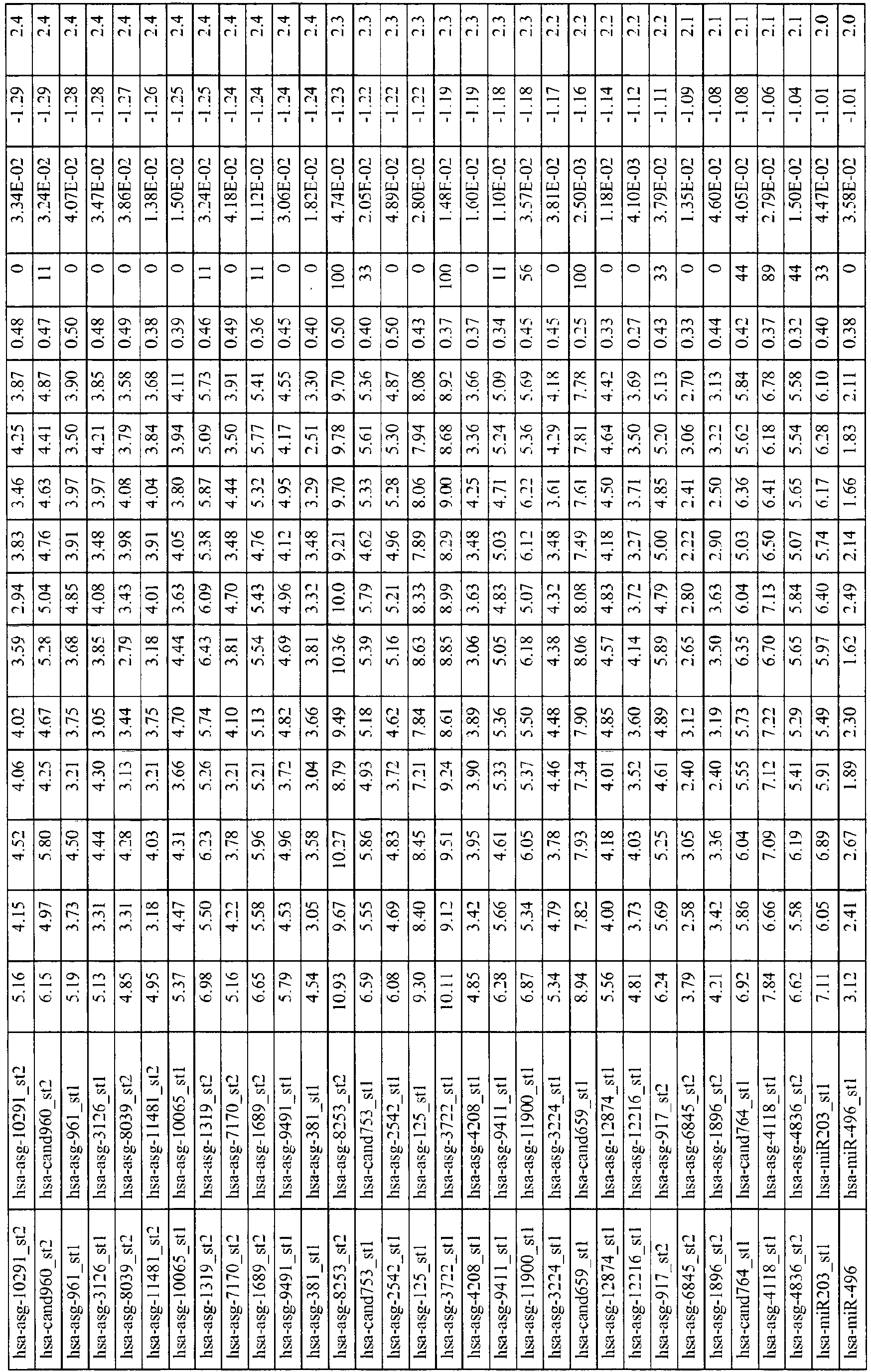

### EXAMPLE 28:

### MIRNAS DIFFERENTALLY EXPRESSED BETWEEN NORMAL KIDNEY TISSUE AND NORMAL LYMPH NODES

To identify miRNAs that may be useful markers for metastasis of cancerous kidney cells to lymph nodes, the inventors identified miRNAs that are expressed at significantly higher levels in normal kidney tissue than in cancer-negative lymph nodes.

Total RNA, purified from one normal human kidney sample (FirstChoice^{®} Human Kidney Total RNA, cat. no. AM7976; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 173 miRNAs (75 verified, 98 predicted) with significant differential expression between the normal kidney and normal lymph node samples (log2Diff kidney vs LN neg ≥1 and p-value<0.05) (Table 27). Among the verified miRNAs, hsa-miR-429 was expressed at a level more than 200-fold higher in the kidney tissue sample; hsa-miR-200a, -187, and -204 were expressed between 50- and 100-fold higher in the kidney sample; hsa-miR-200b, -489, -184 and -215 were expressed between 20- and 50-fold higher in kidney; 16 miRNAs were expressed between 10- and 20-fold higher in kidney; and 24 miRNAs were expressed between 5- and 10-fold higher in the kidney sample than in normal lymph nodes. Among the predicted miRNAs, hsa-asg-4441_st1 expression in the kidney sample was over 50-fold higher than in the normal lymph node samples; hsa-miR-14182_st1, -14153_st2, -7637_st2 and -4915_st1 were expressed between 20-and 50-fold higher in the kidney sample; 11 miRNAs were expressed between 10- and 20-fold higher in kidney; and 28 miRNAs were expressed between 5- and 10-fold higher in the kideny sample than in the normal lymph nodes.

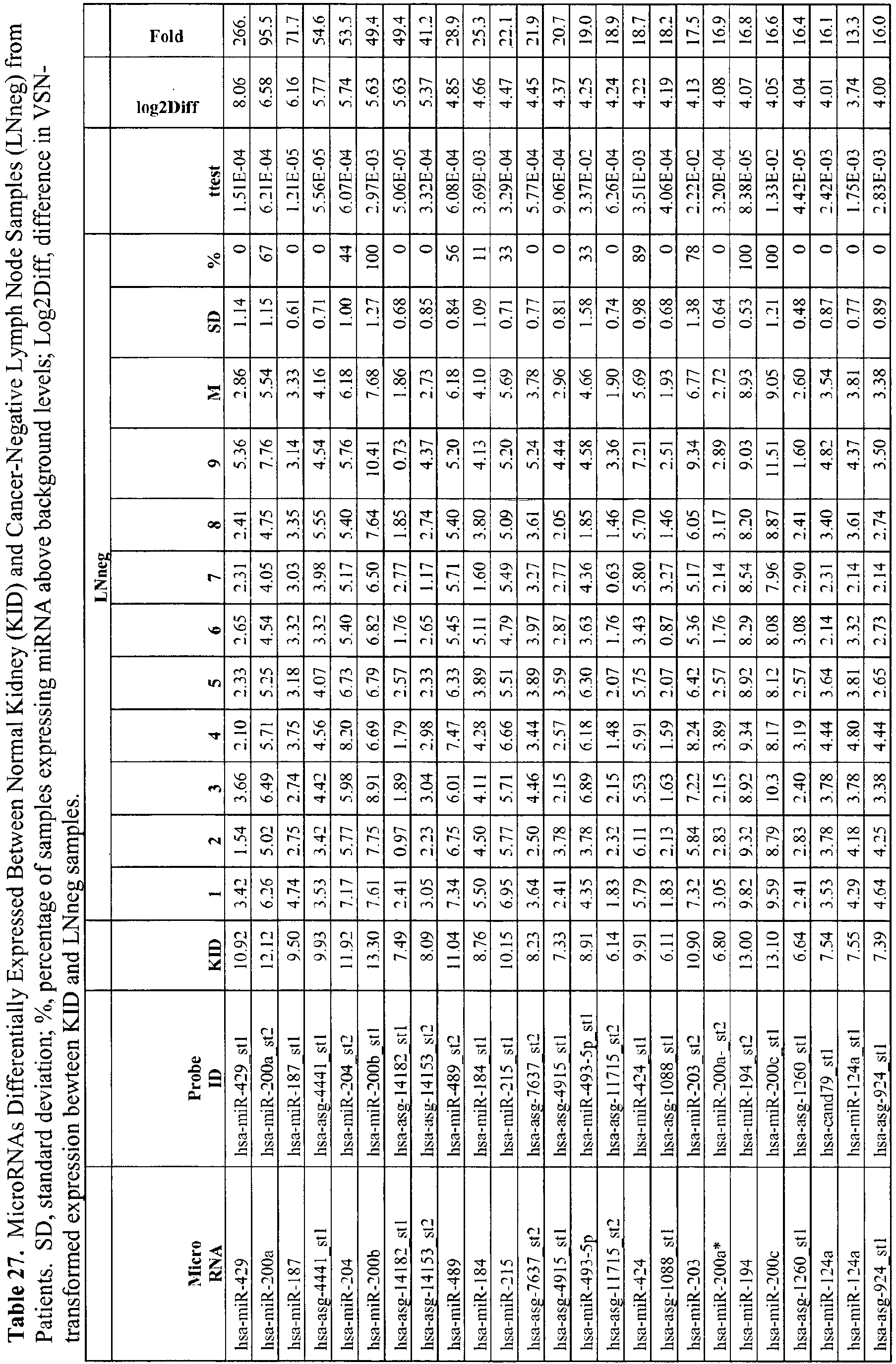

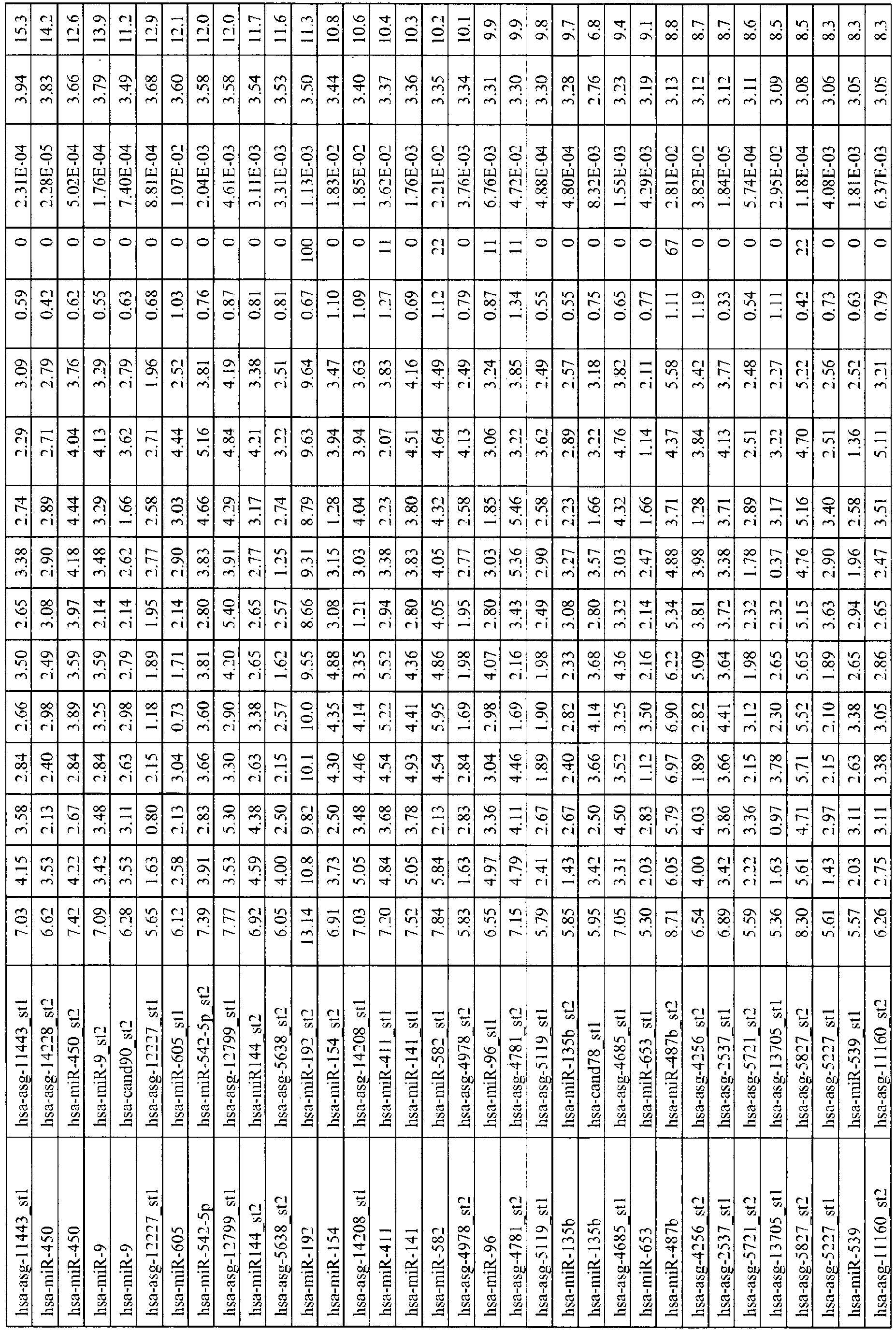

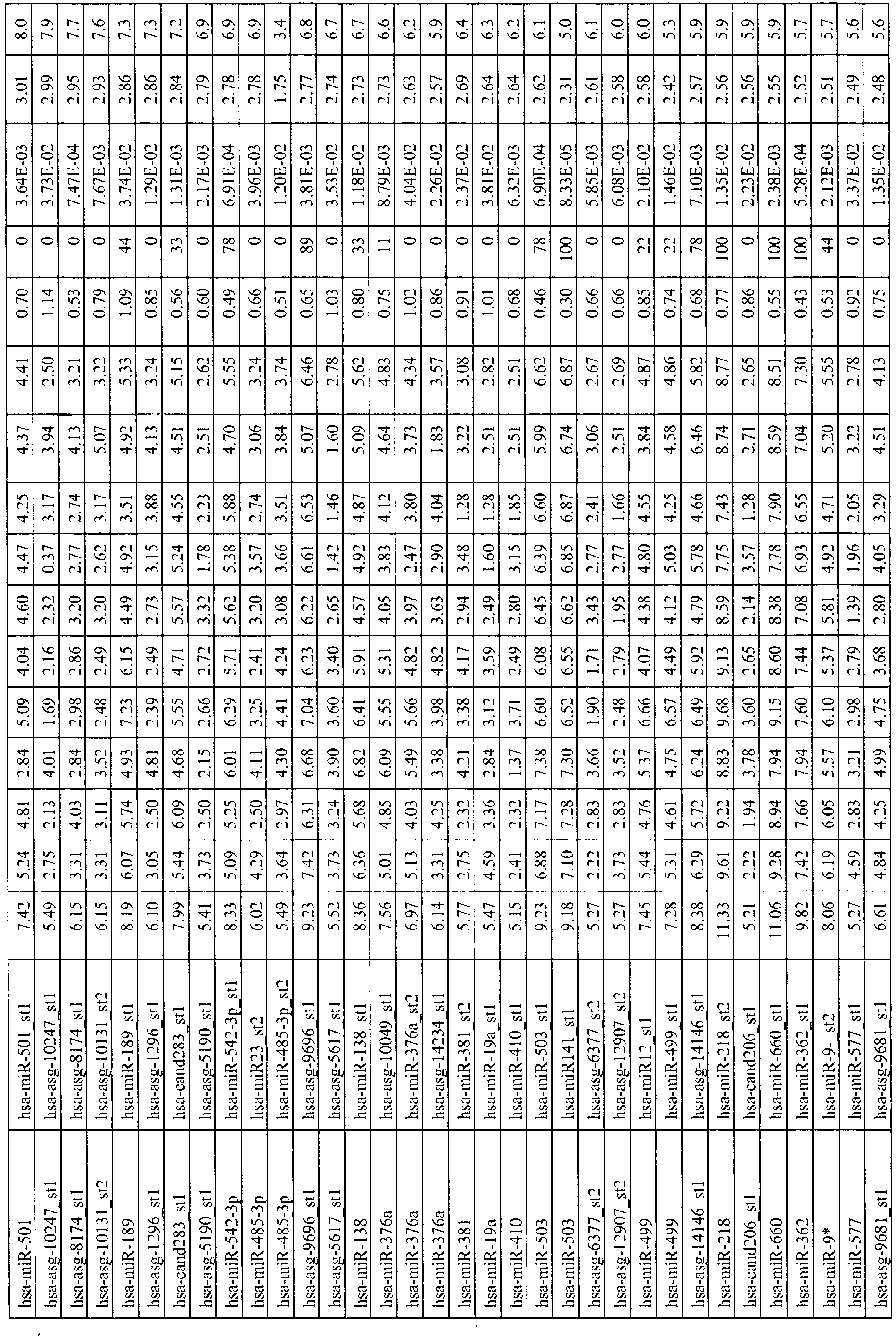

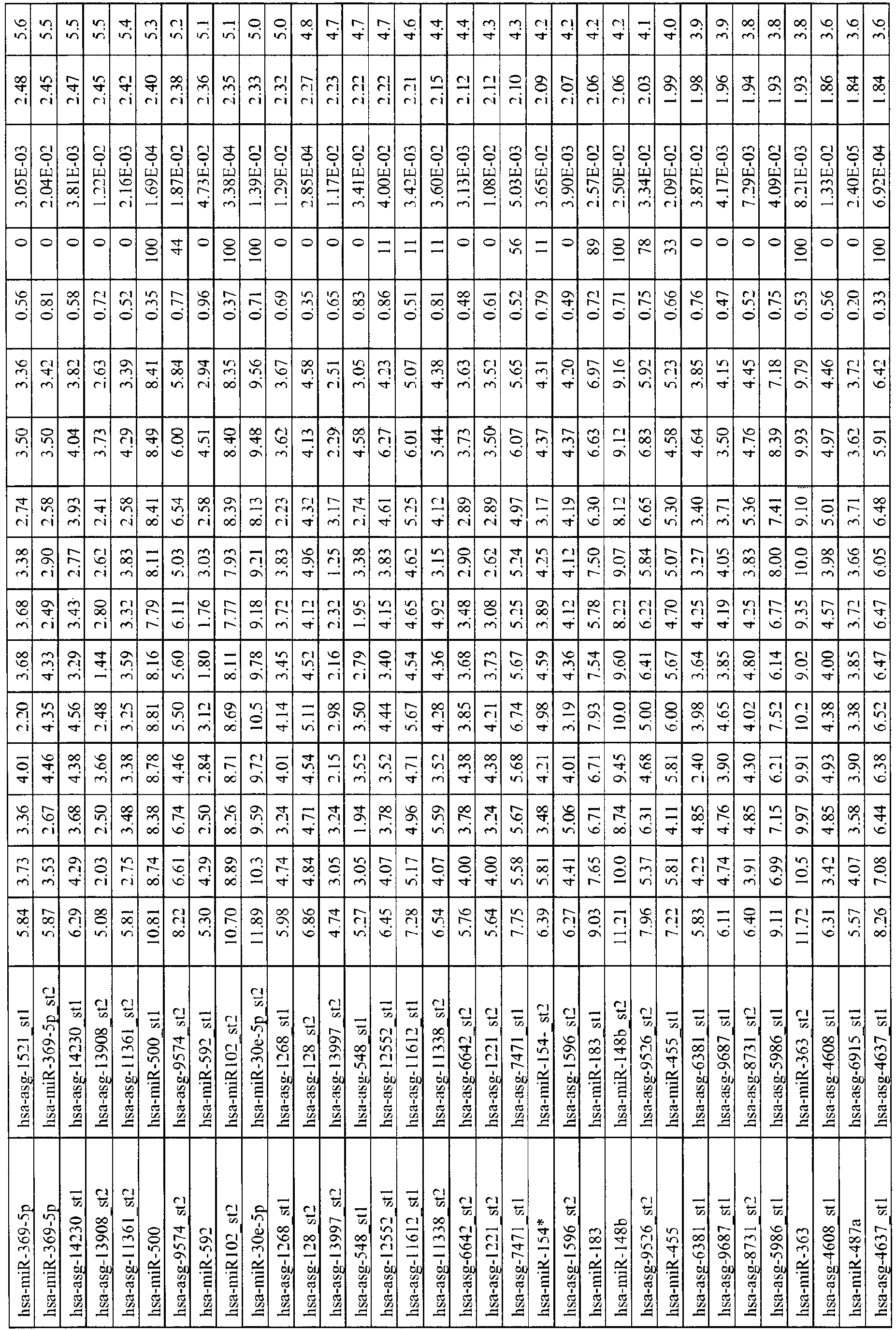

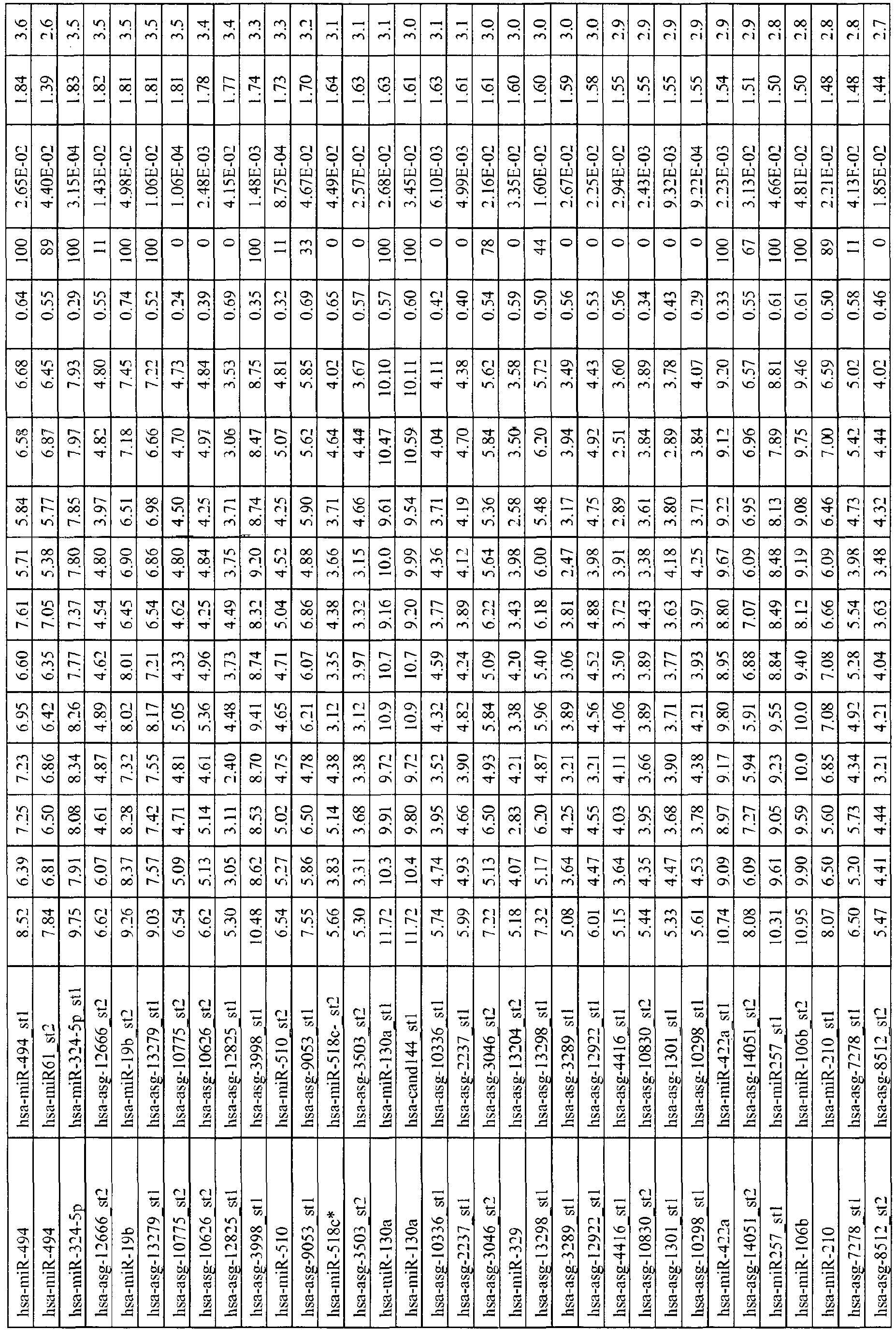

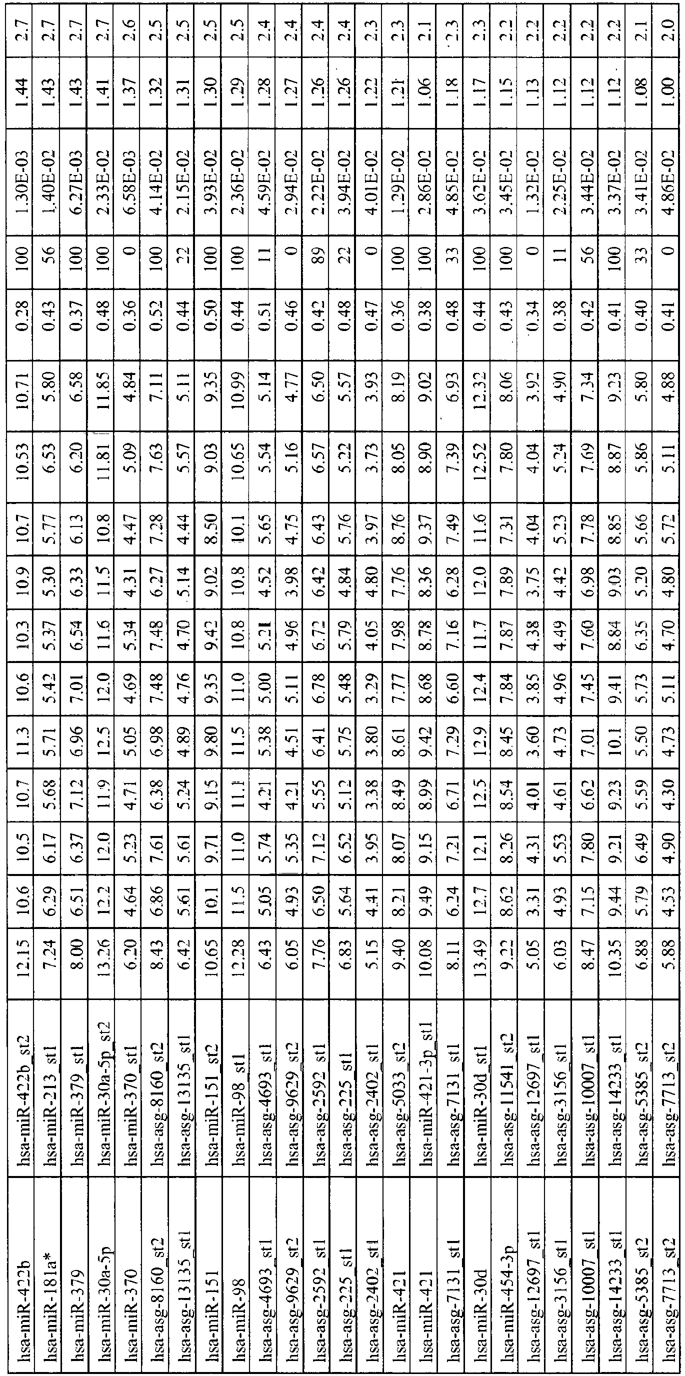

### EXAMPLE 29:

### MIRNAS DIFFERENTALLY EXPRESSED BETWEEN NORMAL STOMACH TISSUE AND NORMAL LYMPH NODES

To identify miRNAs that may be useful markers for metastasis of cancerous stomach cells to lymph nodes, the inventors identified miRNAs that are expressed at significantly higher levels in normal stomach tissue than in cancer-negative lymph nodes.

Total RNA, purified from one normal human stomach sample (FirstChoice^{®} Human Stomach Total RNA, cat. no. AM7996; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 99 miRNAs (35 verified, 64 predicted) with significant differential expression between the normal stomach sample and normal lymph nodes (|log2Diff| I stomach vs LN neg ≥1 and p-value<0.05) (Table 28). Among the verified miRNAs, hsa-miR-375 showed an expression level over 600-fold higher in the stomach sample; hsa-miR-429 and -200a were expressed at levels between 100- and 200-fold higher in the stomach sample; 7 miRNAs (hsa-miR-205, -200b, -203, -141, -200a*, -200c and -605) were expressed between 20- and 90-fold higher in stomach; 6 miRNAs were expressed between 10- and 20-fold higher in stomach; and 11 miRNAs were expressed between 5- and 10-fold higher in the stomach sample than in normal lymph node samples. Among the predicted miRNAs, hsa-asg-14153_st2 was expressed at a level over 100-fold higher in the stomach sample; 5 miRNAs (hsa-asg-7637_st2, -924_st1, -11715_st2, -14182_st1, and - 12227_st1) were expressed between 20-and 60-fold higher in stomach; 14 miRNAs were expressed between 10- and 20-fold higher in stomach; and 21 miRNAs were expressed between 5- and 10-fold higher in the stomach sample than in normal lymph nodes.

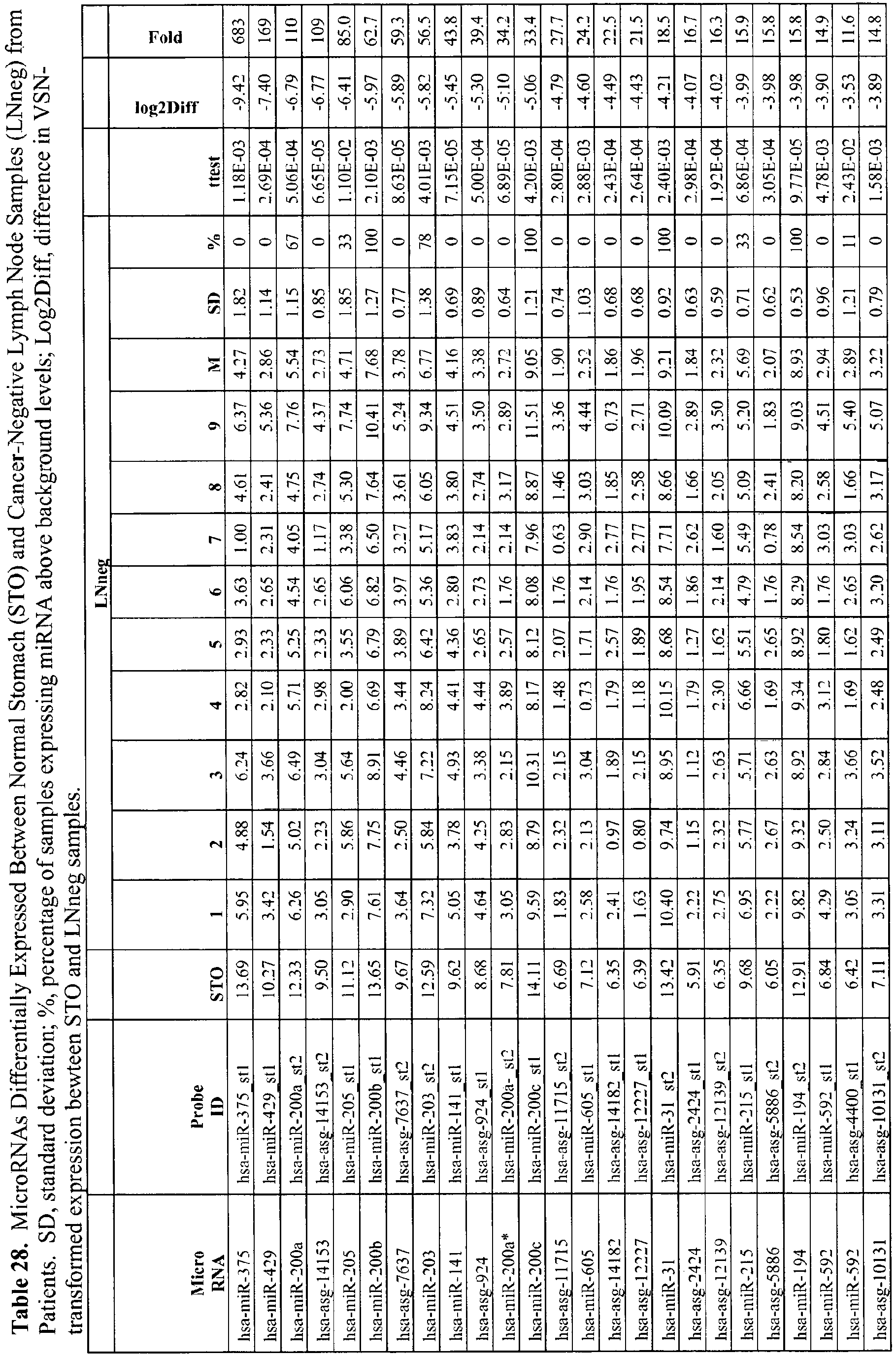

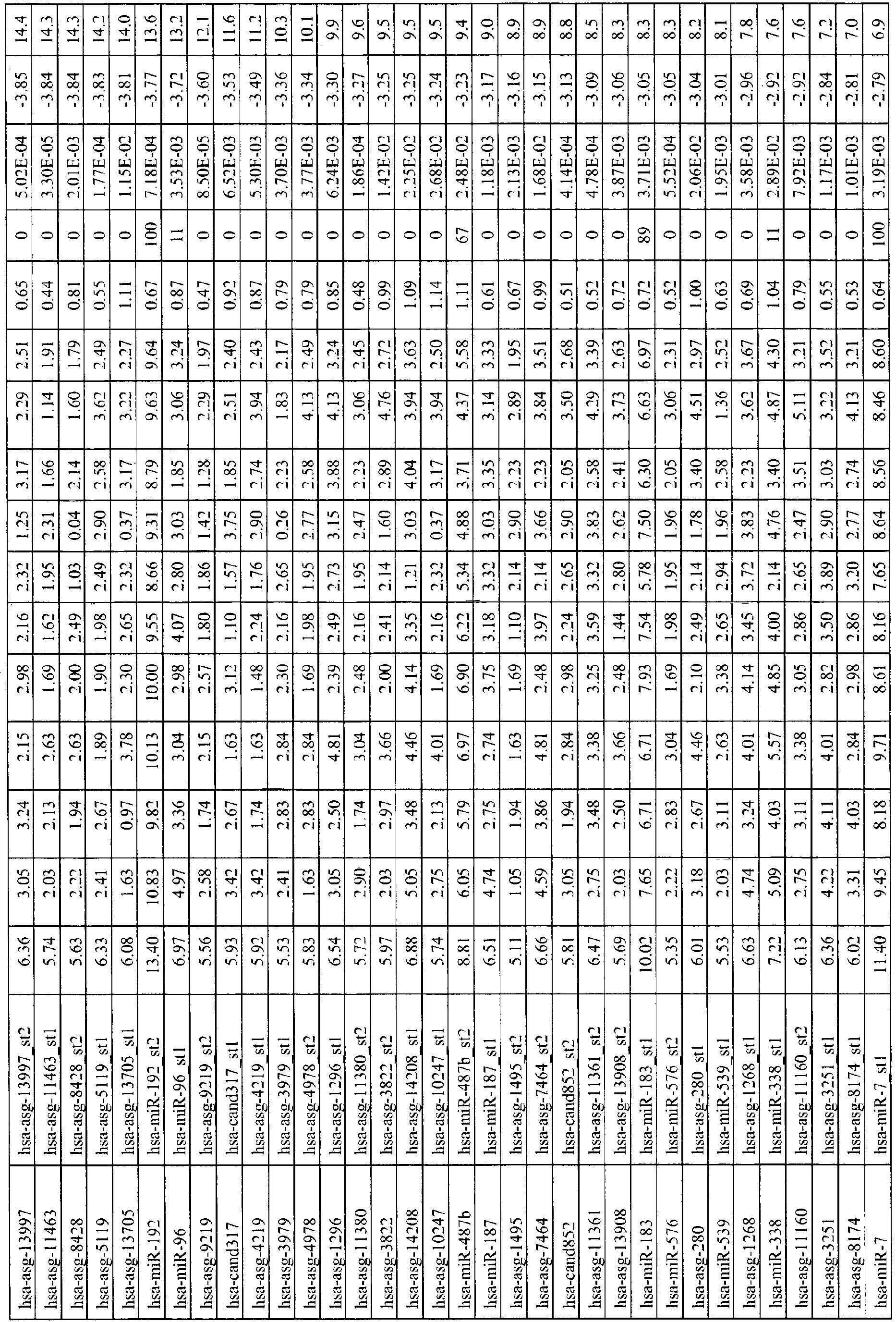

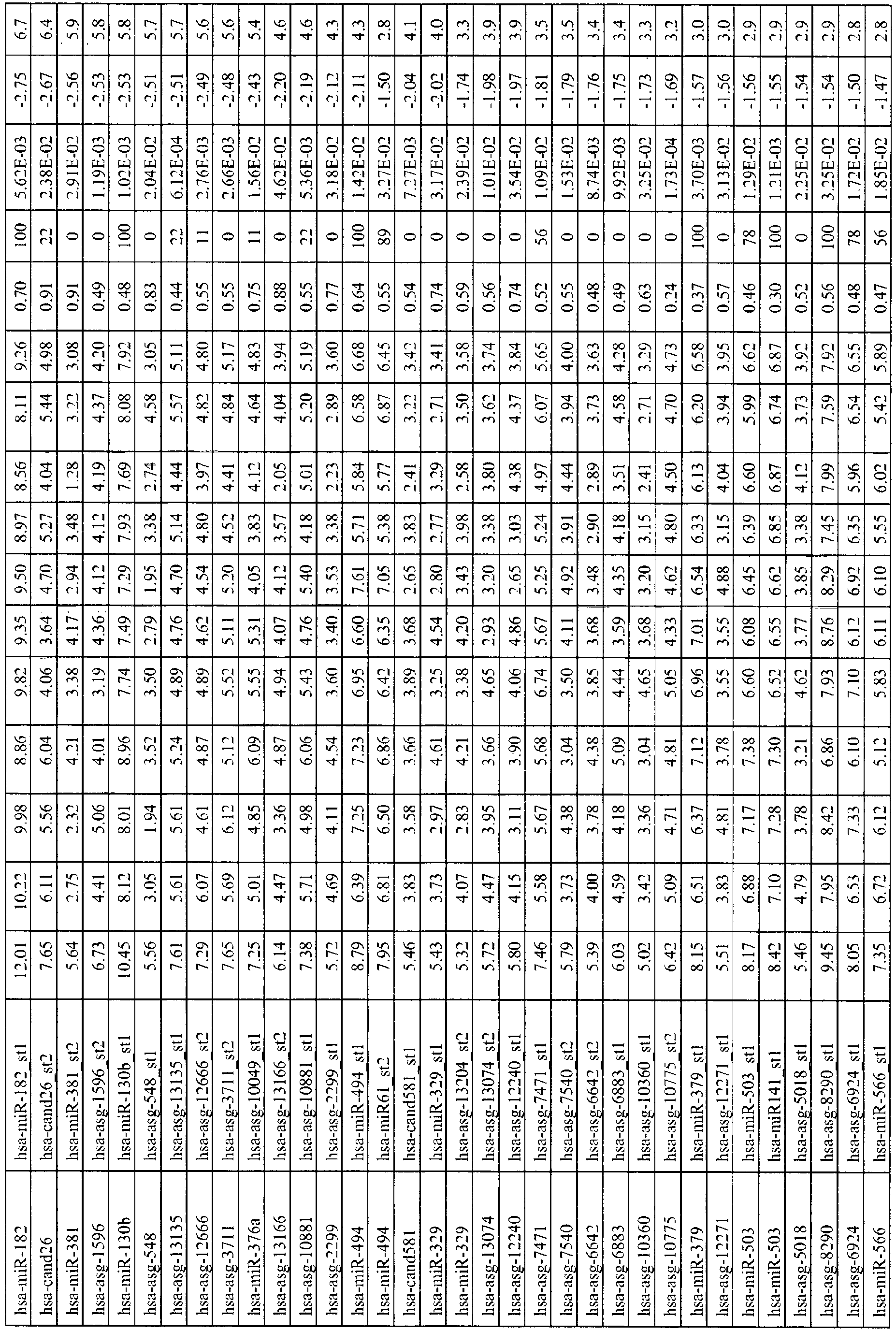

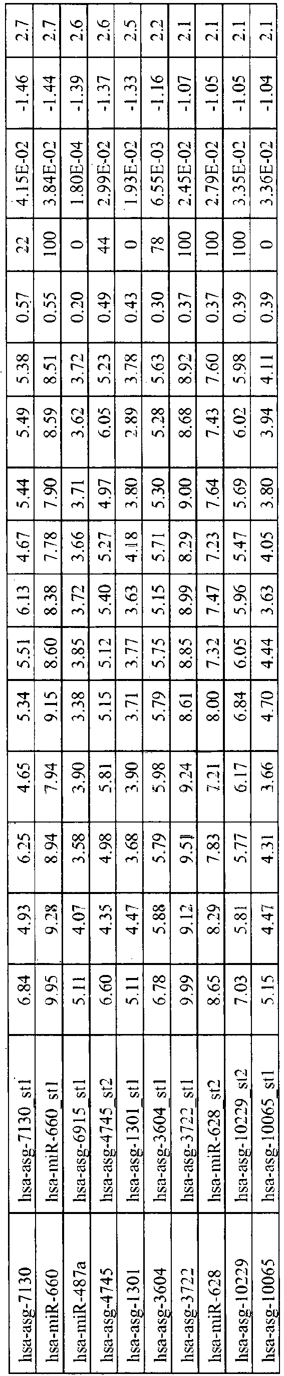

### EXAMPLE 30:

### MIRNAS DIFFERENTALLY EXPRESSED BETWEEN NORMAL TESTICLE TISSUE AND NORMAL LYMPH NODES

To identify miRNAs that may be useful markers for metastasis of cancerous testes cells to lymph nodes, the inventors identified miRNAs that are expressed at significantly higher levels in normal testes tissue than in cancer-negative lymph nodes.

Total RNA, purified from one normal human testicle sample (FirstChoice^{®} Human Testicle Total RNA, cat. no.AM7972; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 412 miRNAs (100 verified, 312 predicted) with significant differential expression between the normal testicle and normal lymph node samples (|log2Diff| I testicle vs LN neg ≥1 and p-value<0.05) (Table 29). Among the verified miRNAs, hsa-miR-513 was expressed at a level over 200-fold higher in the testicle sample than in the lymh node samples; hsa-miR-514 and -509 were expressed at levels between 100- and 200-fold higher in the testicle sample; 9 miRNAs (hsa-miR-493-5p, -506, - 383, -411, -493-3p, -510, -187, -508 and -517a) were expressed at levels between 50- and 100-fold higher in the testicle sample; 27 miRNAs were expressed between 20- and 50-fold higher in testicle; 29miRNAs were expressed between 10- and 20-fold higher in testicle compared to LNneg; and 14 miRNAs were expressed between 5- and 10-fold higher in the testicle sample than in normal lymph nodes. Among the predicted miRNAs, hsa-asg-5638_st2 was expressed at a level over 800-fold higher in the testicle sample than in the normal lymph nodes; hsa-miR-5630_st2, -1088_st1 and -11338_st2) were expressed at levels between 100-and 200-fold higher in the testicle sample; 6 miRNAs were expressed between 50- and 100-fold higher in testicle; 33 miRNAs were expressed between 20- and 50-fold higher in testicle; 59 miRNAs were expressed between 10- and 50-fold higher in testicle; and 97 miRNAs were expressed between 5- and 10-fold higher in the testicle sample than in normal lymph nodes.

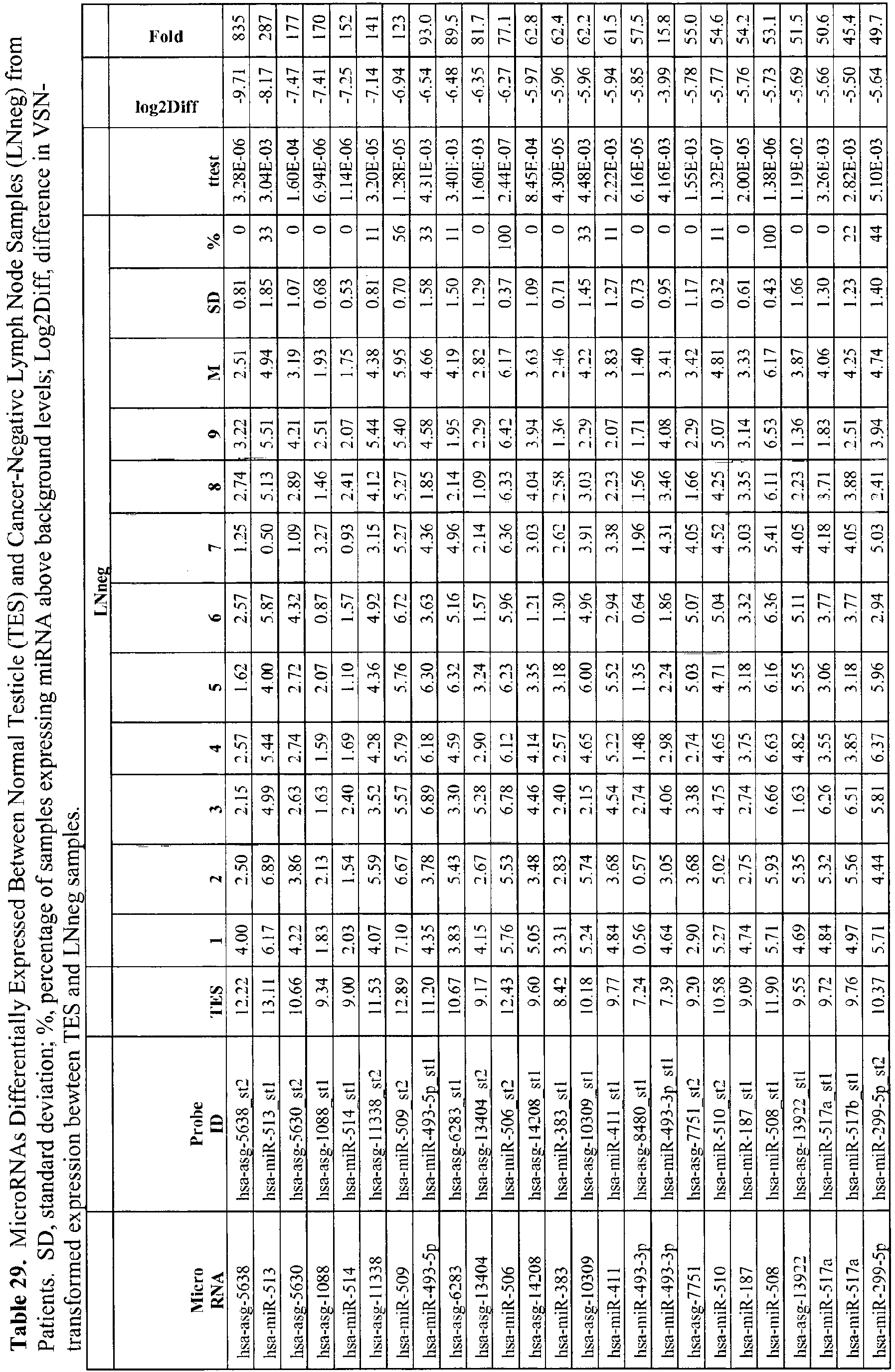

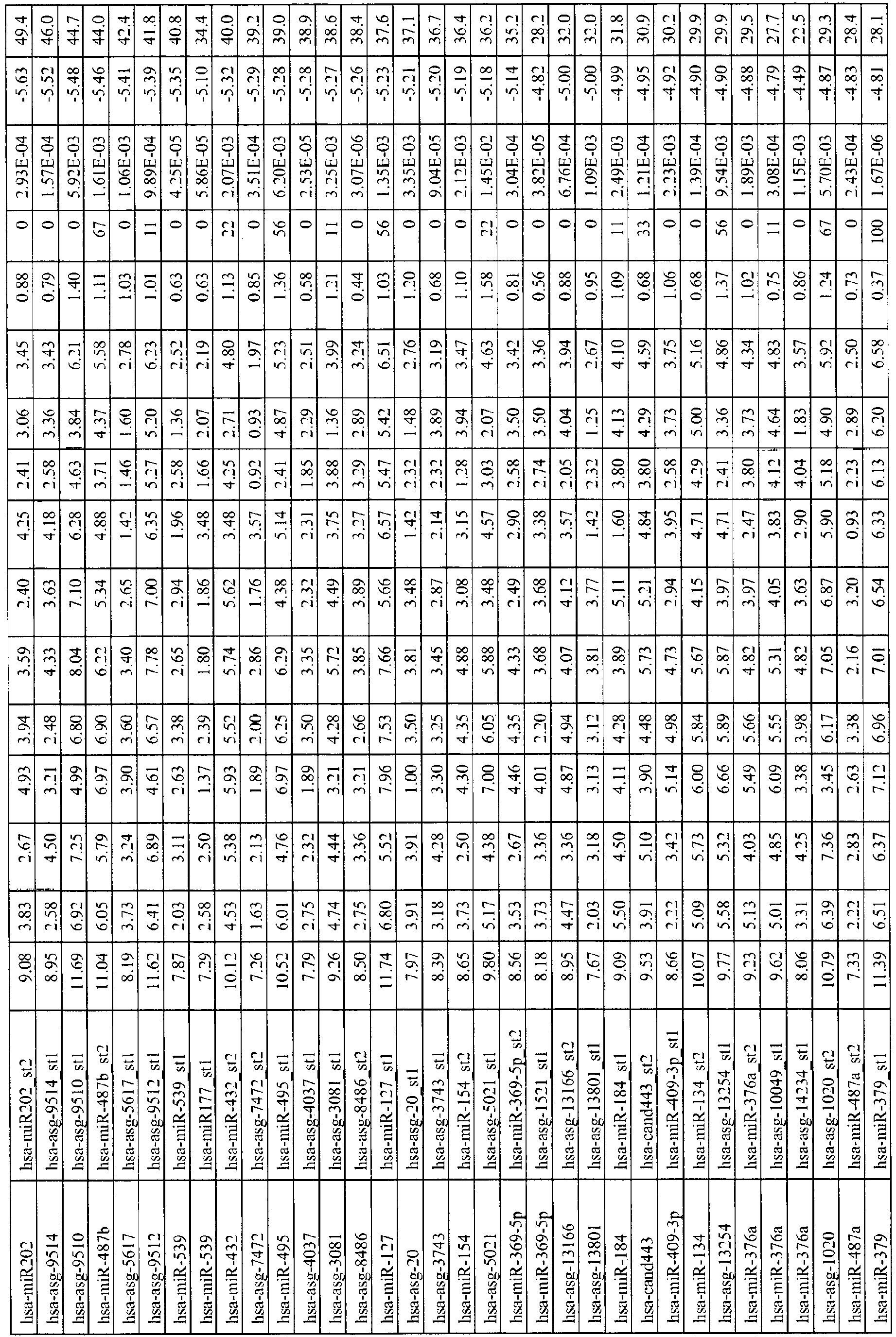

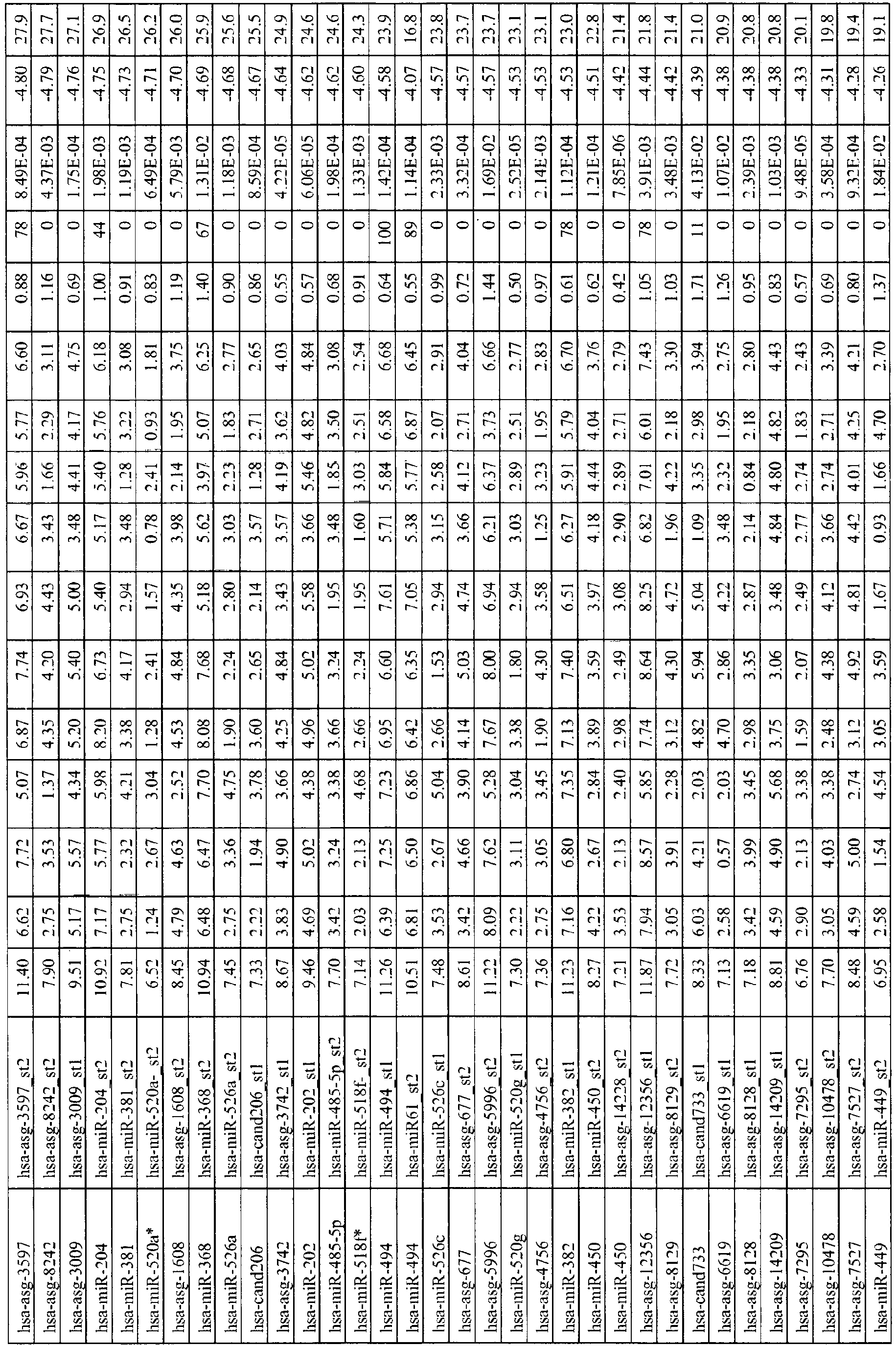

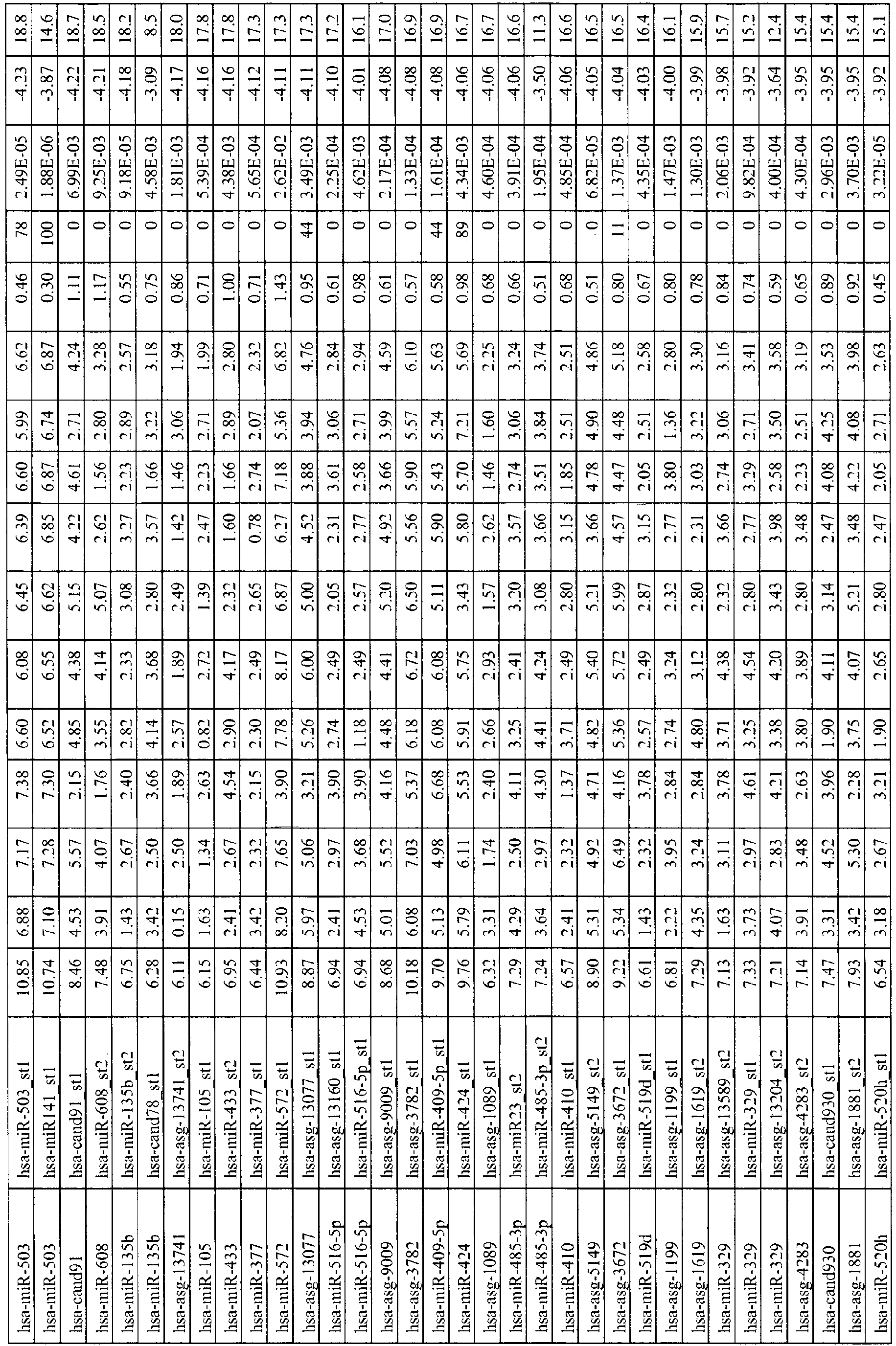

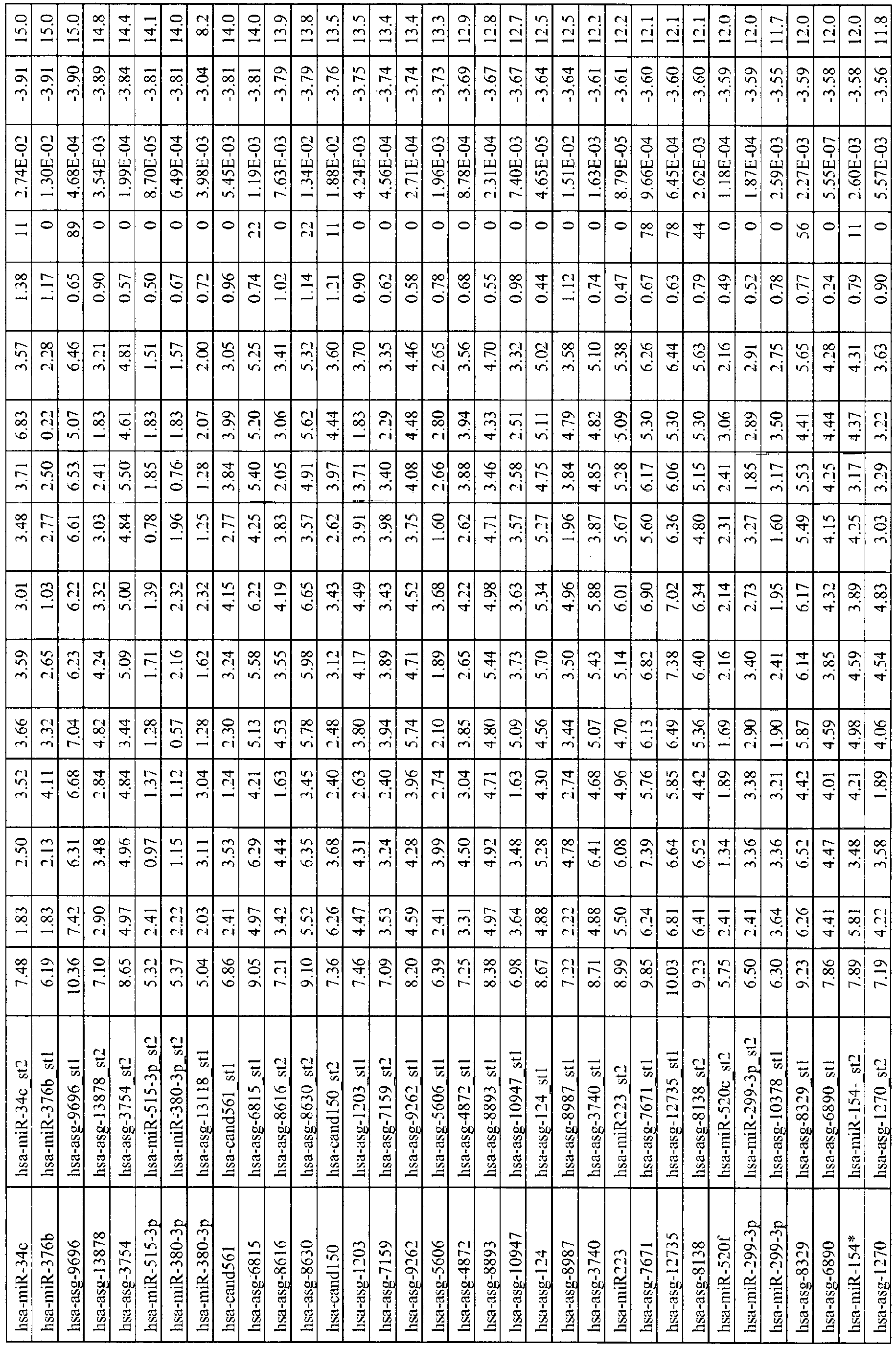

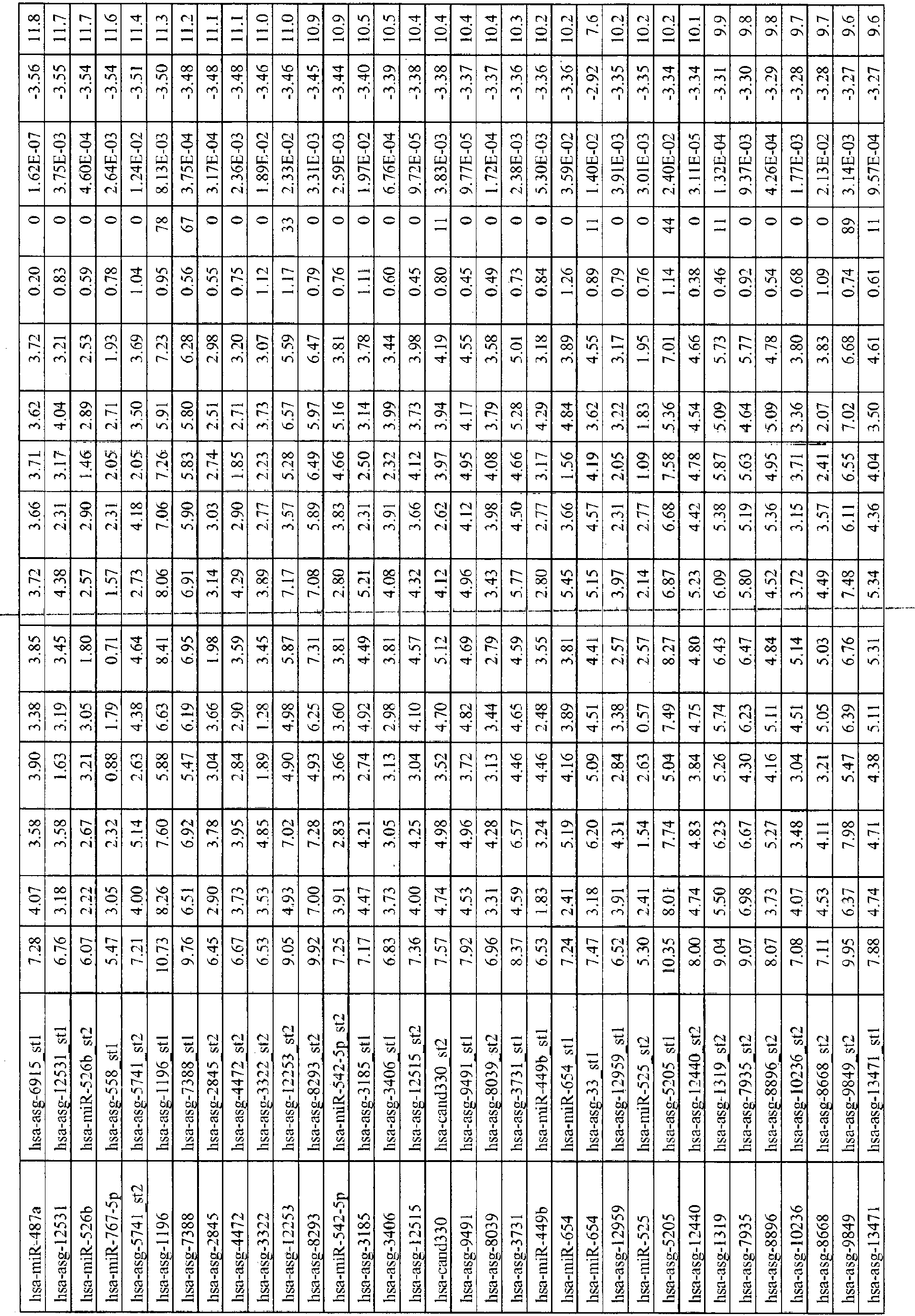

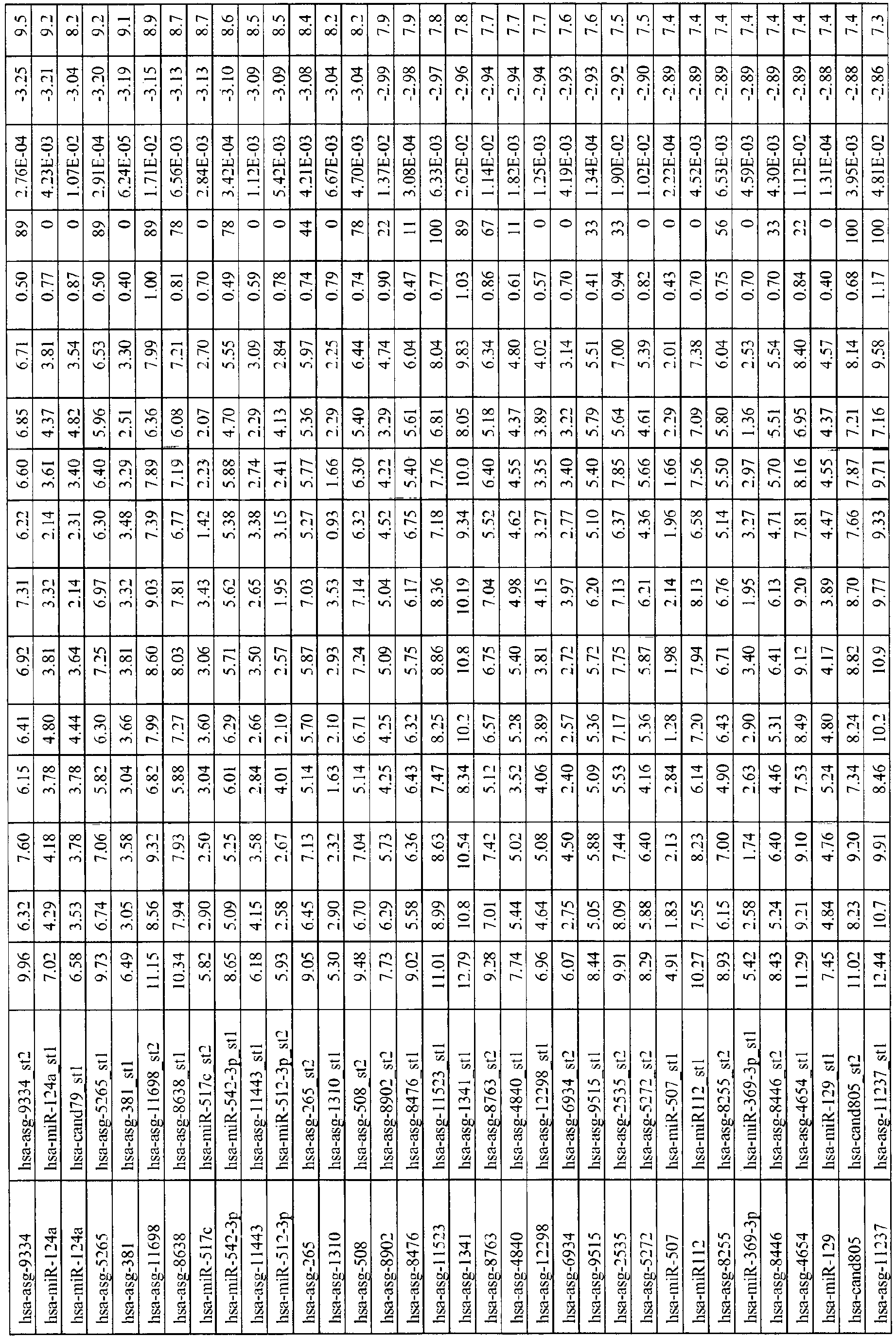

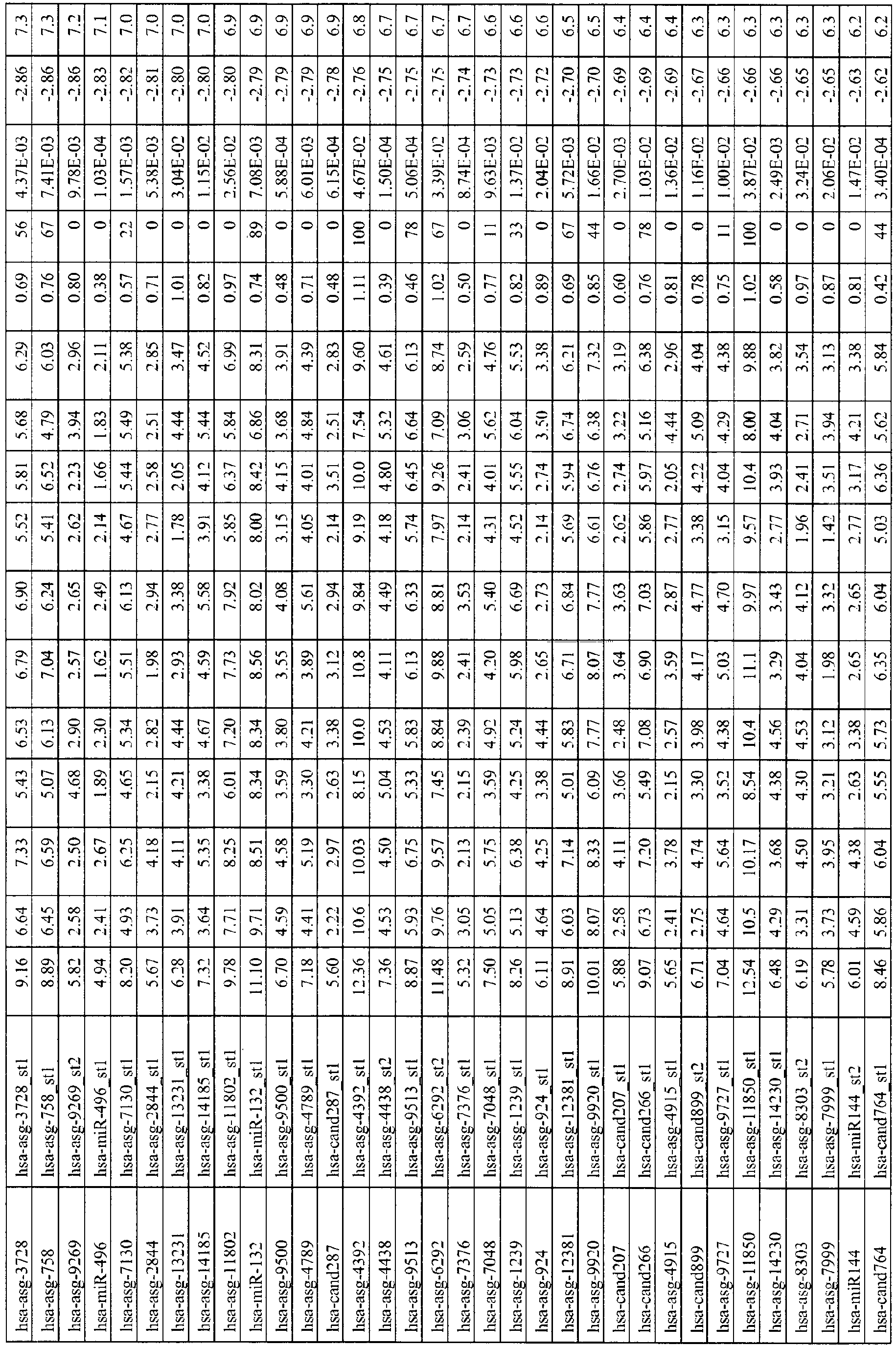

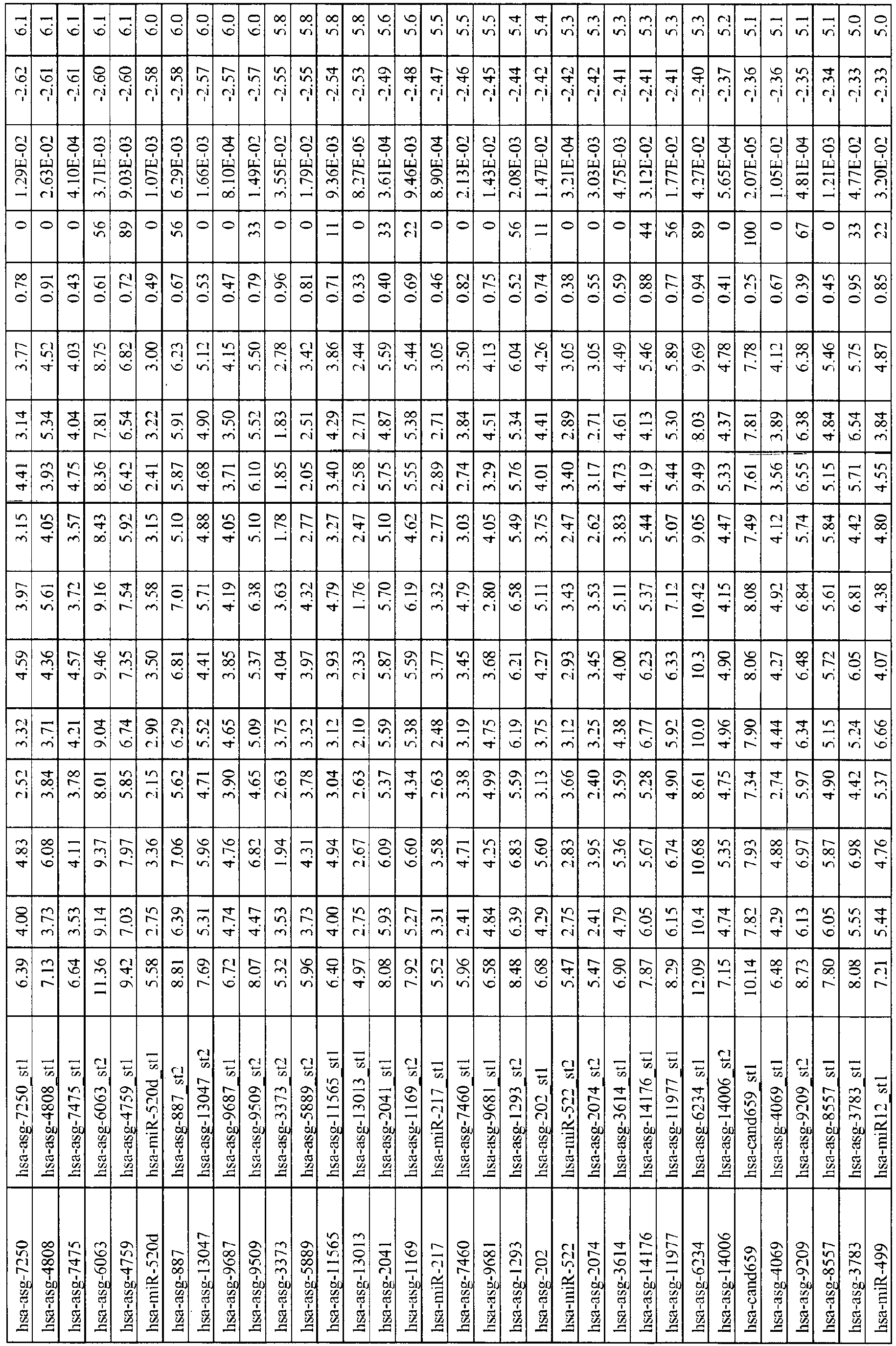

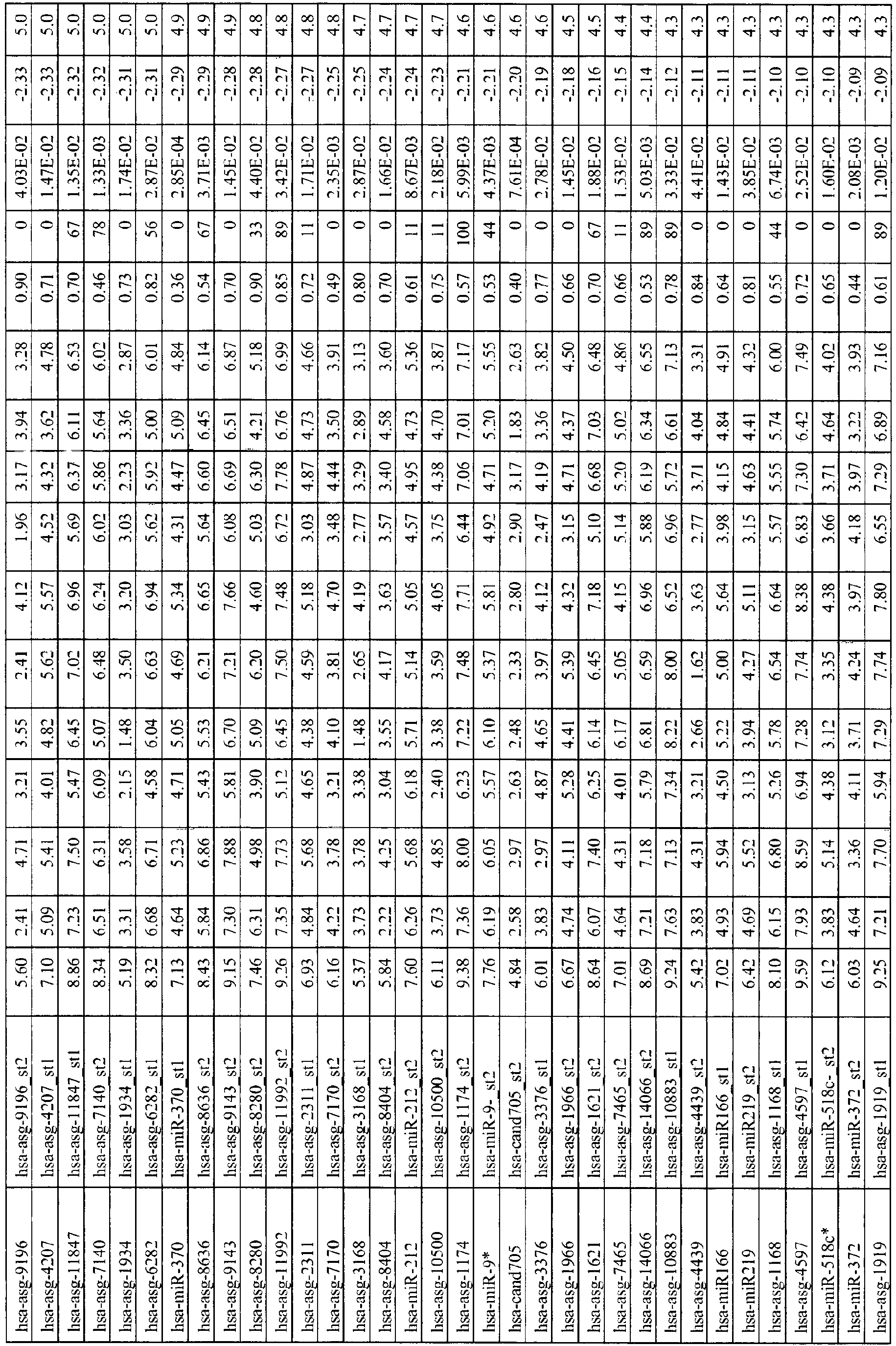

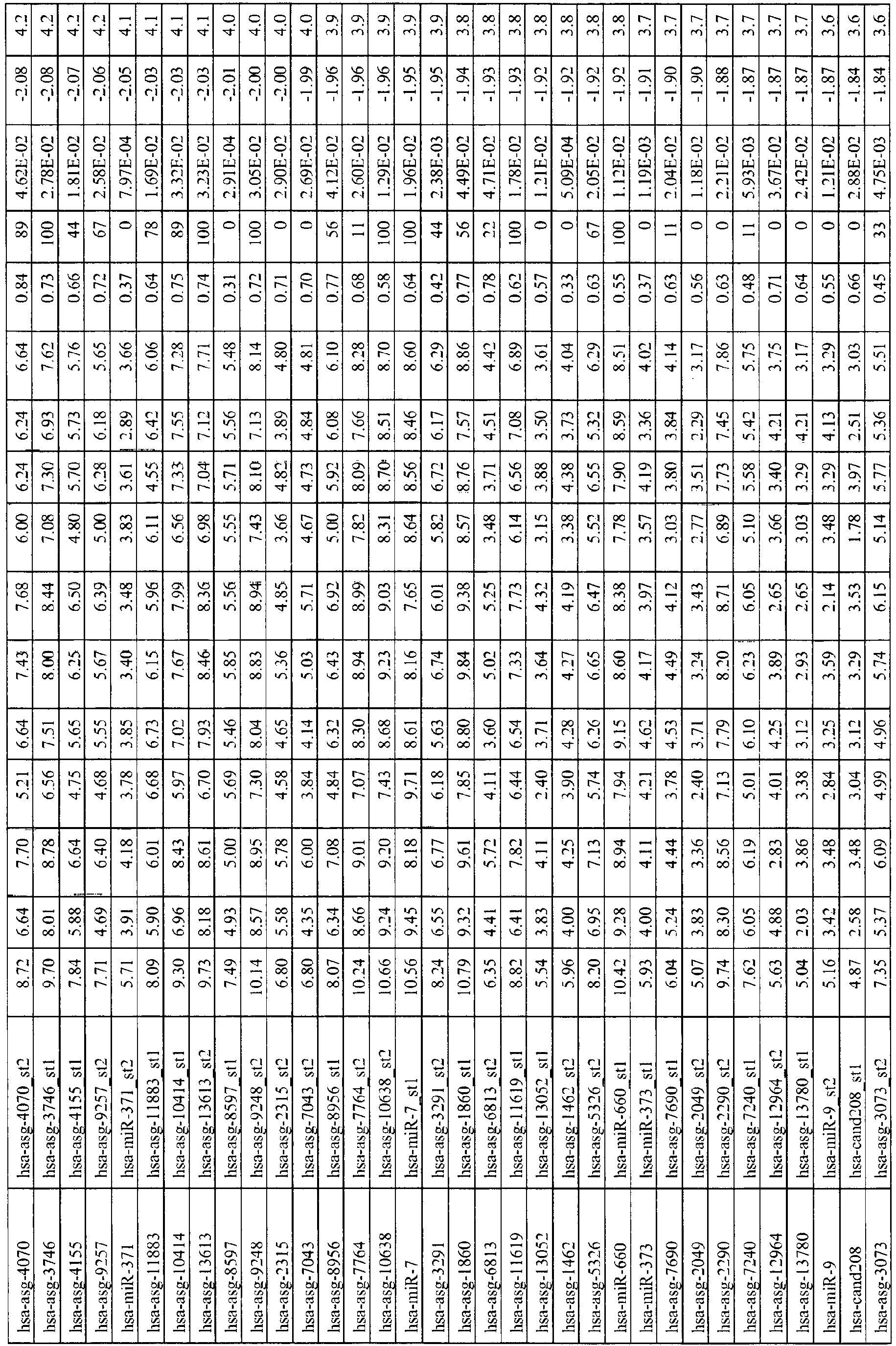

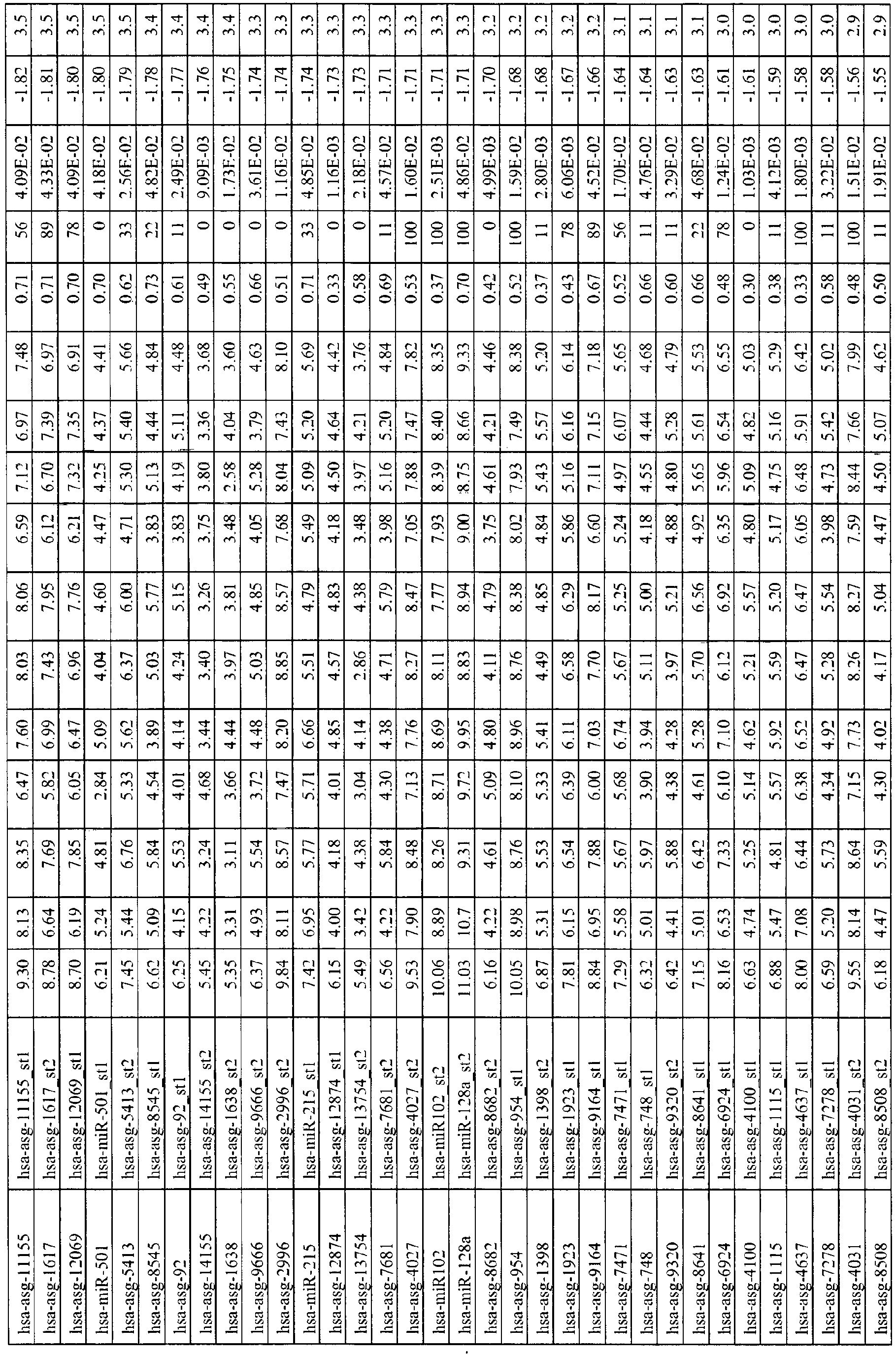

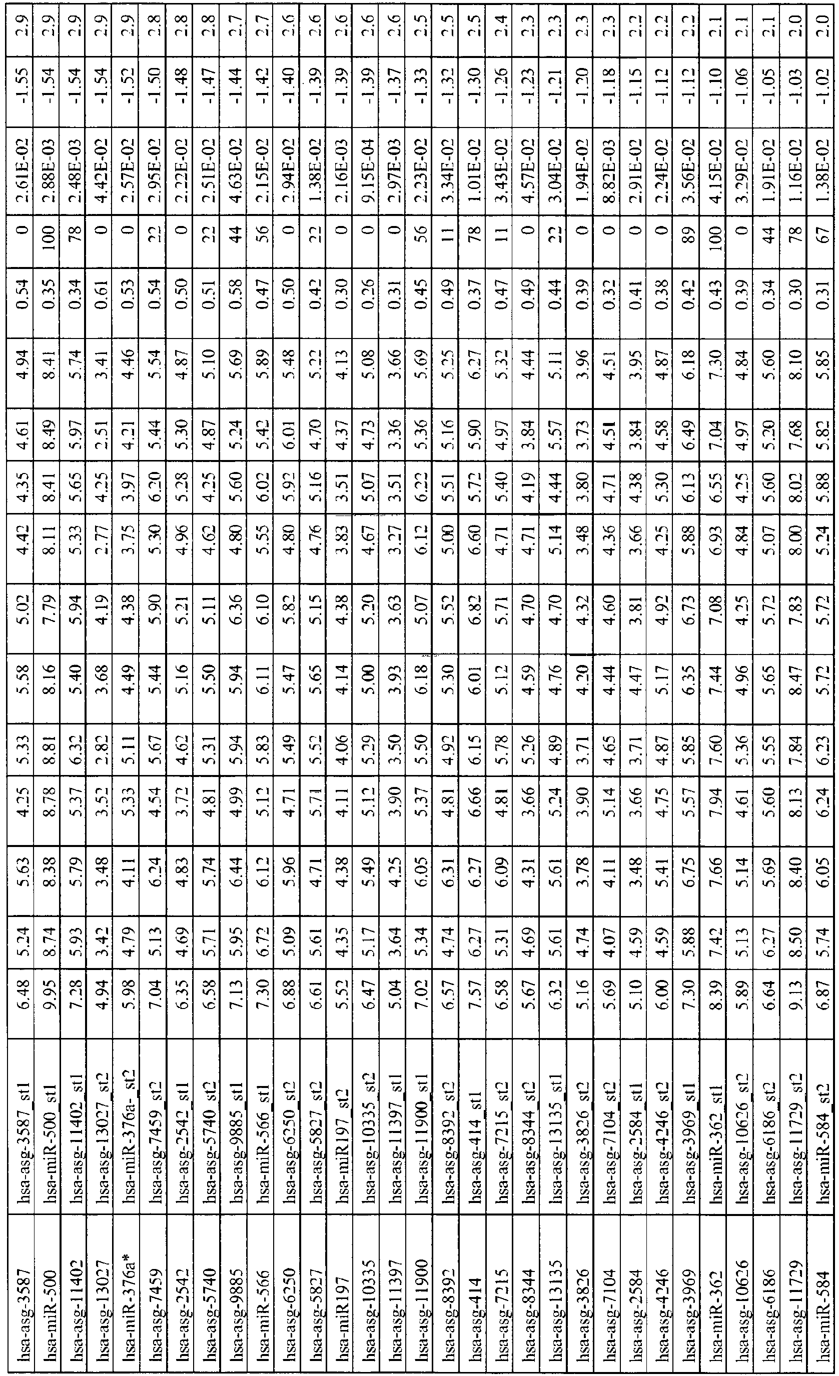

### EXAMPLE 31:

### MIRNAS DIFFERENTIALLY EXPRESSED BETWEEN NORMAL UTERUS TISSUE AND NORMAL LYMPH NODES

To identify miRNAs that may be useful markers for metastasis of cancerous uterus cells to lymph nodes, the inventors identified miRNAs that are expressed at significantly higher levels in normal uterus tissue than in cancer-negative lymph nodes.

Total RNA, purified from one normal human uterus sample (FirstChoice^{®} Human Uterus Total RNA, cat. no. AM7892; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 120 miRNAs (64 verified, 56 predicted) with significant differential expression between the normal uterus and normal lymph nodes (|log2Diff| I uterus vs LN neg ≥1 and p-value<0.05) (Table 30). Among the verified miRNAs, 5 miRNAs (hsa-miR-411, -299-5p, -381, -495 and -204) were expressed at levels between 20- and 25-- fold higher in the uterus sample; 16 miRNAs were expressed at levels between 10- and 20-fold higher in the uterus sample; and 16 miRNAs were expressed at levels between 5- and 10-fold higher in the uterus sample thann in the normal lymph nodes. Among the predicted miRNAs, hsa-asg-13404_st2 and hsa-miR202_st2 were expressed at levels between 25- and 35-fold higher in the uterus sample; 6 miRNA (hsa-asg-5021_st1 -13254_st1, -11443_st1, - 5617_st1, -14065_st2 and hsa-miR144_st2) were expressed at levels between 10-and 20-fold higher in the uterus; and 14 miRNAs were expressed at levels between 5- and 10-fold higher in the uterus sample than in normal lymph nodes.

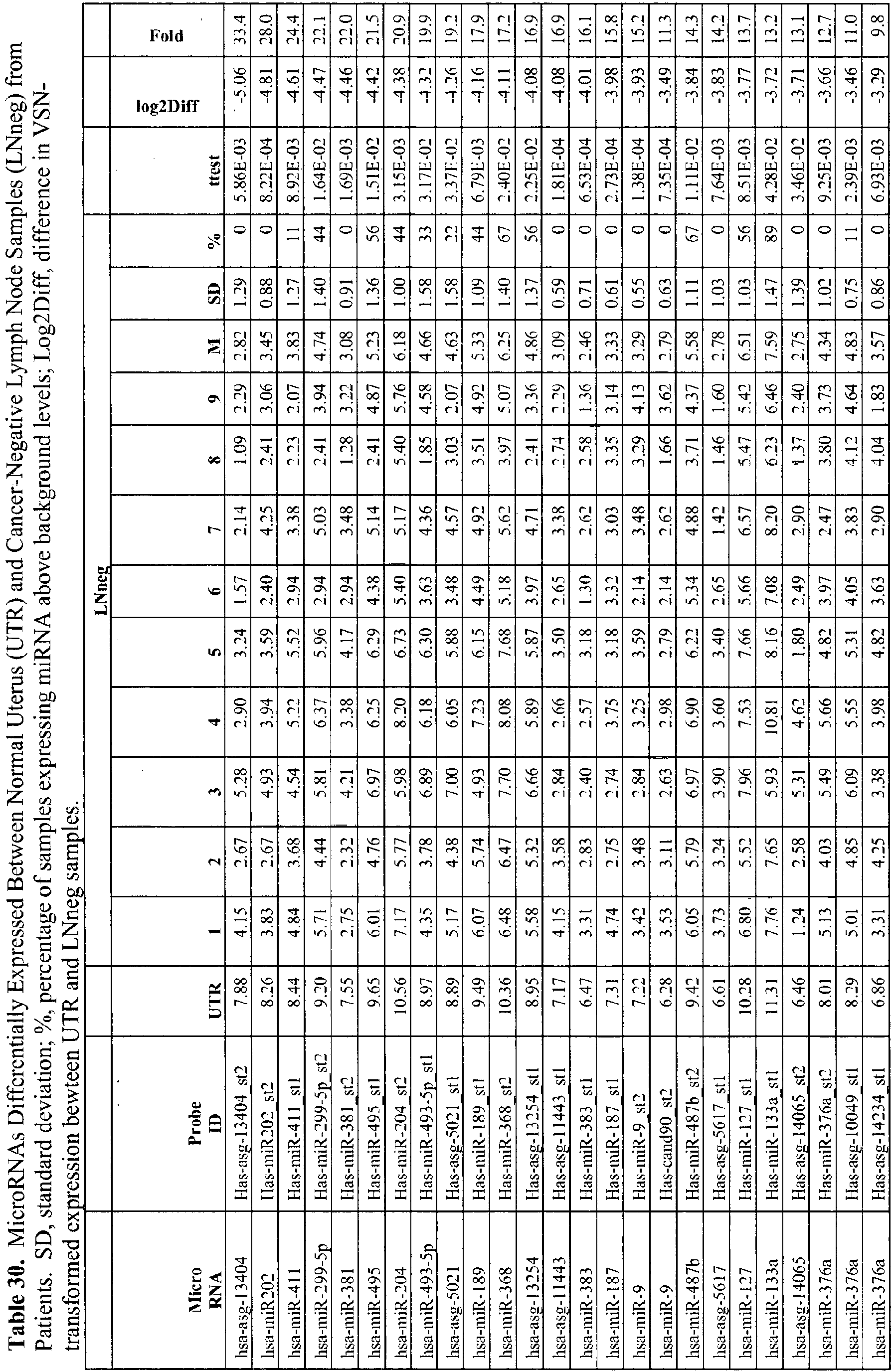

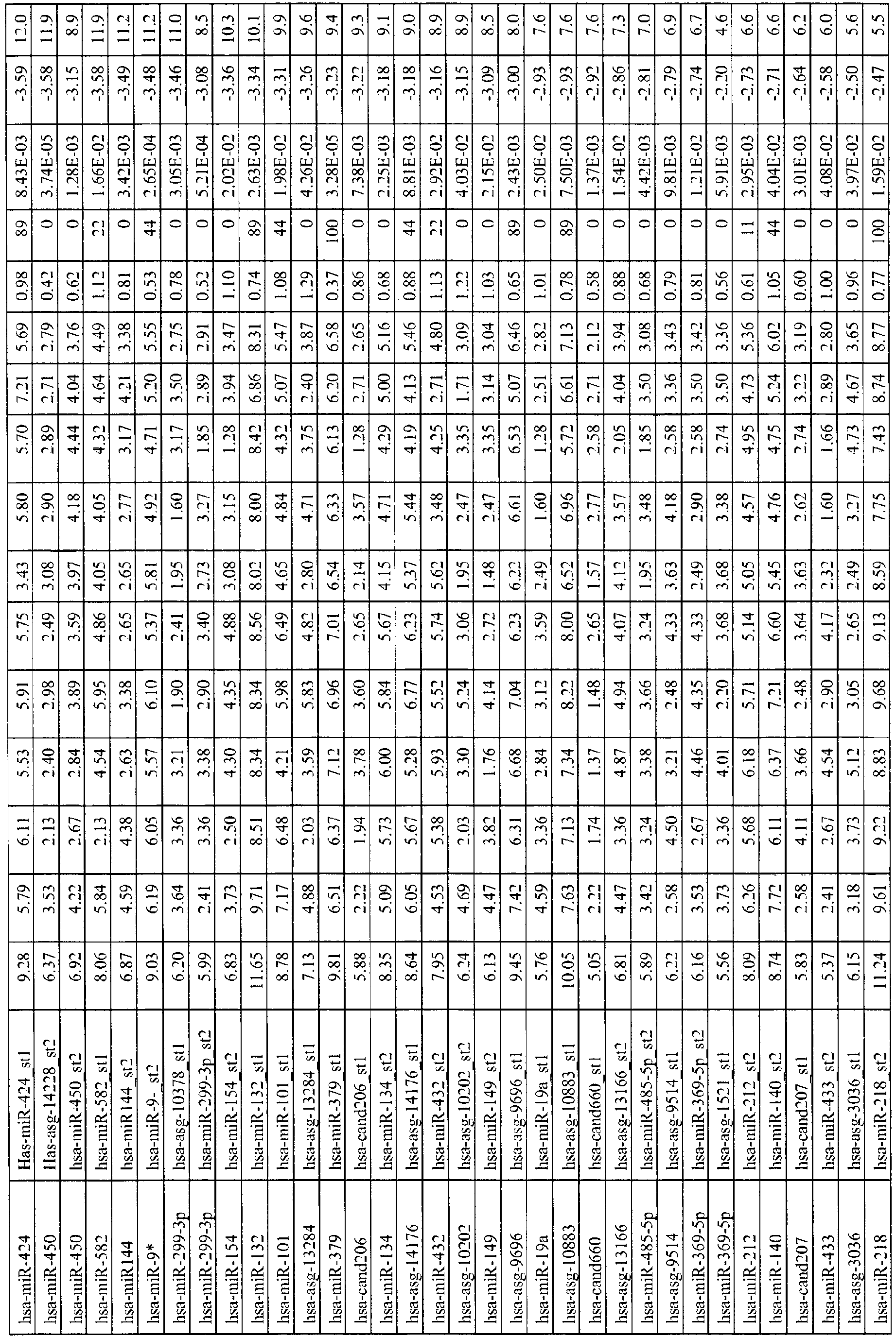

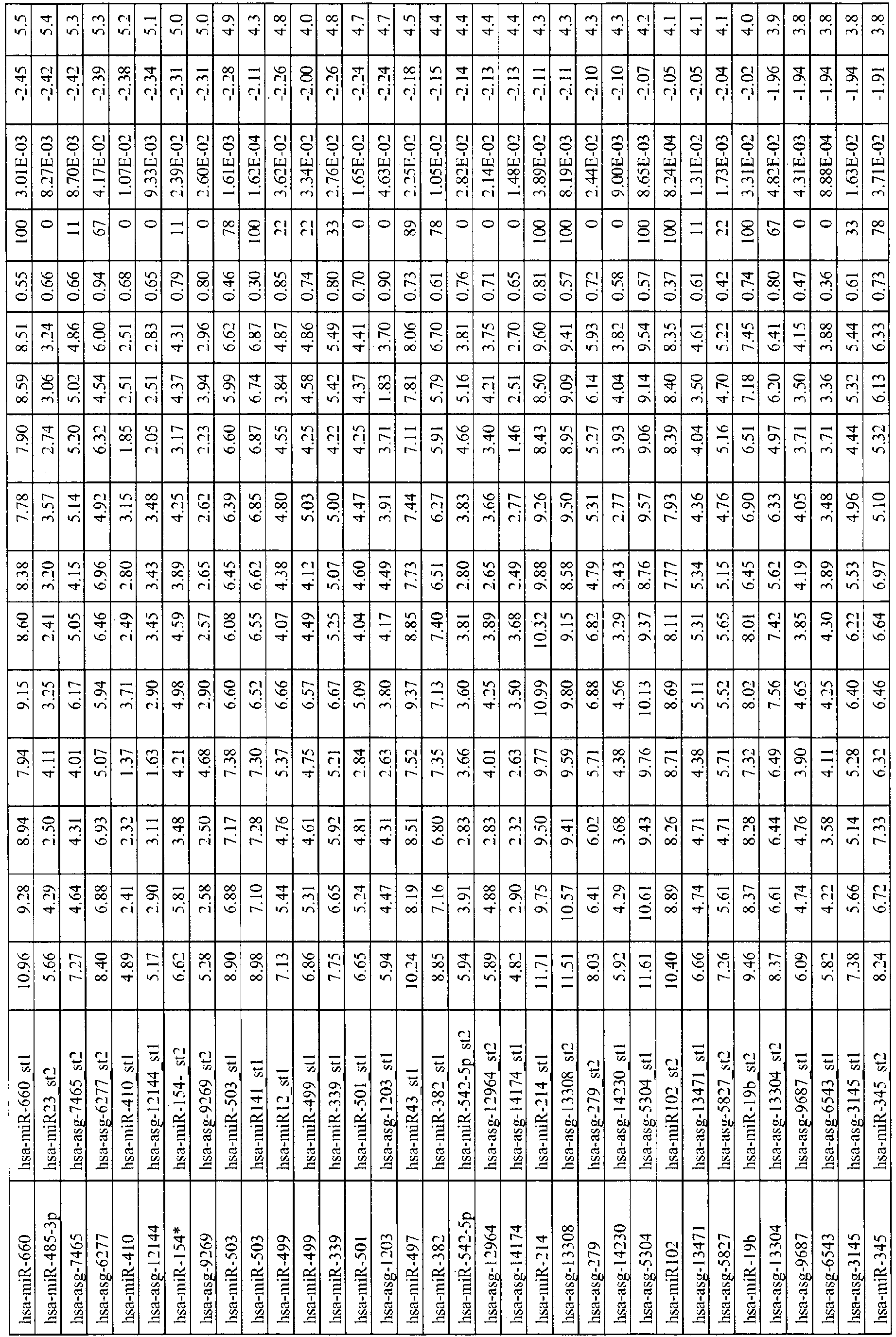

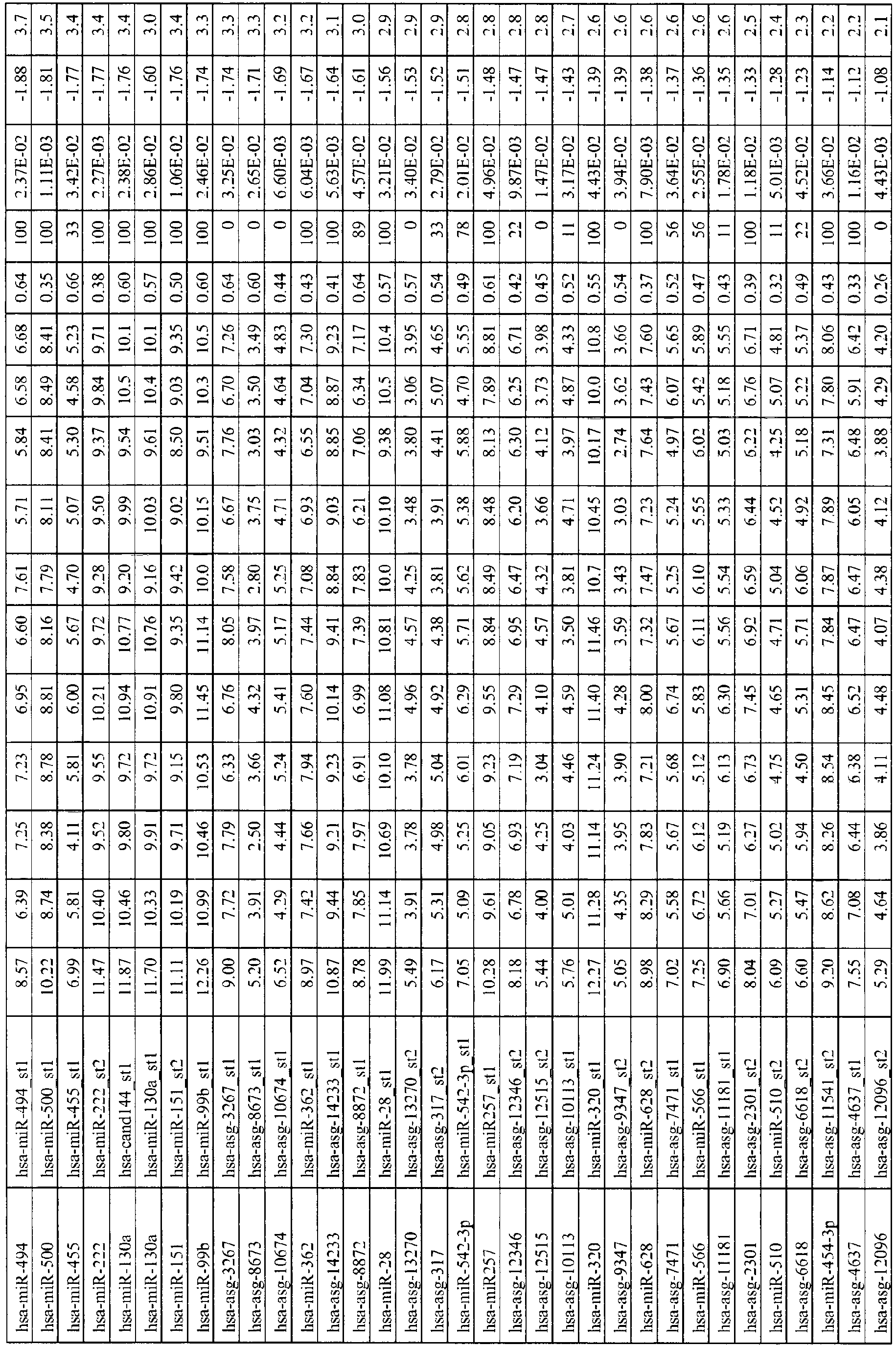

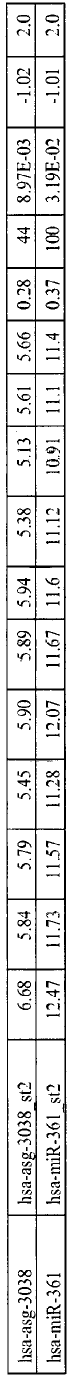

### EXAMPLE 32:

### MIRNAS DIFFERENTALLY EXPRESSED BETWEEN NORMAL SPLEEN TISSUE AND NORMAL LYMPH NODES

To identify miRNAs that may be useful markers for metastasis of cancerous spleen cells to lymph nodes, the inventors identified miRNAs that are expressed at significantly higher levels in normal spleen tissue than in cancer-negative lymph nodes.

Total RNA, purified from one normal human spleen sample (FirstChoice^{®} Human Spleen Total RNA, cat. no. AM7970 Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 133 miRNAs (67 verified, 66 predicted) with significant differential expression between the normal spleen and normal lymph node samples (|log2Diff| I spleen vs LN neg ≥1 and p-value<0.05) (Table 31). Among the verified miRNAs, 7 miRNAs (hsa-miR-495, -493-5p, -493-3p, 338, -368, -487b and -139) were expressed at levels between 20- and 50-fold higher in the spleen sample; 17 miRNAs were expressed at levels between 10- and 20-fold higher in spleen; and 18 miRNAs were expressed at levels between 5- and 10-fold higher in the spleen sample than in normal lymph nodes. Among the predicted miRNAs, 3 miRNAs (hsa-asg-13404_st2, 13166_st2 and hsa-miR202_st2) were expressed at levels between 20- and 40-fold higher in the spleen sample; 9 miRNAs were expressed at levels between 10-and 20-fold higher in spleen; and 9 miRNAs were expressed at levels between 5- and 10-fold higher in the spleen sample than in normal lymph nodes.

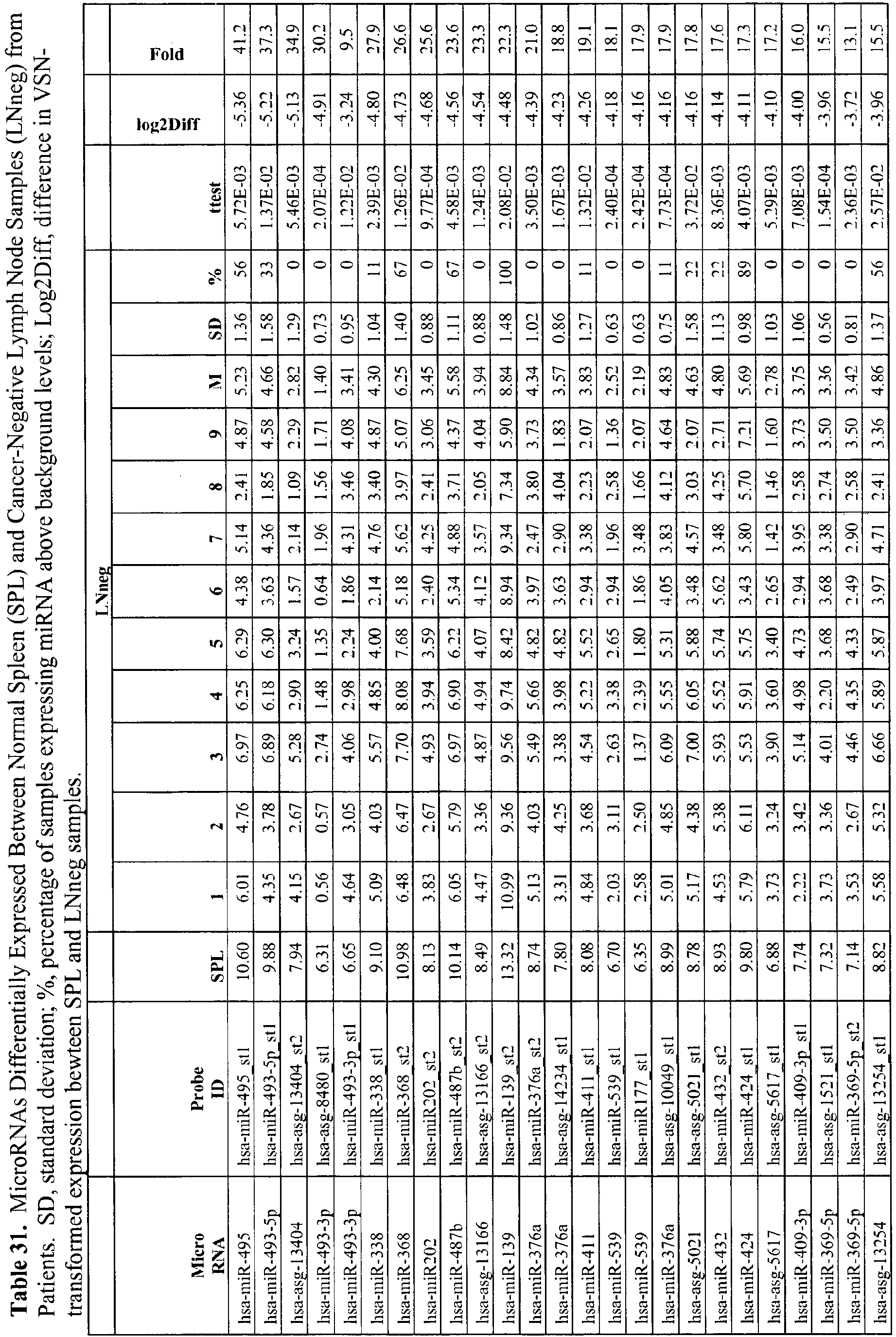

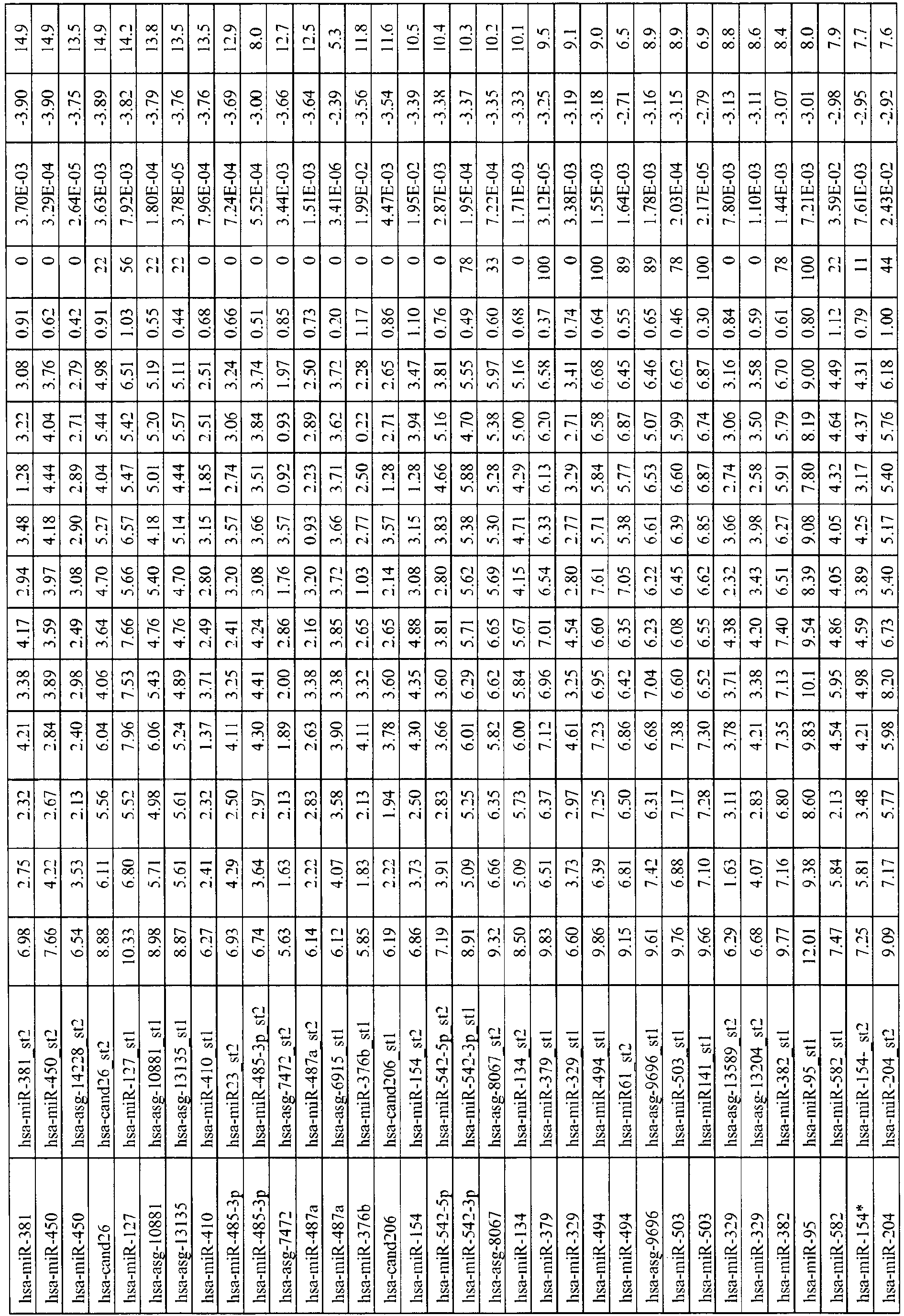

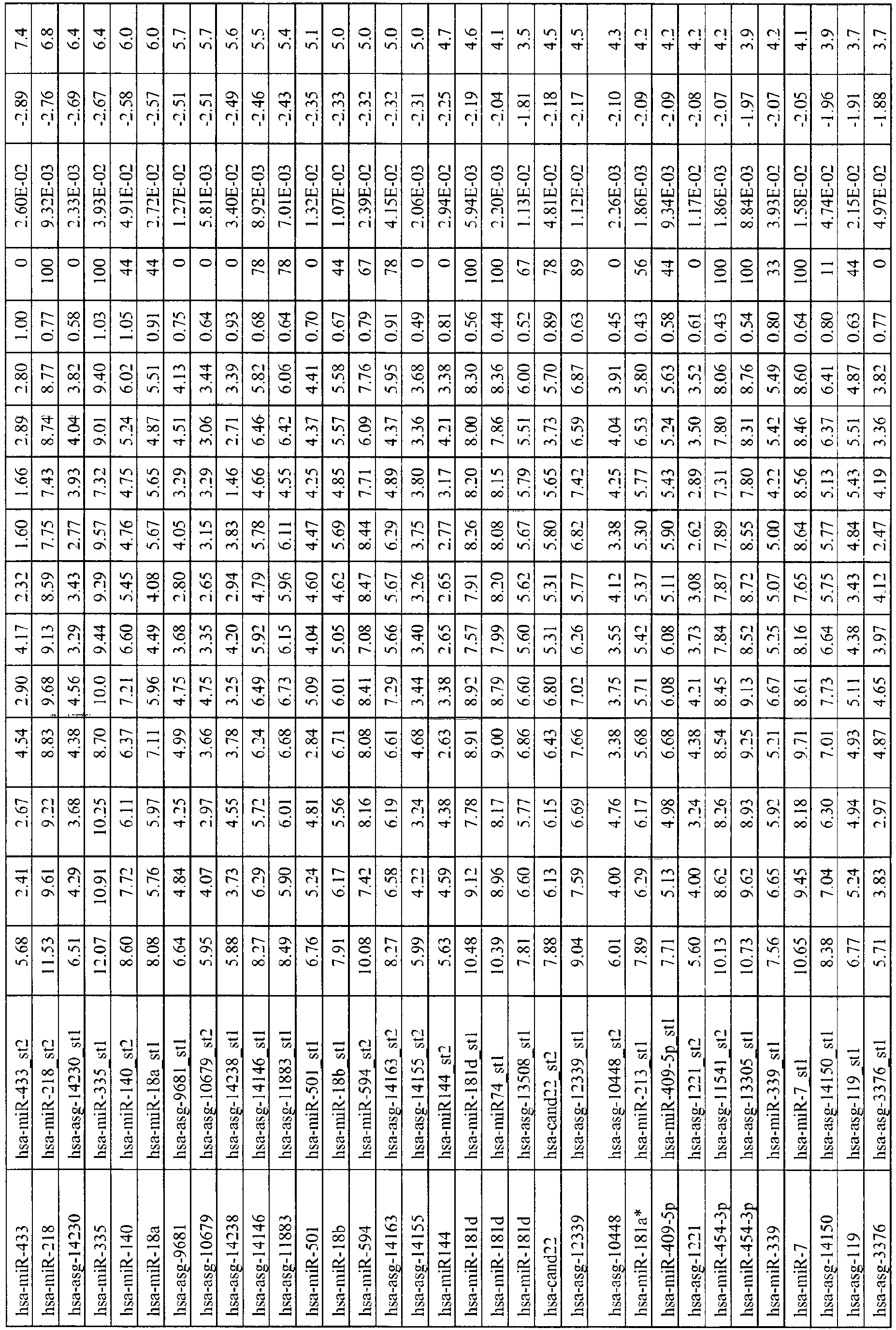

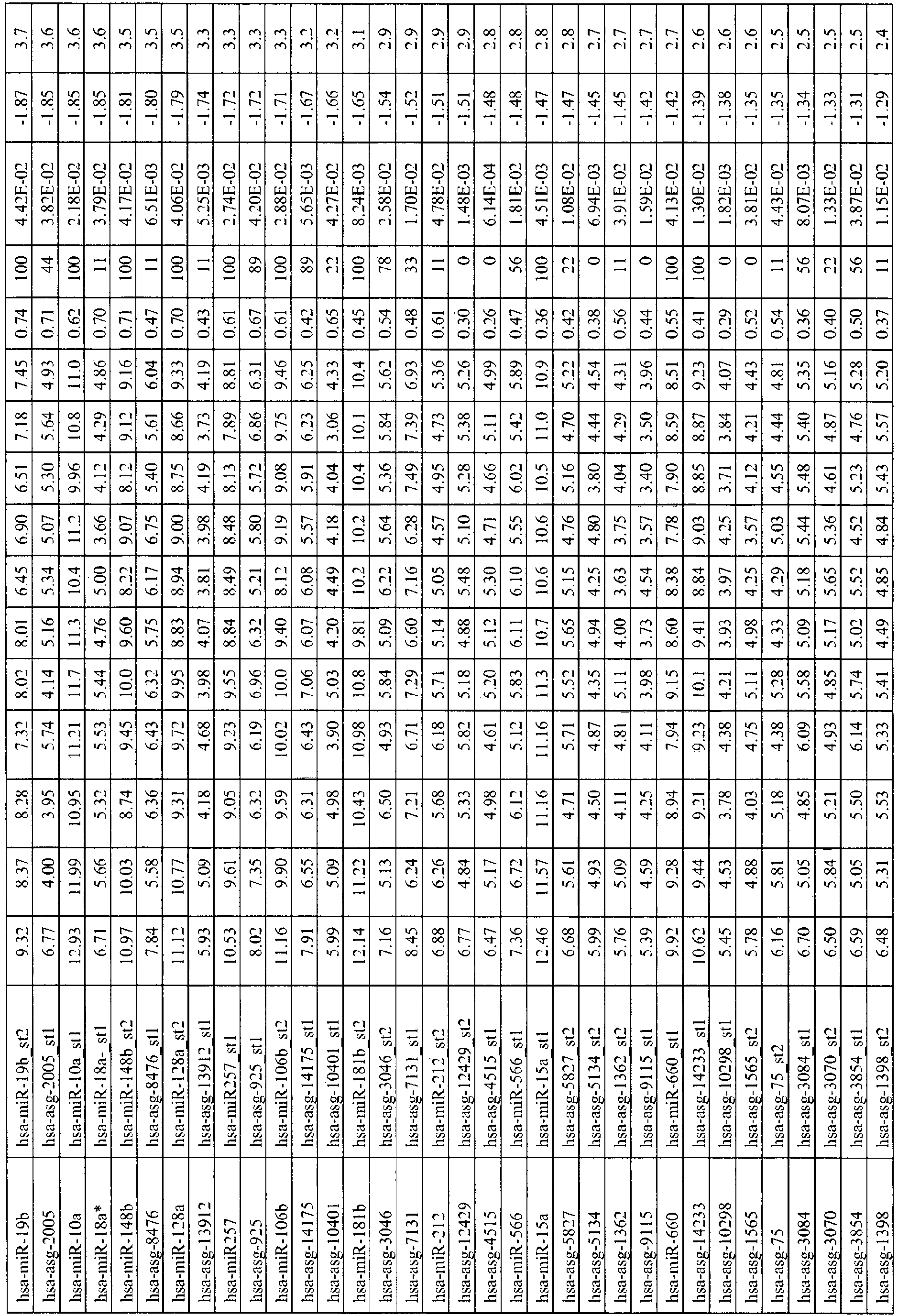

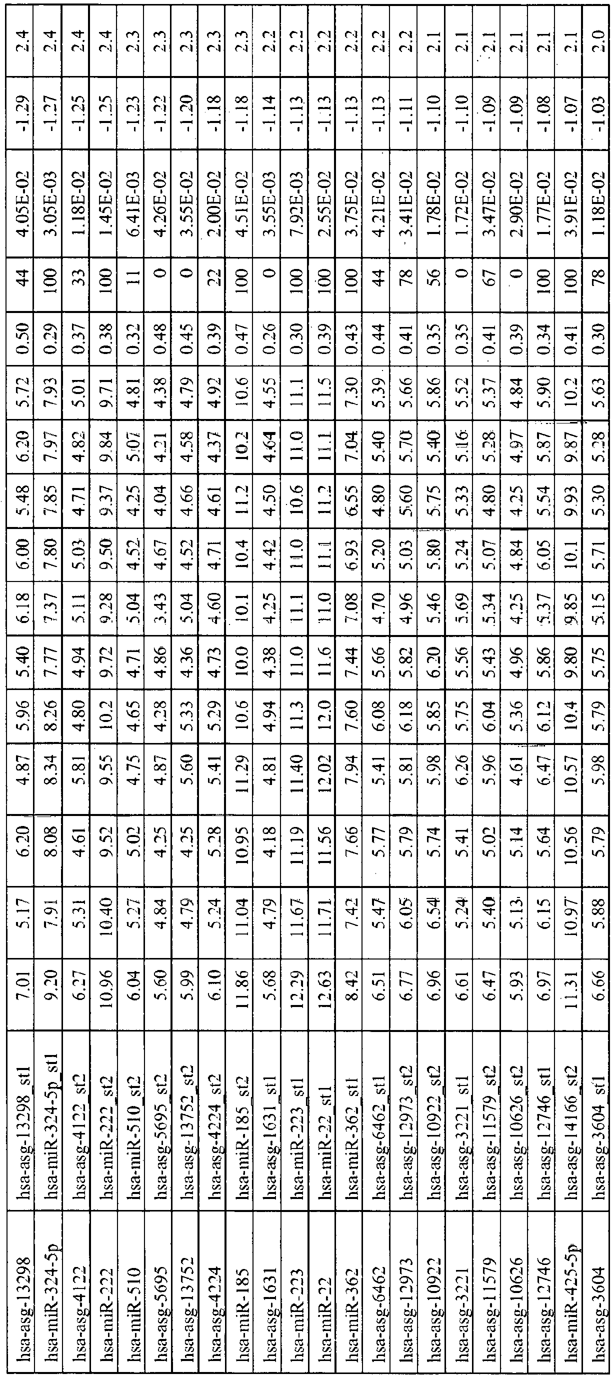

### EXAMPLE 33:

### MIRNAS DIFFERENTALLY EXPRESSED BETWEEN NORMAL TONGUE TISSUE AND NORMAL LYMPH NODES

To identify miRNAs that may be useful markers for metastasis of cancerous tongue cells to lymph nodes, the inventors identified miRNAs that are expressed at significantly higher levels in normal tongue tissue than in cancer-negative lymph nodes.

Total RNA, purified from one normal human tongue sample (FirstChoice^{®} Human Tongue Total RNA, cat. no. B7800-2, Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 158 miRNAs (62 verified, 96 predicted) with significant differential expression between thenormal tongue and normal lymph node sample (|log2Diff| I tongue vs LN neg ≥1 and p-value<0.05) (Table 32). Among the verified miRNAs, hsa-miR-206, -205 and -375 were expressed at levels between 150- and 400- fold higher in the tongue sample; 9 miRNAs (hsa-miR-381, -499, -133b, -203, -429, -299-5p, - 411, -133a and -493-5p) were expressed at levels between 50- and 100-fold higher in tongue; 9 miRNAs were expressed at levels between 20- and 50-fold higher in tongue compared to LNneg; 11 miRNA were expressed at levels between 10- and 20-fold higher in tongue; and 22 miRNAs were expressed at levels between 5- and 10-fold higher in the tongue sample than in the lymph node samples. Among the predicted miRNAs, 9 miRNAs (hsa-asg-2027_st1, -14153_st2, -5617_st1, -2345_st2, -3376_st1, -7637_st2, -1794_st1, -5021_st1, and hsa-cand14_st1) were expressed at levels between 20- and 50-fold higher in tongue; 24 miRNAs were expressed at levels between 10- and 20- fold higher in the tongue sample compared to normal lymph nodes; and 30 miRNAs were expressed at levels between 5- and 10-fold higher in the tongue sample than in normal lymph nodes.

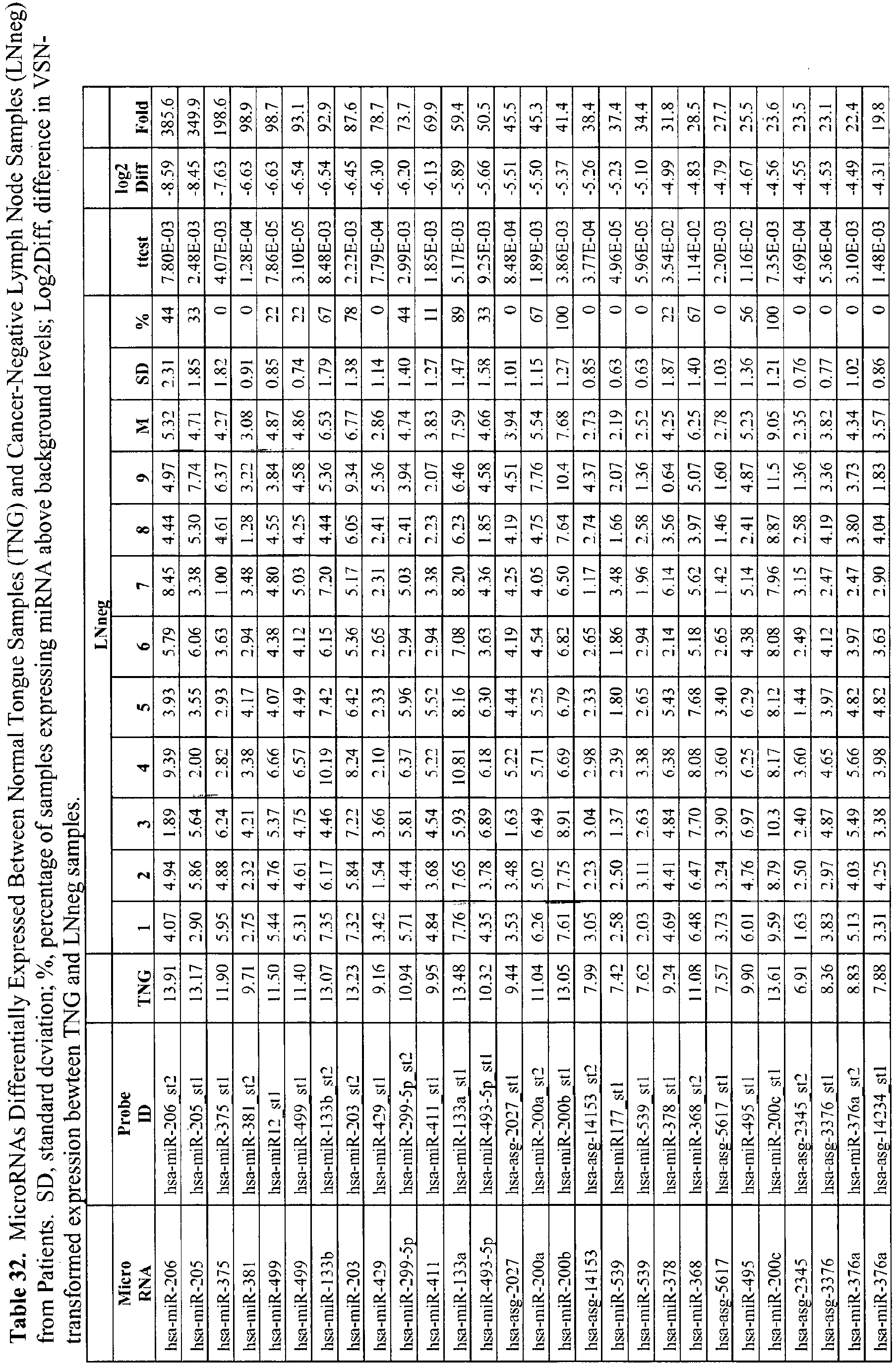

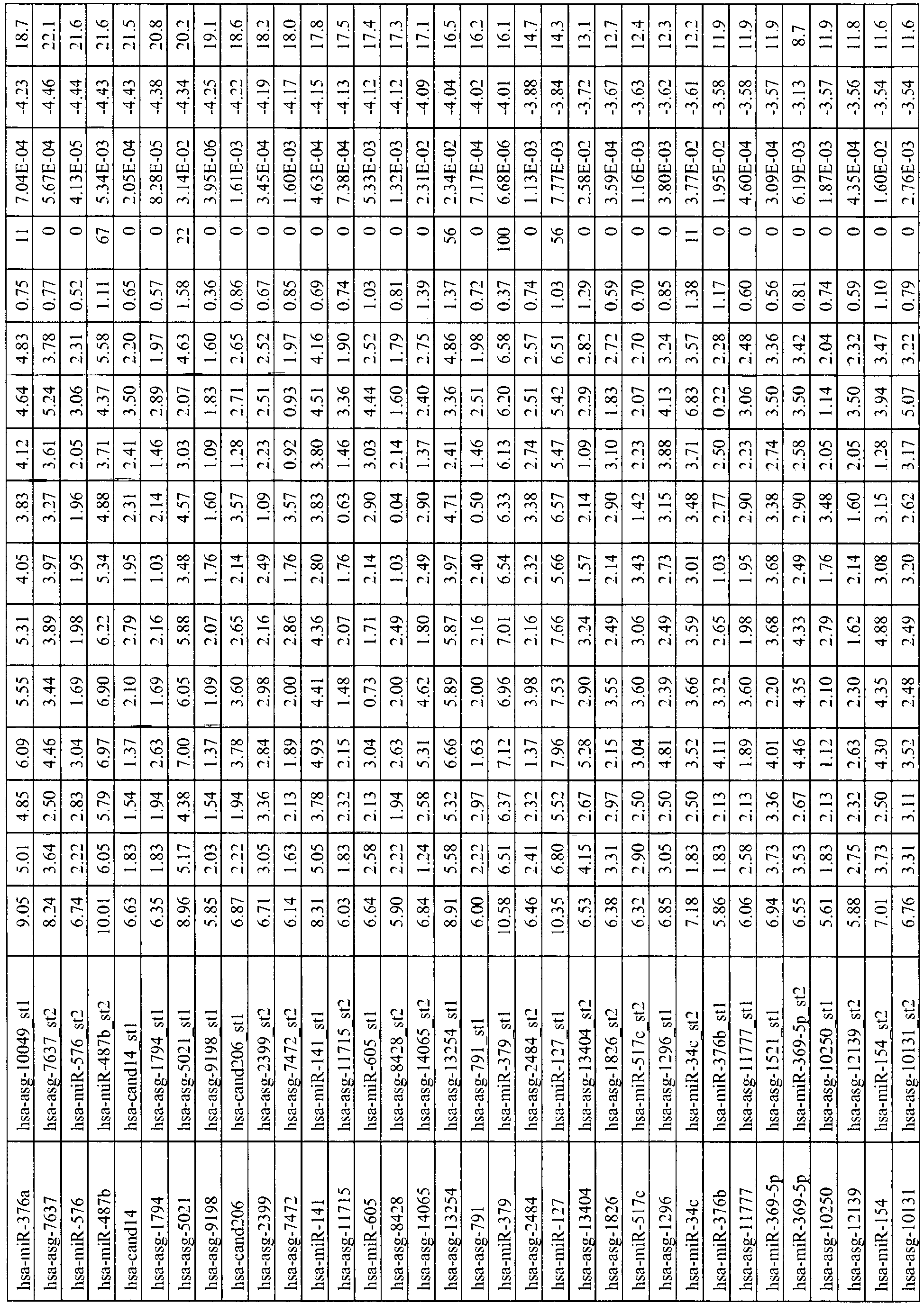

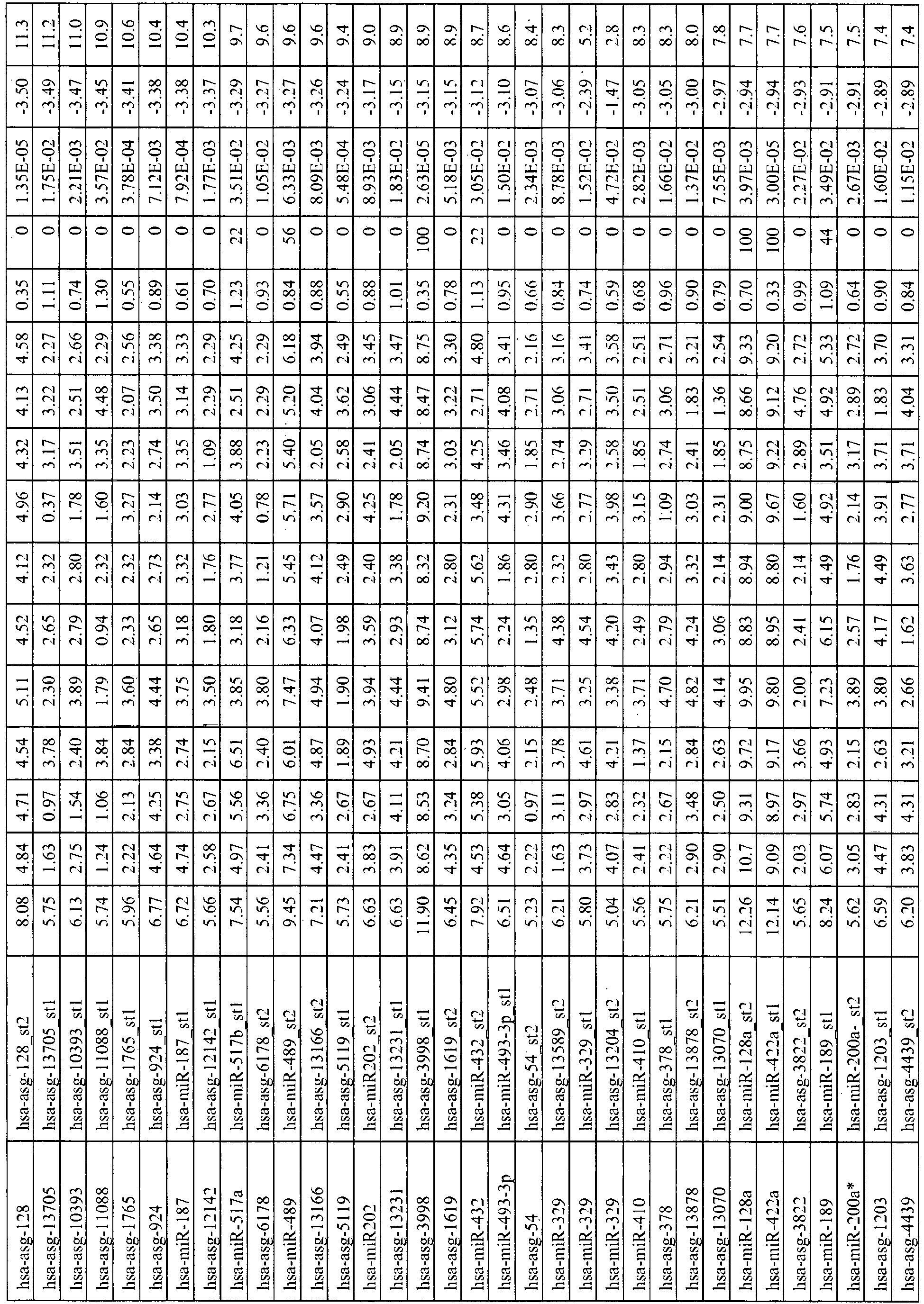

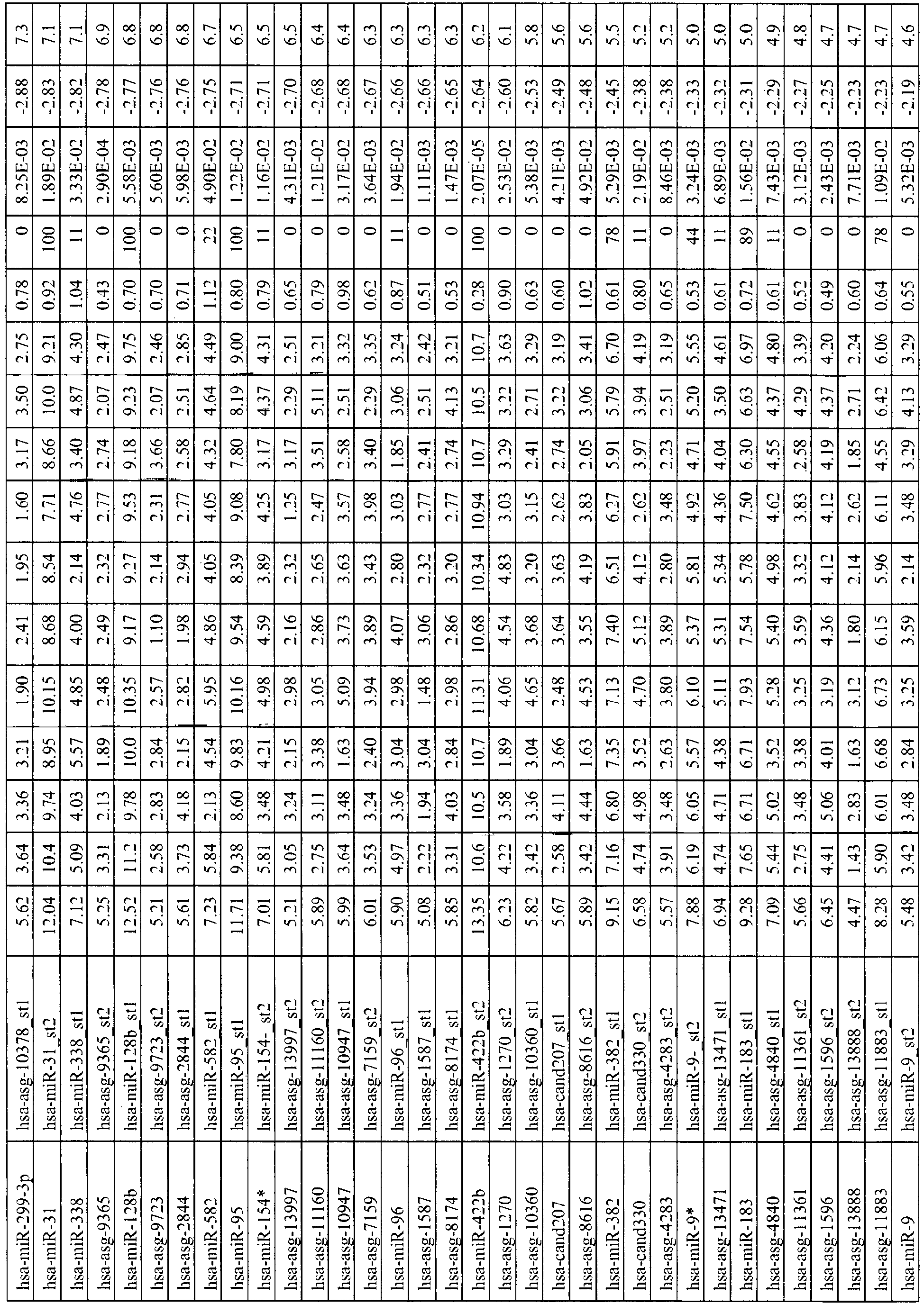

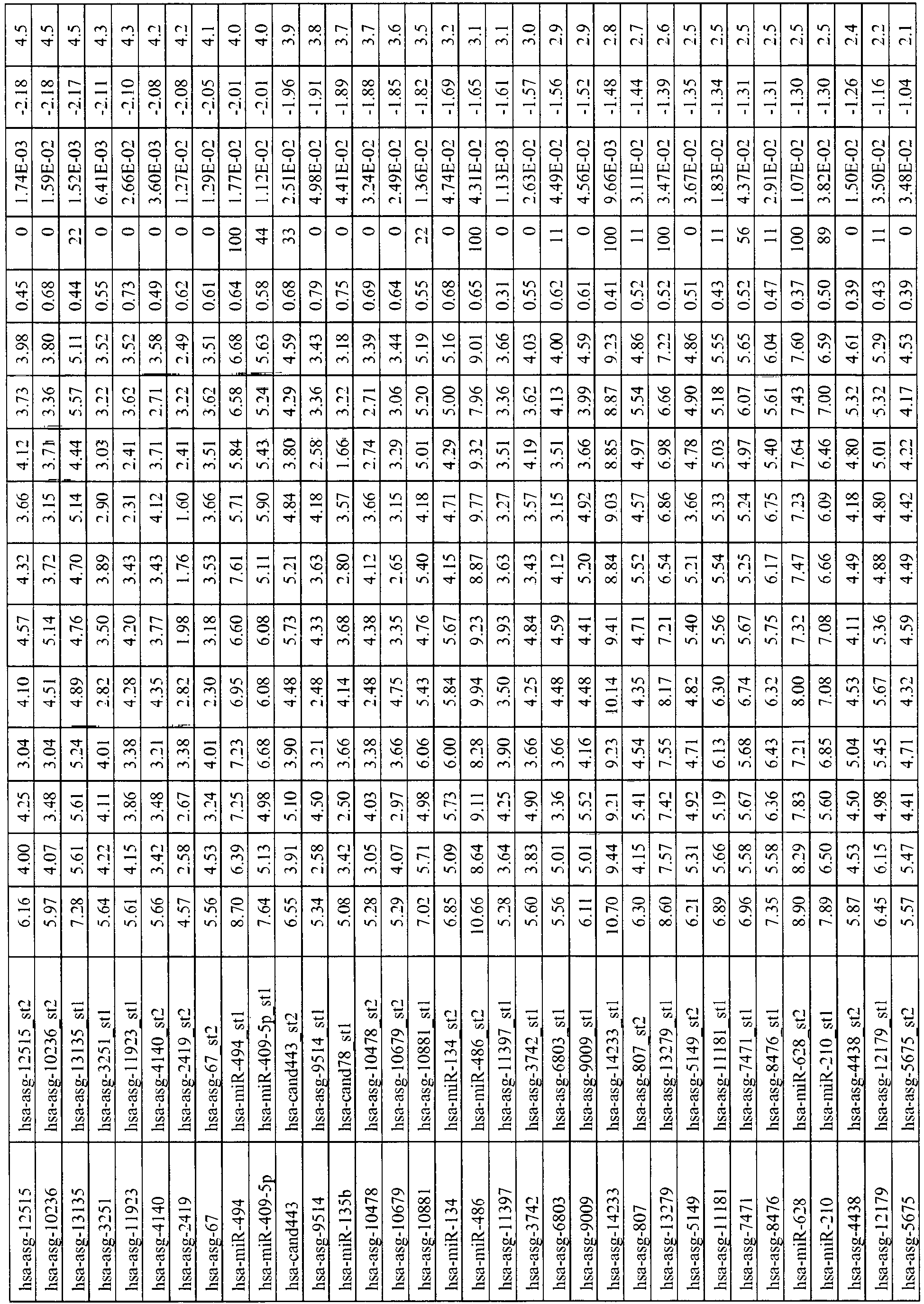

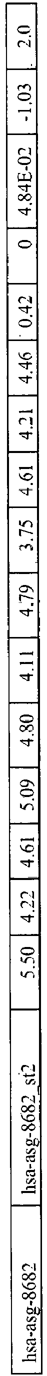

### EXAMPLE 34:

### MIRNAS DIFFERENTALLY EXPRESSED BETWEEN NORMAL BRAIN TISSUE AND NORMAL LYMPH NODES

To identify miRNAs that may be useful markers for metastasis of cancerous brain cells to lymph nodes, the inventors identified miRNAs that are expressed at significantly higher levels in normal brain tissue than in cancer-negative lymph nodes.

Total RNA, purified from one normal human brain sample (FirstChoice^{®} Human Brain Total RNA, cat. no. AM7962; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 180 miRNAs (81 verified, 99 predicted) with significant differential expression between the normal brain and normal lymph node samples (log2Diff brain vs LN neg ≥1 and p-value<0.05) (Table 33). Among the verified miRNAs, hsa-miR-124a was expressed at a level more than 1000-fold higher in the brain sample; 5 miRNAs (hsa-miR-433, -383, -129, -487b, and -323) were expressed at levels between 100- and 210-fold higher in brain; 12 miRNAs were expressed at levels between 50- and 100-fold higher in brain; 21 miRNAs were expressed at levels between 20- and 50-fold higher in brain; 15 miRNAs were expressed at levels between 10- and 20-fold higher in brain; and 15 miRNAs were expressed at levels between 5- and 10-fold higher in the brain sample than in normal lymph nodes. Among the predicted miRNAs, hsa-asg-13159_st1 was expressed at a level over 1000-fold higher in the brain sample; 3 miRNAs (hsa-miR-13404_st2, -3373_st2 and hsa-miR202_st2) were expressed at levels between 100- and 200-fold higher in brain; 4 miRNAs were expressed at levels between 50- and 100- fold higher in brain; 10 miRNAs were expressed at levels between 20- and 50-fold higher in brain; 20 miRNAs were expressed at levels between 10- and 20- fold higher in brain; and 27 miRNAs were expressed at levels between 5- and 10-fold higher in the brain sample than in normal lymph nodes.

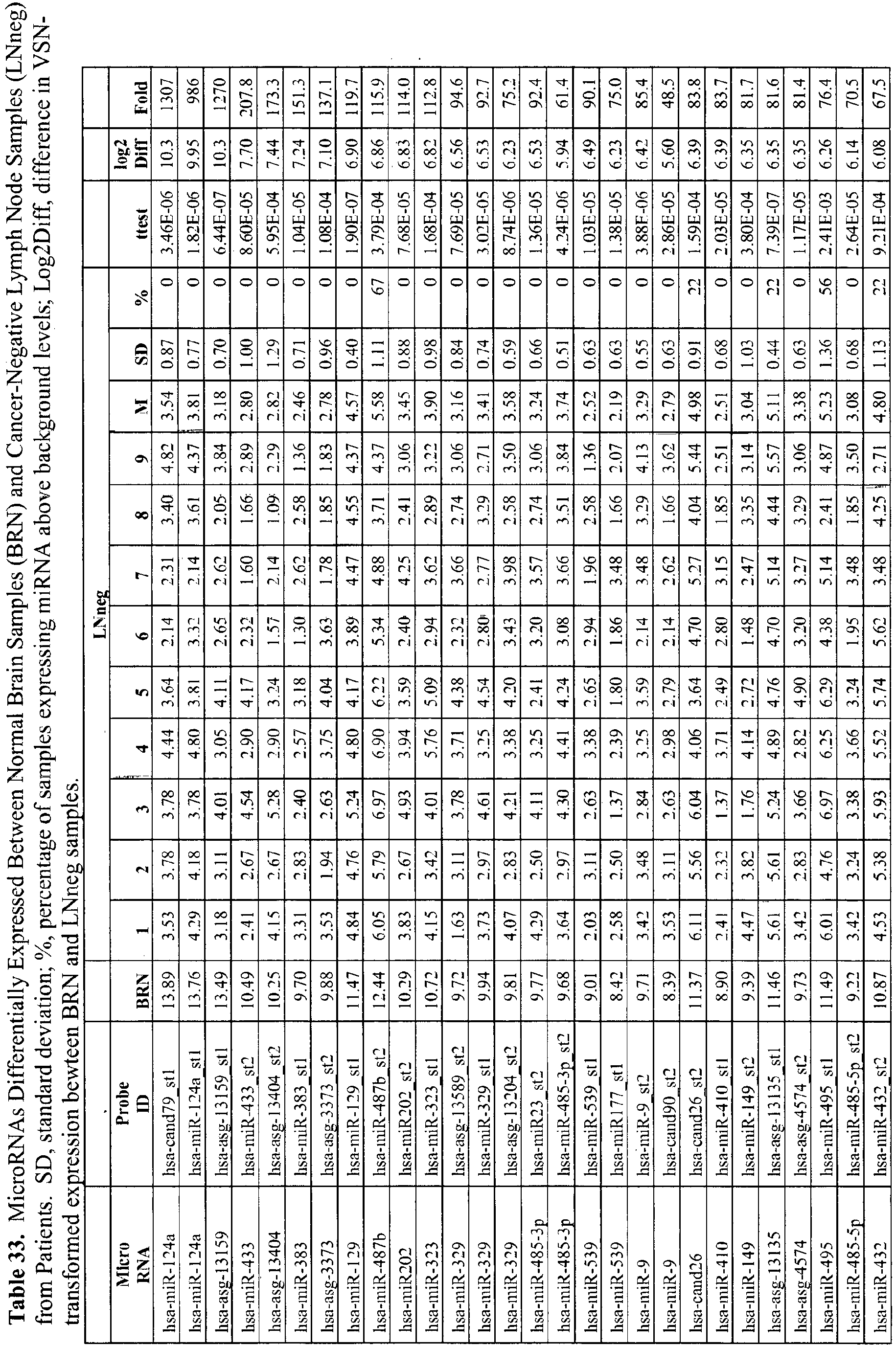

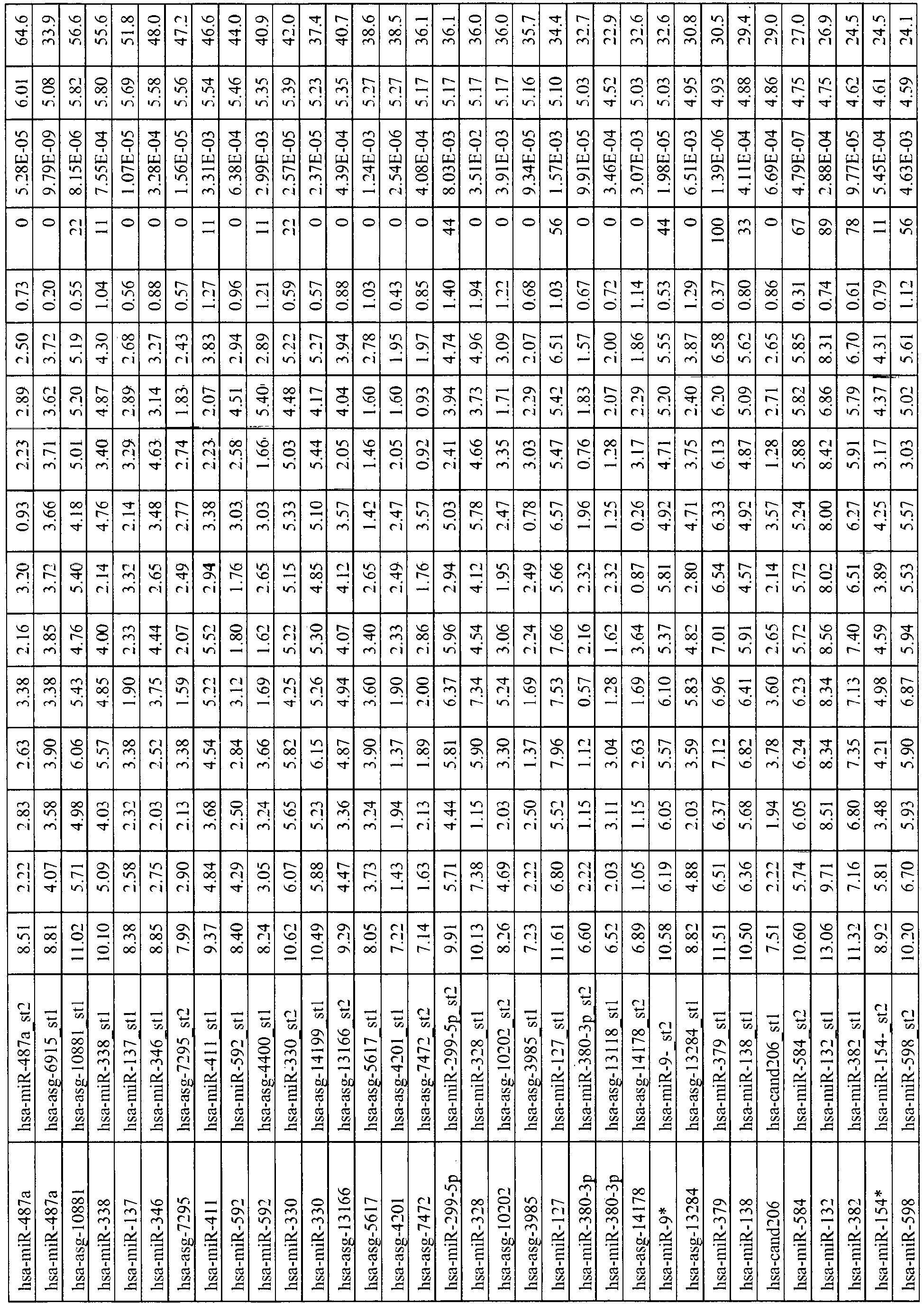

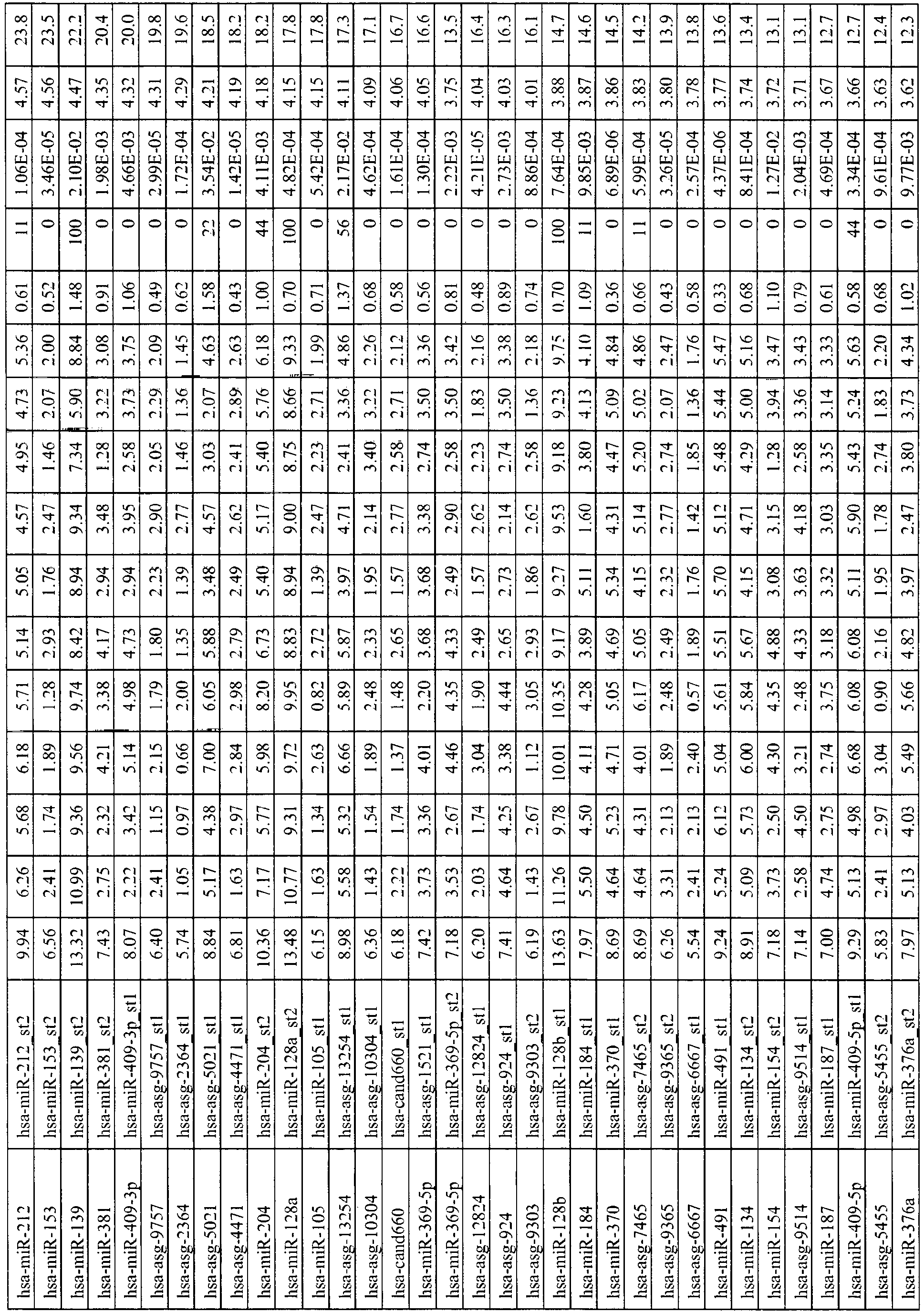

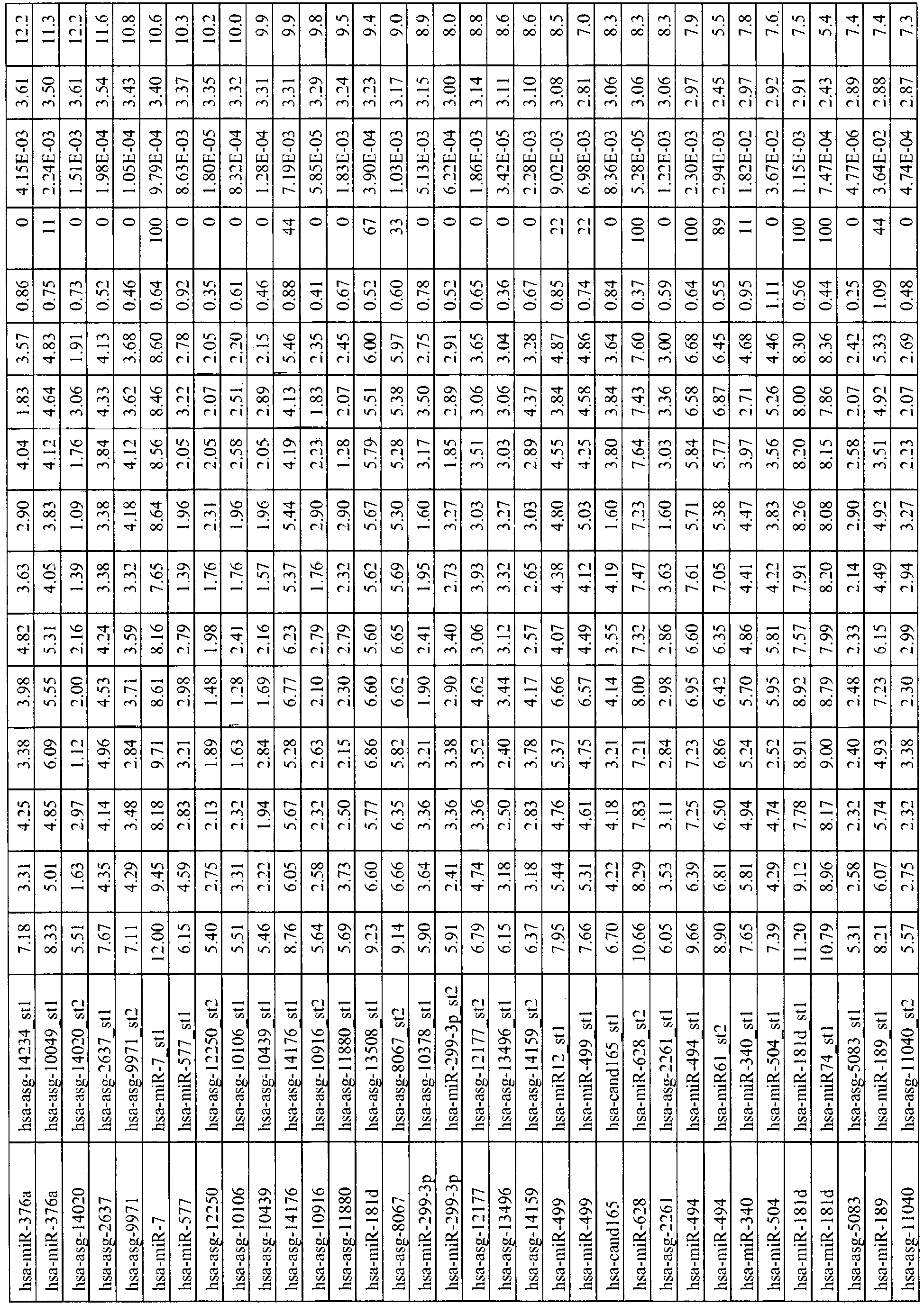

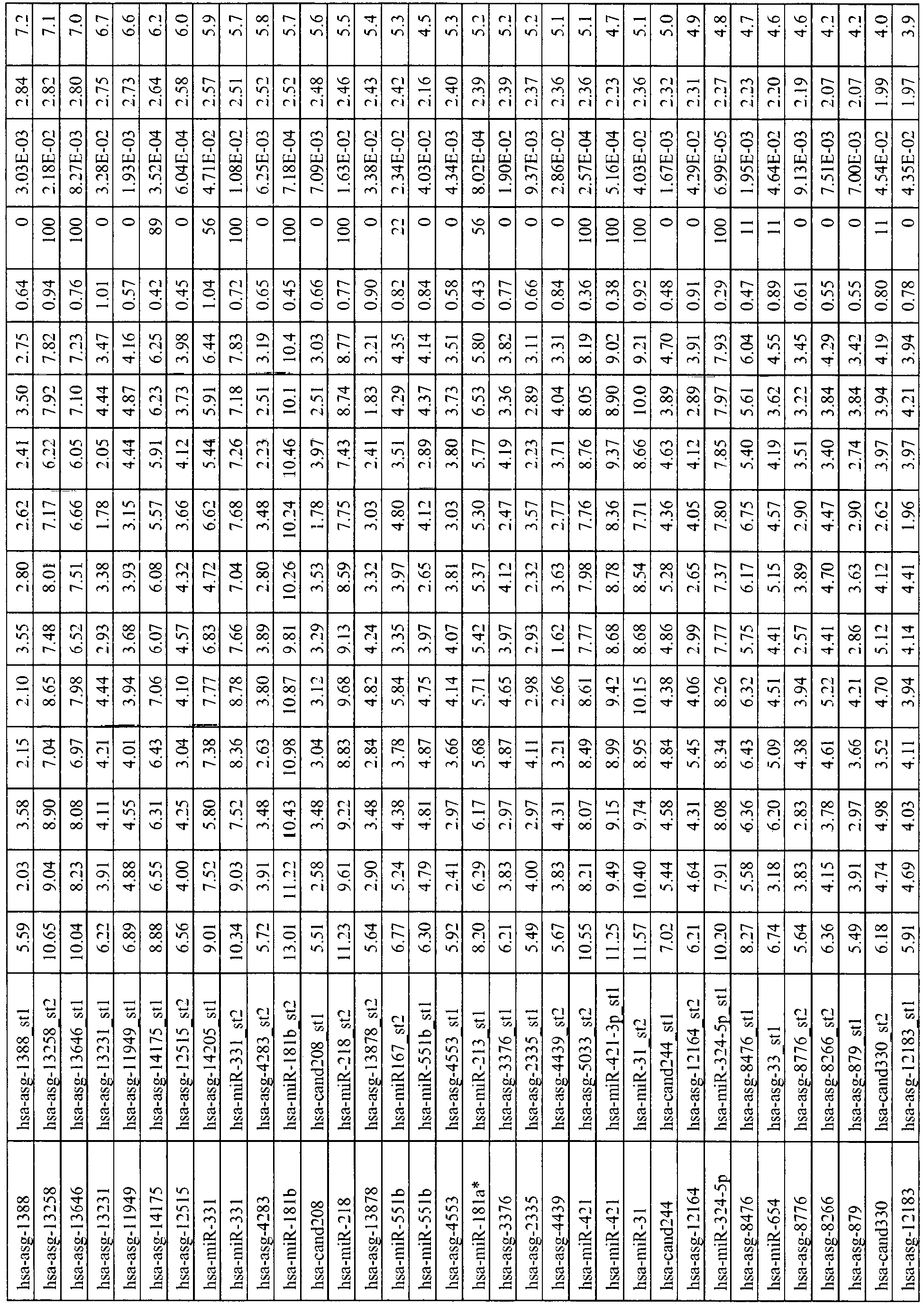

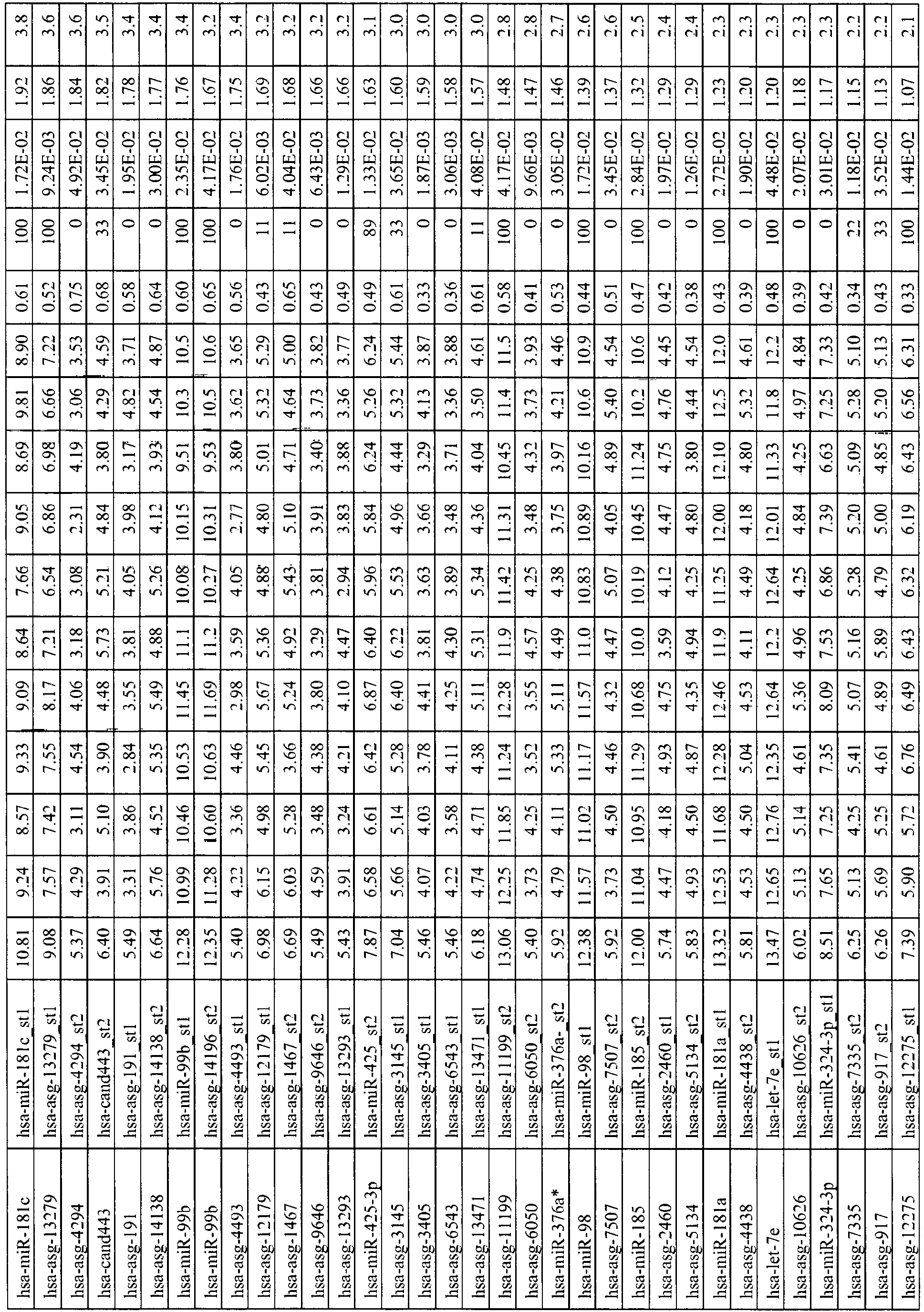

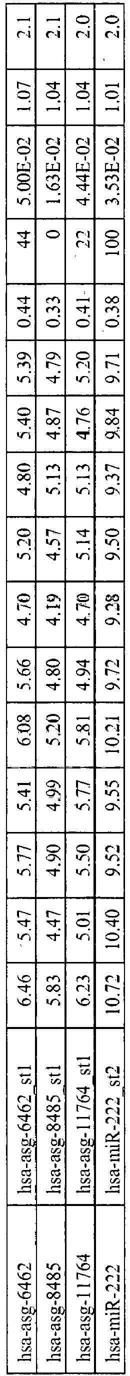

### EXAMPLE 35:

### MIRNAS DIFFERENTALLY EXPRESSED BETWEEN NORMAL THYMUS TISSUE AND NORMAL LYMPH NODES

To identify miRNAs that may be useful markers for metastasis of cancerous thymus cells to lymph nodes, the inventors identified miRNAs that are expressed at significantly higher levels in normal thymus tissue than in cancer-negative lymph nodes.

Total RNA, purified from one normal human thymus sample (FirstChoice^{®} Human Thymus Total RNA, cat. no. AM7964; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 108 miRNAs (44 verified, 64 predicted) with significant differential expression between the normal thymus and normal lymph node samples (|log2Diff| thymus vs LN neg ≥1 and p-value<0.05) (Table 34). Among the verified miRNAs, hsa-miR-205 was expressed at a level over 300- fold higher in the thymus sample; hsa-miR-383 and -429 were expressed at levels between 50- and 70-fold higher in thymus; 15 miRNAs were expressed at levels between 10- and 40-fold higher in thymus; and 15 miRNAs were expressed at levels between 5- and 10-fold higher in the thymus sample than in normal lymph nodes. Among the predicted miRNAs, hsa-asg-14153_st2 was expressed at a level over 50-fold higher in the thymus sample; 6 miRNAs (hsa-miR-7637_st2, -13404_st2, - 1088_st1, -11715_st2, -3711_st2, and hsa-miR202_st2) were expressed at levels between 10-and 30-fold higher in thymus; and 17 miRNAs were expressed at levels between 5- and 10-fold higher in the thymus samples than in normal lymph nodes.

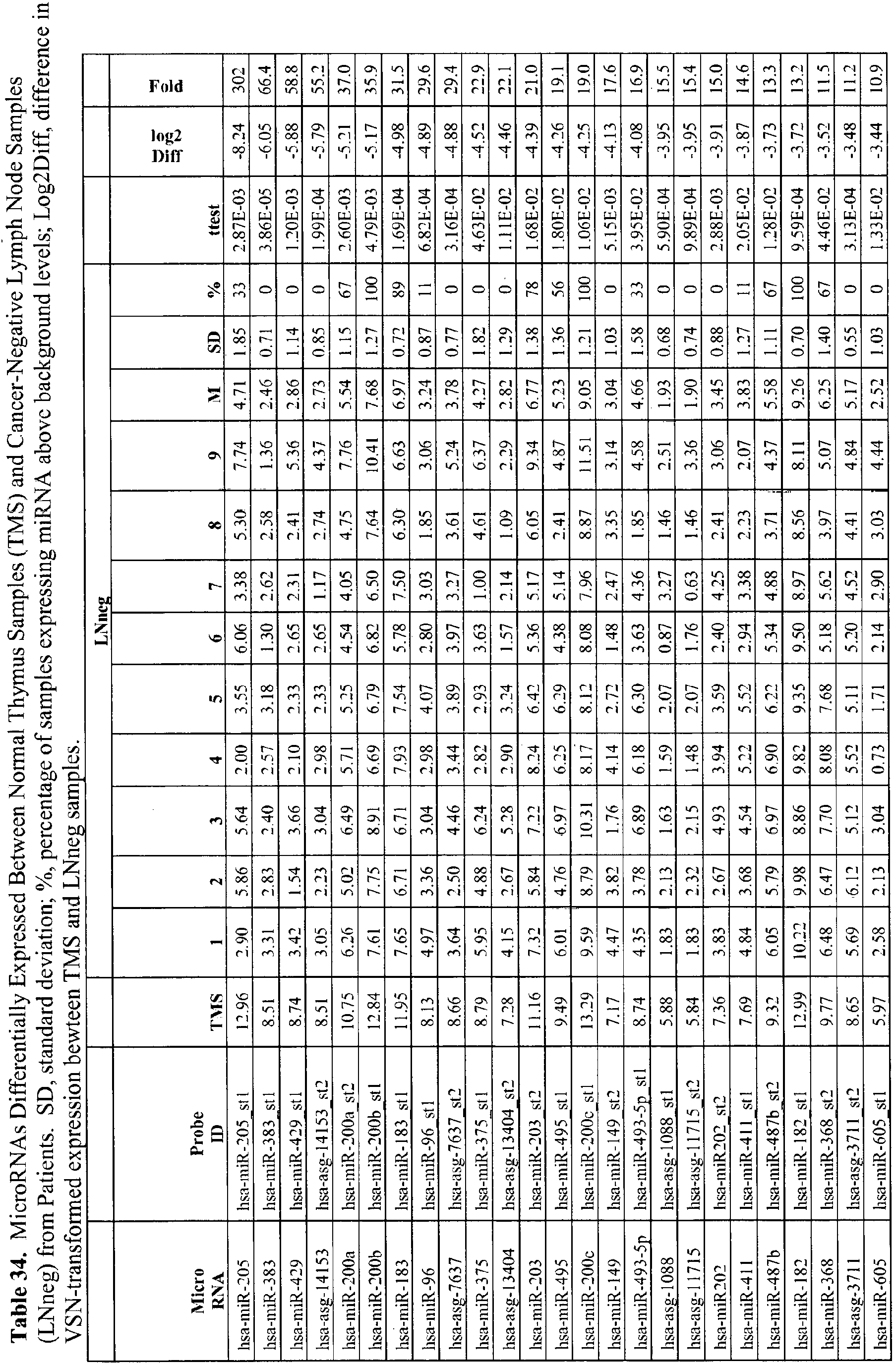

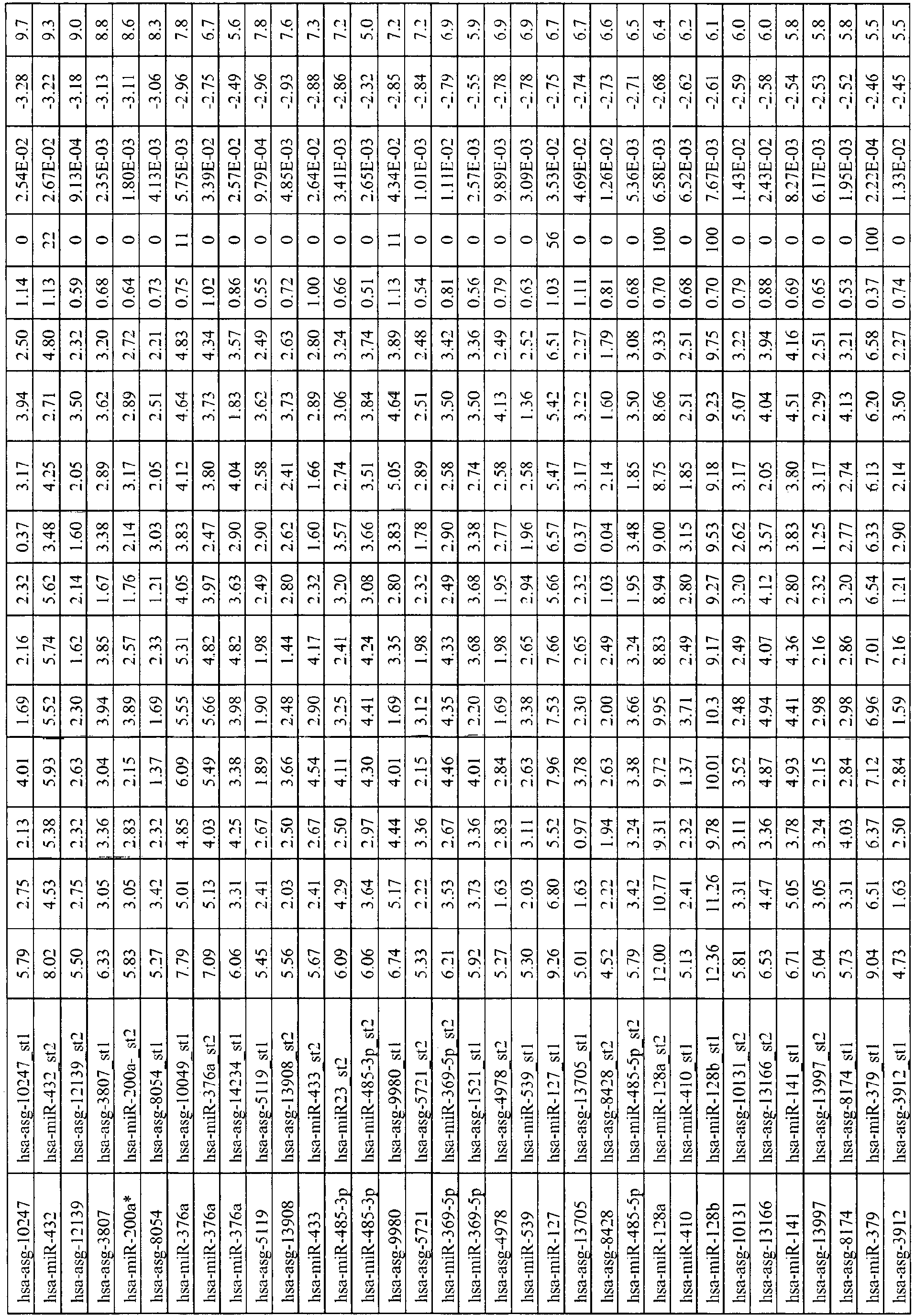

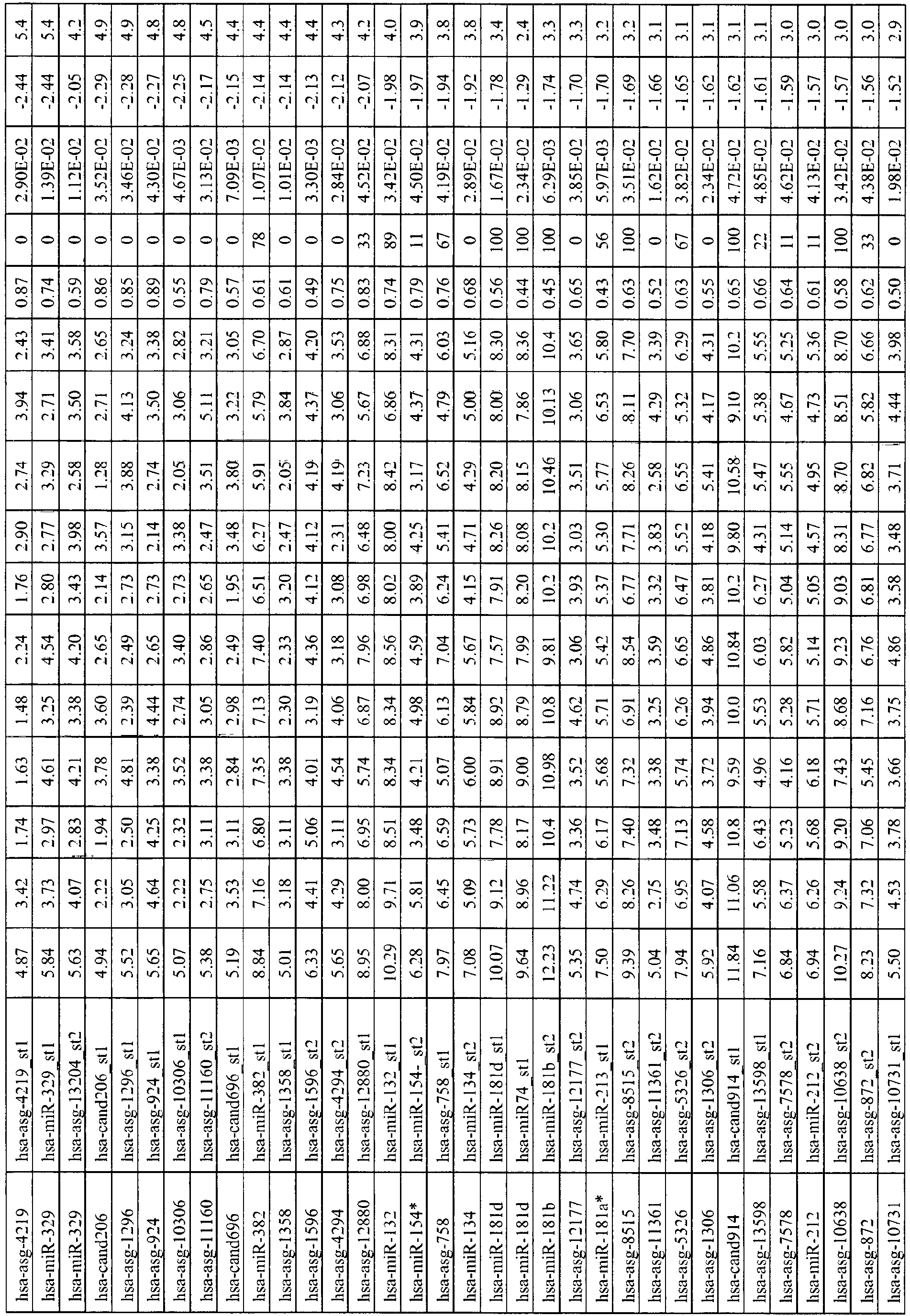

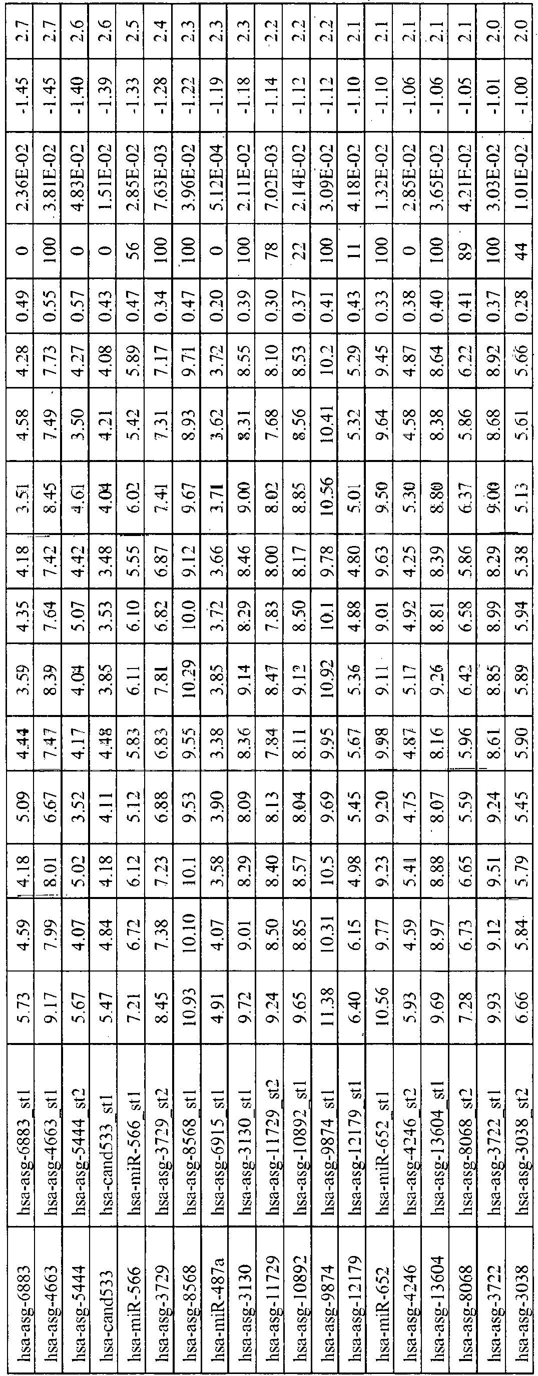

### EXAMPLE 36:

### MIRNAS DIFFERENTALLY EXPRESSED BETWEEN NORMAL TRACHEA TISSUE AND NORMAL LYMPH NODES

To identify miRNAs that may be useful markers for metastasis of cancerous trachea cells to lymph nodes, the inventors identified miRNAs that are expressed at significantly higher levels in normal trachea tissue than in cancer-negative lymph nodes.

Total RNA, purified from one normal human trachea sample (FirstChoice^{®} Human Trachea Total RNA, cat. no. AM6846; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 97 miRNAs (38 verified, 59 predicted) with significant differential expression between the normal trachea and normal lymph node samples (|log2Diff| trachea vs LN neg ≥1 and p-value<0.05) (Table 35). Among the verified miRNAs, hsa-miR-375 and -205 were expressed at levels between 300- and 400-fold higher in the trachea sample; hsa-miR-429 was expressed at a level between 100- and 200-fold higher in trachea; hsa-miR-200a, -200b, and -449 were expressed at levels between 50- and 100-fold higher in trachea; 12 miRNas were expressed at levels between 10- and 50-fold higher in trachea; and 12 miRNAs were expressed at levels between 5- and 10-fold higher in the trachea sample than in normal lymph nodes. Among the predicted miRNAs, hsa-asg-13231_st1 and -14153_st2 were expressed at levels over 100-fold higher in the trachea sample; 4 miRNAs (hsa-asg-4439_st2, -9269_st2, -7637_st2, and cand150_st2) were expressed at levels between 50- and 100-fold higher in trachea; 17 miRNAs were expressed at levels between 10- and 30-fold higher in trachea; and 15 miRNAs were expressed at levels between 5- and 10-fold higher in the trachea sample than in normal lymph nodes.

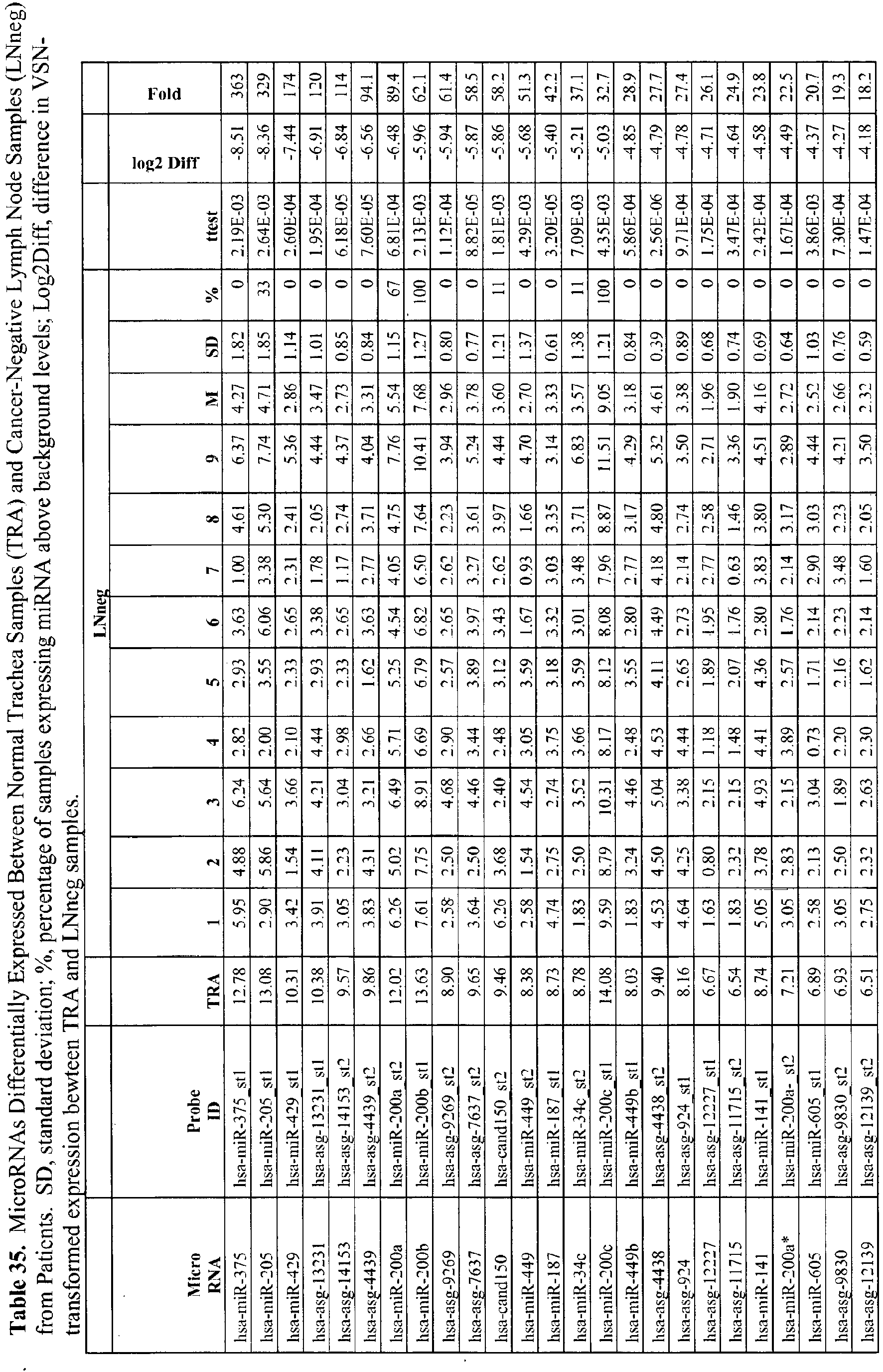

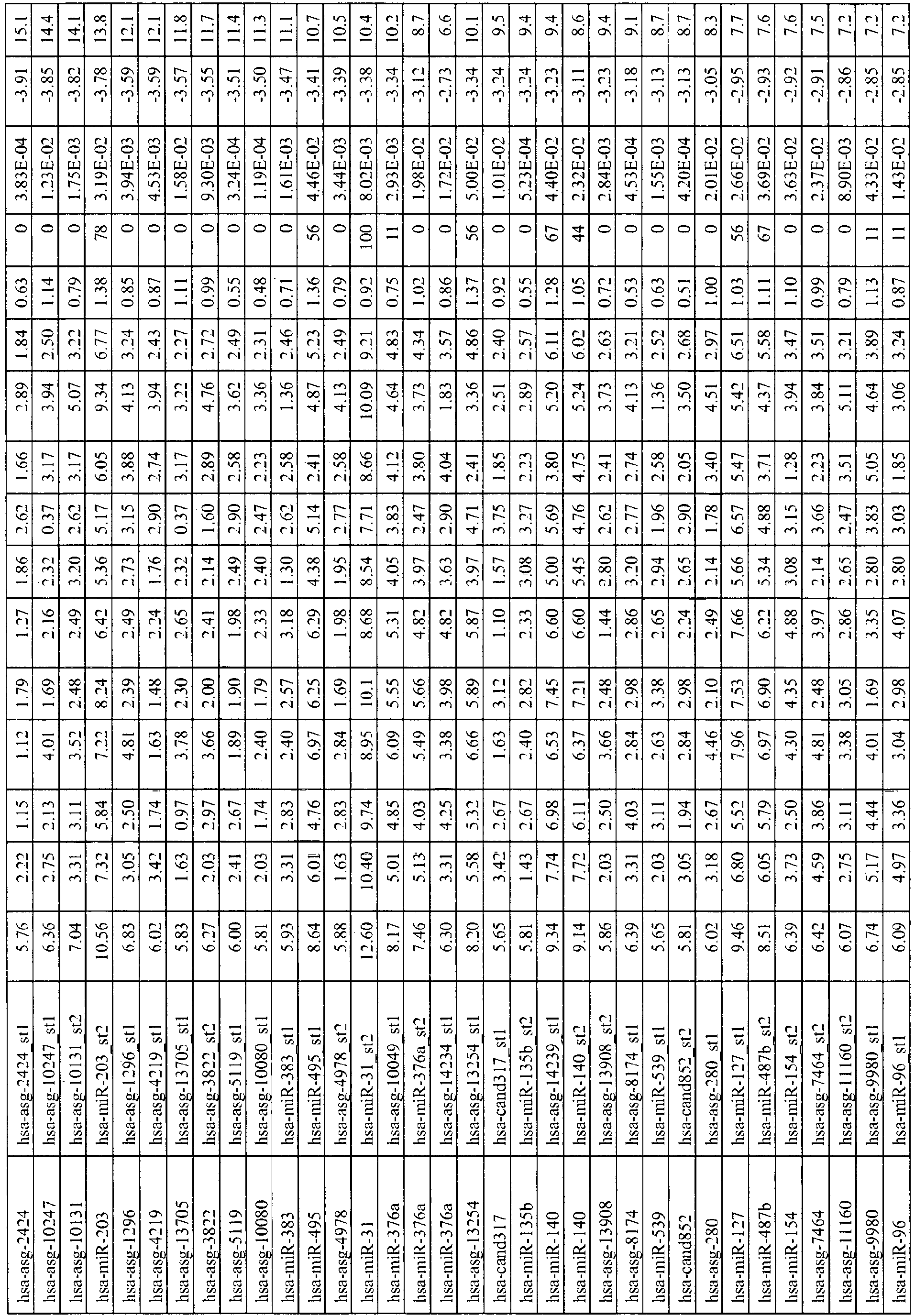

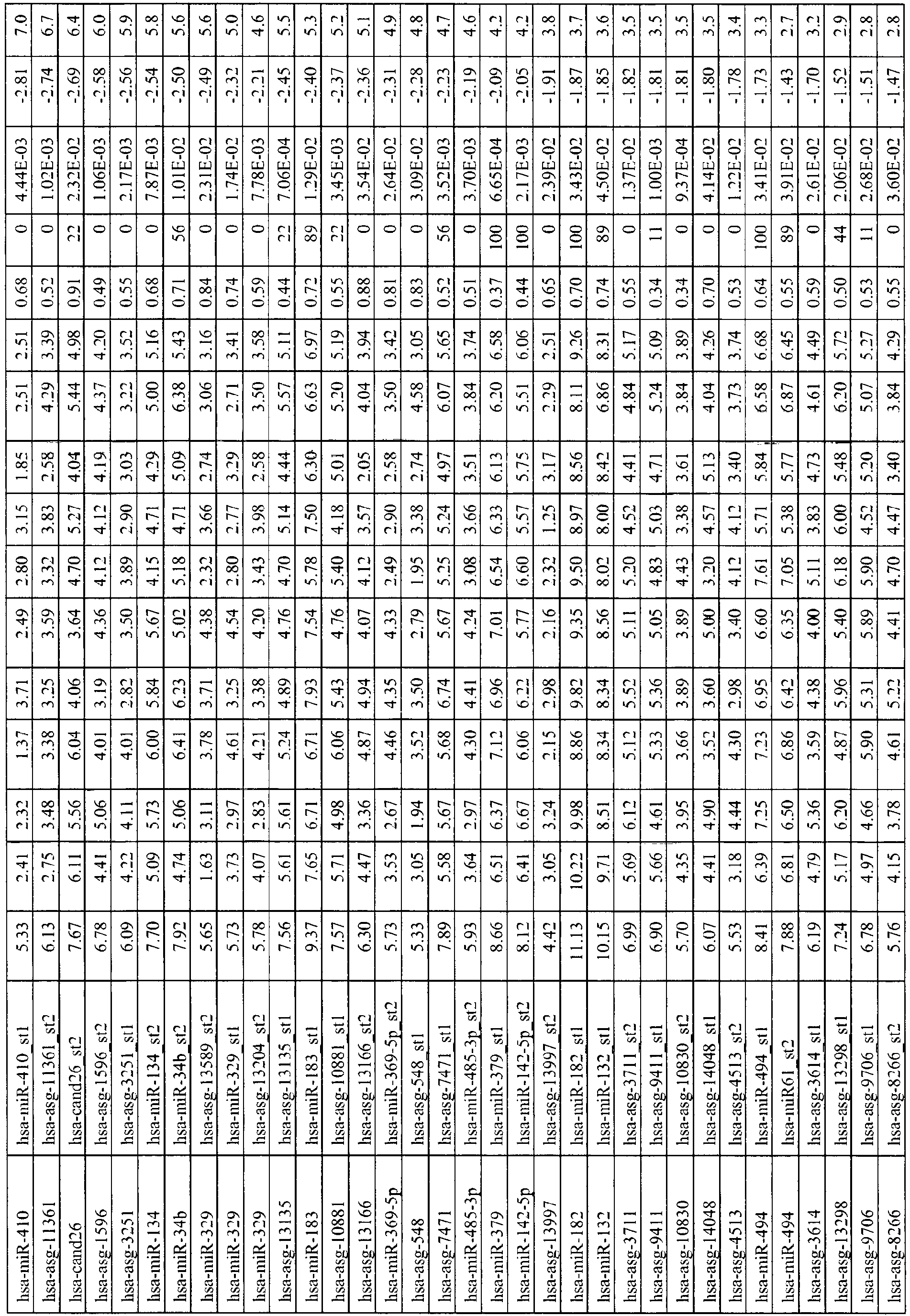

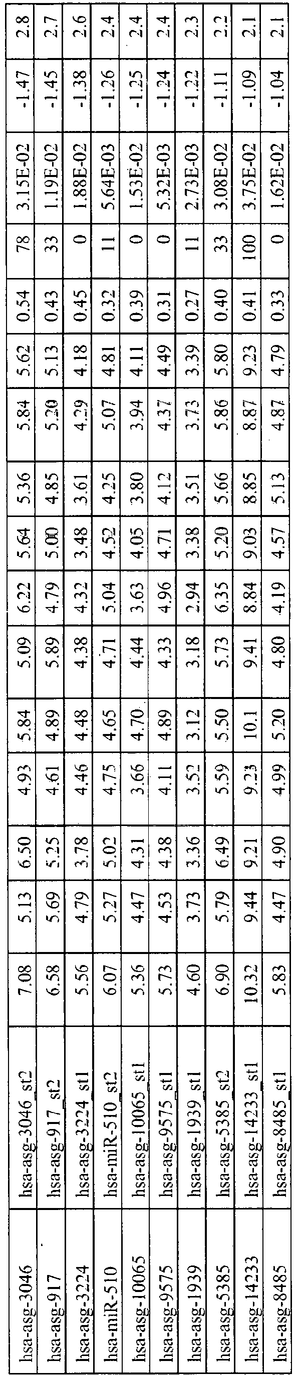

### EXAMPLE 37:

### MIRNAS DIFFERENTALLY EXPRESSED BETWEEN NORMAL SMALL INTESTINE TISSUE AND NORMAL LYMPH NODES

To identify miRNAs that may be useful markers for metastasis of cancerous small intestine cells to lymph nodes, the inventors identified miRNAs that are expressed at significantly higher levels in normal small intestine tissue than in cancer-negative lymph nodes.

Total RNA, purified from one normal human small intestine sample (FirstChoice^{®} Human Small Intestine Total RNA, cat. no. AM7984; Ambion, Austin, TX, USA) and from nine normal human lymph node samples (FirstChoice^{®} Human Lymph Node Total RNA, cat. no. AM7894; Ambion, Austin, TX, USA) was purchased from Ambion. miRNA purification and labeling and microarray expression analyses were performed as described above in Example 2.

The inventors identified 413 miRNAs (28 verified, 385 predicted) with significant differential expression between the normal small intestine and normal lymph node samples (|log2Diff| small intestine vs LN neg ≥1 and p-value<0.05) (Table 36) Among the verified miRNAs, hsa-miR-429 and -375 were expressed at levels between 85- and 100 -fold higher in the small intestine sample; 4 miRNAs (hsa-miR-215, -200a, -187, and -200b)were expressed at levels between 25- and 50-fold higher in small intestine; 5 miRNAs (hsa-miR-141, -200c, -605, -194, and -802) were expressed at levels between 10- and 20-fold higher in small intestine; and 5 miRNAs were expressed at levels between 5- and 10-fold higher in the small intestine sample than in normal lymph nodes. Among the predicted miRNAs, 6 miRNA (hsa-asg-9939_st2, -7846_st1, -6880_st1, -14182_st1, -6838_st1, and -924_st1) were expressed at levels between 25- and 40-fold higher in the small intestine sample; 13 miRNAs were expressed at levels between 10- and 20-fold higher in small intestine; and 56 miRNAs were expressed at levels between 5- and 10-fold higher in the small intestine sample than in normal lymph nodes.

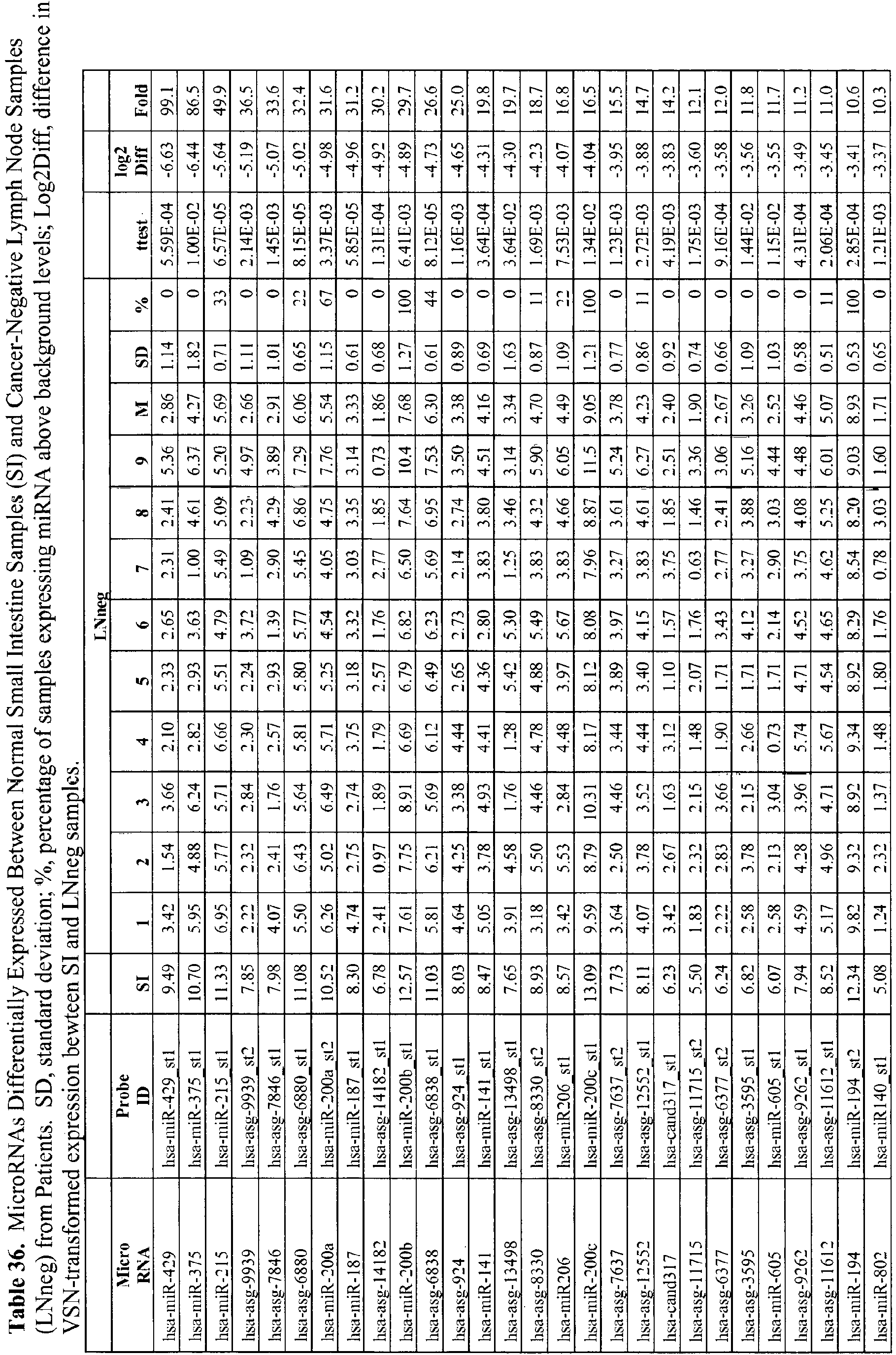

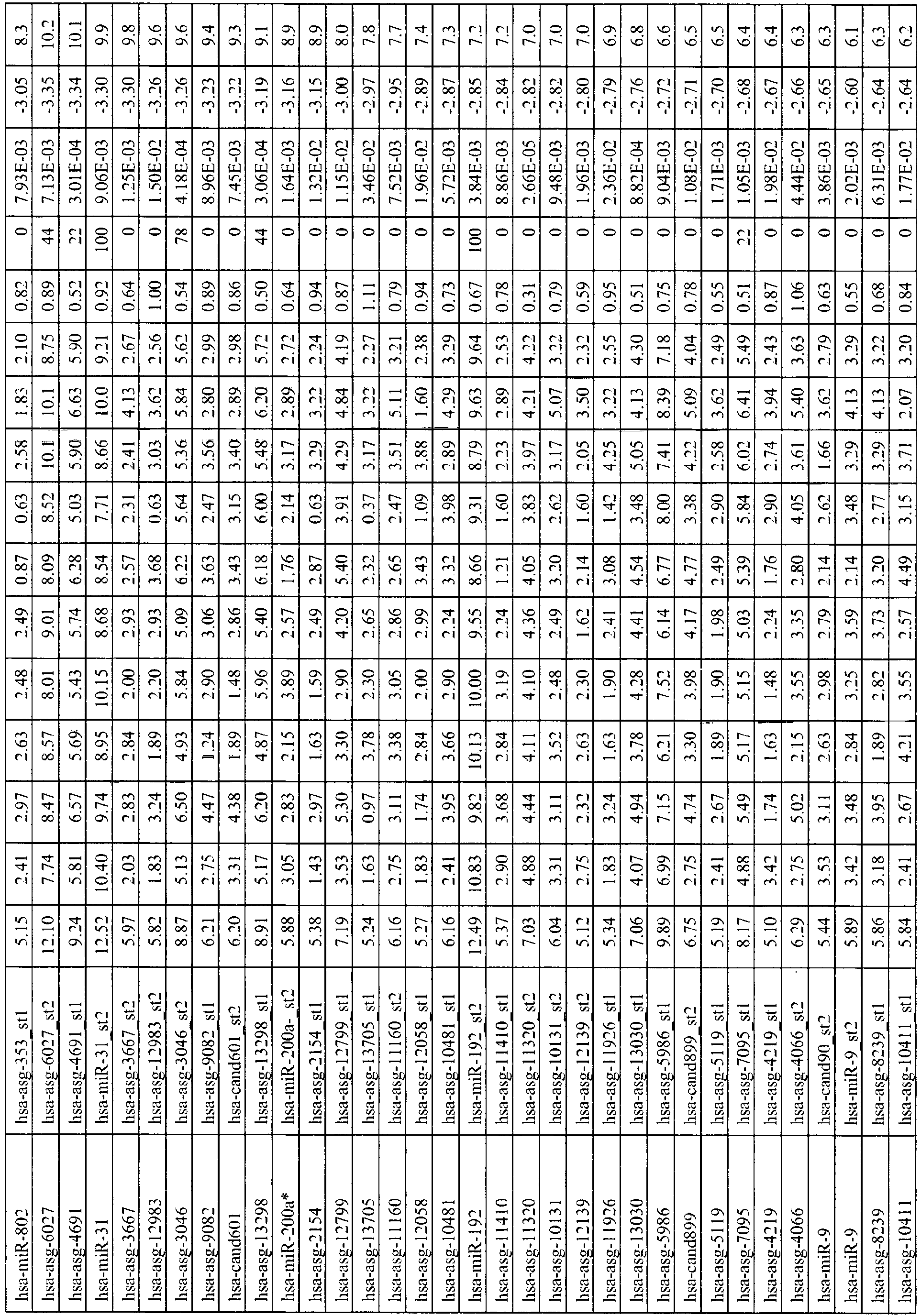

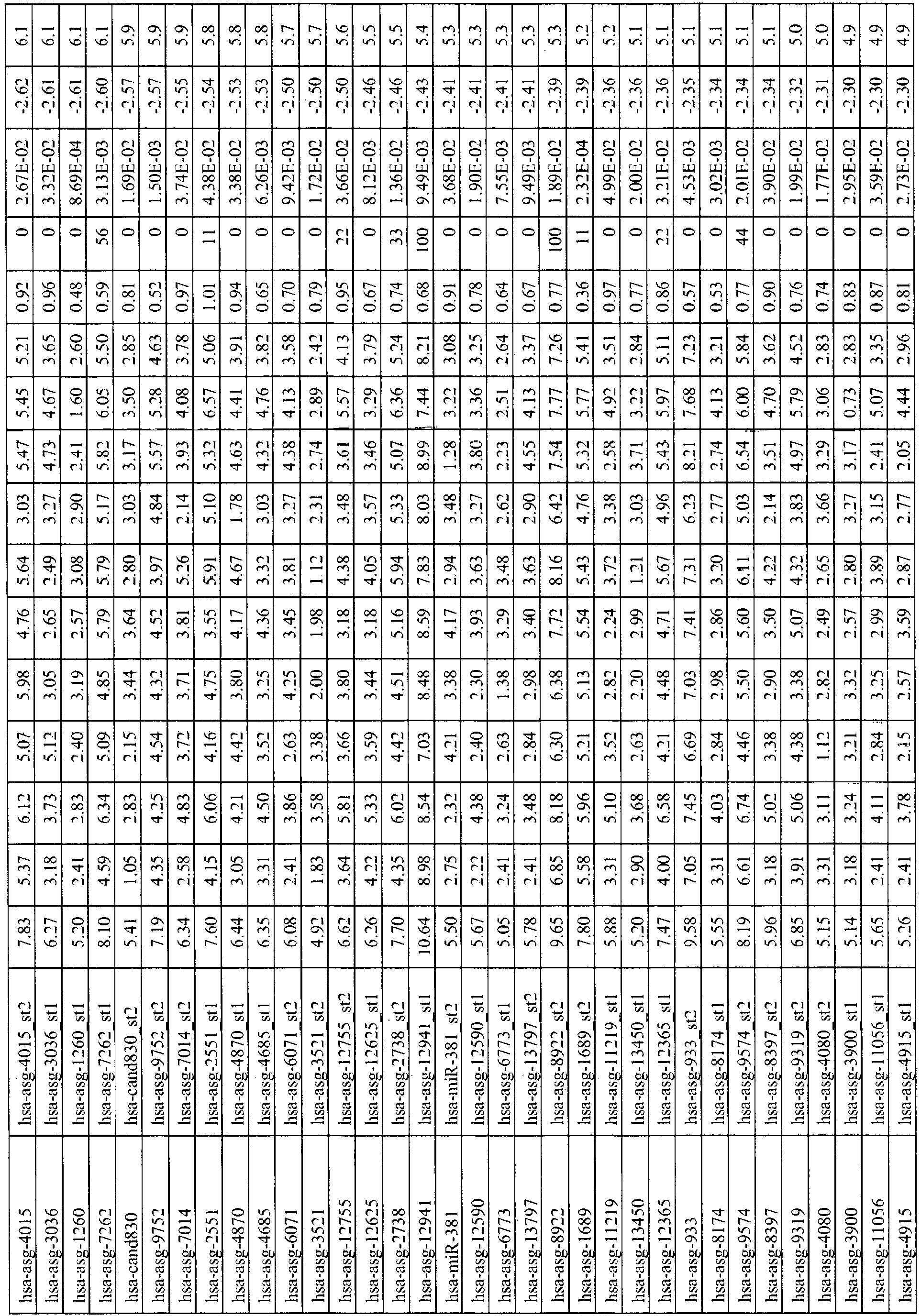

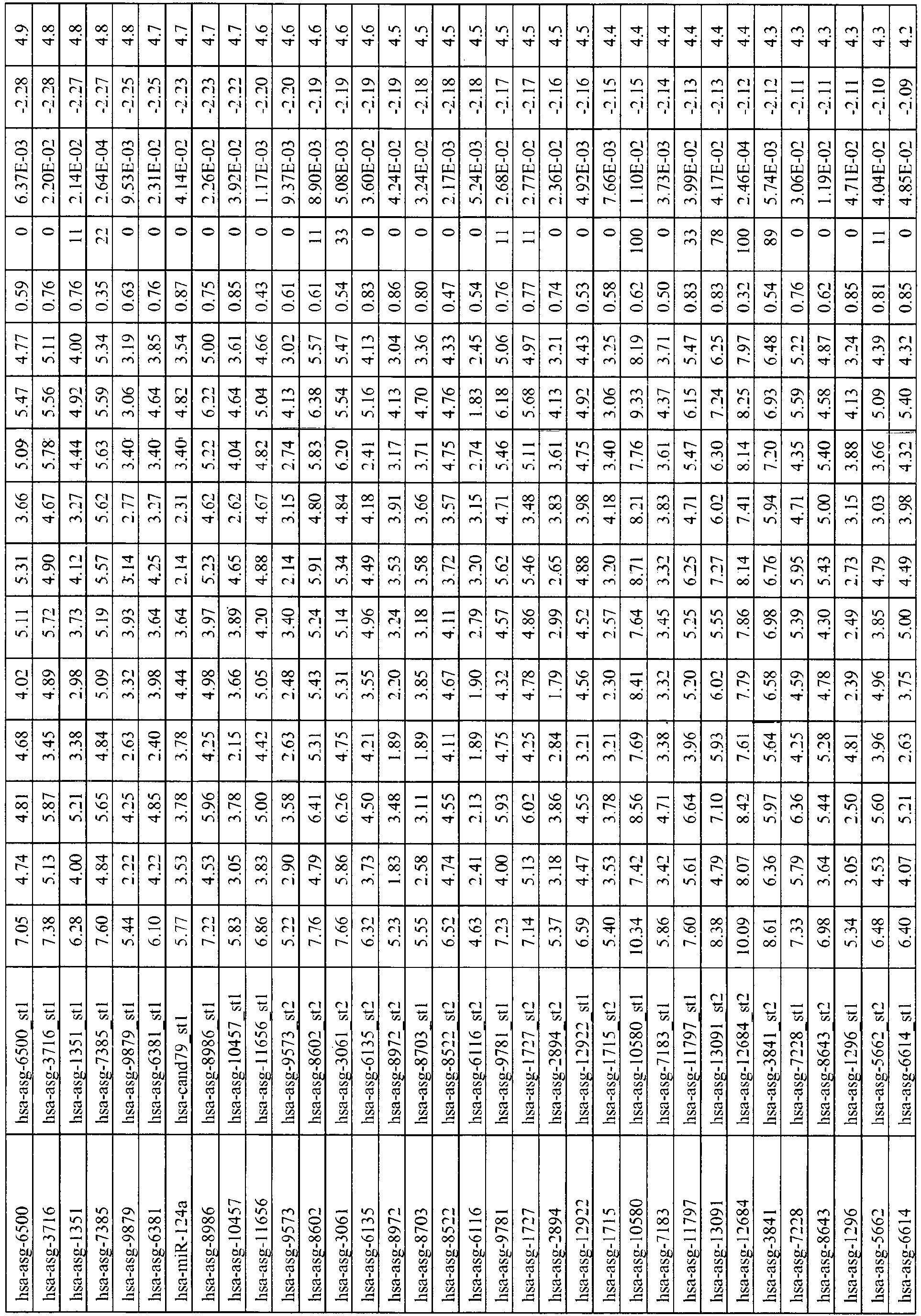

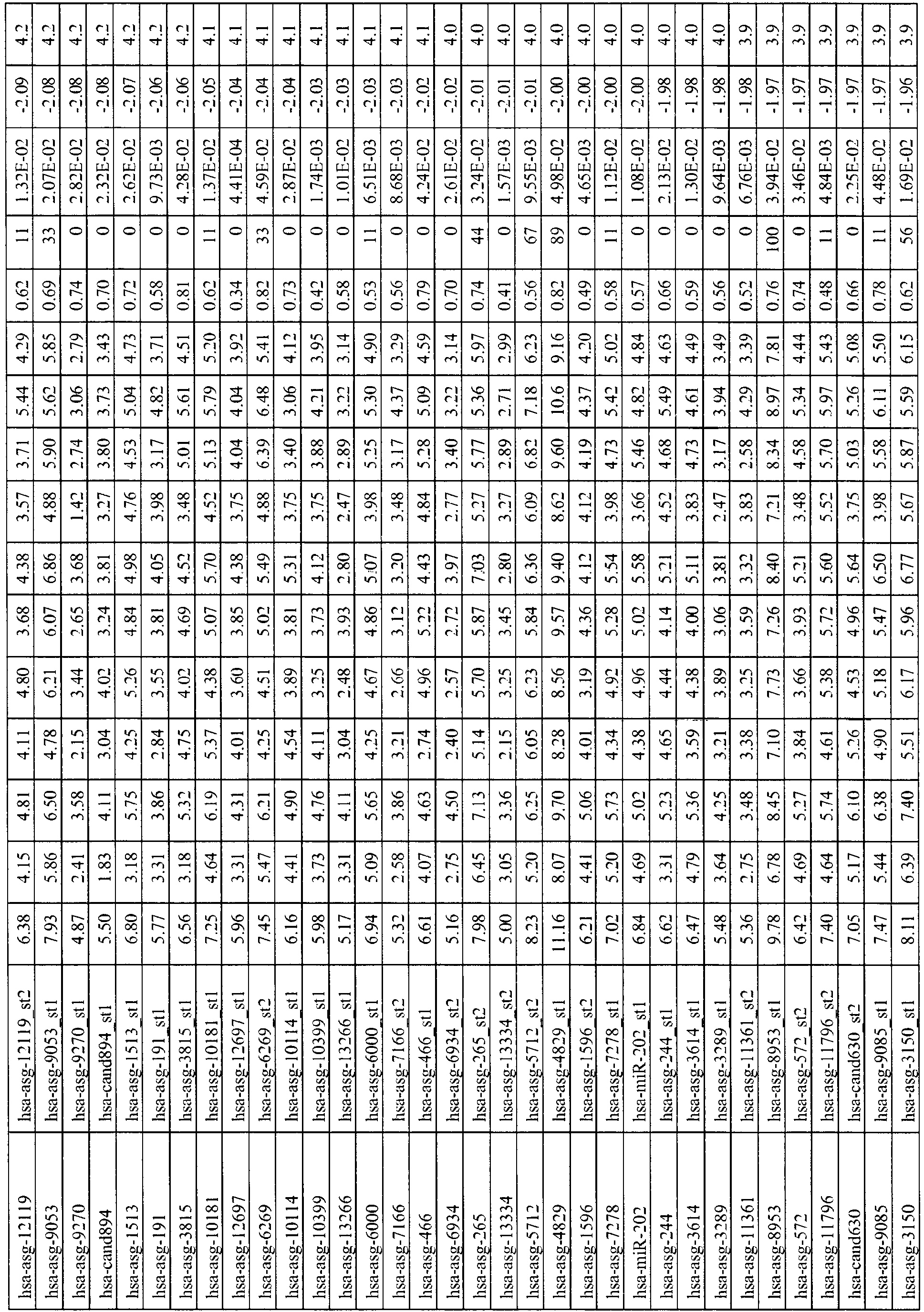

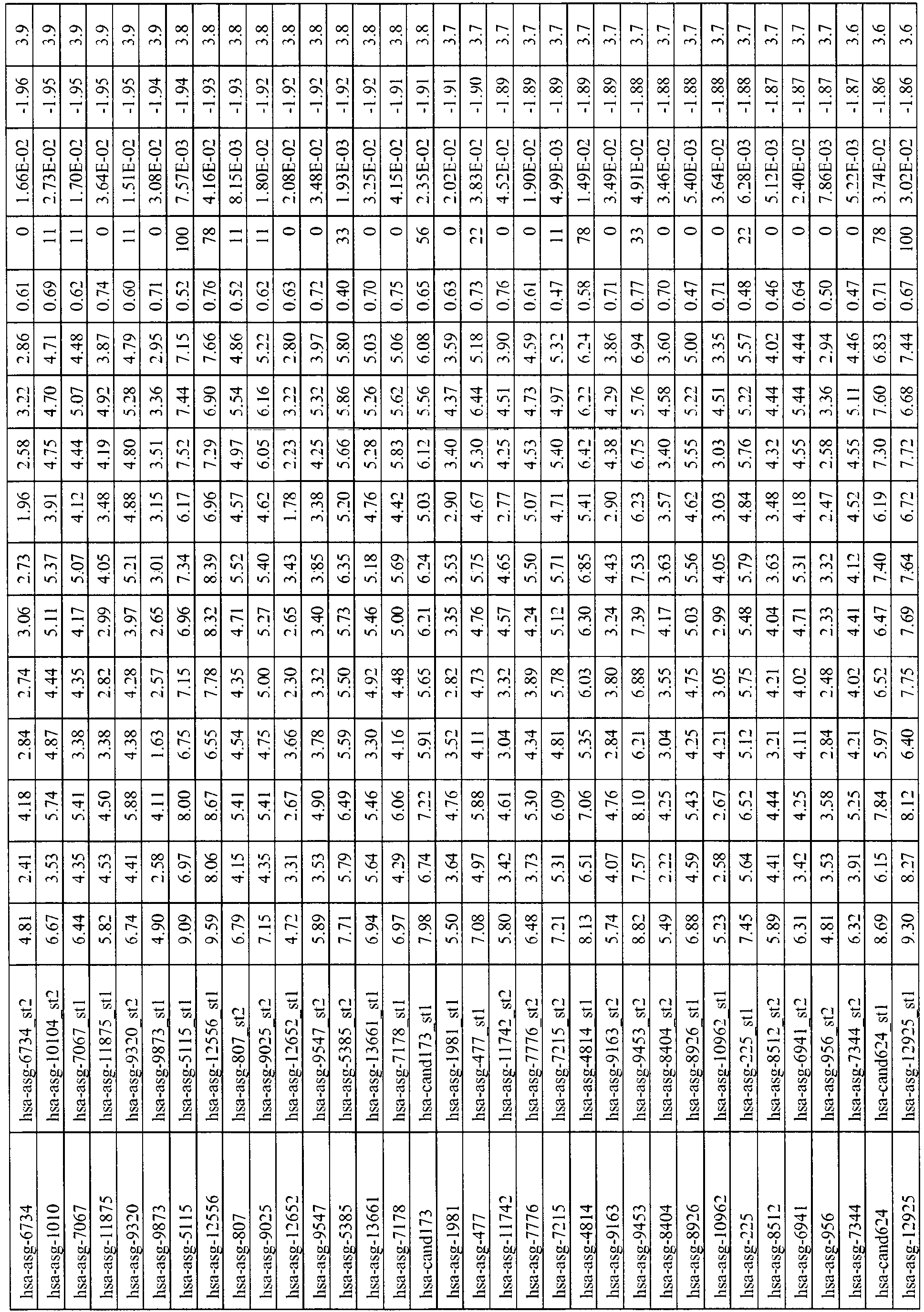

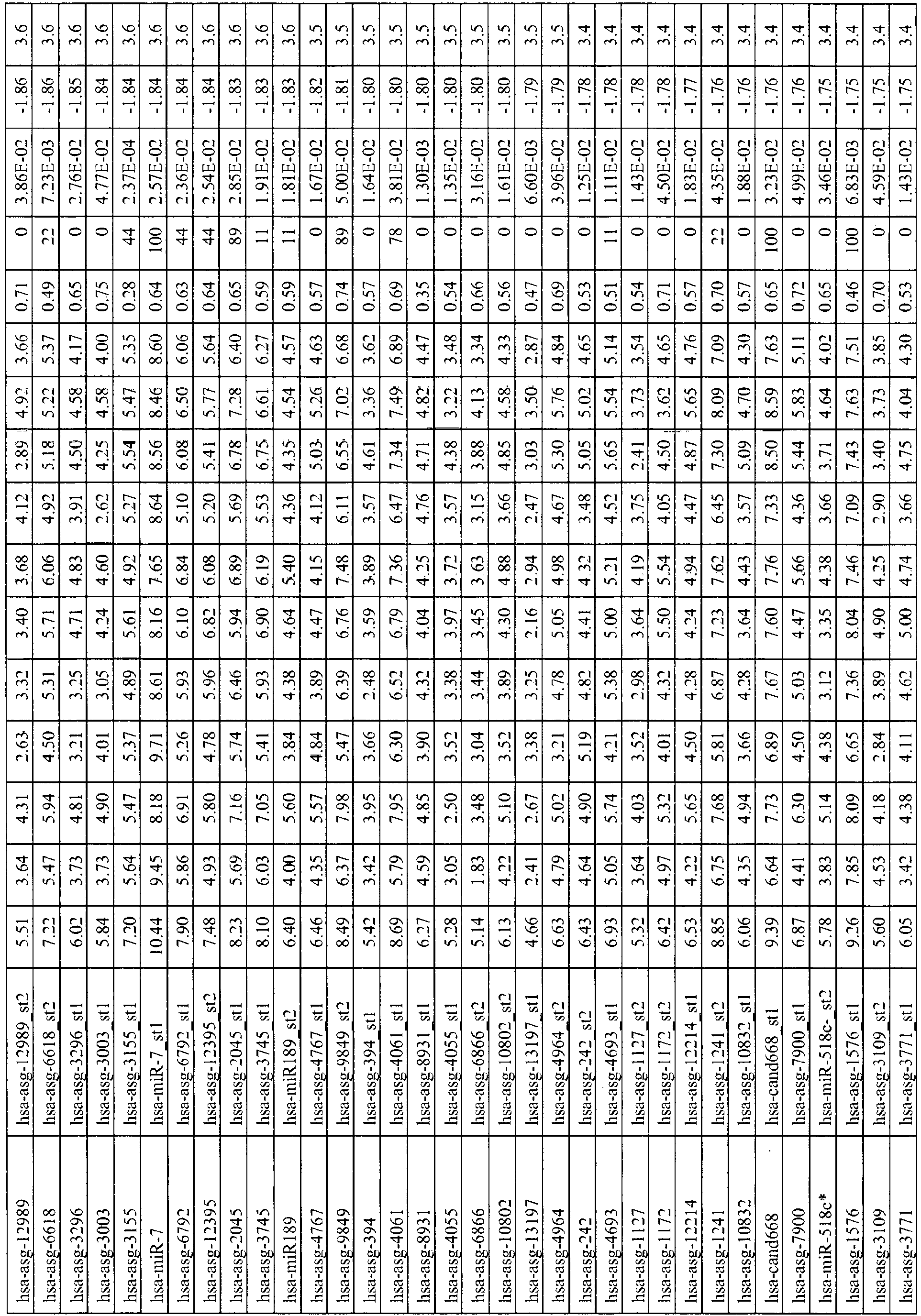

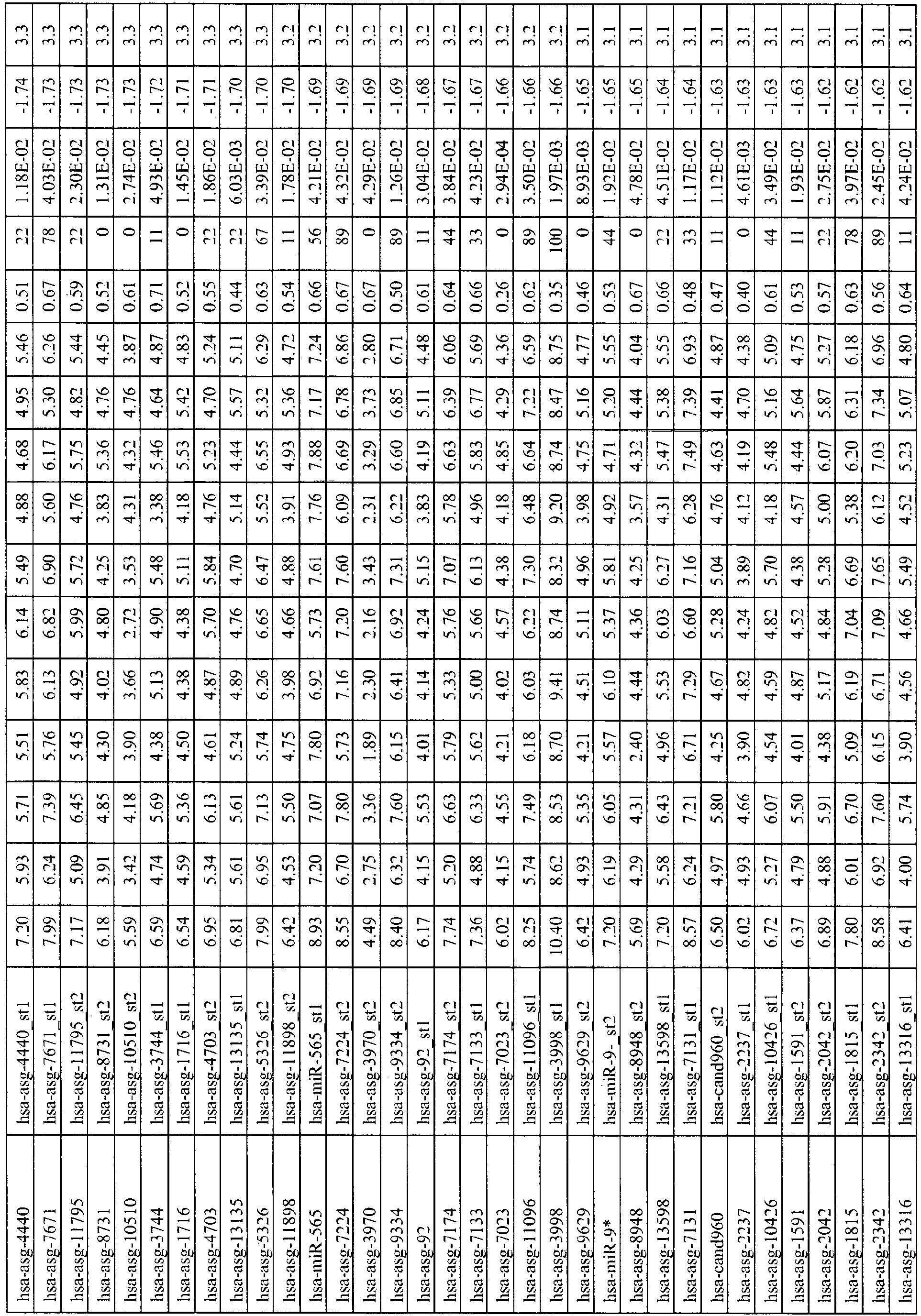

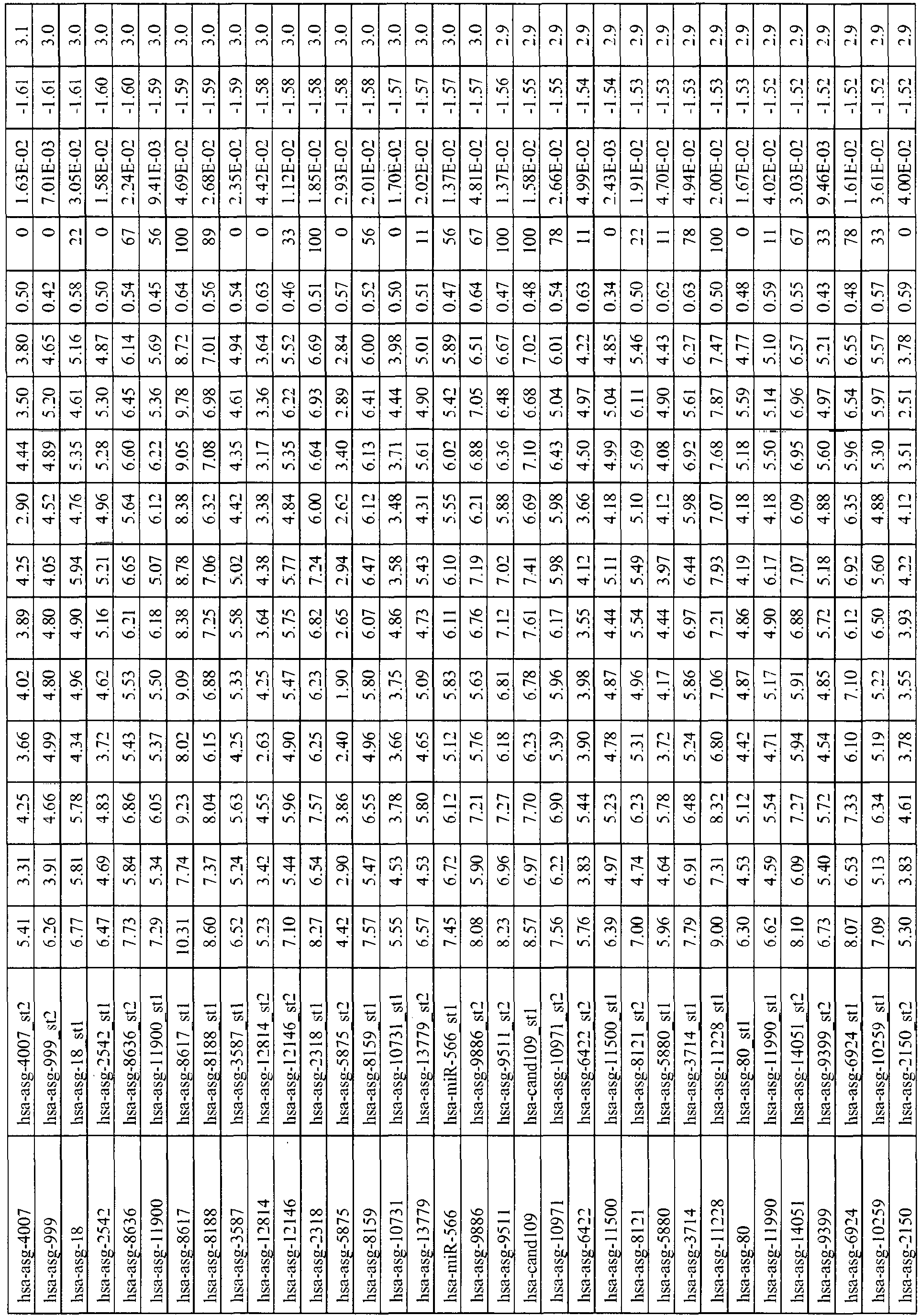

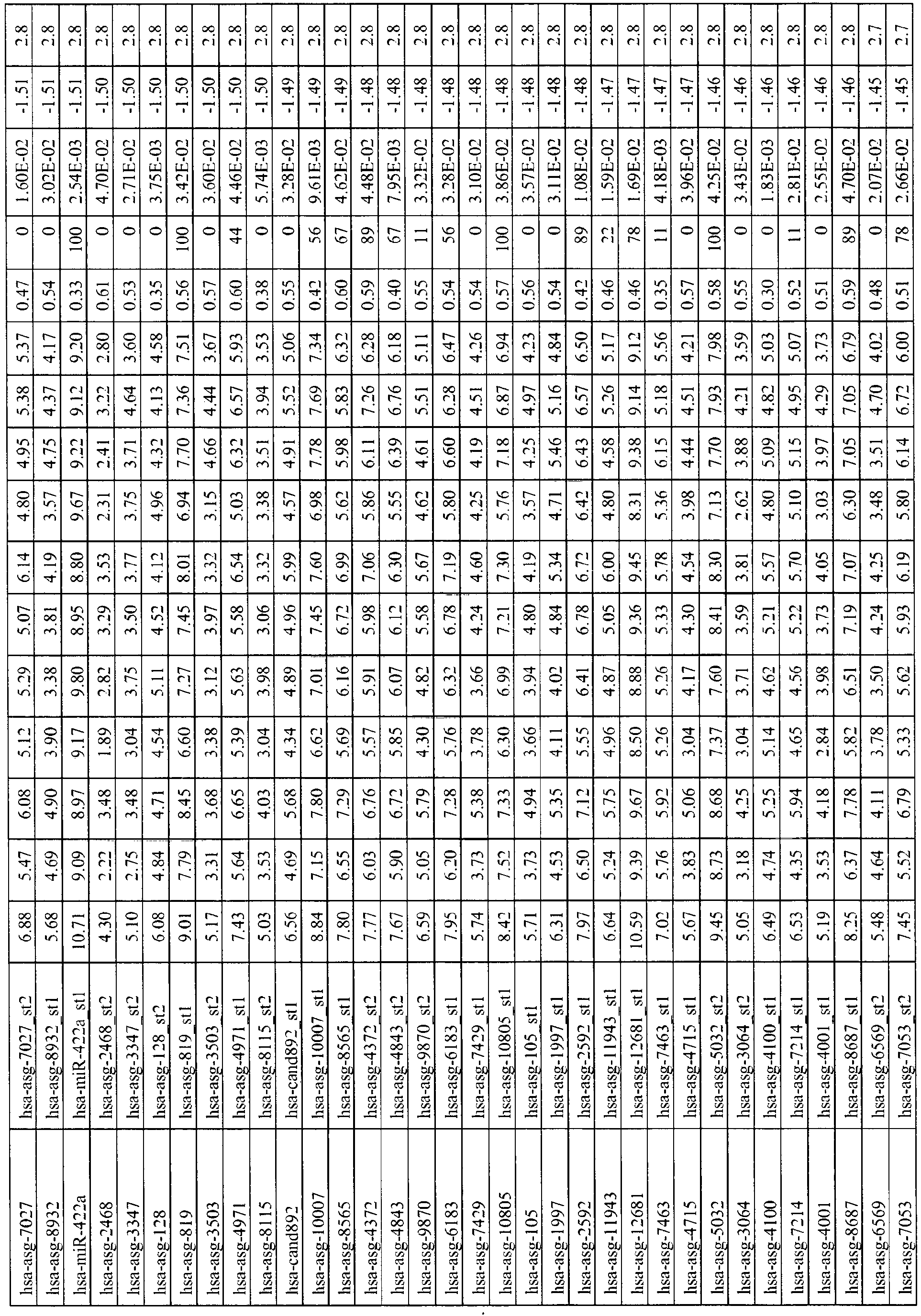

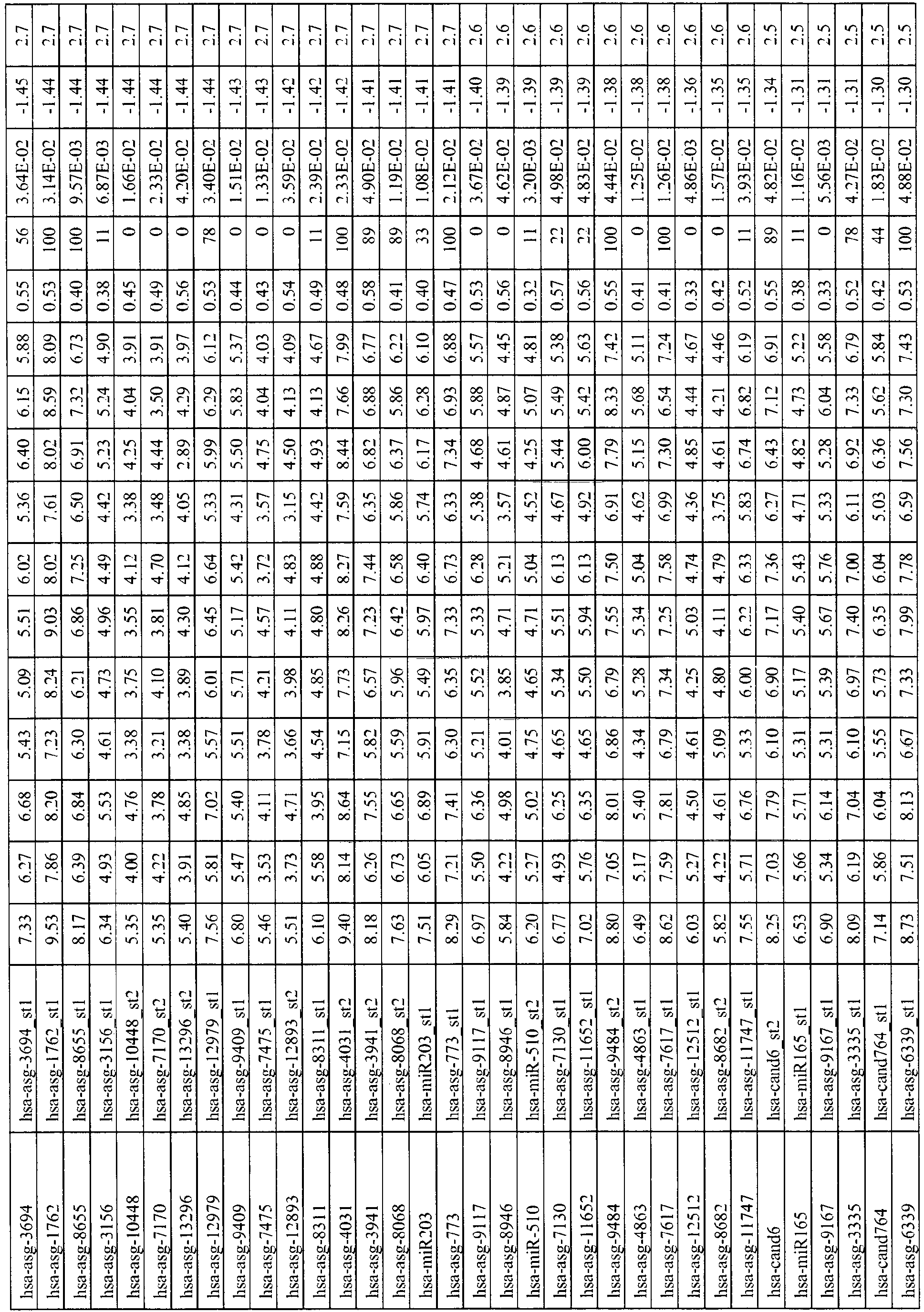

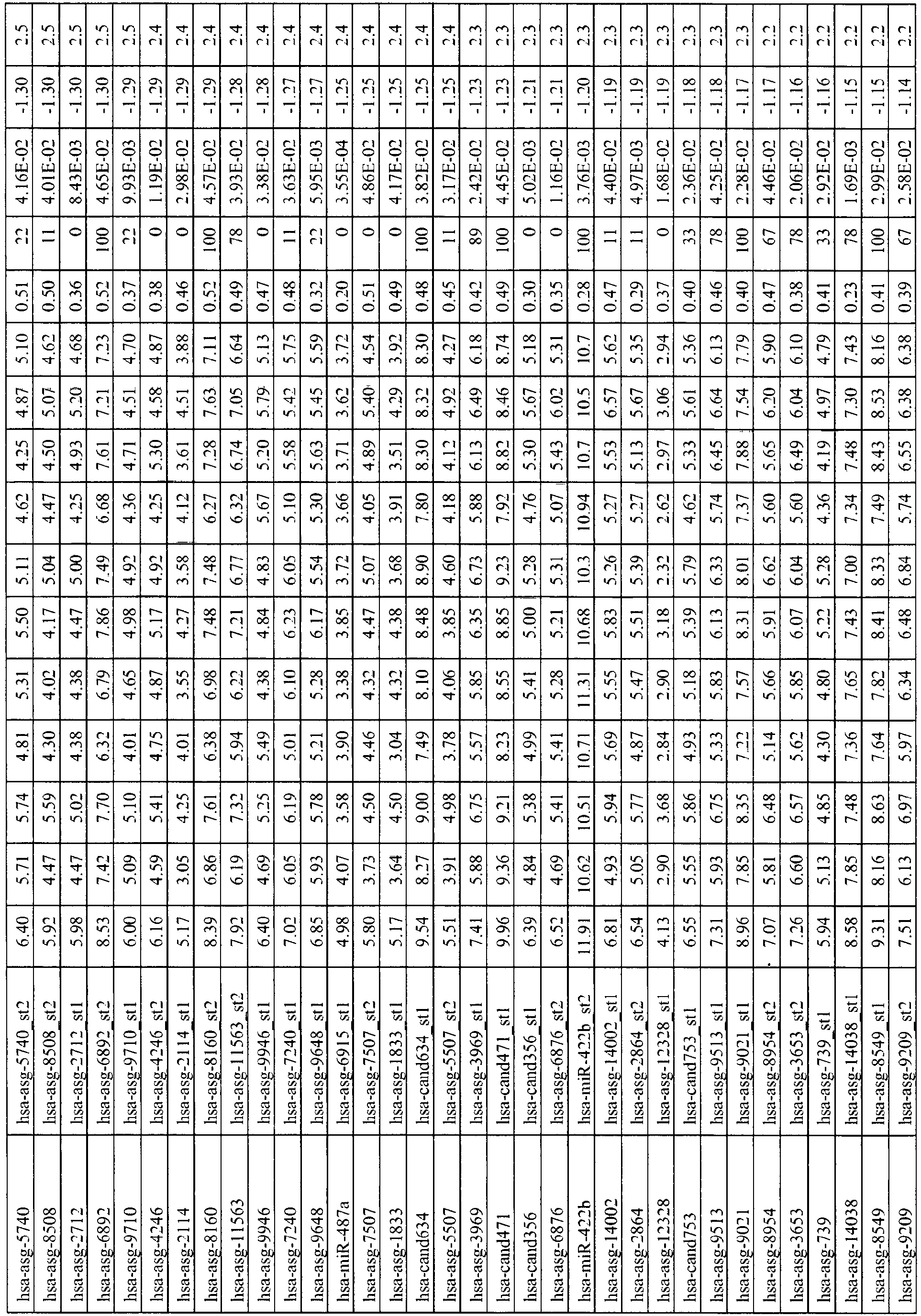

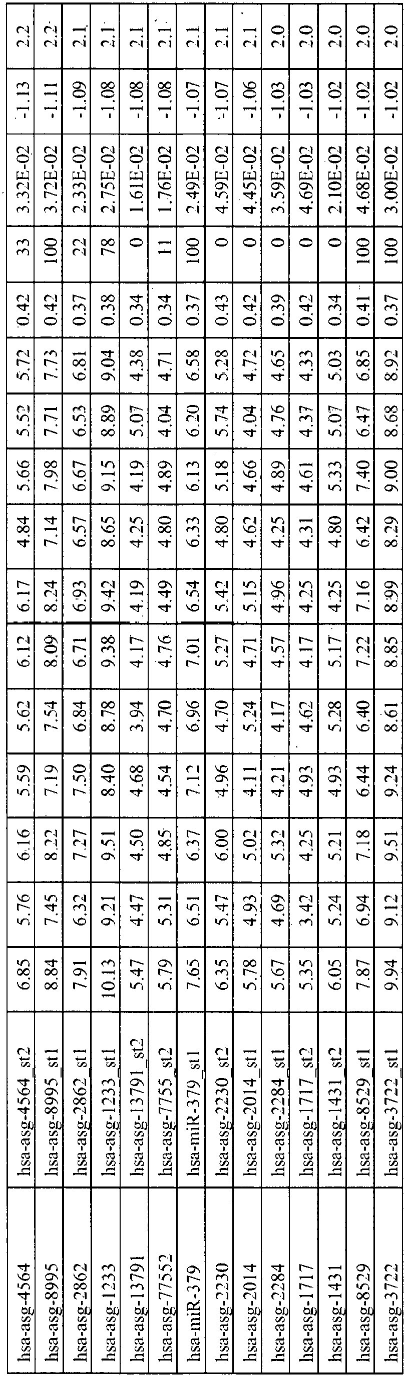

### EXAMPLE 38:

### DIFFERENTIALLY-EXPRESSED MIRNAS, MOST COMMONLY OBSERVED, BETWEEN 21 NORMAL TISSUE TYPES AND NORMAL LYMPH NODES

The inventors compiled a listing of miRNAs representing those that were observed by microarray expression analysis to be the miRNAs most frequently differentially-expressed between each of 21 normal tissue types and normal lymph nodes. The listing describes miRNAs whose differential expression was characterized by a p-value<0.05 and a level of expression at least 10-fold higher in a particular tissue compared to normal lymph nodes. The presence of a "1" in the column indicates that a differentially expressed miRNA fulfills the criteria described above for a particular tissue; whereas a "0" in the column indicates that the miRNA does not fulfill the criteria. The column on the right indicates the number of time a miRNA was identified as differentially expressed between the 21 normal tissues and normal lymph nodes. Among the most common differentially expressed miRNAs identified, four (hsa-miR-200a, -200b, -200c, and -429) were identified as differentially expressed between fifteen different tissues and normal lymph nodes; hsa-miR-605 and -187 were identified as differentially expressed between 14 or 13 different tissues, respectively, and normal lymph nodes; five miRNAs (the predicted miRNA hsa-asg-7637_st2 and the verified miRNAs hsa-miR-203, -375, -411, and -487b) were identified as differentially expressed between 12 different tissues and normal lymph nodes; 5 miRNAs (two predicted miRNAs, hsa-asg-14153_st2, and -5021_st1 and three verified miRNAs, hsa-miR-368, -493-5p, and -495) were identified 11 times; and 4 miRNAs (hsa-miR-141, -154, -205, and 381) were identified as differentially expressed between 10 different tissues and normal lymph nodes. In addition, a total of 229 miRNAs were found differentially expressed between only one particular tissue and normal lymph nodes. These miRNAs may potentially be used to specifically identify or confirm the origin of the tissue present in a metastatic lymph node..

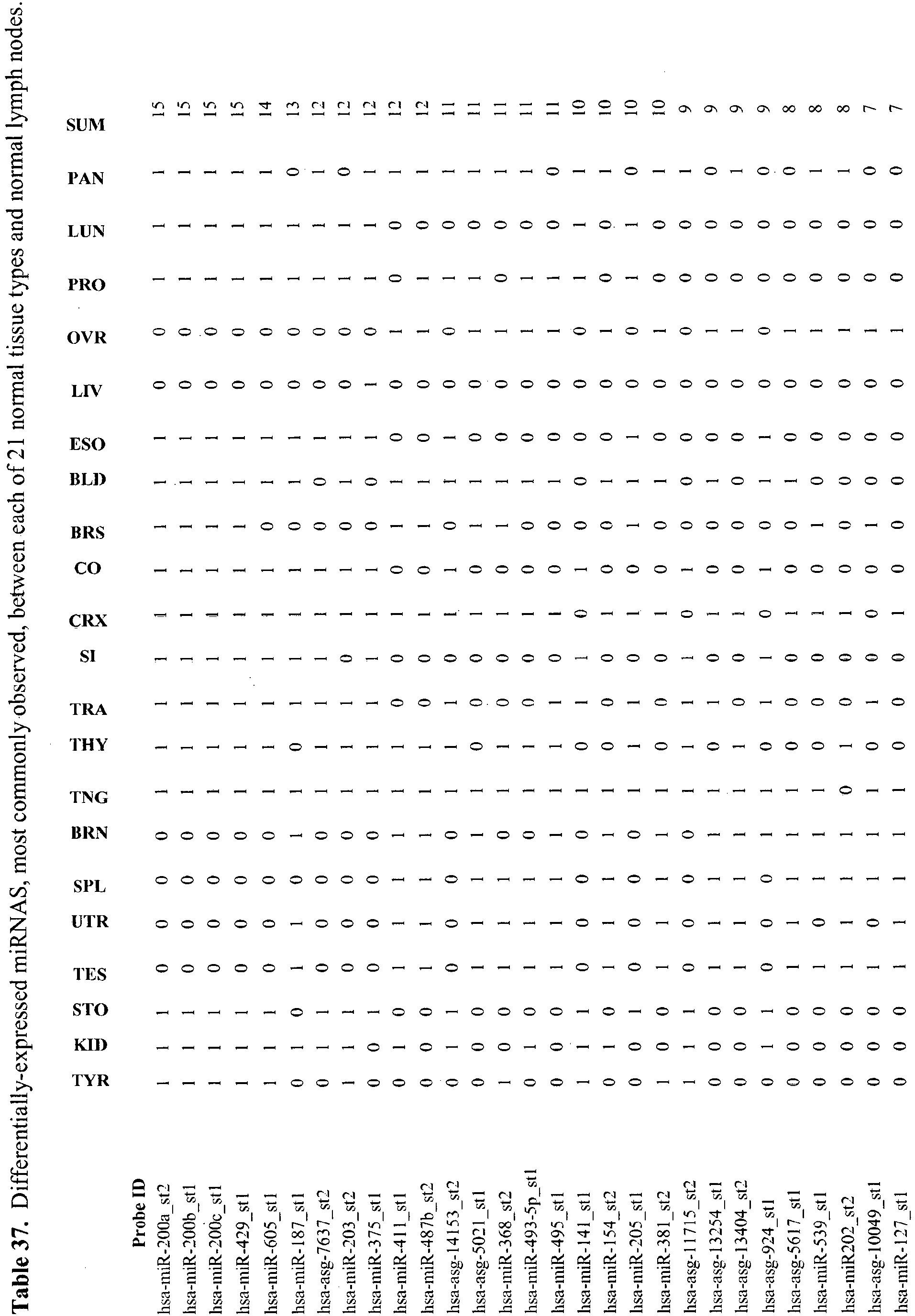

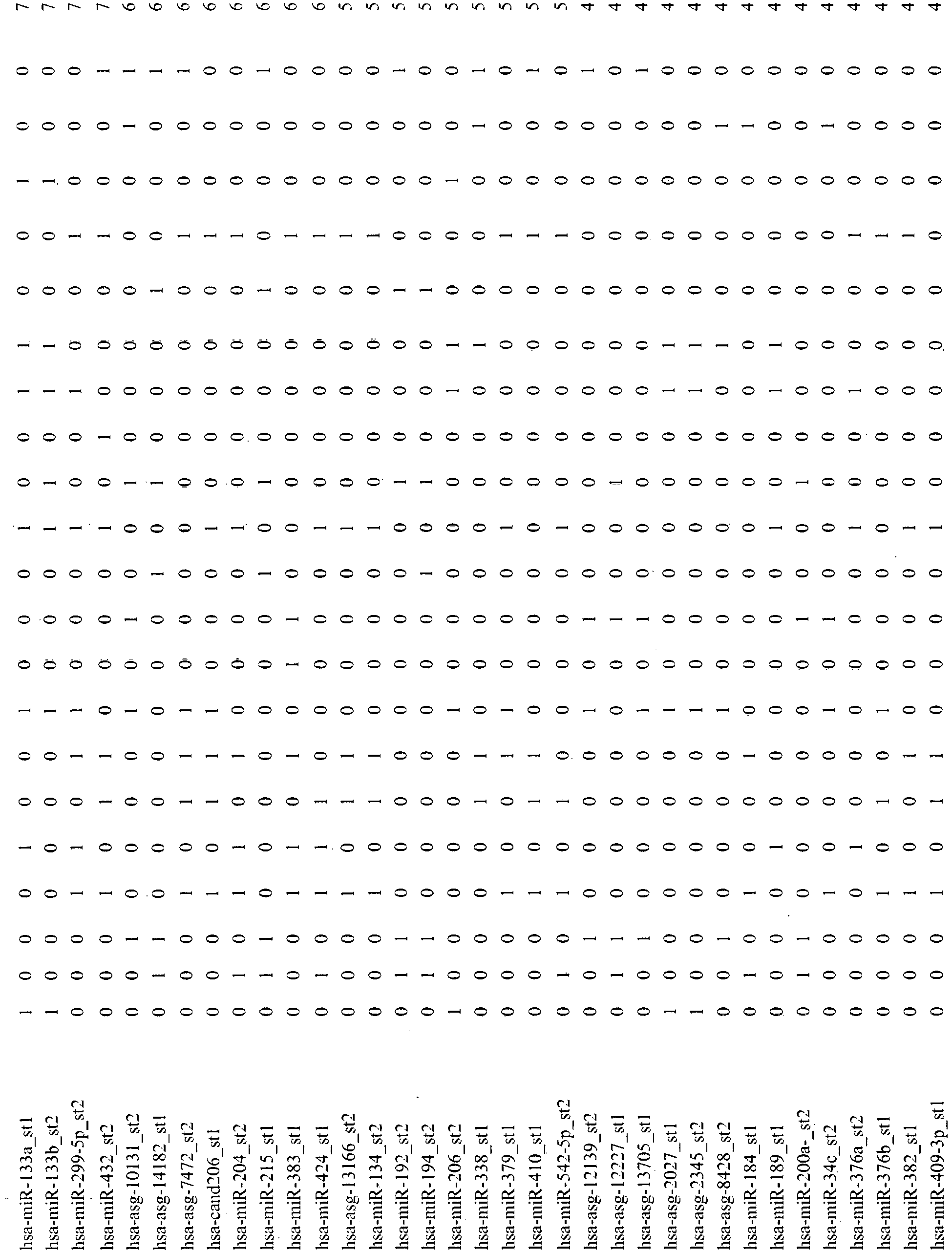

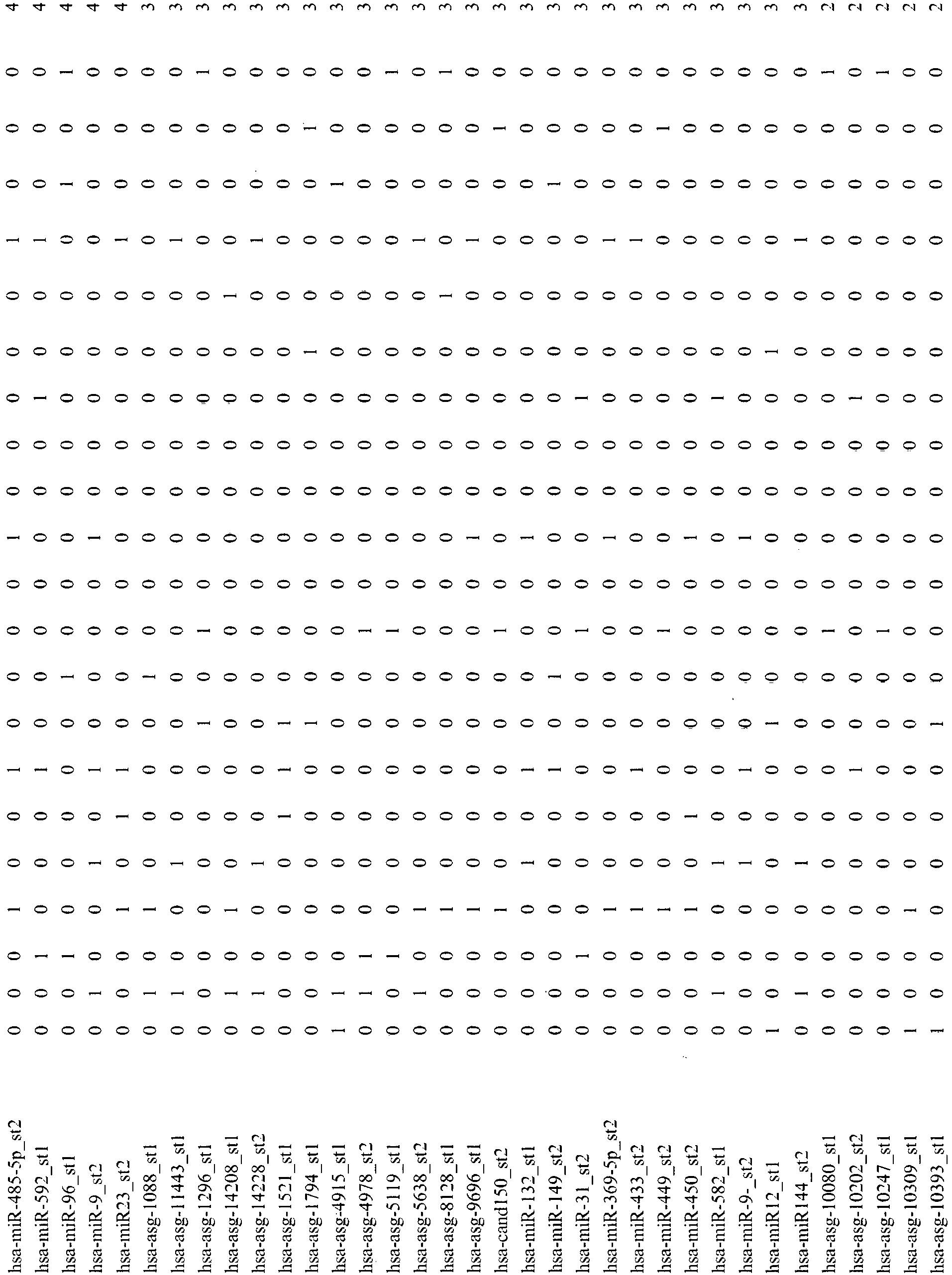

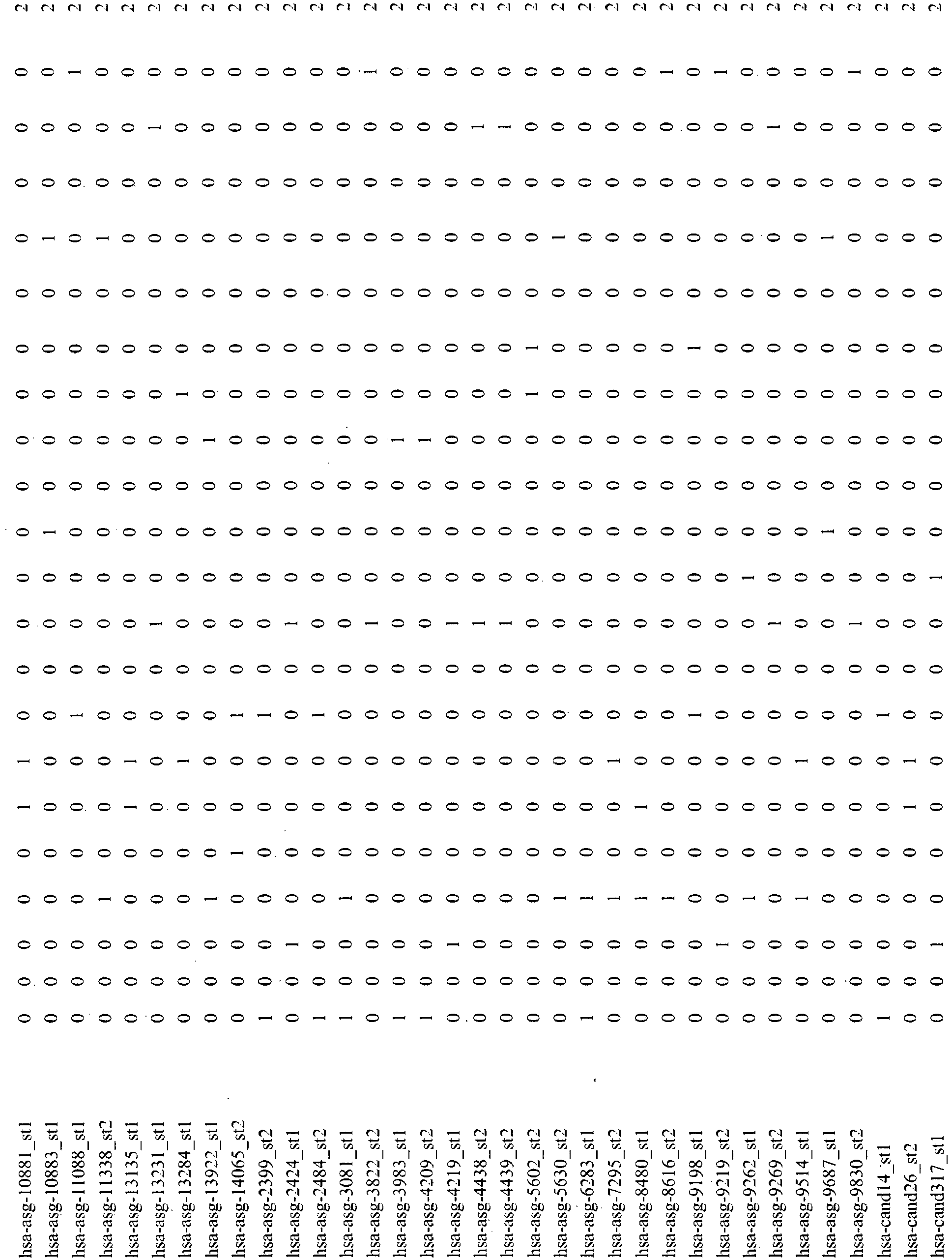

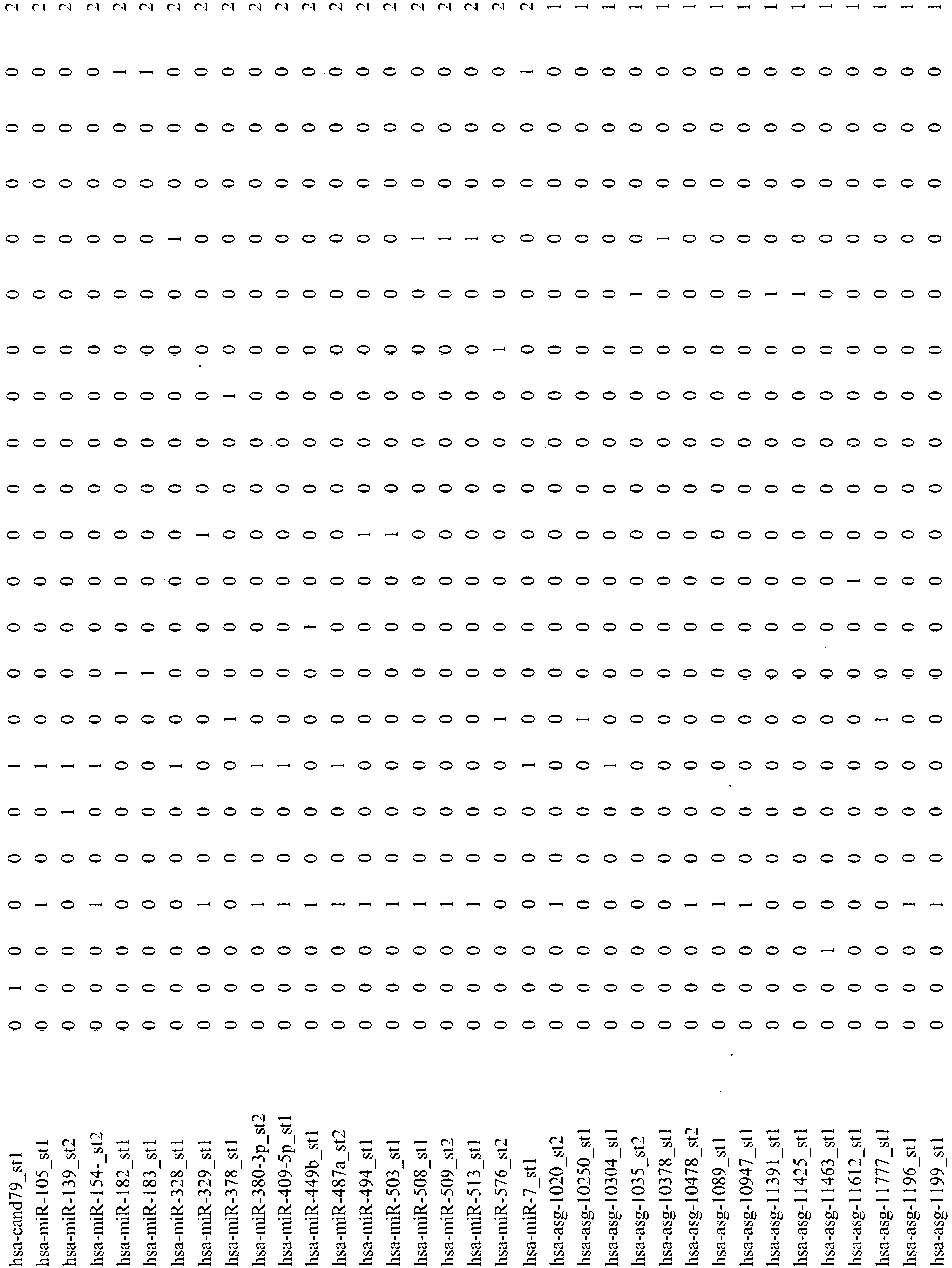

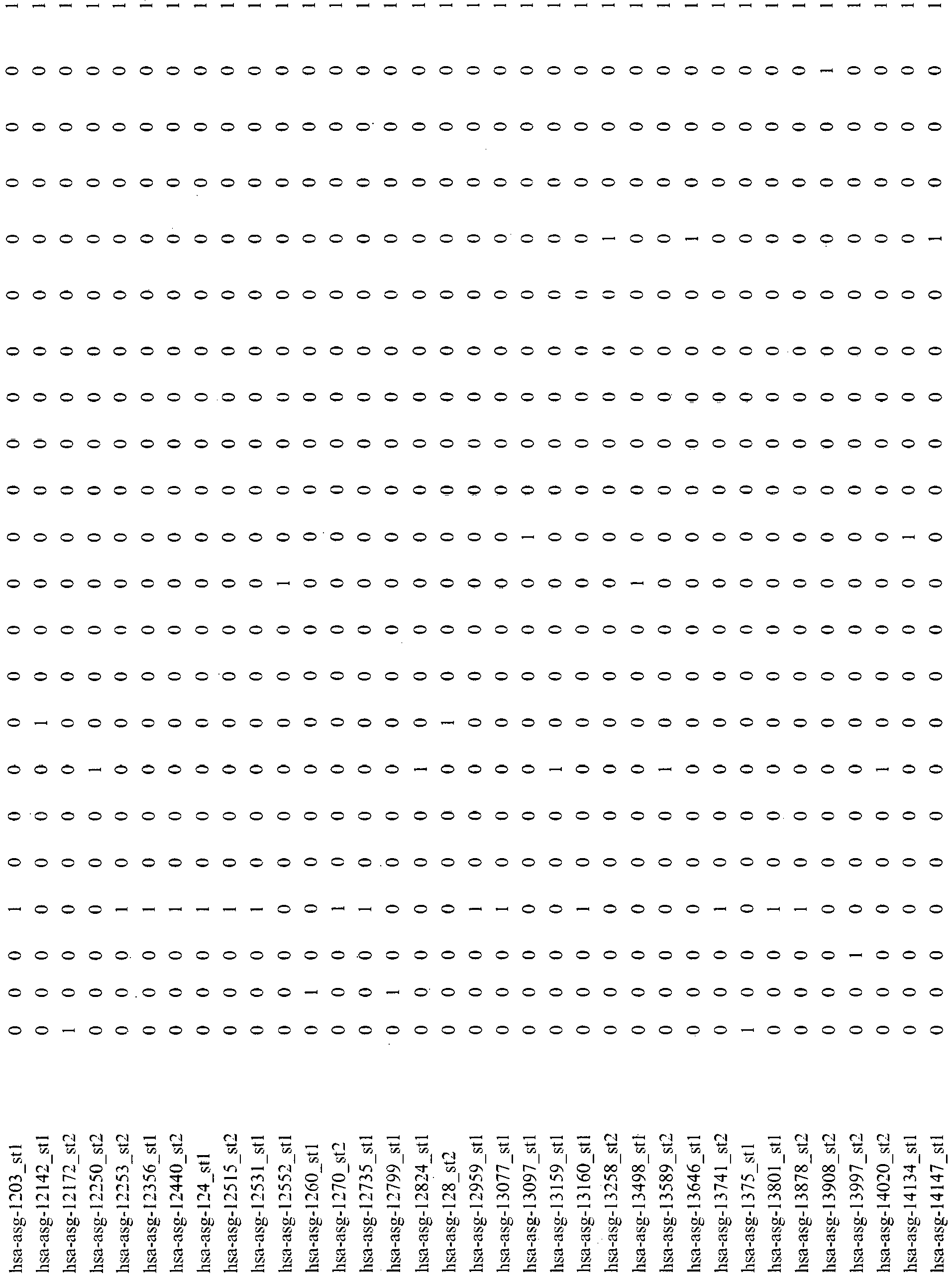

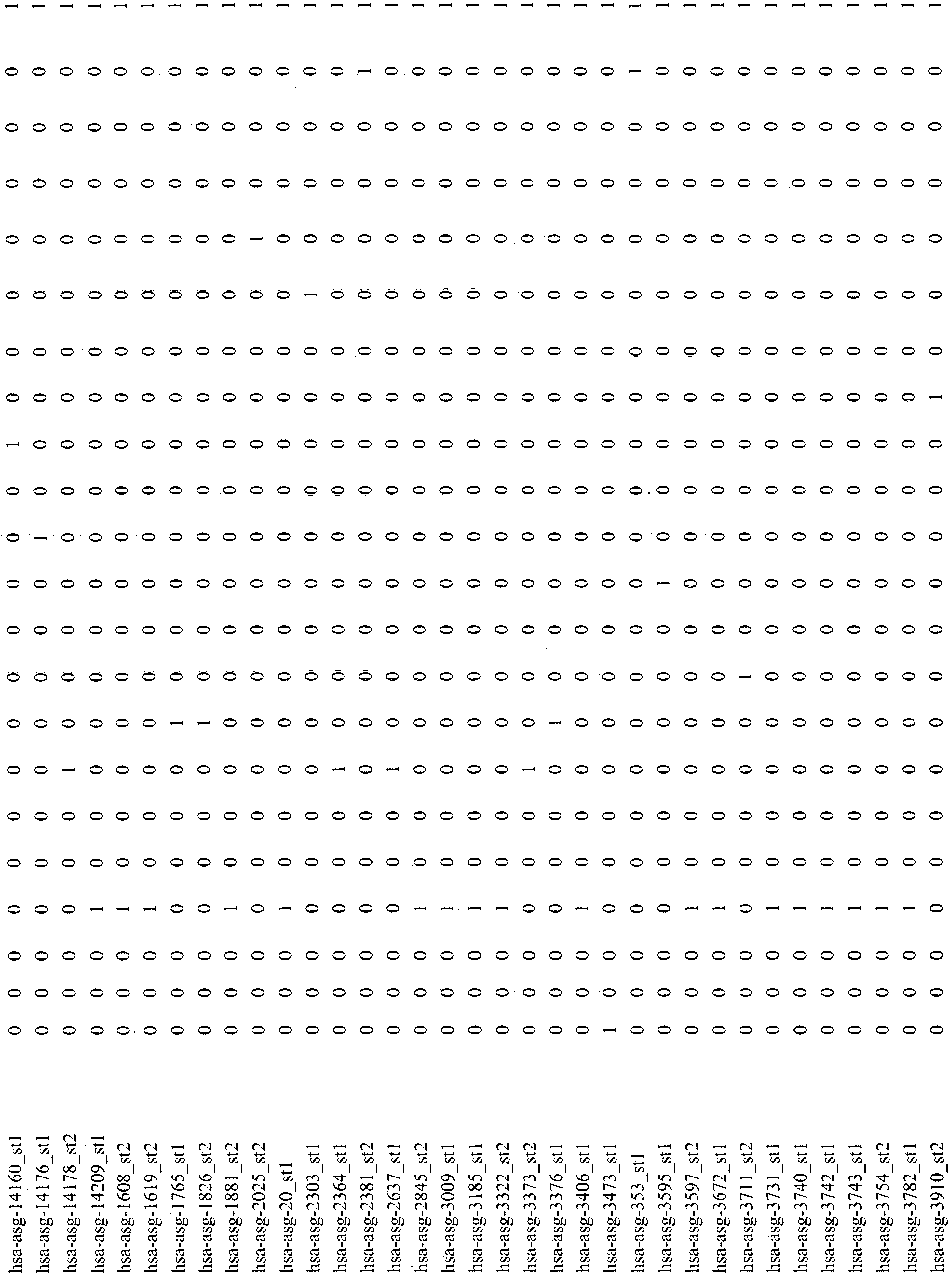

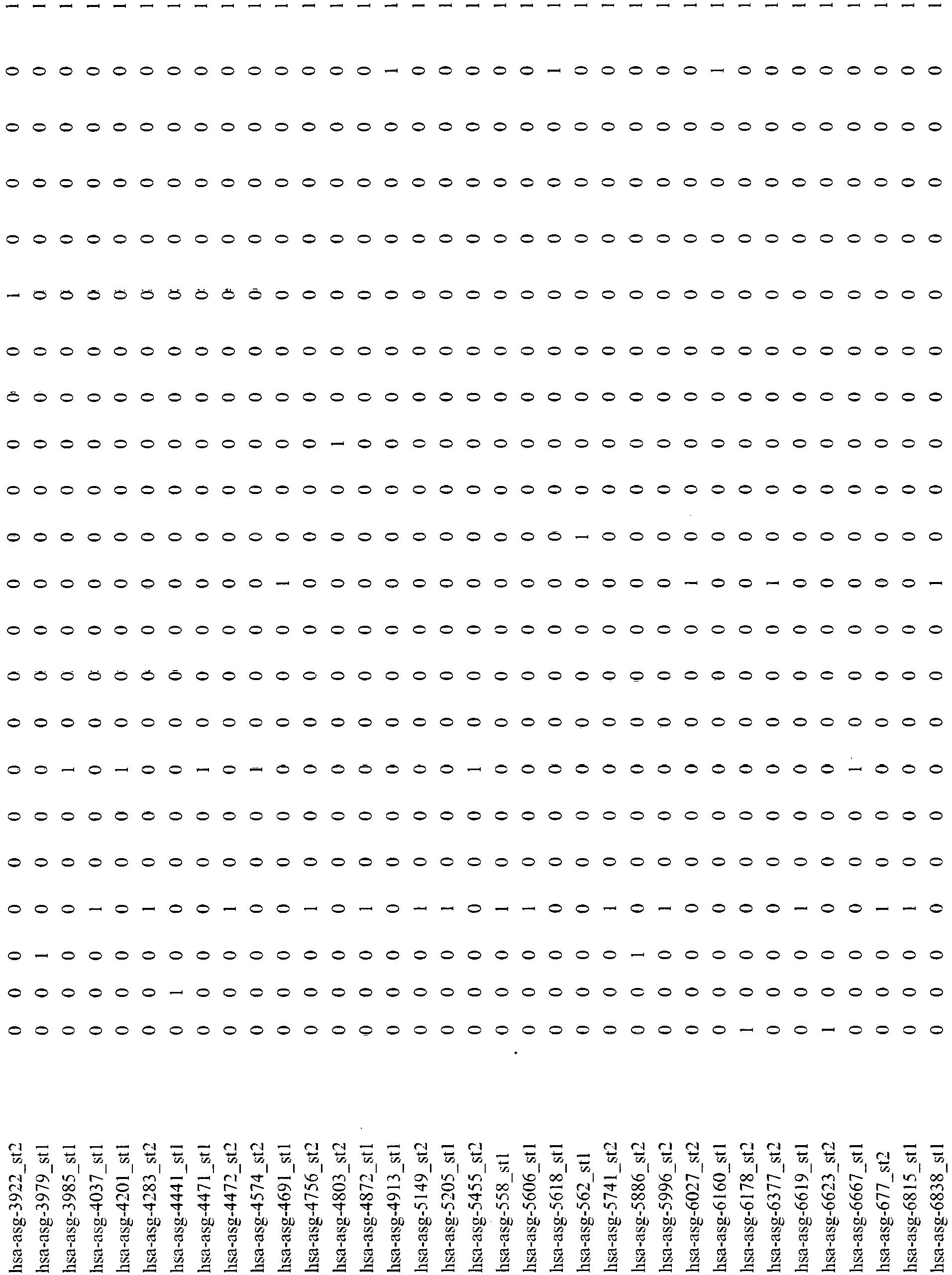

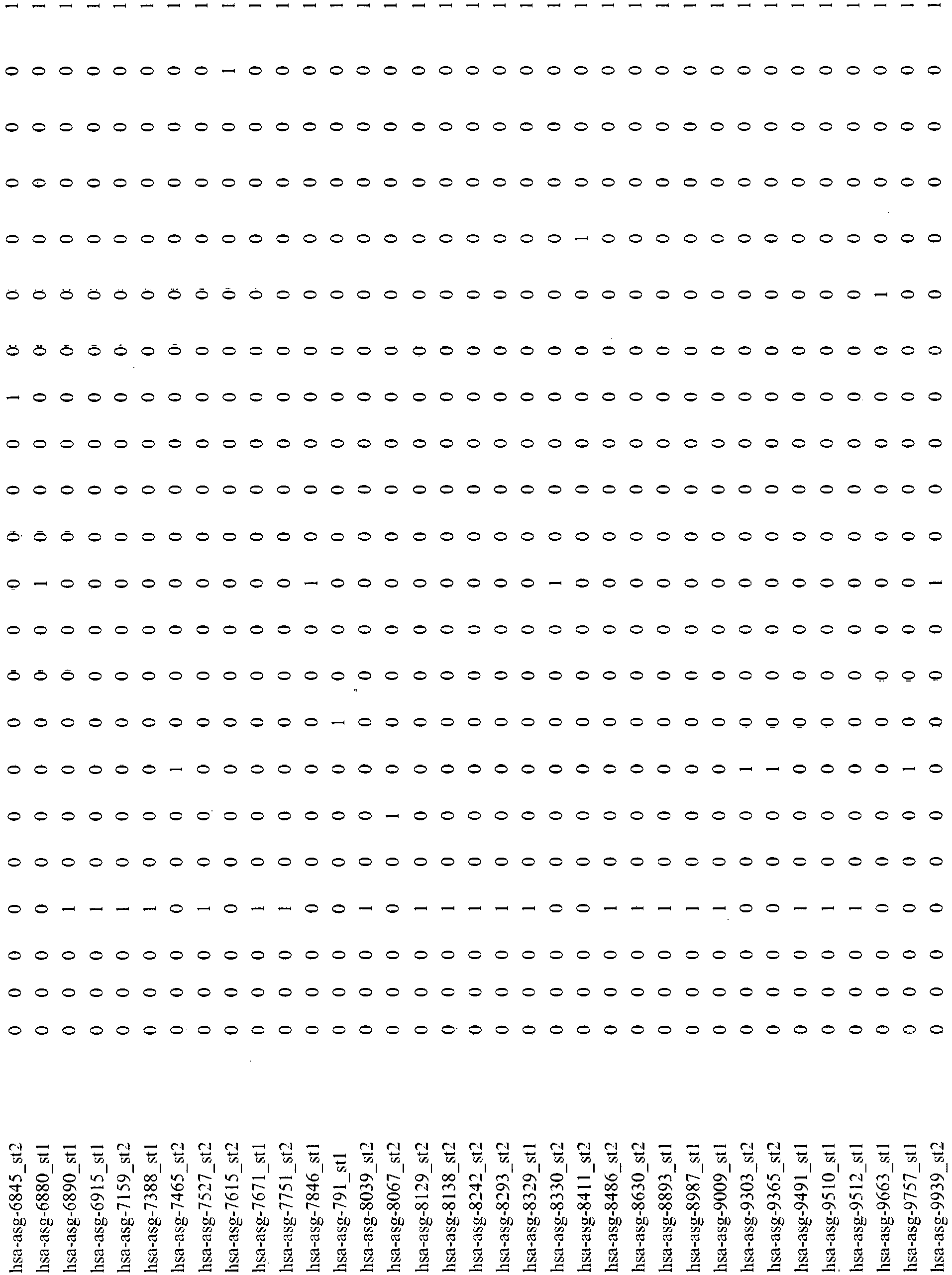

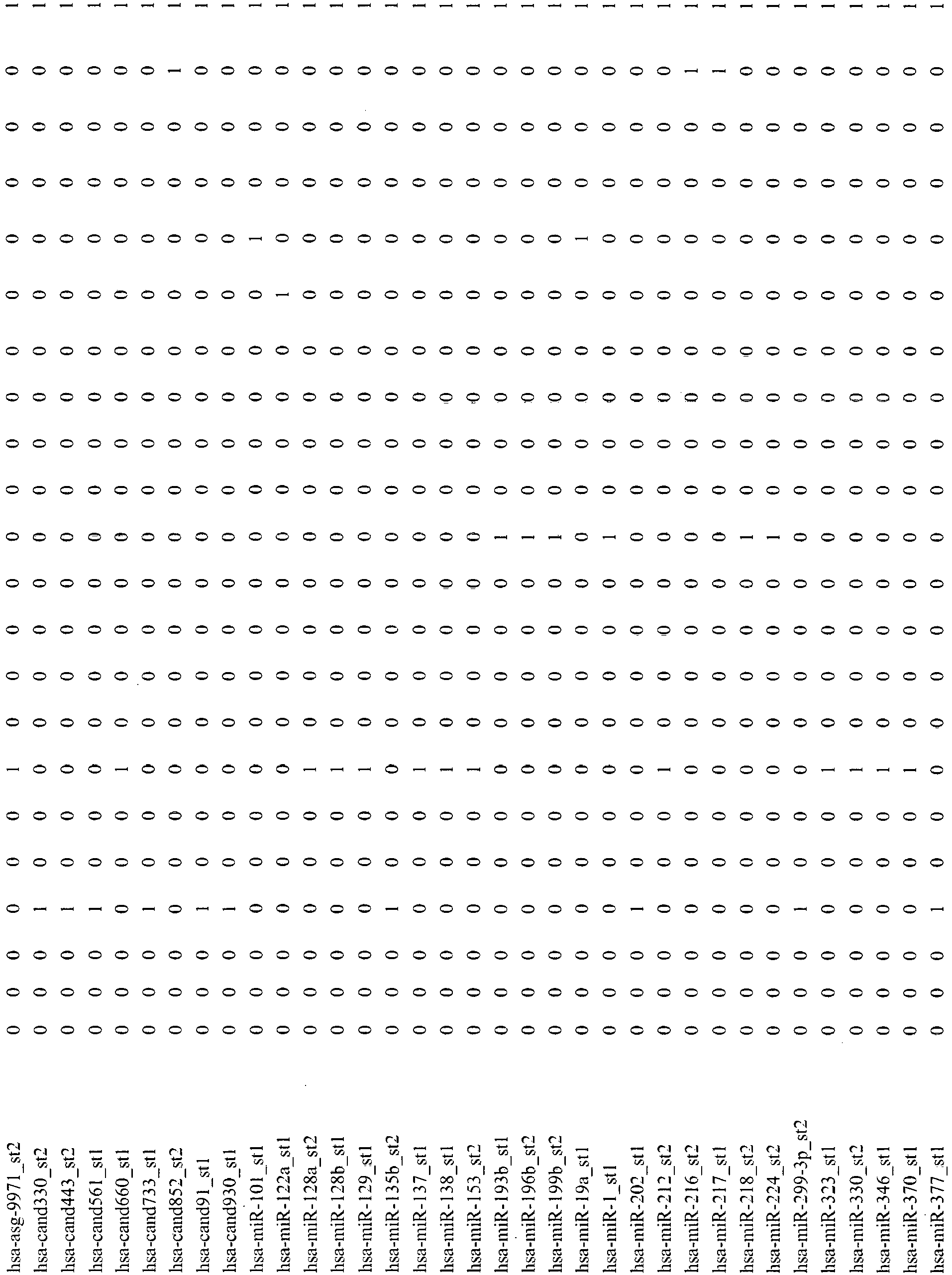

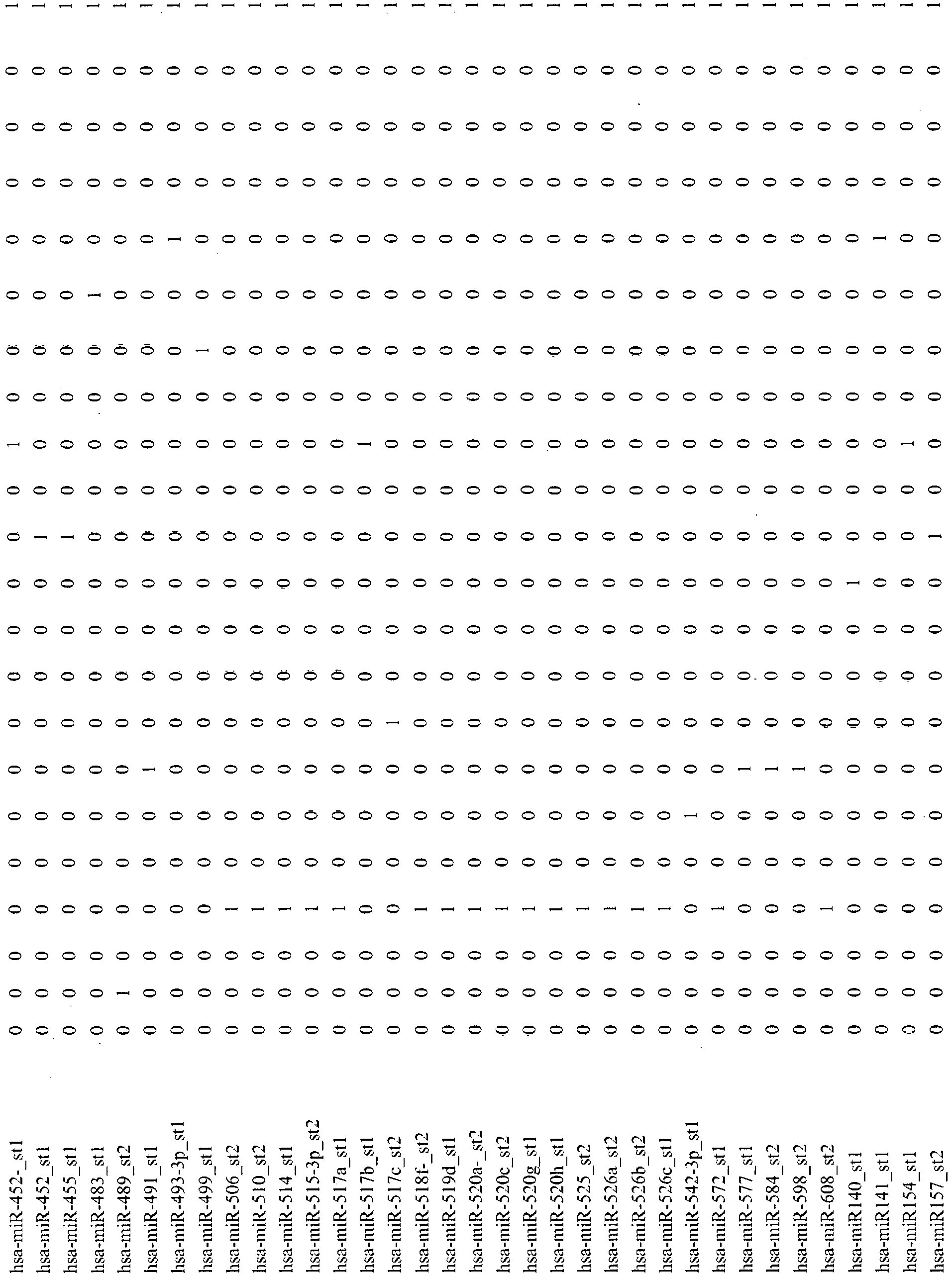

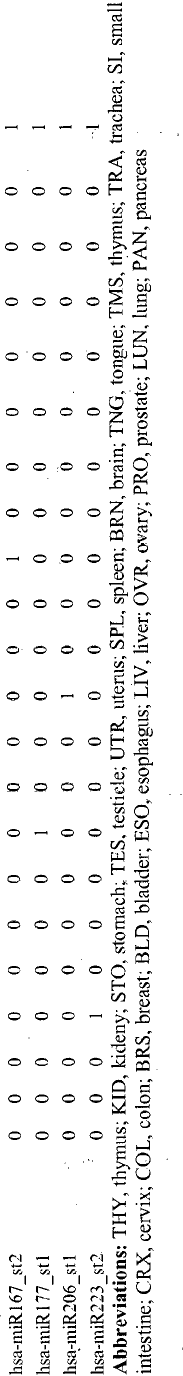

### EXAMPLE 39:

### MIRNA COMBINATIONS FOR THE DETECTION OF LYMPH NODE METASTASES FROM DIFFERENT CANCER TYPES

The inventors evaluated the most differentially-expressed miRNA markers between positive and negative lymph nodes from patients with cervical cancer (Example 3),colon cancer (Example 17) and breast cancer (Example 40) and determined that several useful markers (miR-141, -203, miR-429, miR-200a, miR-200b, and miR-200c) are conserved for these different cancer types. qRT-PCR quantification of these miRNAs, from positive and negative lymph nodes from patients with cervical cancer and colon cancer, was performed as described above in Example 7, Example 17, and Example 40) (FIG. 12A). As shown in FIG. 12B, the summation of the normalized Ct values for representative combinations of these miRNAs increased the separation between the positive and negative lymph nodes in each cancer type. More generally, common miRNA markers useful for the determination of metastasis of multiple cancers can be identified after comparison of the different normal tissues and normal lymph nodes, as reported in Example 38 (Table 37).

### EXAMPLE 40

### MIRNAS DIFFERENTIALLY EXPRESSED BETWEEN PAIRED CANCER-POSITIVE AND CANCER-NEGATIVE AXILLARY LYMPH NODES FROM BREAST CANCER PATIENTS

To identify miRNAs that may be useful markers for the presence of breast cancer metastasis in axillary lymph nodes, the inventors identified miRNAs that are expressed at significantly higher levels in cancer-positive lymph nodes (LNpos) compared to cancer-negative lymph nodes (LNneg) from breast cancer patients.

FFPE samples of paired cancer-positive (LNpos 1-4) and cancer-negative (LNneg 1-4) axillary lymph nodes from ductal breast cancer patients were purchased from ProteoGenex (ProteoGenex, Inc.; Culver City, CA, USA) (Table 38). A pathology report and hematoxilin-eosin (H&E)-stained slides were available for each lymph node sample. H&E slides were reviewed by a pathologist at Asuragen to confirm lymph node statuses as cancer cell-positive or cancer cell-negative. Positive lymph node 1 (LNpos1) contained only a minute amount of tumor cells and was excluded from the analysis. Its counterpart sample, LNneg1, was kept in the analysis, unmatched.

**Table 38. Patient and tissue sample pathology information for four, paired cancer-positive (LNpos) and cancer-negative (LNneg) axillary lymph nodes from four independent ductal breast cancer patients. TNM stage (Sobin and Wittekind, 2002); AJCC Stage (Greene, 2002); LN Mets, number of lymph nodes with metastases/total number of lymph nodes examined.**

| **Sample ID** | **LN Cancer Diagnosis** | **Sex** | **Patient Age (yr) Ethnicity** | **Patient's Histological Diagnosis** | **Tumor size (cm)** | **TNM Stage** | **AJCC Stage** | **LN Mets** |
|---|---|---|---|---|---|---|---|---|
| LNneg1 | normal | F | 72 Caucasian | infiltrating ductal carcinoma, moderately differentiated | 2.5 | T2N1M0 | IIB | 1/11 |
| LNpos1 | metastatic | | | | | | | |
| LNneg2 | normal | F | 46 Caucasian | infiltrating ductal and lobular carcinoma, moderately differentiated | 2.5 | T2N2M0 | IIIA | 5/9 |
| LNpos2 | metastatic | | | | | | | |
| LNneg3 | normal | F | 66 Caucasian | infiltrating ductal carcinoma, moderately differentiated | 3.5 | T2N1M0 | IIB | 1/11 |
| LNpos3 | metastatic | | | | | | | |
| LNneg4 | normal | F | 65 Caucasian | infiltrating ducal carcinoma, moderately differentiated | 3 | T2N1M0 | IIB | 1/8 |
| LNpos4 | metastatic | | | | | | | |

Total RNA isolation from the FFPE lymph node samples was performed using the RecoverAll™ Total Nucleic Acid Isolation Kit (cat. No. AM 1975; Ambion, Inc.; Austin, TX, USA), as described above in Example 1 and analyzed on miRNA microarrays, as described below.

miRNA expression was compared between three paired LN samples from three individual patients (LNpos2-4; LNneg2-4) plus one unmatched cancer-negative lymph node (LNneg1). Total RNA from each sample (200 ng) was processed by Asuragen Services (Asuragen Inc., Austin, TX, USA), according to the company's standard operating procedures. RNA labeling was adapted from a published method (Wang *et. al.,* 2006) that was modified so that RNA is end-labeled with biotin instead of Cy3. Briefly, total RNA is dephosphorylated with calf intestinal phosphatase and the pCp-Biotin labeling molecule is ligated to the 3' ends of the RNA molecules. Labeled RNA was purified using Bio-Spin 6 columns (Bio-Rad Laboratories, Inc.; Hercules, CA, USA). Labeled RNA was hybridized to a custom-made microRNA array (miRChip V1.0, manufactured by Affymetrix, Santa Clara, CA, USA for Ambion), as described above in Example 2. Hybridization, washing, staining, imaging, and signal extraction were performed as recommended by the miRChip manufacturer, except that the 20X GeneChip® Eukaryotic Hybridization Control cocktail was omitted. Signal processing was as described above in Example 2.

A total of 146 miRNAs (50 verified and 96 predicted) exhibited significant differential expression between positive (LNpos) and negative (LNneg) lymph node samples (*t*-test (LNpos vs LNneg) < 0.05 and log2Diff (LNpos vs LNneg) ≥1; Flag =1) (Table 39). Among the verified miRNAs, four (hsa-miR-375, -200b, -141, and -200a) had average expression levels between 100- and 350-fold higher in LNpos compared to LNneg; eight (hsa-miR-182, -196a, -200c, -203, -183, -429, -542-5p, and -421-3p) had average expression levels between 10- and 90-fold higher in LNpos; and nine had average expression levels between 5- and 10- fold higher in LNpos samples than in LNneg samples (Table 39). Among the predicted miRNAs, two (hsa-asg-2924_st1 and -1937_st1) had average expression levels at least 10-fold higher in LNpos and thirteen had average expression levels between 5- and 9-fold higher in LNpos than in LNneg samples. In addition, four miRNAs (hsa-asg-6116_st2, - 6449_st1, -10487_st1, and -2449_st1) had average expression levels between 5- and 20 fold lower in LNpos compared to LNneg (Table 39).

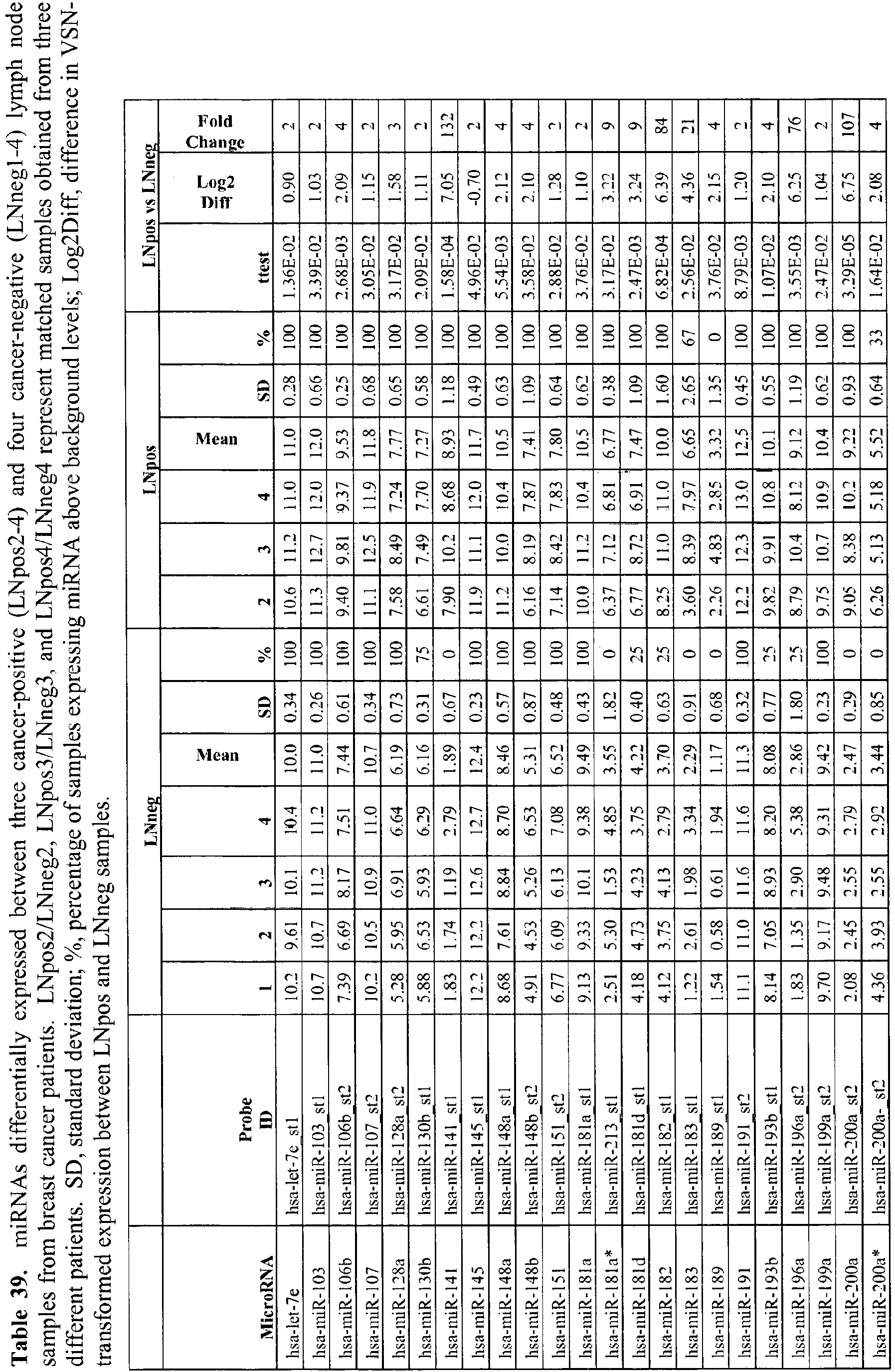

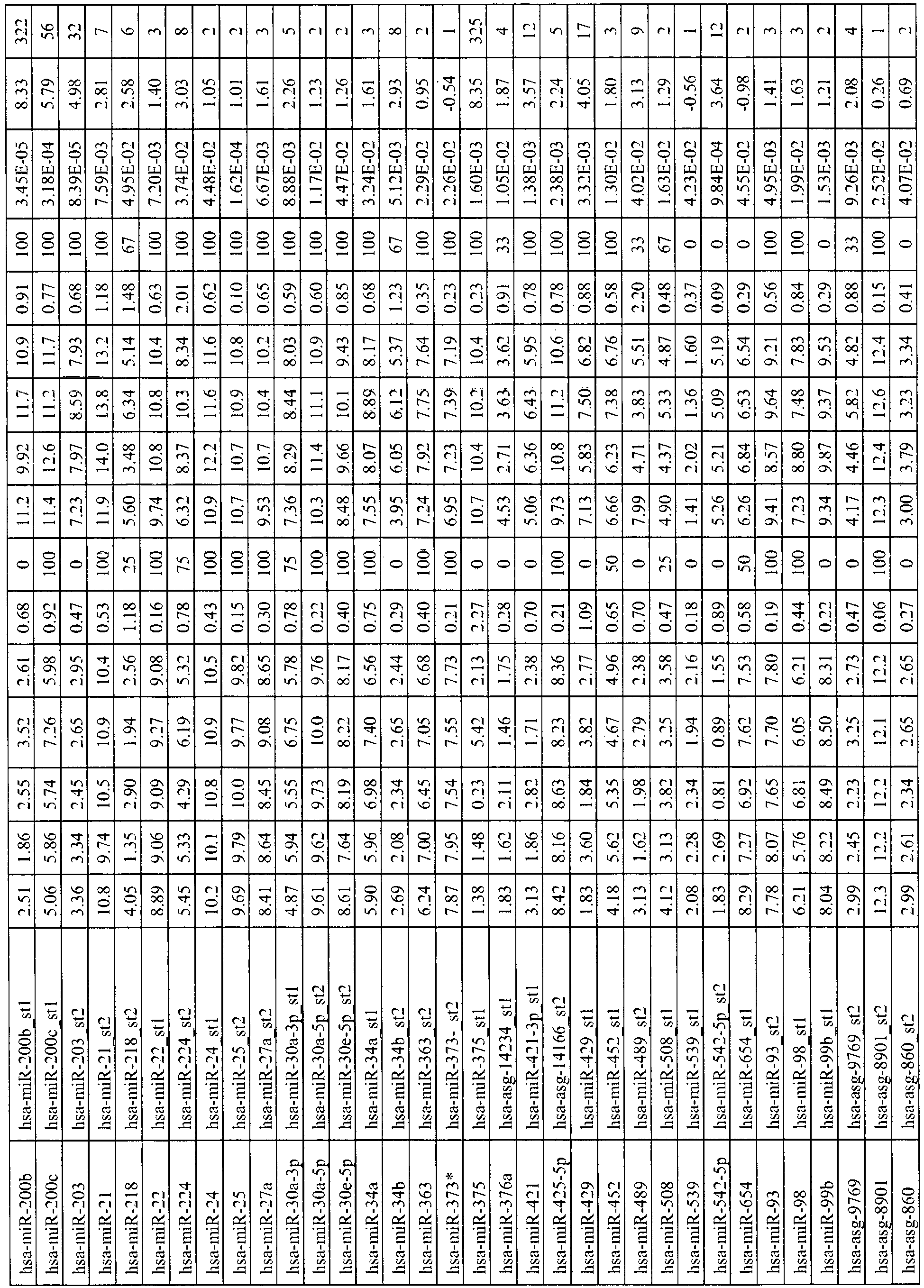

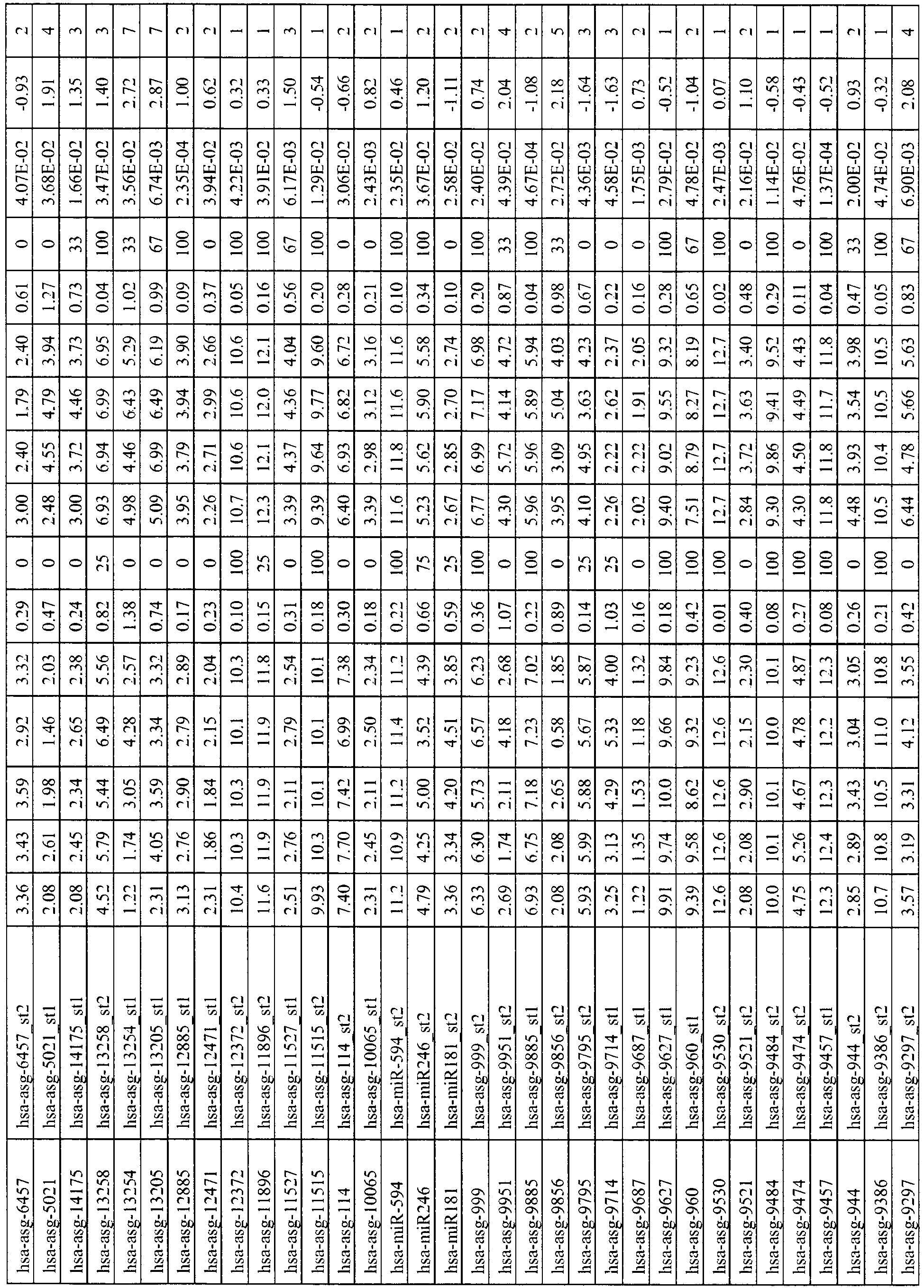

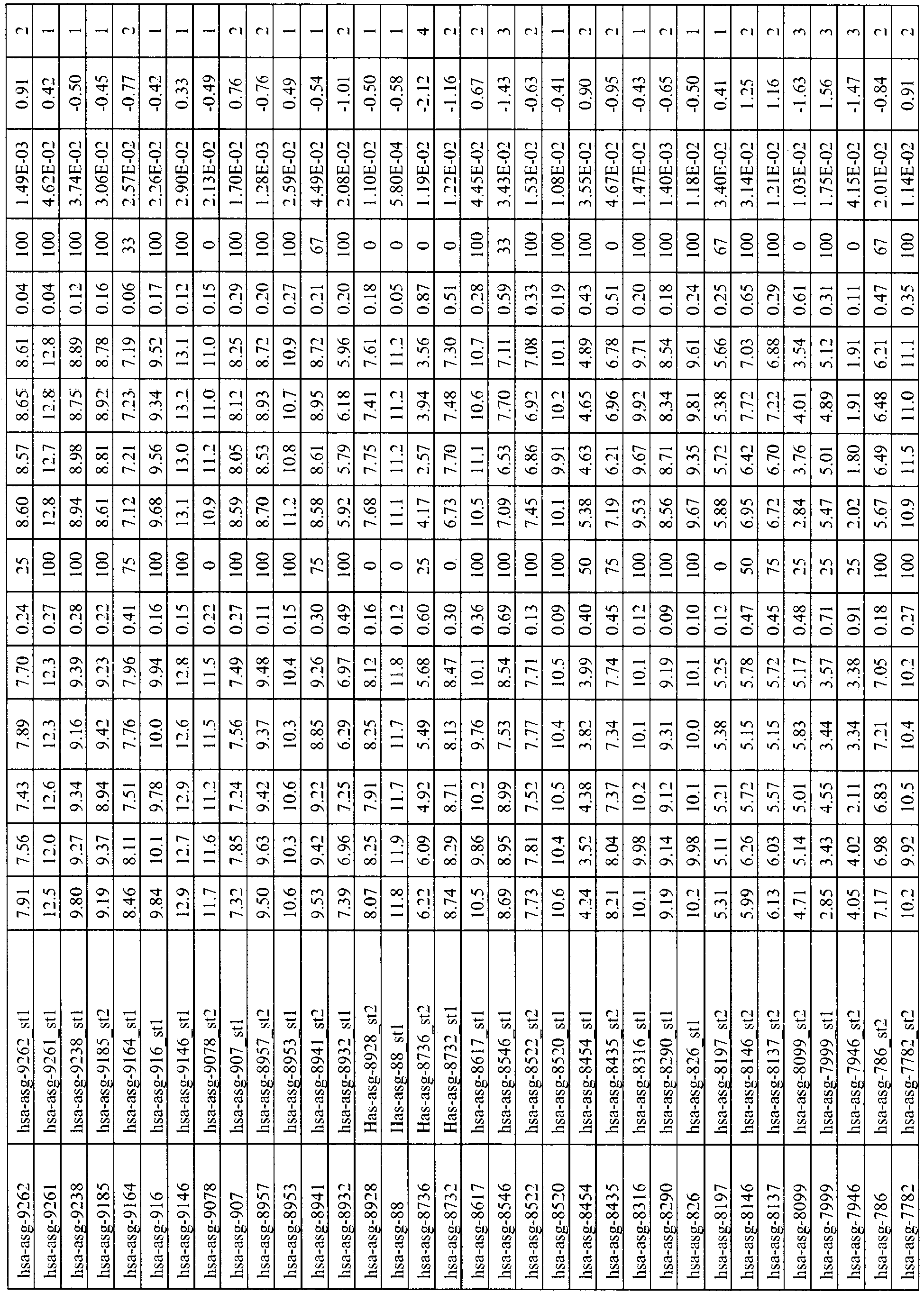

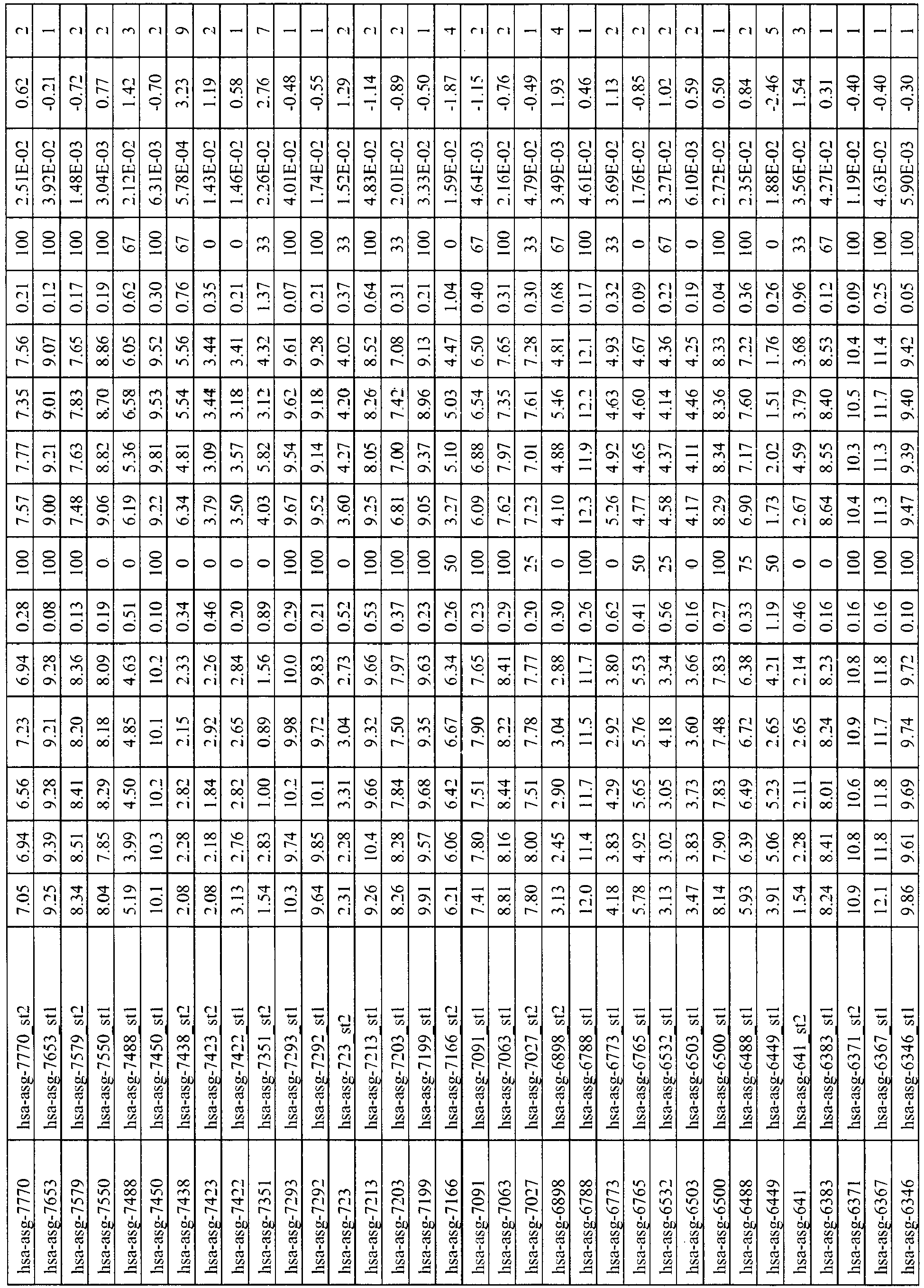

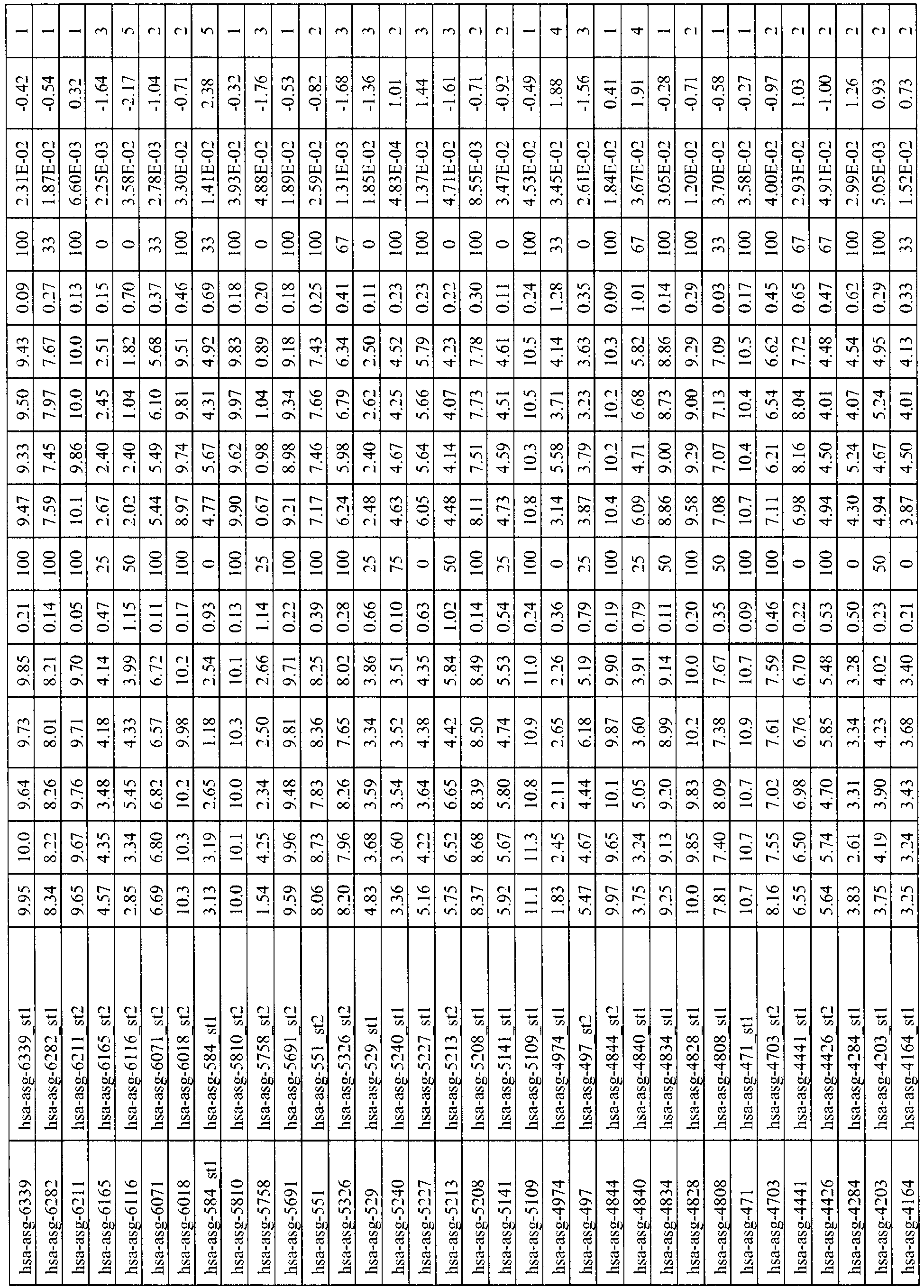

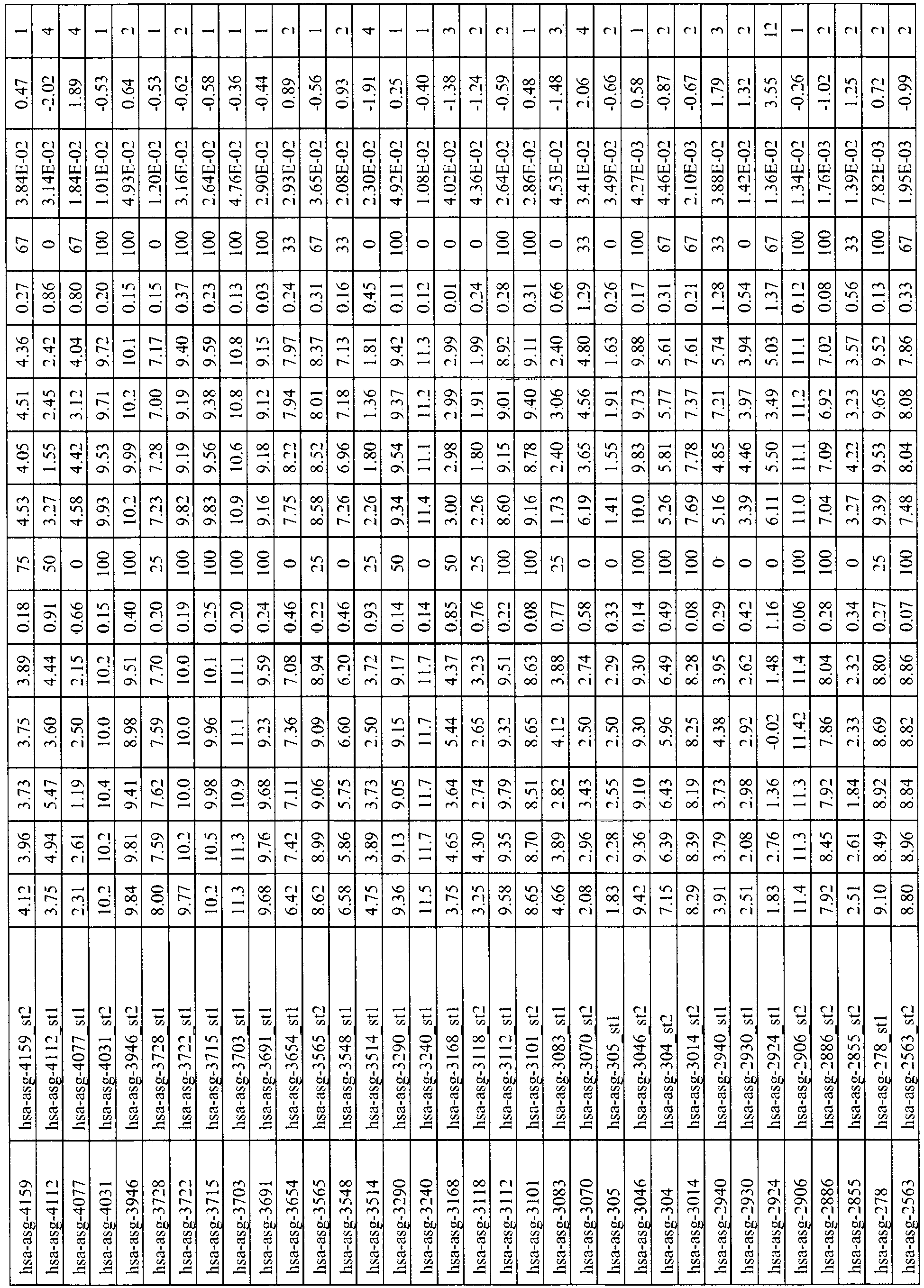

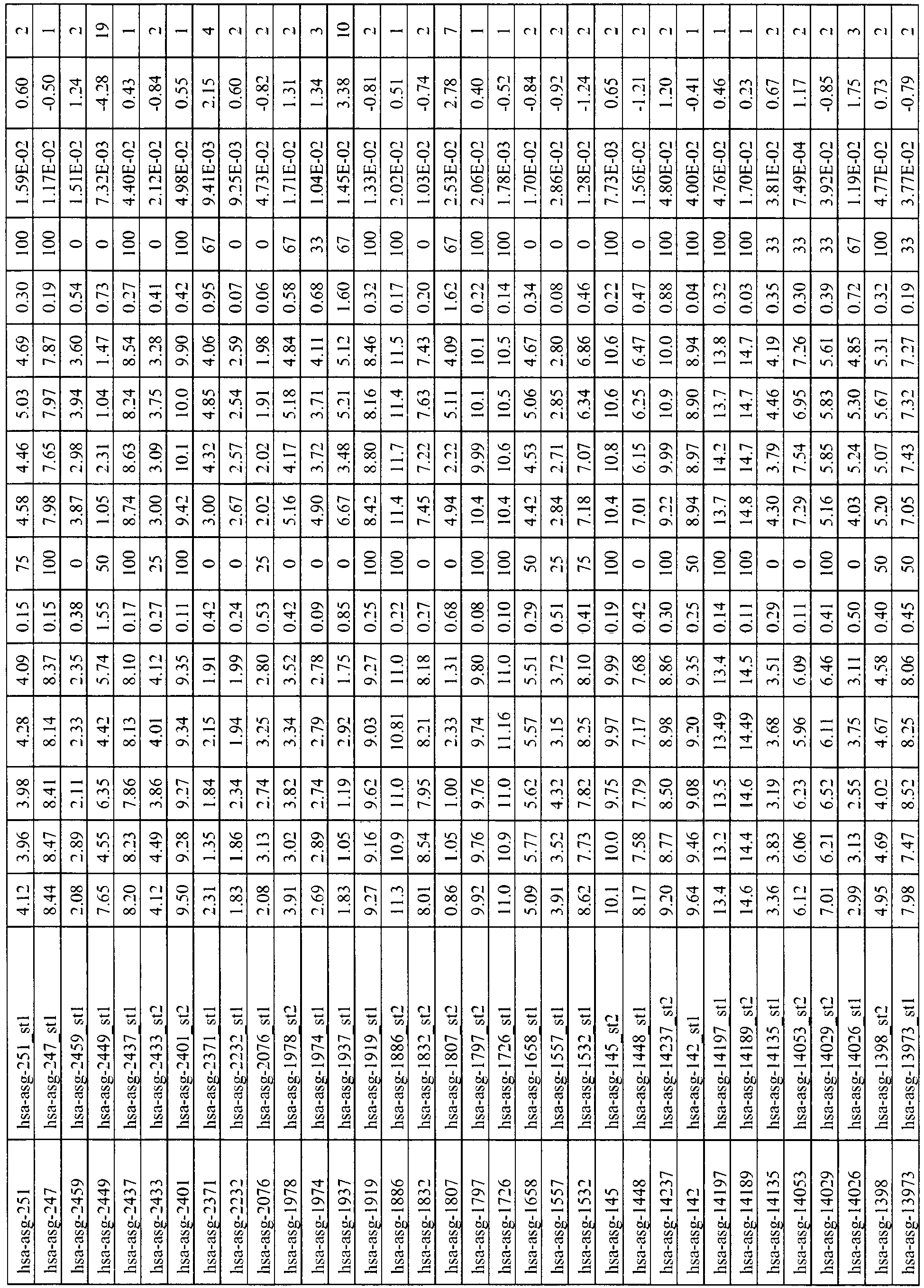

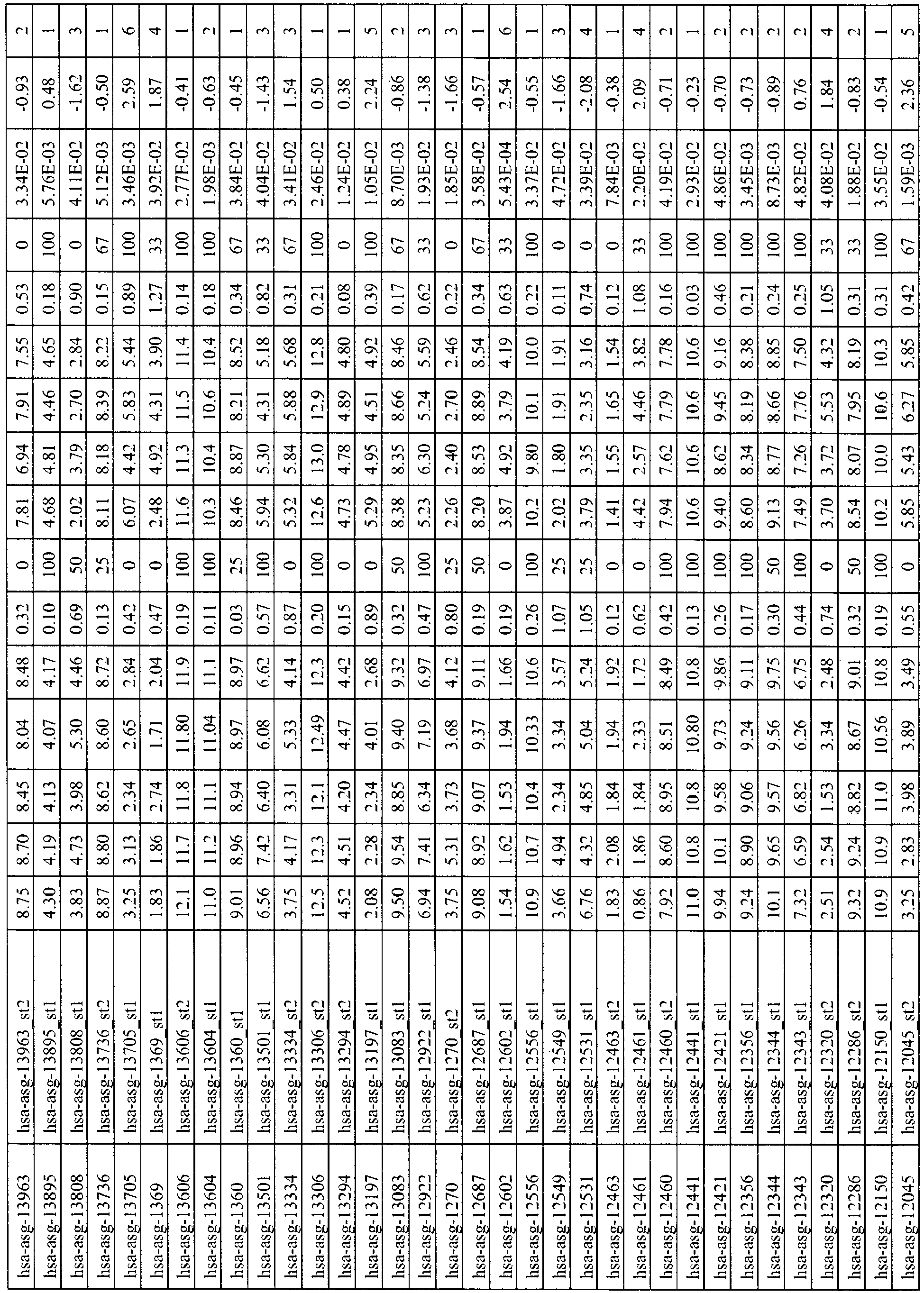

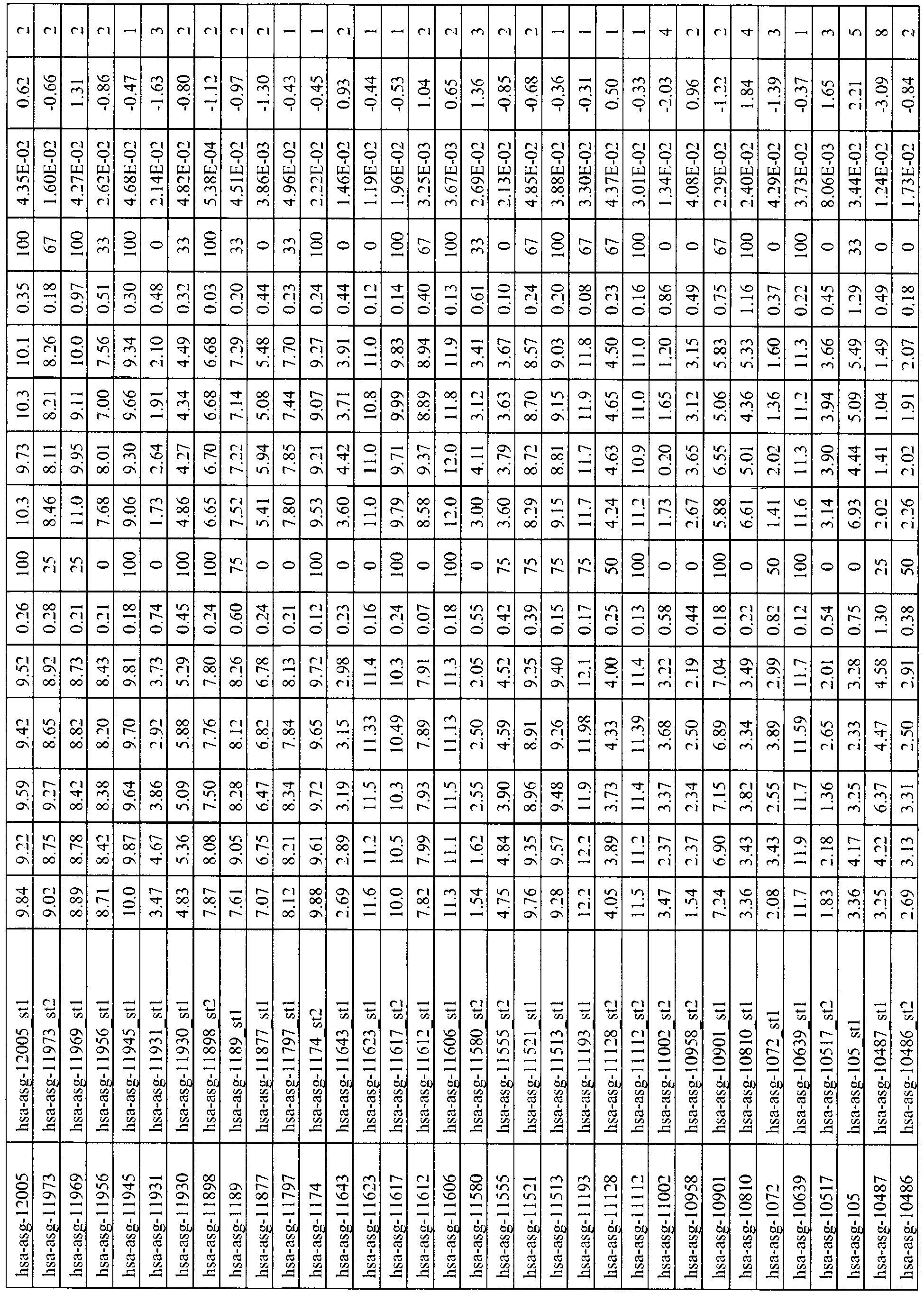

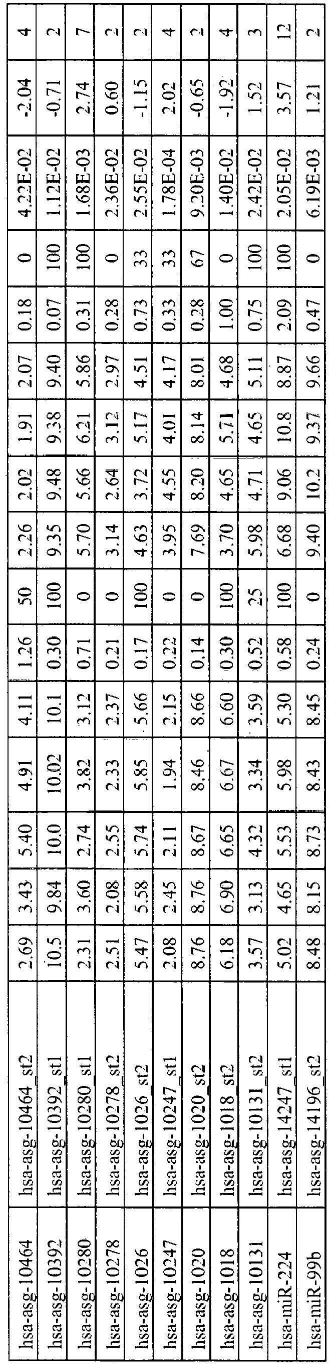

### EXAMPLE 41:

### MIRNAS DIFFERENTIALLY EXPRESSED BETWEEN NORMAL BREAST TISSUE AND CANCER-NEGATIVE AXILLARY LYMPH NODES FROM BREAST CANCER PATIENTS

To identify the miRNAs differentially expressed between normal breast tissue samples and cancer negative axillary lymph nodes, the inventors compared the average miRNA expression levels in three normal human breast tissue samples (described above in Example 19) with those in the four cancer-negative axillary lymph nodes from ductal breast cancer patients (described above in Example 40). RNA labeling and microarray expression analysis was performed as described above in Example 40. A total of 620 miRNAs (168 verified and 452 predicted) were significantly differentially expressed between normal breast (NBr) and negative lymph node (LNneg) samples (Log2Diff(NBr vs LNneg)≥1; t-test (NBr vs LNneg) <0.05) (Table 40). Among the verified miRNAs, only three (hsa-miR-155, -150, and -155) had average expression levels that were lower (3-6 fold) in NBr compared to LNneg. One (hsa-miR-205) had an average expression level at least 1,000 fold higher in NBr compared to negative LNneg; seven (hsa-miR-200b, -368, -200a, -141, -199b, -411, and - 335) had average expression levels 100- to 500-fold higher in NBr; eleven (hsa-miR-1, -224, -218, -200c, -551b, -19a, -140, -495, -182, -424, and -451) had average expression levels 50-to 95-fold higher in NBr samples; nineteen (hsa-miR-452, -486, -429, -189, -96, -421-3p, - 133a, -30a-3p, -487b, -452, -154, -375, -127, -22, -148b, -379, -19b, -489, and -152) had average expression levels 20- to 49-fold higher in NBr; and 98 had expression levels 5- to 19-fold higher in NBr samples compared to LNneg (Table 40). Among the predicted miRNAs, three (hsa-asg-5021_st1, -13254_st1, and hsa-miR154_st1) had average expression levels more than 50-fold lower in LNneg compared to NBr samples, and 24 had average expression levels 10- to 50-fold lower in LNneg compared to NBr samples (Table 40).

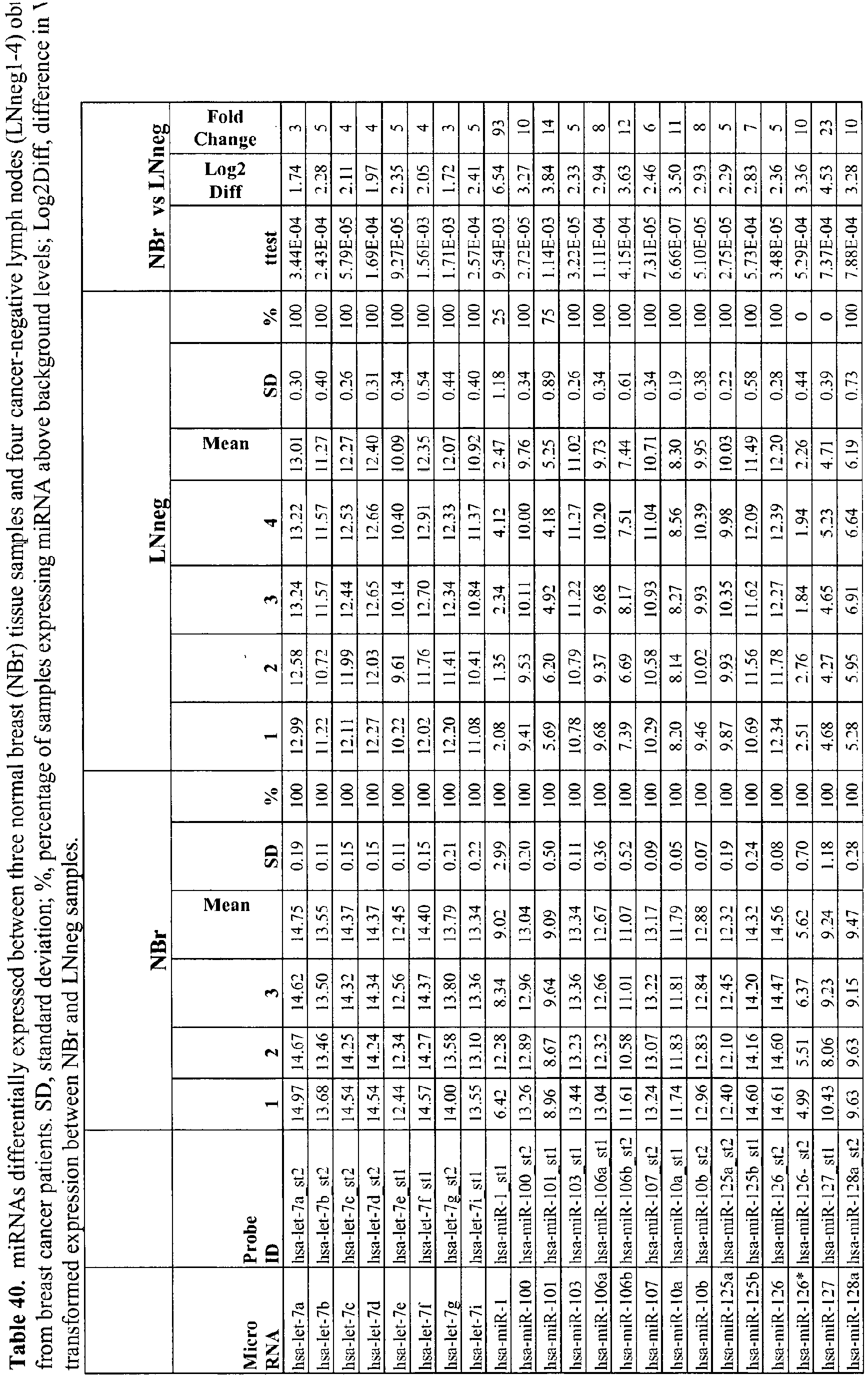

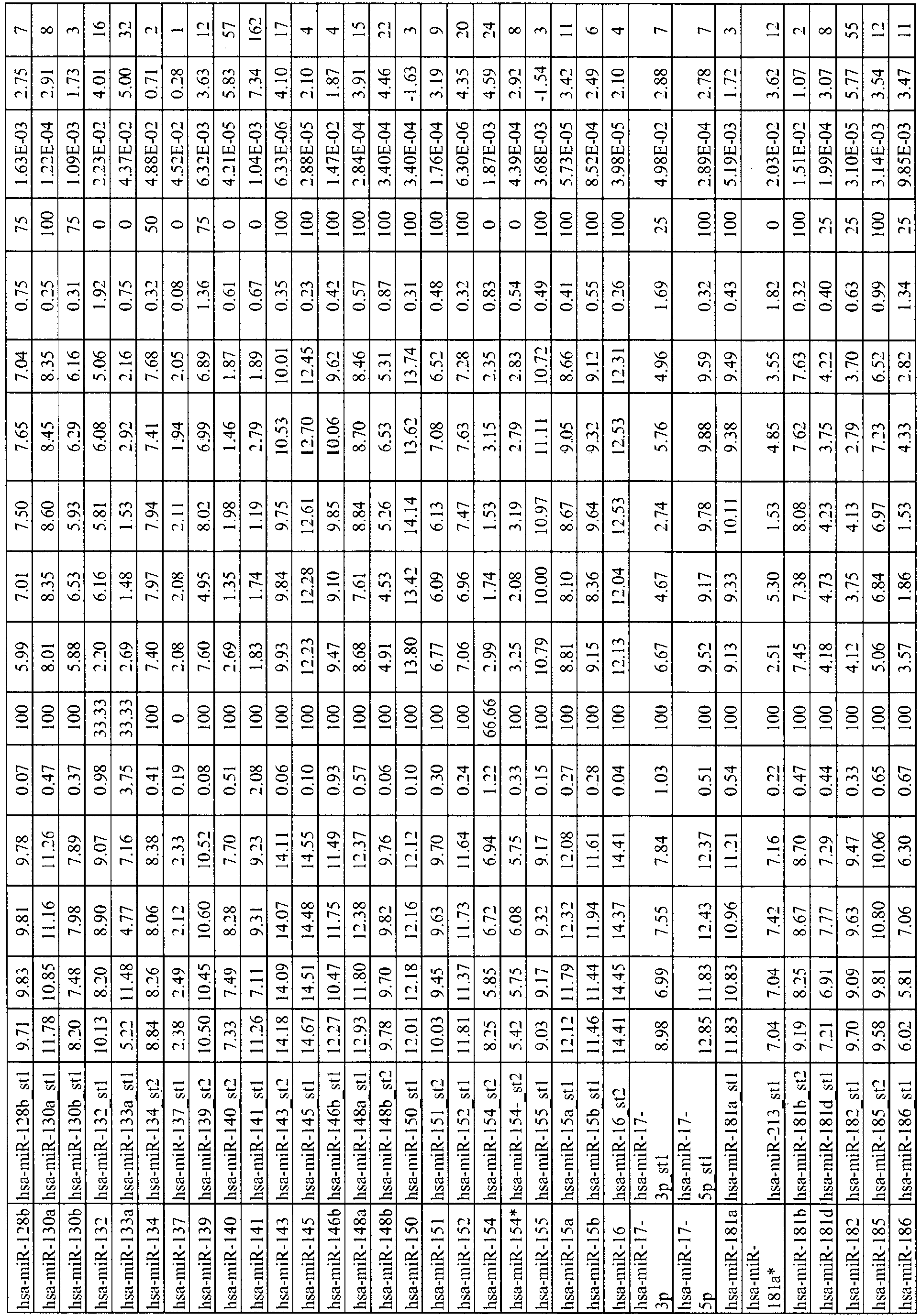

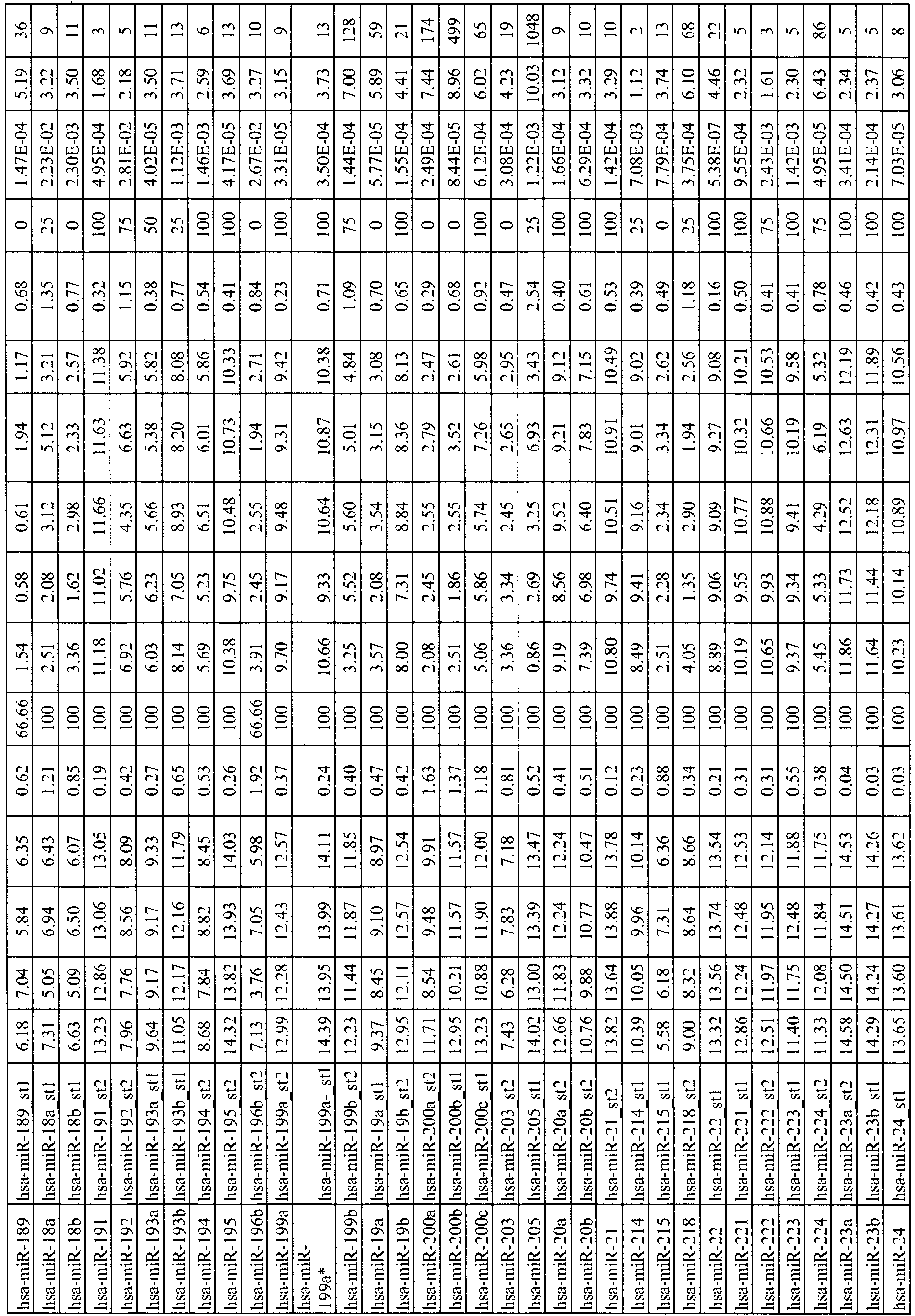

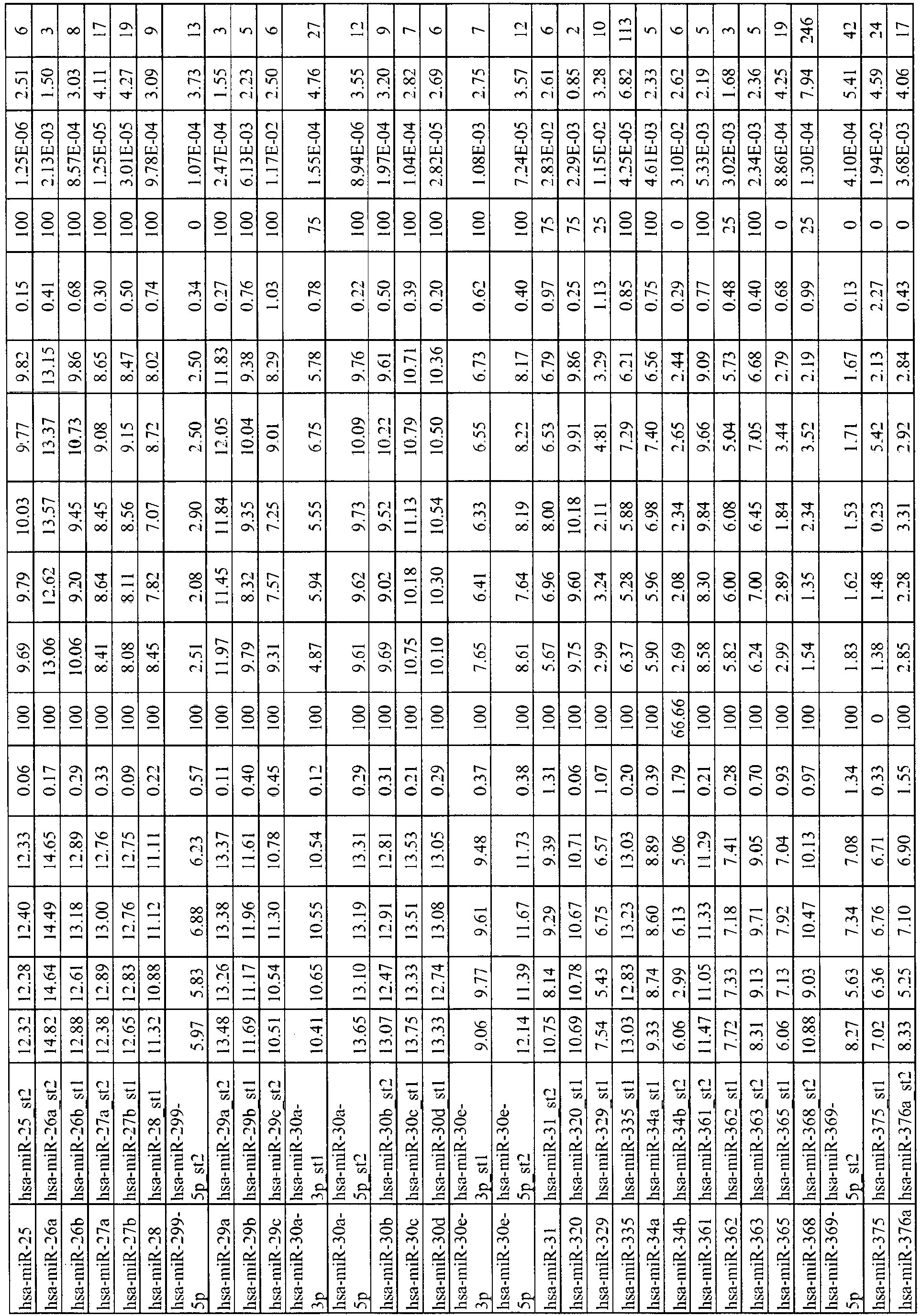

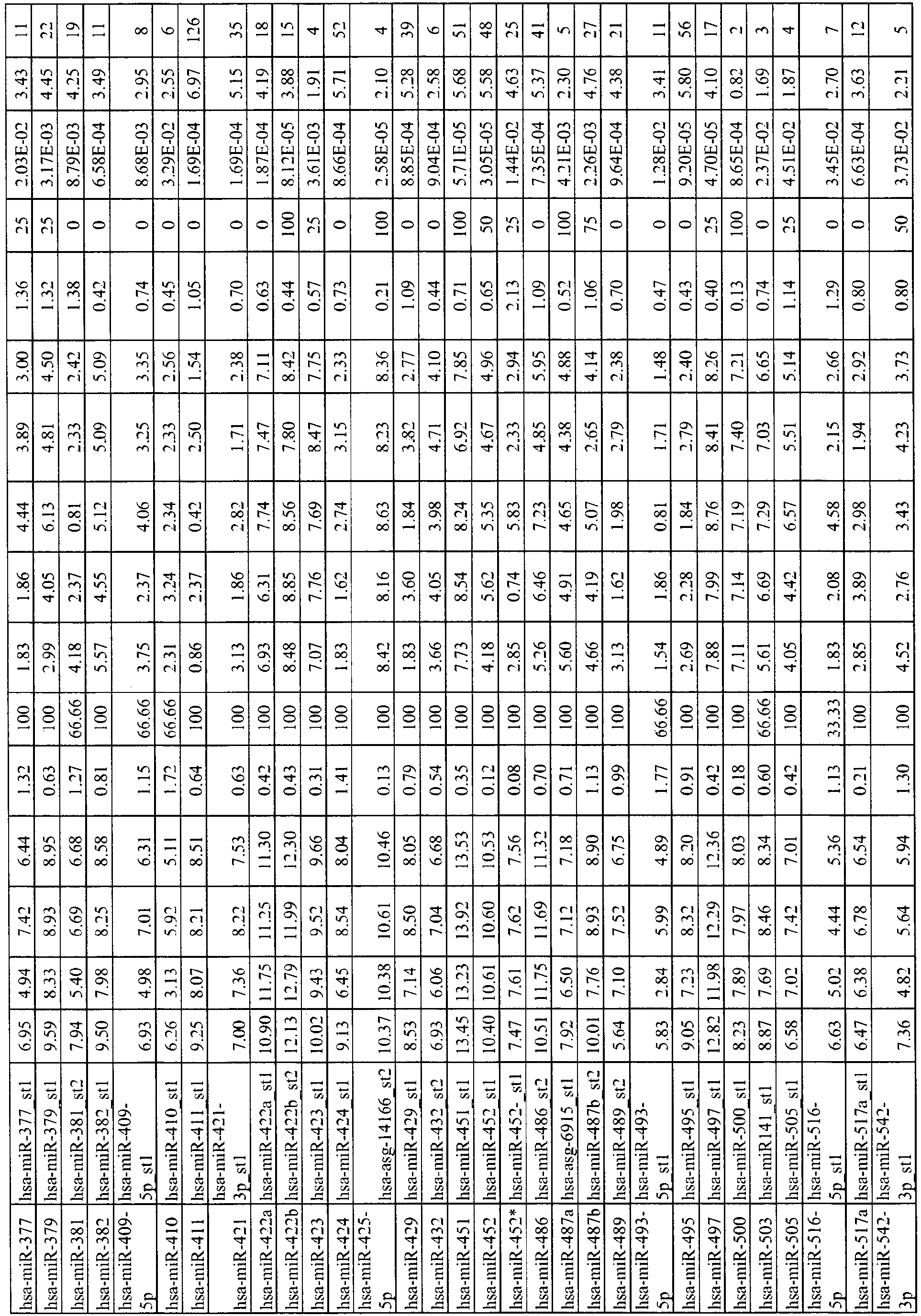

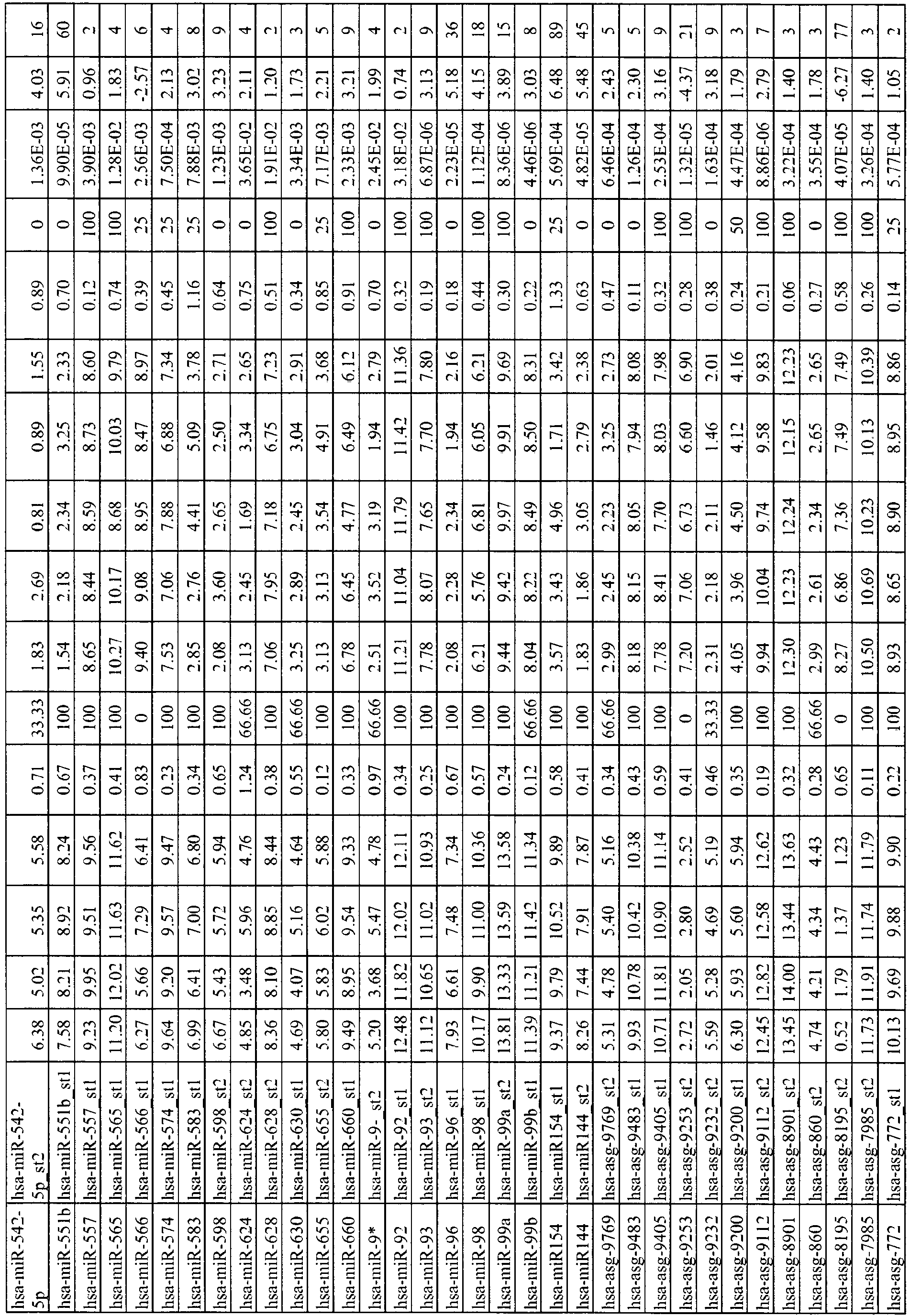

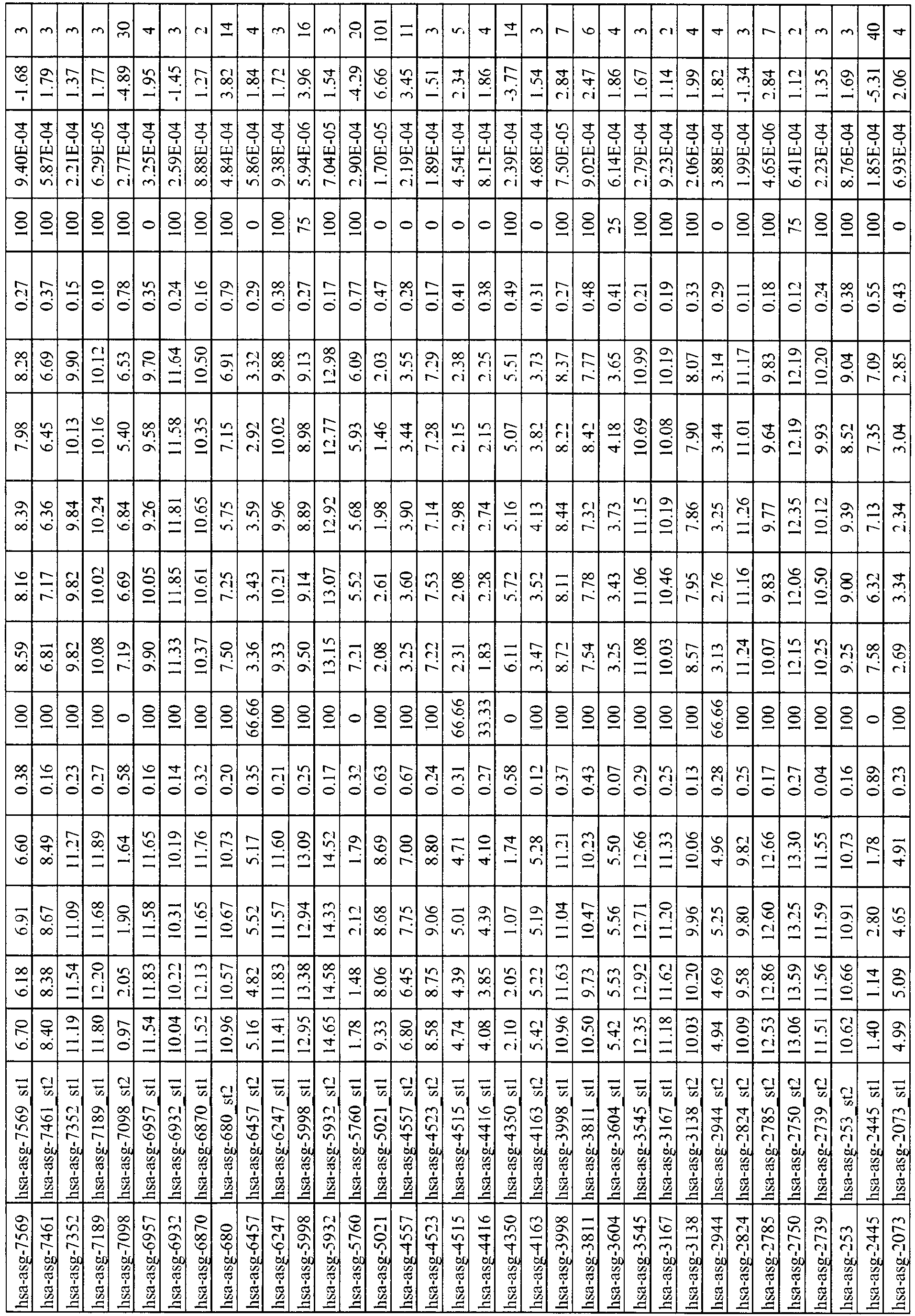

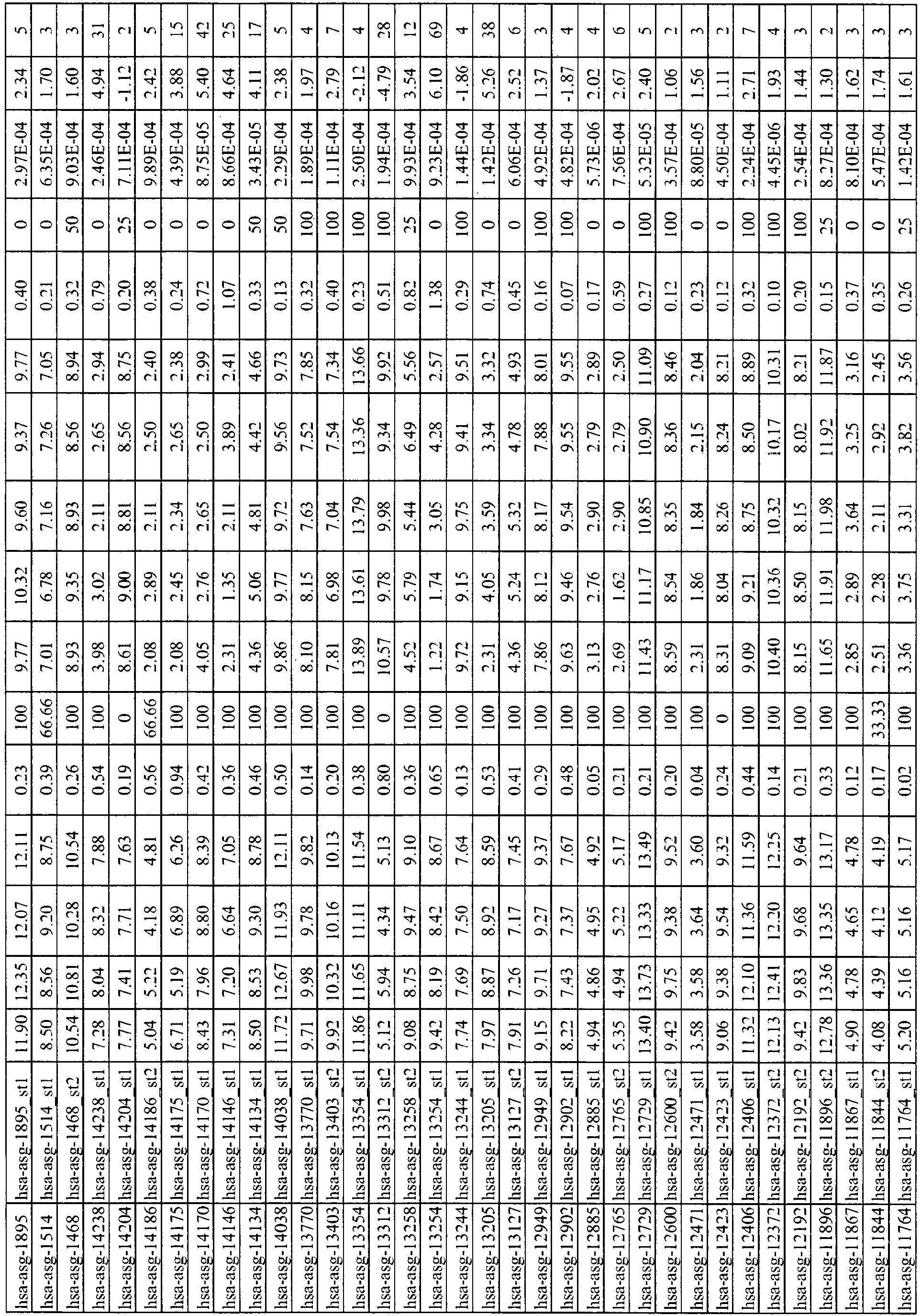

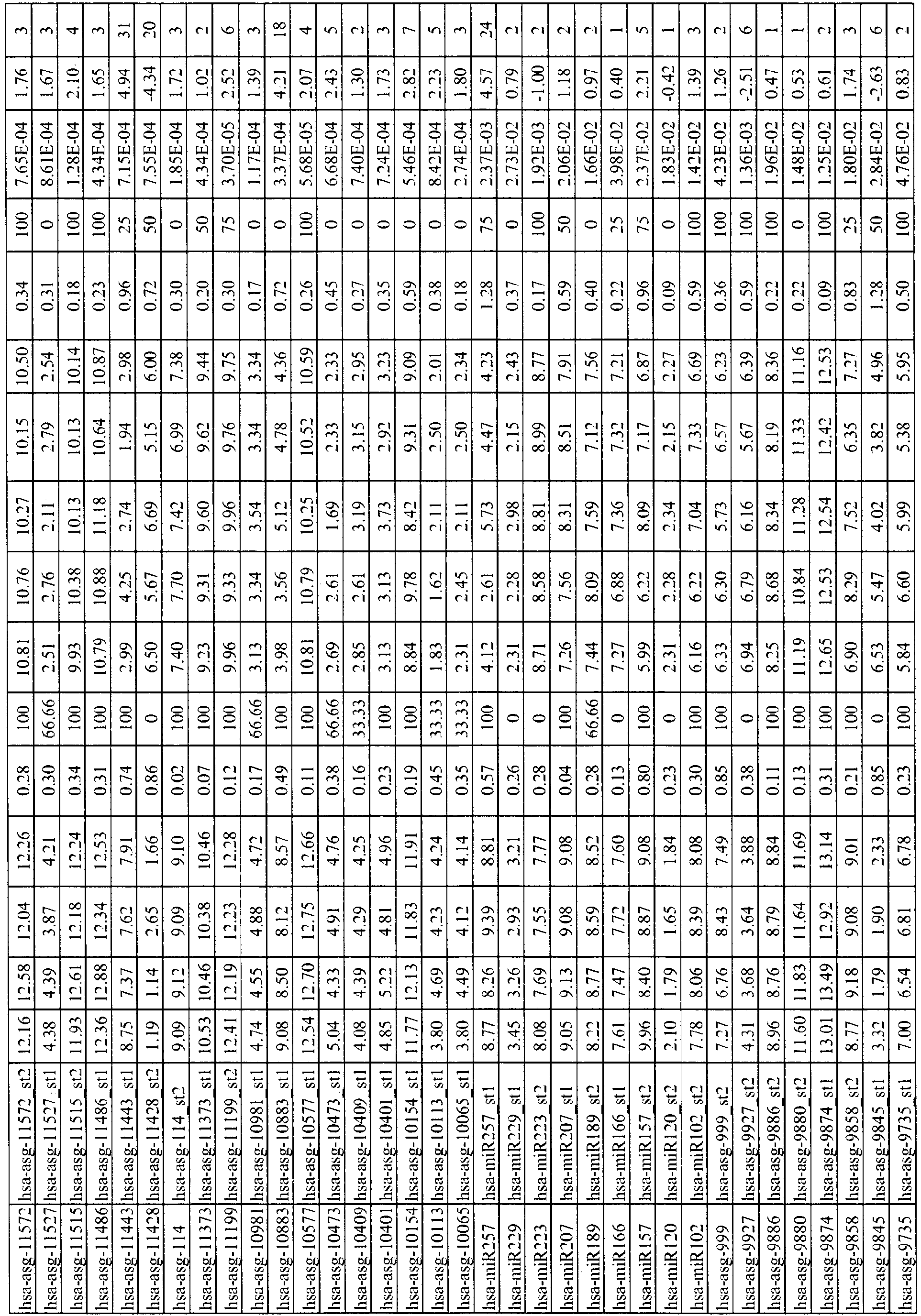

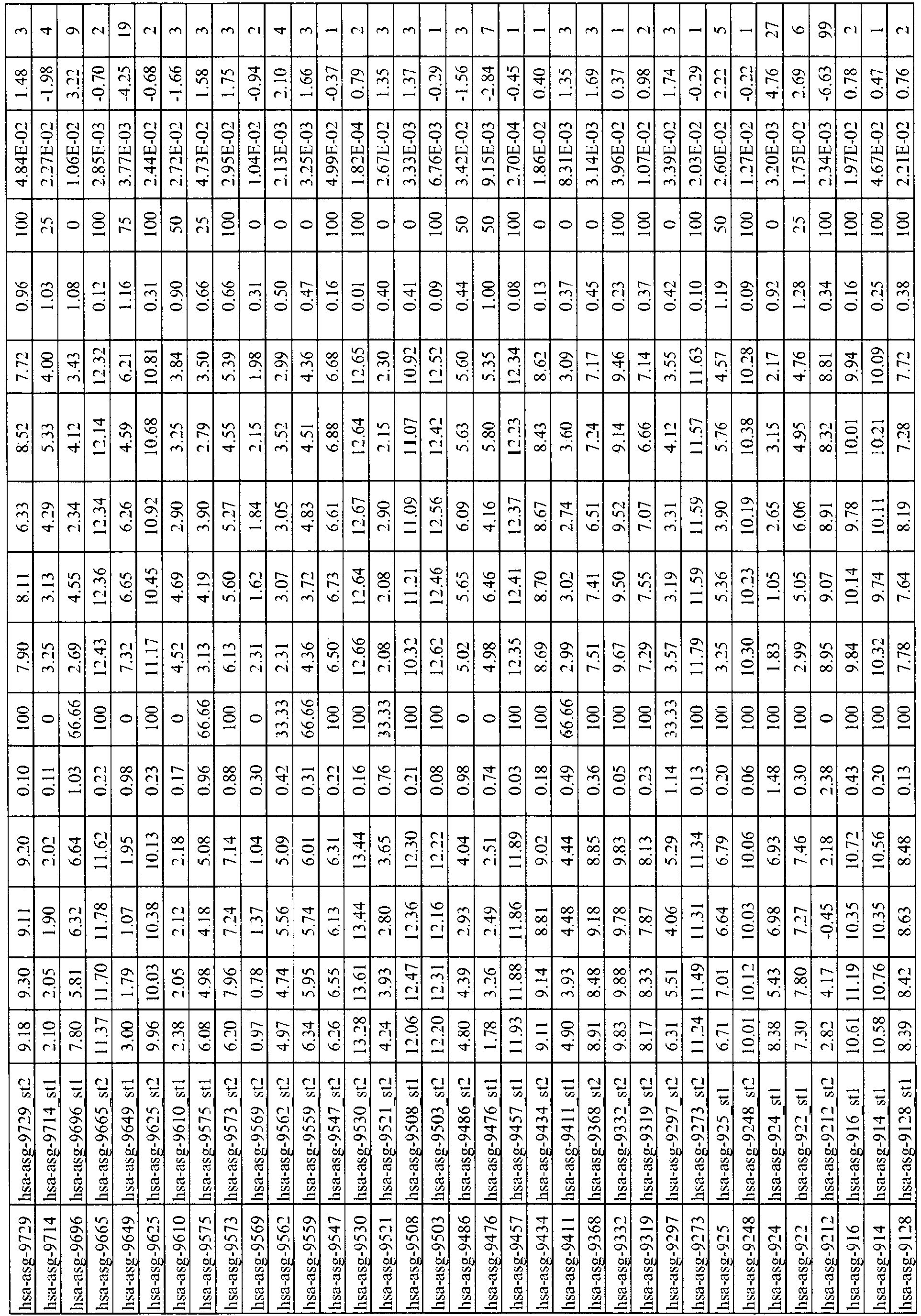

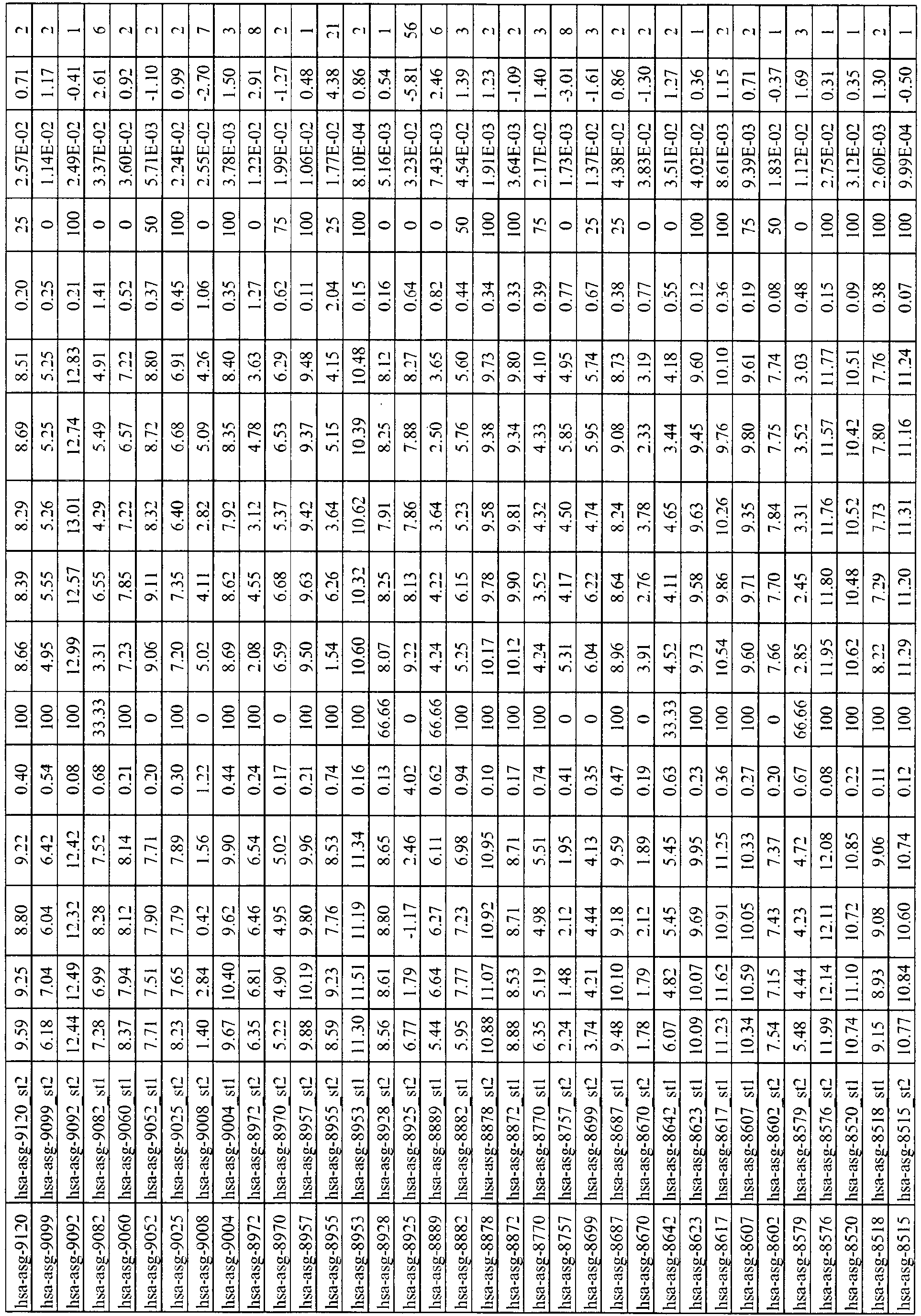

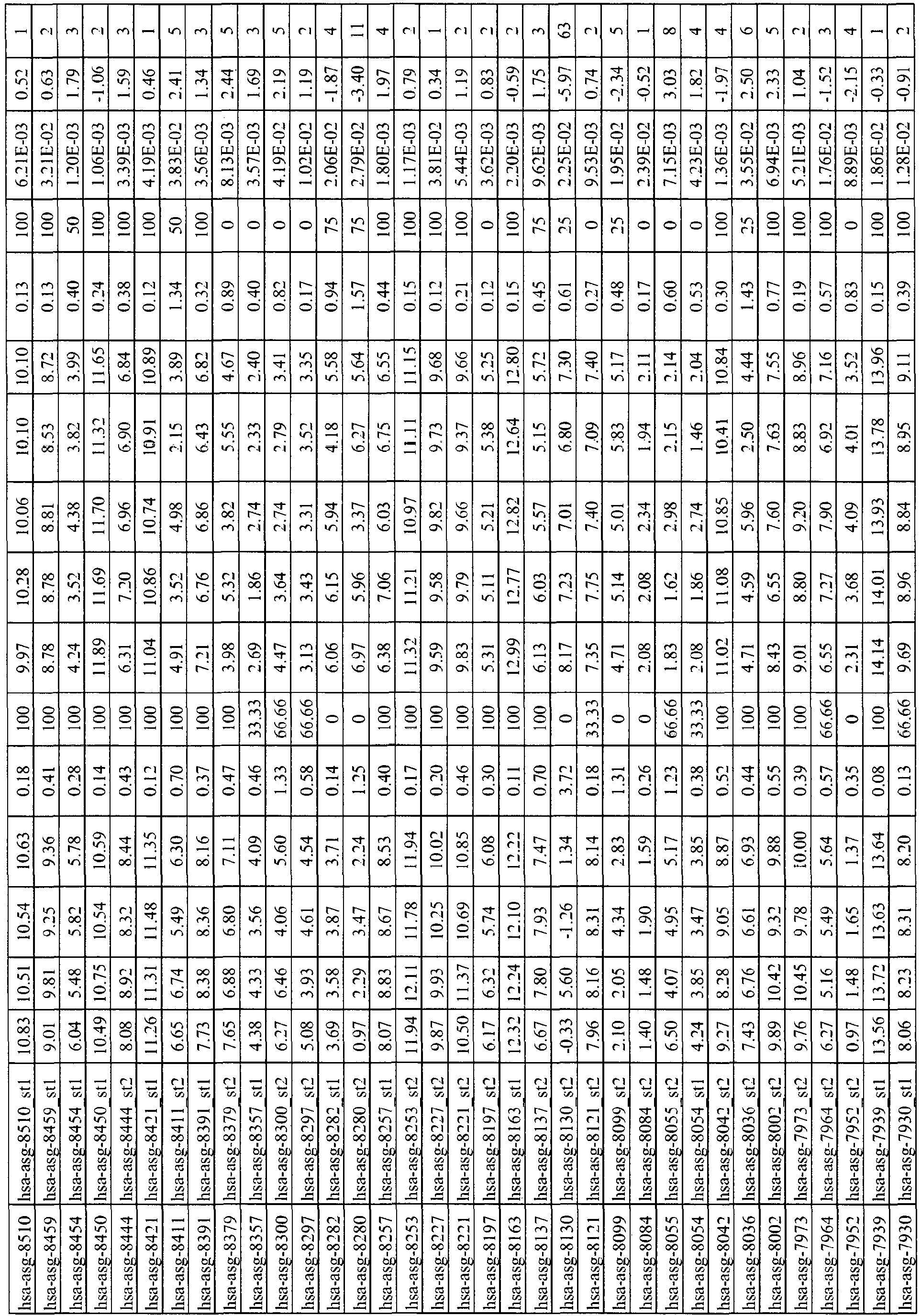

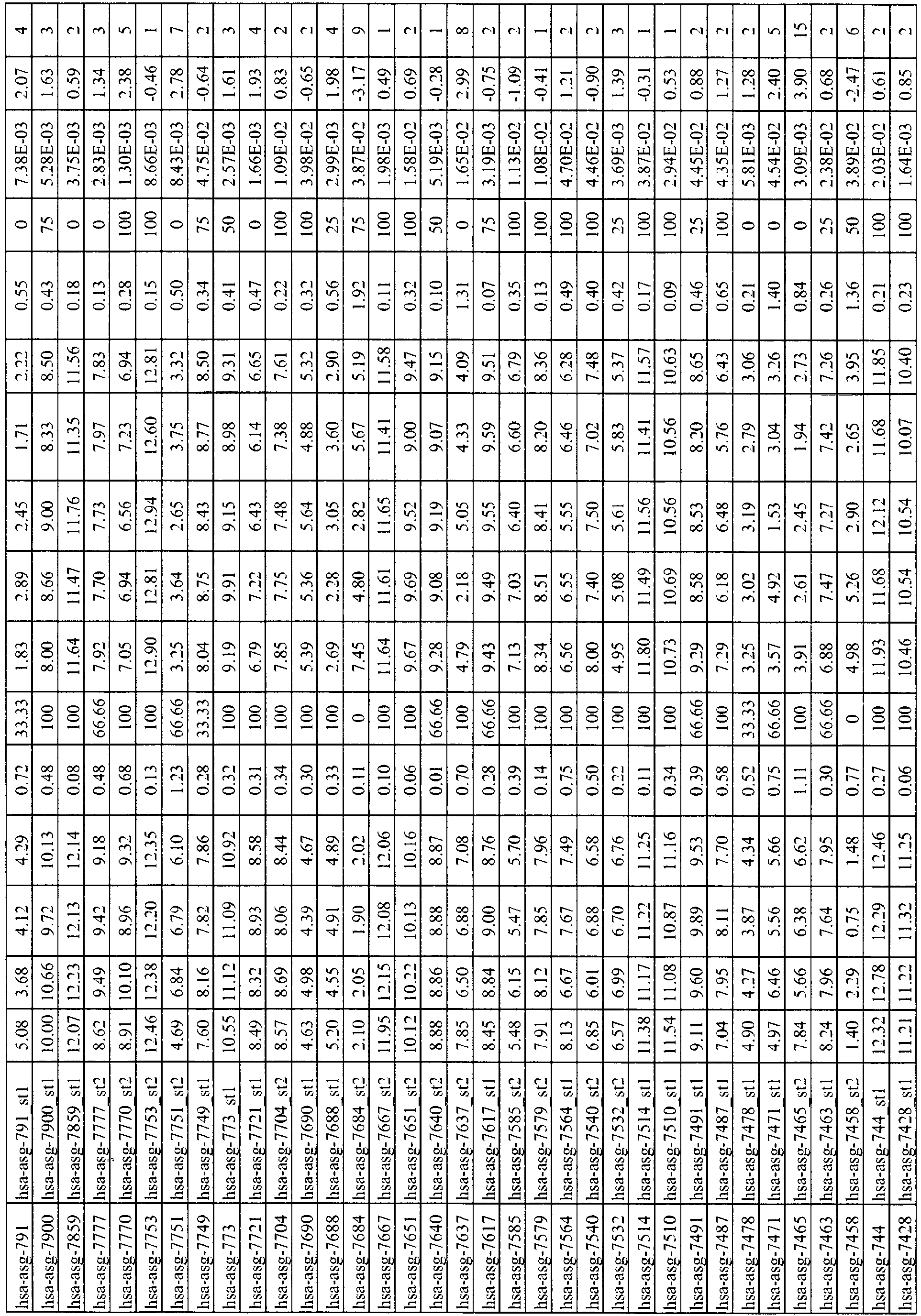

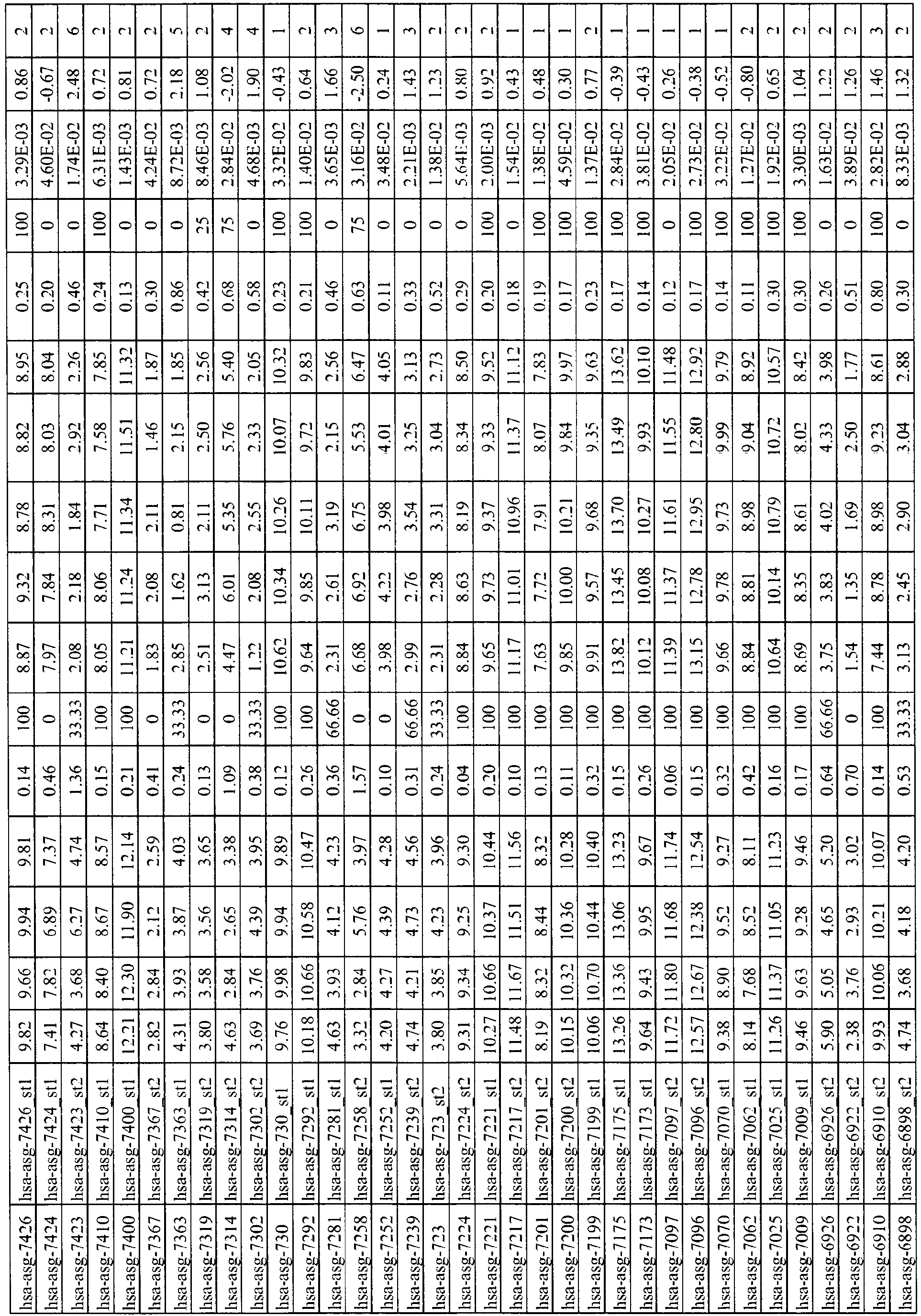

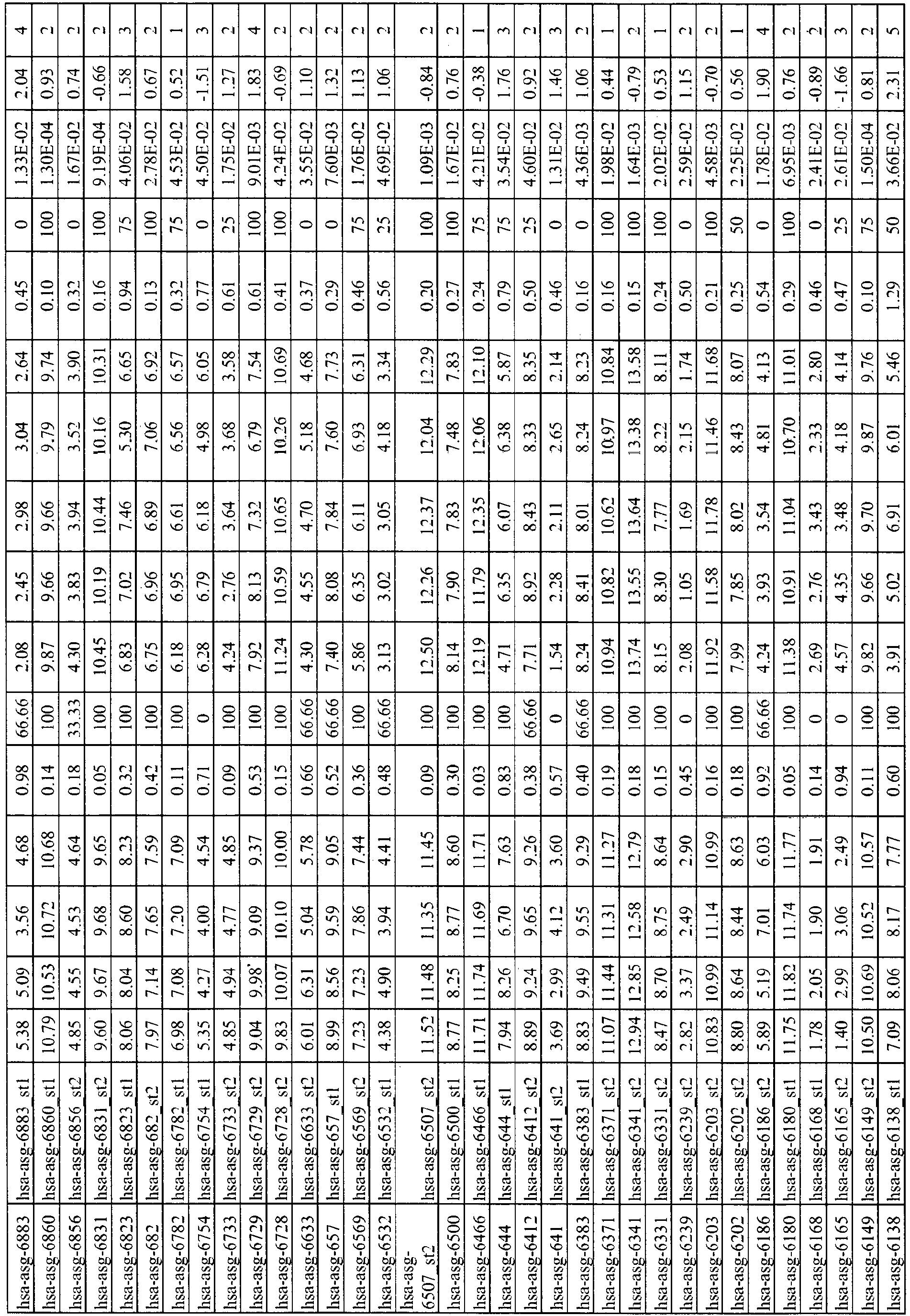

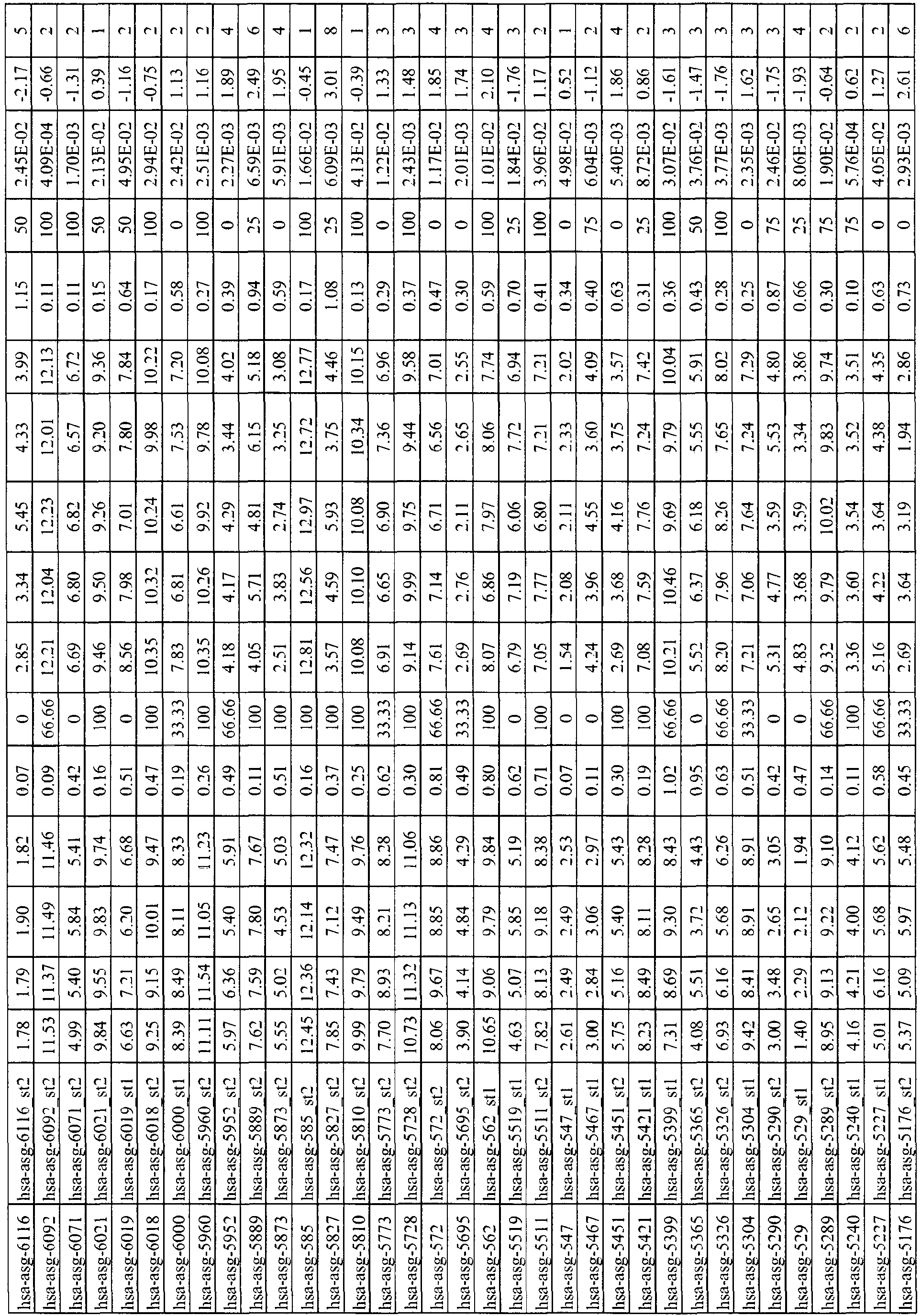

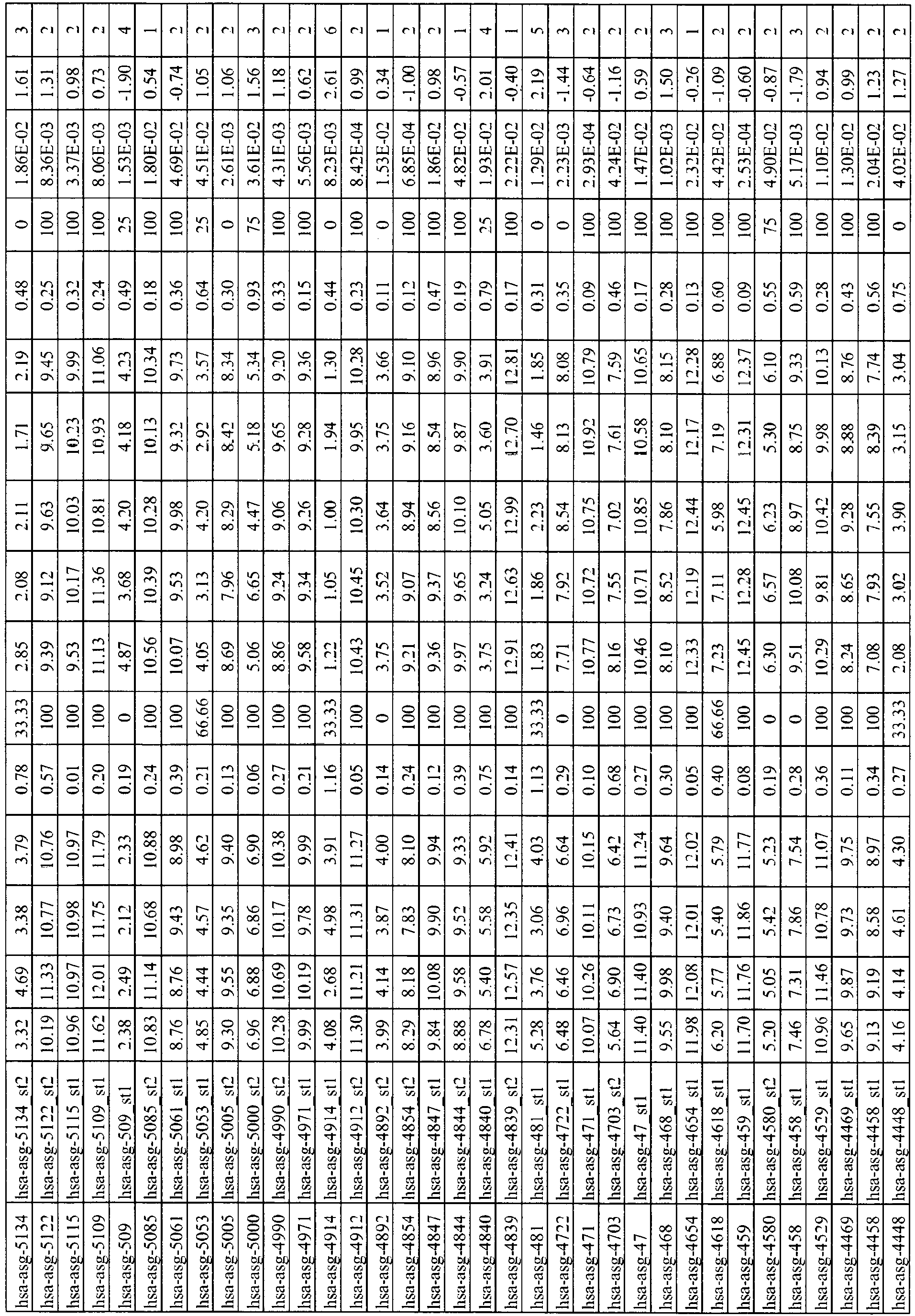

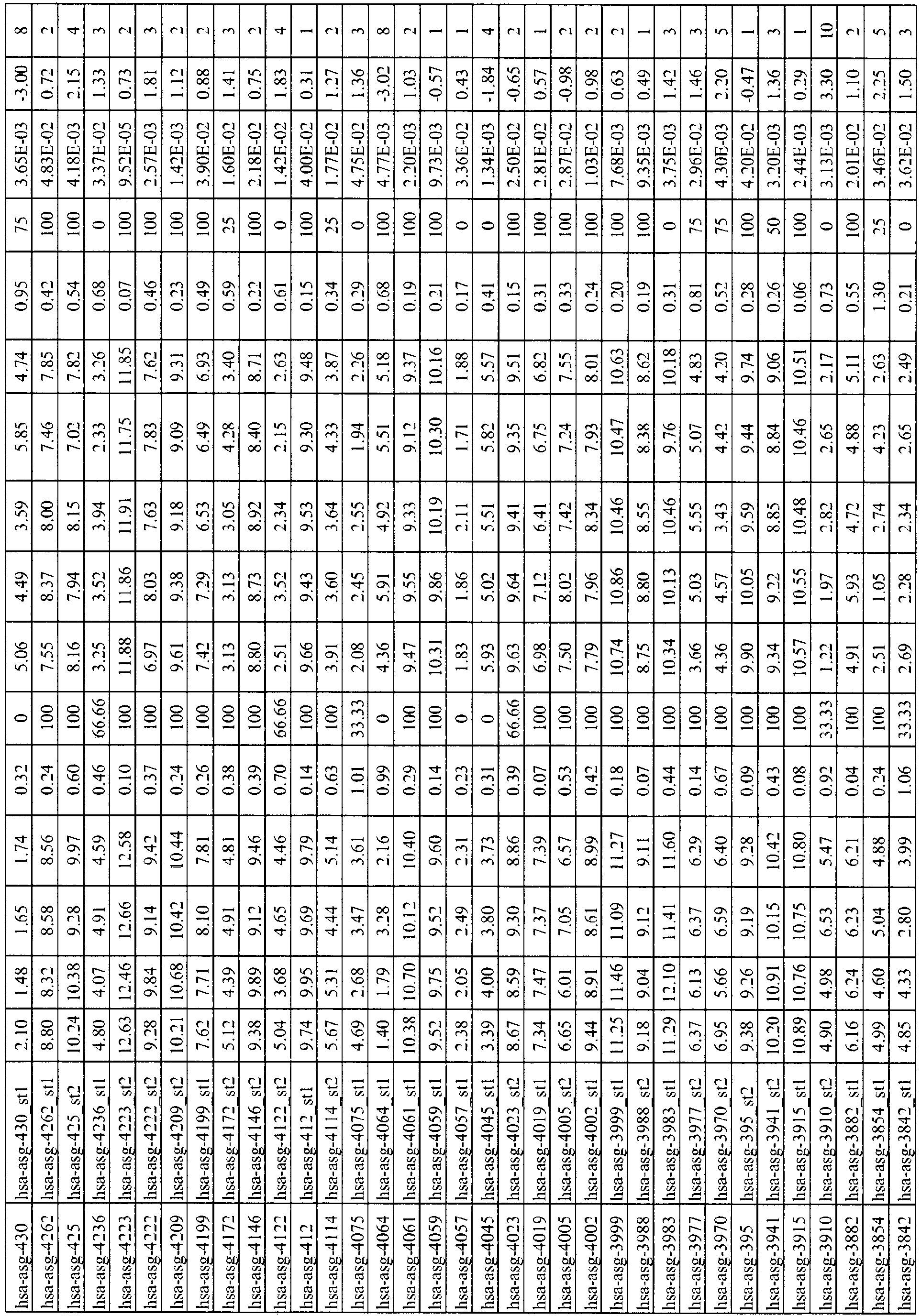

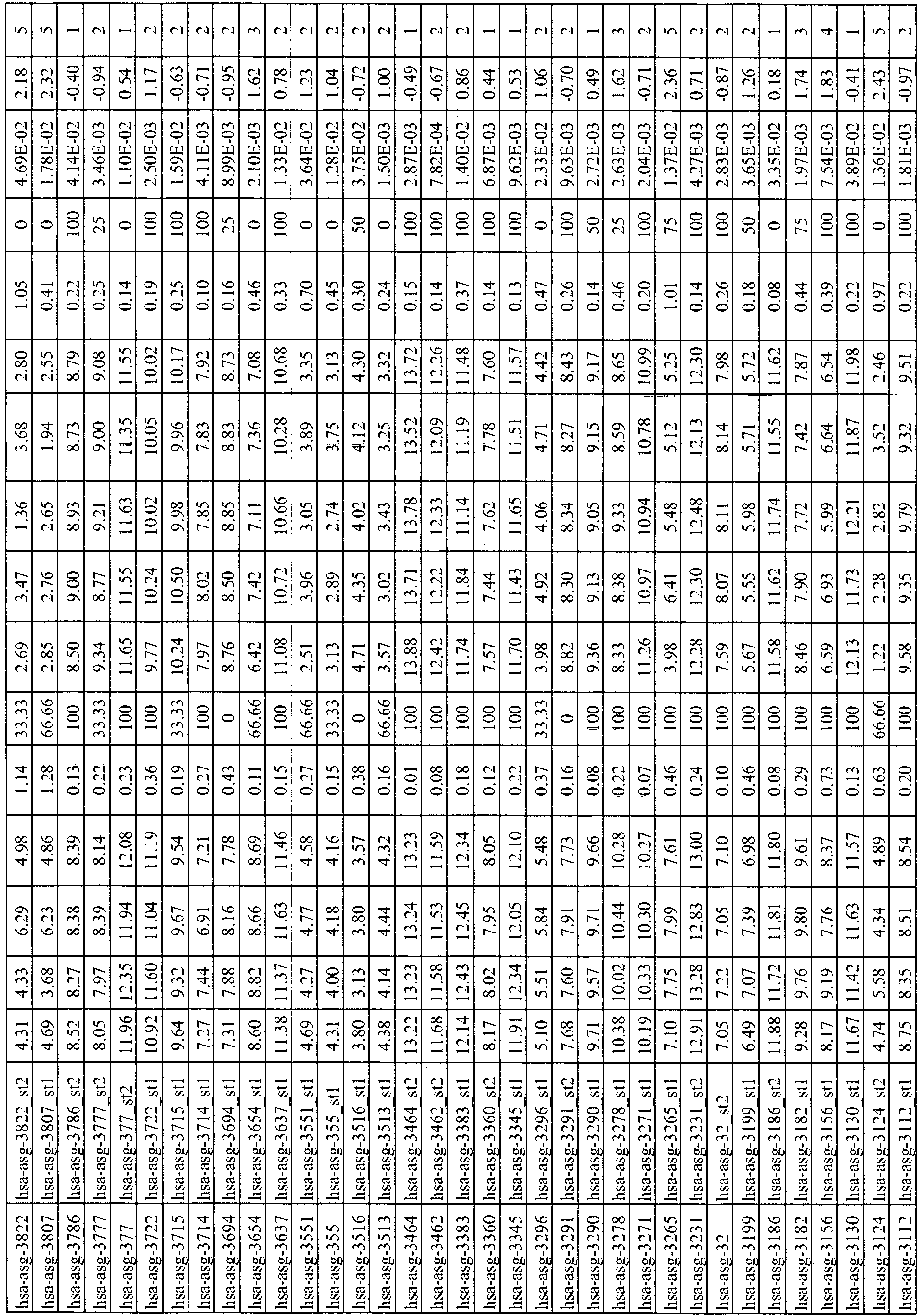

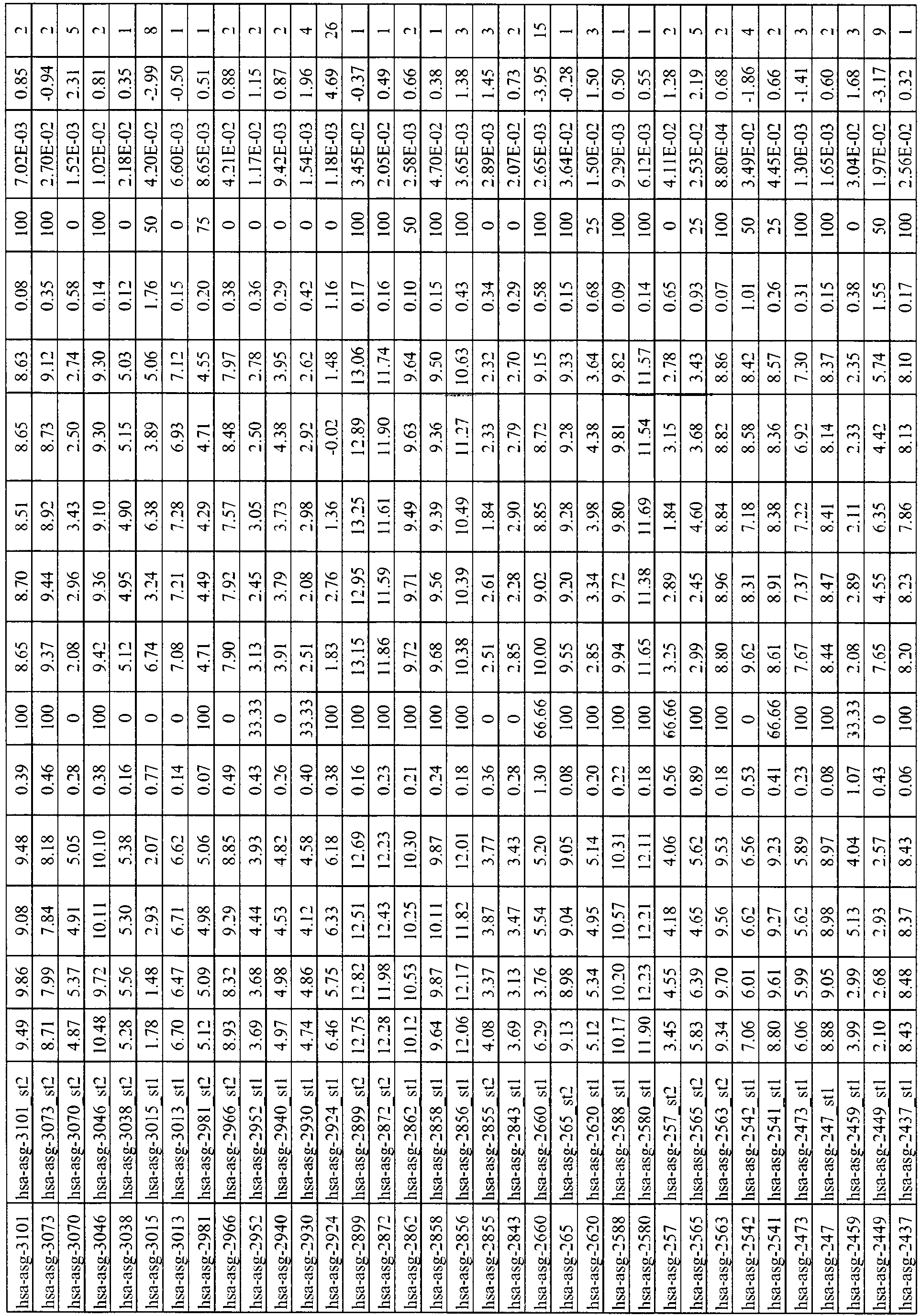

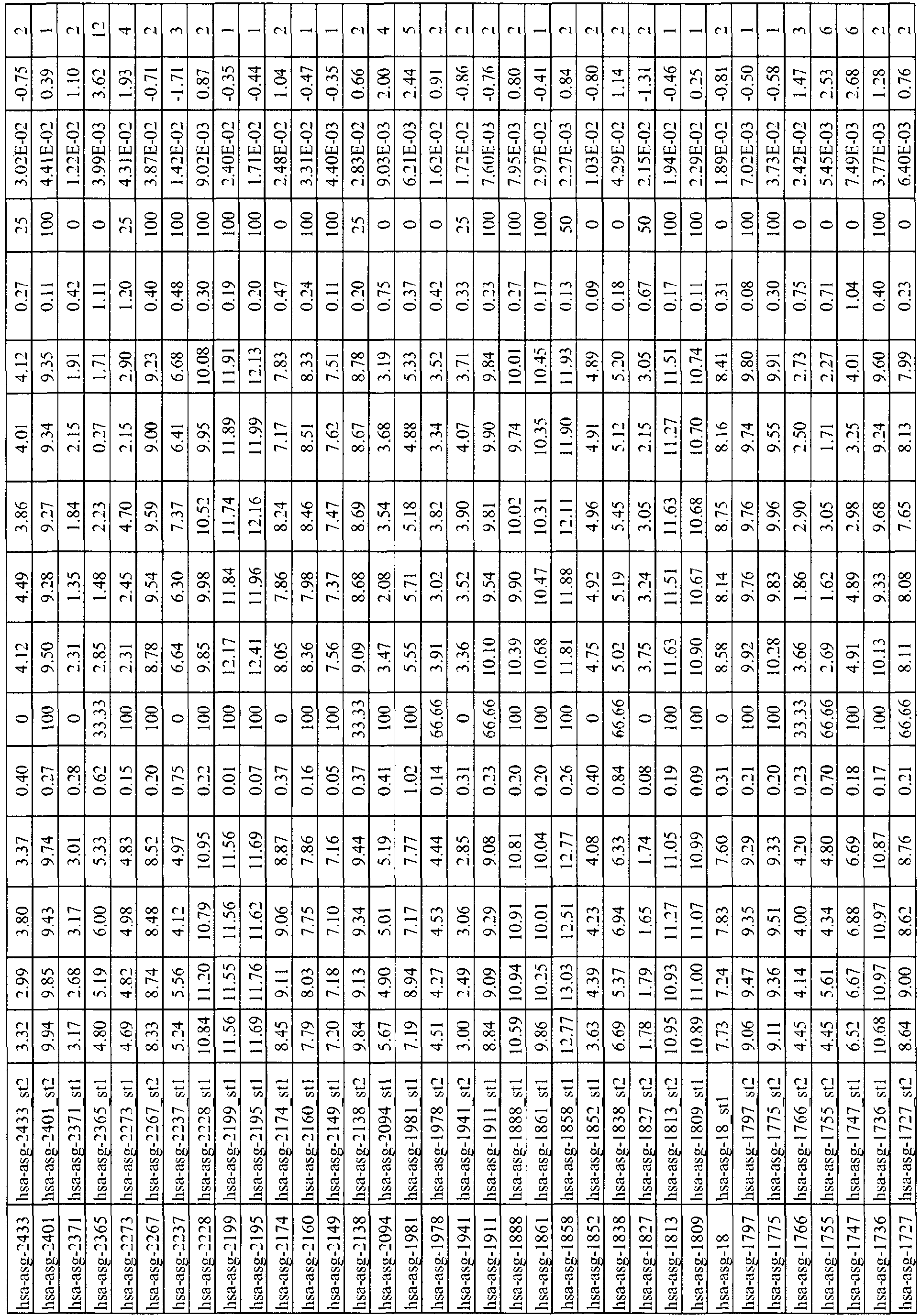

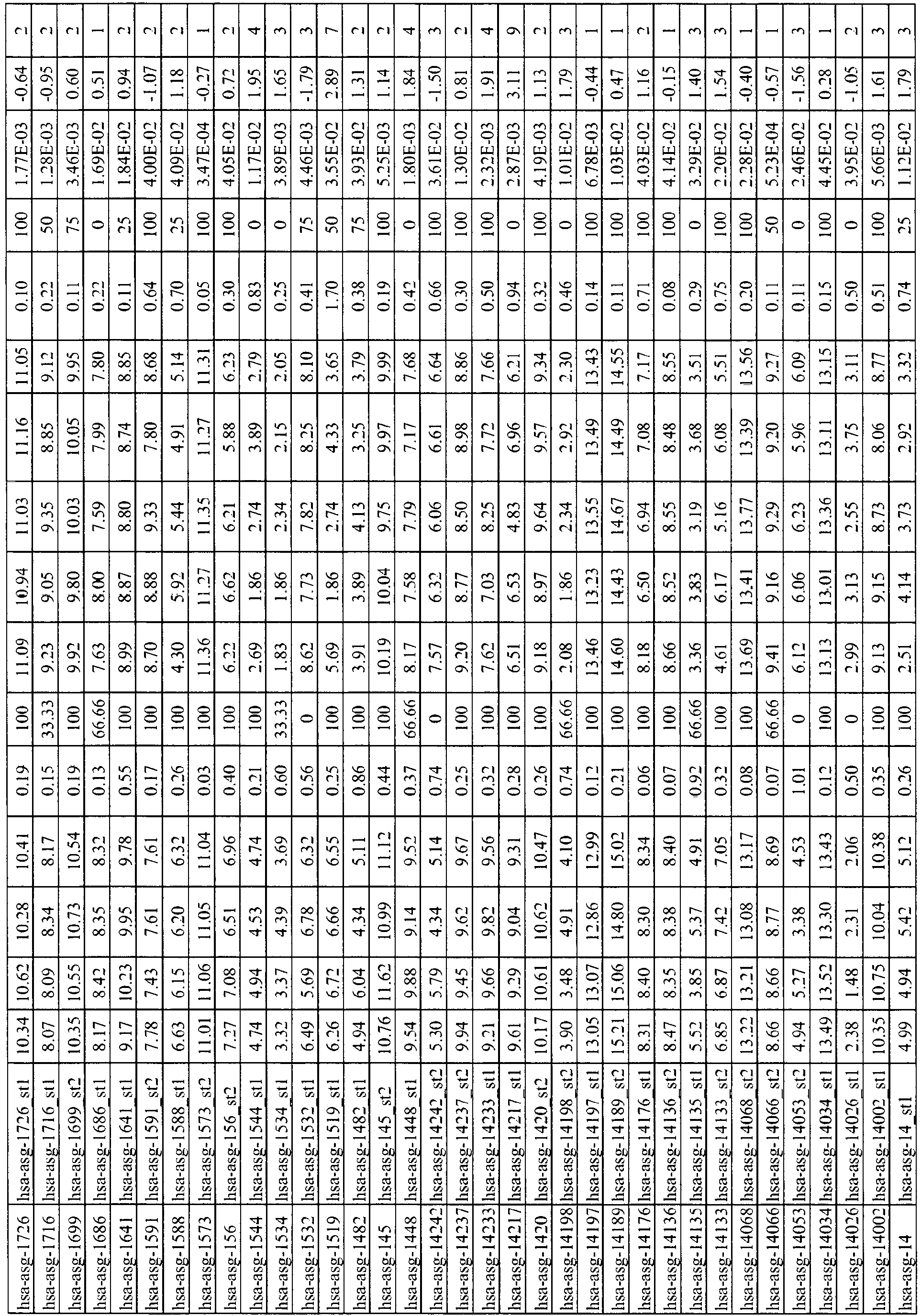

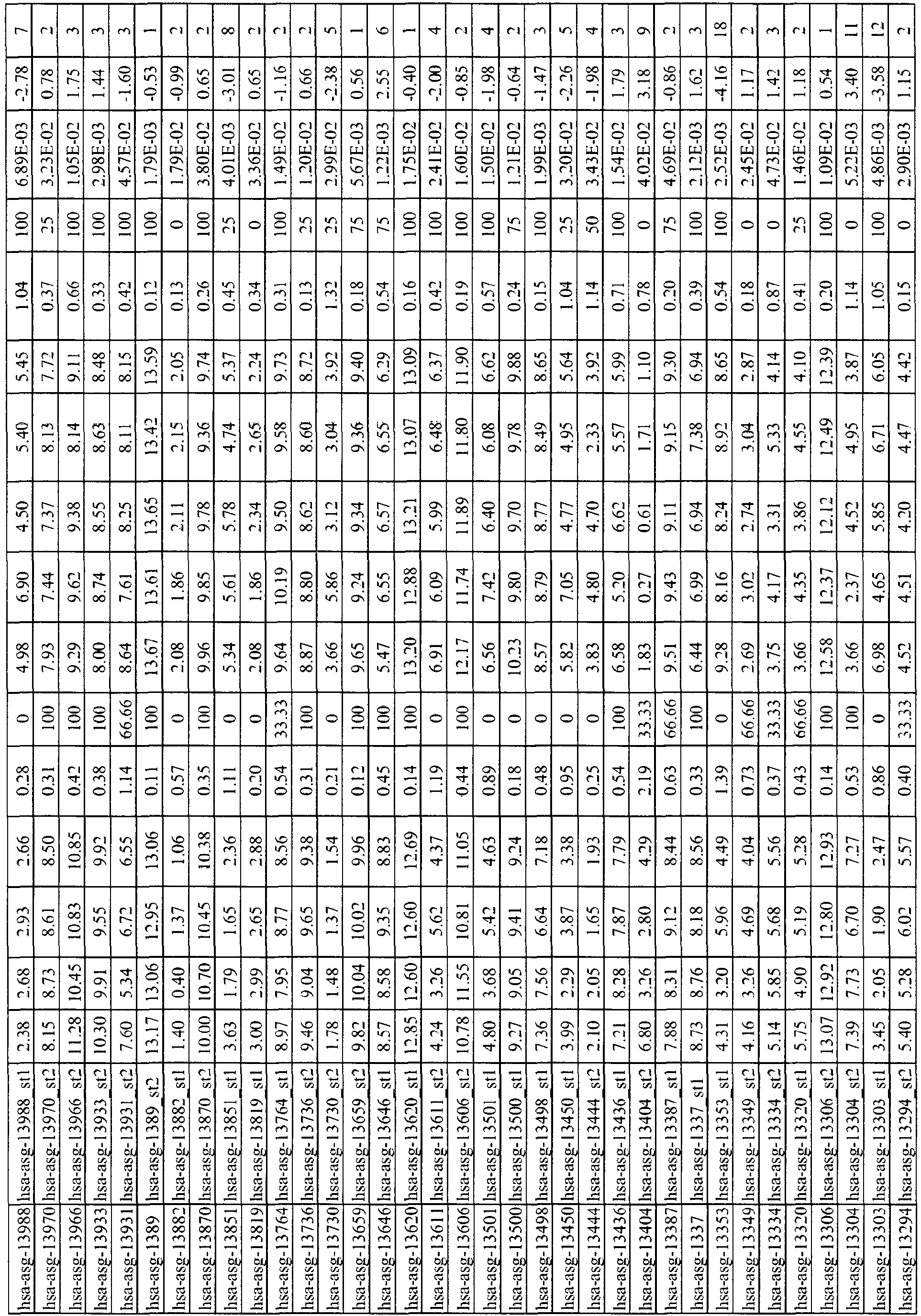

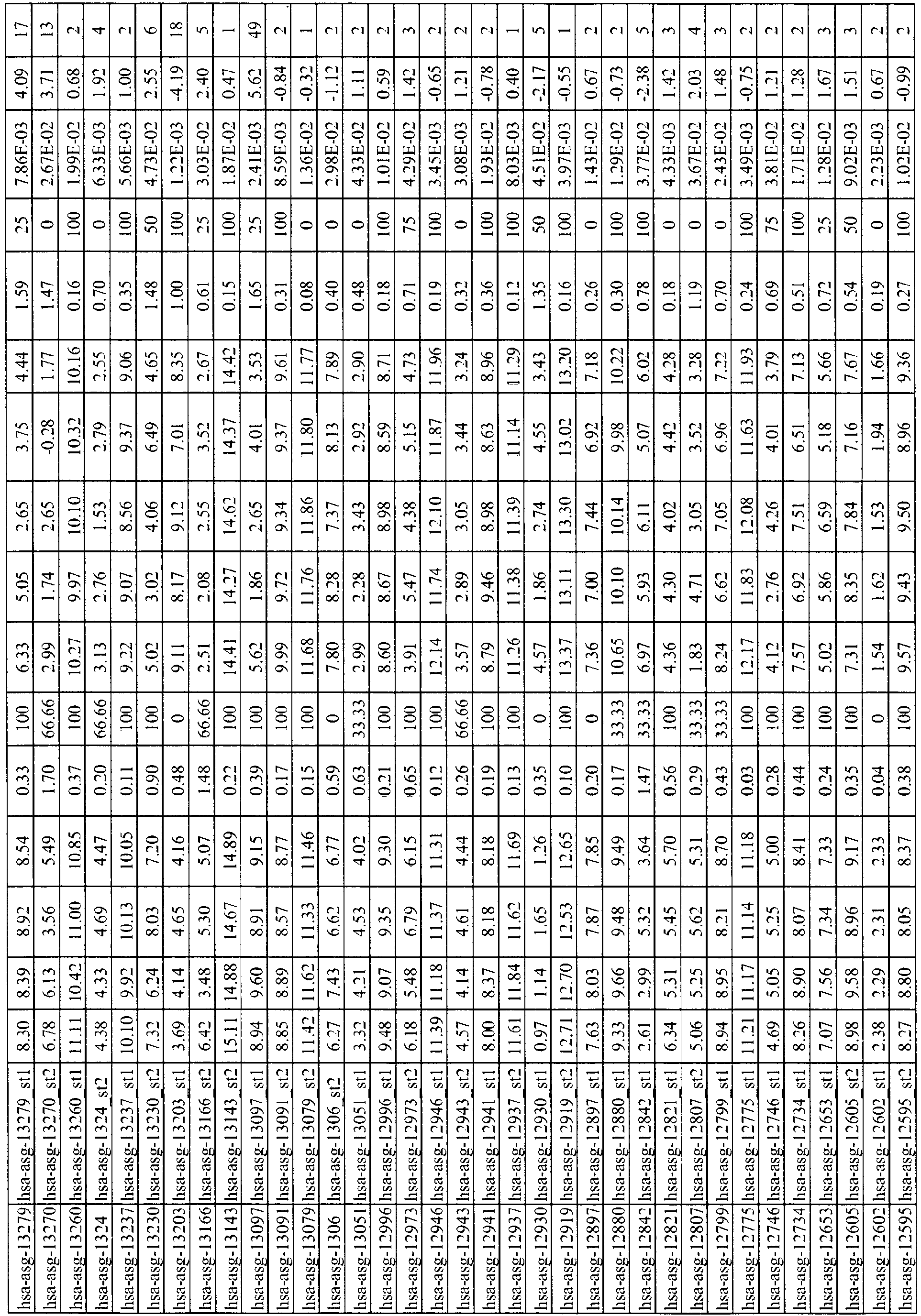

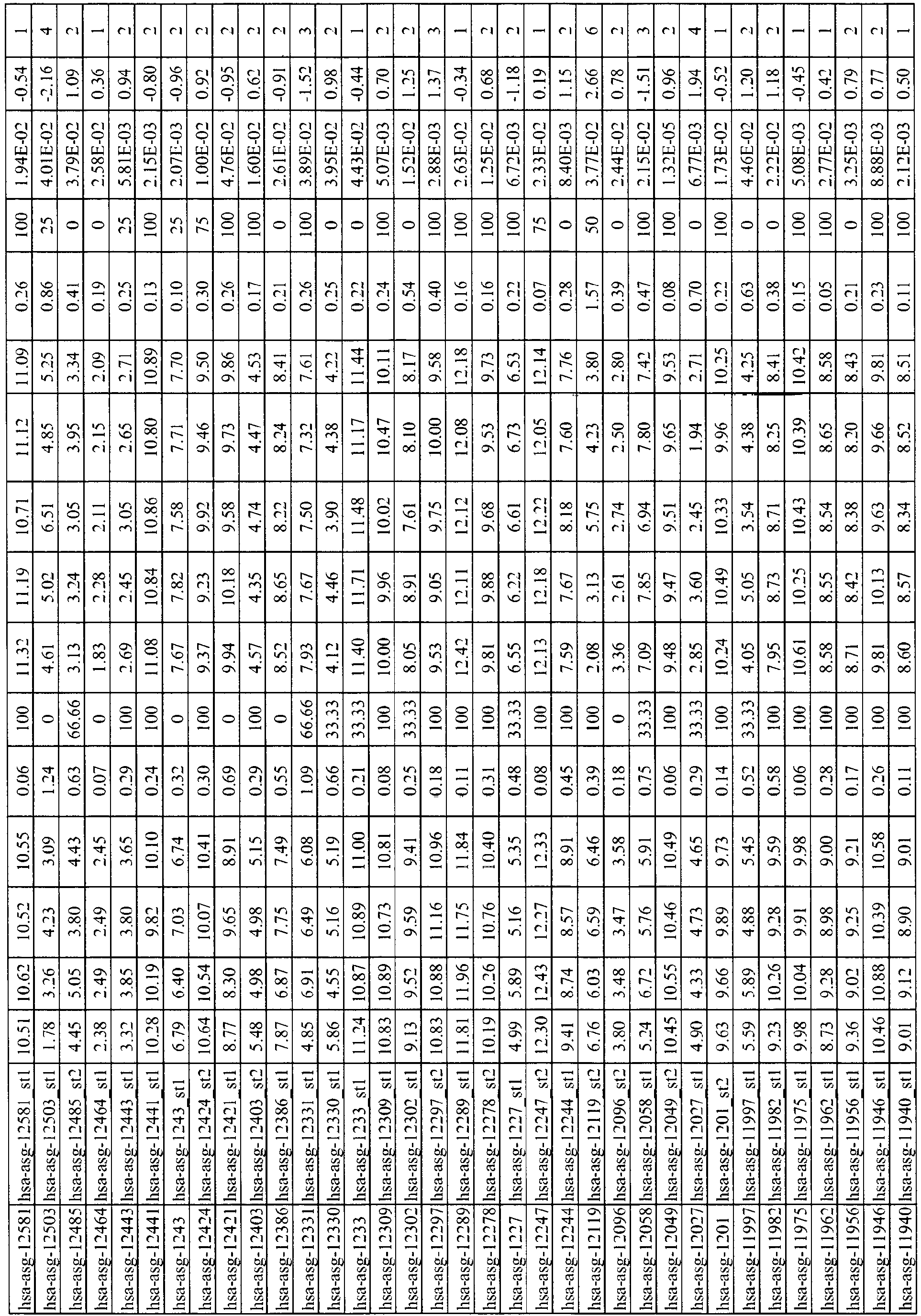

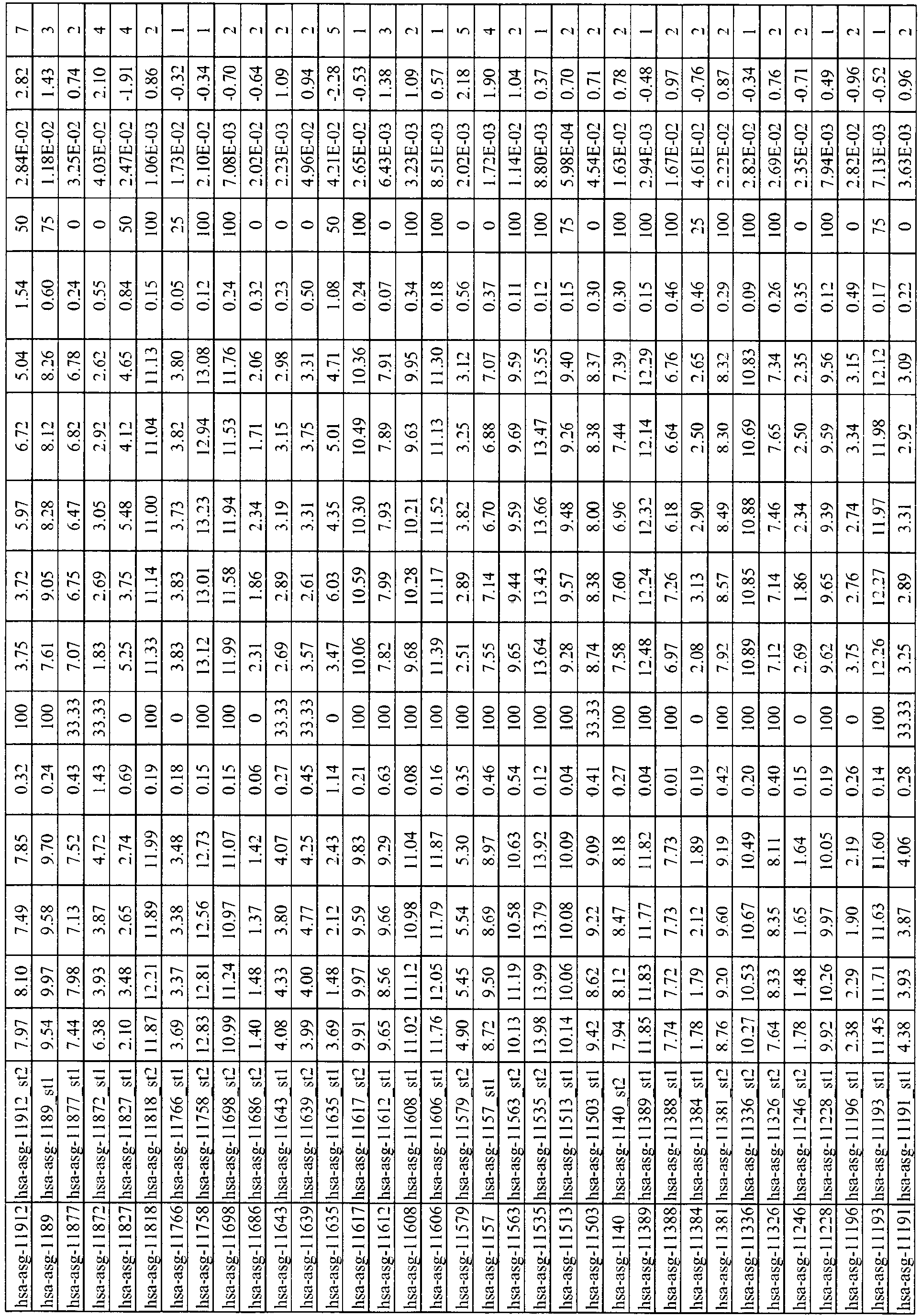

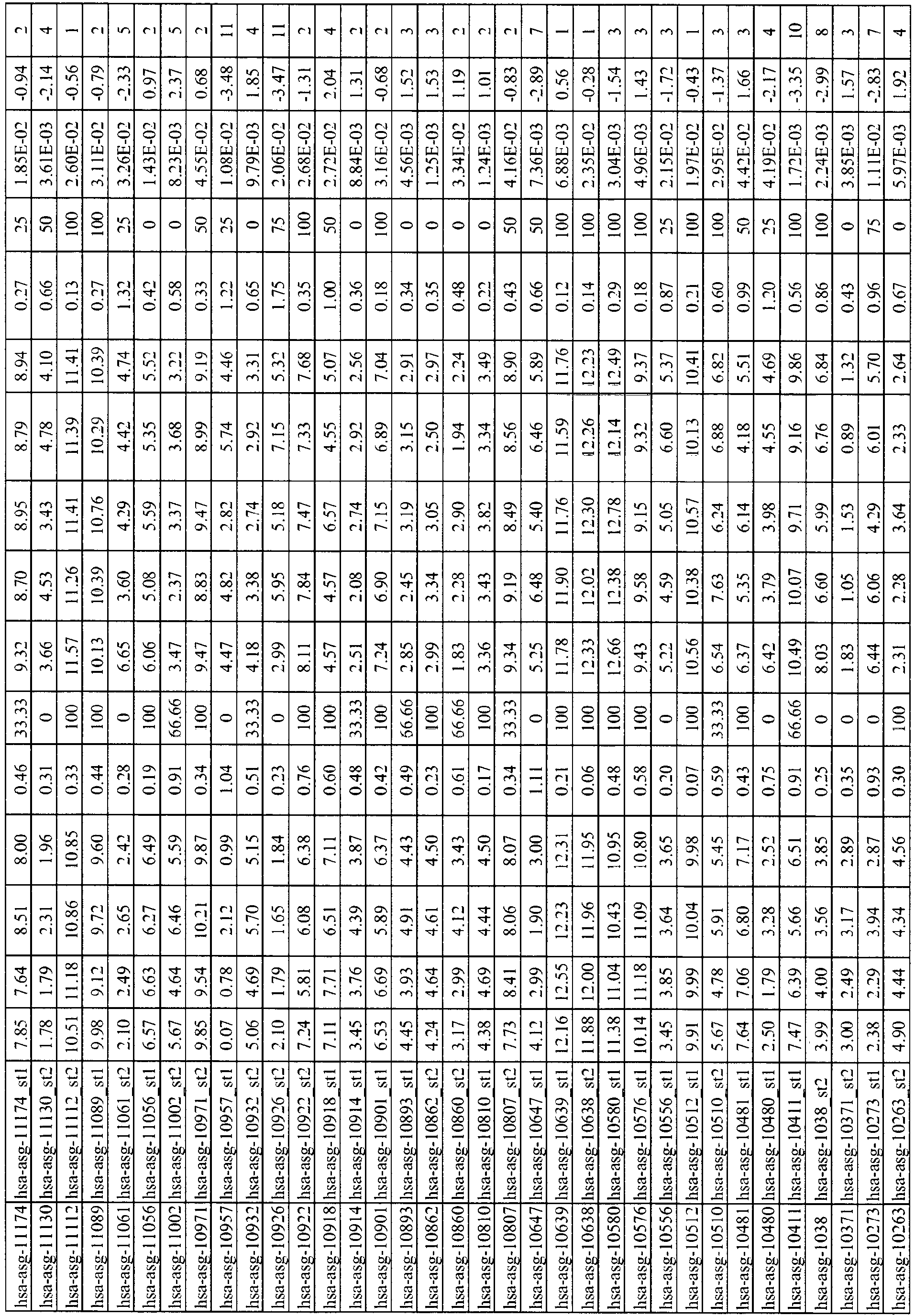

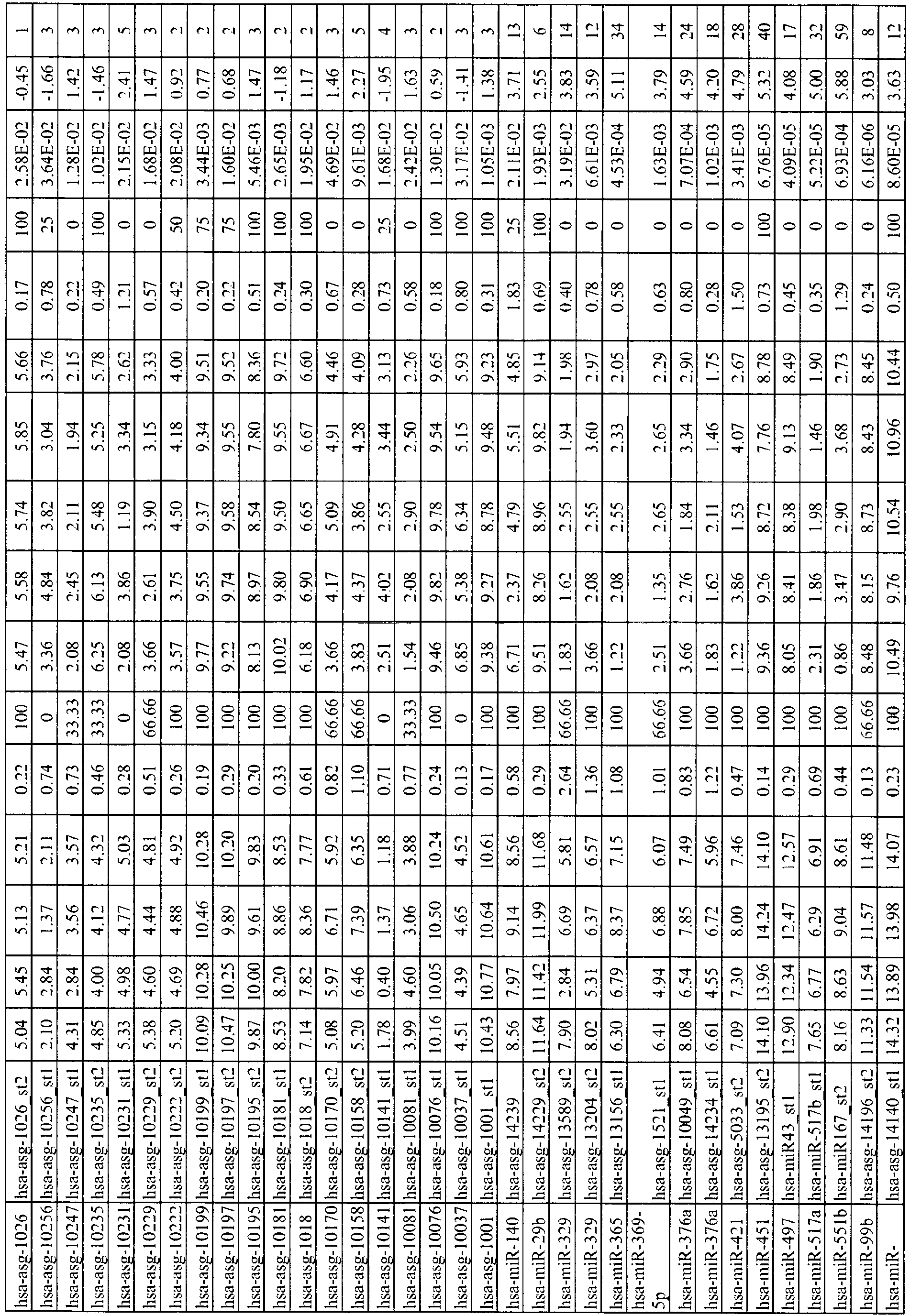

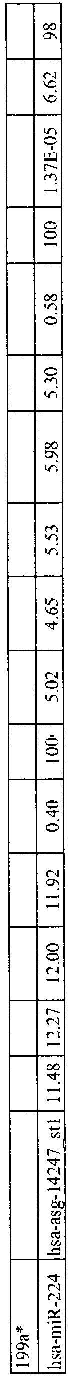

### EXAMPLE 42

### COMMON MIRNAS DIFFERENTIALLY EXPRESSED BETWEEN NORMAL BREAST TISSUE SAMPLES AND CANCER-NEGATIVE AXILLARY LYMPH NODES AND BETWEEN CANCER-POSITIVE AND CANCER-NEGATIVE AXILLARY LYMPH NODES FROM BREAST CANCER PATIENTS

The inventors determined the common miRNAs that are differentially expressed between normal breast tissue (NBr) samples and cancer-negative axillary lymph nodes (LNneg) (identified in Example 41) and between cancer-positive (LNpos) and cancer-negative (LNneg) axillary lymph nodes from breast cancer patients (identified in Example 40). For this analysis, differentially expressed miRNAs were identified, using a p-value<0.05 and a fold-change>2 (Table 39 and Table 40). These miRNAs represent particularly useful biomarkers for monitoring the presence of breast cancer metastasis to axillary lymph nodes.

Among the 50 verified miRNAs expressed at higher levels in LNpos than in LNneg samples, 45 also had average levels of expression higher in NBr tissues than in LNneg samples (Table 41). Among these, five miRNAs (hsa-miR-200b, -200a, -141, -200c, and - 182) had a level of expression between 50- and 500-fold lower in LNneg than their average level of expression in either NBr or LNpos samples..

**Table 41. miRNAs differentially expressed between both normal breast (NBr) tissue samples and cancer-negative axillary lymph nodes (LNneg) and between cancer-positive (LNpos) and cancer-negative (LNneg) axillary lymph nodes.**

| **Probe ID** | **MicroRNA** | **Fold Change (NBr vs LNneg)** | **Fold Change (LNpos vs LNneg)** |
|---|---|---|---|
| hsa-miR-375_st1 | hsa-miR-375 | 24 | 325 |
| hsa-miR-200b_st1 | hsa-miR-200b | 499 | 322 |
| hsa-miR-141_st1 | hsa-miR-141 | 162 | 132 |
| hsa-miR-200a_st2 | hsa-miR-200a | 174 | 107 |
| hsa-miR-182_st1 | hsa-miR-182 | 55 | 84 |
| hsa-miR-200c_st1 | hsa-miR-200c | 65 | 56 |
| hsa-miR-203_st2 | hsa-miR-203 | 19 | 32 |
| hsa-miR-429_st1 | hsa-miR-429 | 39 | 17 |
| hsa-miR-542-5p_st2 | hsa-miR-542-5p | 16 | 12 |
| hsa-miR-421-3p_st1 | hsa-miR-421 | 35 | 12 |
| hsa-miR-181d_st1 | hsa-miR-181d | 8 | 9 |
| hsa-miR-213_st1 | hsa-miR-181a* | 12 | 9 |
| hsa-miR-489_st2 | hsa-miR-489 | 21 | 9 |
| hsa-miR-224_st2 | hsa-miR-224 | 86 | 8 |
| hsa-miR-34b_st2 | hsa-miR-34b | 6 | 8 |
| hsa-miR-21_st2 | hsa-miR-21 | 10 | 7 |
| hsa-miR-218_st2 | hsa-miR-218 | 68 | 6 |
| hsa-miR-30a-3p_st1 | hsa-miR-30a-3p | 27 | 5 |
| hsa-asg-14166 st2 | hsa-miR-425-5p | 4 | 5 |
| hsa-miR-189_st1 | hsa-miR-189 | 36 | 4 |
| hsa-miR-148a_st1 | hsa-miR-148a | 15 | 4 |
| hsa-miR-148b_st2 | hsa-miR-148b | 22 | 4 |
| hsa-miR-193a_st1 | hsa-miR-193a | 11 | 4 |
| hsa-miR-106b_st2 | hsa-miR-106b | 12 | 4 |
| hsa-miR-376a_st2 | hsa-miR-376a | 17 | 4 |
| hsa-miR-452_st1 | hsa-miR-452 | 48 | 3 |
| hsa-miR-98_st1 | hsa-miR-98 | 18 | 3 |
| hsa-miR-34a_st1 | hsa-miR-34a | 5 | 3 |
| hsa-miR-27a_st2 | hsa-miR-27a | 17 | 3 |
| hsa-miR-128a_st2 | hsa-miR-128a | 10 | 3 |
| hsa-miR-93_st2 | hsa-miR-93 | 9 | 3 |
| hsa-miR-22_st1 | hsa-miR-22 | 22 | 3 |
| hsa-miR-151_st2 | hsa-miR-151 | 9 | 2 |
| hsa-miR-30e-5p_st2 | hsa-miR-30e-5p | 12 | 2 |
| hsa-miR-30a-5p_st2 | hsa-miR-30a-5p | 12 | 2 |
| hsa-miR-99b_st1 | hsa-miR-99b | 8 | 2 |
| hsa-miR-191_st2 | hsa-miR-191 | 3 | 2 |
| hsa-miR-107_st2 | hsa-miR-107 | 6 | 2 |
| hsa-miR-130b_st1 | hsa-miR-130b | 3 | 2 |
| hsa-miR-181a_st1 | hsa-miR-181 a | 3 | 2 |
| hsa-miR-24_st1 | hsa-miR-24 | 8 | 2 |
| hsa-miR-199a_st2 | hsa-miR-199a | 9 | 2 |
| hsa-miR-103_st1 | hsa-miR-103 | 5 | 2 |
| hsa-miR-25_st2 | hsa-miR-25 | 6 | 2 |
| hsa-miR-363_st2 | hsa-miR-363 | 5 | 2 |

### EXAMPLE 43:

### QRT-PCR VERIFICATION OF DIFFERENTIALLY EXPRESSED MIRNAS IN NORMAL BREAST TISSUE AND CANCER-POSITIVE AND CANCER-NEGATIVE LYMPH NODES FROM BREAST CANCER PATIENTS

The inventors also evaluated the differential expression of selected miRNAs (identified in Example 40 and Example 41) in LNpos and LNneg samples from breast cancer patients (Table 38, Example 40), by qRT-PCR. qRT-PCR reactions were performed using Taqman® MicroRNA Assays (Applied Biosystems; Foster City, CA, USA) specific for eleven miRNAs differentially expressed between LNpos and LNneg and between NBr and LNneg samples (hsa-miR-200b, -200a, -203, -182, -200c, -429, -141, -375, -196a, -205, and - 183). Assays were performed according to the manufacturer's instructions with 15 ng of input total RNA from the seven samples (LNpos2-4 and LNneg1-4). Reactions were incubated in a 7900HT Real-Time PCR system (Applied Biosystems, Foster City, CA, USA). Initial data analysis was done using the 7500 Fast System SDS V2.3 software (Applied Biosystems). miR-638, whose expression level did not change between the different samples, was used for normalization. Results of qRT-PCR expression analysis for the eleven miRNAs were compared with those obtained from microarray expression analysis (FIG. 13A, FIG. 13B) and confirmed the microarray results. Interestingly, the average expression level of hsa-miR-205 was higher in only two (LNpos2 and 3) of the three positive lymph node samples compared to the LNneg samples. In one positive lymph node (LNpos4), however, the average expression level of miR-205 was reduced compared to that observed in negative lymph nodes, indicating some heterogeneity in the expression of miR-205 in metastatic axillary lymph nodes of breast cancer patients.

### EXAMPLE 44:

### MICROARRAY ANALYSIS OF MIRNAS DIFFERENTIALLY EXPRESSED BETWEEN A DISTINCT GROUP OF CANCER-POSITIVE AND CANCER-NEGATIVE LYMPH NODES FROM BREAST CANCER PATIENTS

A second and independent set of FFPE samples of ten cancer-positive (LNpos5-14) and five cancer-negative (LNneg5-9) axillary lymph nodes from ductal breast cancer patients were purchased from ProteoGenex (ProteoGenex, Inc.; Culver City, CA, USA) (Table 42). A pathology report and hematoxilin-eosin (H&E)-stained slides were available for each lymph node sample. H&E slides were reviewed by a pathologist at Asuragen to confirm lymph node statuses as cancer cell-positive or cancer cell-negative.

**Table 42. Patient and tissue sample pathology information for ten cancer-positive (LNpos) and five cancer-negative (LNneg) axillary lymph nodes from 15 independent ductal breast cancer patients. TNM stage (Sobin and Wittekind, 2002); AJCC Stage (Greene, 2002); LN Mets, number of lymph nodes with metastases/total number of lymph nodes examined.**

| **Sample ID** | **LN Cancer Diagnosis** | **Sex** | **Patient Age (yr) Ethnicity** | **Patient's Histological Diagnosis** | **Tumor size (cm)** | **TNM Stage** | **AJCC Stage** | **LN Mets** |
|---|---|---|---|---|---|---|---|---|
| LNpos5 | metastatic | F | 65 Caucasian | infiltrating ductal carcinoma | 4.3x3x22 | T2N2M0 | IIIA | 7/10 |
| LNpos6 | metastatic | F | 73 Caucasian | infiltrating ductal carcinoma | 4x2x3.5 | T4bN2M0 | IIIB | 11/11 |
| LNpos7 | metastatic | F | 54 Caucasian | infiltrating ductal carcinoma | 3x2.3x1.5 | T2N1M0 | IIB | 2/22 |
| LNpos8 | metastatic | F | 44 Caucasian | infiltrating ducal carcinoma | 4.5x4.5x2.3 | T2N2M0 | IIIA | 12/18 |
| LNpos9 | metastatic | F | 44 Caucasian | infiltrating ductal carcinoma | 5x3x4.5 | T2N2M0 | IIIA | 9/16 |
| LNpos10 | metastatic | F | 42 Caucasian | infiltrating ductal carcinoma | 2.5x3.2x1.6 | T2N1M0 | IIB | 3/13 |
| LNpos11 | metastatic | F | 69 Caucasian | infiltrating ductal carcinoma | 5.5x3x2.5 | T3N2M0 | IIIA | 13/37 |
| LNpos12 | metastatic | F | 44 Caucasian | infiltrating ductal carcinoma | 5x4.5x4 | T2N2M0 | IIIA | 6/13 |
| LNpos13 | metastatic | F | 54 Caucasian | infiltrating ductal carcinoma | 1.8x3x1; 5x3.7x3.5 | T2N2M0 | IIIA | 7/15 |
| LNpos14 | metastatic | F | 46 Caucasian | infiltrating ductal carcinoma | 2.8x2.5x2.7 | T2N1M0 | IIB | 2/5 |
| LNneg5 | normal | F | 50 Caucasian | infiltrating ductal carcinoma | 3 | T2N0M0 | IIA | 0/9 |
| LNneg6 | normal | F | 50 Caucasian | infiltrating ductal carcinoma | 2.3 | T2N1M0 | IIB | 1/4 |
| LNneg7 | normal | F | 60 Caucasian | infiltrating ductal carcinoma | N/A | T1N1M0 | IIA | 2/6 |
| LNneg8 | normal | F | 35 Caucasian | infiltrating ductal carcinoma | 2.5 | T2N1M0 | IIB | 2/12 |
| LNneg9 | normal | F | 49 Caucasian | infiltrating ductal carcinoma | 3.2x3x2.3 | T2N1M0 | IIB | 2/9 |

Total RNA isolation from the FFPE lymph node samples was performed using the RecoverAll™ Total Nucleic Acid Isolation Kit (cat. No. AM1975; Ambion, Inc.; Austin, TX, USA), as described above in Example 1 and analyzed on miRNA microarrays, as described below.

These samples were evaluated using the Agilent Human miRNA Microarray (V2) (Agilent Technologies, Inc.; Santa Clara, CA USA), which contains probes for 723 human and 76 human viral microRNAs from the miRBase database V.10.1 (Griffiths-Jones *et al.,* 2006). Array hybridization, washing, staining, imaging, and signal extraction were performed according to Agilent's recommended procedures, as explained below.

**miRNA array expression analysis:** Samples for miRNA profiling studies were processed by Asuragen Services (Asuragen, Inc.; Austin, TX, USA). Total RNA from each sample (100 ng) was dephosphorylated and a pCp-Cy3 labeling molecule was ligated to the 3'end of the RNA molecules, following the manufacturer's recommendations (Agilent Technologies, Inc. Santa Clara, USA). The labeled RNA was purified using a Bio-Spin P-6 column (Bio-Rad Laboratories Inc.; Hercules, CA, USA).

**miRNA array signal processing:** The signal processing implemented for the Agilent miRNA array is a multi-step process involving probe specific signal detection calls, background correction, and global normalization. For each probe, the contribution of signal due to background was estimated and removed by the Agilent Feature Extraction software as part of the data file output. Similarly, detection calls were based on the Agilent Feature Extraction software. Arrays within a specific analysis experiment were normalized together according to the VSN method described elsewhere (Huber *et al.,* 2002).

**Background estimate and correction and probe detection:** Three types of data are provided to evaluate each hybridization. The "Total Gene Signal" is the total probe signal multiplied by the number of probes per gene and is calculated after the background effects have been accounted for. The "Total Gene Error" is the square root of the square of the total probe error multiplied by the number of probes per gene. The "Total Probe Error" is the robust average for each replicated probe multiplied by the total number of probe replicates. The "Detection Call" is a binary number that indicates if the gene was detected on the miRNA microarray. Probes detected at least once across all samples in the experiment were considered for statistical analysis.

**Global normalization:** The inventors have found that the Variance Stabilization Normalization (VSN) algorithm provides an ideal balance of accuracy and precision while optimizing sensitivity and specificity of signal. One advantage of VSN, is that it accommodates negative values by using the generalized log₂ transformation.

**Generalized log₂ transformed:** The post-normalized data scale is reported as generalized log₂ data. The distribution of microarray data is typically log normal *(i.e.,* it tends to follow a normal distribution pattern after log transformation). A normal distributed data is amendable to classical statistical treatments, including t-tests and one-way or two-way ANOVA.

For statistical hypothesis testing, a two-sample t-test, with assumption of equal variance, was applied. This test is used to define which probes are considered to be significantly differentially expressed, or "significant", based on false discovery rate set at 0.05.

LNpos5-14 and LNneg5-9 lymph node specimens from breast cancer patients were evaluated. A total of 204 human miRNAs were expressed above background level in the LNpos samples, representing 28% of the human miRNAs present on the arrays. A total of 176 human miRNAs were expressed above background level in the LNneg samples, representing 24% of the human miRNAs present on the arrays.

A total of 61 miRNAs were significantly differentially expressed between LNpos and LNneg samples (Table 43). Among these, 54 miRNA had average expression levels higher in LNpos compare to LNneg samples. Three miRNAs (hsa-miR-200c, -141, and - 200b) had average expression levels between 170- and 400-fold higher in LNpos, six miRNAs (hsa-miR-183, -200a, -375, -429, -205, and -96) had average expression levels between 20- and 65- higher in LNpos, and nine miRNAs (hsa-miR-196a, -21, -200a*, -21*, - 210, -200b*, -503, -301a, -and -425) had average expression levels between 5- and 10- fold higher in LNpos. A total of three miRNAs (hsa-miR-150, -139-5p, and -139-3p) had average expression levels between 2- and 5- fold lower in LNpos than in LNneg samples.

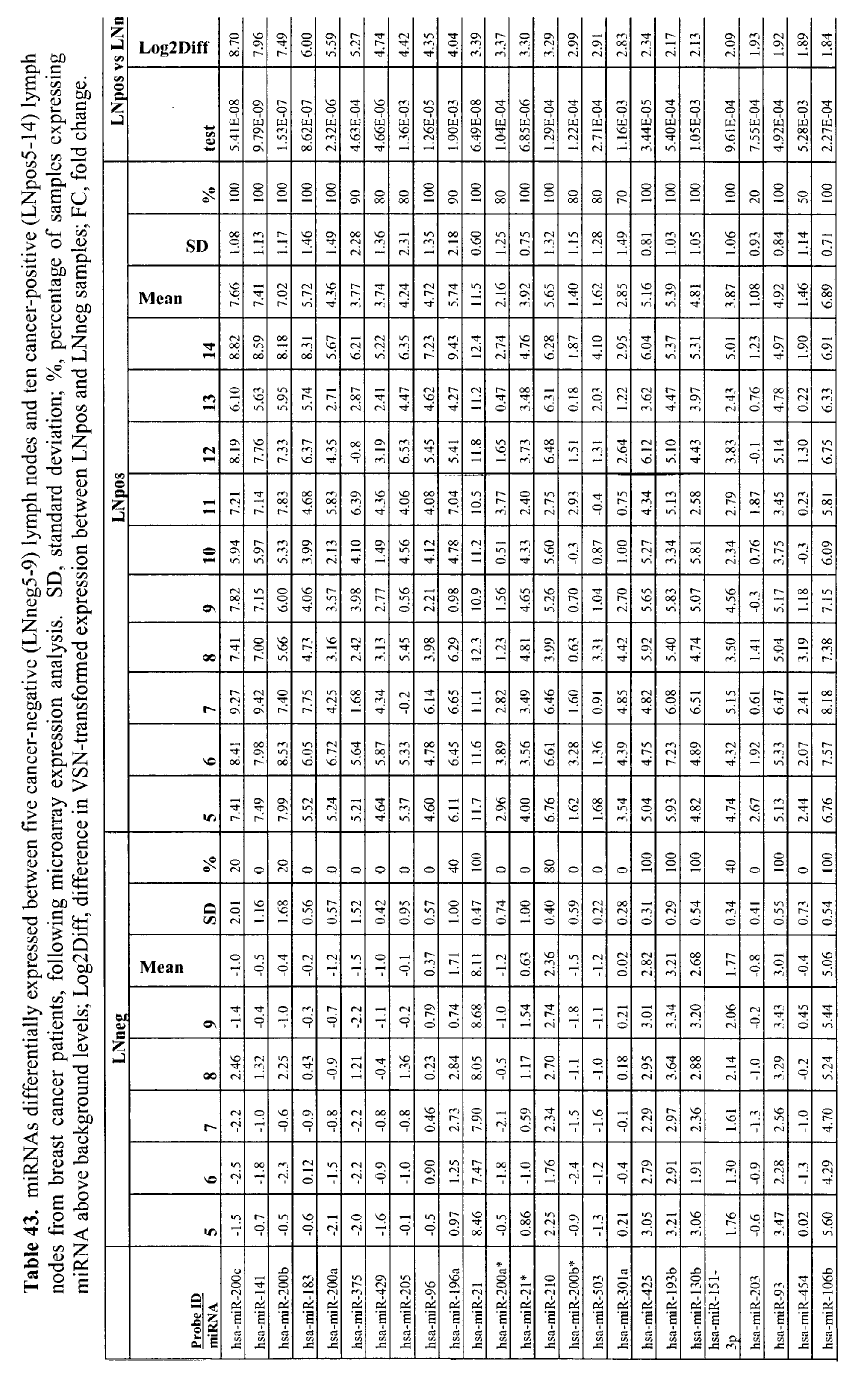

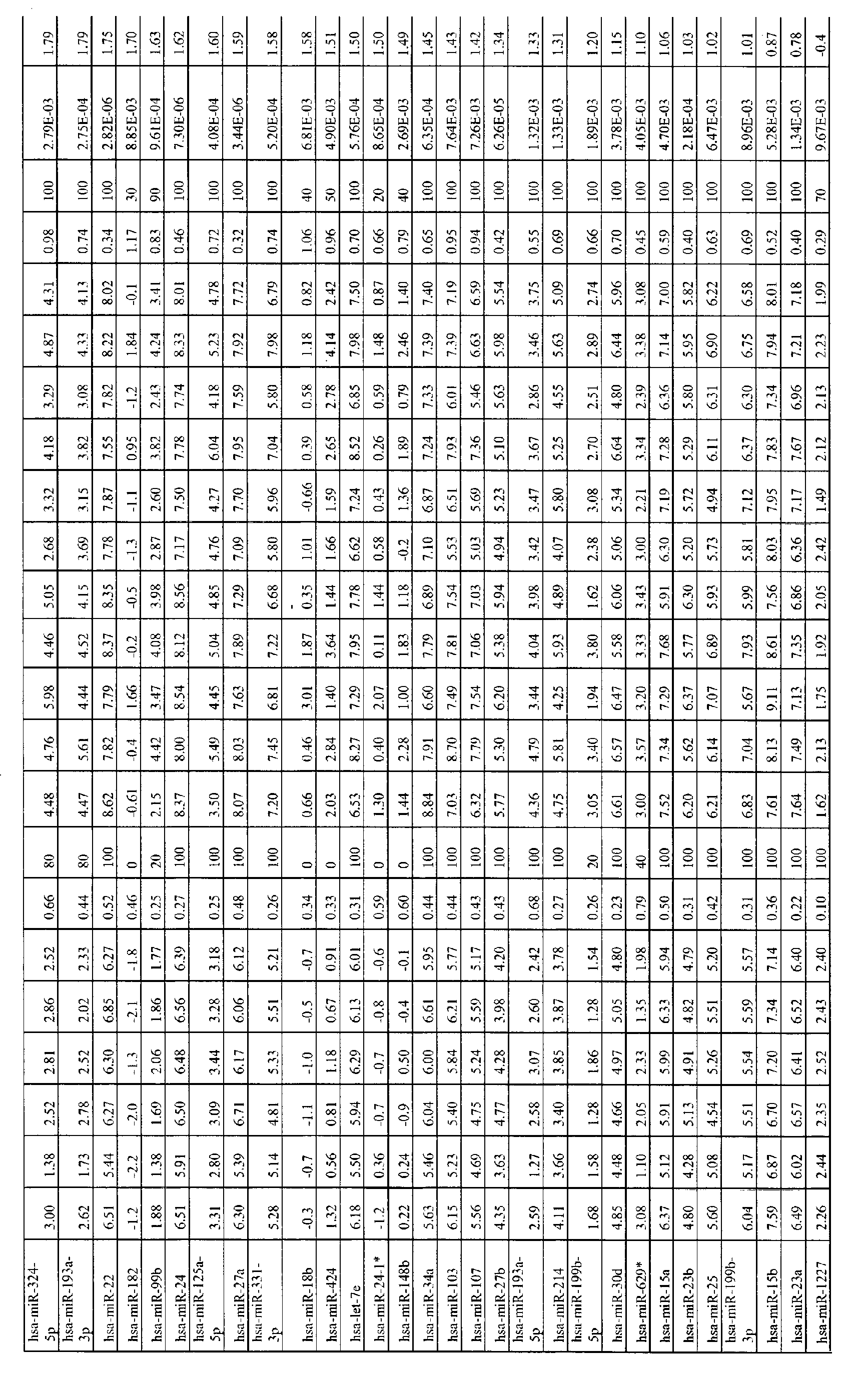

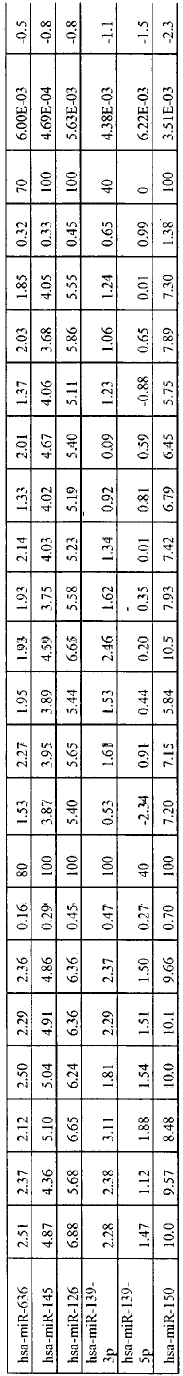

### EXAMPLE 45:

### QRT-PCR VERIFICATION OF DIFFERENTIALLY EXPRESSED MICRORNAS IN TWO GROUP OF CANCER-POSITIVE AND CANCER-NEGATIVE LYMPH NODES FROM BREAST CANCER PATIENTS

The inventors evaluated the differential expression of ten selected miRNAs (miR-429, -200a, -375, -200b, -203, -141, -200c, -183, -196a, and -205) in LNpos and LNneg samples from breast cancer patients (Table 38, Example 40 and Table 42, Example 44), by qRT-PCR. qRT-PCR reactions were performed using Taqman® MicroRNA Assays (Applied Biosystems; Foster City, CA, USA) specific for the ten microRNAs. Assays were performed according to the manufacturer's instructions with 15 ng of input total RNA from the samples (LNpos2-4, LNpos5-14 and LNneg1-9). Reactions were incubated in a 7900HT Real-Time PCR system (Applied Biosystems, Foster City, CA, USA). Initial data analysis was done using the 7500 Fast System SDS V2.3 software (Applied Biosystems). With this combined set of samples, miR-638 expression level was found to be more variable across the samples than in qRT-PCR reactions performed with only samples from Table 38 (Example 43). Here, the miR-16 expression level was constant among the different samples. As a consequence, the inventors chose to use the mean of the sum of the Ct values for both miR-16 and miR-638 (1/2(Ct miR-16 + Ct miR-638)) for normalization (dCt= Ct miRNA -1/2(Ct miR-16 + Ct miR-638). Average expression levels of each miRNA in LNneg and LNpos of the two independent sets of samples are illustrated after normalization to miR-16 and miR-638 (FIG. 14A, FIG. 14B). qRT-PCR quantification, using miR-16 and miR-638 as normalizers, confirmed the differential expression of the ten miRNAs in the LNneg1-4 and LNpos2-4 samples, (FIG. 14A), as well as in LNpos 5-14 and LNneg 5-9 (FIG. 14B), validating their differential expression in LNneg and LNpos from breast cancer patients. Combined qRT-PCR data for the two sets of samples (LNpos2-14 and LNneg1-9) (FIG. 15) showed that seven miRNAs in particular, miR-429, -200a, -200b, -203, -141, -200c, and -375, represent useful markers for the identification of breast cancer cells in axillary lymph nodes of breast cancer patients.

### EXAMPLE 46:

### IDENTIFICATION OF CLASSIFIERS FOR CANCER-POSITIVE LYMPH NODES IN PATIENTS WITH DUCTAL BREAST CANCER

The inventors have demonstrated that miRNA quantification and expression analysis can differentiate cancer-positive from cancer-negative axillary lymph nodes from breast cancer patients. To further demonstrate the utility of specific miRNA markers as diagnostic analytes, the inventors quantified specific combinations of miRNAs by qRT-PCR to determine if any such combinations enhanced the distinction between cancer-positive and cancer-negative lymph nodes from breast cancer patients. qRT-PCR assays were performed as described above in Example 45. For the miRNA combinations miR-429+miR-200a, miR-429+miR-141, miR-200b+miR-203, the normalized Ct values to both miR-16 and miR-638 were added. For the combinations, miR-429-miR-150 and miR-200a-miR-150, the raw Ct values of each miRNA were subtracted. The inventors determined that quantification of several miRNA combinations such as miR-429+miR-200a, miR-429+miR-141, miR-200b+miR-203, miR-429-miR-150, and miR200a-miR-150, enhances the distinction between cancer-positive and cancer-negative lymph nodes from breast cancer patients (FIG. 16).

### EXAMPLE 47:

### ADDITIONAL MICROARRAY ANALYSIS OF MICRORNA DIFFERENTIALLY EXPRESSED BETWEEN CANCER-POSITIVE AND CANCER-NEGATIVE INGUINAL LYMPH NODES FROM CERVICAL CANCER PATIENTS

Further microRNA microarray expression analysis was performed on the LNpos and LNneg inguinal lymph nodes from cervical cancer patients that have been described above in Example 1, Table 3. Here, the microRNA array used for expression analysis was the Agilent Human miRNA Microarray (V2) (Agilent Technologies, Inc.; Santa Clara, CA, USA), described above in Example 44. Microarray expression analysis was as described above in Example 44.

A total of 324 human miRNAs were expressed above background level in the LNpos samples, representing 45% of the miRNAs present on the arrays. A total of 260 miRNAs were expressed above background level in the LNneg samples, representing 36% of the miRNAs present on the arrays.

A total of 107 human miRNAs were significantly differentially expressed between LNpos and LNneg (Table 44). Among these, 57 were up-regulated (Log2 diff (LNpos vs LNneg) ≥ 1) and 40 were down-regulated (Log2 diff (LNpos vs LNneg) ≤ 1) by more than 2-fold in LNpos as compared with LNneg samples. Twenty-two miRNAs were expressed at levels at least 5-fold higher (Log2 diff (LNpos vs LNneg) ≤ 2.3) in the LNpos samples, with a Student t-test *p*-value ≤ 0.01. Of those, 10 miRNAs (hsa-miR-205, -200b, -203, -200a, -429, -200c, -141, -224, - 135b, and -200b*) were expressed at levels at least 10-fold higher in the LNpos samples (Log2 diff (LNpos vs LNneg) ≥ 3.3). Among the miRNAs expressed at lower levels in the LNpos samples, only one (hsa-miRNA-342-5p) was under-expressed by at least 5-fold compared to the LNneg samples, with a Student t-test p-value ≤ 0.01.

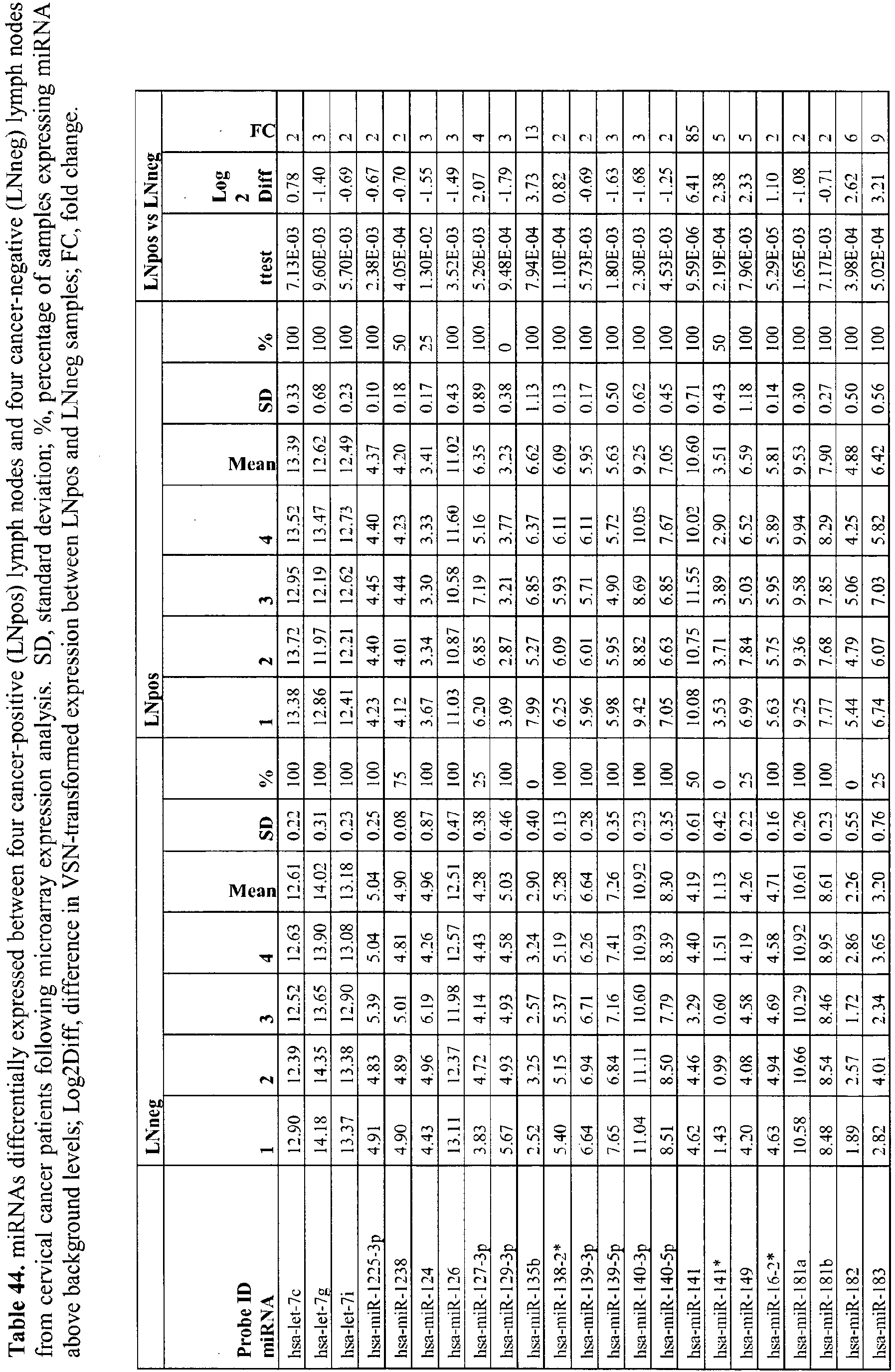

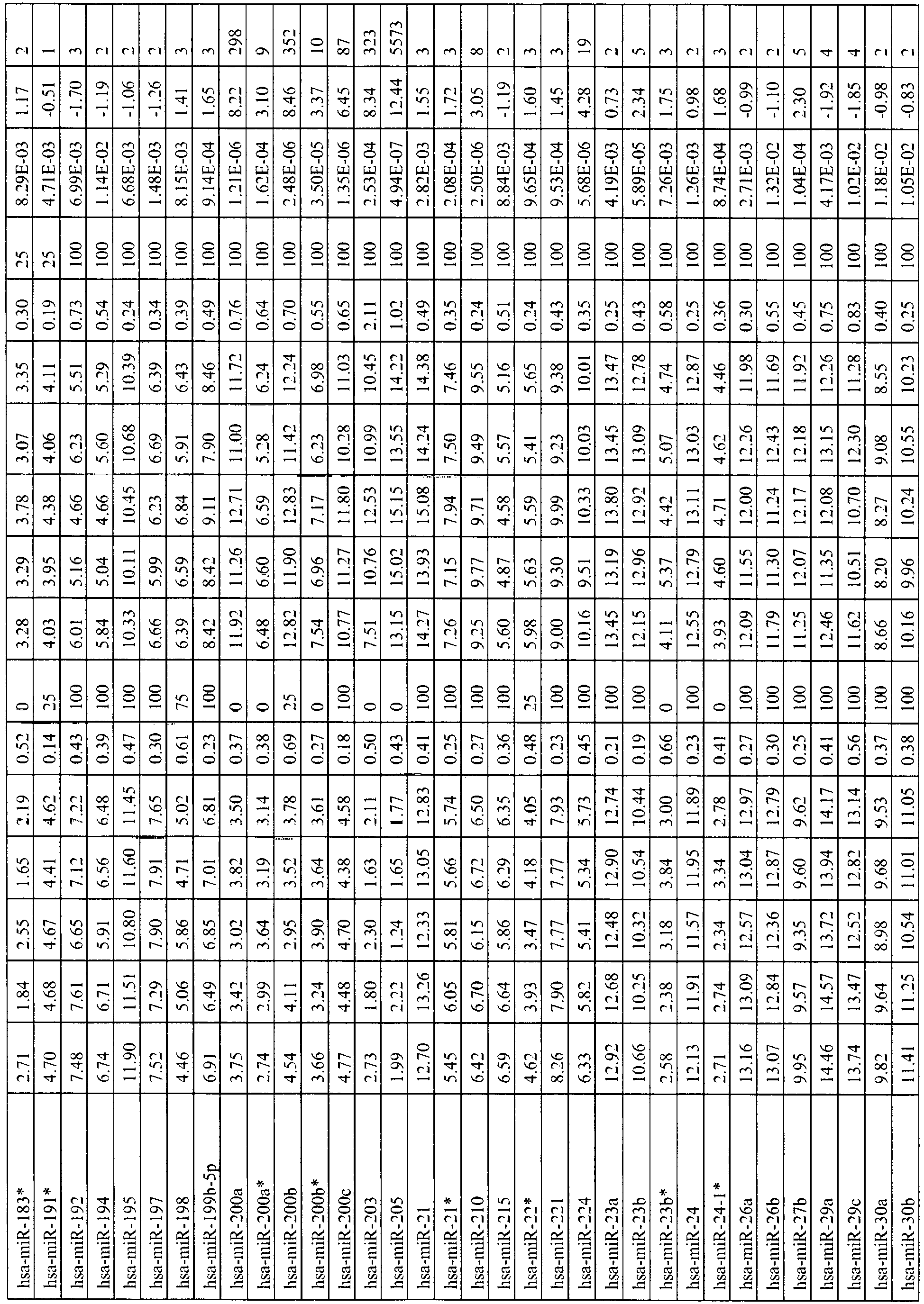

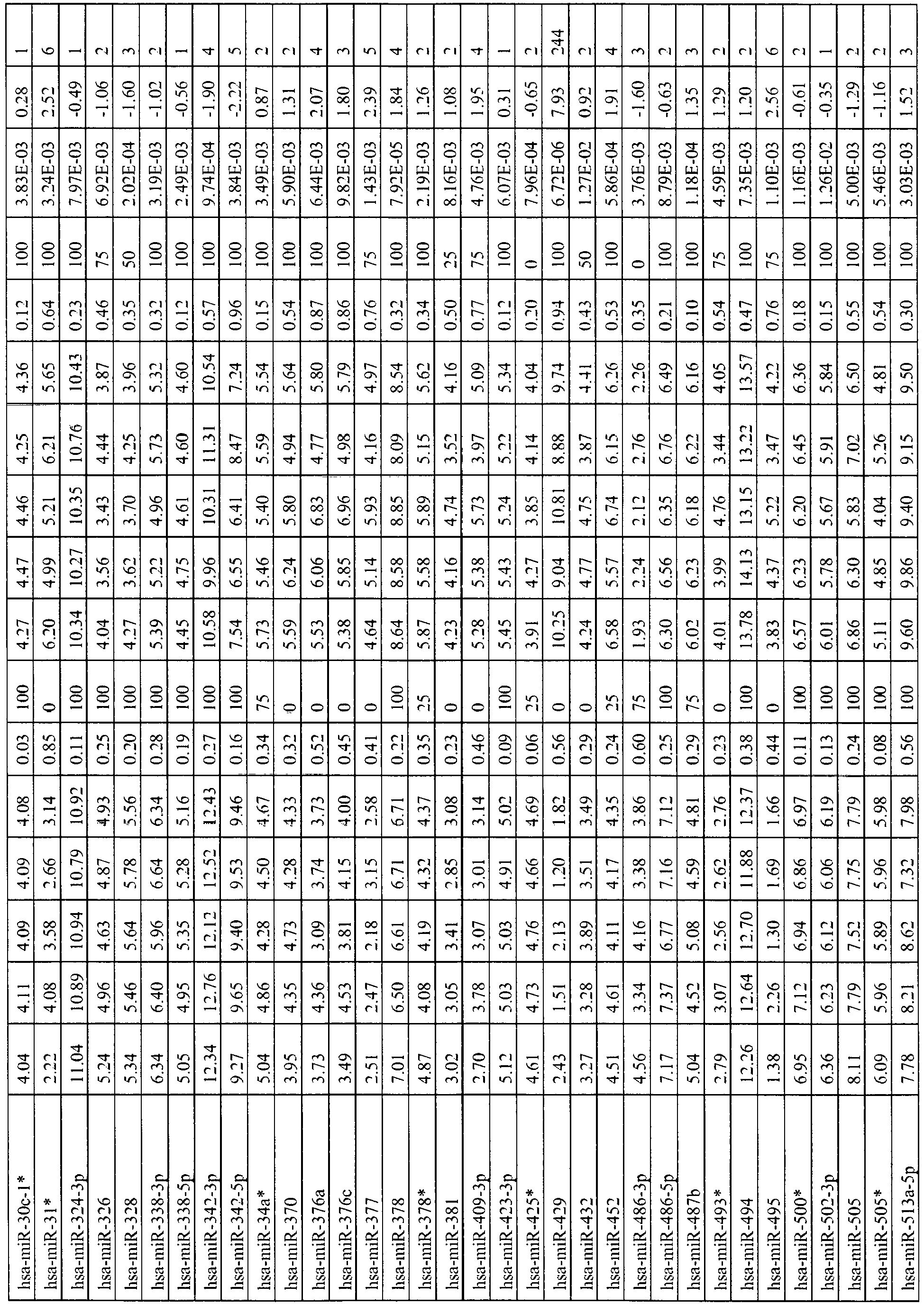

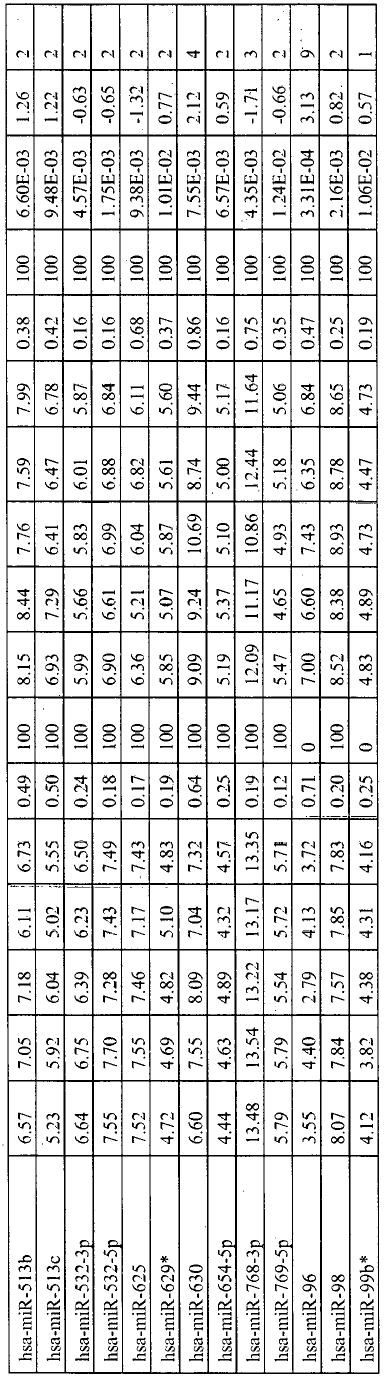

### EXAMPLE 48:

### QRT-PCR VERIFICATION OF DIFFERENTIALLY EXPRESSED MIRNAS USING FFPE MELANOMA SAMPLESAND FFPE CANCER-POSITIVE LYMPH NODES FROM MELANOMA PATIENTS

The inventors further evaluated differential expression of miRNAs using a set of formalin-fixed, paraffin-embedded (FFPE) tissue samples that included four cancer-positive lymph nodes (LNpos5-8) and three melanoma samples (MEL4-6) (Tables 8 and 9). These samples were independent and distinct from the frozen melanoma biopsy samples (Table 8) and the frozen cancer-positive lymph node samples (Table 9) previously evaluated (Examples 11, 12, and 13).

QRT-PCR quantification of 14 selected miRNAs was performed using TaqMan® MicroRNA Assays (Applied Biosystems) as described in Example 13. Differential expression of the miRNAs was evaluated by comparing the expression level of each miRNA in MEL4-6 (FIG. 17A) and in LNpos5-8 (FIG. 17B) with their expression levels in LNneg1-7. These experiments confirmed the differential expression, between MEL4-6 and LNneg1-7, for the 14 miRNAs (FIG. 17A). These experiments also verified the differential expression of miR-513 (FIGs. 17A, 17B) which had been previously identified as differentially expressed by array analysis (Table 10, Table 11), but which had not been evaluated by qRT-PCR. In addition, these experiments confirmed the differential expression of seven of the 14 miRNAs between cancer-positive lymph nodes (LNpos5-8) and cancer-negative lymph nodes (LNneg1-7) (FIG. 17B). The cancer-positive lymph node designated LNpos8 was an outlier for miR-211, the most differentially expressed miRNA of the fourteen evaluated here (FIG. 17B). In addition, LNpos8, like LNpos1A, had higher expression of miR-203 than was observed for the LNneg samples. However, the differential expression of the other miRNAs in LNpos8 vs LNneg1-7 was consistent with the pattern observed for the other cancer-positive lymph nodes (LNpos5-7, FIG. 17B).

QRT-PCR quantification of fourteen different miRNAs, in all 15 lymph node samples from melanoma patients (Table 9) is summarized in FIG. 18. The inventors have identified two subgroups of cancer-positive lymph nodes (LNposA, LNposB) from melanoma patients. The LNposA subgroup is represented by a single cancer-positive lymph node - LNposlA. The LNposB subgroup is represented by the other cancer-positive lymph nodes (LNpos2-8). Within the LNposB subgroup LNpos8 is an outlier for hsa-miR-211 and hsa-miR-203 expression.

The inventors have shown that miRNA array profiling and qRT-PCR analysis can distinguish between cancer-positive and cancer-negative lymph nodes from melanoma patients. The inventors have identified seven miRNAs (hsa-miR-211, -514, -506, -509, -508, -513, and -584) (FIG. 18) that are expressed at significantly higher levels in all cancer-positive lymph nodes and melanoma samples than in cancer-negative lymph nodes. The results are consistent when analyses are performed with either frozen biopsy specimens or FFPE tissue samples. These miRNAs represent effective markers for the identification of metastatic melanoma in the lymph nodes.

In addition, the inventors identified six miRNAs (hsa-miR-200b, -200c, -203, - 375, -429, and -592) that are expressed at significantly higher levels in the LNposA subgroup of cancer-positive lymph nodes (FIG. 8, FIG. 18) and in melanoma samples (FIG. 7, FIG. 17) compared to cancer-negative lymph nodes (LNneg). These six miRNAs were not significantly differentially expressed between the LNposB subgroup of cancer-positive lymph nodes and cancer-negative lymph nodes (LNneg) (FIG. 18). These miRNAs represent useful diagnostic markers for distinguishing between the two subgroups (LNposA, LNposB) of cancer-positive lymph nodes in metastatic melanoma patients. The heterogeneity observed in the miRNA expression profiles among the cancer-positive lymph nodes correlates with the fact that melanoma is a heterogeneous disease that involves multiple biological pathways.

### EXAMPLE 49:

### MICRORNA COMBINATIONS AS CLASSIFIERS FOR CANCER-POSITIVE LYMPH NODES IN PATIENTS WITH MELANOMA

The inventors have demonstrated that miRNAs can distinguish cancer-positive from cancer-negative lymph nodes taken from melanoma patients. To further refine the utility of specific microRNA markers as diagnostic analytes, the inventors quantified specific combinations of miRNAs by qRT-PCR and identified combinations that enhanced the distinction between cancer-positive and cancer-negative lymph nodes from melanoma patients. qRT-PCR assays were performed as described above in Example 13. For combinations of two or more miRNAs, the Ct values from the quantification of individual miRNAs were added following their normalization to miR-16. The inventors determined that quantification of several miRNA combinations (miR-211+miR-514; miR-211+miR-506; miR-211+miR-514+miR-506; miR-211+miR-509+miR-508) enhances the distinction between cancer-positive and cancer-negative lymph nodes from melanoma patients (FIG. 19). These combinations serve as non-limiting examples. Other combinations may also be used and combinations may include subtraction of average Ct values. In that case, if an even number of miRNAs is present in the combination, the subtraction of Ct values in a combination eliminates the need to normalize the data.

### REFERENCES

Bagga et al., Cell 122(4):553-563, 2005.
Bentwich et al., Nat. Genet, 37(7):766-770, 2005.
Berezikov et al., Cell 120(1):21-24, 2005.
Breslow, Ann. Surg. 172(5):902-908, 1970.
Calin and Croce, Nat. Rev. Cancer, 6(11):857-866, 2006.
Carrington et al. Science, 301(5631):336-338, 2003.
Clark et al., Cancer Res., 29(3):705-727, 1969.
Coello et al., Clin. Lung Cancer, 5:214-225, 2004.
Cummins et al., Proc. Natl. Acad. Sci. USA 103(10):3687-3692, 2006.
Davison et al., Meth. Enzymol., 411:14-34, 2006.
D'Cunha et, al., Lung Cancer, 48:241-246, 2005.
Doleshal et al., J. Mol. Diag. 10(3):203-211, 2008.
Esquela-Kerscher and Slack, Nat Rev Cancer, 6(4):259-269, 2006.
Ferris et al., Cancer Res., 65:2147-2156, 2005.
Gillanders et al., Ann.Surg., 239:828-837, 2004.
Greene, AJCC Cancer Staging Manual (6th ed.) Greene et al. (eds.). Springer-Verlag, New York. 2002.
Griffiths-Jones et al., Nucleic Acids Res., 34 (Database Issue):D140-D144, 2006.
Houvenaeghel et al., J.Clin.Oncol., 24:1814-1822, 2006.
Huber et al., Bioinformatics, 18:Suppl 1:S96-104, 2002.
Hughes et al., Ann.Surg., 243:389-398, 2006.
Kammula et al., J.Clin.Oncol., 22:3989-3996, 2004.
Lagos-Quintana et al., Science, 294(5543):853-858, 2001.
Lau et al., Science, 294(5543):858-862, 2001.
Lee and Ambros, Science, 294(5543):862-864, 2001.
Lim et al., Nature, 433(7027):769-773, 2005.
MAQC Consortium, Nature Biotech. 24:1151-1161, 2006.
Roberts et al., Am. J. Surg., 186:324-329, 2003.
Schurr et al., J. Surg. Oncol., 94:307-315, 2006.
Scoggins et al., J.Clin.Oncol., 24:2849-2857, 2006.
Shingara et al., RNA, 9:1461-1470, 2005
Sobin and Wittekind, TNM Classification of Malignant Tumors (6th ed.), Sobin and
Wittekind (eds.), John Wiley and Sons, New Jersey. 2002.
Takeuchi et al., J.Clin.Oncol., 22:2671-2680, 2004.
Wang et al., RNA 13:1-9. 2007.
Xi et al., Clin.Cancer Res., 11:1099-1109, 2005.
Xi et al., Clin.Cancer Res., 12:2484-2491, 2006.
Xie et al., Nature 434(7031):338-345, 2005

## Claims

1. A method of assessing a patient comprising the steps of:
(a) measuring miR-205 levels in a lymph node sample; and
(b) determining if a lymph node metastasis from the cervix is present in the sample by evaluating the expression levels of miR-205.

2. The method of claim 1, further comprising providing a diagnosis, prognosis, or staging of the patient's condition or disease.

3. The method of claim 1, further comprising measuring expression levels of two or more miRNAs selected from hsa-miR-200a, hsa-miR-200b, or hsa-miR-203.

4. The method of claim 1, comprising measuring expression levels of hsa-miR-205 and hsa-miR-429.

5. The method of claim 1, comprising measuring expression levels of hsa-miR-205 and hsa-miR-203.

6. The method of claim 1, comprising measuring expression levels of hsa-miR-205, hsa-miR-429, and hsa-miR-203.

7. The method of claim 1, wherein the lymph node sample is obtained from a lymph node positioned locally to a cancer or suspected cancer.

8. The method of claim 7 wherein the lymph node is a sentinel lymph node (SLN).

9. The method of claim 7, wherein the lymph node is an inguinal lymph node.

10. The method of claim 1, wherein the miRNA expression levels in the lymph node sample are measured by using RT-PCR and/or hybridization.

## Patentansprüche

1. Verfahren des Beurteilens eines Patienten, das folgende Schritte umfasst:
(a) Messen von miR-205-Konzentrationen in einer Lymphknotenprobe und
(b) Feststellen, ob in der Probe eine Lymphknotenmetastase vom Gebärmutterhals vorhanden ist, indem die Expressionsgrade von miR-205 evaluiert werden.

2. Verfahren nach Anspruch 1, ferner umfassend das Vorsehen einer Diagnose, Prognose oder Beurteilung des Stadiums des Zustands oder der Krankheit des Patienten.

3. Verfahren nach Anspruch 1, ferner umfassend das Messen von Expressionsgraden von mindestens zwei miRNAs, die aus hsa-miR-200a, hsa-miR-200b oder hsa-miR-203 ausgewählt sind.

4. Verfahren nach Anspruch 1, umfassend das Messen von Expressionsgraden von hsa-miR-205 und hsa-miR-429.

5. Verfahren nach Anspruch 1, umfassend das Messen von Expressionsgraden von hsa-miR-205 und hsa-miR-203.

6. Verfahren nach Anspruch 1, umfassend das Messen von Expressionsgraden von hsa-miR-205, hsa-miR-429 und hsa-miR-203.

7. Verfahren nach Anspruch 1, wobei die Lymphknotenprobe von einem Lymphknoten erhalten wird, der lokal zu einem Krebs oder vermuteten Krebs positioniert ist.

8. Verfahren nach Anspruch 7, wobei es sich bei dem Lymphknoten um einen Wächterlymphknoten (Sentinel-Lymphknoten, SLN) handelt.

9. Verfahren nach Anspruch 7, wobei es sich bei dem Lymphknoten um einen Leistenlymphknoten handelt.

10. Verfahren nach Anspruch 1, wobei die miRNA-Expressionsgrade in der Lymphknotenprobe mittels RT-PCR und/oder Hybridisierung gemessen werden.

## Revendications

1. Méthode d'évaluation d'un patient comprenant les étapes suivantes consistant à :
(a) mesurer les taux de miR-205 dans un échantillon de ganglion lymphatique ; et
(b) déterminer si des métastases des ganglions lymphatiques provenant du col de l'utérus sont présentes dans l'échantillon par évaluation des taux d'expression de miR-205.

2. Méthode selon la revendication 1, comprenant en outre la fourniture d'un diagnostic, d'un pronostic, ou la détermination du stade de l'affection ou de la maladie du patient.

3. Méthode selon la revendication 1, comprenant en outre la mesure des taux d'expression de deux miARN ou plus choisis parmi hsa-miR-200a, hsa-miR-200b ou hsa-miR-203.

4. Méthode selon la revendication 1, comprenant la mesure des taux d'expression de hsa-miR-205 et hsa-miR-429.

5. Méthode selon la revendication 1, comprenant la mesure des taux d'expression de hsa-miR-205 et hsa-miR-203.

6. Méthode selon la revendication 1, comprenant la mesure des taux d'expression de hsa-miR-205, hsa-miR-429 et hsa-miR-203.

7. Méthode selon la revendication 1, dans laquelle l'échantillon de ganglion lymphatique est obtenu à partir d'un ganglion lymphatique positionné localement par rapport à un cancer ou un cancer suspecté.

8. Méthode selon la revendication 7, dans laquelle le ganglion lymphatique est un ganglion lymphatique sentinelle (GLS).

9. Méthode selon la revendication 7, dans laquelle le ganglion lymphatique est un ganglion lymphatique inguinal.

10. Méthode selon la revendication 1, dans laquelle les taux d'expression de miARN dans l'échantillon de ganglion lymphatique sont mesurés en utilisant une RT-PCR et/ou une hybridation.
